# EUROPEAN PATENT SPECIFICATION

(11) **EP 3 558 963 B1**
(45) Date of publication and mention of the grant of the patent: **23.03.2022**
(21) Application number: 17829877.4
(22) Date of filing: 21.12.2017
(51) Int. Cl.: C07D 401/04, C07D 519/00, A61P 37/00, A61K 31/519, A61K 31/4375

(54) **BICYCLIC HETEROAROMATIC COMPOUNDS AS IMMUNOMODULATORS**
BICYCLISCHE HETEROAROMATISCHE VERBINDUNGEN ALS IMMUNMODULATOREN
COMPOSÉS HÉTÉROAROMATIQUES BICYCLIQUES UTILISÉS EN TANT QU'IMMUNOMODULATEURS

(30) Priority: 22.12.2016 US 201662438038 P; 19.04.2017 US 201762487362 P; 28.08.2017 US 201762551011 P
(43) Date of publication of application: 30.10.2019
(73) Proprietor: Incyte Corporation, Wilmington, DE 19803 (US)
(72) Inventor: QI, Chao, Wilmington Delaware 19803 (US); KONKOL, Leah C., Wilmington Delaware 19803 (US); WU, Liangxing, Wilmington Delaware 19803 (US); LAJKIEWICZ, Neil, Wilmington Delaware 19803 (US); HE, Chunhong, Wilmington Delaware 19803 (US); XIAO, Kaijiong, Wilmington Delaware 19803 (US); ZHU, Wenyu, Wilmington Delaware 19803 (US); LI, Zhenwu, Wilmington Delaware 19803 (US); MEI, Song, Wilmington Delaware 19803 (US); YAO, Wenqing, Wilmington Delaware 19803 (US)
(74) Representative: Carpmaels & Ransford LLP
(86) International application number: PCT/US2017/067984
(87) International publication number: WO 2018/119286

(56) References cited:
- WO-A1-2017/112730
- WO-A2-2011/082400
- TRYFON ZARGANES-TZITZIKAS ET AL: "Inhibitors of programmed cell death 1 (PD-1): a patent review (2010-2015)", EXPERT OPINION ON THERAPEUTIC PATENTS., vol. 26, no. 9, 19 September 2016 (2016-09-19), pages 973-977, XP055394015, GB ISSN: 1354-3776, DOI: 10.1080/13543776.2016.1206527

## Description

### FIELD OF THE INVENTION

The present application is concerned with pharmaceutically active compounds. The disclosure provides compounds as well as their compositions and their uses. The compounds modulate PD-1/PD-L1 protein/protein interaction and are useful in the treatment of various diseases including infectious diseases and cancer.

### BACKGROUND OF THE INVENTION

The immune system plays an important role in controlling and eradicating diseases such as cancer. However, cancer cells often develop strategies to evade or to suppress the immune system in order to favor their growth. One such mechanism is altering the expression of co-stimulatory and co-inhibitory molecules expressed on immune cells (Postow et al, J. Clinical Oncology 2015, 1-9). Blocking the signaling of an inhibitory immune checkpoint, such as PD-1, has proven to be a promising and effective treatment modality.

Programmed cell death-1 (PD-1), also known as CD279, is a cell surface receptor expressed on activated T cells, natural killer T cells, B cells, and macrophages (Greenwald et al, Annu. Rev. Immunol 2005, 23:515-548; Okazaki and Honjo, Trends Immunol 2006, (4):195-201). It functions as an intrinsic negative feedback system to prevent the activation of T-cells, which in turn reduces autoimmunity and promotes self-tolerance. In addition, PD-1 is also known to play a critical role in the suppression of antigen-specific T cell response in diseases like cancer and viral infection (Sharpe et al, Nat Immunol 2007 8, 239-245; Postow et al, J. Clinical Oncol 2015, 1-9).

The structure of PD-1 consists of an extracellular immunoglobulin variable-like domain followed by a transmembrane region and an intracellular domain (Parry et al, Mol Cell Biol 2005, 9543-9553). The intracellular domain contains two phosphorylation sites located in an immunoreceptor tyrosine-based inhibitory motif and an immunoreceptor tyrosine-based switch motif, which suggests that PD-1 negatively regulates T cell receptor-mediated signals. PD-1 has two ligands, PD-L1 and PD-L2 (Parry et al, Mol Cell Biol 2005, 9543-9553; Latchman et al, Nat Immunol 2001, 2, 261-268), and they differ in their expression patterns. PD-L1 protein is upregulated on macrophages and dendritic cells in response to lipopolysaccharide and GM-CSF treatment, and on T cells and B cells upon T cell receptor and B cell receptor signaling. PD-L1 is also highly expressed on almost all tumor cells, and the expression is further increased after IFN-γ treatment (Iwai et al, PNAS2002, 99(19): 12293-7; Blank et al, Cancer Res 2004, 64(3): 1140-5). In fact, tumor PD-L1 expression status has been shown to be prognostic in multiple tumor types (Wang et al, Eur J Surg Oncol 2015; Huang et al, Oncol Rep 2015; Sabatier et al, Oncotarget 2015, 6(7): 5449-5464). PD-L2 expression, in contrast, is more restricted and is expressed mainly by dendritic cells (Nakae et al, J Immunol 2006, 177:566-73). Ligation of PD-1 with its ligands PD-L1 and PD-L2 on T cells delivers a signal that inhibits IL-2 and IFN-γ production, as well as cell proliferation induced upon T cell receptor activation (Carter et al, Eur J Immunol 2002, 32(3):634-43; Freeman et al, J Exp Med 2000, 192(7): 1027-34). The mechanism involves recruitment of SHP-2 or SHP-1 phosphatases to inhibit T cell receptor signaling such as Syk and Lck phosphorylation (Sharpe et al, Nat Immunol 2007, 8, 239-245). Activation of the PD-1 signaling axis also attenuates PKC-θ activation loop phosphorylation, which is necessary for the activation of NF-κB and AP1 pathways, and for cytokine production such as IL-2, IFN-γ and TNF (Sharpe et al, Nat Immunol 2007, 8, 239-245; Carter et al, Eur J Immunol 2002, 32(3):634-43; Freeman et al, J Exp Med 2000, 192(7):1027-34).

Several lines of evidence from preclinical animal studies indicate that PD-1 and its ligands negatively regulate immune responses. PD-1-deficient mice have been shown to develop lupus-like glomerulonephritis and dilated cardiomyopathy (Nishimura et al, Immunity 1999, 11:141-151; Nishimura et al, Science 2001, 291:319-322). Using an LCMV model of chronic infection, it has been shown that PD-1/PD-L1 interaction inhibits activation, expansion and acquisition of effector functions of virus-specific CD8 T cells (Barber et al, Nature 2006, 439, 682-7). Together, these data support the development of a therapeutic approach to block the PD-1-mediated inhibitory signaling cascade in order to augment or "rescue" T cell response. Accordingly, there is a need for new compounds that block PD-1/PD-L1 protein/protein interaction.

The search between 2010 and 2015 for non-mAb inhibitors of PD-1, including small molecules, peptides, cyclo-peptides, and macrocycles is reviewed by Tryfon Zarganes-Tzitzikas et al. in Expert opinion on therapeutic patents, vol. 26, no. 9, 19 September 2016 pages 973-977, XP055394015.

An assay to identify modulators of the PD-1 :PD-L pathway and PD-1 :PD-L pathway modulators, e.g., compounds and pharmaceutical compositions thereof are discussed in WO 2011/082400.

### SUMMARY

The present invention provides a compound of Formula (I): 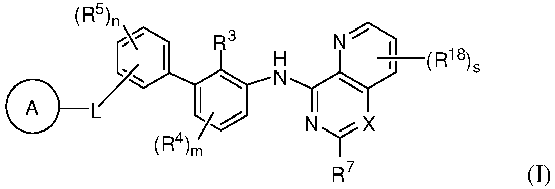 or a pharmaceutically acceptable salt thereof, wherein constituent variables are defined herein and in the claims.

The present invention further provides a pharmaceutical composition comprising a compound of the invention, or a pharmaceutically acceptable salt thereof, and one or more pharmaceutically acceptable excipients or carriers.

The present invention further provides compounds and compositions which may be used in methods of inhibiting PD-1/PD-L1 interaction, said method comprising administering to a patient a compound of the invention, or a pharmaceutically acceptable salt thereof.

The present invention further provides compounds of the invention, or a pharmaceutically acceptable salt thereof, and compositions of the invention, for use in methods of treating a disease or disorder associated with inhibition of PD-1/PD-L1 interaction wherein said disease or disorder is cancer or an infection.

The present invention further provides compounds of the invention, or a pharmaceutically acceptable salt thereof, and compositons of the invention for use in methods of enhancing, stimulating and/or increasing the immune response in a patient wherein the patient has cancer of an infection.

### DETAILED DESCRIPTION

### I. Compounds

This disclosure provides, *inter alia,* a compound of Formula (I): 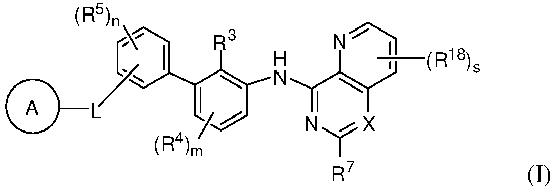 or a pharmaceutically acceptable salt or a stereoisomer thereof, wherein:
ring A is 5- to 14-membered heteroaryl, 4- to 14-membered heterocycloalkyl, C₆₋₁₀ aryl or C₃₋₁₄ cycloalkyl, wherein the 5- to 14-membered heteroaryl and 4- to 14-membered heterocycloalkyl each has 1-4 heteroatoms as ring members selected from B, P, N, O and S, wherein the P, N or S atom as ring members is optionally oxidized and one or more carbon atoms as ring members are each optionally replaced by a carbonyl group; and wherein ring A is optionally substituted with 1, 2, 3, 4 or 5 R⁶ substituents;
L is a bond, -C(O)NR¹³-, -NR¹³C(O)-, -C(=S)NR¹³-, -NR¹³C(=S)-, -C(=NR¹³)NR¹³-, - NR¹³C(=NR¹³)-, -C(=NOR¹³)NR¹³-, -NR¹³C(=NOR¹³)-, -C(=NCN)NR¹³-, -NR¹³C(=NCN)-, O, - (CR¹⁴R¹⁵)_{q}-, -(CR¹⁴R¹⁵)_{q}-O-_{,} -O(CR¹⁴R¹⁵)_{q}-_{,} -NR¹³-, -(CR¹⁴R¹⁵)_{q}-NR¹³-, -NR¹³-(CR¹⁴R¹⁵)_{q}-, - CH=CH-, -C≡C-, -SO₂NR¹³-, -NR¹³SO₂-, -NR¹³SO₂NR¹³-, -NR¹³C(O)O-, -OC(O)NR¹³ or-NR¹³C(O)NR¹³-;
X is N or CR¹⁷;
R³ is methyl, halo, CN or C₁₋₄ haloalkyl;
R⁴ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁵ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁶, R⁷, R¹⁷ and R¹⁸ are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-10 membered heterocycloalkyl)-C₁₋₄ alkyl-, CN, NO₂, OR^{a}, SR^{a}, NHOR^{a}, C(O)R^{a}, C(O)NR^{a}R^{a}, C(O)OR^{a}, C(O)NR^{a}S(O)₂R^{a}, OC(O)R^{a}, OC(O)NR^{a}R^{a}, NHR^{a}, NR^{a}R^{a}, NR^{a}C(O)R^{a}, NR^{a}C(=NR^{a})R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, S(O)2NR^{a}C(O)R^{a}, -P(O)R^{a}R^{a}, -P(O)(OR^{a})(OR^{a}), -B(OH)₂, -B(OR^{a})₂ and S(O)₂NR^{a}R^{a}, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, 3, 4 or 5 independently selected R^{b} substituents;
or two R⁶ substituents attached to the same ring carbon atom taken together with the ring carbon atom to which they are attached form spiro C₃₋₆ cycloalkyl or spiro 4- to 7-membered heterocycloalkyl, each of which is optionally substituted with 1, 2, or 3 independently selected R^{f} substituents;
each R¹³ is independently H, C₁₋₆ haloalkyl or C₁₋₆ alkyl optionally substituted with a substituent selected from C₁₋₄ alkyl, C₁₋₄ alkoxy, C₁₋₄ haloalkyl, C₁₋₄ haloalkoxy, CN, halo, OH, - COOH, NH₂, -NHC₁₋₄ alkyl and -N(C₁₋₄ alkyl)₂;
R¹⁴ and R¹⁵ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R¹⁴ or R¹⁵ are each optionally substituted with 1, 2, or 3 independently selected R^{q} substituents;
or R¹⁴ and R¹⁵ taken together with the carbon atom to which they are attached form 3-, 4-, 5- or 6-membered cycloalkyl or 3-, 4-, 5- or 6-membered heterocycloalkyl, each of which is optionally substituted with 1 or 2 independently selected R^{q} substituents;
each R^{a} is independently selected from H, CN, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-14 membered heteroaryl, 4-14 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, and (4-14 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-14 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-14 membered heterocycloalkyl)-C₁₋₄ alkyl- of R^{a} are each optionally substituted with 1, 2, 3, 4, or 5 independently selected R^{d} substituents;
each R^{d} is independently selected from C₁₋₆ alkyl, C₁₋₆ haloalkyl, halo, C₆₋₁₀ aryl, 5-14 membered heteroaryl, C₃₋₁₀ cycloalkyl, 4-14 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-14 membered heterocycloalkyl)-C₁₋₄ alkyl-, CN, NH₂, NHOR^{e}, OR^{e}, SR^{e}, C(O)R^{e}, C(O)NR^{e}R^{e}, C(O)OR^{e}, C(O)NR^{e}S(O)2R^{e}, OC(O)R^{e}, OC(O)NR^{e}R^{e}, NHR^{e}, NR^{e}R^{e}, NR^{e}C(O)R^{e}, NR^{e}C(=NR^{e})R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)2R^{e}, S(O)2NR^{e}C(O)R^{e}, NR^{e}S(O)2R^{e}, NR^{e}S(O)2NR^{e}R^{e}, -P(O)R^{e}R^{e}, -P(O)(OR^{e})(OR^{e}), -B(OH)2, -B(OR^{e})2 and S(O)2NR^{e}R^{e}, wherein the C₁₋₆ alkyl, C₁₋₆haloalkyl, C₆₋₁₀ aryl, 5-14 membered heteroaryl, C₃₋₁₀ cycloalkyl, 4-14 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, and (4-14 membered heterocycloalkyl)-C₁₋₄ alkyl- of R^{d} are each optionally substituted with 1, 2, or 3 independently selected R^{f} substituents;
each R^{e} is independently selected from H, C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{e} are each optionally substituted with 1, 2 or 3 independently selected R^{f} substituents;
each R^{b} substituent is independently selected from halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, OH, NH₂, NO₂, NHOR^{c}, OR^{c}, SR^{c}, C(O)R^{c}, C(O)NR^{c}R^{c}, C(O)OR^{c}, C(O)NR^{c}S(O)₂R^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(=NR^{c})R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c}, S(O)₂NRC(O)R^{c}, -P(O)R^{c}R^{c}, -P(O)(OR^{c})(OR^{c}), -B(OH)₂, -B(OR^{c})₂ and S(O)₂NR^{c}R^{c}; wherein the C₁₋₆ alkyl, C₁₋₆ haloalkyl, C₁₋₆ haloalkoxy, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-and (4-10 membered heterocycloalkyl)-C₁₋₄ alkyl- of R^{b} are each further optionally substituted with 1, 2, or 3 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{c} are each optionally substituted with 1, 2, 3, 4, or 5 independently selected R^{f} substituents;
each R^{f} is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{g}, OR^{g}, SR^{g}, C(O)R^{g}, C(O)NR^{g}R^{g}, C(O)OR^{g}, C(O)NR^{g}S(O)₂R^{g}, OC(O)R^{g}, OC(O)NR^{g}R^{g}, NHR^{g}, NR^{g}R^{g}, NR^{g}C(O)R^{g}, NR^{g}C(=NR^{g})R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, S(O)₂NR^{g}C(O)R^{g}, NR^{g}S(O)₂R^{g}, NR^{g}S(O)₂NR^{g}R^{g}, -P(O)R^{g}R^{g}, - P(O)(OR^{g})(OR^{g}), -B(OH)₂, -B(OR^{g})₂ and S(O)₂NR^{g}R^{g}; wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{f} are each optionally substituted with 1, 2, 3, 4, or 5 independently selected Rⁿ substituents;
each Rⁿ is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{o}, OR^{o}, SR°, C(O)R^{o}, C(O)NR^{o}R^{o}, C(O)OR^{o}, C(O)NR^{o}S(O)₂R^{o}, OC(O)R^{o}, OC(O)NR^{o}R^{o}, NHR°, NR^{o}R^{o}, NR^{o}C(O)R^{o}, NR^{o}C(=NR^{o})R^{o}, NR^{o}C(O)NR^{o}R^{o}, NR^{o}C(O)OR^{o}, C(=NR^{o})NR^{o}R^{o}, NR^{o}C(=NR^{o})NR^{o}R^{o}, S(O)R^{o}, S(O)NR^{o}R^{o}, S(O)₂R^{o}, S(O)₂NR^{o}C(O)R^{o}, NR^{o}S(O)₂R^{o}, NR^{o}S(O)₂NR^{o}R^{o}, -P(O)R^{o}R^{o}, - P(O)(OR^{o})(OR^{o}), -B(OH)₂, -B(OR^{o})₂ and S(O)₂NR^{o}R^{o}, wherein the C₁₋₄ alkyl, C₁₋₄haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of Rⁿ are each optionally substituted with 1, 2 or 3 independently selected R^{q} substituents;
each R^{g} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{g} are each optionally substituted with 1, 2, or 3 independently selected R^{p} substituents;
each R^{p} is independently selected from C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{r}, OR^{r}, SR^{r}, C(O)R^{r}, C(O)NR^{r}R^{r}, C(O)OR^{r}, C(O)NR^{r}S(O)₂R^{r}, OC(O)R^{r}, OC(O)NR^{r}R^{r}, NHR^{r}, NR^{r}R^{r}, NR^{r}C(O)R^{r}, NR^{r}C(=NR^{r})R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, S(O)₂NR^{r}C(O)R^{r}, NR^{r}S(O)₂R^{r}, NR^{r}S(O)₂NR^{r}R^{r}, -P(O)R^{r}R^{r}, -P(O)(OR^{r})(OR^{r}), -B(OH)₂, -B(OR^{r})₂ and S(O)₂NR^{r}R^{r}, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{p} is optionally substituted with 1, 2 or 3 independently selected R^{q} substituents;
or any two R^{a} substituents together with the boron, phosphorus or nitrogen atom to which they are attached form a 4-, 5-, 6-, 7-, 8-, 9- or 10-membered heterocycloalkyl group optionally substituted with 1, 2 or 3 independently selected R^{h} substituents;
each R^{h} is independently selected from C₁₋₆ alkyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl-, C₁₋₆ haloalkyl, C₁₋₆ haloalkoxy, C₂₋₆ alkenyl, C₂₋₆ alkynyl, halo, CN, ORⁱ, SRⁱ, NHORⁱ, C(O)Rⁱ, C(O)NRⁱRⁱ, C(O)ORⁱ, C(O)NRⁱS(O)₂Rⁱ, OC(O)Rⁱ, OC(O)NRⁱRⁱ, NHRⁱ, NRⁱRⁱ, NRⁱC(O)Rⁱ, NRⁱC(=NRⁱ)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, S(O)₂NRC(O)Rⁱ, NRⁱS(O)₂Rⁱ, NRⁱS(O)₂NRⁱRⁱ, -P(O)RⁱRⁱ, - P(O)(ORⁱ)(ORⁱ), -B(OH)₂, -B(ORⁱ)₂ and S(O)₂NRⁱRⁱ, wherein the C₁₋₆ alkyl, C₂₋₆ alkenyl, C₂₋₆ alkynyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl- of R^{h} are each further optionally substituted by 1, 2, or 3 independently selected R^{j} substituents;
each R^{j} is independently selected from C₃₋₆ cycloalkyl, C₆₋₁₀ aryl, 5 or 6-membered heteroaryl, 4-7 membered heterocycloalkyl, C₂₋₄ alkenyl, C₂₋₄ alkynyl, halo, C₁₋₄ alkyl, C₁₋₄ haloalkyl, C₁₋₄ haloalkoxy, CN, NHOR^{k}, OR^{k}, SR^{k}, C(O)R^{k}, C(O)NR^{k}R^{k}, C(O)O_{R}^{k}, C(O)NR^{k}S(O)₂R^{k}, OC(O)R^{k}, OC(O)NR^{k}R^{k}, NHR^{k}, NR^{k}R^{k}, NR^{k}C(O)R^{k}, NR^{k}C(=NR^{k})R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, S(O)₂NR^{k}C(O)R^{k}, NR^{k}S(O)₂R^{k}, NR^{k}S(O)₂NR^{k}R^{k}, -P(O)R^{k}R^{k}, -P(O)(OR^{k})(OR^{k}), - B(OH)₂, -B(OR^{k})₂ and S(O)₂NR^{k}R^{k}, wherein the C₁₋₄ alkyl, C₃₋₆ cycloalkyl, C₆₋₁₀ aryl, 5- or 6-membered heteroaryl, 4-7 membered heterocycloalkyl, C₂₋₄ alkenyl, C₂₋₄ alkynyl, C₁₋₄ haloalkyl and C₁₋₄ haloalkoxy of R^{j} are each optionally substituted with 1, 2 or 3 independently selected R^{q} substituents;
or two R^{h} groups attached to the same carbon atom of the 4- to 10-membered heterocycloalkyl taken together with the carbon atom to which they are attached form a C₃₋₆ cycloalkyl or 4- to 6-membered heterocycloalkyl having 1-2 heteroatoms as ring members selected from O, N or S;
or any two R^{c} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents;
or any two R^{e} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents;
or any two R^{g} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents;
or any two Rⁱ substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents, or 1, 2, or 3 independently selected R^{q} substituents;
or any two R^{k} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents, or 1, 2, or 3 independently selected R^{q} substituents;
or any two R^{o} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents;
or any two R^{r} substituents together with the boron, phosphorus or nitrogen atom to which they are attached form a 4-, 5-, 6-, 7-, 8-, 9- or 10-membered heterocycloalkyl group optionally substituted with 1, 2, or 3 independently selected R^{h} substituents;
each Rⁱ, R^{k}, R^{o} or R^{r} is independently selected from H, C₁₋₄ alkyl, C₃₋₆ cycloalkyl, C₆₋₁₀ aryl, 5 or 6-membered heteroaryl, 4-7 membered heterocycloalkyl, C₁₋₆ haloalkyl, C₁₋₆ haloalkoxy, C₂₋₄ alkenyl, and C₂₋₄ alkynyl, wherein the C₁₋₄ alkyl, C₃₋₆ cycloalkyl, C₆₋₁₀ aryl, 5 or 6-membered heteroaryl, 4-7 membered heterocycloalkyl, C₂₋₄ alkenyl, and C₂₋₄ alkynyl of Rⁱ, R^{k}, R° or R^{r} are each optionally substituted with 1, 2 or 3 R^{q} substituents;
each R^{q} is independently selected from halo, OH, CN, -COOH, NH₂, -NH-C₁₋₆ alkyl, - N(C₁₋₆ alky)₂, C₁₋₆ alkyl, C₁₋₆ alkoxy, C₁₋₆ alkylthio, C₁₋₆ haloalkyl, C₁₋₆ haloalkoxy, phenyl, 5-6 membered heteroaryl, 4-6 membered heterocycloalkyl and C₃₋₆ cycloalkyl, wherein the C₁₋₆ alkyl, phenyl, C₃₋₆ cycloalkyl, 4-6 membered heterocycloalkyl, and 5-6 membered heteroaryl of R^{q} are each optionally substituted with 1, 2, or 3 substituents selected from halo, OH, CN, -COOH, NH₂, C₁₋₄ alkyl, C₁₋₄ alkoxy, C₁₋₄ haloalkyl, C₁₋₄ haloalkoxy, phenyl, C₃₋₁₀ cycloalkyl, 5-6 membered heteroaryl and 4-6 membered heterocycloalkyl;
the subscript m is an integer of 0, 1, 2 or 3;
the subscript n is an integer of 0, 1, 2 or 3;
each subscript q is independently an integer of 1, 2, 3 or 4; and
the subscript s is an integer of 1, 2, or 3.

The present invention provides a compound of Formula (I), or a pharmaceutically acceptable salt thereof, wherein:
ring A is 5- to 10-membered heteroaryl, 4- to 11-membered heterocycloalkyl, C₆₋₁₀ aryl or C₃₋₁₀ cycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, 3, 4 or 5 R⁶ substituents;
L is a bond, -C(O)NR¹³-, -NR¹³C(O)-, O, -(CR¹⁴R¹⁵ )_{q}-, -(CR¹⁴R¹⁵)_{q}-O-, -O(CR¹⁴R¹⁵)_{q}-, - NR¹³-, -(CR¹⁴R¹⁵)_{q}-NR¹³-, -NR¹³-(CR¹⁴R¹⁵)_{q}-, -CH=CH-, -C≡C-, -SO₂NR¹³-, -NR¹³SO₂-, - NR¹³C(O)O- or -NR¹³C(O)NR¹³-;
X is N or CR¹⁷;
R³ is methyl, halo, CN or C₁₋₄ haloalkyl;
R⁴ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁵ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁶, R⁷, R¹⁷ and R¹⁸ are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-10 membered heterocycloalkyl)-C₁₋₄ alkyl-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)2R^{a}, NR^{a}S(O)₂NR^{a}R^{a}, S(O)R^{a}, S(O)NR^{a}R^{a}, S(O)2R^{a}, and S(O)₂NR^{a}R^{a}, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, 3, 4 or 5 R^{b} substituents;
or two R⁶ substituents attached to the same ring carbon atom taken together with the ring carbon atom to which they are attached form spiro C₃₋₆ cycloalkyl or spiro 4- to 7-membered heterocycloalkyl, each of which is optionally substituted with 1, 2, or 3 independently selected R^{f} substituents;
each R¹³ is independently H, C₁₋₆ haloalkyl or C₁₋₆ alkyl optionally substituted with a substituent selected from C₁₋₄ alkyl, C₁₋₄ alkoxy, C₁₋₄ haloalkyl, C₁₋₄ haloalkoxy, CN, halo, OH, - COOH, NH₂, -NHC₁₋₄ alkyl and -N(C₁₋₄ alkyl)₂;
R¹⁴ and R¹⁵ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R¹⁴ or R¹⁵ are each optionally substituted with 1, 2, or 3 independently selected R^{q} substituents;
or R¹⁴ and R¹⁵ taken together with the carbon atom to which they are attached form 3-, 4-, 5- or 6-membered cycloalkyl or 3-, 4-, 5- or 6-membered heterocycloalkyl, each of which is optionally substituted with 1 or 2 R^{q} substituents;
each R^{a} is independently selected from H, CN, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{a} are each optionally substituted with 1, 2, 3, 4, or 5 R^{d} substituents;
each R^{d} is independently selected from C₁₋₆ alkyl, C₁₋₆ haloalkyl, halo, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)2NR^{e}R^{e}, and S(O)₂NR^{e}R^{e}, wherein the C₁₋₆ alkyl, C₁₋₆ haloalkyl, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{d} are each optionally substituted with 1, 2, or 3 independently selected R^{f} substituents;
each R^{e} is independently selected from H, C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{e} are each optionally substituted with 1, 2 or 3 independently selected R^{f} substituents;
each R^{b} substituent is independently selected from halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, OH, NH₂, NO2, NHOR°, OR^{c}, SR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} and S(O)₂NR^{c}R^{c}; wherein the C₁₋₆ alkyl, C₁₋₆ haloalkyl, C₁₋₆ haloalkoxy, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-and (4-10 membered heterocycloalkyl)-C₁₋₄ alkyl- of R^{b} are each further optionally substituted with 1, 2, or 3 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{c} are each optionally substituted with 1, 2, 3, 4, or 5 R^{f} substituents;
each R^{f} is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g}, and S(O)₂NR^{g}R^{g}; wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{f} are each optionally substituted with 1, 2, 3, 4, or 5 Rⁿ substituents;
each Rⁿ is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{o}, OR^{o}, SR°, C(O)R^{o}, C(O)NR^{o}R^{o}, C(O)OR^{o}, OC(O)R^{o}, OC(O)NR^{o}R^{o}, NHR°, NR^{o}R^{o}, NR^{o}C(O)R^{o}, NR^{o}C(O)NR^{o}R^{o}, NR^{o}C(O)OR^{o}, C(=NR^{o})NR^{o}R^{o}, NR^{o}C(=NR^{o})NR^{o}R^{o}, S(O)R^{o}, S(O)NR^{o}R^{o}, S(O)₂R^{o}, NR^{o}S(O)₂R^{o}, NR^{o}S(O)₂NR^{o}R^{o}, and S(O)₂NR^{o}R^{o}, wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of Rⁿ are each optionally substituted with 1, 2 or 3 independently selected R^{q} substituents;
each R^{g} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{g} are each optionally substituted with 1, 2, or 3 R^{p} substituents;
each R^{p} is independently selected from C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} and S(O)₂NR^{r}R^{r}, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{p} is optionally substituted with 1, 2 or 3 R^{q} substituents;
or any two R^{a} substituents together with the nitrogen atom to which they are attached form a 4-, 5-, 6-, 7-, 8-, 9- or 10-membered heterocycloalkyl group optionally substituted with 1, 2 or 3 R^{h} substituents;
each R^{h} is independently selected from C₁₋₆ alkyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl-, C₁₋₆ haloalkyl, C₁₋₆ haloalkoxy, C₂₋₆ alkenyl, C₂₋₆ alkynyl, halo, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ, and S(O)₂NRⁱRⁱ, wherein the C₁₋₆ alkyl, C₂₋₆ alkenyl, C₂₋₆ alkynyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl- of R^{h} are each further optionally substituted by 1, 2, or 3 R^{j} substituents;
each R^{j} is independently selected from C₃₋₆ cycloalkyl, C₆₋₁₀ aryl, 5 or 6-membered heteroaryl, 4-7 membered heterocycloalkyl, C₂₋₄ alkenyl, C₂₋₄ alkynyl, halo, C₁₋₄ alkyl, C₁₋₄ haloalkyl, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k}, and S(O)₂NR^{k}R^{k}, wherein the C₁₋₄ alkyl, C₃₋₆ cycloalkyl, C₆₋₁₀ aryl, 5- or 6-membered heteroaryl, 4-6 membered heterocycloalkyl, C₂₋₄ alkenyl, C₂₋₄ alkynyl, C₁₋₄ haloalkyl, and C₁₋₄ haloalkoxy of R^{j} are each optionally substituted with 1, 2 or 3 independently selected R^{q} substituents;
or two R^{h} groups attached to the same carbon atom of the 4- to 10-membered heterocycloalkyl taken together with the carbon atom to which they are attached form a C₃₋₆ cycloalkyl or 4- to 6-membered heterocycloalkyl having 1-2 heteroatoms as ring members selected from O, N or S;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
or any two R^{e} substituents together with the nitrogen atom to which they are attached form a 4-, 5-, 6-, or 7-membered heterocycloalkyl group optionally substituted with 1, 2, or 3 independently selected R^{h} substituents;
or any two R^{g} substituents together with the nitrogen atom to which they are attached form a 4-, 5-, 6-, or 7-membered heterocycloalkyl group optionally substituted with 1, 2, or 3 independently selected R^{h} substituents;
or any two R^{o} substituents together with the nitrogen atom to which they are attached form a 4-, 5-, 6-, or 7-membered heterocycloalkyl group optionally substituted with 1, 2, or 3 independently selected R^{h} substituents;
or any two R^{r} substituents together with the nitrogen atom to which they are attached form a 4-, 5-, 6-, or 7-membered heterocycloalkyl group optionally substituted with 1, 2, or 3 independently selected R^{h} substituents;
each Rⁱ, R^{k}, R^{o} or R^{r} is independently selected from H, C₁₋₄ alkyl, C₃₋₆ cycloalkyl, C₆₋₁₀ aryl, 5 or 6-membered heteroaryl, 4-7 membered heterocycloalkyl, C₁₋₆ haloalkyl, C₁₋₆ haloalkoxy, C₂₋₄ alkenyl, and C₂₋₄ alkynyl, wherein the C₁₋₄ alkyl, C₃₋₆ cycloalkyl, C₆₋₁₀ aryl, 5 or 6-membered heteroaryl, 4-7 membered heterocycloalkyl, C₂₋₄ alkenyl, and C₂₋₄ alkynyl of Rⁱ, R^{k}, R^{o} or R^{r} are each optionally substituted with 1, 2 or 3 R^{q} substituents;
each R^{q} is independently selected from halo, OH, CN, -COOH, NH₂, -NH-C₁₋₆ alkyl, - N(C₁₋₆ alky)₂, C₁₋₆ alkyl, C₁₋₆ alkoxy, C₁₋₆ alkylthio, C₁₋₆ haloalkyl, C₁₋₆ haloalkoxy, phenyl, 5-6 membered heteroaryl, 4-6 membered heterocycloalkyl and C₃₋₆ cycloalkyl, wherein the C₁₋₆ alkyl, phenyl, C₃₋₆ cycloalkyl, 4-6 membered heterocycloalkyl, and 5-6 membered heteroaryl of R^{q} are each optionally substituted with 1, 2, or 3 substituents selected from halo, OH, CN, -COOH, NH2, C₁₋₄ alkyl, C₁₋₄ alkoxy, C₁₋₄ haloalkyl, C₁₋₄ haloalkoxy, phenyl, C₃₋₁₀ cycloalkyl, 5-6 membered heteroaryl and 4-6 membered heterocycloalkyl;
the subscript m is an integer of 0, 1, 2 or 3;
the subscript n is an integer of 0, 1, 2 or 3;
each subscript q is independently an integer of 1, 2, 3 or 4; and
the subscript s is an integer of 1, 2, or 3.

In some embodiments, any two Rⁱ substituents together with the nitrogen atom to which they are attached form a 4-, 5-, 6-, 7-, 8-, 9- or 10--membered heterocycloalkyl group optionally substituted with 1, 2, or 3 independently selected R^{q} substituents;
or any two R^{k} substituents together with the nitrogen atom to which they are attached form a 4-, 5-, 6-, 7-, 8-, 9- or 10-membered heterocycloalkyl group optionally substituted with 1, 2, or 3 independently selected R^{q} substituents.

In some embodiments, provided herein is a compound of Formula (I) or a pharmaceutically acceptable salt thereof, wherein (1) when L is -C(O)NH-, ring A is not 4,5,6,7-tetrahydro-1H-imidazo[4,5-c]pyridin-2-yl; (2) when L is a bond, ring A is not [1,2,4]triazolo[1,5-a]pyridin-2-yl; (3) when L is a bond, ring A is not 2-benzoxazolyl; and (4) when L is -C(O)NH-, ring A is not 2-pyridyl.

In some embodiments, provided herein is a compound of Formula (I) or a pharmaceutically acceptable salt thereof, wherein (1) when L is -C(O)NH-, ring A is not 4,5,6,7-tetrahydro-1H-imidazo[4,5-c]pyridin-2-yl; (2) when L is a bond, ring A is not [1,2,4]triazolo[1,5-a]pyridin-2-yl; (3) when L is a bond, ring A is not 2-benzoxazolyl; or (4) when L is -C(O)NH-, ring A is not 2-pyridyl.

In some embodiments, provided herein is a compound of Formula (Ia): 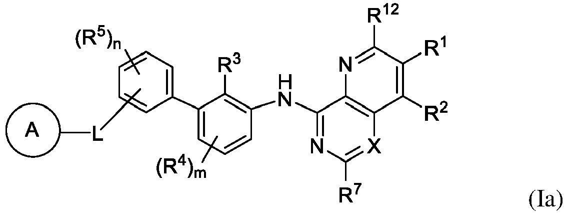 or a pharmaceutically acceptable salt thereof, wherein:
R¹⁷ is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, OH, -COOH,-C(O)NH₂, NH₂, -NHC₁₋₄ alkyl and -N(C₁₋₄ alkyl)₂;
R⁷ is H, C₁₋₄ alkyl, C₁₋₄ alkoxy, C₁₋₄ haloalkyl, C₁₋₄ haloalkoxy, CN, halo, OH, -COOH, NH2, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo or -C(O)NH₂;
R⁸ and R⁹ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R⁸ or R⁹ are each optionally substituted with 1, 2 or 3 independently selected R^{q} substituents;
or R⁸ and R⁹ taken together with the carbon atom to which they are attached form 3-, 4-, 5- or 6-membered cycloalkyl or 4-, 5-, 6- or 7-membered heterocycloalkyl, each of which is optionally substituted with 1 or 2 R^{q} substituents;
or R⁸ and R¹⁰ taken together with the atoms to which they are attached form 4-, 5-, 6- or 7-membered heterocycloalkyl, having zero to one additional heteroatoms as ring members selected from O, N or S, wherein the 4-, 5-, 6- or 7-membered heterocycloalkyl formed by R⁸ and R¹⁰ are each optionally substituted with 1 or 2 R^{q} substituents;
R¹⁰ and R¹¹ are each independently selected from H, C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, (4-10 membered heterocycloalkyl)-C₁₋₄ alkyl-, -C(O)R^{g}, -C(O)OR^{g}, -C(O)NR^{g}R^{g}, -SO₂R^{g} and -SO₂NR^{g}R^{g}, wherein the C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, and (4-10 membered heterocycloalkyl)-C₁₋₄ alkyl- of R¹⁰ or R¹¹ are each optionally substituted with 1, 2, or 3 independently selected R^{d} substituents;
or R¹⁰ and R¹¹ taken together with the nitrogen atom to which they are attached form 4-, 5-, 6-, 7-, 8-, 9-, 10-, or 11-membered heterocycloalkyl, wherein the 4-11membered heterocycloalkyl is each optionally substituted with 1, 2 or 3 R^{f} substituents;
   R¹² is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂; and
the subscript p is an integer of 1, 2, 3 or 4.

In some embodiments, provided herein is a compound of Formula (Ia), or a pharmaceutically acceptable salt thereof, wherein:
R¹⁷ is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, OH, -COOH,-C(O)NH₂, NH₂, -NHC₁₋₄ alkyl and -N(C₁₋₄ alkyl)₂;
R⁷ is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo or -C(O)NH₂;
R⁸ and R⁹ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R⁸ or R⁹ are each optionally substituted with 1, 2 or 3 independently selected R^{q} substituents;
or R⁸ and R⁹ taken together with the carbon atom to which they are attached form 3-, 4-, 5- or 6-membered cycloalkyl or 4-, 5-, 6- or 7-membered heterocycloalkyl, each of which is optionally substituted with 1 or 2 R^{q} substituents;
or R⁸ and R¹⁰ taken together with the atoms to which they are attached form 4-, 5-, 6- or 7-membered heterocycloalkyl, having zero to one additional heteroatoms as ring members selected from O, N or S, wherein the 4-, 5-, 6- or 7-membered heterocycloalkyl formed by R⁸ and R¹⁰ are each optionally substituted with 1 or 2 R^{q} substituents;
R¹⁰ and R¹¹ are each independently selected from H, C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, (4-10 membered heterocycloalkyl)-C₁₋₄ alkyl-, -C(O)R^{g}, -C(O)OR^{g}, -C(O)NR^{g}R^{g}, -SO₂R^{g} and -SO₂NR^{g}R^{g}, wherein the C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, and (4-10 membered heterocycloalkyl)-C₁₋₄ alkyl- of R¹⁰ or R¹¹ are each optionally substituted with 1, 2, or 3 independently selected R^{d} substituents;
or R¹⁰ and R¹¹ taken together with the nitrogen atom to which they are attached form 4-, 5-, 6-, 7-, 8-, 9-, 10-, or 11-membered heterocycloalkyl, wherein the 4-11membered heterocycloalkyl is each optionally substituted with 1, 2 or 3 R^{f} substituents; and R¹² is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂.

In some embodiments, the compound provided herein is a compound having Formula (II): 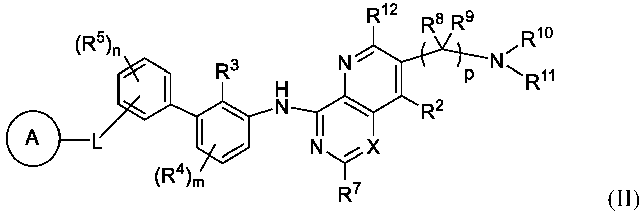 or a pharmaceutically acceptable salt thereof.

In some embodiments, the compound provided herein is a compound having Formula (IIa): 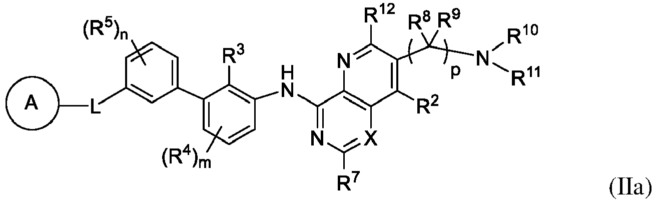 or a pharmaceutically acceptable salt thereof.

In some embodiments, the compound provided herein is a compound having Formula (IIa-1): 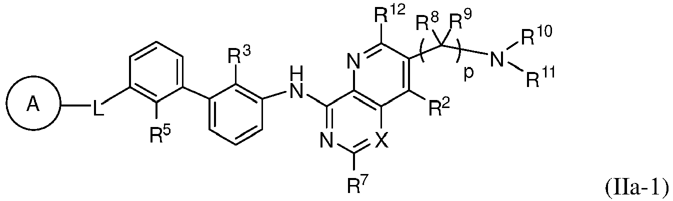 or a pharmaceutically acceptable salt thereof, wherein:
ring A is 5- to 10-membered heteroaryl, 4- to 11-membered heterocycloalkyl or C₆₋₁₀ aryl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;L is a bond, -C(O)NH-, -NH- or -OCH₂-, wherein the carbonyl group in the -C(O)NH- linkage or the oxygen atom in the -OCH₂- linkage is attached to ring A; and
X is CH or N.

In some embodiments, the compound provided herein is a compound having Formula (IIa-2): 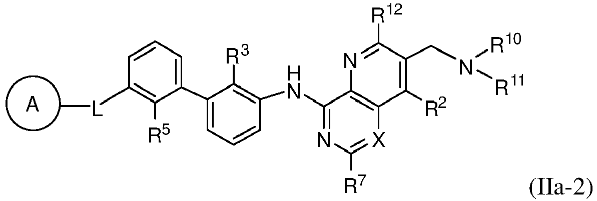 or a pharmaceutically acceptable salt thereof.

In some embodiments, the compound provided herein is a compound having Formula (IIb): 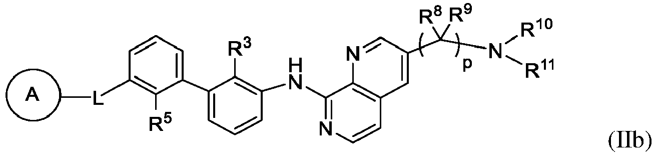 or a pharmaceutically acceptable salt thereof.

In some embodiments, the compound provided herein is a compound having Formula (IIc): 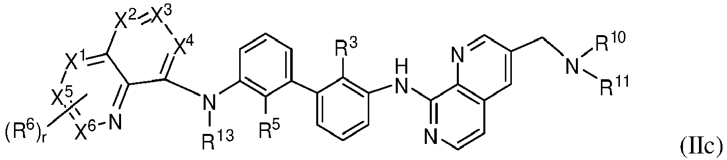 or a pharmaceutically acceptable salt thereof, wherein:
X¹, X², X³, X⁴, X⁵ and X⁶ are each independently N or CH, with the proviso that X¹, X⁵ and X⁶ are not simultaneously N;
R¹³ is H or C₁₋₄ alkyl; and
the subscript r is an integer of 1, 2 or 3.

In some embodiments, the compound provided herein is a compound having Formula (IIc-1): 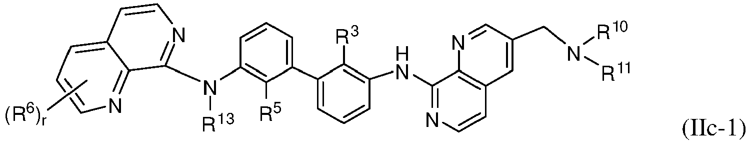 or a pharmaceutically acceptable salt thereof.

In some embodiments, R¹³ is H.

In some embodiments, the compound provided herein is a compound having Formula (IId): 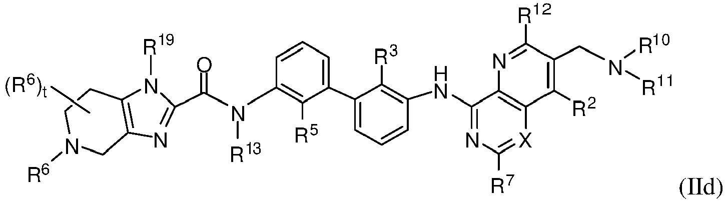 or a pharmaceutically acceptable salt thereof, wherein:
R¹³ is H or C₁₋₄ alkyl;
R¹⁹ is H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, or (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R¹⁸ are each optionally substituted with 1, 2, or 3 R^{b} substituents; and
the subscript t is an integer of 0, 1 or 2.

In some embodiments, the compound provided herein is a compound having Formula (IId-1): 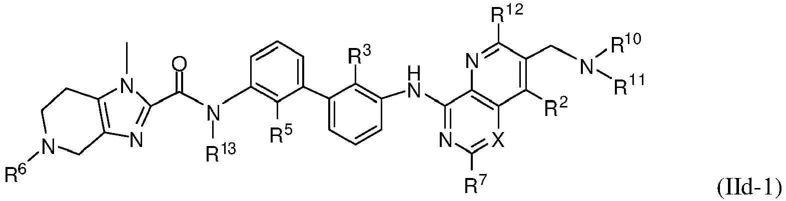 or a pharmaceutically acceptable salt thereof.

In some embodiments, the compound provided herein is a compound having Formula (IIe): 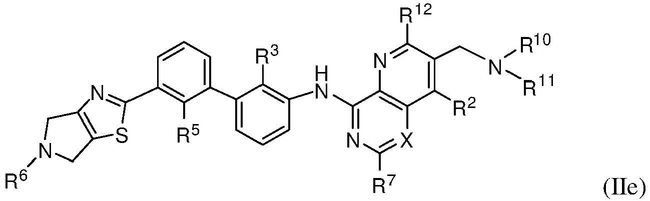 or a pharmaceutically acceptable salt thereof.

In some embodiments, the compound provided herein is a compound having Formula (IIf): 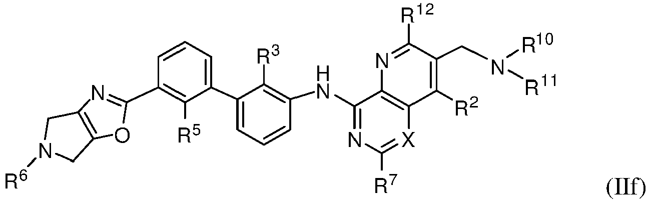 or a pharmaceutically acceptable salt thereof.

In some embodiments, the compound provided herein is a compound having Formula (III): 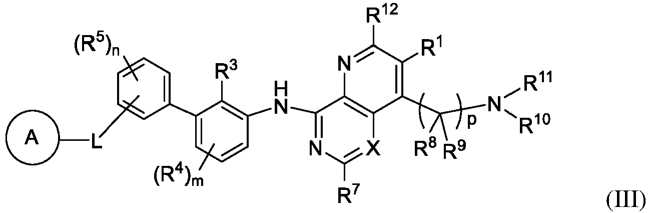 or a pharmaceutically acceptable salt thereof.

In some embodiments, the compound provided herein is a compound having Formula (IIIa): 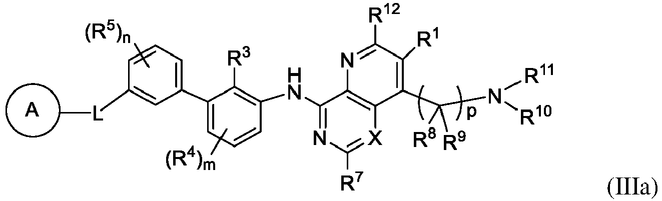 or a pharmaceutically acceptable salt thereof.

In some embodiments, the compound provided herein is a compound having Formula (IIIb): 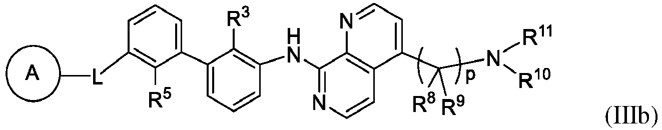 or a pharmaceutically acceptable salt thereof.

In some embodiments, provided herein are compounds having Formula (IV): 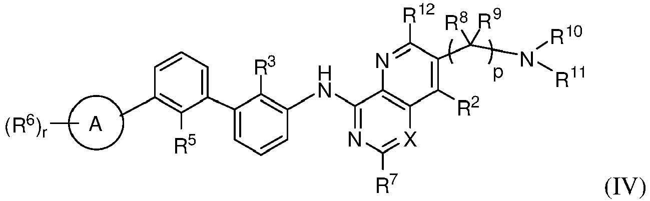 or a pharmaceutically acceptable salt thereof, wherein the subscript r is 1, 2, 3, 4 or 5. In some embodiments, X is N or CH. In one embodiment, ring A is pyridyl, for example, 2-pyridyl. In some embodiments, the subscript n is 0, 1 or 2 and each R⁵ is independently C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂. In certain instances, R⁵ is halo or C₁₋₄ alkyl. In some embodiments, the subscript m is 0. In some embodiments, the subscript r is 1 or 2. In some embodiments, R¹² is H, C₁₋₄ alkyl, C₁₋₄ alkoxy, C₁₋₄ haloalkyl, C₁₋₄ haloalkoxy, CN, halo, - COOH, NH₂, -NHC₁₋₄ alkyl or -N(C₁₋₄ alkyl)₂. In one embodiment, R² is H. In some embodiments, the subscript p is 1 and R⁸ and R⁹ are each H. In one embodiment, R¹⁰ is H. In some embodiments, R⁸ and R¹⁰ taken together form 4- to 6-membered heterocycloalkyl, optionally substituted with 1 or 2 R^{q} substituents. In some embodiments, R¹⁰ and R¹¹ taken together form 4- to 6-membered heterocycloalkyl, optionally substituted with 1 or 2 R^{q} substituents.

In some embodiments, provided herein are compounds having Formula (V): 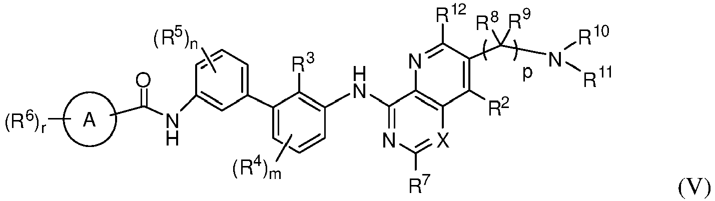 or a pharmaceutically acceptable salt thereof, wherein the subscript r is 1, 2, 3, 4 or 5, the other variables of Formula (V) are as defined in any embodiment disclosed herein. In some embodiments, the subscript r is 1 or 2.

In some embodiments, provided herein are compounds having Formula (VI): 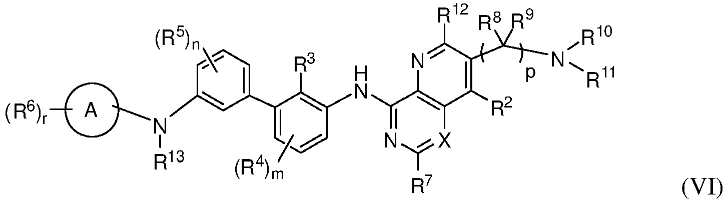 or a pharmaceutically acceptable salt thereof, wherein the subscript r is 1, 2, 3, 4 or 5, the other variables of Formula (VI) are as defined in any embodiment disclosed herein. In some embodiments, the subscript r is 1 or 2.

In some embodiments, provided herein are compounds having Formula (VIIa) or (VIIb): 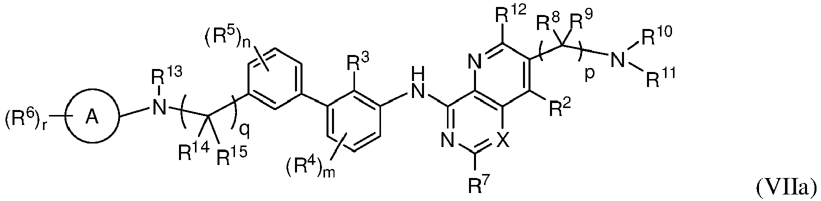 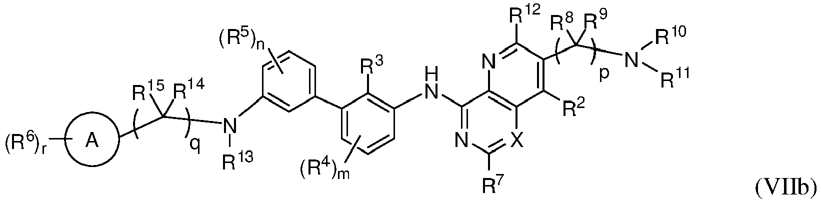 or a pharmaceutically acceptable salt thereof, wherein the subscript r is 1, 2, 3, 4 or 5, the other variables of Formula (VIIa) or (VIIb) are as defined in any embodiment disclosed herein. In some embodiments, the subscript r is 1 or 2.

In some embodiments, provided herein are compounds having Formula (VIIIa) or (VIIIb): 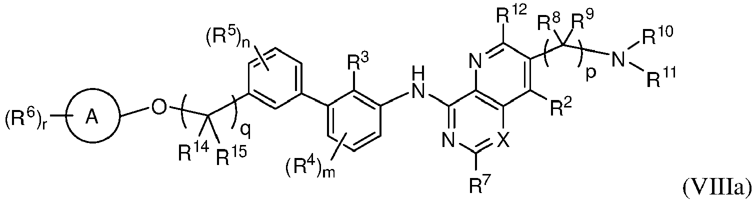 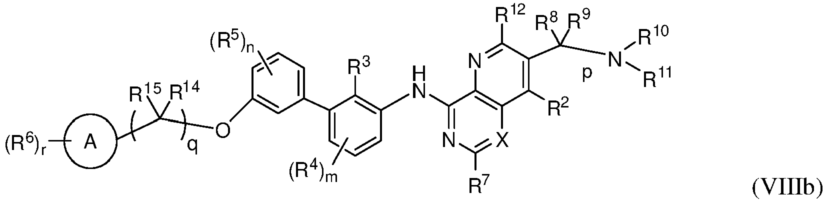 or a pharmaceutically acceptable salt thereof, wherein the subscript r is 1, 2, 3, 4 or 5, the other variables of Formula (VIIIa) or (VIIIb) are as defined in any embodiment disclosed herein. In some embodiments, the subscript r is 1 or 2.

In some embodiments, ring A is is 5- to 14-membered heteroaryl, 4- to 14-membered heterocycloalkyl or C₆₋₁₀ aryl, wherein the 5- to 14-membered heteroaryl and 4- to 14-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, 3, or 4 R⁶ substituents. In some embodiments, ring A is is 5- to 14-membered heteroaryl or 4- to 14-membered heterocycloalkyl, wherein the 5- to 14-membered heteroaryl and 4- to 14-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, or 3 R⁶ substituents. In some embodiments, ring A is is 5- to 14-membered heteroaryl, wherein the 5- to 14-membered heteroaryl has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, or 3 R⁶ substituents. In some embodiments, ring A is is 4- to 14-membered heterocycloalkyl, wherein the 4- to 14-membered heterocycloalkyl has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, or 3 R⁶ substituents.

In some embodiments, ring A is selected from: 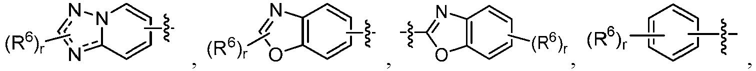 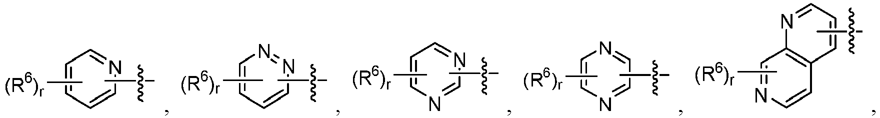 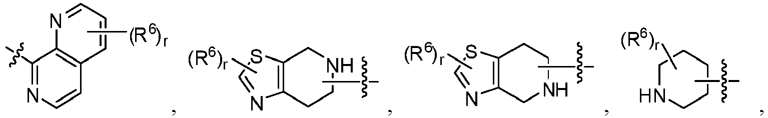 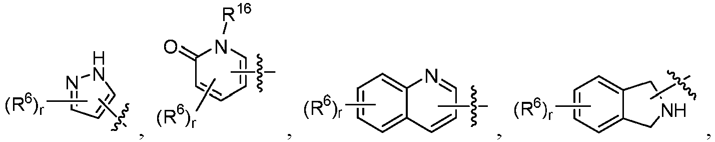 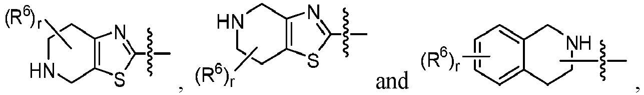 wherein each subscript r is an integer of 1, 2, 3, 4 or 5; R¹⁶ is C₁₋₆ alkyl; and the wavy line indicates the point of attachment to L.

In some embodiments, ring A is selected from: 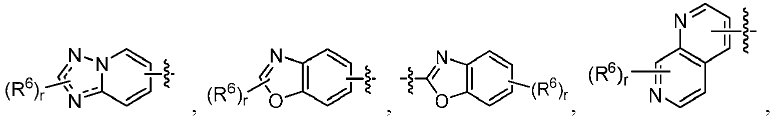 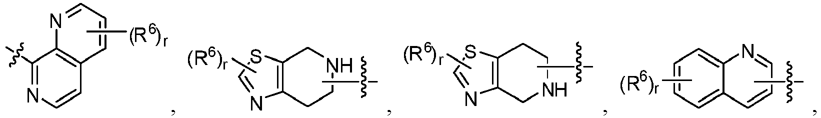 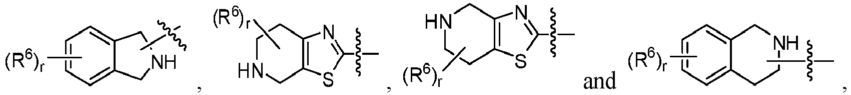 wherein each subscript r is an integer of 1, 2, 3, 4 or 5; and the wavy line indicates the point of attachment to L.

In some embodiments, ring A is selected from: 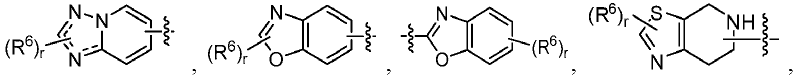 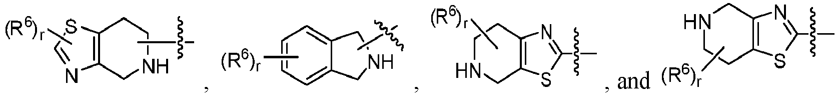 wherein each subscript r is an integer of 1, 2, 3, 4 or 5; and the wavy line indicates the point of attachment to L.

In some embodiments, ring A is selected from: 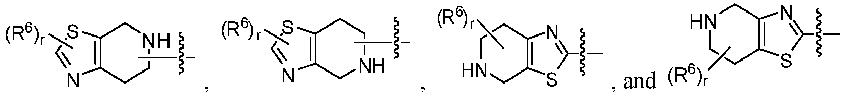 wherein each subscript r is an integer of 1, 2, 3, 4 or 5; and the wavy line indicates the point of attachment to L.

In some embodiments, ring A is 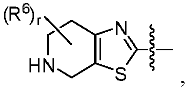 wherein each subscript r is an integer of 1, 2, 3, 4 or 5; and the wavy line indicates the point of attachment to L.

In some embodiments, ring A is selected from: 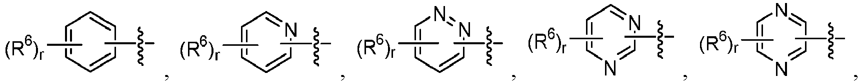 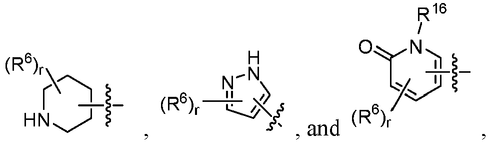 wherein each subscript r is an integer of 1, 2, 3, 4 or 5; R¹⁶ is C₁₋₆ alkyl; and the wavy line indicates the point of attachment to L.

In some embodiments, ring A is selected from: 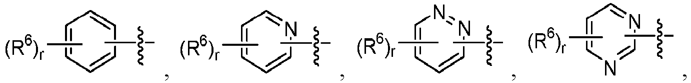 and 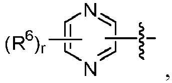 wherein each subscript r is an integer of 1, 2, 3, 4 or 5; and the wavy line indicates the point of attachment to L.

In some embodiments, ring A is 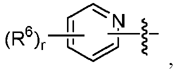 wherein each subscript r is an integer of 1, 2, 3, 4 or 5; and the wavy line indicates the point of attachment to L.

In some embodiments, ring A is 2-pyridyl, optionally substituted with 1, 2, 3, or 4 independently selected R⁶ substituents.

In some embodiments, ring A is 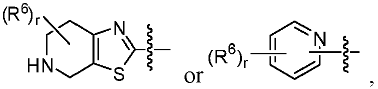 wherein each subscript r is an integer of 1, 2, 3, 4 or 5; and the wavy line indicates the point of attachment to L.

In some embodiments, ring A is selected from: 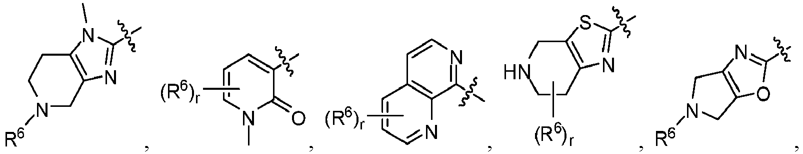 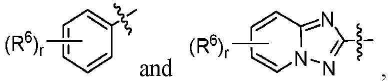 wherein each subscript r is an integer of 1, 2 or 3.

In some embodiments, ring A is selected from:

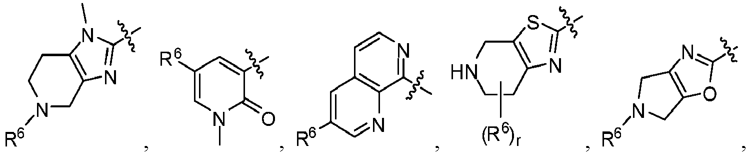

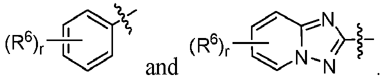

In some embodiments, L is a bond, -C(O)NR¹³-, -NR¹³C(O)-, -(CR¹⁴R¹⁵)_{q}-O-, - O(CR¹⁴R¹⁵)_{q}-, -NR¹³-, or -CH=CH-. In some embodiments, L is a bond, -C(O)NR¹³-, - NR¹³C(O)-, -(CR¹⁴R¹⁵)_{q}-O-, -O(CR¹⁴R¹⁵)_{q}-, or -NR¹³-. In some embodiments, L is -C(O)NR¹³-, -NR¹³C(O)-, -(CR¹⁴R¹⁵)_{q}-O-, -O(CR¹⁴R¹⁵)_{q}-, or -NR¹³-. In some embodiments, L is a bond, - C(O)NR¹³-, -NR¹³C(O)-, -NR¹³-, or -CH=CH-. In some embodiments, L is a bond, -NH-, - CH=CH- or -C(O)NH-, wherein the carbonyl group in the -C(O)NH- linkage is attached to ring A. In some embodiments, L is a bond. In some embodiments, L is -C(O)NR¹³- (e.g., -C(O)NH-), wherein the carbonyl group is attached to ring A. In some embodiments, L is a bond, -NR¹³-, - (CR¹⁴R¹⁵)_{q}O-, -O(CR¹⁴R¹⁵)_{q}-, -(CR¹⁴R¹⁵)_{q}NR¹³- or -NR¹³-(CR¹⁴R¹⁵)_{q}-, wherein the subscript q is 1, 2 or 3. In certain instances, R¹⁴ and R¹⁵ are each indepednently H or C₁₋₄ alkyl. In other instances, R¹⁴ and R¹⁵ taken together form C₃₋₆ cycloalkyl or 4- 6-membered heterocycloalkyl, each of which is optionally substituted with 1 or 2 R^{q} substituents.

In some embodiments, L is a bond.

In some embodiments, L is -NR¹³-. In certain instances, R¹³ is H or C₁₋₄ alkyl.

In some embodiments, L is -CH₂O- or -OCH₂-.

In some embodiments, L is -NR¹³CH₂- or -CH₂NR¹³. In certain instances, R¹³ is H or C₁₋₄ alkyl.

In some embodiments, L is -C(O)NH-.

In some embodiments, L is -NH-.

In some embodiments, the subscript m is 0, 1, or 2. In some embodiments, the subscript m is 0 or 1. In some embodiments, the subscript m is 0.

In some embodiments, R⁵ is C₁₋₄ alkyl, C₁₋₄ alkoxy, C₁₋₄ haloalkyl, C₁₋₄ haloalkoxy, CN, halo, or OH. In some embodiments, R⁵ is C₁₋₄ alkyl, C₁₋₄ alkoxy, CN, halo, or OH. In some embodiments, R⁵ is C₁₋₄ alkyl or halo. In some embodiments, R⁵ is C₁₋₄ alkyl (e.g., methyl). In some embodiments, R⁵ is halo (e.g., Cl).

In some embodiments, the subscript n is an integer of 0, 1, or 2. In some embodiments, the subscript n is an integer of 1 or 2. In some embodiments, the subscript n is an integer of 1.

In some embodiments, the subscript n is 1 and R⁵ is halo or C₁₋₄ alkyl. In some embodiments, the subscript n is 1 and R⁵ is halo. In some embodiments, the subscript n is 1 and R⁵ is C₁₋₄ alkyl.

In some embodiments, R³ is methyl, halo, or CN. In some embodiments, R³ is methyl. In some embodiments, R³ is halo (e.g., Cl). In some embodiments, R³ is CN. In some embodiments, R³ is methyl, CN or Cl.

In some embodiments, R¹² is H, C₁₋₄ alkyl, C₁₋₄ alkoxy, C₁₋₄ haloalkyl, C₁₋₄ haloalkoxy, CN, halo, or OH. In some embodiments, R¹² is H, halo, CN, C₁₋₄ alkyl or C₁₋₄ alkoxy. In some embodiments, R¹² is H, halo, or C₁₋₄ alkoxy. In some embodiments, R¹² is H.

In some embodiments, R⁷ is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy are each optionally substituted with 1 or 2 substituents independently selected from CN, halo and -C(O)NH₂. In some embodiments, R⁷ is H, halo, CN, C₁₋₄ alkyl, C₁₋₄ alkoxy or C₁₋₄ haloalkoxy, wherein the C₁₋₄ alkyl and C₁₋₄ alkoxy of R⁷ are each optionally substituted with CN. In some embodiments, R⁷ is H, halo, CN, or C₁₋₄ alkyl. In some embodiments, R⁷ is H or C₁₋₄ alkyl. In some embodiments, R⁷ is H.

In some embodiments, one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, and OH. In some embodiments, one of R¹ and R² is -(CR⁸R⁹)ₚ-NR¹⁰R¹¹ and the other is H, C₁₋₄ alkyl, C₁₋₄ alkoxy, C₁₋₄ haloalkyl, or C₁₋₄ haloalkoxy. In some embodiments, one of R¹ and R² is -(CR⁸R⁹)ₚ-NR¹⁰R¹¹ and the other is H or C₁₋₄ alkyl. In some embodiments, one of R¹ and R² is -(CR⁸R⁹)ₚ-NR¹⁰R¹¹ and the other is H.

In some embodiments, R² is H.

In some embodiments, R¹ is H.

In some embodiments, R², R⁷ and R¹² are each H.

In some embodiments, R³ and R⁵ are each independently halo, methyl or CN.

In some embodiments, the subscript p is an integer of 1, 2, or 3. In some embodiments, the subscript p is an integer of 1 or 2. In some embodiments, the subscript p is 1.

In some embodiments, R⁸ and R⁹ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, and C₁₋₄ haloalkoxy, wherein the C₁₋₄ alkyl, C₁₋₄ alkoxy, C₁₋₄ haloalkyl, and C₁₋₄ haloalkoxy of R⁸ or R⁹ are each optionally substituted with 1 or 2 independently selected R^{q} substituents. In some embodiments, R⁸ and R⁹ are each independently selected from H, halo, CN, C₁₋₄ alkyl, and C₁₋₄ alkoxy, wherein the C₁₋₄ alkyl and C₁₋₄ alkoxy of R⁸ or R⁹ are each optionally substituted with 1 or 2 independently selected R^{q} substituents. In some embodiments, R⁸ and R⁹ are each independently selected from H and C₁₋₄ alkyl. In some embodiments, R⁸ is H. In some embodiments, R⁹ is H.

In some embodiments, R⁸ and R⁹ are each H.

In some embodiments, R¹⁰ and R¹¹ are each independently selected from H, C₁₋₆ alkyl, and C₁₋₆ haloalkyl, wherein the C₁₋₆ alkyl and C₁₋₆ haloalkyl of R¹⁰ or R¹¹ are each optionally substituted with 1, 2, or 3 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents.

In some embodiments, R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents.

In some embodiments, R¹⁰ and R¹¹ are each independently selected from H and C₁₋₆ alkyl optionally substituted with 1 or 2 independently selected R^{f} substituents. In some embodiments, R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents.

In some embodiments, R¹⁰ is H.

In some embodiments, R¹¹ is 2-hydroxyethyl, [1-(hydroxymethyl)cyclopropyl]methyl, [1-(hydroxymethyl)cyclobutyl]methyl or 2-(dimethylamino)-2-oxo-ethyl.

In some embodiments, R¹¹ is 1-hydroxy-2-propyl, 2-carboxyethyl, or 2-hydroxycyclopentyl.

In some embodiments, -NR¹⁰R¹¹ is (2-hydroxyethyl)amino, 3-hydroxypyrrolidin-1-yl, (R)-3-hydroxypyrrolidin-1-yl, (S)- 3-hydroxypyrrolidin-1-yl, 3-carboxypyrrolidin-1-yl, (R)-3-carboxypyrrolidin-1-yl, (S)-3-carboxypyrrolidin-1-yl, 3-carboxyazetidin-1-yl, *(S)*-3-carboxyazetidin-1-yl, *(R)*-3-carboxyazetidin-1-yl, 2-carboxy-1-piperidinyl, (R)-2-carboxy-1-piperidinyl, (S)- 2-carboxy-1-piperidinyl, 2-oxooxazolidin-3-yl, [1-(hydroxymethyl)cyclopropyl]methylamino, [1-(hydroxymethyl)cyclobutyl]methylamino, [2-(dimethylamino)-2-oxo-ethyl]amino, 3-(dimethylaminocarbonyl)pyrrolidin-1-yl, (R)-3-(dimethylaminocarbonyl)pyrrolidin-1-yl, (S)-3-(dimethylaminocarbonyl)pyrrolidin-1-yl, 2-hydroxypropylamino, 2-hydroxy-2-methylpropylamino, or 3-methyl-3-carboxypyrrolidin-1-yl.

In some embodiments, -NR¹⁰R¹¹ is (2-hydroxyethyl)amino, 3-hydroxypyrrolidin-1-yl, 3-carboxypyrrolidin-1-yl, 3-carboxyazetidin-1-yl, *(S)*-3-carboxyazetidin-1-yl, *(R)*-3-carboxyazetidin-1-yl, 2-carboxy-1-piperidinyl, 2-oxooxazolidin-3-yl, [1-(hydroxymethyl)cyclopropyl]methylamino, [1-(hydroxymethyl)cyclobutyl]methylamino or [2-(dimethylamino)-2-oxo-ethyl]amino.

In some embodiments, -NR¹⁰R¹¹ is (2-hydroxyethyl)amino, 3-hydroxypyrrolidin-1-yl, 3-carboxypyrrolidin-1-yl, 3-carboxyazetidin-1-yl, *(S)*-3-carboxyazetidin-1-yl, *(R)*-3-carboxyazetidin-1-yl, 2-carboxy-1-piperidinyl, 2-oxooxazolidin-3-yl, [1-(hydroxymethyl)cyclopropyl]methylamino, [1-(hydroxymethyl)cyclobutyl]methylamino, [2-(dimethylamino)-2-oxo-ethyl]amino, 3-(dimethylaminocarbonyl)pyrrolidin-1-yl, 2-hydroxypropylamino, 2-hydroxy-2-methylpropylamino, or 3-methyl-3-carboxypyrrolidin-1-yl.

In some embodiments, -NR¹⁰R¹¹ is (2-hydroxyethyl)amino, 3-hydroxypyrrolidin-1-yl, 3-carboxypyrrolidin-1-yl, 3-carboxyazetidin-1-yl, 2-carboxy-1-piperidinyl, 2-oxooxazolidin-3-yl, [1-(hydroxymethyl)cyclopropyl]methylamino, [1-(hydroxymethyl)cyclobutyl]methylamino or [2-(dimethylamino)-2-oxo-ethyl]amino.

In some embodiments, -NR¹⁰R¹¹ is 1-pyrrolidinyl, (3-carboxy-3-methyl)pyrrolidin-1-yl, (R)-(3-carboxy-3-methyl)pyrrolidin-1-yl, (S)-(3-carboxy-3-methyl)pyrrolidin-1-yl, (1-hydroxy-2-propyl)amino, (R)-(1-hydroxy-2-propyl)amino, (S)-(1-hydroxy-2-propyl)amino, (3-hydroxy-3-methyl)pyrrolidin-1-yl, (R)-(3-hydroxy-3-methyl)pyrrolidin-1-yl, (S)-(3-hydroxy-3-methyl)pyrrolidin-1-yl, (2-hydroxycyclopentyl)amino, ((1R,2S)-2-hydroxycyclopentyl)amino, ((1R,2R)-2-hydroxycyclopentyl)amino, ((1S,2S)-2-hydroxycyclopentyl)amino, ((1S,2R)-2-hydroxycyclopentyl)amino, 2-carboxyethylamino, 3-(carboxymethyl)pyrrolidin-1-yl, or 5-carboxy-2-azabicyclo[2.2.1]heptan-2-yl.

In some embodiments, X is N or CR¹⁷, wherein R¹⁷ is H, C₁₋₄ alkyl, C₁₋₄ alkoxy, C₁₋₄ haloalkyl, C₁₋₄ haloalkoxy, CN, or halo. In some embodiments, X is N or CR¹⁷, wherein R¹⁷ is H or C₁₋₄ alkyl. In some embodiments, X is N or CH. In some embodiments, X is N. In some embodiments, X is CR¹⁷ (e.g., CH).

In some embodiments, R⁶, R⁷, R¹⁷ and R¹⁸ are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-10 membered heterocycloalkyl)-C₁₋₄ alkyl-, CN, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, and NR^{a}C(O)OR^{a}, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, or 3 independently selected R^{b} substituents.

In some embodiments, R⁶, R⁷, R¹⁷ and R¹⁸ are each independently selected from H, halo, C₁₋₆ alkyl, C₂₋₆ alkenyl, C₂₋₆ alkynyl, C₁₋₆ haloalkyl, C₁₋₆ haloalkoxy, 5-14 membered heteroaryl, 4-10 membered heterocycloalkyl, (5-14 membered heteroaryl)-C₁₋₄ alkyl-, (4-10 membered heterocycloalkyl)-C₁₋₄ alkyl-, CN, OR^{a}, C(O)R^{a}, C(O)NR^{a}R^{a}, C(O)OR^{a}, NHR^{a}, NR^{a}R^{a}, NR^{a}C(O)R^{a}, and NR^{a}C(O)OR^{a}, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, or 3 independently selected R^{b} substituents.

In some embodiments, R⁶, R⁷, R¹⁷ and R¹⁸ are each independently selected from H, halo, C₁₋₆ alkyl, (5-14 membered heteroaryl)-C₁₋₄ alkyl-, (4-10 membered heterocycloalkyl)-C₁₋₄ alkyl-, CN, OR^{a}, and C(O)R^{a}, wherein the C₁₋₆ alkyl, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, or 3 independently selected R^{b} substituents.

In some embodiments, R⁶ is H, C₁₋₆ alkyl, (3-carboxypyrrolidin-1-yl)methyl, (R)-(3-carboxypyrrolidin-1-yl)methyl, (S)-(3-carboxypyrrolidin-1-yl)methyl, (3-hydroxypyrrolidin-1-yl)methyl, (R)-(3-hydroxypyrrolidin-1-yl)methyl, (S)-(3-hydroxypyrrolidin-1-yl)methyl, (2-hydroxyethylamino)methyl, (2-hydroxy-2-methylpropylamino)methyl, 2-(dimethylamino)ethanoyl, 2-(3-carboxyazetidin-1-yl)ethanoyl, (R)-2-(3-carboxyazetidin-1-yl)ethanoyl, (S)-2-(3-carboxyazetidin-1-yl)ethanoyl, 2-(2-carboxypiperidin-1-yl)ethanoyl, (R)-2-(2-carboxypiperidin-1-yl)ethanoyl, (S)-2-(2-carboxypiperidin-1-yl)ethanoyl, 2-(3-carboxypyrrolidin-1-yl)ethanoyl, (S)-2-(3-carboxypyrrolidin-1-yl)ethanoyl, (R)-2-(3-carboxypyrrolidin-1-yl)ethanoyl, (5-cyanopyridin-3-yl)methoxy, halo or CN.

In some embodiments, R⁶ is (4-carboxycyclohexyl)methyl, trans-(4-carboxycyclohexyl)methyl, cis-(4-carboxycyclohexyl)methyl, 1-carboxy-2-propyl, (R)-1-carboxy-2-propyl, (S)-1-carboxy-2-propyl, (4-carboxy-4-methylcyclohexyl)methyl, 2-pyrrolidinyl, 2-(3-hydroxypyrrolidin-1-yl)acetyl, 2-((R)-3-hydroxypyrrolidin-1-yl)acetyl, 2-((S)-3-hydroxypyrrolidin-1-yl)acetyl, 2-(3-hydroxyazetidin-1-yl)acetyl, 2-((2-hydroxyethyl)(methyl)amino)acetyl, (4-carboxycyclohexyl)ethyl, 4-carboxycyclohexyl, 4-carboxy-4-methylcyclohexyl, dimethylglycyl, or N-ethyl-N-methylglycyl.

In some embodiments, each R^{a} is independently selected from H, CN, C₁₋₆ alkyl, C₁₋₄ haloalkyl, C₂₋₆ alkenyl, C₂₋₆ alkynyl, 5-14 membered heteroaryl, 4-14 membered heterocycloalkyl, (5-14 membered heteroaryl)-C₁₋₄ alkyl-, and (4-14 membered heterocycloalkyl)-C₁₋₄ alkyl-, wherein the C₁₋₆ alkyl, C₂₋₆ alkenyl, C₂₋₆ alkynyl, 5-14 membered heteroaryl, 4-14 membered heterocycloalkyl, (5-14 membered heteroaryl)-C₁₋₄ alkyl- and (4-14 membered heterocycloalkyl)-C₁₋₄ alkyl- of R^{a} are each optionally substituted with 1, 2, or 3 independently selected R^{d} substituents. In some embodiments, each R^{a} is independently selected from H, CN, C₁₋₆ alkyl, 5-14 membered heteroaryl, 4-14 membered heterocycloalkyl, (5-14 membered heteroaryl)-C₁₋₄ alkyl-, and (4-14 membered heterocycloalkyl)-C₁₋₄ alkyl-, wherein the C₁₋₆ alkyl, 5-14 membered heteroaryl, 4-14 membered heterocycloalkyl, (5-14 membered heteroaryl)-C₁₋₄ alkyl- and (4-14 membered heterocycloalkyl)-C₁₋₄ alkyl- of R^{a} are each optionally substituted with 1 or 2 independently selected R^{d} substituents. In some embodiments, each R^{a} is independently selected from H, CN, C₁₋₆ alkyl, (5-14 membered heteroaryl)-C₁₋₄ alkyl- , and (4-14 membered heterocycloalkyl)-C₁₋₄ alkyl-, wherein the C₁₋₆ alkyl, (5-14 membered heteroaryl)-C₁₋₄ alkyl- and (4-14 membered heterocycloalkyl)-C₁₋₄ alkyl- of R^{a} are each optionally substituted with 1 or 2 independently selected R^{d} substituents.

In some embodiments, each R^{d} is independently selected from C₁₋₆ alkyl, C₁₋₆ haloalkyl, halo, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, and NR^{e}C(O)R^{e}, wherein the C₁₋₆ alkyl of R^{d} are each optionally substituted with 1 or 2 independently selected R^{f} substituents. In some embodiments, each R^{d} is independently selected from C₁₋₆ alkyl, CN, NH₂, OR^{e}, C(O)R^{e}, C(O)NR^{e}R^{e}, C(O)OR^{e}, NHR^{e}, or NR^{e}R^{e}.

In some embodiments, each R^{e} is independently selected from H, C₁₋₆ alkyl, C₁₋₆ haloalkyl, C₂₋₆ alkenyl, and C₂₋₆ alkynyl. In some embodiments, each R^{e} is independently selected from H and C₁₋₆ alkyl.

In some embodiments, each R^{b} substituent is independently selected from halo, C₁₋₆ alkyl, C₂₋₆ alkenyl, C₂₋₆ alkynyl, C₁₋₆ haloalkyl, C₁₋₆haloalkoxy, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl- , CN, OH, NH₂, OR^{c}, C(O)R^{c}, C(O)NR^{c}R^{c}, C(O)OR^{c}, OC(O)R^{c}, OC(O)NR^{c}R^{c}, NHR^{c}, NR^{c}R^{c}, and NR^{c}C(O)R^{c}; wherein the C₁₋₆ alkyl, C₁₋₆ haloalkyl, C₁₋₆ haloalkoxy, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄alkyl-, C₃₋₁₀ cycloalkyl-C₁₋₄ alkyl-, (5-10 membered heteroaryl)-C₁₋₄ alkyl-and (4-10 membered heterocycloalkyl)-C₁₋₄ alkyl- of R^{b} are each further optionally substituted with 1 or 2 independently selected R^{d} substituents. In some embodiments, each R^{b} substituent is independently selected from halo, C₁₋₆ alkyl, C₂₋₆ alkenyl, C₂₋₆ alkynyl, C₁₋₆ haloalkyl, C₁₋₆ haloalkoxy, CN, OH, NH₂, OR^{c}, C(O)R^{c}, C(O)NR^{c}R^{c}, C(O)OR^{c}, NHR^{c}, and NR^{c}R^{c}; wherein the C₁₋₆ alkyl, C₁₋₆ haloalkyl, C₁₋₆ haloalkoxy, C₂₋₆ alkenyl, and C₂₋₆ alkynyl of R^{b} are each further optionally substituted with 1 or 2 independently selected R^{d} substituents. In some embodiments, each R^{b} substituent is independently selected from halo, C₁₋₆ alkyl, C₂₋₆ alkenyl, C₂₋₆ alkynyl, CN, OH, NH₂, OR^{c}, C(O)R^{c}, C(O)NR^{c}R^{c}, C(O)OR^{c}, NHR^{c}, and NR^{c}R^{c}; wherein the C₁₋₆ alkyl, C2-6 alkenyl, and C₂₋₆ alkynyl of R^{b} are each further optionally substituted with 1 or 2 independently selected R^{d} substituents.

In some embodiments, each R^{c} is independently selected from H, C₁₋₆ alkyl, C₁₋₄ haloalkyl, C₂₋₆ alkenyl, and C₂₋₆ alkynyl, wherein the C₁₋₆ alkyl, C₂₋₆ alkenyl, and C₂₋₆ alkynyl of R^{c} are each optionally substituted with 1 or 2 independently selected R^{f} substituents. In some embodiments, each R^{c} is independently selected from H and C₁₋₆ alkyl optionally substituted with 1 or 2 independently selected R^{f} substituents.

In some embodiments, each R^{f} is independently selected from C₁₋₄ alkyl, C₁₋₄ haloalkyl, C₂₋₆ alkenyl, C₂₋₆ alkynyl, halo, CN, OR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, and NR^{g}C(O)R^{g}; wherein the C₁₋₄ alkyl, C₁₋₄haloalkyl, C₂₋₆ alkenyl, and C₂₋₆ alkynyl of R^{f} are each optionally substituted with 1 or 2 independently selected Rⁿ substituents. In some embodiments, each R^{f} is independently selected from C₁₋₄ alkyl, halo, CN, OR^{g}, C(O)R^{g}, NHR^{g}, and NR^{g}R^{g}; wherein the C₁₋₄ alkyl is optionally substituted with 1 or 2 independently selected Rⁿ substituents. In some embodiments, each R^{f} is independently selected from C₁₋₄ alkyl, halo, and OR^{g}.

In some embodiments, each R^{g} is independently selected from H, C₁₋₆ alkyl, C₁₋₄ haloalkyl, C₂₋₆ alkenyl, and C₂₋₆ alkynyl, wherein the C₁₋₆ alkyl, C₂₋₆ alkenyl, and C₂₋₆ alkynyl of R^{g} are each optionally substituted with 1 or 2 independently selected R^{p} substituents. In some embodiments, each R^{g} is independently selected from H and C₁₋₆ alkyl.

In some embodiments, provided herein is a compound of Formula (I) or (Ia), or a pharmaceutically acceptable salt thereof, wherein:
ring A is 5- to 10-membered heteroaryl, 4- to 11-membered heterocycloalkyl, or C₆₋₁₀ aryl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³-, -NR¹³C(O)-, -(CR¹⁴R¹⁵)_{q}-O-, -O(CR¹⁴R¹⁵)_{q}-, -NR¹³-, or CH=CH-;
X is N or CR¹⁷, wherein R¹⁷ is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, and OH;
R³ is methyl, halo, CN or C₁₋₄ haloalkyl;
R⁴ is C₁₋₄ alkyl, C₁₋₄ alkoxy, or C₁₋₄ haloalkyl;
R⁵ is C₁₋₄ alkyl, C₁₋₄ alkoxy, C₁₋₄ haloalkyl, C₁₋₄ haloalkoxy, CN, halo, or OH;
each R⁶ is independently selected from H, halo, C₁₋₆ alkyl, C₂₋₆ alkenyl, C₂₋₆ alkynyl, C₁₋₆ haloalkyl, C₁₋₆ haloalkoxy, 5-14 membered heteroaryl, 4-10 membered heterocycloalkyl, (5-14 membered heteroaryl)-C₁₋₄ alkyl-, (4-10 membered heterocycloalkyl)-C₁₋₄ alkyl-, CN, NO₂, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, or NR^{a}C(O)OR^{a}, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H, C₁₋₄ alkyl, C₁₋₄ alkoxy, C₁₋₄ haloalkyl, C₁₋₄ haloalkoxy, CN, halo, or OH;
R⁸ and R⁹ are each independently selected from H, halo, CN, OH, -COOH, C₁₋₄ alkyl, C₁₋₄ alkoxy, -NHC₁₋₄ alkyl, -N(C₁₋₄alkyl)₂, and C₁₋₄ haloalkyl;
R¹⁰ and R¹¹ are each independently selected from H, C₁₋₆ alkyl, C₁₋₆ haloalkyl, -C(O)R^{g}, - C(O)OR^{g}, and -C(O)NR^{g}R^{g}, wherein the C₁₋₆ alkyl and C₁₋₆ haloalkyl of R¹⁰ or R¹¹ are each optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H, C₁₋₄ alkyl, C₁₋₄ alkoxy, C₁₋₄ haloalkyl, C₁₋₄ haloalkoxy, CN, halo, or OH;
each R¹³ is independently H, C₁₋₆ haloalkyl or C₁₋₆ alkyl;
R¹⁴ and R¹⁵ are each independently selected from H, halo, or C₁₋₄ alkyl;
each R^{a} is independently selected from H, CN, C₁₋₆ alkyl, C₁₋₄ haloalkyl, C₂₋₆ alkenyl, C₂₋₆ alkyny, (5-14 membered heteroaryl)-C₁₋₄ alkyl-, and (4-14 membered heterocycloalkyl)-C₁₋₄ alkyl-, wherein the C₁₋₆ alkyl, C₂₋₆ alkenyl, C₂₋₆ alkynyl, (5-14 membered heteroaryl)-C₁₋₄ alkyl-and (4-14 membered heterocycloalkyl)-C₁₋₄ alkyl- of R^{a} are each optionally substituted with 1, 2, 3 or independently selected R^{d} substituents;
each R^{d} is independently selected from C₁₋₆ alkyl, C₁₋₆ haloalkyl, halo, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, and NR^{e}C(O)R^{e};
each R^{e} is independently selected from H, C₁₋₆ alkyl, C₁₋₆ haloalkyl, C₂₋₆ alkenyl, and C₂₋₆ alkynyl;
each R^{b} substituent is independently selected from halo, C₁₋₆ alkyl, C₁₋₆ haloalkyl, C₁₋₆ haloalkoxy, CN, OH, NH₂, NO₂, OR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, and NR^{c}C(O)OR^{c}; wherein the C₁₋₄ alkyl, C₁₋₄ haloalkyl, and C₁₋₄ haloalkoxy of R^{b} are each further optionally substituted with 1 or 2 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl- of R^{c} are each optionally substituted with 1, 2, or 3 R^{f} substituents;
each R^{f} is independently selected from C₁₋₄ alkyl, C₁₋₄ haloalkyl, C₂₋₆ alkenyl, C₂₋₆ alkynyl, halo, CN, OR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, and NR^{g}C(O)OR^{g};
each R^{g} is independently selected from H, C₁₋₆ alkyl, C₁₋₄ haloalkyl, C₂₋₆ alkenyl, and C₂₋₆ alkynyl;
each R^{h} is independently selected from C₁₋₆ alkyl, C₁₋₆ haloalkyl, C₁₋₆ haloalkoxy, C₂₋₆ alkenyl, C₂₋₆ alkynyl, halo, CN, ORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, and NRⁱC(O)ORⁱ, wherein the C₁₋₆ alkyl, C₂₋₆ alkenyl, and C₂₋₆ alkynyl of R^{h} are each further optionally substituted by 1, 2, or 3 R^{j} substituents;
each R^{j} is independently selected from C₂₋₄ alkenyl, C₂₋₄ alkynyl, halo, C₁₋₄ alkyl, C₁₋₄ haloalkyl, and CN;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each Rⁱ is independently selected from H, C₁₋₄ alkyl, C₁₋₆ haloalkyl, C₁₋₆ haloalkoxy, C₂₋₄ alkenyl, and C₂₋₄ alkynyl;
the subscript m is an integer of 0, 1, or 2;
the subscript n is an integer of 0, 1, or 2; and
the subscript p is an integer of 1, 2, or 3.

In some embodiments, provided herein is a compound of Formula (I) or (Ia), or a pharmaceutically acceptable salt thereof, wherein:
ring A is 5- to 10-membered heteroaryl or 4- to 11-membered heterocycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³-, -NR¹³C(O)-, -NR¹³-, or CH=CH-;
X is N or CR¹⁷, wherein R¹⁷ is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, and OH;
R³ is methyl, halo, CN or C₁₋₄ haloalkyl;
R⁴ is C₁₋₄ alkyl, C₁₋₄ alkoxy, or C₁₋₄ haloalkyl;
R⁵ is C₁₋₄ alkyl, C₁₋₄ alkoxy, C₁₋₄ haloalkyl, C₁₋₄ haloalkoxy, CN, halo, or OH;
each R⁶ is independently selected from H, halo, C₁₋₆ alkyl, C₂₋₆ alkenyl, C₂₋₆ alkynyl, C₁₋₆ haloalkyl, C₁₋₆ haloalkoxy, 5-14 membered heteroaryl, 4-10 membered heterocycloalkyl, (5-14 membered heteroaryl)-C₁₋₄ alkyl-, (4-10 membered heterocycloalkyl)-C₁₋₄ alkyl-, CN, NO₂, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, or NR^{a}C(O)OR^{a}, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H, C₁₋₄ alkyl, C₁₋₄ alkoxy, C₁₋₄ haloalkyl, C₁₋₄ haloalkoxy, CN, halo, or OH;
R⁸ and R⁹ are each independently selected from H, halo, CN, OH, -COOH, C₁₋₄ alkyl, C₁₋₄ alkoxy, -NHC₁₋₄ alkyl, -N(C₁₋₄ alkyl)2, and C₁₋₄ haloalkyl;
R¹⁰ and R¹¹ are each independently selected from H, C₁₋₆ alkyl, C₁₋₆ haloalkyl, -C(O)R^{g}, - C(O)OR^{g}, and -C(O)NR^{g}R^{g}, wherein the C₁₋₆ alkyl and C₁₋₆haloalkyl of R¹⁰ or R¹¹ are each optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H, C₁₋₄ alkyl, C₁₋₄ alkoxy, C₁₋₄ haloalkyl, C₁₋₄ haloalkoxy, CN, halo, or OH;
each R¹³ is independently H, C₁₋₆ haloalkyl or C₁₋₆ alkyl;
each R^{a} is independently selected from H, CN, C₁₋₆ alkyl, C₁₋₄ haloalkyl, C₂₋₆ alkenyl, and C₂₋₆ alkynyl;
each R^{d} is independently selected from C₁₋₆ alkyl, C₁₋₆ haloalkyl, halo, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, and NR^{e}C(O)R^{e};
each R^{e} is independently selected from H, C₁₋₆ alkyl, C₁₋₆ haloalkyl, C₂₋₆ alkenyl, and C₂₋₆ alkynyl;
each R^{b} substituent is independently selected from halo, C₁₋₆ alkyl, C₁₋₆ haloalkyl, C₁₋₆ haloalkoxy, CN, OH, NH₂, NO2, OR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, and NR^{c}C(O)OR^{c}; wherein the C₁₋₄ alkyl, C₁₋₄ haloalkyl, and C₁₋₄ haloalkoxy of R^{b} are each further optionally substituted with 1 or 2 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl- of R^{c} are each optionally substituted with 1, 2, or 3 R^{f} substituents;
each R^{f} is independently selected from C₁₋₄ alkyl, C₁₋₄ haloalkyl, C₂₋₆ alkenyl, C₂₋₆ alkynyl, halo, CN, OR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, and NR^{g}C(O)OR^{g};
each R^{g} is independently selected from H, C₁₋₆ alkyl, C₁₋₄ haloalkyl, C₂₋₆ alkenyl, and C₂₋₆ alkynyl;
each R^{h} is independently selected from C₁₋₆ alkyl, C₁₋₆ haloalkyl, C₁₋₆ haloalkoxy, C₂₋₆ alkenyl, C₂₋₆ alkynyl, halo, CN, ORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, and NRⁱC(O)ORⁱ, wherein the C₁₋₆ alkyl, C₂₋₆ alkenyl, and C₂₋₆ alkynyl of R^{h} are each further optionally substituted by 1, 2, or 3 R^{j} substituents;
each R^{j} is independently selected from C₂₋₄ alkenyl, C₂₋₄ alkynyl, halo, C₁₋₄ alkyl, C₁₋₄ haloalkyl, and CN;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each R¹ is independently selected from H, C₁₋₄ alkyl, C₁₋₆ haloalkyl, C₁₋₆ haloalkoxy, C₂₋₄ alkenyl, and C₂₋₄ alkynyl;
the subscript m is an integer of 0, 1, or 2;
the subscript n is an integer of 0, 1, or 2; and
the subscript p is an integer of 1, 2, or 3.

In some embodiments provided herein is a compound of Formula (I) or (Ia), or a pharmaceutically acceptable salt thereof, wherein:
ring A is 5- to 10-membered heteroaryl or 4- to 11-membered heterocycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³- or -NR¹³C(O)-;
X is CR¹⁷, wherein R¹⁷ is H or C₁₋₄ alkyl;
one of R¹ and R² is -(CR⁸R⁹)ₚ-NR¹⁰R¹¹ and the other is H, C₁₋₄ alkyl, or C₁₋₄ alkoxy;
R³ is methyl, or halo;
R⁴ is C₁₋₄ alkyl or C₁₋₄ alkoxy;
R⁵ is C₁₋₄ alkyl, C₁₋₄ alkoxy, or halo;
each R⁶ is independently selected from H, halo, C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H or C₁₋₄ alkyl;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H or C₁₋₄ alkyl;
each R¹³ is independently H or C₁₋₆ alkyl;
each R^{b} substituent is independently selected from halo, C₁₋₆ alkyl, OH, NH₂, C(O)OR^{c}, NHR^{c}, and NR^{c}R^{c};
each R^{c} is independently selected from H, C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl-, wherein the C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl- of R^{c} are each optionally substituted with 1 or 2 R^{f} substituents;
each R^{f} is independently selected from C₁₋₄ alkyl, C₂₋₆ alkenyl, C₂₋₆ alkynyl, halo, OR^{g}, and C(O)OR^{g};
each R^{g} is independently selected from H and C₁₋₆ alkyl;
each R^{h} is independently selected from C₁₋₆ alkyl, C₂₋₆ alkenyl, C₂₋₆ alkynyl, halo, CN, ORⁱ, and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each Rⁱ is independently selected from H and C₁₋₄ alkyl;
the subscript m is an integer of 0 or 1;
the subscript n is an integer of 0 or 1; and
the subscript p is an integer of 1 or 2.

In some embodiments provided herein is a compound of Formula (I) or (Ia), or a pharmaceutically acceptable salt thereof, wherein:
ring A is 5- to 10-membered heteroaryl, wherein the 5- to 10-membered heteroaryl has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1 or 2 R⁶ substituents;
L is a bond, -C(O)NR¹³- or -NR¹³C(O)-;
X is CR¹⁷, wherein R¹⁷ is H;
one of R¹ and R² is -(CR⁸R⁹)ₚ-NR¹⁰R¹¹ and the other is H;
R³ is methyl, or halo;
R⁴ is C₁₋₄ alkyl or C₁₋₄ alkoxy;
R⁵ is C₁₋₄ alkyl or halo;
each R⁶ is independently selected from H, C₁₋₆ alkyl, and (4-10 membered heterocycloalkyl)-C₁₋₄ alkyl-, wherein the C₁₋₆ alkyl and (4-10 membered heterocycloalkyl)-C₁₋₄ alkyl- of R⁶ are each optionally substituted with 1 or 2 R^{b} substituents;
R⁷ is H;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H;
R¹³ is H;
each R^{b} substituent is independently selected from OH, C(O)OR^{c}, NHR^{c}, and NR^{c}R^{c};
each R^{c} is independently selected from H, C₁₋₆ alkyl, and C₃₋₁₀ cycloalkyl, wherein the C₁₋₆ alkyl, and C₃₋₁₀ cycloalkyl of R^{c} are each optionally substituted with 1 or 2 R^{f} substituents;
each R^{f} is independently selected from OR^{g}, and C(O)OR^{g};
R^{g} is H;
each R^{h} is independently selected from ORⁱ and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
Rⁱ is H;
the subscript m is an integer of 0 or 1;
the subscript n is an integer of 0 or 1; and
the subscript p is an integer of 1 or 2.

In some embodiments provided herein is a compound of Formula (I) or (Ia), or a pharmaceutically acceptable salt thereof, wherein:
ring A is 5- to 10-membered heteroaryl or 4- to 11-membered heterocycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³-, -NR¹³-, or -NR¹³C(O)-;
X is CR¹⁷, wherein R¹⁷ is H or C₁₋₄ alkyl;
one of R¹ and R² is -(CR⁸R⁹)ₚ-NR¹⁰R¹¹ and the other is H, C₁₋₄ alkyl, or C₁₋₄ alkoxy;
R³ is methyl, or halo;
R⁴ is C₁₋₄ alkyl or C₁₋₄ alkoxy;
R⁵ is C₁₋₄ alkyl, C₁₋₄ alkoxy, or halo;
each R⁶ is independently selected from H, halo, C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H or C₁₋₄ alkyl;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H or C₁₋₄ alkyl;
each R¹³ is independently H or C₁₋₆ alkyl;
each R^{b} substituent is independently selected from halo, C₁₋₆ alkyl, OH, NH₂, C(O)OR^{c}, NHR^{c}, and NR^{c}R^{c};
each R^{c} is independently selected from H, C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl-, wherein the C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl- of R^{c} are each optionally substituted with 1 or 2 R^{f} substituents;
each R^{f} is independently selected from C₁₋₄ alkyl, C₂₋₆ alkenyl, C₂₋₆ alkynyl, halo, OR^{g}, and C(O)OR^{g};
each R^{g} is independently selected from H and C₁₋₆ alkyl;
each R^{h} is independently selected from C₁₋₆ alkyl, C₂₋₆ alkenyl, C₂₋₆ alkynyl, halo, CN, ORⁱ, and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each Rⁱ is independently selected from H and C₁₋₄ alkyl;
the subscript m is an integer of 0 or 1;
the subscript n is an integer of 0 or 1; and
the subscript p is an integer of 1 or 2.

In some embodiments provided herein is a compound of Formula (I) or (Ia), or a pharmaceutically acceptable salt thereof, wherein:
ring A is 5- to 10-membered heteroaryl, wherein the 5- to 10-membered heteroaryl has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1 or 2 R⁶ substituents;
L is a bond, -C(O)NR¹³-, -NR¹³-, or -NR¹³C(O)-;
X is CR¹⁷, wherein R¹⁷ is H;
one of R¹ and R² is -(CR⁸R⁹)ₚ-NR¹⁰R¹¹ and the other is H;
R³ is methyl, or halo;
R⁴ is C₁₋₄ alkyl or C₁₋₄ alkoxy;
R⁵ is C₁₋₄ alkyl or halo;
each R⁶ is independently selected from H, C₁₋₆ alkyl, and (4-10 membered heterocycloalkyl)-C₁₋₄ alkyl-, wherein the C₁₋₆ alkyl and (4-10 membered heterocycloalkyl)-C₁₋₄ alkyl- of R⁶ are each optionally substituted with 1 or 2 R^{b} substituents;
R⁷ is H;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H;
R¹³ is H;
each R^{b} substituent is independently selected from OH, C(O)OR^{c}, NHR^{c}, and NR^{c}R^{c};
each R^{c} is independently selected from H, C₁₋₆ alkyl, and C₃₋₁₀ cycloalkyl, wherein the C₁₋₆ alkyl, and C₃₋₁₀ cycloalkyl of R^{c} are each optionally substituted with 1 or 2 R^{f} substituents;
each R^{f} is independently selected from OR^{g}, and C(O)OR^{g};
R^{g} is H;
each R^{h} is independently selected from ORⁱ and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
Rⁱ is H;
the subscript m is an integer of 0 or 1;
the subscript n is an integer of 0 or 1; and
the subscript p is an integer of 1 or 2.

It is further appreciated that certain features of the invention, which are, for clarity, described in the context of separate embodiments, can also be provided in combination in a single embodiment (while the embodiments are intended to be combined as if written in multiply dependent form). Conversely, various features of the invention which are, for brevity, described in the context of a single embodiment, can also be provided separately or in any suitable subcombination. Thus, it is contemplated as features described as embodiments of the compounds of Formula (I) can be combined in any suitable combination.

At various places in the present specification, certain features of the compounds are disclosed in groups or in ranges. It is specifically intended that such a disclosure include each and every individual subcombination of the members of such groups and ranges. For example, the term "C₁₋₆ alkyl" is specifically intended to individually disclose (without limitation) methyl, ethyl, C₃ alkyl, C₄ alkyl, C₅ alkyl and C₆ alkyl.

The term "n-membered," where n is an integer, typically describes the number of ring-forming atoms in a moiety where the number of ring-forming atoms is n. For example, piperidinyl is an example of a 6-membered heterocycloalkyl ring, pyrazolyl is an example of a 5-membered heteroaryl ring, pyridyl is an example of a 6-membered heteroaryl ring and 1,2,3,4-tetrahydro-naphthalene is an example of a 10-membered cycloalkyl group.

At various places in the present specification, variables defining divalent linking groups may be described. It is specifically intended that each linking substituent include both the forward and backward forms of the linking substituent. For example, -NR(CR'R")ₙ-includes both -NR(CR'R")ₙ- and -(CR'R")ₙNR- and is intended to disclose each of the forms individually. Where the structure requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "substituted" means that an atom or group of atoms formally replaces hydrogen as a "substituent" attached to another group. The term "substituted", unless otherwise indicated, refers to any level of substitution, e.g., mono-, di-, tri-, tetra- or penta-substitution, where such substitution is permitted. The substituents are independently selected, and substitution may be at any chemically accessible position. It is to be understood that substitution at a given atom is limited by valency. It is to be understood that substitution at a given atom results in a chemically stable molecule. The phrase "optionally substituted" means unsubstituted or substituted. The term "substituted" means that a hydrogen atom is removed and replaced by a substituent. A single divalent substituent, e.g., oxo, can replace two hydrogen atoms.

The term "Cₙ₋ₘ" indicates a range which includes the endpoints, wherein n and m are integers and indicate the number of carbons. Examples include C₁₋₄, C₁₋₆ and the like.

The term "alkyl," employed alone or in combination with other terms, refers to a saturated hydrocarbon group that may be straight-chained or branched. The term "Cₙ₋ₘ alkyl," refers to an alkyl group having n to m carbon atoms. An alkyl group formally corresponds to an alkane with one C-H bond replaced by the point of attachment of the alkyl group to the remainder of the compound. In some embodiments, the alkyl group contains from 1 to 6 carbon atoms, from 1 to 4 carbon atoms, from 1 to 3 carbon atoms, or 1 to 2 carbon atoms. Examples of alkyl moieties include, but are not limited to, chemical groups such as methyl, ethyl, *n*-propyl, isopropyl, *n*-butyl, *tert*-butyl, isobutyl, *sec*-butyl; higher homologs such as 2-methyl-1-butyl, *n*-pentyl, 3-pentyl, n-hexyl, 1,2,2-trimethylpropyl and the like.

The term "alkenyl," employed alone or in combination with other terms, refers to a straight-chain or branched hydrocarbon group corresponding to an alkyl group having one or more double carbon-carbon bonds. An alkenyl group formally corresponds to an alkene with one C-H bond replaced by the point of attachment of the alkenyl group to the remainder of the compound. The term "Cₙ₋ₘ alkenyl" refers to an alkenyl group having n to m carbons. In some embodiments, the alkenyl moiety contains 2 to 6, 2 to 4, or 2 to 3 carbon atoms. Example alkenyl groups include, but are not limited to, ethenyl, *n*-propenyl, isopropenyl, n-butenyl, *sec*-butenyl and the like.

The term "alkynyl," employed alone or in combination with other terms, refers to a straight-chain or branched hydrocarbon group corresponding to an alkyl group having one or more triple carbon-carbon bonds. An alkynyl group formally corresponds to an alkyne with one C-H bond replaced by the point of attachment of the alkyl group to the remainder of the compound. The term "Cₙ₋ₘ alkynyl" refers to an alkynyl group having n to m carbons. Example alkynyl groups include, but are not limited to, ethynyl, propyn-1-yl, propyn-2-yl and the like. In some embodiments, the alkynyl moiety contains 2 to 6, 2 to 4, or 2 to 3 carbon atoms.

The term "alkylene," employed alone or in combination with other terms, refers to a divalent alkyl linking group. An alkylene group formally corresponds to an alkane with two C-H bond replaced by points of attachment of the alkylene group to the remainder of the compound. The term "Cₙ₋ₘ alkylene" refers to an alkylene group having n to m carbon atoms. Examples of alkylene groups include, but are not limited to, ethan-1,2-diyl, propan-1,3-diyl, propan-1,2-diyl, butan-1,4-diyl, butan-1,3-diyl, butan-1,2-diyl, 2-methyl-propan-1,3-diyl and the like.

The term "alkoxy," employed alone or in combination with other terms, refers to a group of formula -O-alkyl, wherein the alkyl group is as defined above. The term "Cₙ₋ₘ alkoxy" refers to an alkoxy group, the alkyl group of which has n to m carbons. Example alkoxy groups include methoxy, ethoxy, propoxy (e.g., n-propoxy and isopropoxy), *t*-butoxy and the like. In some embodiments, the alkyl group has 1 to 6, 1 to 4, or 1 to 3 carbon atoms.

The term "alkylthio," employed alone or in combination with other terms, refers to a group of formula -S-alkyl, wherein the alkyl group is as defined above. The term "Cₙ₋ₘ alkylthio" refers to an alkylthio group, the alkyl group of which has n to m carbons. Example alkylthio groups include methylthio, ethylthio, etc. In some embodiments, the alkyl group of the alkylthio group has 1 to 6, 1 to 4, or 1 to 3 carbon atoms.

The term "amino," employed alone or in combination with other terms, refers to a group of formula -NH₂.

The term "carbonyl", employed alone or in combination with other terms, refers to a -C(=O)- group, which also may be written as C(O).

The term "cyano" or "nitrile" refers to a group of formula -C=N, which also may be written as -CN.

The terms "halo" or "halogen", used alone or in combination with other terms, refers to fluoro, chloro, bromo and iodo. In some embodiments, "halo" refers to a halogen atom selected from F, Cl, or Br. In some embodiments, halo groups are F.

The term "haloalkyl," employed alone or in combination with other terms, refers to an alkyl group in which one or more of the hydrogen atoms has been replaced by a halogen atom. The term "Cₙ₋ₘ haloalkyl" refers to a Cₙ₋ₘ alkyl group having n to m carbon atoms and from at least one up to {2(n to m)+1} halogen atoms, which may either be the same or different. In some embodiments, the halogen atoms are fluoro atoms. In some embodiments, the haloalkyl group has 1 to 6 or 1 to 4 carbon atoms. Example haloalkyl groups include CF₃, C₂F₅, CHF₂, CCl₃, CHCl₂, C₂Cl₅ and the like. In some embodiments, the haloalkyl group is a fluoroalkyl group.

The term "haloalkoxy," employed alone or in combination with other terms, refers to a group of formula -O-haloalkyl, wherein the haloalkyl group is as defined above. The term "Cₙ₋ₘ haloalkoxy" refers to a haloalkoxy group, the haloalkyl group of which has n to m carbons. Example haloalkoxy groups include trifluoromethoxy and the like. In some embodiments, the haloalkoxy group has 1 to 6, 1 to 4, or 1 to 3 carbon atoms.

The term "oxo" refers to an oxygen atom as a divalent substituent, forming a carbonyl group when attached to carbon, or attached to a heteroatom forming a sulfoxide or sulfone group, or an *N*-oxide group. In some embodiments, heterocyclic groups may be optionally substituted by 1 or 2 oxo (=O) substituents.

The term "sulfido" refers to a sulfur atom as a divalent substituent, forming a thiocarbonyl group (C=S) when attached to carbon.

The term "aromatic" refers to a carbocycle or heterocycle having one or more polyunsaturated rings having aromatic character (*i.e.*, having (4n + 2) delocalized *π* (pi) electrons where n is an integer).

The term "aryl," employed alone or in combination with other terms, refers to an aromatic hydrocarbon group, which may be monocyclic or polycyclic (e.g., having 2 fused rings). The term "Cₙ₋ₘ aryl" refers to an aryl group having from n to m ring carbon atoms. Aryl groups include, e.g., phenyl, naphthyl, indanyl, indenyl and the like. In some embodiments, aryl groups have from 6 to about 10 carbon atoms. In some embodiments aryl groups have 6 carbon atoms. In some embodiments aryl groups have 10 carbon atoms. In some embodiments, the aryl group is phenyl. In some embodiments, the aryl group is naphthyl.

The term "heteroatom" used herein is meant to include boron, phosphorus, sulfur, oxygen and nitrogen.

The term "heteroaryl" or "heteroaromatic," employed alone or in combination with other terms, refers to a monocyclic or polycyclic aromatic heterocycle having at least one heteroatom ring member selected from boron, phosphorus, sulfur, oxygen and nitrogen. In some embodiments, the heteroaryl ring has 1, 2, 3 or 4 heteroatom ring members independently selected from nitrogen, sulfur and oxygen. In some embodiments, any ring-forming N in a heteroaryl moiety can be an N-oxide. In some embodiments, the heteroaryl has 5-14 ring atoms including carbon atoms and 1, 2, 3 or 4 heteroatom ring members independently selected from nitrogen, sulfur and oxygen. In some embodiments, the heteroaryl has 5-14, or 5-10 ring atoms including carbon atoms and 1, 2, 3 or 4 heteroatom ring members independently selected from nitrogen, sulfur and oxygen. In some embodiments, the heteroaryl has 5-6 ring atoms and 1 or 2 heteroatom ring members independently selected from nitrogen, sulfur and oxygen. In some embodiments, the heteroaryl is a five-membered or six-membered heteroaryl ring. In other embodiments, the heteroaryl is an eight-membered, nine-membered or ten-membered fused bicyclic heteroaryl ring. Example heteroaryl groups include, but are not limited to, pyridinyl (pyridyl), pyrimidinyl, pyrazinyl, pyridazinyl, pyrrolyl, pyrazolyl, azolyl, oxazolyl, thiazolyl, imidazolyl, furanyl, thiophenyl, quinolinyl, isoquinolinyl, naphthyridinyl (including 1,2-, 1,3-, 1,4-, 1,5-, 1,6-, 1,7-, 1,8-, 2,3- and 2,6-naphthyridine), indolyl, benzothiophenyl, benzofuranyl, benzisoxazolyl, imidazo[1,2-*b*]thiazolyl, purinyl, and the like.

A five-membered heteroaryl ring is a heteroaryl group having five ring atoms wherein one or more *(e.g.,* 1, 2 or 3) ring atoms are independently selected from N, O and S. Exemplary five-membered ring heteroaryls include thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl and 1,3,4-oxadiazolyl.

A six-membered heteroaryl ring is a heteroaryl group having six ring atoms wherein one or more *(e.g.,* 1, 2 or 3) ring atoms are independently selected from N, O and S. Exemplary six-membered ring heteroaryls are pyridyl, pyrazinyl, pyrimidinyl, triazinyl and pyridazinyl.

The term "cycloalkyl," employed alone or in combination with other terms, refers to a non-aromatic hydrocarbon ring system (monocyclic, bicyclic or polycyclic), including cyclized alkyl and alkenyl groups. The term "Cₙ₋ₘ cycloalkyl" refers to a cycloalkyl that has n to m ring member carbon atoms. Cycloalkyl groups can include mono- or polycyclic (e.g., having 2, 3 or 4 fused rings) groups and spirocycles. Cycloalkyl groups can have 3, 4, 5, 6, 7, 8, 9, 10, 11, 12, 13, or 14 ring-forming carbons (C₃₋₁₄). In some embodiments, the cycloalkyl group has 3 to 14 members, 3 to 10 members, 3 to 6 ring members, 3 to 5 ring members, or 3 to 4 ring members. In some embodiments, the cycloalkyl group is monocyclic. In some embodiments, the cycloalkyl group is monocyclic or bicyclic. In some embodiments, the cycloalkyl group is a C₃₋₆ monocyclic cycloalkyl group. Ring-forming carbon atoms of a cycloalkyl group can be optionally oxidized to form an oxo or sulfido group. Cycloalkyl groups also include cycloalkylidenes. In some embodiments, cycloalkyl is cyclopropyl, cyclobutyl, cyclopentyl or cyclohexyl. Also included in the definition of cycloalkyl are moieties that have one or more aromatic rings fused (*i*.*e*., having a bond in common with) to the cycloalkyl ring, *e.g.,* benzo or thienyl derivatives of cyclopentane, cyclohexane and the like. A cycloalkyl group containing a fused aromatic ring can be attached through any ring-forming atom including a ring-forming atom of the fused aromatic ring. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcarnyl, bicyclo[1.1.1]pentanyl, bicyclo[2.1.1]hexanyl, and the like. In some embodiments, the cycloalkyl group is cyclopropyl, cyclobutyl, cyclopentyl, or cyclohexyl.

The term "heterocycloalkyl," employed alone or in combination with other terms, refers to a non-aromatic ring or ring system, which may optionally contain one or more alkenylene groups as part of the ring structure, which has at least one heteroatom ring member independently selected from boron, nitrogen, sulfur oxygen and phosphorus, and which has 4-14 ring members, 4-10 ring members, 4-7 ring members, or 4-6 ring members. Included within the term "heterocycloalkyl" are monocyclic 4-, 5-, 6- and 7-membered heterocycloalkyl groups. Heterocycloalkyl groups can include mono- or bicyclic or polycyclic (e.g., having two or three fused or bridged rings) ring systems or spirorcycles. In some embodiments, the heterocycloalkyl group is a monocyclic group having 1, 2 or 3 heteroatoms independently selected from nitrogen, sulfur and oxygen. Ring-forming carbon atoms and heteroatoms of a heterocycloalkyl group can be optionally oxidized to form an oxo or sulfido group or other oxidized linkage (*e.g.,* C(O), S(O), C(S) or S(O)₂, *N*-oxide *etc.*) or a nitrogen atom can be quaternized. The heterocycloalkyl group can be attached through a ring-forming carbon atom or a ring-forming heteroatom. In some embodiments, the heterocycloalkyl group contains 0 to 3 double bonds. In some embodiments, the heterocycloalkyl group contains 0 to 2 double bonds. Also included in the definition of heterocycloalkyl are moieties that have one or more aromatic rings fused (*i*.*e*., having a bond in common with) to the heterocycloalkyl ring, *e.g.,* benzo or thienyl derivatives of piperidine, morpholine, azepine, *etc.* A heterocycloalkyl group containing a fused aromatic ring can be attached through any ring-forming atom including a ring-forming atom of the fused aromatic ring. Examples of heterocycloalkyl groups include azetidinyl, azepanyl, dihydrobenzofuranyl, dihydrofuranyl, dihydropyranyl, morpholino, 3-oxa-9-azaspiro[5.5]undecanyl, 1-oxa-8-azaspiro[4.5]decanyl, piperidinyl, piperazinyl, oxopiperazinyl, pyranyl, pyrrolidinyl, quinuclidinyl, tetrahydrofuranyl, tetrahydropyranyl, 1,2,3,4-tetrahydroquinolinyl, tropanyl, tetrahydrothiazolopyridinyl (e.g., 4,5,6,7-tetrahydrothiazolo[5,4-c]pyridin-2-yl) and thiomorpholino.

The term "arylalkyl," employed alone or in combination with other terms, refers to an aryl-(alkylene)- group where aryl and alkylene are as defined herein. An example arylalkyl group is benzyl.

The term "heteroarylalkyl," employed alone or in combination with other terms, refers to an heteroaryl-(alkylene)- group, where heteroaryl and alkylene are as defined herein. An example heteroarylalkyl group is pyridylmethyl.

The term "cycloalkylalkyl," employed alone or in combination with other terms, refers to a cycloalkyl-(alkylene)- group, where cycloalkyl and alkylene are as defined herein. An example cycloalkylalkyl group is cyclopropylmethyl.

The term "heterocycloalkylalkyl," employed alone or in combination with other terms, refers to a heterocycloalkyl-(alkylene)- group, where heterocycloalkyl and alkylene are as defined herein. An example heterocycloalkylalkyl group is azetidinylmethyl.

At certain places, the definitions or embodiments refer to specific rings (e.g., an azetidine ring, a pyridine ring, *etc.*)*.* Unless otherwise indicated, these rings can be attached to any ring member provided that the valency of the atom is not exceeded. For example, an azetidine ring may be attached at any position of the ring, whereas an azetidin-3-yl ring is attached at the 3-position.

The compounds described herein can be asymmetric (e.g., having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically inactive starting materials are known in the art, such as by resolution of racemic mixtures or by stereoselective synthesis. Many geometric isomers of olefins, C=N double bonds and the like can also be present in the compounds described herein, and all such stable isomers are contemplated in the present invention. *Cis* and *trans* geometric isomers of the compounds of the present invention are described and may be isolated as a mixture of isomers or as separated isomeric forms.

Resolution of racemic mixtures of compounds can be carried out by any of numerous methods known in the art. One method includes fractional recrystallization using a chiral resolving acid which is an optically active, salt-forming organic acid. Suitable resolving agents for fractional recrystallization methods are, e.g., optically active acids, such as the D and L forms of tartaric acid, diacetyltartaric acid, dibenzoyltartaric acid, mandelic acid, malic acid, lactic acid or the various optically active camphorsulfonic acids such as β-camphorsulfonic acid. Other resolving agents suitable for fractional crystallization methods include stereoisomerically pure forms of α-methylbenzylamine (*e*.*g*., *S* and *R* forms, or diastereomerically pure forms), 2-phenylglycinol, norephedrine, ephedrine, N-methylephedrine, cyclohexylethylamine, 1,2-diaminocyclohexane and the like.

Resolution of racemic mixtures can also be carried out by elution on a column packed with an optically active resolving agent (e.g., dinitrobenzoylphenylglycine). Suitable elution solvent composition can be determined by one skilled in the art.

In some embodiments, the compounds of the invention have the (*R*)-configuration. In other embodiments, the compounds have the (*S*)-configuration. In compounds with more than one chiral centers, each of the chiral centers in the compound may be independently (R) or (*S*), unless otherwise indicated.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, enamine - imine pairs, and annular forms where a proton can occupy two or more positions of a heterocyclic system, *e.g.,* 1*H*- and 3*H*-imidazole, 1*H*-*,* 2*H*- and 4*H*- 1,2,4-triazole, 1*H*- and 2*H*- isoindole and 1*H*- and 2*H*-pyrazole. Tautomeric forms can be in equilibrium or sterically locked into one form by appropriate substitution.

Compounds of the invention can also include all isotopes of atoms occurring in the intermediates or final compounds. Isotopes include those atoms having the same atomic number but different mass numbers. For example, isotopes of hydrogen include tritium and deuterium. One or more constituent atoms of the compounds of the invention can be replaced or substituted with isotopes of the atoms in natural or non-natural abundance. In some embodiments, the compound includes at least one deuterium atom. For example, one or more hydrogen atoms in a compound of the present disclosure can be replaced or substituted by deuterium. In some embodiments, the compound includes two or more deuterium atoms. In some embodiments, the compound includes 1, 2, 3, 4, 5, 6, 7, 8, 9, 10, 11 or 12 deuterium atoms. Synthetic methods for including isotopes into organic compounds are known in the art.

The term, "compound," as used herein is meant to include all stereoisomers, geometric isomers, tautomers and isotopes of the structures depicted. The term is also meant to refer to compounds of the inventions, regardless of how they are prepared, e.g., synthetically, through biological process (e.g., metabolism or enzyme conversion), or a combination thereof.

All compounds, and pharmaceutically acceptable salts thereof, can be found together with other substances such as water and solvents (e.g., hydrates and solvates) or can be isolated. When in the solid state, the compounds described herein and salts thereof may occur in various forms and may, e.g., take the form of solvates, including hydrates. The compounds may be in any solid state form, such as a polymorph or solvate, so unless clearly indicated otherwise, reference in the specification to compounds and salts thereof should be understood as encompassing any solid state form of the compound.

In some embodiments, the compounds of the invention, or salts thereof, are substantially isolated. By "substantially isolated" is meant that the compound is at least partially or substantially separated from the environment in which it was formed or detected. Partial separation can include, e.g., a composition enriched in the compounds of the invention. Substantial separation can include compositions containing at least about 50%, at least about 60%, at least about 70%, at least about 80%, at least about 90%, at least about 95%, at least about 97%, or at least about 99% by weight of the compounds of the invention, or salt thereof.

The phrase "pharmaceutically acceptable" is employed herein to refer to those compounds, materials, compositions and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The expressions, "ambient temperature" and "room temperature," as used herein, are understood in the art, and refer generally to a temperature, e.g., a reaction temperature, that is about the temperature of the room in which the reaction is carried out, *e.g.,* a temperature from about 20 °C to about 30 °C.

The present invention also includes pharmaceutically acceptable salts of the compounds described herein. The term "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the non-toxic salts of the parent compound formed, e.g., from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (e.g., methanol, ethanol, iso-propanol or butanol) or acetonitrile (MeCN) are preferred. Lists of suitable salts are found in Remington's Pharmaceutical Sciences, 17th Ed., (Mack Publishing Company, Easton, 1985), p. 1418, Berge et al., J. Pharm. Sci., 1977, 66(1), 1-19 and in Stahl et al., Handbook of Pharmaceutical Salts: Properties, Selection, and Use, (Wiley, 2002). In some embodiments, the compounds described herein include the N-oxide forms.

### II. Synthesis

Compounds of the invention, including salts thereof, can be prepared using known organic synthesis techniques and can be synthesized according to any of numerous possible synthetic routes, such as those in the Schemes below.

The reactions for preparing compounds of the invention can be carried out in suitable solvents which can be readily selected by one of skill in the art of organic synthesis. Suitable solvents can be substantially non-reactive with the starting materials (reactants), the intermediates or products at the temperatures at which the reactions are carried out, e.g., temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of compounds of the invention can involve the protection and deprotection of various chemical groups. The need for protection and deprotection, and the selection of appropriate protecting groups, can be readily determined by one skilled in the art. The chemistry of protecting groups is described, *e.g.,* in Kocienski, Protecting Groups, (Thieme, 2007); Robertson, Protecting Group Chemistry, (Oxford University Press, 2000); Smith et al., March's Advanced Organic Chemistry: Reactions, Mechanisms, and Structure, 6th Ed. (Wiley, 2007); Peturssion et al., "Protecting Groups in Carbohydrate Chemistry," J. Chem. Educ., 1997, 74(11), 1297; and Wuts et al., Protective Groups in Organic Synthesis, 4th Ed., (Wiley, 2006).

Reactions can be monitored according to any suitable method known in the art. For example, product formation can be monitored by spectroscopic means, such as nuclear magnetic resonance spectroscopy (e.g., ¹H or ¹³C), infrared spectroscopy, spectrophotometry (e.g., UV-visible), mass spectrometry or by chromatographic methods such as high performance liquid chromatography (HPLC) or thin layer chromatography (TLC).

The Schemes below provide general guidance in connection with preparing the compounds of the invention. One skilled in the art would understand that the preparations shown in the Schemes can be modified or optimized using general knowledge of organic chemistry to prepare various compounds of the invention.

Compounds of Formula **I** can be synthesized using a process shown in Scheme 1. In Scheme 1, a suitable halo (Hal¹)-substituted [4.4.0] aromatic heterocycle **1-1** is reacted with a suitable halo (Hal²)-substitued aniline **1-2** to produce compound **1-3** under standard S_{N}Ar conditions using an acid such as, but not limited to, sulfuric acid, or base such as, but not limited to, potassium *tert*-butoxide. Compounds of formula **1-3** may also be synthesized under standard metal catlyzed cross-coupling reaction conditions (such as Buchwald-Hartwig coupling reaction, *e.g.,* in the presence of a palladium catalyst (*e.g.*, [(4,5-bis(dipheny1phosphino)-9,9-dimethylxanthene)-2-(2'-amino-1,1'-biphenyl)]palladium(II) methanesulfonate) and a base (e.g., cesium carbonate)). Then the aromatic halide 1-3 can be reacted with a suitable coupling reagent **1-4** (where M is, *e.g.,* -B(OH)₂) to provide the product of formula **I** under standard metal catalyzed cross-coupling reaction conditions (such as Suzuki coupling reaction, *e.g.,* in the presence of a palladium catalyst (*e.g.,* [1,1'-bis(diphenylphosphino)ferrocene]dichloropalladium(II)) and a base *(e.g.,* a bicarbonate or a carbonate base)).

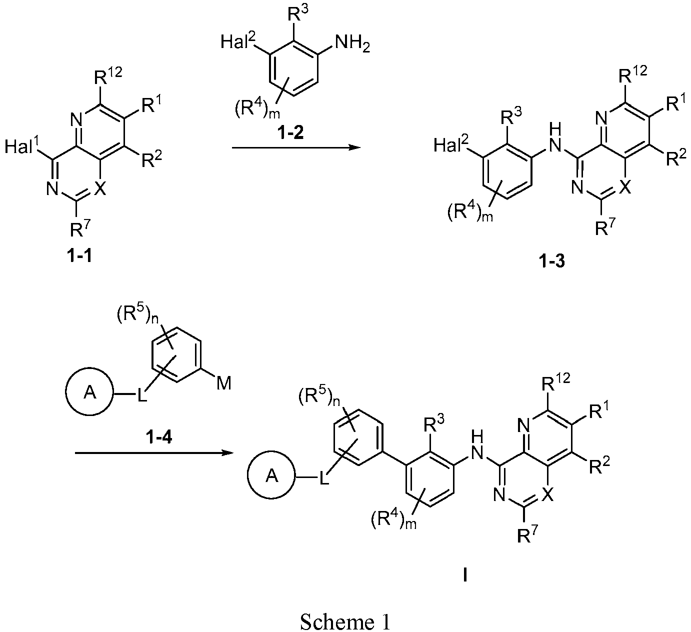

Compounds of formula **II** can be synthesized using a process shown in Scheme 2. A suitable halo (Hal¹)-substituted [4.4.0] aromatic heterocycle **2-1** can be reacted with a suitable halo (Hal²)-substitued aniline **2-2** to produce formula **2-3** under S_{N}Ar conditions using an acid such as, but not limited to, sulfuric acid, or base such as, but not limited to, potassium *tert*-butoxide. Compounds of formula **2-3** may also be synthesized under standard metal catlyzed cross-coupling reaction conditions (such as Buchwald-Hartwig coupling reaction, *e.g.,* in the presence of a palladium catalyst (*e.g.,* [(4,5-bis(diphenylphosphino)-9,9-dimethylxanthene)-2-(2'-amino-1,1'-biphenyl)]palladium(II) methanesulfonate) and a base (e.g., cesium carbonate)). Then the aromatic halide **2-3** is reacted with a suitable coupling reagent **2-4** (where M is, *e.g.,* -B(OH)₂) to form the bi-aryl bond of formula **2-5** under standard metal catalyzed cross-coupling reaction conditions (such as Suzuki coupling reaction, *e.g.,* in the presence of a palladium catalyst (*e.g.*, [1,1'-bis(diphenylphosphino)ferrocene]dichloropalladium(II)) and a base *(e.g.,* a bicarbonate or a carbonate base)). The vinyl group in compound 2-5 can be oxidatively cleaved to afford an aldehyde in the presence of suitable reagents such as, but not limited to, OsO₄ and NaIO₄. Then the compound of formula II can be obtained by a reductive amination between the aldehyde derivative and a suitable amine 2-6 in a proper solvent such as THF or DCM using a reducing agent such as, but not limited to, sodium triacetoxyborohydride, optionally in the presence of an acid such as acetic acid or a base such as DIPEA.

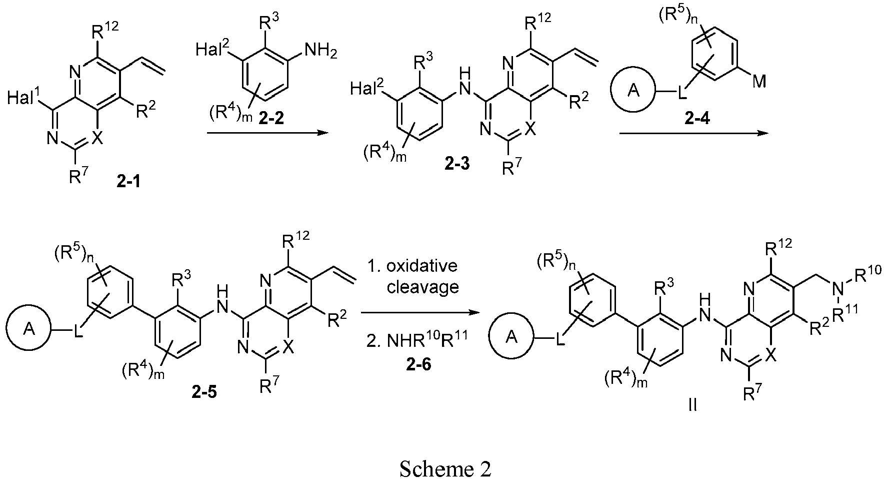

Compounds of formula **II** can be alternatively synthesized using a process shown in Scheme 3. The vinyl group of a suitable halo (Hal¹)-substituted [4.4.0] aromatic heterocycle 3-1 can be oxidatively cleaved to afford an aldehyde in the presence of suitable reagents such as, but not limited to, OsO₄ and NaIO₄. Then the compound of formula **3-3** can be obtained by a reductive amination between the aldehyde derivative and a suitable amine **3-2** in a proper solvent such as THF or DCM using a reducing agent such as, but not limited to, sodium triacetoxyborohydride, optionally in the presence of an acid such as acetic acid or a base such as DIPEA. The compound of formula **3-5** can be synthesized by reacting formula **3-3** with a suitable halo (Hal²)-substitued aniline **3-4** under standard S_{N}Ar conditions using an acid such as, but not limited to, sulfuric acid, or base such as, but not limited to, potassium *tert*-butoxide. Compounds of formula **3-5** may also be synthesized under standard metal catlyzed cross-coupling reaction conditions (such as Buchwald-Hartwig coupling reaction, *e.g.,* in the presence of a palladium catalyst *(e.g.,* [(4,5-bis(diphenylphosphino)-9,9-dimethylxanthene)-2-(2' -amino-1,1' -biphenyl)]palladium(II) methanesulfonate) and a base (e.g., cesium carbonate)). Then the aromatic halide **3-5** is reacted with a suitable coupling reagent **3-6** (where M is, *e*.*g*., -B(OH)₂) to provide compounds of formula **II** under standard metal catalyzed cross-coupling reaction conditions (such as Suzuki coupling reaction, *e.g.,* in the presence of a palladium catalyst (*e.g.,* [1,1'-bis(diphenylphosphino)ferrocene]dichloropalladium(II)) and a base *(e.g.,* a bicarbonate or a carbonate base)).

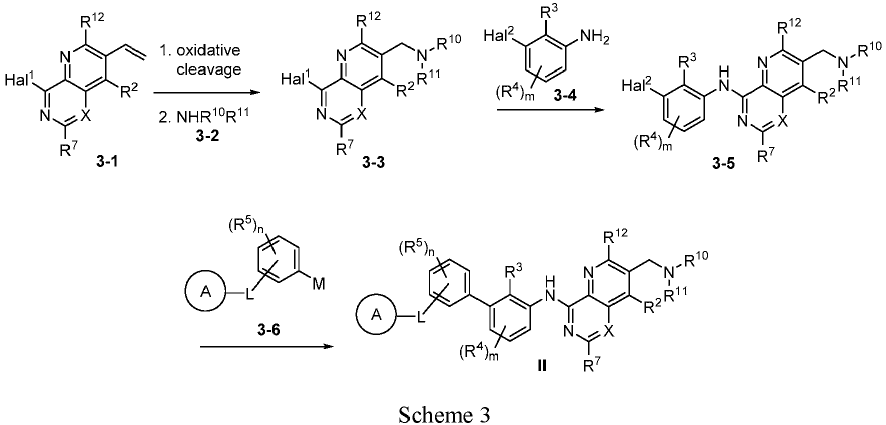

Compounds of formula **III** can be synthesized using a process shown in Scheme 4. A suitable halo (Hal¹)-substituted [4.4.0] aromatic heterocycle **4-1** can be reacted with a suitable halo (Hal²)-substitued aniline **4-2** to produce a compound of formula **4-3** under standard S_{N}Ar conditions using an acid such as, but not limited to, sulfuric acid, or base such as, but not limited to, potassium *tert*-butoxide. Compounds of formula **4-3** may also be synthesized under standard metal catlyzed cross-coupling reaction conditions (such as Buchwald-Hartwig coupling reaction, *e.g.,* in the presence of a palladium catalyst (*e.g.*, [(4,5-bis(diphenylphosphino)-9,9-dimethylxanthene)-2-(2' -amino-1,1' -biphenyl)]palladium(II) methanesulfonate) and a base (e.g., cesium carbonate)). Then the aromatic halide **4-3** can be reacted with a suitable coupling reagent **4-4** (where M is, *e.g.,* -B(OH)₂) to form the bi-aryl bond of formula **4-5** under standard metal catalyzed cross-coupling reaction conditions (such as Suzuki coupling reaction, *e.g.,* in the presence of a palladium catalyst (*e.g.*, [1,1'-bis(diphenylphosphino)ferrocene]dichloropalladium(II)) and a base *(e.g.,* a bicarbonate or a carbonate base)). The vinyl group in compound **4-5** can be oxidatively cleaved to afford an aldehyde in the presence of suitable reagents such as, but not limited to, OsO₄ and NaIO₄. Then the compound of formula **III** is obtained by a reductive amination between the aldehyde derivative and a suitable amine **4-6** in a proper solvent such as THF or DCM using a reducing agent such as, but not limited to, sodium triacetoxyborohydride, optionally in the presence of an acid such as acetic acid or a base such as DIPEA.

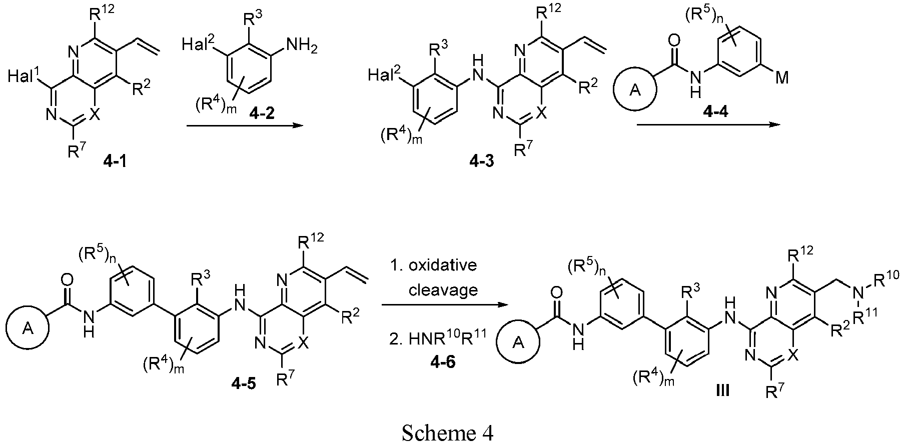

Compounds of formula **III** can be alternatively synthesized using a process shown in Scheme 5. The vinyl group of a suitable halo (Hal¹)-substituted [4.4.0] aromatic heterocycle **5-1** can be oxidatively cleaved to afford an aldehyde in the presence of suitable reagents such as, but not limited to, OsO₄ and NaIO₄. Then the compound of formula **5-3** is obtained by a reductive amination between the aldehyde derivative and a suitable amine **5-2** in a proper solvent such as THF or DCM using a reducing agent such as, but not limited to, sodium triacetoxyborohydride, optionally in the presence of an acid such as acetic acid or a base such as DIPEA. The compound of formula **5-5** can be synthesized by reacting formula **5-3** with a suitable halo (Hal²)-substitued aniline **5-4** under standard S_{N}Ar conditions using an acid such as, but not limited to, sulfuric acid, or base such as, but not limited to, potassium *tert-*butoxide. Compounds of formula **5-5** may also be synthesized under standard metal catlyzed cross-coupling reaction conditions (such as Buchwald-Hartwig coupling reaction, e.g., in the presence of a palladium catalyst (e.g., [(4,5-bis(diphenylphosphino)-9,9-dimethylxanthene)-2-(2' -amino-1,1' -biphenyl)]palladium(II) methanesulfonate) and a base *(e.g.,* cesium carbonate)). Then the aromatic halide **5-5** is reacted with a suitable coupling reagent **5-6** (where M is, *e.g.,* -B(OH)₂) to provide compounds of formula **II** under standard metal catalyzed cross-coupling reaction conditions (such as Suzuki coupling reaction, e.g., in the presence of a palladium catalyst (*e*.*g*., [1,1'-bis(diphenylphosphino)ferrocene]dichloropalladium(II)) and a base *(e.g.,* a bicarbonate or a carbonate base)).

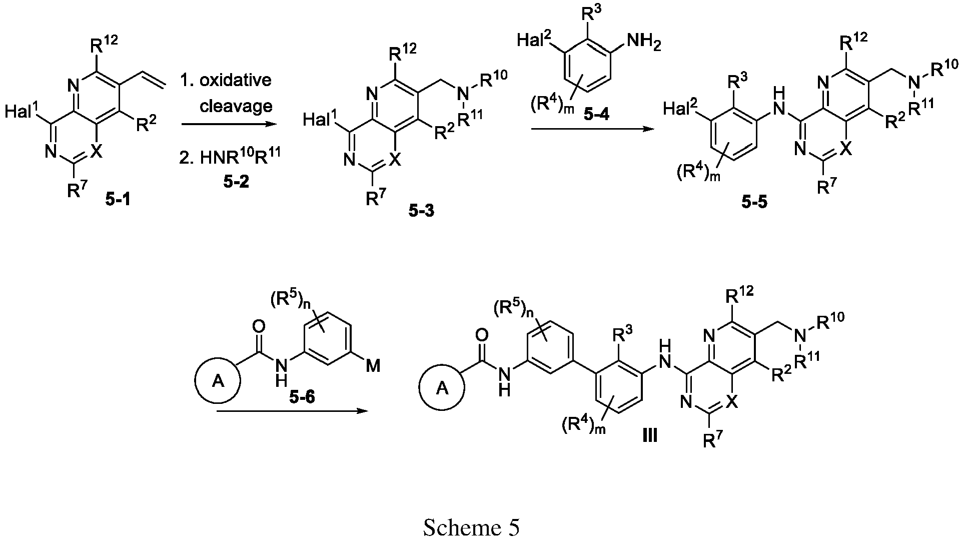

### III. Uses of the Compounds

Compounds of the present disclosure can inhibit the activity of PD-1/PD-L1 protein/protein interaction and, thus, are useful in treating diseases and disorders associated with activity of PD-1 and the diseases and disorders associated with PD-L1 including its interaction with other proteins such as PD-1 and B7-1 (CD80). In certain embodiments, the compounds of the present disclosure, or pharmaceutically acceptable salts thereof, are useful for therapeutic administration to enhance immunity in cancer, chronic infection or sepsis, including enhancement of response to vaccination. In some embodiments, the present disclosure provides compounds and compositions for use in a method for inhibiting the PD-1/PD-L1 protein/protein interaction. The method includes administering to an individual or a patient a compound of Formula (I) or of any of the formulas as described herein, or of a compound as recited in any of the claims and described herein, or a pharmaceutically acceptable salt thereof. The compounds of the present disclosure can be used alone, in combination with other agents or therapies or as an adjuvant or neoadjuvant for the treatment of diseases or disorders, including cancer or infection diseases. For the uses described herein, any of the compounds of the disclosure, including any of the embodiments thereof, may be used.

The compounds of the present disclosure inhibit the PD-1/PD-L1 protein/protein interaction, resulting in a PD-1 pathway blockade. The blockade of PD-1 can enhance the immune response to cancerous cells and infectious diseases in mammals, including humans. In some embodiments, the present disclosure provides treatment of an individual or a patient *in vivo* using a compound of Formula (I) or a salt thereof such that growth of cancerous tumors is inhibited. A compound of Formula (I) or of any of the formulas as described herein, or a compound as recited in any of the claims and described herein, or a salt thereof, can be used to inhibit the growth of cancerous tumors. Alternatively, a compound of Formula (I) or of any of the formulas as described herein, or a compound as recited in any of the claims and described herein, or a salt thereof, can be used in conjunction with other agents or standard cancer treatments, as described below. In one embodiment, the present disclosure provides compounds and compositions for use in a method for inhibiting growth of tumor cells *in vitro.* The method includes contacting the tumor cells *in vitro* with a compound of Formula (I) or of any of the formulas as described herein, or of a compound as recited in any of the claims and described herein, or of a salt thereof. In another embodiment, the present disclosure provides compounds and compositions for use in a method for inhibiting growth of tumor cells in an individual or a patient. The method includes administering to the individual or patient in need thereof a therapeutically effective amount of a compound of Formula (I) or of any of the formulas as described herein, or of a compound as recited in any of the claims and described herein, or a salt thereof.

In some embodiments, provided herein are compounds and compositions for use in a method for treating cancer. The method includes administering to a patient in need thereof, a therapeutically effective amount of a compound of Formula (I) or any of the formulas as described herein, a compound as recited in any of the claims and described herein, or a salt thereof. Examples of cancers include those whose growth may be inhibited using compounds of the disclosure and cancers typically responsive to immunotherapy.

Examples of cancers that are treatable using the compounds of the present disclosure include, but are not limited to, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, endometrial cancer, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or urethra, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T -cell lymphoma, environmentally induced cancers including those induced by asbestos, and combinations of said cancers. The compounds of the present disclosure are also useful for the treatment of metastatic cancers, especially metastatic cancers that express PD-L1.

In some embodiments, cancers treatable with compounds of the present disclosure include melanoma (e.g., metastatic malignant melanoma), renal cancer (e.g. clear cell carcinoma), prostate cancer (e.g. hormone refractory prostate adenocarcinoma), breast cancer, colon cancer, lung cancer (e.g. non-small cell lung cancer and small cell lung cancer), squamous cell head and neck cancer, urothelial cancer (e.g. bladder and cancers with high microsatellite instability (MSI^{high}). Additionally, the disclosure includes refractory or recurrent malignancies whose growth may be inhibited using the compounds of the disclosure.

In some embodiments, cancers that are treatable using the compounds of the present disclosure include, but are not limited to, solid tumors (e.g., prostate cancer, colon cancer, esophageal cancer, endometrial cancer, ovarian cancer, uterine cancer, renal cancer, hepatic cancer, pancreatic cancer, gastric cancer, breast cancer, lung cancer, cancers of the head and neck, thyroid cancer, glioblastoma, sarcoma, bladder cancer, etc.), hematological cancers (e.g., lymphoma, leukemia such as acute lymphoblastic leukemia (ALL), acute myelogenous leukemia (AML), chronic lymphocytic leukemia (CLL), chronic myelogenous leukemia (CML), DLBCL, mantle cell lymphoma, Non-Hodgkin lymphoma (including relapsed or refractory NHL and recurrent follicular), Hodgkin lymphoma or multiple myeloma) and combinations of said cancers.

In some embodiments, cancers that are treatable using the compounds of the present disclosure include, but are not limited to, cholangiocarcinoma, bile duct cancer, triple negative breast cancer, rhabdomyosarcoma, small cell lung cancer, leiomyosarcoma, hepatocellular carcinoma, Ewing's sarcoma, brain cancer, brain tumor, astrocytoma, neuroblastoma, neurofibroma, basal cell carcinoma, chondrosarcoma, epithelioid sarcoma, eye cancer, Fallopian tube cancer, gastrointestinal cancer, gastrointestinal stromal tumors, hairy cell leukemia, intestinal cancer, islet cell cancer, oral cancer, mouth cancer, throat cancer, laryngeal cancer, lip cancer, mesothelioma, neck cancer, nasal cavity cancer, ocular cancer, ocular melanoma, pelvic cancer, rectal cancer, renal cell carcinoma, salivary gland cancer, sinus cancer, spinal cancer, tongue cancer, tubular carcinoma, urethral cancer, and ureteral cancer.

In some embodiments, the compounds of the present disclosure can be used to treat sickle cell disease and sickle cell anemia.

In some embodiments, diseases and indications that are treatable using the compounds of the present disclosure include, but are not limited to hematological cancers, sarcomas, lung cancers, gastrointestinal cancers, genitourinary tract cancers, liver cancers, bone cancers, nervous system cancers, gynecological cancers, and skin cancers.

Exemplary hematological cancers include lymphomas and leukemias such as acute lymphoblastic leukemia (ALL), acute myelogenous leukemia (AML), acute promyelocytic leukemia (APL), chronic lymphocytic leukemia (CLL), chronic myelogenous leukemia (CML), diffuse large B-cell lymphoma (DLBCL), mantle cell lymphoma, Non-Hodgkin lymphoma (including relapsed or refractory NHL and recurrent follicular), Hodgkin lymphoma, myeloproliferative diseases (e.g., primary myelofibrosis (PMF), polycythemia vera (PV), essential thrombocytosis (ET)), myelodysplasia syndrome (MDS), T-cell acute lymphoblastic lymphoma (T-ALL) and multiple myeloma (MM).

Exemplary sarcomas include chondrosarcoma, Ewing's sarcoma, osteosarcoma, rhabdomyosarcoma, angiosarcoma, fibrosarcoma, liposarcoma, myxoma, rhabdomyoma, rhabdosarcoma, fibroma, lipoma, harmatoma, and teratoma.

Exemplary lung cancers include non-small cell lung cancer (NSCLC), small cell lung cancer, bronchogenic carcinoma (squamous cell, undifferentiated small cell, undifferentiated large cell, adenocarcinoma), alveolar (bronchiolar) carcinoma, bronchial adenoma, chondromatous hamartoma, and mesothelioma.

Exemplary gastrointestinal cancers include cancers of the esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma), and colorectal cancer.

Exemplary genitourinary tract cancers include cancers of the kidney (adenocarcinoma, Wilm's tumor [nephroblastoma]), bladder and urethra (squamous cell carcinoma, transitional cell carcinoma, adenocarcinoma), prostate (adenocarcinoma, sarcoma), and testis (seminoma, teratoma, embryonal carcinoma, teratocarcinoma, choriocarcinoma, sarcoma, interstitial cell carcinoma, fibroma, fibroadenoma, adenomatoid tumors, lipoma).

Exemplary liver cancers include hepatoma (hepatocellular carcinoma), cholangiocarcinoma, hepatoblastoma, angiosarcoma, hepatocellular adenoma, and hemangioma.

Exemplary bone cancers include, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma, and giant cell tumors

Exemplary nervous system cancers include cancers of the skull (osteoma, hemangioma, granuloma, xanthoma, osteitis deformans), meninges (meningioma, meningiosarcoma, gliomatosis), brain (astrocytoma, meduoblastoma, glioma, ependymoma, germinoma (pinealoma), glioblastoma, glioblastoma multiform, oligodendroglioma, schwannoma, retinoblastoma, congenital tumors), and spinal cord (neurofibroma, meningioma, glioma, sarcoma), as well as neuroblastoma and Lhermitte-Duclos disease.

Exemplary gynecological cancers include cancers of the uterus (endometrial carcinoma), cervix (cervical carcinoma, pre -tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma).

Exemplary skin cancers include melanoma, basal cell carcinoma, squamous cell carcinoma, Kaposi's sarcoma, moles dysplastic nevi, lipoma, angioma, dermatofibroma, and keloids.In some embodiments, diseases and indications that are treatable using the compounds of the present disclosure include, but are not limited to, sickle cell disease (e.g., sickle cell anemia), triple-negative breast cancer (TNBC), myelodysplastic syndromes, testicular cancer, bile duct cancer, esophageal cancer, and urothelial carcinoma.

PD-1 pathway blockade with compounds of the present disclosure can also be used for treating infections such as viral, bacteria, fungus and parasite infections. The present disclosure provides compounds and compositions for use in a method for treating infections such as viral infections. The method includes administering to a patient in need thereof, a therapeutically effective amount of a compound of Formula (I) or any of the formulas as described herein, a compound as recited in any of the claims and described herein, a salt thereof. Examples of viruses causing infections treatable by compounds and compositions of the present disclosure include, but are not limit to, human immunodeficiency virus, human papillomavirus, influenza, hepatitis A, B, C or D viruses, adenovirus, poxvirus, herpes simplex viruses, human cytomegalovirus, severe acute respiratory syndrome virus, ebola virus, and measles virus. In some embodiments, viruses causing infections treatable by compounds and compositions of the present disclosure include, but are not limit to, hepatitis (A, B, or C), herpes virus (e.g., VZV, HSV-1, HAV-6, HSV-II, and CMV, Epstein Barr virus), adenovirus, influenza virus, flaviviruses, echovirus, rhinovirus, coxsackie virus, cornovirus, respiratory syncytial virus, mumpsvirus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

The present disclosure provides compounds and compositions for use in a method for treating bacterial infections. The method includes administering to a patient in need thereof, a therapeutically effective amount of a compound of Formula (I) or any of the formulas as described herein, a compound as recited in any of the claims and described herein, or a salt thereof. Non-limiting examples of pathogenic bacteria causing infections treatable by compounds and compositions of the disclosure include chlamydia, rickettsial bacteria, mycobacteria, staphylococci, streptococci, pneumonococci, meningococci and conococci, klebsiella, proteus, serratia, pseudomonas, legionella, diphtheria, salmonella, bacilli, cholera, tetanus, botulism, anthrax, plague, leptospirosis, and Lyme's disease bacteria.

The present disclosure provides compounds and compositions for use in a method for treating fungus infections. The method includes administering to a patient in need thereof, a therapeutically effective amount of a compound of Formula (I) or any of the formulas as described herein, a compound as recited in any of the claims and described herein, or a salt thereof. Non-limiting examples of pathogenic fungi causing infections treatable by compounds and compositions of the disclosure include Candida (albicans, krusei, glabrata, tropicalis, etc.), Cryptococcus neoformans, Aspergillus (fumigatus, niger, etc.), Genus Mucorales (mucor, absidia, rhizophus), Sporothrix schenkii, Blastomyces dermatitidis, Paracoccidioides brasiliensis, Coccidioides immitis and Histoplasma capsulatum.

The present disclosure provides compounds and compositions for use in a method for treating parasite infections. The method includes administering to a patient in need thereof, a therapeutically effective amount of a compound of Formula (I) or any of the formulas as described herein, a compound as recited in any of the claims and described herein, or a salt thereof. Non-limiting examples of pathogenic parasites causing infections treatable by compounds and compositions of the disclosure include Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondi, and Nippostrongylus brasiliensis.

It is believed that compounds of Formula (I), or any of the embodiments thereof, may possess satisfactory pharmacological profile and promising biopharmaceutical properties, such as toxicological profile, metabolism and pharmacokinetic properties, solubility, and permeability. It will be understood that determination of appropriate biopharmaceutical properties is within the knowledge of a person skilled in the art, e.g., determination of cytotoxicity in cells or inhibition of certain targets or channels to determine potential toxicity.

The terms "individual" or "patient," used interchangeably, refer to any animal, including mammals, preferably mice, rats, other rodents, rabbits, dogs, cats, swine, cattle, sheep, horses, or primates, and most preferably humans.

The phrase "therapeutically effective amount" refers to the amount of active compound or pharmaceutical agent that elicits the biological or medicinal response in a tissue, system, animal, individual or human that is being sought by a researcher, veterinarian, medical doctor or other clinician.

As used herein, the term "treating" or "treatment" refers to one or more of (1) inhibiting the disease; e.g., inhibiting a disease, condition or disorder in an individual who is experiencing or displaying the pathology or symptomatology of the disease, condition or disorder (*i*.*e*., arresting further development of the pathology and/or symptomatology); and (2) ameliorating the disease; e.g., ameliorating a disease, condition or disorder in an individual who is experiencing or displaying the pathology or symptomatology of the disease, condition or disorder (*i*.*e*., reversing the pathology and/or symptomatology) such as decreasing the severity of disease.

In some embodiments, the compounds of the invention are useful in preventing or reducing the risk of developing any of the diseases referred to herein; *e.g.,* preventing or reducing the risk of developing a disease, condition or disorder in an individual who may be predisposed to the disease, condition or disorder but does not yet experience or display the pathology or symptomatology of the disease.

### Combination Therapies

Cancer cell growth and survival can be impacted by multiple signaling pathways. Thus, it is useful to combine different enzyme/protein/receptor inhibitors, exhibiting different preferences in the targets which they modulate the activities of, to treat such conditions. Targeting more than one signaling pathway (or more than one biological molecule involved in a given signaling pathway) may reduce the likelihood of drug-resistance arising in a cell population, and/or reduce the toxicity of treatment.

The compounds of the present disclosure can be used in combination with one or more other enzyme/protein/receptor inhibitors or one or more therapies for the treatment of diseases, such as cancer or infections. Examples of diseases and indications treatable with combination therapies include those as described herein. Examples of cancers include solid tumors and liquid tumors, such as blood cancers. Examples of infections include viral infections, bacterial infections, fungus infections or parasite infections. For example, the compounds of the present disclosure can be combined with one or more inhibitors of the following kinases for the treatment of cancer: Akt1, Akt2, Akt3, TGF-βP, PKA, PKG, PKC, CaM-kinase, phosphorylase kinase, MEKK, ERK, MAPK, mTOR, EGFR, HER2, HER3, HER4, INS-R, IGF-1R, IR-R, PDGFαR, PDGFβR, PI3K (alpha, beta, gamma, delta), CSFIR, KIT, FLK-II, KDR/FLK-1, FLK-4, flt-1, FGFR1, FGFR2, FGFR3, FGFR4, c-Met, Ron, Sea, TRKA, TRKB, TRKC, TAM kinases (Axl, Mer, Tyro3), FLT3, VEGFR/Flt2, Flt4, EphA1, EphA2, EphA3, EphB2, EphB4, Tie2, Src, Fyn, Lck, Fgr, Btk, Fak, SYK, FRK, JAK, ABL, ALK and B-Raf. In some embodiments, the compounds of the present disclosure can be combined with one or more of the following inhibitors for the treatment of cancer or infections. Non-limiting examples of inhibitors that can be combined with the compounds of the present disclosure for treatment of cancer and infections include an FGFR inhibitor (FGFR1, FGFR2, FGFR3 or FGFR4, e.g., INCB54828, INCB62079 and INCB63904), a JAK inhibitor (JAK1 and/or JAK2, e.g., ruxolitinib, baricitinib or INCB39110), an IDO inhibitor (e.g., epacadostat, NLG919, BMS-986205), an LSD1 inhibitor (e.g., INCB59872 and INCB60003), a TDO inhibitor, a PI3K-delta inhibitor (e.g., INCB50797 and INCB50465), a PI3K-gamma inhibitor such as PI3K-gamma selective inhibitor, a Pim inhibitor (e.g., INCB53914), a CSF1R inhibitor, a TAM receptor tyrosine kinases (Tyro-3, Axl, and Mer), an adenosine receptor antagonist (e.g., A2a/A2b receptor antagonist), an HPK1 inhibitor, an histone deacetylase inhibitor (HDAC) such as an HDAC8 inhibitor, an angiogenesis inhibitor, an interleukin receptor inhibitor, bromo and extra terminal family members inhibitors (for example, bromodomain inhibitors or BET inhibitors such as INCB54329 and INCB57643), a poly ADP ribose polymerase (PARP) inhibitor such as rucaparib, olaparib, niraparib, veliparib, or talazoparib, an arginase inhibitor (INCB01158), and an adenosine receptor antagonist or combinations thereof.

Compounds of the present disclosure can be used in combination with one or more immune checkpoint inhibitors. Exemplary immune checkpoint inhibitors include inhibitors against immune checkpoint molecules such as CD27, CD28, CD40, CD122, CD96, CD73, CD47, OX40, GITR, CSF1R, JAK, PI3K delta, PI3K gamma, TAM, arginase, CD137 (also known as 4-1BB), ICOS, A2AR, B7-H3, B7-H4, BTLA, CTLA-4, LAG3, TIM3, VISTA, PD-1, PD-L1 and PD-L2. In some embodiments, the immune checkpoint molecule is a stimulatory checkpoint molecule selected from CD27, CD28, CD40, ICOS, OX40, GITR and CD137. In some embodiments, the immune checkpoint molecule is an inhibitory checkpoint molecule selected from A2AR, B7-H3, B7-H4, BTLA, CTLA-4, IDO, KIR, LAG3, PD-1, TIM3, and VISTA. In some embodiments, the compounds provided herein can be used in combination with one or more agents selected from KIR inhibitors, TIGIT inhibitors, LAIR1 inhibitors, CD160 inhibitors, 2B4 inhibitors and TGFR beta inhibitors.

In some embodiments, the inhibitor of an immune checkpoint molecule is anti-PDl antibody, anti-PD-Ll antibody, or anti-CTLA-4 antibody.

In some embodiments, the inhibitor of an immune checkpoint molecule is an inhibitor of PD-1, e.g., an anti-PD-1 monoclonal antibody. In some embodiments, the anti-PD-1 monoclonal antibody is nivolumab, pembrolizumab (also known as MK-3475), pidilizumab, SHR-1210, PDR001, or AMP-224. In some embodiments, the anti-PD-1 monoclonal antibody is nivolumab or pembrolizumab. In some embodiments, the anti-PDl antibody is pembrolizumab. In some embodiments, the anti PD-1 antibody is SHR-1210.

In some embodiments, the inhibitor of an immune checkpoint molecule is an inhibitor of PD-L1, e.g., an anti-PD-Ll monoclonal antibody. In some embodiments, the anti-PD-Ll monoclonal antibody is BMS-935559, MEDI4736, MPDL3280A (also known as RG7446), or MSB0010718C. In some embodiments, the anti-PD-Ll monoclonal antibody is MPDL3280A or MEDI4736.

In some embodiments, the inhibitor of an immune checkpoint molecule is an inhibitor of CTLA-4, e.g., an anti-CTLA-4 antibody. In some embodiments, the anti-CTLA-4 antibody is ipilimumab or tremelimumab.

In some embodiments, the inhibitor of an immune checkpoint molecule is an inhibitor of LAG3, e.g., an anti-LAG3 antibody. In some embodiments, the anti-LAG3 antibody is BMS-986016, LAG525 or INCAGN2385.

In some embodiments, the inhibitor of an immune checkpoint molecule is an inhibitor of TIM3, e.g., an anti-TIM3 antibody. In some embodiments, the anti-TIM3 antibody is INCAGN2390, MBG453, or TSR-022.

In some embodiments, the inhibitor of an immune checkpoint molecule is an inhibitor of GITR, e.g., an anti-GITR antibody. In some embodiments, the anti-GITR antibody is TRX518, MK-4166, INCAGN1876, MK-1248, AMG228, BMS-986156, GWN323, or MEDI1873.

In some embodiments, the inhibitor of an immune checkpoint molecule is an inhibitor of OX40, e.g., an anti-OX40 antibody or OX40L fusion protein. In some embodiments, the anti-OX40 antibody is MEDI0562, MOXR-0916, PF-04518600, GSK3174998, or BMS-986178. In some embodiments, the OX40L fusion protein is MEDI6383.

Compounds of the present disclosure can be used in combination with one or more agents for the treatment of diseases such as cancer. In some embodiments, the agent is an alkylating agent, a proteasome inhibitor, a corticosteroid, or an immunomodulatory agent. Examples of an alkylating agent include cyclophosphamide (CY), melphalan (MEL), and bendamustine. In some embodiments, the proteasome inhibitor is carfilzomib. In some embodiments, the corticosteroid is dexamethasone (DEX). In some embodiments, the immunomodulatory agent is lenalidomide (LEN) or pomalidomide (POM).

The compounds of the present disclosure can further be used in combination with other methods of treating cancers, for example by chemotherapy, irradiation therapy, tumor-targeted therapy, adjuvant therapy, immunotherapy or surgery. Examples of immunotherapy include cytokine treatment (e.g., interferons, GM-CSF, G-CSF, IL-2), CRS-207 immunotherapy, cancer vaccine, monoclonal antibody, adoptive T cell transfer, Toll receptor agonists, STING agonists, oncolytic virotherapy and immunomodulating small molecules, including thalidomide or JAK1/2 inhibitor and the like. The compounds can be administered in combination with one or more anti-cancer drugs, such as a chemotherapeutics. Example chemotherapeutics include any of: abarelix, aldesleukin, alemtuzumab, alitretinoin, allopurinol, altretamine, anastrozole, arsenic trioxide, asparaginase, azacitidine, bevacizumab, bexarotene, baricitinib, bleomycin, bortezombi, bortezomib, busulfan intravenous, busulfan oral, calusterone, capecitabine, carboplatin, carmustine, cetuximab, chlorambucil, cisplatin, cladribine, clofarabine, cyclophosphamide, cytarabine, dacarbazine, dactinomycin, dalteparin sodium, dasatinib, daunorubicin, decitabine, denileukin, denileukin diftitox, dexrazoxane, docetaxel, doxorubicin, dromostanolone propionate, eculizumab, epirubicin, erlotinib, estramustine, etoposide phosphate, etoposide, exemestane, fentanyl citrate, filgrastim, floxuridine, fludarabine, fluorouracil, fulvestrant, gefitinib, gemcitabine, gemtuzumab ozogamicin, goserelin acetate, histrelin acetate, ibritumomab tiuxetan, idarubicin, ifosfamide, imatinib mesylate, interferon alfa 2a, irinotecan, lapatinib ditosylate, lenalidomide, letrozole, leucovorin, leuprolide acetate, levamisole, lomustine, meclorethamine, megestrol acetate, melphalan, mercaptopurine, methotrexate, methoxsalen, mitomycin C, mitotane, mitoxantrone, nandrolone phenpropionate, nelarabine, nofetumomab, olaparib, oxaliplatin, paclitaxel, pamidronate, panitumumab, pegaspargase, pegfilgrastim, pemetrexed disodium, pentostatin, pipobroman, plicamycin, procarbazine, quinacrine, rasburicase, rituximab, ruxolitinib, rucaparib, sorafenib, streptozocin, sunitinib, sunitinib maleate, tamoxifen, temozolomide, teniposide, testolactone, thalidomide, thioguanine, thiotepa, topotecan, toremifene, tositumomab, trastuzumab, tretinoin, uracil mustard, valrubicin, vinblastine, vincristine, vinorelbine, vorinostat, niraparib, veliparib, talazoparib and zoledronate.

Other anti-cancer agent(s) include antibody therapeutics such as trastuzumab (Herceptin), antibodies to costimulatory molecules such as CTLA-4 (e.g., ipilimumab), 4-1BB (e.g. urelumab, utomilumab), antibodies to PD-1 and PD-L1, or antibodies to cytokines (IL-10, TGF-β, etc.). Examples of antibodies to PD-1 and/or PD-L1 that can be combined with compounds of the present disclosure for the treatment of cancer or infections such as viral, bacteria, fungus and parasite infections include, but are not limited to, nivolumab, pembrolizumab, MPDL3280A, MEDI-4736 and SHR-1210.

In some embodiments, the anti-cancer agent is an alkylating agent, a proteasome inhibitor, a corticosteroid, or an immunomodulatory agent. Examples of an alkylating agent include cyclophosphamide (CY), melphalan (MEL), and bendamustine. In some embodiments, the proteasome inhibitor is carfilzomib. In some embodiments, the corticosteroid is dexamethasone (DEX). In some embodiments, the immunomodulatory agent is lenalidomide (LEN) or pomalidomide (POM).

The compounds of Formula (I) or any of the formulas as described herein, a compound as recited in any of the claims and described herein, or salts thereof can be used in combination with an immune checkpoint inhibitor for the treatment of cancer and viral infections.

Exemplary immune checkpoint inhibitors include inhibitors against immune checkpoint molecules such as CD27, CD28, CD40, CD122, CD96, CD73, CD47, OX40, GITR, CSF1R, JAK, PI3K delta, PI3K gamma, TAM, arginase, CD137 (also known as 4-1BB), ICOS, A2AR, B7-H3, B7-H4, BTLA, CTLA-4, LAG3, TIM3, VISTA, PD-1, PD-L1 and PD-L2. In some embodiments, the immune checkpoint molecule is a stimulatory checkpoint molecule selected from CD27, CD28, CD40, ICOS, OX40, GITR and CD137. In some embodiments, the immune checkpoint molecule is an inhibitory checkpoint molecule selected from A2AR, B7-H3, B7-H4, BTLA, CTLA-4, IDO, KIR, LAG3, PD-1, TIM3, and VISTA. In some embodiments, the compounds provided herein can be used in combination with one or more agents selected from KIR inhibitors, TIGIT inhibitors, LAIR1 inhibitors, CD160 inhibitors, 2B4 inhibitors and TGFR beta inhibitors.

In some embodiments, the inhibitor of an immune checkpoint molecule is anti-PDl antibody, anti-PD-Ll antibody, or anti-CTLA-4 antibody.

In some embodiments, the inhibitor of an immune checkpoint molecule is an inhibitor of PD-1, e.g., an anti-PD-1 monoclonal antibody. In some embodiments, the anti-PD-1 monoclonal antibody is nivolumab, pembrolizumab (also known as MK-3475), pidilizumab, SHR-1210, PDR001, or AMP-224. In some embodiments, the anti-PD-1 monoclonal antibody is nivolumab or pembrolizumab. In some embodiments, the anti-PDl antibody is pembrolizumab.

In some embodiments, the inhibitor of an immune checkpoint molecule is an inhibitor of PD-L1, e.g., an anti-PD-L1 monoclonal antibody. In some embodiments, the anti-PD-Ll monoclonal antibody is BMS-935559, MEDI4736, MPDL3280A (also known as RG7446), or MSB0010718C. In some embodiments, the anti-PD-Ll monoclonal antibody is MPDL3280A or MEDI4736.

In some embodiments, the inhibitor of an immune checkpoint molecule is an inhibitor of CTLA-4, e.g., an anti-CTLA-4 antibody. In some embodiments, the anti-CTLA-4 antibody is ipilimumab.

In some embodiments, the inhibitor of an immune checkpoint molecule is an inhibitor of LAG3, e.g., an anti-LAG3 antibody. In some embodiments, the anti-LAG3 antibody is BMS-986016 or LAG525.

In some embodiments, the inhibitor of an immune checkpoint molecule is an inhibitor of GITR, e.g., an anti-GITR antibody. In some embodiments, the anti-GITR antibody is TRX518 or MK-4166.

In some embodiments, the inhibitor of an immune checkpoint molecule is an inhibitor of OX40, e.g., an anti-OX40 antibody or OX40L fusion protein. In some embodiments, the anti-OX40 antibody is MEDI0562. In some embodiments, the OX40L fusion protein is MED16383.

The compounds of the present disclosure can further be used in combination with one or more anti-inflammatory agents, steroids, immunosuppressants or therapeutic antibodies.

The compounds of Formula (I) or any of the formulas as described herein, a compound as recited in any of the claims and described herein, or salts thereof can be combined with another immunogenic agent, such as cancerous cells, purified tumor antigens (including recombinant proteins, peptides, and carbohydrate molecules), cells, and cells transfected with genes encoding immune stimulating cytokines. Non-limiting examples of tumor vaccines that can be used include peptides of melanoma antigens, such as peptides of gp100, MAGE antigens, Trp-2, MARTI and/or tyrosinase, or tumor cells transfected to express the cytokine GM-CSF.

The compounds of Formula (I) or any of the formulas as described herein, a compound as recited in any of the claims and described herein, or salts thereof can be used in combination with a vaccination protocol for the treatment of cancer. In some embodiments, the tumor cells are transduced to express GM-CSF. In some embodiments, tumor vaccines include the proteins from viruses implicated in human cancers such as Human Papilloma Viruses (HPV), Hepatitis Viruses (HBV and HCV) and Kaposi's Herpes Sarcoma Virus (KHSV). In some embodiments, the compounds of the present disclosure can be used in combination with tumor specific antigen such as heat shock proteins isolated from tumor tissue itself. In some embodiments, the compounds of Formula (I) or any of the formulas as described herein, a compound as recited in any of the claims and described herein, or salts thereof can be combined with dendritic cells immunization to activate potent anti-tumor responses.

The compounds of the present disclosure can be used in combination with bispecific macrocyclic peptides that target Fe alpha or Fe gamma receptor-expressing effectors cells to tumor cells. The compounds of the present disclosure can also be combined with macrocyclic peptides that activate host immune responsiveness.

The compounds of the present disclosure can be used in combination with bone marrow transplant for the treatment of a variety of tumors of hematopoietic origin.

The compounds of Formula (I) or any of the formulas as described herein, a compound as recited in any of the claims and described herein, or salts thereof can be used in combination with vaccines, to stimulate the immune response to pathogens, toxins, and self antigens. Examples of pathogens for which this therapeutic approach may be particularly useful, include pathogens for which there is currently no effective vaccine, or pathogens for which conventional vaccines are less than completely effective. These include, but are not limited to, HIV, Hepatitis (A, B, & C), Influenza, Herpes, Giardia, Malaria, Leishmania, Staphylococcus aureus, Pseudomonas Aeruginosa.

Viruses causing infections treatable by compounds and compositions of the present disclosure include, but are not limit to human papillomavirus, influenza, hepatitis A, B, C or D viruses, adenovirus, poxvirus, herpes simplex viruses, human cytomegalovirus, severe acute respiratory syndrome virus, ebola virus, measles virus, herpes virus (e.g., VZV, HSV-1, HAV-6, HSV-II, and CMV, Epstein Barr virus), flaviviruses, echovirus, rhinovirus, coxsackie virus, cornovirus, respiratory syncytial virus, mumpsvirus, rotavirus, measles virus, rubella virus, parvovirus, vaccinia virus, HTLV virus, dengue virus, papillomavirus, molluscum virus, poliovirus, rabies virus, JC virus and arboviral encephalitis virus.

Pathogenic bacteria causing infections treatable by compounds and compositions of the disclosure include, but are not limited to, chlamydia, rickettsial bacteria, mycobacteria, staphylococci, streptococci, pneumonococci, meningococci and conococci, klebsiella, proteus, serratia, pseudomonas, legionella, diphtheria, salmonella, bacilli, cholera, tetanus, botulism, anthrax, plague, leptospirosis, and Lyme's disease bacteria.

Pathogenic fungi causing infections treatable by compounds and compositions of the disclosure include, but are not limited to, Candida (albicans, krusei, glabrata, tropicalis, etc.), Cryptococcus neoformans, Aspergillus (fumigatus, niger, etc.), Genus Mucorales (mucor, absidia, rhizophus), Sporothrix schenkii, Blastomyces dermatitidis, Paracoccidioides brasiliensis, Coccidioides immitis and Histoplasma capsulatum.

Pathogenic parasites causing infections treatable by compounds and compositions of the disclosure include, but are not limited to, Entamoeba histolytica, Balantidium coli, Naegleriafowleri, Acanthamoeba sp., Giardia lambia, Cryptosporidium sp., Pneumocystis carinii, Plasmodium vivax, Babesia microti, Trypanosoma brucei, Trypanosoma cruzi, Leishmania donovani, Toxoplasma gondi, and Nippostrongylus brasiliensis.

When more than one pharmaceutical agent is administered to a patient, they can be administered simultaneously, separately, sequentially, or in combination (e.g., for more than two agents).

### IV. Formulation, Dosage Forms and Administration

When employed as pharmaceuticals, the compounds of the present disclosure can be administered in the form of pharmaceutical compositions. Thus the present disclosure provides a composition comprising a compound of Formula (I) or any of the formulas as described herein, a compound as recited in any of the claims and described herein, or a pharmaceutically acceptable salt thereof, or any of the embodiments thereof, and at least one pharmaceutically acceptable carrier or excipient. These compositions can be prepared in a manner well known in the pharmaceutical art, and can be administered by a variety of routes, depending upon whether local or systemic treatment is indicated and upon the area to be treated. Administration may be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or may be, *e.g.,* by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

This invention also includes pharmaceutical compositions which contain, as the active ingredient, the compound of the present disclosure or a pharmaceutically acceptable salt thereof, in combination with one or more pharmaceutically acceptable carriers or excipients. In some embodiments, the composition is suitable for topical administration. In making the compositions of the invention, the active ingredient is typically mixed with an excipient, diluted by an excipient or enclosed within such a carrier in the form of, *e.g.,* a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, *e.g.,* up to 10% by weight of the active compound, soft and hard gelatin capsules, suppositories, sterile injectable solutions and sterile packaged powders.

In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g., about 40 mesh.

The compounds of the invention may be milled using known milling procedures such as wet milling to obtain a particle size appropriate for tablet formation and for other formulation types. Finely divided (nanoparticulate) preparations of the compounds of the invention can be prepared by processes known in the art see, *e.g.,* WO 2002/000196.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup and methyl cellulose. The formulations can additionally include: lubricating agents such as talc, magnesium stearate and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

In some embodiments, the pharmaceutical composition comprises silicified microcrystalline cellulose (SMCC) and at least one compound described herein, or a pharmaceutically acceptable salt thereof. In some embodiments, the silicified microcrystalline cellulose comprises about 98% microcrystalline cellulose and about 2% silicon dioxide w/w.

In some embodiments, the composition is a sustained release composition comprising at least one compound described herein, or a pharmaceutically acceptable salt thereof, and at least one pharmaceutically acceptable carrier or excipient. In some embodiments, the composition comprises at least one compound described herein, or a pharmaceutically acceptable salt thereof, and at least one component selected from microcrystalline cellulose, lactose monohydrate, hydroxypropyl methylcellulose and polyethylene oxide. In some embodiments, the composition comprises at least one compound described herein, or a pharmaceutically acceptable salt thereof, and microcrystalline cellulose, lactose monohydrate and hydroxypropyl methylcellulose. In some embodiments, the composition comprises at least one compound described herein, or a pharmaceutically acceptable salt thereof, and microcrystalline cellulose, lactose monohydrate and polyethylene oxide. In some embodiments, the composition further comprises magnesium stearate or silicon dioxide. In some embodiments, the microcrystalline cellulose is Avicel PH102^{™}. In some embodiments, the lactose monohydrate is Fast-flo 316^{™}. In some embodiments, the hydroxypropyl methylcellulose is hydroxypropyl methylcellulose 2208 K4M (e.g., Methocel K4 M Premier^{™}) and/or hydroxypropyl methylcellulose 2208 K100LV (e.g., Methocel K00LV^{™}). In some embodiments, the polyethylene oxide is polyethylene oxide WSR 1105 (*e.g.*, Polyox WSR 1105^{™}).

In some embodiments, a wet granulation process is used to produce the composition. In some embodiments, a dry granulation process is used to produce the composition.

The compositions can be formulated in a unit dosage form, each dosage containing from about 5 to about 1,000 mg (1 g), more usually about 100 mg to about 500 mg, of the active ingredient. In some embodiments, each dosage contains about 10 mg of the active ingredient. In some embodiments, each dosage contains about 50 mg of the active ingredient. In some embodiments, each dosage contains about 25 mg of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The components used to formulate the pharmaceutical compositions are of high purity and are substantially free of potentially harmful contaminants (e.g., at least National Food grade, generally at least analytical grade, and more typically at least pharmaceutical grade). Particularly for human consumption, the composition is preferably manufactured or formulated under Good Manufacturing Practice standards as defined in the applicable regulations of the U.S. Food and Drug Administration. For example, suitable formulations may be sterile and/or substantially isotonic and/or in full compliance with all Good Manufacturing Practice regulations of the U.S. Food and Drug Administration.

The active compound may be effective over a wide dosage range and is generally administered in a therapeutically effective amount. It will be understood, however, that the amount of the compound actually administered will usually be determined by a physician, according to the relevant circumstances, including the condition to be treated, the chosen route of administration, the actual compound administered, the age, weight, and response of the individual patient, the severity of the patient's symptoms and the like.

The therapeutic dosage of a compound of the present invention can vary according to, *e.g.,* the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (e.g., hydrophobicity), and the route of administration. For example, the compounds of the invention can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from in vitro or animal model test systems.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, e.g., about 0.1 to about 1000 mg of the active ingredient of the present invention.

The tablets or pills of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compounds and compositions of the present invention can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face mask, tent, or intermittent positive pressure breathing machine. Solution, suspension, or powder compositions can be administered orally or nasally from devices which deliver the formulation in an appropriate manner.

Topical formulations can contain one or more conventional carriers. In some embodiments, ointments can contain water and one or more hydrophobic carriers selected from, e.g., liquid paraffin, polyoxyethylene alkyl ether, propylene glycol, white Vaseline, and the like. Carrier compositions of creams can be based on water in combination with glycerol and one or more other components, e.g., glycerinemonostearate, PEG-glycerinemonostearate and cetylstearyl alcohol. Gels can be formulated using isopropyl alcohol and water, suitably in combination with other components such as, e.g., glycerol, hydroxyethyl cellulose, and the like. In some embodiments, topical formulations contain at least about 0.1, at least about 0.25, at least about 0.5, at least about 1, at least about 2 or at least about 5 wt % of the compound of the invention. The topical formulations can be suitably packaged in tubes of, *e.g.,* 100 g which are optionally associated with instructions for the treatment of the select indication, e.g., psoriasis or other skin condition.

The amount of compound or composition administered to a patient will vary depending upon what is being administered, the purpose of the administration, such as prophylaxis or therapy, the state of the patient, the manner of administration and the like. In therapeutic applications, compositions can be administered to a patient already suffering from a disease in an amount sufficient to cure or at least partially arrest the symptoms of the disease and its complications. Effective doses will depend on the disease condition being treated as well as by the judgment of the attending clinician depending upon factors such as the severity of the disease, the age, weight and general condition of the patient and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers or stabilizers will result in the formation of pharmaceutical salts.

The therapeutic dosage of a compound of the present invention can vary according to, *e.g.,* the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (e.g., hydrophobicity), and the route of administration. For example, the compounds of the invention can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or animal model test systems.

### V Labeled Compounds and Assay Methods

The compounds of the present disclosure can further be useful in investigations of biological processes in normal and abnormal tissues. Thus, another aspect of the present invention relates to labeled compounds of the invention (radio-labeled, fluorescent-labeled, *etc.*) that would be useful not only in imaging techniques but also in assays, both *in vitro* and *in vivo*, for localizing and quantitating PD-1 or PD-L1 protein in tissue samples, including human, and for identifying PD-L1 ligands by inhibition binding of a labeled compound. Accordingly, the present invention includes PD-1/PD-L1 binding assays that contain such labeled compounds.

The present invention further includes isotopically-substituted compounds of the disclosure. An "isotopically-substituted" compound is a compound of the invention where one or more atoms are replaced or substituted by an atom having the same atomic number but different atomic mass or mass number e.g., a different atomic mass or mass number from the atomic mass or mass number typically found in nature (*i.e.*, naturally occurring). It is to be understood that a "radio-labeled" compound is a compound that has incorporated at least one isotope that is radioactive (e.g., radionuclide). Suitable radionuclides that may be incorporated in compounds of the present invention include but are not limited to ³H (also written as T for tritium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I. The radionuclide that is incorporated in the instant radio-labeled compounds will depend on the specific application of that radio-labeled compound. For example, for *in vitro* PD-L1 protein labeling and competition assays, compounds that incorporate ³H, ¹⁴C, ⁸²Br, ¹²⁵I, ¹³¹I, ³⁵S or will generally be most useful. For radio-imaging applications ¹¹C, ¹⁸F, ¹²⁵I, ¹²³I, ¹²⁴I, ¹³¹I, ⁷⁵Br, ⁷⁶Br or ⁷⁷Br will generally be most useful.

It is understood that a "radio-labeled" or "labeled compound" is a compound that has incorporated at least one radionuclide. In some embodiments the radionuclide is selected from the group consisting of ³H, ¹⁴C, ¹²⁵I, ³⁵S and ⁸²Br. Synthetic methods for incorporating radio-isotopes into organic compounds are applicable to compounds provided herein and are well known in the art.

A radio-labeled compound of the invention can be used in a screening assay to identify and/or evaluate compounds. In gereranl terms, a newly synthesized or identified compound (*i*.*e*., test compound) which is labeled can be evaluated for its ability to bind a PD-L1 protein by monitoring its concentration variation when contacting with the PD-L1 protein, through tracking of the labeling. For example, a test compound (radio-labeled) can be evaluated for its ability to reduce binding of another compound which is known to bind to a PD-L1 protein (*i.e.*, standard compound). Accordingly, the ability of a test compound to compete with the standard compound for binding to the PD-L1 protein directly correlates to its binding affinity. Conversely, in some other screening assays, the standard compound is labeled and test compounds are unlabeled. Accordingly, the concentration of the labeled standard compound is monitored in order to evaluate the competition between the standard compound and the test compound, and the relative binding affinity of the test compound is thus ascertained.

### VI. Kits

The present disclosure also includes pharmaceutical kits useful, *e.g.,* in the treatment or prevention of diseases or disorders associated with the activity of PD-L1 including its interaction with other proteins such as PD-1 and B7-1 (CD80), such as cancer or infections, which include one or more containers containing a pharmaceutical composition comprising a therapeutically effective amount of a compound of Formula (I), or any of the embodiments thereof. Such kits can further include one or more of various conventional pharmaceutical kit components, such as, e.g., containers with one or more pharmaceutically acceptable carriers, additional containers, *etc.*, as will be readily apparent to those skilled in the art. Instructions, either as inserts or as labels, indicating quantities of the components to be administered, guidelines for administration, and/or guidelines for mixing the components, can also be included in the kit.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results. The compounds of the Examples have been found to inhibit the activity of PD-1/PD-L1 protein/protein interaction according to at least one assay described herein.

### EXAMPLES

Experimental procedures for compounds of the invention are provided below. Open Access Preparative LCMS Purification of some of the compounds prepared was performed on Waters mass directed fractionation systems. The basic equipment setup, protocols and control software for the operation of these systems have been described in detail in literature. *See*, *e.g.*, Blom, "Two-Pump At Column Dilution Configuration for Preparative LC-MS", K. Blom, J. Combi. Chem., 2002, 4, 295-301; Blom et al., "Optimizing Preparative LC-MS Configurations and Methods for Parallel Synthesis Purification", J. Combi. Chem., 2003, 5, 670-83; and Blom et al., "Preparative LC-MS Purification: Improved Compound Specific Method Optimization", J. Combi. Chem., 2004, 6, 874-883.

### Example 1: 2-(((8-((2-chloro-2'-methyl-3'-(4,5,6,7-tetrahydrothiazolo[5,4-c]pyridin-2-yl)-[1,1'-biphenyl]-3-yl)amino)-1,7-naphthyridin-3-yl)methyl)amino)ethan-1-ol

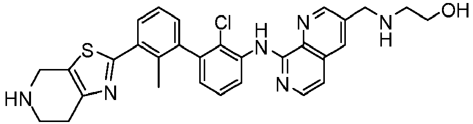

### Step 1: 8-chloro-3-vinyl-1,7-naphthyridine

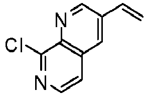

A mixture of 3-bromo-8-chloro-1,7-naphthyridine (PharmaBlock, cat#PBLJ2743: 0.200 g, 0.821 mmol), 4,4,5,5-tetramethyl-2-vinyl-1,3,2-dioxaborolane (Aldrich, cat#663348: 153 µL, 0.904 mmol), sodium carbonate (0.174 g, 1.64 mmol) and [1,1'-bis(dicyclohexylphosphino)ferrocene]dichloropalladium(II) (Aldrich, cat#701998: 6.2 mg, 0.0082 mmol) in tert-butyl alcohol (5.91 mL, 61.8 mmol) and water (6 mL, 300 mmol) was degassed and sealed. It was stirred at 110 °C for 2 h. The reaction mixture was cooled then extracted with ethyl acetate (3x 20 mL). The combined organic layers were washed with brine, dried over MgSO₄, filtered and concentrated under reduced pressure. The crude residue was used directly in the next step without further purification. LC-MS calculated for C₁₀H₈ClN₂ (M+H)⁺: m/z = 191.0; found 191.0.

### Step 2: 8-chloro-1,7-naphthyridine-3-carbaldehyde

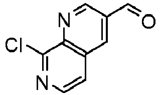

A flask was charged with 8-chloro-3-vinyl-1,7-naphthyridine (391. mg, 2.05 mmol), 1,4-dioxane (40. mL), a stir bar and water (40. mL). To this suspension was added a 4% w/w mixture of osmium tetraoxide in water (0.84 mL, 0.132 mmol). The reaction was stirred for 5 min then sodium periodate (3.23 g, 15.11 mmol) was added and stirred for 3 h. The mixture was diluted with water (20 mL) and EtOAc (20 mL). The layers were separated and the aqueous layer was further extracted with EtOAc (2 X 20 mL). The combined organic extracts were washed with brine, dried over sodium sulfate, filtered, and concentrated in vacuo. The crude aldehyde was purified by silica gel chromatography (0 → 60% EtOAc/hexanes). LC-MS calculated for C₉H₆ClN₂O (M+H)⁺: m/z = 193.0; found 192.9.

### Step 3: 2-{[(8-chloro-1,7-naphthyridin-3-yl)methyl]amino}ethanol

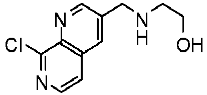

A mixture of 8-chloro-1,7-naphthyridine-3-carbaldehyde (0.160 g, 0.831 mmol) and ethanolamine (Aldrich, cat#398136: 251 µL, 4.15 mmol) in methylene chloride (6 mL, 100 mmol) and N,N-diisopropylethylamine (868 µL, 4.98 mmol) was stirred at rt for 1 h. Sodium triacetoxyborohydride (0.528 g, 2.49 mmol) was carefully added in portions. The reaction was stirred at rt for 2 h. To the mixture was then carefully added sodium tetrahydroborate (157 mg, 4.15 mmol) and methanol (1 mL) and the reaction mixture was stirred overnight under nitrogen. The reaction was quenched with a saturated aqueous solution of sodium bicarbonate. The mixture was then extracted with a 3:1 mixture of chlorofom/isopropyl alcohol. The combined organic layers were washed with brine, dried over sodium sulfate, then concentrated in vacuo. The crude residue was purified by column chromatography (0 → 50% methanol/DCM) and was obtained as an off white solid. LC-MS calculated for C₁₁H₁₃ClN₃O (M+H)⁺: m/z = 238.1; found 238.1.

### Step 4: 2-(((8-((3-bromo-2-chlorophenyl)amino)-1,7-naphthyridin-3-yl)methyl)amino)ethan-1-ol

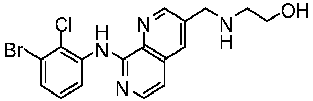

To a vial was added 3-bromo-2-chloroaniline (Enamine, cat# EN300-105778: 0.021 g, 0.101 mmol) and 2-(((8-chloro-1,7-naphthyridin-3-yl)methyl)amino)ethan-1-ol (0.020 g, 0.084 mmol). The solids were suspended in isopropanol (0.421 ml). Sulfuric acid (4.48 µl, 0.084 mmol) was added to the reaction mixture and then heated to 100 °C for 1 h. After cooling, the mixture was quenched with a saturated aqueous sodium bicarbonate solution, and extracted with 3:1 chloroform/isopropyl alcohol. The combined organic extracts were dried over magnesium sulfate, filtered, and concentrated in vacuo. The crude residue was purified using silica gel chromatography (1:1 DCM/MeOH) to afford a yellow solid. LC-MS calculated for C₁₇H₁₇BrClN₄O (M+H)⁺: m/z = 407.0; found 407.2.

### Step 5: tert-butyl 2-(3-chloro-2-methylphenyl)-6,7-dihydrothiazolo[5,4-c]pyridine-5(4H)-carboxylate

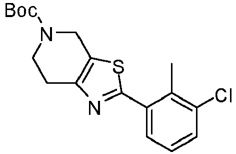

To a vial was added (3-chloro-2-methylphenyl)boronic acid (Combi-blocks, cat#BB-2035: 640 mg, 3.76 mmol), tert-butyl 2-bromo-6,7-dihydro[1,3]thiazolo[5,4-c]pyridine-5(4H)-carboxylate (AstaTech, cat#AB1021: 1000. mg, 3.133 mmol), sodium carbonate (996 mg, 9.40 mmol), tert-butyl alcohol (160 mmol), water (600 mmol) [1,1'-bis(di-cyclohexylphosphino)ferrocene]dichloropalladium(II) (Aldrich, cat#701998: 240 mg, 0.31 mmol). The mixture was sparged with nitrogen, then heated at 105°C for 1.5 h. The mixture was concentrated, dissolved with DCM, and purified using silica gel chromatography (40% EtOAc/hexanes). LC-MS calculated for C₁₈H₂₂ClN₂O₂S (M+H)⁺: m/z = 365.1; found 365.1.

### Step 6: tert-butyl 2-(2-methyl-3-(4,4,5,5-tetramethyl-1,3,2-dioxaborolan-2-yl)phenyl)-6, 7-dihydrothiazolo[-5,4-c]pyridine-5(4H)-carboxylate

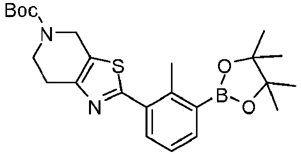

A mixture of tert-butyl 2-(3-chloro-2-methylphenyl)-6,7-dihydro[1,3]thiazolo[5,4-c]pyridine-5(4H)-carboxylate (261 mg, 0.715 mmol), 4,4,5,5,4',4',5',5'-octamethyl-[2,2']bi[[1,3,2]dioxaborolanyl] (Aldrich, cat#473294: 545 mg, 2.14 mmol), palladium acetate (6.42 mg, 0.0286 mmol), K₃PO₄ (455 mg, 2.14 mmol) and 2-(dicyclohexylphosphino)-2',6'-dimethoxy-1,1'-biphenyl (Strem Chemicals, cat#15-1143: 29.4 mg, 0.0715 mmol) in 1,4-Dioxane was degassed and stirred at rt for 16 h. The mixture was diluted with DCM, and washed with water. The organic layer was concentrated in vacuo and purified by silica-gel chromatography (5% EtOAc/DCM). LC-MS calculated for C₂₄H₃₄BN₂O₄S (M+H)⁺: m/z = 457.2; found 457.3.

### Step 7: tert-butyl 2-(2'-chloro-3'-(3-((2-hydroxyethylamino)methyl)-1,7-naphthyridin-8-ylamino)-2-methylbiphenyl-3-yl)-6,7-dihydrothiazolo[-5,4-c]pyridine--5(4H)-carboxylate

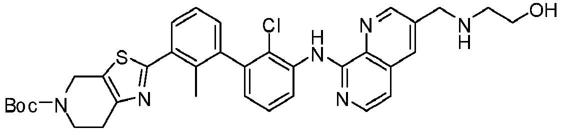

To a vial was added tert-butyl 2-(2-methyl-3-(4,4,5,5-tetramethyl-1,3,2-dioxaborolan-2-yl)phenyl)-6,7-dihydrothiazolo[5,4-c]pyridine-5(4H)-carboxylate (0.013 g, 0.029 mmol), 2-(((8-((3-bromo-2-chlorophenyl)amino)-1,7-naphthyridin-3-yl)methyl)amino)ethan-1-ol (0.008 g, 0.020 mmol), sodium carbonate (6.24 mg, 0.059 mmol), [1,1'-bis(diphenylphosphino)ferrocene]dichloropalladium(II) (1.436 mg, 1.962 µmol), 1,4-dioxane (0.346 ml), and water (0.046 ml). The mixture was degassed, sealed, and heated to 90 °C whilst stirring for 4 h. After cooling, the mixture was diluted with DCM and water. The layers were separated and the aqueous layer was further extracted. The combined organic layers were dried over magnesium sulfate, filtered, concentrated in vacuo, and purified by silica gel chromatography (MeOH/DCM). LC-MS calculated for C₃₅H₃₈ClN₆O₃S (M+H)⁺: m/z = 657.2; found 657.5.

### Step 8: 2-(((8-((2-chloro-2'-methyl-3'-(4,5,6,7-tetrahydrothiazolo[5,4-c]pyridin-2-yl)-[1,1'-biphenyl]-3-yl)amino)-1,7-naphthyridin-3-yl)methyl)amino)ethan-1-ol

A vial was charged with tert-butyl 2-(2'-chloro-3'-(3-((2-hydroxyethylamino)methyl)-1,7-naphthyridin-8-ylamino)-2-methylbiphenyl-3-yl)-6,7-dihydrothiazolo[5,4-c]pyridine-5(4H)-carboxylate (13 mg, 0.020 mmol), DCM (0.4 mL) and TFA (0.010 mL, 1 mmol). The resulting mixture was stirred for 1 h, open to air. The mixture was then dissolved in MeOH and purified by prep HPLC (pH = 2, acetonitrile/water+TFA) to provide the compound as the TFA salt. LC-MS calculated for C₃₀H₃₀ClN₆OS (M+H)⁺: m/z = 557.2; found 557.3.

### Example 2: 1-(((6-(2-fluoro-3'-(3-((2-hydroxyethylamino)methyl)-1,7-naphthytidin-8-ylamino)-2'-methylbiphenyl-3-ylcarbamoyl)pyridin-3-yl)methyl)amino)cyclobutanecarboxylic acid

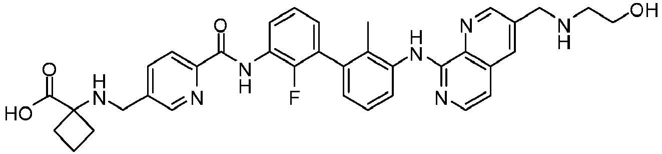

### Step 1: 2-(((8-((3-bromo-2-methylphenyl)amino)-1,7-naphthyridin-3-yl)methyl)amino)ethan-1-ol

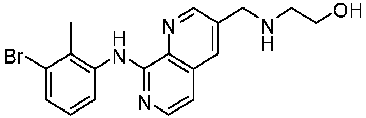

This compound was prepared using a similar procedure as described for Example 1, Step 4 with 3-bromo-2-methylaniline (Aldrich, cat#530018) replacing 3-bromo-2-chloroaniline. The crude compound was purified using column chromatography (0 → 50% MeOH/DCM). LC-MS calculated for C₁₈H₂₀BrN₄O (M+H)⁺: m/z = 387.1; found 387.2.

### Step 2: 5-(dimethoxymethyl)-N-(2-fluoro-3-(4,4,5,5-tetramethyl-1,3,2-dioxaborolan-2-yl)phenyl)picolinamide

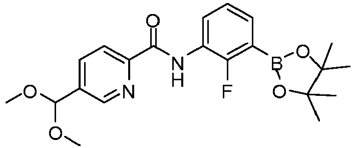

To a solution of 2-fluoro-3-(4,4,5,5-tetramethyl-1,3,2-dioxaborolan-2-yl)aniline (Combi-Blocks, cat#PN-5021: 200 mg, 0.844 mmol) and methyl 5-(dimethoxymethyl)picolinate (Combi-Blocks, cat#QY-1318: 196 mg, 0.928 mmol) in THF (8436 µl) was added 1.0 M potassium tert-butoxide in THF (1265 µl, 1.265 mmol) at rt. The mixture was stirred at rt for 2 h. Water and EtOAc were added, and the layers were separated. The aqueous layer was further extracted with ethyl acetate, and the combined organic layers were washed with brine, dried over magnesium sulfate, filtered, and concentrated in vacuo. The crude residue was purified using silica gel chromatography (30% EtOAc/hexanes). LC-MS calculated for C₂₁H₂₇BFN₂O₅ (M+H)⁺: m/z = 417.2; found 417.3.

### Step 3: 5-(dimethoxymethyl)-N-(2-fluoro-3'-((3-(((2-hydroxyethyl)amino)methyl)-1,7-naphthyridin-8-yl)amino)-2'-methyl-[1,1'-biphenyl]-3-yl)picolinamide

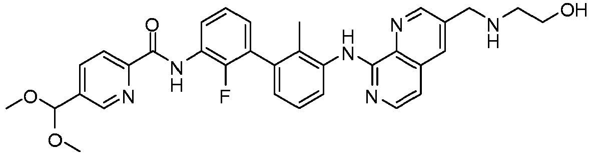

To a vial was added 5-(dimethoxymethyl)-N-(2-fluoro-3-(4,4,5,5-tetramethyl-1,3,2-dioxaborolan-2-yl)phenyl)picolinamide (0.161 g, 0.387 mmol), 2-(((8-((3-bromo-2-methylphenyl)amino)-1,7-naphthyridin-3-yl)methyl)amino)ethan-1-ol (0.10 g, 0.258 mmol), sodium carbonate (0.041 g, 0.387 mmol), [1,1'-bis(diphenylphosphino)ferrocene]dichloropalladium(II) (0.019 g, 0.026 mmol), 1,4-dioxane (4.56 ml), and water (0.608 ml). The mixture was degassed, sealed, and heated to 90 °C whilst stirring for 4 h. After cooling, the mixture was diluted with DCM and water, and the layers were separated. The aqueous layer was further extracted with DCM, and the combined organic layers were dried over magnesium sulfate, filtered, and concentrated in vacuo. The crude residue was purified by silica gel chromatography (20% MeOH/DCM) to provide the desired product. LC-MS calculated for C₃₃H₃₄FN₆O₄ (M+H)⁺: m/z = 597.3; found 597.2.

### Step 4: N-(2-fluoro-3'-((3-(((2-hydroxyethyl)amino)methyl)-1,7-naphthyridin-8-yl)amino)-2'-methyl-[1,1'-biphenyl]-3-yl)-5-formylpicolinamide

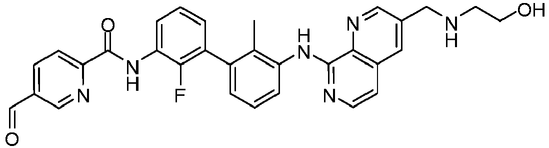

To a solution of 5-(dimethoxymethyl)-N-(2-fluoro-3'-((3-(((2-hydroxyethyl)amino)methyl)-1,7-naphthyridin-8-yl)amino)-2'-methyl-[1,1'-biphenyl]-3-yl)picolinamide (0.068 g, 0.114 mmol) in DCM (1.899 ml) was added TFA (0.439 ml, 5.70 mmol). The mixture was stirred at for 2 h. The mixture was concentrated and the residue was dissolved in DCM, and washed with a saturated aqueous NaHCO₃ solution. The layers were separated and the aqueous layer was further extracted with DCM. The combined organic layers were dried over magnesium sulfate, filtered and concentrated in vacuo. The crude product was used directly in the next step without further purification. LC-MS calculated for C₃₁H₂₈FN₆O₃ (M+H)⁺: m/z = 551.2; found 551.2.

### Step 5: 1-(((6-(2-fluoro-3'-(3-((2-hydroxyethylamino)methyl)-1,7-naphthyridin-8-ylamino)-2'-methylbiphenyl-3-ylcarbamoyl)pyridin-3-yl)methyl)amino)cyclobutanecarboxylic acid

To a vial was added N-(2-fluoro-3'-((3-(((2-hydroxyethyl)amino)methyl)-1,7-naphthyridin-8-yl)amino)-2'-methyl-[1,1'-biphenyl]-3-yl)-5-formylpicolinamide (0.030 g, 0.054 mmol), 1-aminocyclobutane-1-carboxylic acid (Aldrich, cat#652369: 0.019 g, 0.163 mmol), dichloromethane (0.893 ml) and triethylamine (0.016 ml, 0.115 mmol). The reaction was stirred at rt for 2 h, then sodium triacetoxyborohydride (0.058 g, 0.272 mmol) and acetic acid (9.36 µl, 0.163 mmol) were added. The reaction was stirred for 2 h, then the mixture was diluted with methanol and purified by prep HPLC (pH = 2, acetonitrile/water+TFA) to provide the desired compound as the TFA salt. LC-MS calculated for C₃₆H₃₇FN₇O₄ (M+H)⁺: m/z = 650.3; found 650.3

### Example 3: (S)-1-((6-((2-fluoro-3'-((3-(((2-hydroxyethyl)amino)methyl)-1,7-naphthyridin-8-yl)amino)-2'-methyl-[1,1'-biphenyl]-3-yl)carbamoyl)pyridin-3-yl)methyl)piperidine-2-carboxylic acid

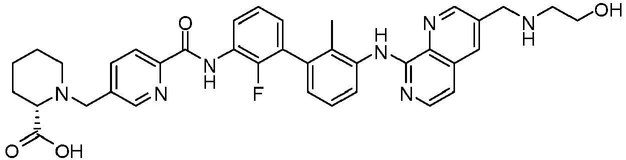

This compound was prepared using a similar procedure as described for Example 2, Step 5 with L-pipecolinic acid (Alfa Aesar, cat# L15373) replacing 1-aminocyclobutane-1-carboxylic acid. LC-MS calculated for C₃₇H₃₉FN₇O₄ (M+H)⁺: m/z = 664.3; found 664.3.

### Example 4: N-(2-fluoro-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamide

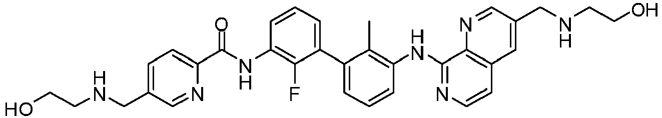

This compound was prepared using a similar procedure as described for Example 2, Step 5 with ethanolamine (Aldrich, cat# 398136) replacing 1-aminocyclobutane-1-carboxylic acid. LC-MS calculated for C₃₃H₃₅FN₇O₃ (M+H)⁺: m/z = 596.3; found 596.2.

### Example 5: N-(2-chloro-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamide

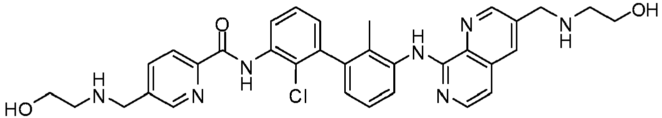

### Step 1: 2-chloro-3-(4,4, 5, 5-tetramethyl-1,3,2-dioxaborolan-2-yl)aniline

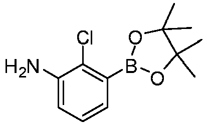

4,4,5,5,4',4',5',5'-Octamethyl-[2,2']bi[[1,3,2]dioxaborolanyl] (1.48 g, 5.81 mmol), potassium acetate (0.428 g, 4.36 mmol), 3-bromo-2-chloroaniline (Enamine, cat# EN300-105778: 0.300 g, 1.453 mmol), 1,4-dioxane (7.27 ml) and [1,1'-bis(diphenylphosphino)ferrocene] dichloropalladium (II) (0.053 g, 0.073 mmol) was stirred in a closed vial flushed with argon at 110 °C for 2 h. The mixture was cooled, diluted with EtOAc, and filtered over celite. The filtrate was concentrated and purified by silica gel chromatography (20% EtOAc/hexanes). LC-MS calculated for C₁₂H₁₈BClNO₂ (M+H)⁺: m/z = 254.1; found 254.1.

### Step 2: N-(2-chloro-3-(4,4,5,5-tetramethyl-1,3,2-dioxaborolan-2-yl)phenyl)-5-(dimethoxymethyl)picolinamide

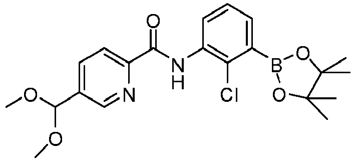

This compound was prepared using a similar procedure as described for Example 2, Step 2 with 2-chloro-3-(4,4,5,5-tetramethyl-1,3,2-dioxaborolan-2-yl)aniline replacing 2-fluoro-3-(4,4,5,5-tetramethyl-1,3,2-dioxaborolan-2-yl)aniline. LC-MS calculated for C₂₁H₂₇BClN₂O₅ (M+H)⁺: m/z = 433.2; found 433.1.

### Step 3: N-(2-chloro-3'-((3-(((2-hydroxyethyl)amino)methyl)-1,7-naphthyridin-8-yl)amino)-2'-methyl-[1,1'-biphenyl]-3-yl)-5-(dimethoxymethyl)picolinamide

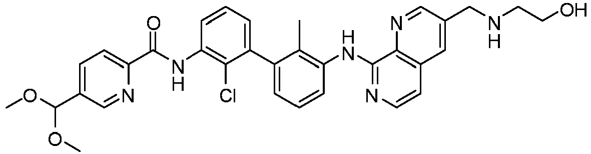

This compound was prepared using a similar procedure as described for Example 2, Step 3 with N-(2-chloro-3-(4,4,5,5-tetramethyl-1,3,2-dioxaborolan-2-yl)phenyl)-5-(dimethoxymethyl)picolinamide replacing 5-(dimethoxymethyl)-N-(2-fluoro-3-(4,4,5,5-tetramethyl-1,3,2-dioxaborolan-2-yl)phenyl)picolinamide. LC-MS calculated for C₃₃H₃₄ClN₆O₄ (M+H)⁺: m/z = 613.2; found 613.2.

### Step 4: N-(2-chloro-3'-((3-(((2-hydroxyethyl)amino)methyl)-1,7-naphthyridin-8-yl)amino)-2'-methyl-[1,1'-biphenyl]-3-yl)-5-formylpicolinamide

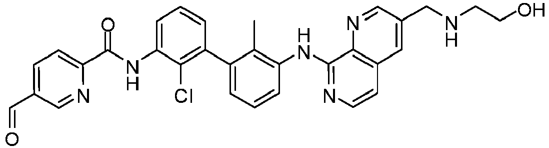

This compound was prepared using a similar procedure as described for Example 2, Step 4 with N-(2-chloro-3'-((3-(((2-hydroxyethyl)amino)methyl)-1,7-naphthyridin-8-yl)amino)-2'-methyl-[1,1'-biphenyl]-3-yl)-5-(dimethoxymethyl)picolinamide replacing 5-(dimethoxymethyl)-N-(2-fluoro-3'-((3-(((2-hydroxyethyl)amino)methyl)-1,7-naphthyridin-8-yl)amino)-2'-methyl-[1,1'-biphenyl]-3-yl)picolinamide. LC-MS calculated for C₃₁H₂₈ClN₆O₃ (M+H)⁺: m/z = 567.2; found 567.2.

### Step 5: N-(2-chloro-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamide

To a vial was added N-(2-chloro-3'-((3-(((2-hydroxyethyl)amino)methyl)-1,7-naphthyridin-8-yl)amino)-2'-methyl-[1,1'-biphenyl]-3-yl)-5-formylpicolinamide (0.015 g, 0.026 mmol), ethanolamine (Aldrich, cat# 398136: 0.0049 g, 0.163 mmol), dichloromethane (0.893 ml) and N,N-diisopropylethylamine (0.028 mL, 0.159 mmol). The reaction was stirred at rt for 2 h, then sodium triacetoxyborohydride (0.058 g, 0.272 mmol) was added. The reaction was stirred for 2 h, then the mixture was diluted with methanol and purified by prep HPLC (pH = 2, acetonitrile/water+TFA; then pH = 10, acetonitrile/water+NH₄OH). LC-MS calculated for C₃₃H₃₅ClN₇O₃ (M+H)⁺: m/z = 612.2; found 612.2.

### Example 6: N-(2-chloro-3'-((3-(((R)-3-hydroxypyrrolidin-1-yl)methyl)-1,7-naphthyridin-8-yl)amino)-2'-methyl-[1,1'- biphenyl]-3-yl)-5-(((R)-3-hydroxypyrrolidin-1-yl)methyl)picolinamide

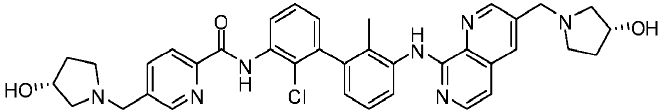

### Step 1: N-(3-bromo-2-methylphenyl)-3-vinyl-1,7-naphthyridin-8-amine

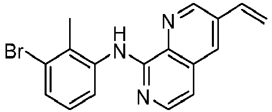

In a vial, 3-bromo-2-methylaniline (Aldrich, cat#530018: 0.931 ml, 7.55 mmol) and 8-chloro-3-vinyl-1,7-naphthyridine (Example 1, Step 1: 1.20 g, 6.29 mmol) were suspended in isopropanol (31.5 ml). Sulfuric acid (0.336 ml, 6.29 mmol) was added to the reaction mixture. The resulting mixture was heated to 100 °C for 1 h whilst stirring. The mixture was cooled, quenched with aqueous saturated sodium bicarbonate, and diluted with DCM. The layers were separated and the water layer was further extracted with DCM. The combined organic layers were dried over magnesium sulfate, filtered and concentrated in vacuo. The crude solid was purified by column chromatography (0→1%Methanol/DCM) to provide the desired compound as a yellow solid. LC-MS calculated for C₁₇H₁₅BrN₃ (M+H)⁺: m/z = 340.0; found 340.1.

### Step 2: 2'-chloro-2-methyl-N3-(3-vinyl-1,7-naphthyridin-8-yl)-[1,1'-biphenyl]-3,3'-diamine

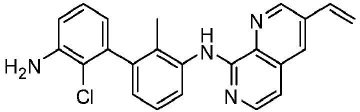

To a flask was added 2-chloro-3-(4,4,5,5-tetramethyl-1,3,2-dioxaborolan-2-yl)aniline (Example 5, Step 1: 1.414 g, 5.58 mmol), N-(3-bromo-2-methylphenyl)-3-vinyl-1,7-naphthyridin-8-amine (1.2646 g, 3.72 mmol), sodium carbonate (0.591 g, 5.58 mmol), [1,1'-bis(diphenylphosphino)ferrocene] dichloropalladium (II) (0.272 g, 0.372 mmol), 1,4-dioxane (32.8 ml), and water (4.37 ml). The mixture was degassed, sealed, and heated to 90 °C whilst stirring for 4 h. The mixture was cooled, diluted with EtOAc, and the layers were separated. The aqueous layer was further extracted with EtOAc, and the combined organic layers were washed with brine, dried of magnesium sulfate, filtered, and concentrated in vacuo. The crude residue was then purified by silica gel chromatography (20% EtOAc/hexanes) to provide the desired compound as a yellow solid. LC-MS calculated for C₂₃H₂₀ClN₄ (M+H)⁺: m/z = 387.1; found 387.1.

### Step 3: N-(2-chloro-2'-methyl-3'-((3-vinyl-1,7-naphthyridin-8-yl)amino)-[1,1'-biphenyl]-3-yl)-5-(dimethoxymethyl)picolinamide

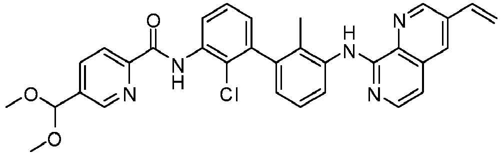

To a solution of 2'-chloro-2-methyl-N3-(3-vinyl-1,7-naphthyridin-8-yl)-[1,1'-biphenyl]-3,3'-diamine (0.682 g, 1.763 mmol) and methyl 5-(dimethoxymethyl)picolinate (Combi-Blocks, cat#QY-1318: 0.372 g, 1.763 mmol) in THF (17.63 ml) was added 1.0 M Potassium tert-butoxide in THF (2.64 ml, 2.64 mmol) at rt. The mixture was stirred at rt for 2 h. Water was added to quench the reaction. The layers were separated and the water layer was further extracted with ethyl acetate. The combined organic layers were washed with brine, dried over magnesium sulfate, filtered and concentrated in vacuo. The crude orange foam was used directly in the next step without further purification. LC-MS calculated for C₃₂H₂₉ClN₅O₃ (M+H)⁺: m/z = 566.2; found 566.3.

### Step 4: N-(2-chloro-2'-methyl-3'-((3-vinyl-1,7-naphthyridin-8-yl)amino)-[1,1'-biphenyl]-3-yl)-5-formylpicolinamide

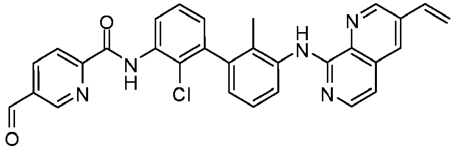

This compound was prepared using a similar procedure as described for Example 2, Step 4 with N-(2-chloro-2'-methy1-3'-((3-vinyl-1,7-naphthyridin-8-yl)arnino)-[1,1'-biphenyl]-3-yl)-5-(dimethoxymethyl)picolinamide replacing 5-(dimethoxymethyl)-N-(2-fluoro-3'-((3-(((2-hydroxyethyl)amino)methyl)-1,7-naphthyridin-8-yl)amino)-2'-methyl-[1,1'-biphenyl]-3-yl)picolinamide. LC-MS calculated for C₃₀H₂₃ClN₅O₂ (M+H)⁺: m/z = 520.2; found 520.2.

### Step 5: (R)-N-(2-chloro-2'-methyl-3'-((3-vinyl-1,7-naphtkvridin-8-yl)amino)-[1,1'-biphenyl]-3-yl)-5-((3-hydroxypyrrolidin-1-yl)methyl)picolinamide

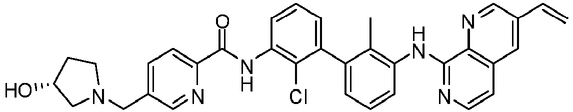

A mixture of N-(2-chloro-2'-methyl-3'-((3-vinyl-1,7-naphthyridin-8-yl)amino)-[1,1'-biphenyl]-3-yl)-5-formylpicolinamide (0.180 g, 0.346 mmol) and (R)-3-hydroxypyrrolidine (Combi-Blocks, cat#AM-2005: 0.090 g, 1.038 mmol) in methylene chloride (1.731 ml) and N,N-diisopropylethylamine (0.301 ml, 1.731 mmol) was stirred at rt for 1 h. Sodium triacetoxyborohydride (0.220 g, 1.038 mmol) was carefully added in portions. The reaction was stirred at rt for 2 h. The reaction was quenched with a saturated aqueous solution of sodium bicarbonate. The mixture was then extracted with a 3:1 mixture of chloroform/IPA. The combined organic layers were washed with brine, dried over sodium sulfate, and then concentrated in vacuo. The crude residue was purified by column chromatography (0 → 20% methanol/DCM). LC-MS calculated for C₃₄H₃₂ClN₆O₂ (M+H)⁺: m/z = 591.2; found 591.4.

### Step 6: (R)-N-(2-chloro-3'-((3-formyl-1,7-naphthyridin-8-yl)amino)-2'-methyl-[1,1'-biphenyl]-3-yl)--5-((3-hydroxypyrrolidin-1-yl)methyl)picolinamide

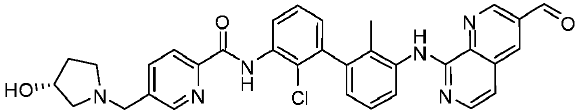

A flask was charged with (R)-N-(2-chloro-2'-methyl-3'-((3-vinyl-1,7-naphthyridin-8-yl)amino)-[1,1'-biphenyl]-3-yl)-5-((3-hydroxypyrrolidin-1-yl)methyl)picolinamide (0.241 g, 0.408 mmol), 1,4-dioxane (4.5 mL) and water (2.3 mL). A 4% osmium tetroxide solution in water (0.181 ml, 0.029 mmol) was added to the reaction mixture. After 5 min of stirring, sodium periodate (0.349 g, 1.631 mmol) was added and the mixture was stirred for 3 h. The mixture was diluted with water (2 mL) and EtOAc (5 mL), and the layers were separated. The aqueous layer was further extracted with EtOAc. The combined organic extracts were washed with brine, dried over sodium sulfate, filtered, and concentrated in vacuo. The crude aldehyde was purified by silica gel chromatography (20% MeOH/DCM). LC-MS calculated for C₃₃H₃₀ClN₆O₃ (M+H)⁺: m/z = 593.2; found 593.1.

### Step 7: N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-5-(((R)-3-hydroxypyrrolidin-1-yl)methyl)picolinamide

To a vial was added (R)-N-(2-chloro-3'-((3-formyl-1,7-naphthyridin-8-yl)amino)-2'-methyl-[1,1-biphenyl]-3-yl)-5-((3-hydroxypyrrolidin-1-yl)methyl)picolinamide(0.030 g, 0.051 mmol), ethanolamine (Aldrich, cat#398136: 9.3 mg, 0.152 mmol), dichloromethane (0.829 ml) and N,N-diisopropylethylamine (0.053 ml, 0.303 mmol). The reaction was stirred at rt for 2 h, then sodium triacetoxyborohydride (0.054 g, 0.253 mmol) was added. The reaction was stirred for 2 h, then the mixture was diluted with methanol and purified by prep HPLC (pH = 2, acetonitrile/water+TFA) to provide the desired compound as the TFA salt. LC-MS calculated for C₃₇H₃₉ClN₇O₃ (M+H)⁺: m/z = 664.3; found 664.2.

### Example 7: (R)-1-((8-((2'-chloro-3'-(5-(((R)-3-hydroxypyrrolidin-1-yl)methyl)picolinamido)-2-methyl-[1,1'-biphenyl]-3-yl)amino)-1,7-naphthyridin-3-yl)methyl)pyrrolidine-3-carboxylic acid

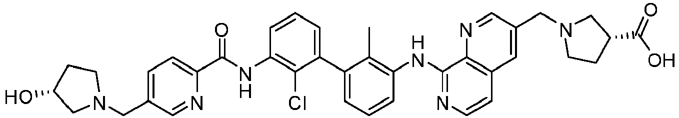

To a vial was added (R)-N-(2-chloro-3'-((3-formyl-1,7-naphthyridin-8-yl)amino)-2'-methyl-[1,1-biphenyl]-3-yl)-5-((3-hydroxypyrrolidin-1-yl)methyl)picolinamide (Example 6, Step 6: 0.030 g, 0.051 mmol), (R)-pyrrolidine-3-carboxylic acid (Combi-Blocks, cat#ST-7698: 0.017 g, 0.152 mmol), dichloromethane (0.829 ml) and triethylamine (0.016 ml, 0.115 mmol). The reaction was stirred at rt for 2 h, then sodium triacetoxyborohydride (0.054 g, 0.253 mmol) and acetic acid (8.69 µl, 0.152 mmol) were added. The reaction was stirred for 2 h, then the mixture was diluted with methanol and purified by prep HPLC (pH = 2, acetonitrile/water+TFA) to provide the compound as the TFA salt. LC-MS calculated for C₃₈H₃₉ClN₇O₄ (M+H)⁺: m/z = 692.3; found 692.2.

### Example 8: (R)-1-((8-((2'-chloro-3'-(5-((3-hydroxypyrrolidin-1-yl)methyl)picolinamido)-2-methyl-[1,1'-biphenyl]-3-yl)amino)-1,7-naphthyridin-3-yl)methyl)azetidine-3-carboxylic acid

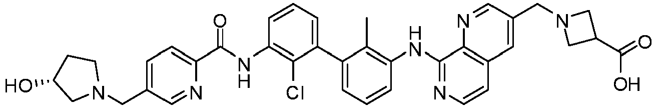

This compound was prepared using a similar procedure as described for Example 7 with azetidine-3-carboxylic acid (Aldrich, cat#391131) replacing (R)-pyrrolidine-3-carboxylic acid. LC-MS calculated for C₃₇H₃₇ClN₇O₄ (M+H)⁺: m/z = 678.3; found 678.3.

### Example 9

### (R)-1-((8-((3'-((3-(((R)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid

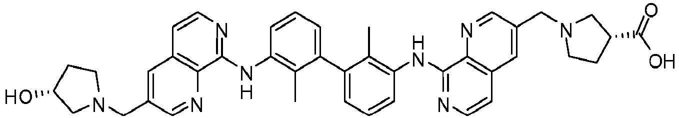

### Step 1: 8-((3-bromo-2-methylphenyl)amino)-1,7-naphthyridine-3-carbaldehyde

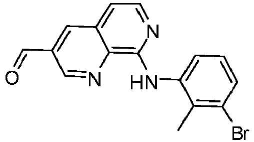

A suspension of (8-((3-bromo-2-methylphenyl)amino)-1,7-naphthyridin-3-yl)methanol (Affinity Research Chemicals, #ARI-0169: 300.0 mg, 0.872 mmol) and manganese dioxide (1515 mg, 17.43 mmol) in DCM (8716 µl) was stirred at 45 °C for 1 h. The reaction was filtered through Celite^{®} and the filtrate was concentrated to yield a crude residue, which was used directly in the next step without further purification. LC-MS calculated for C₁₆H₁₃BrN₃O (M+H)⁺: m/z = 342.0; found 342.0.

### Step2: (R)-1-((8-((3-bromo-2-methylphenyl)amino)-1,7-naphthyridin-3-yl)methyl)pyrrolidin-3-ol

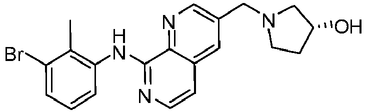

A mixture of 8-((3-bromo-2-methylphenyl)amino)-1,7-naphthyridine-3-carbaldehyde (0.100g, 0.292 mmol) and (*R*)-3-hydroxypyrrolidine (Combi-Blocks, #AM-2005: 0.025 g, 0.292 mmol) in 1,2-dichloroethane (1.46 ml) and *N*,*N-*diisopropylethylamine (0.051 ml, 0.292 mmol) was stirred at rt for 1 h. Sodium triacetoxyborohydride (0.093 g, 0.438 mmol) was carefully added in portions. The reaction was stirred at rt for 2 h, then quenched with a saturated aqueous solution of sodium bicarbonate. The mixture was then extracted with a 3:1 mixture of chlorofom/IPA. The combined organic layers were dried over sodium sulfate, then concentrated in vacuo. The crude residue was purified by silica gel chromatography (0 → 30% methanol/DCM) to give the desired product. LC-MS calculated for C₂₀H₂₂BrN₄O (M+H)⁺: m/z = 413.1; found 413.1.

### Step 3: (8-((2-methyl-3-(4,4,5,5-tetramethyl-1,3,2-dioxaborolan-2-yl)phenyl)amino)-1,7-naphthyridin-3-yl)methanol

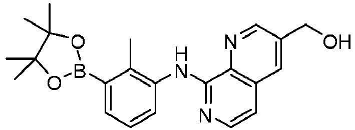

A mixture of (8-((3-bromo-2-methylphenyl)amino)-1,7-naphthyridin-3-yl)methanol (Affinity Research Chemicals, #ARI-0169: 0.300 g, 0.872 mmol), bis(pinacolato)diboron (Aldrich, #473294: 0.266 g, 1.046 mmol), dichloro[1,1'-bis(diphenylphosphino)ferrocene]palladium (II) dichloromethane adduct (0.071 g, 0.087 mmol) and potassium acetate (0.214 g, 2.179 mmol) was charged with nitrogen and stirred at 110 °C for 2 h. The crude was diluted with DCM, and then filtered through Celite^{®}. The filtrate was concentrated, and the resulting residue was used directly in the next step without further purification. LC-MS calculated for C₂₂H₂₇BN₃O₃ (M+H)⁺: m/z = 392.2; found 392.3.

### Step 4: (R)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol

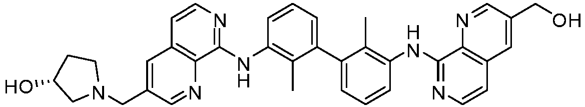

To a vial was added (8-((2-methyl-3-(4,4,5,5-tetramethyl-1,3,2-dioxaborolan-2-yl)phenyl)amino)-1,7-naphthyridin-3-yl)methanol (0.162 g, 0.414 mmol), (*R*)-1-((8-((3-bromo-2-methylphenyl)amino)-1,7-naphthyridin-3-yl)methyl)pyrrolidin-3-ol (0.163g, 0.394 mmol), 1 M aqueous sodium carbonate (0.789 mmol), [1,1'-bis(dicyclohexylphosphino)ferrocene]-dichloropalladium (II) (0.029 g, 0.039 mmol), and 1,4-dioxane (3.48 ml). The mixture was purged with nitrogen, sealed, and heated to 90 °C whilst stirring for 2 h. The mixture was cooled, diluted with EtOAc and filtered through Celite^{®}. The filtrate was concentrated and purified using silica gel chromatography (20% MeOH/DCM) to provide the desired compound as an orange solid. LC-MS calculated for C₃₆H₃₆N₇O₂ (M+H)⁺: m/z = 598.3; found 598.4.

### Step 5: (R)-8-((3'-((3-((3-hydroxypyrrolidin-1-yl)methyl)-1,7-naphthyridin-8-yl)amino)-2,2'-dimethyl-[1,1'-biphenyl]-3-yl)amino)-1,7-naphthyridine-3-carbaldehyde

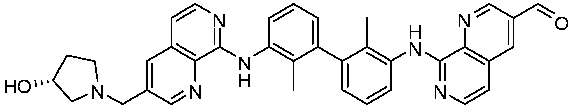

To a solution of (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol (0.0715 g, 0.12 mmol) in DCM (1.20 ml) was added manganese dioxide (0.208 g, 2.392 mmol). The resulting mixture was heated at 45 °C for 30 min. After cooling, the mixture was filtered through Celite^{®} and the filtrate was concentrated. The crude orange solid was used directly in the next step. LC-MS calculated for C₃₆H₃₄N₇O₂ (M+H)⁺: m/z = 596.3; found 596.5.

### Step 6: (R)-1-((8-((3'-((3-(((R)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid

To a vial was added (*R*)-8-((3'-((3-((3-hydroxypyrrolidin-1-yl)methyl)-1,7-naphthyridin-8-yl)amino)-2,2'-dimethyl-[1,1'-biphenyl]-3-yl)amino)-1,7-naphthyridine-3-carbaldehyde (0.013 g, 0.022 mmol), (*R*)-pyrrolidine-3-carboxylic acid (Combi-Blocks, #ST-7698: 7.5 mg, 0.065 mmol), 1,2-dichloroethane (0.336 ml) and triethylamine (9.13 µl, 0.065 mmol). The reaction was stirred at rt for 2 h, then sodium triacetoxyborohydride (0.023 g, 0.109 mmol) and acetic acid (3.75 µl, 0.065 mmol) were added. The reaction was stirred for 2 h, then the mixture was diluted with methanol and purified by prep HPLC (pH = 2, acetonitrile/water+TFA) to give the desired product as the TFA salt. LC-MS calculated for C₄₁H₄₃N₈O₃ (M+H)⁺: m/z = 695.3; found 695.3. ¹H NMR (500 MHz, DMSO) δ 10.72 (br s, 2H), 9.11 (m, 2H), 8.54 (m, 2H), 8.02 (m, 4H), 7.42 (m, 2H), 7.26 (m, 2H), 7.11 (m, 2H), 4.70 (m, 4H), 4.47 (m, 1H), 3.82 - 3.08 (m, 10H), 2.38 - 2.18 (m, 2H), 2.10 (s, 6H), 2.05 - 1.82 (m, 2H).

### Example 10

### (S)-1-((8-((3'-((3-(((R)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid

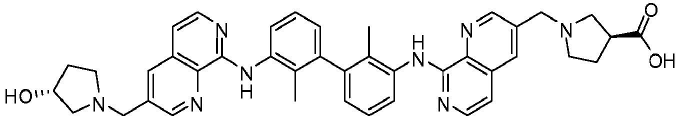

This compound was prepared using similar procedures as described for *Example 9* with (*S*)-pyrrolidine-3-carboxylic acid (Combi-Blocks, #ST-1381) replacing (*R*)-pyrrolidine-3-carboxylic acid in *Step 6.* The reaction was diluted with MeOH and then purified by prep-HPLC (pH = 2, acetonitrile/water+TFA) to give the desired product as the TFA salt. LC-MS calculated for C₄₁H₄₃N₈O₃ (M+H)⁺: m/z = 695.3 ; found 695.3.

### Example 11

### (R)-1-((8-((3'-((3-(((2-hydroxyethyl)amino)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid

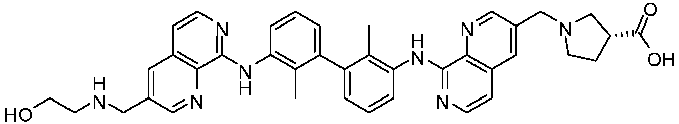

This compound was prepared using similar procedures as described for *Example 9* with ethanolamine (Aldrich, #411000) replacing (*R*)-3-hydroxypyrrolidine in *Step 2.* The reaction mixture was diluted with MeOH and then purified by prep-HPLC (pH = 6.5, acetonitrile/water+NH₄OAc) to give the desired product. LC-MS calculated for C₃₉H₄₁N₈O₃ (M+H)⁺: m/z = 669.3 ; found 669.4.

### Example 12

### (R)-1-((8-((3'-((3-(((S)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid

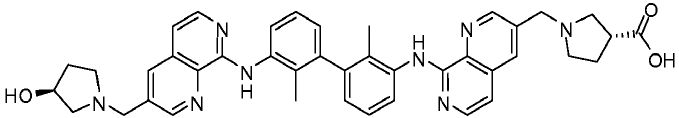

This compound was prepared using similar procedures as described for *Example 9* with (*S*)-3-hydroxypyrrolidine (Combi-Blocks, #SS-7948) replacing (R)-3-hydroxypyrrolidine in *Step 2.* The reaction was diluted with MeOH and then purified by prep-HPLC (pH = 2, acetonitrile/water+TFA) to give the desired product as the TFA salt. LC-MS calculated for C₄₁H₄₃N₈O₃ (M+H)⁺: m/z = 695.3 ; found 695.4.

### Example 13

### (S)-1-((8-((3'-((3-(((S)-3-hydroxypyrrolidin-1-yl)methyl)-1,7-naphthytidin-8-yl)amino)-2,2'-dimethyl-[1,1'-biphenyl]-3-yl)amino)-1,7-naphthyridin-3-yl)methyl)pyrrolidine-3-carboxylic acid

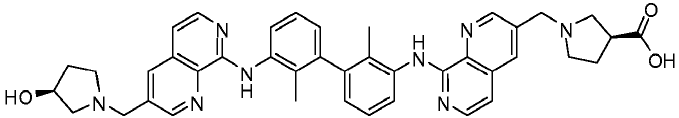

This compound was prepared using similar procedures as described for *Example 9* with (*S*)-3-hydroxypyrrolidine replacing (*R*)-3-hydroxypyrrolidine in *Step* 2 and (*S*)-pyrrolidine-3-carboxylic acid replacing (*R*)-pyrrolidine-3-carboxylic acid in *Step 6.* The reaction mixture was diluted with MeOH and then purified by prep-HPLC (pH = 2, acetonitrile/water+TFA) to give the desired product as the TFA salt. LC-MS calculated for C₄₁H₄₃N₈O₃ (M+H)⁺: m/z = 695.3; found 695.3.

### Example 14

### 1-((8-(2-chloro-3'-(1,5-dimethyl-4,5,6,7-tetrahydro-1H-imidazo[4,5-c]pyridine-2-carboxamido)-2'-methylbiphenyl-3-ylamino)-1,7-naphthyridin-3-yl)methyl)azetidine-3-carboxylic acid

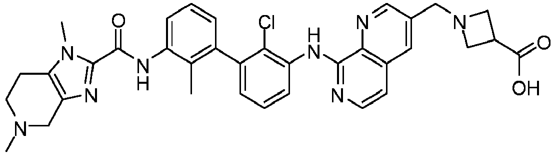

### Step 1: tert-butyl 1-methyl-6,7-dihydro-1H-imidazo[4,5-c]pyridine-5(4H)-carboxylate

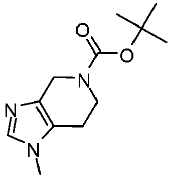

A solution of 1-methyl-4,5,6,7-tetrahydro-1H-imidazo[4,5-c]pyridine (*Accela, cat#SY032476:* 2.0 g, 14.58 mmol) and (Boc)₂O (3.38 mL, 14.58 mmol) in dichloromethane (60.0 mL) was stirred at room temperature for 1 h. The reaction was quenched with saturated aqueous NaHCO₃ solution, and extracted with ethyl acetate. The combined organic layers were washed with brine, dried over Na₂SO₄, filtered, and concentrated under reduced pressure. The crude product was used for next step without further purification. LC-MS calculated for C₁₂H₂₀N₃O₂ (M+H)⁺: m/z = 238.2; found 238.2.

### Step 2: 5-tert-butyl 2-methyl 1-methyl-6,7-dihydro-1H-imidazo[4,5-c]pyridine-2,5(4H)-dicarboxylate

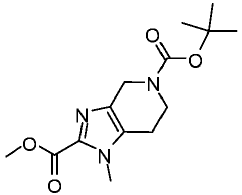

To a solution of tert-butyl 1-methyl-6,7-dihydro-1H-imidazo[4,5-c]pyridine-5(4H)-carboxylate(Crude product from *Step 1*) in tetrahydrofuran (60.0 mL) was added *n-*Butyllithium in hexanes (2.5 M, 7.00 mL, 17.49 mmol) at -78 °C, dropwise. The reaction mixture was stirred at -78 °C for 10 min prior to the addition of methyl chloroformate (1.7 mL, 21.9 mmol). After being stirred at -78 °C for 15 min, the reaction was then quenched with saturated aqueous NaHCO₃ solution, and extracted with ethyl acetate, dried over Na₂SO₄, filtered, and concentrated under reduced pressure. The residue was purified by flash chromatography on a silica gel column eluting with 80% ethyl acetate in hexanes to afford the desired product (). LC-MS calculated for C₁₄H₂₂N₃O₄ (M+H)⁺: m/z = 296.2; found 296.3.

### Step 3: tert-butyl 1-methyl-2-((2-methyl-3-(4,4,5,5-tetramethyl-1,3,2-dioxaborolan-2-yl)phenyl)carbamoyl)-1,4, 6, 7-tetrahydro-5H-imidazo[4,5-c]pyridine-5-carboxylate

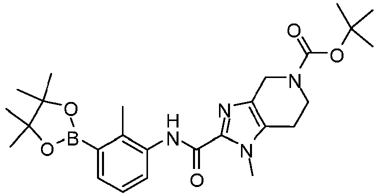

Potassium *tert*-butoxide (0.122 ml, 0.122 mmol) was added to a solution of 5*-tert-*butyl 2-methyl 1-methyl-1,4,6,7-tetrahydro-5H-imidazo[4,5-c]pyridine-2,5-dicarboxylate (30mg, 0.102 mmol) and 2-methyl-3-(4,4,5,5-tetramethyl-1,3,2-dioxaborolan-2-yl)aniline (Combi-Blocks, cat#PN-9127: 23.68 mg, 0.102 mmol) in THF (0.2 ml). After being stirred at rt for 2 h, the reaction mixture was quenched with water, and extracted with ethyl acetate. The combined organic layers were washed with brine, dried over Na₂SO₄, filtered and concentrated under reduced pressure. The residue was purified by flash chromatography on a silica gel column with ethyl acetate in hexanes (0-40%) to afford the product. LC-MS calculated for C₂₆H₃₈BN₄O₅ (M+H)⁺: m/z = 497.3; found 497.2.

### Step 4: 8-chloro-3-vinyl-1,7-naphthyridine

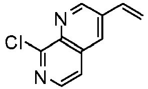

A mixture of 3-bromo-8-chloro-1,7-naphthyridine (PharmaBlock, cat#PBLJ2743: 0.200 g, 0.821 mmol), 4,4,5,5-tetramethyl-2-vinyl-1,3,2-dioxaborolane (Aldrich, cat#663348: 153 µL, 0.904 mmol), sodium carbonate (0.174 g, 1.64 mmol) and [1,1'-bis(di-cyclohexylphosphino)ferrocene]dichloropalladium(II) (Aldrich, cat#701998: 6.2 mg, 0.0082 mmol) in tert-butyl alcohol (5.91 mL, 61.8 mmol) and water (6 mL, 300 mmol) was degassed and sealed. It was stirred at 110 °C for 2 h. The reaction mixture was cooled then extracted with ethyl acetate (3x 20 mL). The combined organic layers were washed with brine, dried over MgSO₄, filtered and concentrated under reduced pressure. The crude residue was used directly in the next step without further purification. LC-MS calculated for C₁₀H₈ClN₂ (M+H)⁺: m/z = 191.0; found 191.0.

### Step 5: N-(3-bromo-2-chlorophenyl)-3-vinyl-1,7-naphthyridin-8-amine

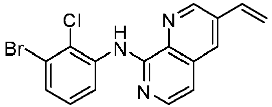

In a vial, 3-bromo-2-chloroaniline (Enamine, cat# EN300-105778 :0.476 g, 2.304 mmol) and 8-chloro-3-vinyl-1,7-naphthyridine (0.366 g, 1.920 mmol) were suspended in isopropanol (9.60 ml). Sulfuric acid (0.102 ml, 1.920 mmol) was added to the reaction mixture. The resulting mixture was heated to 100 °C for 1 h. The mixture was cooled to rt then quenched with aqueous saturated sodium bicarbonate, and diluted with DCM. The layers were separated and the water layer was further extracted with DCM. The combined organic layers were dried over magnesium sulfate, filtered and concentrated in vacuo. The crude solid was purified by column chromatography (0→2% methanol/DCM). LC-MS calculated for C₁₆H₁₂BrClN₃ (M+H)⁺: m/z = 360.0; found 360.0.

### Step 6: 8-((3-bromo-2-chlorophenyl)amino)-1,7-naphthyridine-3-carbaldehyde

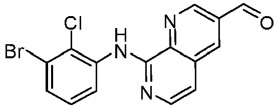

A flask was charged with *N*-(3-bromo-2-chlorophenyl)-3-vinyl-1,7-naphthyridin-8-amine (0.586 g, 1.625 mmol), 1,4-dioxane (40 mL) and water (40 mL). A 4% osmium tetroxide solution in water (0.207 ml, 0.032 mmol) was added to the reaction mixture. After 5 min, sodium periodate (1.390 g, 6.50 mmol) was added. The mixture was stirred overnight at rt. The reaction was diluted with water and ethyl acetate. The layers were separated and the aqueous layer was further extracted with EtOAc. The combined organic extracts were washed with brine, dried over sodium sulfate, filtered, and concentrated in vacuo. The crude product was purified by silica gel chromatography (0→ 60% EtOAc/hexanes). LC-MS calculated for C₁₅H₁₀BrClN₃O (M+H)⁺: m/z = 362.0; found 362.0.

### Step 7: methyl 1-((8-((3-bromo-2-chlorophenyl)amino)-1,7-naphthyridin-3-yl)methyl)azetidine-3-carboxylate

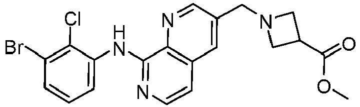

A mixture of 8-((3-bromo-2-chlorophenyl)amino)-1,7-naphthyridine-3-carbaldehyde (0.272 g, 0.750 mmol) and methyl azetidine-3-carboxylate, HCl (Combi-Blocks, cat#SS-3302:125 mg, 0.825 mmol) in methylene chloride (3.75 ml) and *N*,*N*-diisopropylethylamine (0.392 ml, 2.250 mmol) was stirred at rt for 1 h. Sodium triacetoxyborohydride (0.477 g, 2.250 mmol) was added in portions. The reaction was stirred at rt for 2 h, then sodium tetrahydroborate (0.060 ml, 1.500 mmol) and methanol (6 mL) were added carefully. After stirring overnight, the reaction was quenched with a saturated solution of sodium bicarbonate. The mixture was then extracted with a 3:1 mixture of chloroform/isopropanol. The combined organic layers were washed with brine, dried over sodium sulfate, and concentrated under reduced pressure. The crude residue was purified by column chromatography (methanol/DCM). LC-MS calculated for C₂₀H₁₉BrClN₄O₂ (M+H)⁺: m/z = 461.0; found 461.1.

### Step 8: tert-butyl 2-((2'-chloro-3'-((3-((3-(methoxycarbonyl)azetidin-1-yl)methyl)-1,7-naphthyridin-8-yl)amino)-2-methyl-[1,1'-biphenyl]-3-yl)carbamoyl)-1-methyl-1,4, 6,7-tetrahydro-5H-imidazo[4, 5-c]pyridine-5-carboxylate

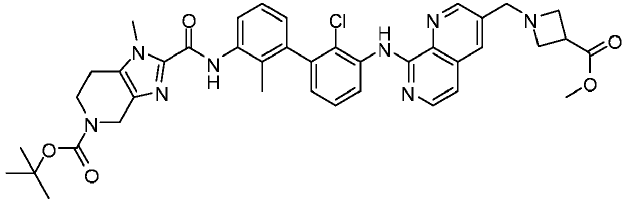

To a vial was added *tert*-butyl 1-methyl-2-((2-methyl-3-(4,4,5,5-tetramethyl-1,3,2-dioxaborolan-2-yl)phenyl)carbamoyl)-1,4,6,7-tetrahydro-5H-imidazo[4,5-c]pyridine-5-carboxylate (Step 3: 0.037 g, 0.074 mmol), methyl 1-((8-((3-bromo-2-chlorophenyl)amino)-1,7-naphthyridin-3-yl)methyl)azetidine-3-carboxylate (0.034 g, 0.074 mmol), sodium carbonate (8.58 mg, 0.081 mmol), (1,1'-bis(diphenylphosphino)ferrocene)-dichloropalladium(II) (5.39 mg, 7.36 µmol), 1,4-dioxane (0.650 ml), and water (0.087 ml). The mixture was degassed, sealed, and heated to 90 °C whilst stirring for 18 h. The mixture was cooled, diluted with water and methylene chloride, and the layers were separated. The aqueous layer was further extracted with methylene chloride and the combined organic layers were dried over MgSO₄, filtered, and concentrated in vacuo. The crude residue was purified by silica gel chromatography (15% MeOH/DCM) to provide the desired product. LC-MS calculated for C₄₀H₄₄ClN₈O₅ (M+H)⁺: m/z = 751.3; found 751.3.

### Step 9: methyl 1-((8-(2-chloro-2'-methyl-3'-(1-methyl-4,5,6,7-tetrahydro-1H-imidazo[4,-5-c]pyridine-2-carboxamido)biphenyl-3-ylamino)-1,7-naphthyridin-3-yl)methyl)azetidine-3-carboxylate

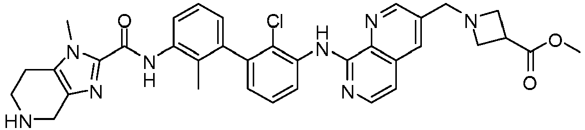

To a vial was added tert-butyl 2-((2'-chloro-3'-((3-((3-(methoxycarbonyl)azetidin-1-yl)methyl)-1,7-naphthyridin-8-yl)amino)-2-methyl-[1,1'-biphenyl]-3-yl)carbamoyl)-1-methyl-1,4,6,7-tetrahydro-5H-imidazo[4,5-c]pyridine-5-carboxylate (0.041 g, 0.055 mmol), DCM (0.6 mL), and TFA (0.084 mL, 1.091 mmol). The reaction was stirred at rt for 1 h. The mixture was concentrated under reduced pressure, and the resulting residue was redissolved in DCM and washed with a saturated aqueous solution of sodium bicarbonate. The layers were separated, and the organic layer was dried over MgSO₄, filtered, and concentrated under reduced pressure. The crude residue was then used directly in the next step without further purification. LC-MS calculated for C₃₅H₃₆ClN₈O₃ (M+H)⁺: m/z = 651.3; found 651.4.

### Step 10: methyl 1-((8-(2-chloro-3'-(1,5-dimethyl-4,5,6,7-tetrahydro-1H-imidazo[4,5-c]pyridine-2-carboxamido)-2'-methylbiphenyl-3-ylamino)-1,7-naphthyridin-3-yl)methyl)azetidine-3-carboxylate

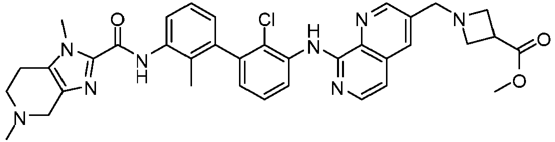

A mixture of methyl 1-((8-((2-chloro-2'-methyl-3'-(1-methyl-4,5,6,7-tetrahydro-1H-imidazo[4,5-c]pyridine-2-carboxamido)-[1,1'-biphenyl]-3-yl)amino)-1,7-naphthyridin-3-yl)methyl)azetidine-3-carboxylate (0.032 g, 0.049 mmol) and 12.3 M formaldehyde in water (7.99 µl, 0.098 mmol) in methylene chloride (0.430 ml) and methanol (0.061 ml) was stirred at rt for 30 min after which time, acetic acid (0.017 ml, 0.295 mmol) and sodium triacetoxyborohydride (0.052 g, 0.246 mmol) were added. The mixture was stirred at rt for 45 min and the reaction was quenched with a saturated aqueous sodium bicarbonate solution. The mixture was extracted with a 3:1 mixture of chloroform/isopropanol, and the combined organic layers were dried over sodium sulfate, filtered, and concentrated under reduced pressure. The crude product was purified by column chromatography (0 → 30% methanol/DCM). LC-MS calculated for C₃₆H₃₈ClN₈O₃ (M+H)⁺: m/z = 665.3; found 665.4.

### Step 11: 1-((8-(2-chloro-3'-(1,5-dimethyl-4,5, 6, 7-tetrahydro-1H-imidazo[4,-5-c]pyridine-2-carboxamido)-2'-methylbiphenyl-3ylamino)-1,7-naphthyridin-3yl)methyl)azetidine-3-carboxylic acid

To a solution of methyl 1-((8-((2-chloro-3'-(1,5-dimethyl-4,5,6,7-tetrahydro-1H-imidazo[4,5-c]pyridine-2-carboxamido)-2'-methyl-[1,1'-biphenyl]-3-yl)amino)-1,7-naphthyridin-3-yl)methyl)azetidine-3-carboxylate (27 mg, 0.041 mmol) in THF (203 µl) was added lithium hydroxide (3.89 mg, 0.162 mmol) in water (203 µl). The mixture was stirred at rt for 30 min. The mixture was diluted with methanol and purified by prep HPLC (pH = 2, acetonitrile/water+TFA) to provide the compound as its TFA salt. LC-MS calculated for C₃₅H₃₆ClN₈O₃ (M+H)⁺: m/z = 651.3; found 651.3.

### Example 15

### (R)-1-((5-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6-oxo-1,6-dihydropyridin-3-yl)methyl)pyrrolidine-3-carboxylic acid

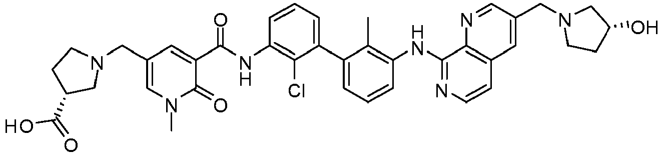

### Step 1: 2-chloro-3-(4,4, 5, 5-tetramethyl-1,3, 2-dioxaborolan-2-yl)aniline

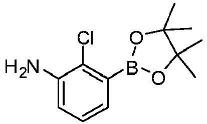

In a vial was combined: 4,4,5,5,4',4',5',5'-Octamethyl-[2,2']bi[[1,3,2]dioxaborolanyl] (6.15 g, 24.22 mmol), potassium acetate (2.85 g, 29.1 mmol), 3-bromo-2-chloroaniline (Enamine, cat# EN300-105778: 2.00 g, 9.69 mmol), 1,4-dioxane (48.4 ml) and [1,1'-bis(diphenylphosphino)ferrocene] dichloropalladium (II) (0.354 g, 0.484 mmol). The vial was flushed with nitrogen and was stirred at 110 °C for 2 h. The mixture was cooled, andfiltered through Celite^{®}, and concentrated under reduced pressure. The crude residue was purified by silica gel chromatography (EtOAc/hexanes) to provide the desired compound as a white solid. LC-MS calculated for C₁₂H₁₈BClNO₂ (M+H)⁺: m/z = 254.1; found 254.1.

### Step 2: 8-((3-bromo-2-methylphenyl)amino)-1,7-naphthyridine-3-carbaldehyde

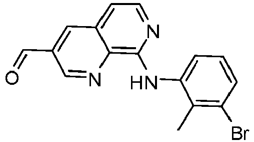

A suspension of (8-((3-bromo-2-methylphenyl)amino)-1,7-naphthyridin-3-yl)methanol (Affinity Research Chemicals, #ARI-0169: 300.0 mg, 0.872 mmol) and manganese dioxide (1515 mg, 17.43 mmol) in DCM (8716 µl) was stirred at 45 °C for 1 h. The reaction was filtered through Celite^{®} and the filtrate was concentrated to yield a crude residue, which was used directly in the next step without further purification. LC-MS calculated for C₁₆H₁₃BrN₃O (M+H)⁺: m/z = 342.0; found 342.0.

### Step 3: (R)-1-((8-((3-bromo-2-methylphenyl)amino)-1,7-naphthyridin-3-yl)methyl)pyrrolidin-3-ol

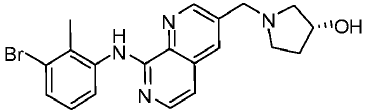

A mixture of 8-((3-bromo-2-methylphenyl)amino)-1,7-naphthyridine-3-carbaldehyde (0.100g, 0.292 mmol) and (*R*)-3-hydroxypyrrolidine (Combi-Blocks, #AM-2005: 0.025 g, 0.292 mmol) in 1,2-dichloroethane (1.46 ml) and *N*,*N-*diisopropylethylamine (0.051 ml, 0.292 mmol) was stirred at rt for 1 h. Sodium triacetoxyborohydride (0.093 g, 0.438 mmol) was added in portions. The reaction was stirred at rt for 2 h, then quenched with a saturated aqueous solution of sodium bicarbonate. The mixture was then extracted with a 3:1 mixture of chloroform/isopropanol. The combined organic layers were dried over sodium sulfate, and concentrated in vacuo. The crude residue was purified by silica gel chromatography (0 → 30% methanol/DCM) to give the desired product. LC-MS calculated for C₂₀H₂₂BrN₄O (M+H)⁺: m/z = 413.1; found 413.1.

### Step 4: (R)-1-((8-(3'-amino-2'-chloro-2-methylbiphenyl-3-ylamino)-1, 7-naphthyridin-3-yl)methyl)pyrrolidin-3-ol

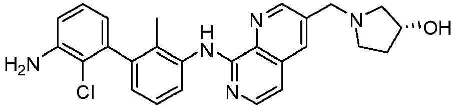

To a vial was added 2-chloro-3-(4,4,5,5-tetramethyl-1,3,2-dioxaborolan-2-yl)aniline (Step 1: 0.101 g, 0.399 mmol), (*R*)-1-((8-((3-bromo-2-methylphenyl)amino)-1,7-naphthyridin-3-yl)methyl)pyrrolidin-3-ol (0.165g, 0.399 mmol), sodium carbonate (0.047 g, 0.439 mmol), (1,1'-bis(diphenylphosphino)ferrocene)-dichloropalladium(II) (0.029 g, 0.040 mmol), 1,4-dioxane (3.52 ml), and water (0.470 ml). The mixture was degassed, sealed, and heated to 90 °C whilst stirring for 18 h. The mixture was cooled, diluted with water and methylene chloride, and the layers were separated. The aqueous layer was further extracted with methylene chloride, and the combined organic layers were dried over MgSO₄, filtered, and concentrated in vacuo. The crude residue was purified by silica gel chromatography (15% MeOH/DCM) to provide the desired product. LC-MS calculated for C₂₆H₂₇ClN₅O (M+H)⁺: m/z = 460.2; found 460.3.

### Step 5: methyl 5-bromo-1-methyl-2-oxo-1,2-dihydropyridine-3-carboxylate

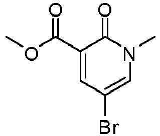

Methyl iodide (1.713 ml, 27.5 mmol) was added to a mixture of 5-bromo-2-hydroxynicotinic acid (Combi-Blocks, cat# CA-4087: 2.0 g, 9.17 mmol), and potassium carbonate (1.811 ml, 20.18 mmol) in methanol (45.9 mL), which was stirred at 70 °C overnight. The solvent was removed, and the crude mixture was extracted with DCM/water. The organic extracts were dried over MgSO₄, filtered, and concentrated under reduced pressure. The crude product was used directly in the next step without further purification. LC-MS calculated for C₈H₉BrNO₃ (M+H)⁺: m/z = 246.0; found 246.0.

### Step 6: 1-methyl-2-oxo-5-vinyl-1,2-dihydropyridine-3-carboxylic acid

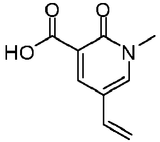

A mixture of methyl 5-bromo-1-methyl-2-oxo-1,2-dihydropyridine-3-carboxylate (745 mg, 3.03 mmol), 4,4,5,5-tetramethyl-2-vinyl-1,3,2-dioxaborolane (565 µl, 3.33 mmol), tetrakis(triphenylphosphine)palladium(0) (175.0 mg, 0.151 mmol), 2.0 M sodium carbonate in water (4543 µl, 9.09 mmol) and 1,4-dioxane (6058 µl) was sparged with nitrogen and then heated at 100 °C for 30 min. The mixture was partitioned between EtOAc and water and the layers separated. The organic layer was washed with brine, dried over MgSO₄, filtered and concentrated under reduced pressure. The crude residue was used directly in the next step without further purification. LC-MS calculated for C₉H₁₀NO₃ (M+H)⁺: m/z = 180.1; found 180.1.

### Step 7: (R)-N-(2-chloro-3'-(3-((3-hydroxypyrrolidin-1-yl)methyl)-1,7-naphthyridin-8-ylamino)-2'-methylbiphenyl-3yl)-1-methyl-2-oxo-5-vinyl-1,2-dihydropyridine-3-carboxamide

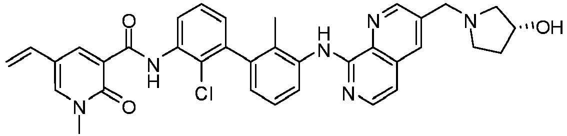

A mixture of 1-methyl-2-oxo-5-vinyl-1,2-dihydropyridine-3-carboxylic acid (0.0550 g, 0.307 mmol), (R)-1-((8-((3'-amino-2'-chloro-2-methyl-[1,1'-biphenyl]-3-yl)amino)-1,7-naphthyridin-3-yl)methyl)pyrrolidin-3-ol (Step 4: 0.141 g, 0.307 mmol), *N-*[(dimethylamino)-1H-1,2,3-triazolo-[4,5-b]pyridin-1-ylmethylene]-*N*-methylmethanaminium hexafluorophosphate *N*-oxide (0.140 g, 0.368 mmol), and *N*,*N*-diisopropylethylamine (0.107 ml, 0.614 mmol) in 1,2-dichloroethane (4.39 ml) was stirred at rt for 2 h. The mixture was concentrated under reduced pressure. The residue was diluted with ethyl acetate, and washed with water followed by brine. The organic layer was dried over MgSO₄, filtered and concentrated under reduced pressure. The crude residue was purified by silica gel chromatography (MeOH/DCM) to provide the desired compound. LC-MS calculated for C₃₅H₃₄ClN₆O₃ (M+H)⁺: m/z = 621.2; found 621.4.

### Step 8: (R)-N-(2-chloro-3'-(3-((3-hydroxypyrrolidin-1-yl)methyl)-1,7-naphthyridin-8-ylamino)-2'-methylbiphenyl-3-yl)-5-formyl-1-methyl-2-oxo-1,2-dihydropyridine-3-carboxamide

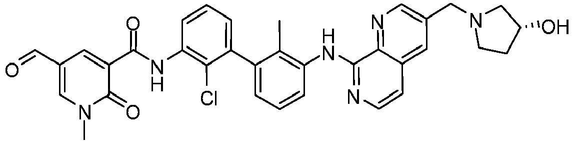

A flask was charged with (*R*)-*N*-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-2-oxo-5-vinyl-1,2-dihydropyridine-3-carboxamide (0.131 g, 0.211 mmol), 1,4-dioxane (40 mL) and water (40 mL). A 4% osmium tetroxide solution in water (0.094 ml, 0.015 mmol) was added to the reaction mixture. After 5 min, sodium periodate (0.361 g, 1.687 mmol) was added. The mixture was stirred overnight at rt. The mixture was diluted with water (2 mL) and 3:1 chloroform/isopropanol (5 mL), and the layers were separated. The organic extract was dried over sodium sulfate, filtered, and concentrated in vacuo. The crude aldehyde was purified by silica gel chromatography (20% MeOH/DCM). LC-MS calculated for C₃₄H₃₂ClN₆O₄ (M+H)⁺: m/z = 623.2; found 623.4.

### Step 9: (R)-1-((5-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6-oxo-1,6-dihydropyridin-3-yl)methyl)pyrrolidine-3-carboxylic acid

To a vial was added (*R*)-*N*-(2-chloro-3'-((3-((3-hydroxypyrrolidin-1-yl)methyl)-1, 7-naphthyridin-8-yl)amino)-2'-methyl-[1,1-biphenyl]-3-yl)-5-formyl-1-methyl-2-oxo-1,2-dihydropyridine-3-carboxamide (0.022 g, 0.035 mmol), (*R*)-pyrrolidine-3-carboxylic acid (Combi-Blocks, cat#ST-7698: 0.012 g, 0.106 mmol), dichloromethane (0.579 ml) and triethylamine (0.016 ml, 0.115 mmol). The reaction was stirred at rt for 2 h, then sodium triacetoxyborohydride (0.037 g, 0.177 mmol) and acetic acid (6.06 µl, 0.106 mmol) were added. The reaction was stirred for 2 h, then the mixture was diluted with methanol and purified by prep HPLC (pH = 2, acetonitrile/water+TFA) to provide the desired compound as its TFA salt. LC-MS calculated for C₃₉H₄₁ClN₇O₅ (M+H)⁺: m/z = 722.3; found 722.2.

### Example 16

### (R)-1-((8-(2,2'-dichloro-3'-(5-((3-hydroxypyrrolidin-1-yl)methyl)-1-methyl-2-oxo-1,2-dihydropyridine-3-carboxamido)biphenyl-3-ylamino)-1,7-naphthyridin-3-yl)methyl)azetidine-3-carboxylic acid

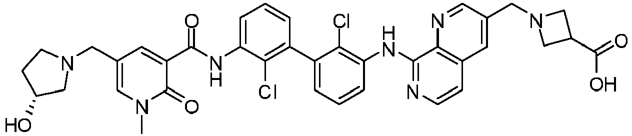

### Step 1: methyl 1-((8-(3'-amino-2,2'-dichlorobiphenyl-3-ylamino)-1,7-naphthyridin-3-yl)methyl)azetidine-3-carboxylate

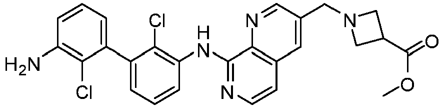

To a vial was added 2-chloro-3-(4,4,5,5-tetramethyl-1,3,2-dioxaborolan-2-yl)aniline (Example 15, Step 1: 0.137 g, 0.539 mmol), methyl 1-((8-((3-bromo-2-chlorophenyl)amino)-1,7-naphthyridin-3-yl)methyl)azetidine-3-carboxylate (Example 14, Step 7: 0.166 g, 0.360 mmol), sodium carbonate (0.057 g, 0.539 mmol), (1,1'-bis(diphenylphosphino)ferrocene)-dichloropalladium(II) (0.026 g, 0.036 mmol), 1,4-dioxane (3.17 ml), and water (0.423 ml). The mixture was degassed, sealed, and heated to 90 °C whilst stirring for 1 h. The mixture was cooled, diluted with water and 3:1 chloroform/isopropanol, and the layers were separated. The aqueous layer was further extracted with 3:1 chloroform/isopropanol, and the combined organic extracts were dried over MgSO₄, filtered, and concentrated in vacuo. The desired compound was purified by silica gel chromatography (20% MeOH/DCM). LC-MS calculated for C₂₆H₂₄Cl₂N₅O₂ (M+H)⁺: m/z = 508.1; found 508.2.

### Step 2: methyl 1-((8-(2,2'-dichloro-3'-(1-methyl-2-oxo-5-vinyl-1,2-dihydropyridine-3-carboxamido)biphenyl-3-ylamino)-1,7-naphthyridin-3-yl)methyl)azetidine-3-carboxylate

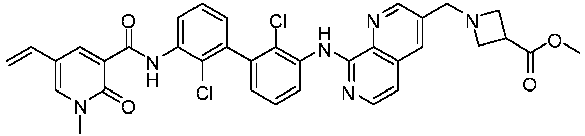

In a vial, 1-methyl-2-oxo-5-vinyl-1,2-dihydropyridine-3-carboxylic acid *(Example 15, Step 6:* 0.051 g, 0.285 mmol) HATU (0.130 g, 0.342 mmol) and *N*,*N*-diisopropylethylamine (0.099 ml, 0.569 mmol) were dissolved in DMF (2.85 ml). After stirring for 5 min, methyl 1-((8-(3'-amino-2,2'-dichlorobiphenyl-3-ylamino)-1,7-naphthyridin-3-yl)methyl)azetidine-3-carboxylate (0.285 mmol) was added, and the resulting mixture was stirred at 40 °C for 24 h. Excess DMF was concentrated, and the resulting oil was diluted with EtOAc and water. The layers were separated and the water layer was further extracted with ethyl acetate. The combined organic layers were washed with brine, dried over magnesium sulfate, filtered and concentrated in vacuo. After concentrating, the crude residue was triturated with DCM and filtered to provide the desired product. The filtrate was purified by silica gel chromatography (20% MeOH/DCM) to provide additional desired product. LC-MS calculated for C₃₅H₃₁Cl₂N₆O₄ (M+H)⁺: m/z = 669.2; found 669.1.

### Step 3: methyl 1-((8-(2,2'-dichloro-3'-(5-formyl-1-methyl-2-oxo-1,2-dihydropyridine-3-carboxamido)biphenyl-3-ylamino)-1,7-naphthyridin-3-yl)methyl)azetidine-3-carboxylate

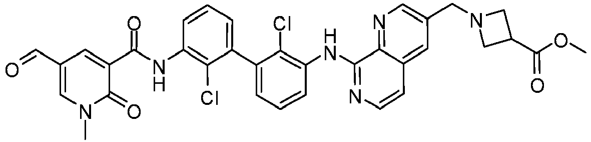

This compound was prepared using similar procedures as described for Example 14 with methyl 1-((8-(2,2'-dichloro-3'-(1-methyl-2-oxo-5-vinyl-1,2-dihydropyridine-3-carboxamido)biphenyl-3-ylamino)-1,7-naphthyridin-3-yl)methyl)azetidine-3-carboxylate replacing N-(3-bromo-2-chlorophenyl)-3-vinyl-1,7-naphthyridin-8-amine in Step 6. The crude product was purified by silica gel chromatography (20% MeOH/DCM) to provide the desired product. LC-MS calculated for C₃₄H₂₉Cl₂N₆O₅ (M+H)⁺: m/z = 671.2; found 671.4.

### Step 4: (R)-methyl1-((8-(2,2'-dichloro-3'-(5-((3-hydroxypyrrolidin-1-yl)methyl)-1-methyl-2-oxo-1,2-dihydropyridine-3-carboxamido)biphenyl-3-ylamino)-1,7-naphthyridin-3-yl)methyl)azetidine-3-carboxylate

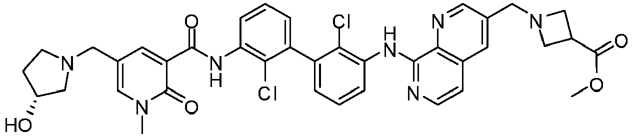

This compound was prepared using similar procedures as described for Example 15 with methyl 1-((8-(2,2'-dichloro-3'-(5-formyl-1-methyl-2-oxo-1,2-dihydropyridine-3-carboxamido)biphenyl-3-ylamino)-1,7-naphthyridin-3-yl)methyl)azetidine-3-carboxylate replacing 8-((3-bromo-2-methylphenyl)amino)-1,7-naphthyridine-3-carbaldehyde in Step 3. The crude product was purified by silica gel chromatography (MeOH/DCM) to provide the desired product. LC-MS calculated for C₃₈H₃₈Cl₂N₇O₅ (M+H)⁺: m/z = 742.2; found 742.4.

### Step 5: (R)-1-((8-(2,2'-dichloro-3'-(5-((3-hydroxypyrrolidin-1-yl)methyl)-1-methyl-2-oxo-1,2-dihydropyridine-3-carboxamido)biphenyl-3-ylamino)-1,7-naphthyridin-3-yl)methyl)azetidine-3-carboxylic acid

This compound was prepared using similar procedures as described for Example 14 with (R)-methyl 1-((8-(2,2'-dichloro-3'-(5-((3-hydroxypyrrolidin-1-yl)methyl)-1-methyl-2-oxo-1,2-dihydropyridine-3-carboxamido)biphenyl-3-ylamino)-1,7-naphthyridin-3-yl)methyl)azetidine-3-carboxylate replacing methyl 1-((8-((2-chloro-3'-(1,5-dimethyl-4,5,6,7-tetrahydro-1H-imidazo[4,5-c]pyridine-2-carboxamido)-2'-methyl-[1,1'-biphenyl]-3-yl)amino)-1,7-naphthyridin-3-yl)methyl)azetidine-3-carboxylate in Step 11. The reaction mixture was diluted with methanol and purified by prep HPLC (pH = 2, acetonitrile/water+TFA) to provide the desired compound as its TFA salt. LC-MS calculated for C₃₇H₃₆Cl₂N₇O₅ (M+H)⁺: m/z = 728.2; found 728.1.

### Example 17

### 1-((8-(2,2'-dichloro-3'-(5-((2-hydroxyethylamino)methyl)-1-methyl-2-oxo-1,2-dihydropyridine-3-carboxamido)biphenyl-3-ylamino)-1,7-naphthyridin-3-yl)methyl)azetidine-3-carboxylic acid

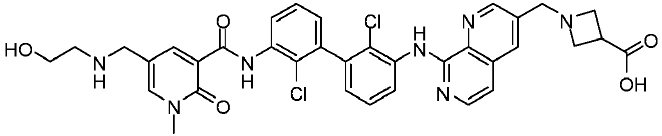

### Step 1: methyl 1-((8-(2,2'-dichloro-3'-(5-((2-hydroxyethylamino)methyl)-1-methyl-2-oxo-1,2-dihydropyridine-3-carboxamido)biphenyl-3-ylamino)-1,7-naphthyridin-3-yl)methyl)azetidine-3-carboxylate

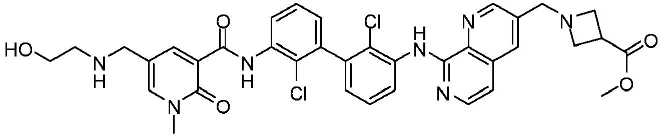

A mixture of methyl 1-((8-((2,2'-dichloro-3'-(5-formyl-1-methyl-2-oxo-1,2-dihydropyridine-3-carboxamido)-[1,1'-biphenyl]-3-yl)amino)-1,7-naphthyridin-3-yl)methyl)azetidine-3-carboxylate (Example 16, Step 3: 0.020g, 0.030 mmol) and ethanolamine (Aldrich, cat#411000: 0.089 mmol) in methylene chloride (0.596 ml) and N,N-diisopropylethylamine (0.026 ml, 0.149 mmol) was stirred at rt for 1 h. Sodium triacetoxyborohydride (0.019 g, 0.089 mmol) was carefully added. The reaction was stirred at rt for 2 h and sodium tetrahydroborate (2.384 µl, 0.060 mmol) and methanol (6 mL) were carefully added. The mixture was stirred overnight, and the reaction was quenched with a saturated solution of sodium bicarbonate. The mixture was then extracted with a 3:1 mixture of chloroform/isopropanol. The combined organic layers were washed with brine, dried over sodium sulfate, and then concentrated in vacuo. The crude residue was purified by column chromatography (0 → 50% methanol/DCM). LC-MS calculated for C₃₆H₃₆Cl₂N₇O₅ (M+H)⁺: m/z = 716.2; found 716.3.

### Step 2: 1-((8-(2,2'-dichloro-3'-(5-((2-hydroxyethylamino)methyl)-1-methyl-2-oxo-1,2-dihydropyridine-3-carboxamido)biphenyl-3-ylamino)-1,7-naphthyridin-3-yl)methyl)azetidine-3-carboxylic acid

This compound was prepared using similar procedures as described for Example 14 with methyl 1-((8-(2,2'-dichloro-3'-(5-((2-hydroxyethylamino)methyl)-1-methyl-2-oxo-1,2-dihydropyridine-3-carboxamido)biphenyl-3-ylamino)-1,7-naphthyridin-3-yl)methyl)azetidine-3-carboxylate replacing methyl 1-((8-((2-chloro-3'-(1,5-dimethyl-4,5,6,7-tetrahydro-1H-imidazo[4,5-c]pyridine-2-carboxamido)-2'-methyl-[1,1'-biphenyl]-3-yl)amino)-1,7-naphthyridin-3-yl)methyl)azetidine-3-carboxylate in Step 11. The reaction mixture was diluted with methanol and purified by prep HPLC (pH = 2, acetonitrile/water+TFA) to provide the desired compound as its TFA salt. LC-MS calculated for C₃₅H₃₄Cl₂N₇O₅ (M+H)⁺: m/z = 702.2; found 702.2.

### Example 18

### 1-((8-(2,2'-dichloro-3'-(5-((2-hydroxy-2-methylpropylamino)methyl)-1-methyl-2-oxo-1,2-dihydropyridine-3-carboxamido)biphenyl-3-ylamino)-1,7-naphthyridin-3-yl)methyl)azetidine-3-carboxylic acid

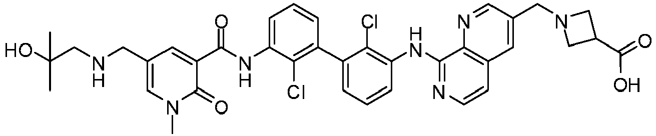

### Step 1: methyl 1-((8-(2,2'-dichloro-3'-(5-((2-hydroxy-2-methylpropylamino)methyl)-1-methyl-2-oxo-1,2-dihydropyridine-3-carboxamido)biphenyl-3-ylamino)-1,7-naphthyridin-3-yl)methyl)azetidine-3-carboxylate

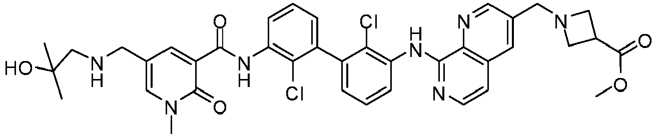

This compound was prepared using similar procedures as described for Example 17 with 1-amino-2-methyl-2-propanol (Aldrich, cat#777625) replacing ethanolamine in Step 1. The crude residue was purified by column chromatography (0 → 50% methanol/DCM). LC-MS calculated for C₃₈H₄₀Cl₂N₇O₅ (M+H)⁺: m/z = 744.2; found 744.4.

### Step 2: 1-((8-(2,2'-dichloro-3'-(5-((2-hydroxy-2-methylpropylamino)methyl)-1-methyl-2-oxo-1,2-dihydropyridine-3-carboxamido)biphenyl-3-ylamino)-1,7-naphthyridin-3-yl)methyl)azetidine-3-carboxylic acid

This compound was prepared using similar procedures as described for Example 14 with methyl 1-((8-(2,2'-dichloro-3'-(5-((2-hydroxy-2-methylpropylamino)methyl)-1-methyl-2-oxo-1,2-dihydropyridine-3-carboxamido)biphenyl-3-ylamino)-1,7-naphthyridin-3-yl)methyl)azetidine-3-carboxylate replacing methyl 1-((8-((2-chloro-3'-(1,5-dimethyl-4,5,6,7-tetrahydro-1H-imidazo[4,5-c]pyridine-2-carboxamido)-2'-methyl-[1,1'-biphenyl]-3-yl)amino)-1,7-naphthyridin-3-yl)methyl)azetidine-3-carboxylate in Step 11. The reaction mixture was diluted with methanol and purified by prep HPLC (pH = 2, acetonitrile/water+TFA) to provide the desired compound as its TFA salt. LC-MS calculated for C₃₇H₃₈Cl₂N₇O₅ (M+H)⁺: m/z = 730.2; found 730.2.

### Example 19

### 2,2'-(((((2,2'-dimethyl-[1,1'-biphenyl]-3,3'-diyl)bis(azanediyl))bis(1,7-naphthyridine-8,3-diyl))bis(methylene))bis(azanediyl))bis(ethan-1-ol)

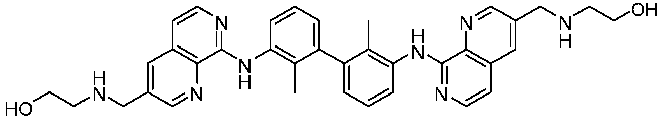

### Step 1: 2-((8-(3-bromo-2-methylphenylamino)-1, 7-naphthyridin-3-yl)amino)ethanol

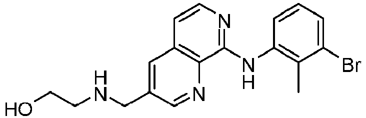

A mixture of 8-((3-bromo-2-methylphenyl)amino)-1,7-naphthyridine-3-carbaldehyde (Example 15, Step 2: 0.100g, 0.292 mmol) and ethanolamine (Aldrich, cat#411000: 0.292 mmol) in 1,2-dichloroethane (1.46 ml) and *N*,*N*-diisopropylethylamine (0.051 ml, 0.292 mmol) was stirred at rt for 1 h. Sodium triacetoxyborohydride (0.093 g, 0.438 mmol) was carefully added in portions. The reaction was stirred at rt for 2 h, then methanol (1 mL) and sodium borohydride (0.584 mmol) were added. The reaction was quenched with a saturated aqueous solution of sodium bicarbonate. The mixture was then extracted with a 3:1 mixture of chloroform/isopropanol. The combined organic layers were dried over sodium sulfate, then concentrated in vacuo. The crude residue was purified by silica gel chromatography (0 → 50% methanol/DCM) to give the desired product. LC-MS calculated for C₁₈H₂₀BrN₄O (M+H)⁺: m/z = 387.1; found 387.2.

### Step 2: (8-((2-methyl-3-(4, 4, 5, 5-tetramethyl-1,3,2-dioxaborolan-2-yl)phenyl)amino)-1, 7-naphthyridin-3-yl) methanol

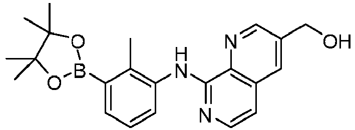

A mixture of (8-((3-bromo-2-methylphenyl)amino)-1,7-naphthyridin-3-yl)methanol (Affinity Research Chemicals, cat#ARI-0169: 0.300 g, 0.872 mmol), bis(pinacolato)diboron (Aldrich, #473294: 0.266 g, 1.046 mmol), dichloro[1,1'-bis(diphenylphosphino)ferrocene]palladium (II) dichloromethane adduct (0.071 g, 0.087 mmol) and potassium acetate (0.214 g, 2.179 mmol) was charged with nitrogen and stirred at 110 °C for 2 h. The crude was diluted with DCM, and then filtered through Celite^{®}. The filtrate was concentrated, and the resulting residue was used directly in the next step without further purification. LC-MS calculated for C₂₂H₂₇BN₃O₃ (M+H)⁺: m/z = 392.2; found 392.3.

### Step 3: 2-((8-(3'-(3-(hydroxymethyl)-1,7-naphthyridin-8-ylamino)-2,2'-dimethylbiphenyl-3-ylamino)-1,7-naphthyridin-3-yl)amino)ethanol

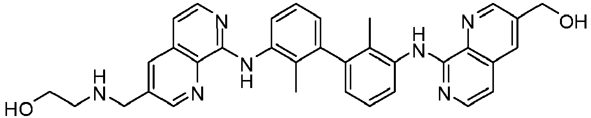

To a vial was added (8-((2-methyl-3-(4,4,5,5-tetramethyl-1,3,2-dioxaborolan-2-yl)phenyl)amino)-1,7-naphthyridin-3-yl)methanol (0.064 g, 0.165 mmol), 2-((8-(3-bromo-2-methylphenylamino)-1,7-naphthyridin-3-yl)methylamino)ethanol (Step 1: 0.058g, 0.150 mmol),1 M aqueous sodium carbonate (0.300 mmol), (1,1'-bis(di-cyclohexylphosphino)ferrocene)-dichloropalladium(II) (10.96 mg, 0.015 mmol), and 1,4-dioxane (1.321 ml). The mixture was degassed, sealed, and heated to 90 °C whilst stirring for 4 h. The mixture was cooled, diluted with EtOAc and filtered through Celite^{®}. The filtrate was concentrated and the crude residue was purified using silica gel chromatography (MeOH/DCM). LC-MS calculated for C₃₄H₃₄N₇O₂ (M+H)⁺: m/z = 572.3; found 572.4.

### Step 4: 8-(3'-(3-((2-hydroxyethylamino)methyl)-1,7-naphthyridin-8-ylamino)-2,2'-dimethylbiphenyl-3-ylamino)-1,7-naphthyridine-3-carbaldehyde

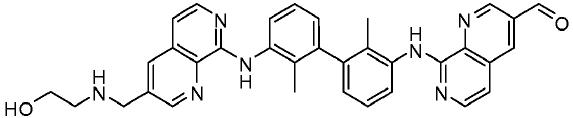

This compound was prepared using similar procedures as described for Example 9 with 2-((8-(3'-(3-(hydroxymethyl)-1,7-naphthyridin-8-ylamino)-2,2'-dimethylbiphenyl-3-ylamino)-1,7-naphthyridin-3-yl)amino)ethanol replacing (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol in *Step 5.* LC-MS calculated for C₃₄H₃₂N₇O₂ (M+H)⁺: m/z = 570.3; found 570.4.

### Step 5: 2,2'-(((((2,2'-dimethyl-[1,1'-biphenyl]-3,3'-diyl)bis(azanediyl))bis(1,7-naphthyridine-8,3-diyl))bis(methylene))bis(azanediyl))bis(ethan-1-ol)

To a vial was added 8-(3'-(3-((2-hydroxyethylamino)methyl)-1,7-naphthyridin-8-ylamino)-2,2'-dimethylbiphenyl-3-ylamino)-1,7-naphthyridine-3-carbaldehyde (0.034 g, 0.065 mmol), ethanolamine (Aldrich, cat#411000: 0.024 mL, 0.194 mmol), dichloromethane (0.997 ml) and *N*,*N*-diisopropylethylamine (0.027 ml, 0.194 mmol). The reaction was stirred at rt for 2 h, then sodium triacetoxyborohydride (0.041 g, 0.194 mmol) and acetic acid (0.011 ml, 0.194 mmol) were added. After 2 h, sodium borohydride (0.130 mmol) and methanol (0.350 mL) were carefully added. The mixture was stirred overnight, then the mixture was diluted with methanol and purified by prep HPLC (pH = 2, acetonitrile/water+TFA) to provide the desired compound as its TFA salt. LC-MS calculated for C₃₆H₃₉N₈O₂ (M+H)⁺: m/z = 615.3; found 615.3.

### Example 20

### (3R,3'R)-1,1'-((((2,2'-dimethyl-[1,1'-biphenyl]-3,3'-diyl)bis(azanediyl))bis(1,7-naphthyridine-8,3-diyl))bis(methylene) )bis(pyrrolidin-3-ol)

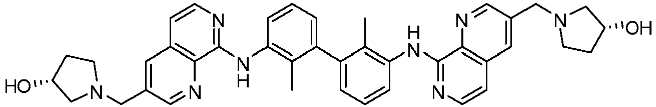

### Step 1: 8-((3-bromo-2-methylphenyl)amino)-1, 7-naphthyridine-3-carbaldehyde

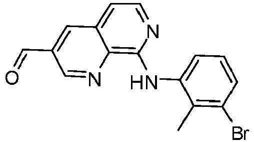

A suspension of (8-((3-bromo-2-methylphenyl)amino)-1,7-naphthyridin-3-yl)methanol (Affinity Research Chemicals, cat#ARI-0169: 300.0 mg, 0.872 mmol) and manganese dioxide (1515 mg, 17.43 mmol) in DCM (8716 µl) was stirred at 45 °C for 1 h. The reaction was filtered through Celite^{®} and the filtrate was concentrated to yield a crude residue, which was used directly in the next step without further purification. LC-MS calculated for C₁₆H₁₃BrN₃O (M+H)⁺: m/z = 342.0; found 342.0.

### Step2: (R)-1-((8-((3-bromo-2-methylphenyl)amino)-1, 7-naphthyridin-3-yl)methyl)pyrrolidin-3-ol

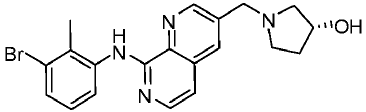

A mixture of 8-((3-bromo-2-methylphenyl)amino)-1,7-naphthyridine-3-carbaldehyde (0.100g, 0.292 mmol) and (*R*)-3-hydroxypyrrolidine (Combi-Blocks, cat#AM-2005: 0.025 g, 0.292 mmol) in 1,2-dichloroethane (1.46 ml) and *N*,*N-*diisopropylethylamine (0.051 ml, 0.292 mmol) was stirred at rt for 1 h. Sodium triacetoxyborohydride (0.093 g, 0.438 mmol) was carefully added in portions. The reaction was stirred at rt for 2 h, then quenched with a saturated aqueous solution of sodium bicarbonate. The mixture was then extracted with a 3:1 mixture of chloroform/isopropanol. The combined organic layers were dried over sodium sulfate, then concentrated in vacuo. The crude residue was purified by silica gel chromatography (0 → 30% methanol/DCM) to give the desired product. LC-MS calculated for C₂₀H₂₂BrN₄0 (M+H)⁺: m/z = 413.1; found 413.1.

### Step 3: (8-((2-methyl-3-(4,4, 5, 5-tetramethyl-1,3,2-dioxaborolan-2-yl)phenyl)amino)-1,7-naphthyridin-3-yl) methanol

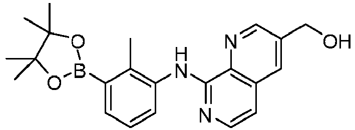

A mixture of (8-((3-bromo-2-methylphenyl)amino)-1,7-naphthyridin-3-yl)methanol (Affinity Research Chemicals, cat#ARI-0169: 0.300 g, 0.872 mmol), bis(pinacolato)diboron (Aldrich, cat#473294: 0.266 g, 1.046 mmol), dichloro[1,1'-bis(diphenylphosphino)ferrocene]palladium (II) dichloromethane adduct (0.071 g, 0.087 mmol) and potassium acetate (0.214 g, 2.179 mmol) was charged with nitrogen and stirred at 110 °C for 2 h. The crude was diluted with DCM, and then filtered through Celite^{®}. The filtrate was concentrated, and the resulting residue was used directly in the next step without further purification. LC-MS calculated for C₂₂H₂₇BN₃O₃ (M+H)⁺: m/z = 392.2; found 392.3.

### Step 4: (R)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol

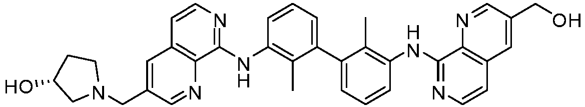

To a vial was added (8-((2-methyl-3-(4,4,5,5-tetramethyl-1,3,2-dioxaborolan-2-yl)phenyl)amino)-1,7-naphthyridin-3-yl)methanol (0.162 g, 0.414 mmol), (*R*)-1-((8-((3-bromo-2-methylphenyl)amino)-1,7-naphthyridin-3-yl)methyl)pyrrolidin-3-ol (0.163g, 0.394 mmol), 1 M aqueous sodium carbonate (0.789 mmol), [1,1'-bis(di-cyclohexylphosphino)ferrocene]-dichloropalladium (II) (0.029 g, 0.039 mmol), and 1,4-dioxane (3.48 ml). The mixture was purged with nitrogen, sealed, and heated to 90 °C whilst stirring for 2 h. The mixture was cooled, diluted with EtOAc and filtered through Celite^{®}. The filtrate was concentrated and purified using silica gel chromatography (20% MeOH/DCM) to provide the desired compound as an orange solid. LC-MS calculated for C₃₆H₃₆N₇O₂ (M+H)⁺: m/z = 598.3; found 598.4.

### Step 5: (R)-8-((3'-((3-((3-hydroxypyrrolidin-1-yl)methyl)-1,7-naphthyridin-8-yl)amino)-2,2'-dimethyl-[1,1'-biphenyl]-3-yl)amino)-1,7-naphthyridine-3-carbaldehyde

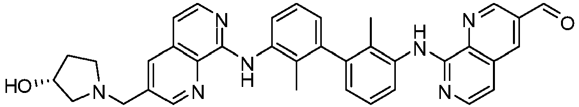

To a solution of (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol (0.0715 g, 0.12 mmol) in DCM (1.20 ml) was added manganese dioxide (0.208 g, 2.392 mmol). The resulting mixture was heated at 45 °C for 30 min. After cooling, the mixture was filtered through Celite^{®} and the filtrate was concentrated. The crude orange solid was used directly in the next step. LC-MS calculated for C₃₆H₃₄N₇O₂ (M+H)⁺: m/z = 596.3; found 596.5.

### Step 6: (3R, 3'R)-1,1'-((((2,2'-dimethyl-[1,1'-biphenyl]-3,3'-diyl)bis(azanediyl))bis(1, 7-naphthyridine-8,3-diyl))bis(methylene))bis(pyrrolidin-3-ol)

To a vial was added (*R*)-8-((3'-((3-((3-hydroxypyrrolidin-1-yl)methyl)-1,7-naphthyridin-8-yl)amino)-2,2'-dimethyl-[1,1'-biphenyl]-3-yl)amino)-1,7-naphthyridine-3-carbaldehyde (0.0085 g, 0.014 mmol), (*R*)-pyrrolidin-3-ol (Combi-Blocks, cat#AM-2005: 4 mg, 0.043 mmol), dichloromethane (0.357 ml) and triethylamine (5.97 µl, 0.043 mmol). The reaction was stirred at rt for 2 h, then sodium triacetoxyborohydride (0.015 g, 0.071 mmol) and acetic acid (2.451 µl, 0.043 mmol) were added. The reaction was stirred for 2 h, then the mixture was diluted with methanol and purified by prep HPLC (pH = 2, acetonitrile/water+TFA) to provide the desired compound as its TFA salt. LC-MS calculated for C₄₀H₄₃N₈O₂ (M+H)⁺: m/z = 667.3; found 667.3. ¹H NMR (600 MHz, DMSO) δ 10.68 (s, 2H), 9.09 (s, 2H), 8.53 (s, 2H), 7.96 (m, 4H), 7.41 (s, 2H), 7.24 (s, 2H), 7.10 (s, 2H), 5.62 (br s, 2H), 4.70 (m, 4H), 4.46 (m, 2H), 3.70-3.10 (ovrlp m, 8H), 2.31 (s, 2H), 2.07 (s, 6H), 1.93 (m, 2H).

### Example 21

### (R)-1-((8-(3'-(3-((2-hydroxyethylantino)methyl)-1,7-naphthyridin-8-ylamino)-2,2'-dimethylbiphenyl-3-ylamino)-1,7-naphthyridin-3-yl)methyl)pyrrolidin-3-ol

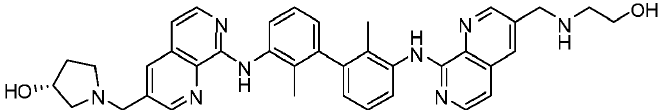

This compound was prepared using similar procedures as described for Example 20 with ethanolamine (Aldrich, cat#411000) replacing (*R*)-pyrrolidin-3-ol in Step 6. The reaction mixture was diluted with methanol and purified by prep HPLC (pH = 2, acetonitrile/water+TFA) to provide the desired compound as its TFA salt. LC-MS calculated for C₃₈H₄₁N₈O₂ (M+H)⁺: m/z = 641.3; found 641.3.

### Example 22

### (3R,3'R)-1,1'-((((2,2'-dichloro-[1,1'-biphenyl]-3,3'-diyl)bis(azanediyl))bis(1,7-naphthyridine-8,3-diyl))bis(methylene))bis(pyrrolidin-3-ol)

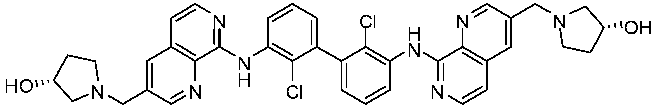

### Step 1: 8-chloro-3-vinyl-1,7-naphthyridine

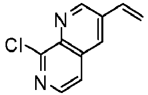

A mixture of 3-bromo-8-chloro-1,7-naphthyridine (PharmaBlock, cat#PBLJ2743: 0.200 g, 0.821 mmol), 4,4,5,5-tetramethyl-2-vinyl-1,3,2-dioxaborolane (Aldrich, cat#663348: 153 µL, 0.904 mmol), sodium carbonate (0.174 g, 1.64 mmol) and [1,1'-bis(di-cyclohexylphosphino)ferrocene]dichloropalladium(II) (Aldrich, cat#701998: 6.2 mg, 0.0082 mmol) in tert-butyl alcohol (5.91 mL, 61.8 mmol) and water (6 mL, 300 mmol) was degassed and sealed. It was stirred at 110 °C for 2 h. The reaction mixture was cooled then extracted with ethyl acetate (3x 20 mL). The combined organic layers were washed with brine, dried over MgSO₄, filtered and concentrated under reduced pressure. The crude residue was used directly in the next step without further purification. LC-MS calculated for C₁₀H₈ClN₂ (M+H)⁺: m/z = 191.0; found 191.0.

### Step 2: 8-chloro-1,7-naphthyridine-3-carbaldehyde

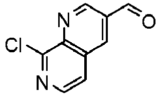

A flask was charged with 8-chloro-3-vinyl-1,7-naphthyridine (391. mg, 2.05 mmol), 1,4-dioxane (40. mL), a stir bar and water (40. mL). To this suspension was added a 4% w/w mixture of osmium tetraoxide in water (0.84 mL, 0.132 mmol). The reaction was stirred for 5 min then sodium periodate (3.23 g, 15.11 mmol) was added and stirred for 3 h. The mixture was diluted with water (20 mL) and EtOAc (20 mL). The layers were separated and the aqueous layer was further extracted with EtOAc (2 X 20 mL). The combined organic extracts were washed with brine, dried over sodium sulfate, filtered, and concentrated in vacuo. The crude aldehyde was purified by silica gel chromatography (0 → 60% EtOAc/hexanes). LC-MS calculated for C₉H₆ClN₂O (M+H)⁺: m/z = 193.0; found 192.9.

### Step 3: (R)-1-((8-chloro-1, 7-naphthyridin-3-yl)methyl)pyrrolidin-3-ol

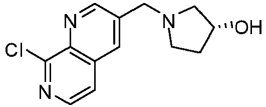

This compound was prepared using similar procedures as described for Example 20 with 8-chloro-1,7-naphthyridine-3-carbaldehyde replacing 8-((3-bromo-2-methylphenyl)amino)-1,7-naphthyridine-3-carbaldehyde in Step 2. The crude amine was purified by silica gel chromatography (0 → 25% MeOH/DCM). LC-MS calculated for C₁₃H₁₅ClN₃O (M+H)⁺: m/z = 264.1; found 264.1.

### Step 4: (R)-1-((8-(3-bromo-2-chlorophenylamino)-1, 7-naphthyridin-3-yl)methyl)pyrrolidin-3-ol

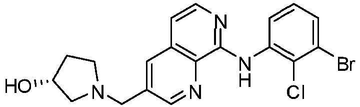

In a vial, 3-bromo-2-chloroaniline (Enamine, cat#EN300-105778: 0.063 g, 0.303 mmol) and (*R*)-1-((8-chloro-1,7-naphthyridin-3-yl)methyl)pyrrolidin-3-ol (0.080 g, 0.303 mmol) were suspended in isopropanol (1.517 ml). Sulfuric acid (0.016 ml, 0.303 mmol) was added to the reaction mixture. The resulting mixture was heated to 100 °C for 1 h. The mixture was cooled, quenched with aqueous saturated sodium bicarbonate, and diluted with 3:1 chloroform/isopropanol. The layers were separated and the water layer was further extracted with 3:1 chloroform/isopropanol. The combined organic layers were dried over magnesium sulfate, filtered and concentrated in vacuo. The crude solid was purified by column chromatography (0→25%Methanol/DCM). LC-MS calculated for C₁₉H₁₉BrClN₄O
(M+H)⁺: m/z = 433.0; found 433.0.

### Step 5: (R)-1-((8-(2-chloro-3-(4,4,5,5-tetramethyl-1,3,2-dioxaborolan-2-yl)phenylamino)-1, 7-naphthyridin-3-yl) methyl)pyrrolidin-3-ol

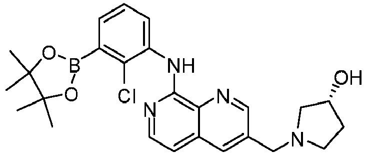

This compound was prepared using similar procedures as described for Example 20 with (*R*)-1-((8-(3-bromo-2-chlorophenylamino)-1,7-naphthyridin-3-yl)methyl)pyrrolidin-3-ol replacing (8-((3-bromo-2-methylphenyl)amino)-1,7-naphthyridin-3-yl)methanol in Step 3. The crude boronic ester was used directly in the next step without further purification. LC-MS calculated for C₂₅H₃₁BClN₄O₃ (M+H)⁺: m/z = 481.2; found 481.2.

### Step 6: (3R, 3'R)-1,1'-((((2,2'-dichloro-[1,1'-biphenyl]-3,3'-diyl)bis(azanediyl))bis(1,7-naphthyridine-8,3-diyl))bis(methylene))bis(pyrrolidin-3-ol)

To a vial was added (*R*)-1-((8-((3-bromo-2-chlorophenyl)amino)-1,7-naphthyridin-3-yl)methyl)pyrrolidin-3-ol (0.010 g, 0.023 mmol), (*R*)-1-((8-((2-chloro-3-(4,4,5,5-tetramethyl-1,3,2-dioxaborolan-2-yl)phenyl)amino)-1,7-naphthyridin-3-yl)methyl)pyrrolidin-3-ol (0.011 g, 0.023 mmol), 1 M aqueous sodium carbonate (0.046 mmol), dioxane (0.231 ml), (1,1'-bis(di-cyclohexylphosphino)ferrocene)-dichloropalladium(II) (1.687 mg, 2.306 µmol), and a stir bar. The mixture was sparged with nitrogen and heated at 90 °C for 2 h. The mixture was diluted with methanol and purified by prep HPLC (pH = 2, acetonitrile/water+TFA) to provide the desired compound as its TFA salt. LC-MS calculated for C₃₈H₃₇Cl₂N₈O₂ (M+H)⁺: m/z = 707.2; found 707.3.

### Example 23

### (R)-1-((4-(3'-(3-(((R)-3-hydroxypyrrolidin-1-yl)methyl)-1,7-naphthyridin-8-ylamino)-2,2'-dimethylbiphenyl-3-ylamino)pyrido[3,2-d]pyrimidin-7-yl)methyl)pyrrolidin-3-ol

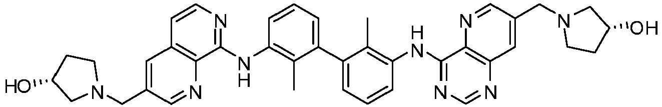

### Step1: (R)-1-((8-(3'-amino-2,2'-dimethylbiphenyl-3-ylamino)-1, 7-naphthyridin-3-yl)methyl)pyrrolidin-3-ol

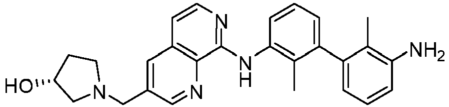

To a vial was added 2-methyl-3-(4,4,5,5-tetramethyl-1,3,2-dioxaborolan-2-yl)aniline (Combi-Blocks, cat#PN-9127: 0.108 g, 0.465 mmol), (*R*)-1-((8-((3-bromo-2-methylphenyl)amino)-1,7-naphthyridin-3-yl)methyl)pyrrolidin-3-ol (Example 15, Step 3: 0.192g, 0.465 mmol), 1 M aqueous sodium carbonate (0.929 mmol), (1,1'-bis(dicyclohexylphosphino)ferrocene)-dichloropalladium(II) (0.034 g, 0.046 mmol), and 1,4-dioxane (3.10 mL). The mixture was degassed, sealed, and heated to 90 °C whilst stirring for 4 h. The mixture was cooled, diluted with EtOAc and filtered through celite. The filtrate was concentrated and the crude solid was purified by column chromatography (0→25%Methanol/DCM). LC-MS calculated for C₂₇H₃₀N₅O (M+H)⁺: m/z = 440.2; found 440.3.

### Step 2: (R)-1-((8-(3 '-(7-bromopyrido[3, 2-d]pyrimidin-4-ylamino)-2,2'-dimethylbiphenyl-3-ylamino)-1,7-naphthyridin-3-yl)methyl)pyrrolidin-3-ol

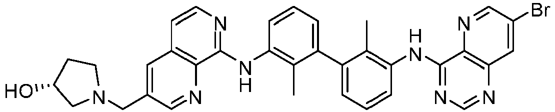

To a vial was added 7-bromo-4-chloropyrido[3,2-d]pyrimidine (Synthonix, cat# B0473: 0.187g, 0.765 mmol), (*R*)-1-((8-((3'-amino-2,2'-dimethyl-[1,1'-biphenyl]-3-yl)amino)-1,7-naphthyndin-3-yl)methyl)pyrrolidm-3-ol (0.336 g, 0.765 mmol), 2-propanol (3.82 ml), a stir bar and sulfuric acid (0.041 ml, 0.765 mmol). The mixture was heated to 100 °C for 2 h. After cooling to rt, the mixture was diluted with 3:1 CHCl₃/isopropanol and aqueous saturated sodium bicarbonate. The layers were separated, and the aqueous phase was further extracted. The combined organic layers were dried over MgSO₄, filtered, and concentrated in vacuo. The crude solid was washed with ether to provide the desired product as a yellow solid. LC-MS calculated for C₃₄H₃₂BrN₈O (M+H)⁺: m/z = 647.2; found 647.3.

### Step 3: (R)-1-((8-(2,2'-dimethyl-3'-(7-vinylpyrido[3,2-d]pyrimidin-4-ylamino)biphenyl-3-ylamino)-1,7-naphthyridin-3-yl)methyl)pyrrolidin-3-ol

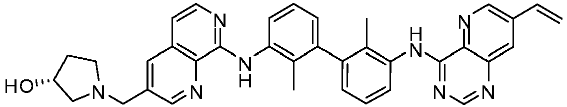

A mixture of (*R*)-1-((8-((3'-((7-bromopyrido[3,2-d]pyrimidin-4-yl)amino)-2,2'-dimethyl-[1,1'-biphenyl]-3-yl)amino)-1,7-naphthyridin-3-yl)methyl)pyrrolidin-3-ol (0.248 g, 0.383 mmol), 4,4,5,5-tetramethyl-2-vinyl-1,3,2-dioxaborolane (0.130 ml, 0.766 mmol), sodium carbonate (0.074 ml, 0.766 mmol) and (1,1'-bis(di-cyclohexylphosphino)ferrocene)-dichloropalladium(II) (0.015 g, 0.019 mmol) in 1,4-dioxane (1.915 mL) and water (0.479 mL) was degassed and sealed. It was stirred at 90 °C for 1.5 h. The mixture was cooled to rt, and water and 3:1 chloroform/isopropanol were added. The layers were separated and the aqueous layer was further extracted with 3:1 chloroform/isopropanol. The combined organic layers were dried over Na₂SO₄, filtered, and concentrated in vacuo. The crude solid was then washed with ether to provide the desired compound as a yellow solid. LC-MS calculated for C₃₆H₃₅N₈O (M+H)⁺: m/z = 595.3; found 595.3.

### Step 4: (R)-4-(3'-(3-((3-hydroxypyrrolidin-1-yl)methyl)-1, 7-naphthyridin-8-ylamino)-2,2'-dimethylbiphenyl-3-ylamino)pyrido[3,2-d]pyrimidine-7-carbaldehyde

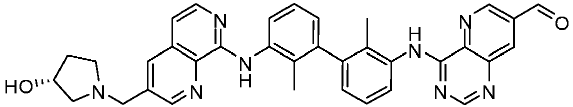

To a flask was added (*R*)-1-((8-((2,2'-dimethyl-3'-((7-vinylpyrido[3,2-d]pyrimidin-4-yl)amino)-[1,1'-biphenyl]-3-yl)amino)-1,7-naphthyridin-3-yl)methyl)pyrrolidin-3-ol (0.128 g, 0.215 mmol), THF (3.8 mL), water (1 mL), sodium periodate (0.655 g, 3.06 mmol), and 4% osmium tetroxide solution in water (0.170 ml, 0.027 mmol). The resulting mixture was stirred for 1 h at rt. The mixture was diluted with water and 3:1 CHCl₃/isopropanol and the layers were separated. The aqueous layer was further extracted with CHCl₃/isopropanol (3:1). The combined organic layers were washed dried over MgSO₄, filtered, and concentrated in vacuo. The resulting solid was washed with ether to provide the desired product as a brown solid. LC-MS calculated for C₃₅H₃₃N₈O2 (M+H)⁺: m/z = 597.3; found 597.5.

### Step 5: (R)-1-((4-(3'-(3-(((R)-3-hydroxypyrrolidin-1-yl)methyl)-1, 7-naphthyridin-8-ylamino)-2,2'-dimethylbiphenyl-3-ylamino)pyrido[3,2-d]pyrimidin-7-yl)methyl)pyrrolidin-3-ol

To a vial was added (*R*)-4-((3'-((3-((3-hydroxypyrrolidin-1-yl)methyl)-1,7-naphthyridin-8-yl)amino)-2,2'-dimethyl-[1,1'-biphenyl]-3-yl)amino)pyrido[3,2-d]pyrimidine-7-carbaldehyde (0.064 g, 0.107 mmol), (*R*)-pyrrolidin-3-ol (Combi-Blocks, cat#AM-2005: 0.037 g, 0.322 mmol), 1,2-dichloroethane (1.073 ml) and triethylamine (0.045 ml, 0.322 mmol). The reaction was stirred at rt for 2 h, then sodium triacetoxyborohydride (0.114 g, 0.536 mmol) and acetic acid (0.018 ml, 0.322 mmol) were added. The reaction was stirred for 2 h, then the mixture was diluted with methanol and purified by prep HPLC (pH = 2, acetonitrile/water+TFA) to provide the desired compound as its TFA salt. LC-MS calculated for C₃₉H₄₂N₉O₂ (M+H)⁺: m/z = 668.3; found 668.3.

### Example 24

### (R)-1-((8-(3'-(7-((2-hydroxyethylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol

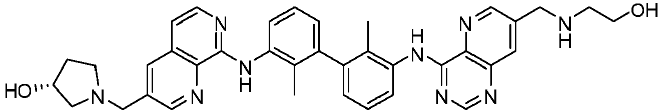

This compound was prepared using similar procedures as described for Example 23 with ethanolamine (Aldrich, cat#411000) replacing (R)-pyrrolidin-3-ol in Step 5. The reaction mixture was diluted with methanol and purified by prep HPLC (pH = 2, acetonitrile/water+TFA) to provide the desired compound as its TFA salt. LC-MS calculated for C₃₇H₄₀N₉O₂ (M+H)⁺: m/z = 642.3; found 642.3.

### Example 25

### (R)-1-((8-(3'-(7-(((2-hydroxyethyl)(methyl)amino)methyl)pyrido[3,2-d]pyrimidin-4-ylamino)-2,2'-dimethylbiphenyl-3-ylamino)-1,7-naphthyridin-3-yl)methyl)pyrrolidin-3-ol

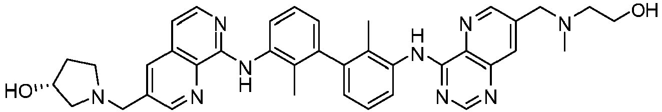

This compound was prepared using similar procedures as described for Example 23 with 2-(methylamino)ethanol (Aldrich, cat#471445) replacing (*R*)-pyrrolidin-3-ol in Step 5. The reaction mixture was diluted with methanol and purified by prep HPLC (pH = 2, acetonitrile/water+TFA) to provide the desired compound as its TFA salt. LC-MS calculated for C₃₈H₄₂N₉O₂ (M+H)⁺: m/z = 656.3; found 656.4.

### Example 26

### (R)-1-((8-(3'-(3-(((R)-3-hydroxypyrrolidin-1-yl)methyl)-1,7-naphthyridin-8-ylamino)-2,2'-dimethylbiphenyl-3-ylamino)-1,7-naphthyridin-3-yl)methyl)-N,N-dimethylpyrrolidine-3-carboxamide

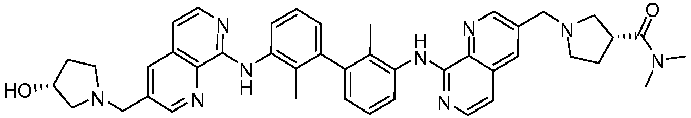

A mixture of (*R*)-1-((8-((3'-((3-(((R)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid (Example 9, Step 6: 0.007g, 5.08 µmol), 2.0 M dimethylamine in THF (0.102 mmol), HATU (2.316 mg, 6.09 µmol), and N,N-diisopropylethylamine (8.84 µl, 0.051 mmol) in DMF (0.051 ml) was stirred at r.t. for 2 h. The reaction mixture was diluted with methanol and purified by prep HPLC (pH = 2, acetonitrile/water+TFA) to provide the desired compound as its TFA salt. LC-MS calculated for C₄₃H₄₈N₉O₂ (M+H)⁺: m/z = 722.4; found 722.4.

### Example 27

### (R)-1-((8-(3'-(7-(((S)-2-hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol

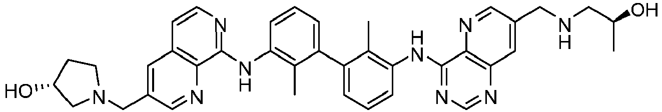

This compound was prepared using similar procedures as described for Example 23 with (*S*)-(+)-1-amino-2-propanol (Aldrich, cat# 238864) replacing (*R*)-pyrrolidin-3-ol in Step 5. The reaction mixture was diluted with methanol and purified by prep HPLC (pH = 2, acetonitrile/water+TFA) to provide the desired compound as its TFA salt. LC-MS calculated for C₃₈H₄₂N₉O₂ (M+H)⁺: m/z = 656.3; found 656.3.

### Example 28

### (R)-1-((8-(3'-(5-(((R)-3-hydroxypyrrolidin-1-yl)methyl)-1-methyl-2-oxo-1,2-dihydropyridine-3-carboxamido)-2,2'-dimethylbiphenyl-3-ylamino)-1,7-naphthyridin-3-yl)methyl)pyrrolidine-3-carboxylic acid

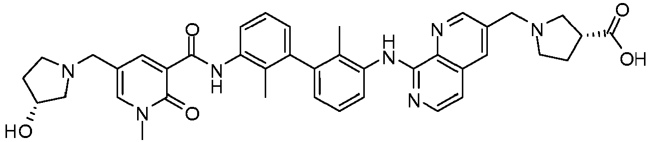

### Step1: N-(3-bromo-2-methylphenyl)-1-methyl-2-oxo-5-vinyl-1,2-dihydropyridine-3-carboxamide

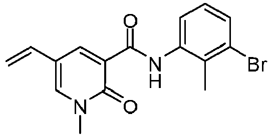

A mixture of 1-methyl-2-oxo-5-vinyl-1,2-dihydropyridine-3-carboxylic acid (Example 15, Step 6: 1.3 g, 7.26 mmol), 3-bromo-2-methylaniline (Aldrich, cat#530018: 0.894 ml, 7.26 mmol), HATU (3.31 g, 8.71 mmol), and *N*,*N*-diisopropylethylamine (2.53 ml, 14.51 mmol) in 1,2-dichloroethane (36.3 ml) was stirred at rt for 2 h. The mixture was concentrated under reduced pressure. The residue was diluted with ethyl acetate, and the resulting mixture was washed with water and brine. The organic layer was dried over MgSO₄, filtered and concentrated under reduced pressure. The crude residue was used directly in the next step without further purification. LC-MS calculated for C₁₆H₁₆BrN₂O₂ (M+H)⁺: m/z = 347.0; found 347.0.

### Step 2: N-(3-bromo-2-methylphenyl)-5-formyl-1-methyl-2-oxo-1,2-dihydropyridine-3-carboxamide

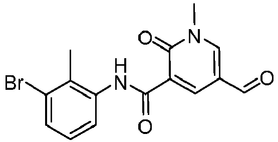

This compound was prepared using similar procedures as described for Example 23, Step 4 with N-(3-bromo-2-methylphenyl)-1-methyl-2-oxo-5-vinyl-1,2-dihydropyridine-3-carboxamide replacing (*R*)-1-((8-((2,2'-dimethyl-3'-((7-vinylpyrido[3,2-d]pyrimidin-4-yl)amino)-[1,1'-biphenyl]-3-yl)amino)-1,7-naphthyridin-3-yl)methyl)pyrrolidin-3-ol . The crude aldehyde was purified by silica gel chromatography (5% MeOH/DCM). LC-MS calculated for C₁₅H₁₄BrN₂O₃ (M+H)⁺: m/z = 349.0; found 349.1.

### Step 3: (R)-N-(3-bromo-2-methylphenyl)-5-((3-hydroxypyrrolidin-1-yl)methyl)-1-methyl-2-oxo-1,2-dihydropyridine-3-carboxamide

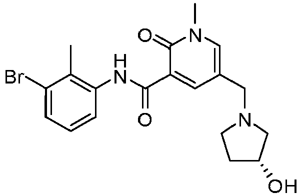

This compound was prepared using similar procedures as described for Example 20, Step 2 with N-(3-bromo-2-methylphenyl)-5-formyl-1-methyl-2-oxo-1,2-dihydropyridine-3-carboxamide replacing 8-((3-bromo-2-methylphenyl)amino)-1,7-naphthyridine-3-carbaldehyde. The crude amine was purified by silica gel chromatography (20% MeOH/DCM). LC-MS calculated for C₁₉H₂₃BrN₃O₃ (M+H)⁺: m/z = 420.1; found 420.1.

### Step 4: (R)-N-(3'-(3-(hydroxymethyl)-1,7-naphthyridin-8-ylamino)-2,2'-dimethylbiphenyl-3-yl)-5-((3-hydroxypyrrolidin-1-yl)methyl)-1-methyl-2-oxo-1,2-dihydropyridine-3-carboxamide

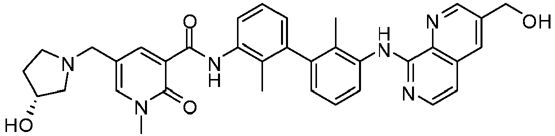

This compound was prepared using similar procedures as described for Example 20, Step 4 with (*R*)-N-(3-bromo-2-methylphenyl)-5-((3-hydroxypyrrolidin-1-yl)methyl)-1-methyl-2-oxo-1,2-dihydropyridine-3-carboxamide replacing (*R*)-1-((8-((3-bromo-2-methylphenyl)amino)-1,7-naphthyridin-3-yl)methyl)pyrrolidin-3-ol. The crude amine was purified by silica gel chromatography (20% MeOH/DCM). LC-MS calculated for C₃₅H₃₇N₆O₄ (M+H)⁺: m/z = 605.3; found 605.3.

### Step 5: (R)-N-(3'-(3-formyl-1,7-naphthyridin-8-ylamino)-2,2'-dimethylbiphenyl-3-yl)-5-((3-hydroxypyrrolidin-1-yl)methyl)-1-methyl-2-oxo-1, 2-dihydropyridine-3-carboxamide

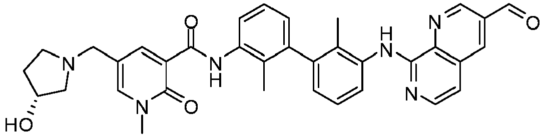

This compound was prepared using similar procedures as described for Example 20, Step 5 with (*R*)-N-(3'-(3-(hydroxymethyl)-1,7-naphthyridin-8-ylamino)-2,2'-dimethylbiphenyl-3-yl)-5-((3-hydroxypyrrolidin-1-yl)methyl)-1-methyl-2-oxo-1,2-dihydropyridine-3-carboxamide replacing (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol. LC-MS calculated for C₃₅H₃₅N₆O₄ (M+H)⁺: m/z = 603.3; found 603.3.

### Step 6: (R)-1-((8-(3'-(5-(((R)-3-hydroxypyrrolidin-1-yl)methyl)-1-methyl-2-oxo-1,2-dihydropyridine-3-carboxamido)-2, 2'-dimethylbiphenyl-3ylamino)-1, 7-naphthyridin-3-yl)methyl)pyrrolidine-3-carboxylic acid

To a vial was added (*R*)-*N*-(3'-(3-formyl-1,7-naphthyridin-8-ylamino)-2,2'-dimethylbiphenyl-3-yl)-5-((3-hydroxypyrrolidin-1-yl)methyl)-1-methyl-2-oxo-1,2-dihydropyridine-3-carboxamide (0.010 g, 0.017 mmol), (*R*)-pyrrolidine-3-carboxylic acid (Combi-Blocks, cat#ST-7698: 6 mg, 0.050 mmol), 1,2-dichloroethane (0.4 ml) and triethylamine (6.94 µl, 0.050 mmol). The reaction was stirred at rt for 2 h, then sodium triacetoxyborohydride (0.018 g, 0.083 mmol) and acetic acid (2.85 µl, 0.050 mmol) were added. The reaction was stirred for 2 h, then the mixture was diluted with methanol and purified by prep HPLC (pH = 2, acetonitrile/water+TFA) to give the desired product as the TFA salt. LC-MS calculated for C₄₀H₄₄N₇O₅ (M+H)⁺: m/z = 702.3; found 702.3.

### Example 29

### (R)-1-((8-(3'-(7-(((R)-2-hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol

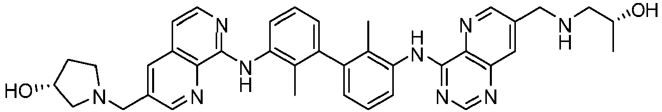

This compound was prepared using similar procedures as described for Example 23 with (R)-(+)-1-amino-2-propanol (Aldrich, cat# 238856) replacing (*R*)-pyrrolidin-3-ol in Step 5. The reaction mixture was diluted with methanol and purified by prep HPLC (pH = 2, acetonitrile/water+TFA) to provide the desired compound as its TFA salt. LC-MS calculated for C₃₈H₄₂N₉O₂ (M+H)⁺: m/z = 656.3; found 656.4.

### Example 30

### (R)-1-((8-(3'-(7-((2-hydroxy-2-methylpropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol

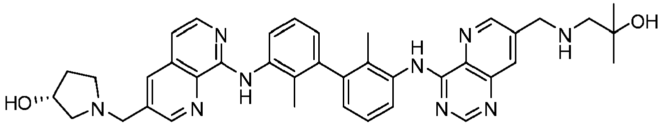

This compound was prepared using similar procedures as described for Example 23 with 1-amino-2-methyl-2-propanol (Aldrich, cat# 777625) replacing (*R*)-pyrrolidin-3-ol in Step 5. The reaction mixture was diluted with methanol and purified by prep HPLC (pH = 2, acetonitrile/water+TFA) to provide the desired compound as its TFA salt. LC-MS calculated for C₃₉H₄₄N₉O₂ (M+H)⁺: m/z = 670.3; found 670.4.

### Example 31

### (R)-1-((8-(2'-chloro-3'-(1,5-dimethyl-4,5,6,7-tetrahydro-1H-imidazo[4,5-c]pyridine-2-carboxamido)-2-methylbiphenyl-3-ylamino)-1,7-naphthyridin-3-yl)methyl)pyrrolidine-3-carboxylic acid

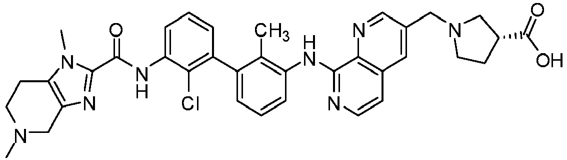

### Step 1: tert-butyl 1-methyl-1,4,6,7-tetrahydro-5H-imidazo[4,5-c]pyridine-5-carboxylate

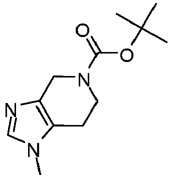

A solution of 1-methyl-4,5,6,7-tetrahydro-1H-imidazo[4,5-c]pyridine (Accela, cat#SY032476: 2.0 g, 14.58 mmol) and (Boc)₂O (3.38 mL, 14.58 mmol) in dichloromethane (60.0 mL) was stirred at room temperature for 1 h. The reaction was quenched with saturated aqueous NaHCO₃ solution, and extracted with ethyl acetate. The combined organic layers were washed with brine, dried over Na₂SO₄, filtered, and concentrated under reduced pressure. The crude product was used for next step without further purification. LC-MS calculated for C₁₂H₂₀N₃O₂ (M+H)⁺: m/z = 238.2; found 238.2.

### Step 2: 5-tert-butyl 2-methyl 1-methyl-6,7-dihydro-1H-imidazo[4,5-c]pyridine-2,5(4H)-dicarboxylate

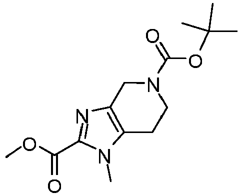

*n*-Butyllithium in hexanes (2.5 M, 7.00 mL, 17.49 mmol) was added to a cold (-78 °C) solution of the crude product from Step 1 in tetrahydrofuran (60.0 mL). The reaction mixture was stirred at -78 °C for 10 min prior to the addition of methyl chloroformate (1.7 mL, 21.9 mmol). After being stirred at -78 °C for 15 min, the reaction was then quenched with saturated aqueous NaHCO₃ solution, and extracted with ethyl acetate, dried over Na₂SO₄, filtered, and concentrated under reduced pressure. The residue was purified by flash chromatography on a silica gel column eluting with 80% ethyl acetate in hexanes to afford the desired product (). LC-MS calculated for C₁₄H₂₂N₃O₄ (M+H)⁺: m/z = 296.2; found 296.3.

### Step 3: tert-butyl 2-((3-bromo-2-chlorophenyl)carbamoyl)-1-methyl-1,4,6,7-tetrahydro-5H-imidazo[4,5-c]pyridine-5-carboxylate

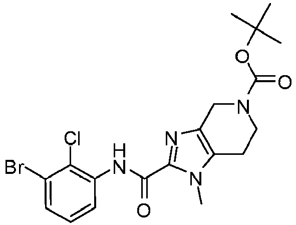

Potassium *tert*-butoxide in tetrahydrofuran (1.0 M, 3.39 mL, 3.39 mmol) was added to a solution of 5-*tert*-butyl 2-methyl 1-methyl-6,7-dihydro-1H-imidazo[4,5-c]pyridine-2,5(4H)-dicarboxylate (Step 2: 500 mg, 1.69 mmol) and 3-bromo-2-chloroaniline (348 mg, 1.69 mmol) in tetrahydrofuran (12.0 mL). After being stirred at room temperature for 1 h, the reaction mixture was quenched with water, and extracted with ethyl acetate. The combined organic layers were washed with brine, dried over Na₂SO₄, filtered, and concentrated under reduced pressure. The residue was purified by flash chromatography on a silica gel column eluting with 50% ethyl acetate in hexanes to afford the desired product. LC-MS calculated for C₁₉H₂₃BrClN₄O₃ (M+H)⁺: m/z = 469.1/471.1; found 469.1/471.1.

### Step 4: N-(3-bromo-2-chlorophenyl)-1,5-dimethyl-4,5,6,7-tetrahydro-1H-imidazo[4,5-c]pyridine-2-carboxamide

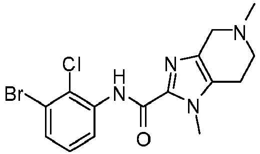

A solution of *tert*-butyl 2-((3-bromo-2-chlorophenyl)carbamoyl)-1-methyl-1,4,6,7-tetrahydro-5H-imidazo[4,5-c]pyridine-5-carboxylate *(Step 3*: 300 mg, 0.64 mmol) in trifluoroacetic acid (0.2 mL) and dichloromethane (0.4 mL) was stirred at room temperature for 1 h. The solvent was evaporated, and the residue was dissolved in tetrahydrofuran (1.0 mL). 37% formaldehyde in water (0.48 mL, 6.39 mmol) and sodium triacetoxyborohydride (406 mg, 1.92 mmol) were successively added. After being stirred at room temperature for 1 h, the mixture was quenched with sat. aq. NaHCO₃ solution and was extracted with ethyl acetate. The combined organic layers were washed with brine, dried over Na₂SO₄, filtered and concentrated under reduced pressure. The residue was purified by flash chromatography on a silica gel column eluting with 10% methanol in dichloromethane to afford the desired product. LC-MS calculated for C₁₅H₁₇BrClN₄O (M+H)⁺: m/z = 383.0/385.0; found 383.0/385.0.

### Step 5: N-(2-chloro-3'-((3-(hydroxymethyl)-1,7-naphthyridin-8-yl)amino)-2'-methyl-[1,1'-biphenyl]-3-yl)-1,5-dimethyl-4,5,6,7-tetrahydro-1H-imidazo[4,5-c]pyridine-2-carboxamide

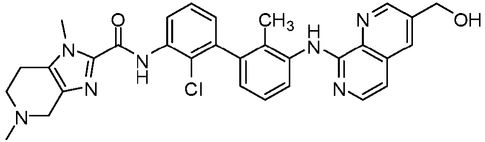

A mixture of N-(3-bromo-2-chlorophenyl)-1,5-dimethyl-4,5,6,7-tetrahydro-1H-imidazo[4,5-c]pyridine-2-carboxamide (Step 4: 60 mg, 0.156 mmol), (8-((2-methyl-3-(4,4,5,5-tetramethyl-1,3,2-dioxaborolan-2-yl)phenyl)amino)-1,7-naphthyridin-3-yl)methanol (Example 9, Step 3: 73.4 mg, 0.188 mmol), sodium carbonate (66.3 mg, 0.626 mmol) and [1,1'-bis(di-cyclohexylphosphino)ferrocene]dichloropalladium(II) (11.8 mg, 0.016 mmol) in 1,4-dioxane (0.8 mL) and water (0.8 mL) was charged with nitrogen and stirred at 100 °C for 2 h. The reaction mixture was diluted with water and extracted with ethyl acetate. The combined organic layers were washed with brine, dried over Na₂SO₄, filtered, and concentrated under reduced pressure. The residue was purified by flash chromatography on a silica gel column eluting with 10% methanol in dichloromethane to afford the desired product. LC-MS calculated for C₃₁H₃₁ClN₇O₂ (M+H)⁺: m/z = 568.2; found 568.3.

### Step 6: N-(2-chloro-3'-((3-formyl-1,7-naphthyridin-8-yl)amino)-2'-methyl-[1,1'-biphenyl]-3-yl)-1,5-dimethyl-4,5,6,7-tetrahydro-1H-imidazo[4,5-c]pyridine-2-carboxamide

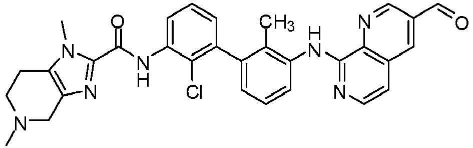

A suspension of N-(2-chloro-3'-((3-(hydroxymethyl)-1,7-naphthyridin-8-yl)amino)-2'-methyl-[1,1'-biphenyl]-3-yl)-1,5-dimethyl-4,5,6,7-tetrahydro-1H-imidazo[4,5-c]pyridine-2-carboxamide (Step 5: 40 mg, 0.070 mmol) and manganese dioxide (92 mg, 1.056 mmol) in dichloromethane (0.5 mL) was stirred at 45 °C for 30 min. The reaction was filtered through a short pad of Celite^{®} and then concentrated to yield a crude residue, which was used directly without further purification. LC-MS calculated for C₃₁H₂₉ClN₇O₂ (M+H)⁺: m/z = 566.2; found 566.2.

### Step 7: (R)-1-((8-(2'-chloro-3'-(1,5-dimethyl-4,5,6, 7-tetrahydro-1H-imidazo[4,5-c]pyridine-2-carboxamido)-2-methylbiphenyl-3-ylamino)-1,7-naphthyridin-3yl)methyl)pyrrolidine-3-carboxylic acid

A mixture of N-(2-chloro-3'-((3-formyl-1,7-naphthyridin-8-yl)amino)-2'-methyl-[1,1'-biphenyl]-3-yl)-1,5-dimethyl-4,5,6,7-tetrahydro-1H-imidazo[4,5-c]pyridine-2-carboxamide *(Step 6:* 39.9 mg, 0.070 mmol) and (*R*)-pyrrolidine-3-carboxylic acid (24.3 mg, 0.211 mmol) in dichloromethane (0.5 mL) was stirred at room temperature for 1 h. Then sodium triacetoxyborohydride (14.92 mg, 0.070 mmol) and acetic acid (4.03 µl, 0.070 mmol) was added. After being stirred at room temperature for 1 h, the reaction was diluted with MeOH and then purified by prep-HPLC (pH = 10, acetonitrile/water+NH₄OH) to give the desired product. LC-MS calculated for C₃₆H₃₈ClN₈O₃ (M+H)⁺: m/z = 665.3; found 665.4.

### Example 32

### (S)-N-(2-chloro-3'-(3-((3-hydroxypyrrolidin-1-yl)methyl)-1,7-naphthyridin-8-ylamino)-2'-methylbiphenyl-3-yl)-1,5-dimethyl-4,5,6,7-tetrahydro-1H-imidazo[4,5-c]pyridine-2-carboxamide

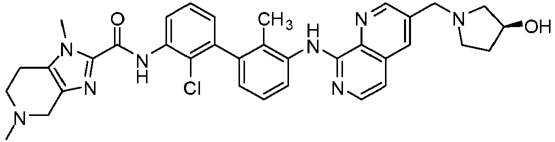

This compound was prepared using similar procedures as described for Example 31, Step 7 with (*S*)-pyrrolidin-3-ol (Combi-Blocks, cat#SS-7948) replacing (*R*)-pyrrolidine-3-carboxylic acid. The reaction mixture was purified by prep-HPLC (pH = 10, acetonitrile/water+NH₄OH) to give the desired product. LC-MS calculated for C₃₅H₃₈ClN₈O₂ (M+H)⁺: m/z = 637.3; found 637.4.

### Example 33

### (R)-1-((8-(2'-chloro-3'-(1,5-dimethyl-4,5,6,7-tetrahydro-1H-imidazo[4,5-c]pyridine-2-carboxamido)-2-methylbiphenyl-3-ylamino)-1,7-naphthyridin-3-yl)methyl)-3-methylpyrrolidine-3-carboxylic acid

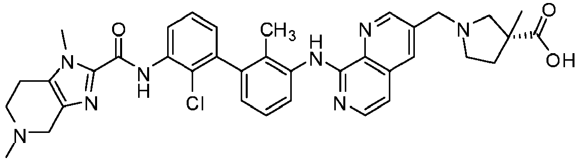

This compound was prepared using similar procedures as described for Example 31, Step 7 with (*R*)-3-methylpyrrolidine-3-carboxylic acid (Ark Pharm, cat#AK601708) replacing (*R*)-pyrrolidine-3-carboxylic acid. The reaction mixture was purified by prep-HPLC (pH = 2, acetonitrile/water+TFA) to give the desired product. LC-MS calculated for C₃₇H₄₀ClN₈O₃ (M+H)⁺: m/z = 679.3; found 679.2.

### Example 34

### (R)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidine-3-carboxylic acid

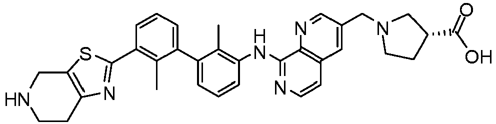

### Step 1: tert-butyl 2-(3'-((3-(hydroxymethyl)-1,7-naphthyridin-8-yl)amino)-2,2'-dimethyl-[1,1'-biphenyl]-3-yl)-6,7-dihydrothiazolo[5,4-c]pyridine-5(4H)-carboxylate

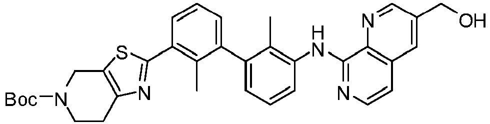

This compound was prepared using similar procedures as described for Example 1, Step 7 with (8-((3-bromo-2-methylphenyl)amino)-1,7-naphthyridin-3-yl)methanol (Affinity Research Chemicals, #ARI-0169) replacing 2-(((8-((3-bromo-2-chlorophenyl)amino)-1,7-naphthyridin-3-yl)methyl)amino)ethan-1-ol. After 5 h, saturated aqueous NaHCO₃ (5 mL) solution was added to the reaction mixture followed by extraction with dichloromethane (5 mL x 3). The combined organic layers were dried over Na₂SO₄, filtered and concentrated. The crude product was used for next step without further purification. LC-MS calculated for C₃₄H₃₆N₅O₃S (M+H)⁺: m/z = 594.2; found 594.3.

### Step 2: tert-butyl 2-(3'-((3-formyl-1, 7-naphthyridin-8-yl)amino)-2,2'-dimethyl-[1,1'-biphenyl]-3-yl)-6,7-dihydrothiazolo[5,4-c]pyridine-5(4H)-carboxylate

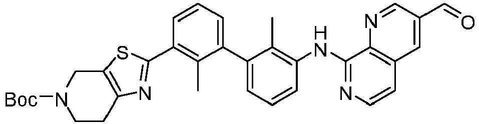

To a solution of *tert*-butyl 2-(3'-((3-(hydroxymethyl)-1,7-naphthyridin-8-yl)amino)-2,2'-dimethyl-[1,1'-biphenyl]-3-yl)-6,7-dihydrothiazolo[5,4-c]pyridine-5(4H)-carboxylate (130 mg, 0.22 mmol) in DCM (2 mL) was added Dess-Martin periodinane (186 mg, 0.44 mmol). After 1 h, saturated NaHCO₃ (5 mL) was added to the reaction mixture followed by extraction with dichloromethane (5 mL x 3). The combined organic layers were dried Na₂SO₄, filtered and concentrated. The crude product was used for next step without further purification. LC-MS calculated for C₃₄H₃₄N₅O₃S (M+H)⁺: m/z = 592.2 ; found 592.3.

### Step 3: (R)-1-((8-((2,2'-dimethyl-3'-(4,5,6,7-tetrahydrothiazolo[5,4-c]pyridin-2-yl)-[1,1'-biphenyl]-3-yl)amino)-1, 7-naphthyridin-3-yl)methyl)pyrrolidine-3-carboxylic acid

To a solution of *tert*-butyl 2-(3'-((3-formyl-1,7-naphthyridin-8-yl)amino)-2,2'-dimethyl-[1,1'-biphenyl]-3-yl)-6,7-dihydrothiazolo[5,4-c]pyridine-5(4H)-carboxylate (10 mg, 0.017 mmol) and DIPEA (5 uL) in DCM (0.5 mL) was added (*R*)-pyrrolidine-3-carboxylic acid (5.8 mg, 0.05 mmol). After 1 h, sodium triacetoxyborohydride (6.6 mg, 0.033 mmol) was added to the reaction mixture. After 2 h, TFA (0.5 mL) was added to the reaction mixture. After another 1 h, the reaction mixture was concentrated, then dissolved in MeOH and purified by prep HPLC (pH = 2, acetonitrile/water+TFA) to provide the compound as the TFA salt. LC-MS calculated for C₃₄H₃₅N₆O₂S (M+H)⁺: m/z = 591.2; found 591.3.

### Example 35

### (R)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol

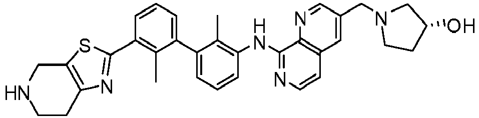

This compound was prepared using similar procedures as described for Example 34 with (*R*)-3-hydroxypyrrolidine replacing (*R*)-pyrrolidine-3-carboxylic acid in Step 3. The reaction mixture was diluted with MeOH then purified by prep-HPLC (pH = 2, acetonitrile/water+TFA) to give the desired product as the TFA salt. LC-MS calculated for C₃₃H₃₅N₆OS (M+H)⁺: m/z = 563.3; found 563.3.

### Example 36

### (S)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol

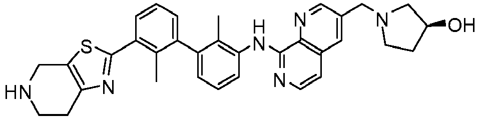

This compound was prepared using similar procedures as described for Example 34 with (*S*)-3-hydroxypyrrolidine replacing (*R*)-pyrrolidine-3-carboxylic acid in Step 3. The reaction mixture was diluted with MeOH then purified by prep-HPLC (pH = 2, acetonitrile/water+TFA) to give the desired product as the TFA salt. LC-MS calculated for C₃₃H₃₅N₆OS (M+H)⁺: m/z = 563.3; found 563.3.

### Example 37

### (R)-2-(dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one

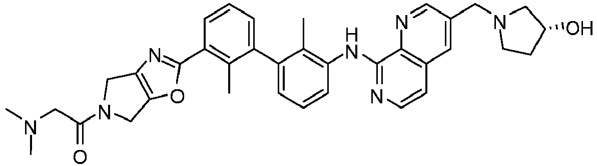

### Step 1: Benzyl 6-oxa-3-azabicyclo[3.1.0]hexane-3-carboxylate

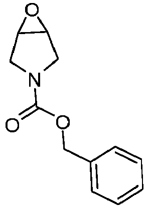

To a solution of benzyl 2,5-dihydro-1H-pyrrole-1-carboxylate (12.4 g, 61.0 mmol) in DCM (200 ml) was added m-CPBA (16.20 g, 61.0 mmol). The resulting mixture was stirred at room temperature for 3h. The reaction was quenched with saturated aqueous NaHCO₃ solution, the organic layer was separated, and the aqueous layer was extracted with DCM. The combined organic layers were dried over Na₂SO₄, filtered and concentrated. The crude product was purified using flash chromatography (eluting with 0-50% ethyl acetate in hexanes) to give the desired product as clear oil (13 g, 97%). LC-MS calculated for C₁₂H₁₄NO₃ (M+H)⁺: m/z = 220.1; found 220.1.

### Step 2: Benzyl 3-amino-4-hydroxypyrrolidine-1-carboxylate

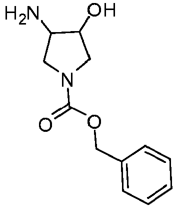

To a flask was charged with benzyl 6-oxa-3-azabicyclo[3.1.0]hexane-3-carboxylate (13.0 g, 59.3 mmol) and ammonium hydroxide (115 ml, 2.96 mol). The reaction mixture was heated at 90 °C overnight. The solvent was removed. The residue was used in the next step without further purification. LC-MS calculated for C₁₂H₁₇N₂O₃ (M+H)⁺: m/z = 237.1; found 237.1.

### Step 3: Benzyl 3-(3-bromo-2-methylbenzamido)-4-hydroxypyrrolidine-1-carboxylate

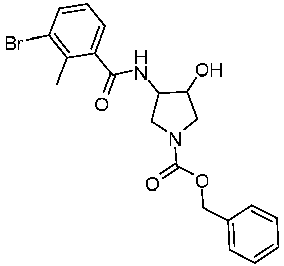

A solution of 3-bromo-2-methylbenzoic acid (9.70 g, 45.1 mmol) in *N,N-*dimethylformamide (226 ml) was added N,N,N',N'-tetramethyl-O-(7-azabenzotriazol-1-yl)uronium hexafluorophosphate (18.87 g, 49.6 mmol). After stirring for 5 min, benzyl 3-amino-4-hydroxypyrrolidine-1-carboxylate (10.66 g, 45.1 mmol) and *N,N-*diisopropylethylamine (23.57 ml, 135 mmol) were added. The reaction mixture was stirred at room temperature for 2 h. The reaction was diluted with water, and the aqueous layer was extracted with DCM. The combined organic layers were dried over Na₂SO₄, filtered and concentrated. The residue was purified with flash chromatography (eluting with 0-60% ethyl acetate in hexanes) to give the desired product (11.5 g, 59%). LC-MS calculated for C₂₀H₂₂BrN₂O₄ (M+H)⁺: m/z = 433.1, 435.1; found 433.1, 435.1.

### Step 4: benzyl 3-(3-bromo-2-methylbenzamido)-4-oxopyrrolidine-1-carboxylate

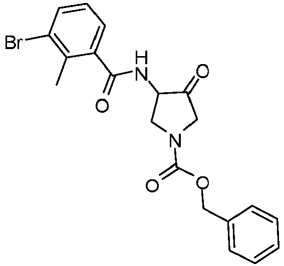

To a solution of benzyl 3-(3-bromo-2-methylbenzamido)-4-hydroxypyrrolidine-1-carboxylate (16.50 g, 38.1 mmol) in DCM (200 ml) was added Dess-Martin periodinane (19.38 g, 45.7 mmol). The resulting mixture was stirred at room temperature for 2 h. The reaction mixture was diluted with Et₂O and 1 M NaOH solution. After stirring for 1 h, the organic layer was separated and dried over Na₂SO₄, filtered and concentrated. The residue was purified with flash chromatography (eluting with 0-50% ethyl acetate in hexanes) to give the desired product (9.2 g, 56%). LC-MS calculated for C₂₀H₂₀BrN₂O₄ (M+H)⁺: m/z = 431.1, 433.1; found 431.1, 433.1.

### Step 5: benzyl 2-(3-bromo-2-methylphenyl)-4,6-dihydro-5H-pyrrolo[3,4-d]oxazole-5-carboxylate

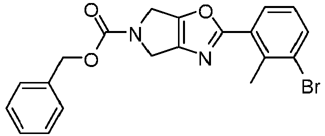

To a solution of benzyl 3-(3-bromo-2-methylbenzamido)-4-oxopyrrolidine-1-carboxylate (9.23g, 21.40 mmol) in 1,4-dioxane (100 ml) was added POCl₃ (1.995 ml, 21.40 mmol). The resulting mixture was stirred at 110 °C for 3 h. After cooling to room temperature, the reaction mixture was diluted with saturated NaHCO₃ solution and ethyl acetate. The aqueous layer was extracted with ethyl acetate. The combined organic layers were washed with brine, dried over Na₂SO₄, filtered and concentrated. The precipitate was collected via filtration and washed with ethyl acetate and hexanes to give the desired product as an off white solid (4.85 g, 55%). LC-MS calculated for C₂₀H₁₈BrN₂O₃ (M+H)⁺: m/z = 413.0, 415.0; found 413.0, 415.0.

### Step 6. 2-(3-Bromo-2-methylphenyl)-5, 6-dihydro-4H-pyrrolo[3, 4-d]oxazole

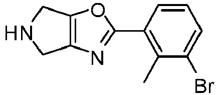

To solution of benzyl 2-(3-bromo-2-methylphenyl)-4,6-dihydro-5H-pyrrolo[3,4-d]oxazole-5-carboxylate (3.70 g, 8.95 mmol) in DCM (60ml) was added 1 M BBr₃ in DCM solution (17.91 ml, 17.91 mmol) at 0 °C. After stirring at same temperature for 1h, the reaction mixture was diluted DCM and saturated NaHCO₃ solution. The resultant precipitate was collected vial filtration and dried under vacuum to give the desired product as white solid (2.0 g, 80%). LC-MS calculated for C₁₂H₁₂BrN₂O (M+H)⁺: m/z = 279.0, 281.0; found 279.0, 281.0.

### Step 7. 1-(2-(3-Bromo-2-methylphenyl)-4,6-dihydro-5H-pyrrolo[3,4-d]oxazol-5-yl)-2-(dimethylamino)ethan-1-one

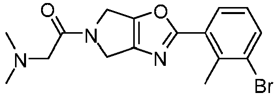

A solution of dimethylglycine (20.5 mg, 0.199 mmol) in *N,N-*dimethylformamide (1 ml) was added *N,N,N'*,*N'*-tetramethyl-O-(7-azabenzotriazol-1-yl)uronium hexafluorophosphate (104 mg, 0.274 mmol). After stirring for 5 min, 2-(3-bromo-2-methylphenyl)-5,6-dihydro-4H-pyrrolo[3,4-d]oxazole (55.5 mg, 0.199 mmol) and *N,N-*diisopropylethylamine (104 µl, 0.596 mmol) were added. The reaction mixture was stirred at room temperature for 2h. The reaction mixture was diluted with water, and the aqueous layer was extracted with DCM. The combined organic layers were dried over Na₂SO₄, filtered and concentrated. The residue was purified with silica gel column (eluting with 0-30% MeOH in DCM) to give the desired product (35 mg, 49%). LC-MS calculated for C₁₆H₁₉BrN₃O₂ (M+H)⁺: m/z = 364.1, 366.1; found 364.1, 366.1.

### Step 8. (R)-1-((8-((3-bromo-2-methylphenyl)amino)-1,7-naphthyridin-3-yl)methyl)pyrrolidin-3-ol

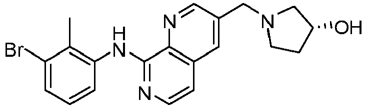

To a mixture of 8-((3-bromo-2-methylphenyl)amino)-1,7-naphthyridine-3-carbaldehyde (Example 9, Step 1: 340 mg, 0.994 mmol), (*R*)-pyrrolidin-3-ol (104 mg, 1.192 mmol) in DCM (1.0 ml) was added sodium triacetoxyborohydride (316 mg, 1.490 mmol). After stirring for 2 h at room temperature, the mixture was purified with flash chromatography (0-100% ethyl acetate in hexanes, then 0-35% methanol in DCM). LC-MS calculated for C₂₀H₂₂BrN₄O (M+H)⁺: m/z = 413.1, 415.1; found 413.1, 415.1.

### Step 9: (R)-1-((8-((2-methyl-3-(4,4,5,5-tetramethyl-1,3,2-dioxaborolan-2-yl)phenyl)amino)-1,7-naphthyridin-3-yl)methyl)pyrrolidin-3-ol

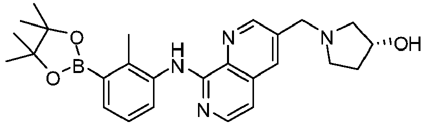

A mixture of (*R*)-1-((8-((3-bromo-2-methylphenyl)amino)-1,7-naphthyridin-3-yl)methyl)pyrrolidin-3-ol (281 mg, 0.680 mmol), bis(pinacolato)diboron (207 mg, 0.816 mmol), dichloro[1,l'-bis(diphenylphosphino)ferrocene]palladium (II) dichloromethane adduct (55.5 mg, 0.068 mmol), 1,4-dioxane (3.4 mL) and potassium acetate (167 mg, 1.700 mmol) was stirred at 90 °C under N₂ atmosphere for 3 h. The crude was diluted with DCM, and then filtered through a pad of Celite^{®}. The filtrate was concentrated and purified with flash chromatography (eluting with ethyl acetate in hexane 0-100%, then methanol/DCM 0-25%) (210 mg, 67%). LC-MS calculated for C₂₆H₃₄BN₄O₃ (M+H)⁺: m/z = 461.3; found 461.2.

### Step 10: (R)-2-(dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one

A microwave vial charged with 1-(2-(3-bromo-2-methylphenyl)-4,6-dihydro-5H-pyrrolo[3,4-d]oxazol-5-yl)-2-(dimethylamino)ethan-1-one (9.49 mg, 0.026 mmol), (*R*)-1-((8-((2-methyl-3-(4,4,5,5-tetramethyl-1,3,2-dioxaborolan-2-yl)phenyl)amino)-1,7-naphthyridin-3-yl)methyl)pyrrolidin-3-ol (12 mg, 0.026 mmol), dicyclohexyl(2',4',6'-triisopropylbiphenyl-2-yl)phosphine-(2'-aminobiphenyl-2-yl)(chloro)palladium (1:1) (2.051 mg, 2.61 µmol) and tripotassium phosphate hydrate (13.21 mg, 0.057 mmol) was evacuated under high vacuum and refilled with nitrogen (repeated three times). 1,4-Dioxane (0.6mL) and water (0.2mL) was added and resulting mixture was stirred at 80 °C for 1 h. The reaction mixture was diluted with methanol and 1 N HCl solution and purified with prep-LC-MS (pH = 2, acetonitrile/water+TFA) to give the desired product as whie solid. LC-MS calculated for C₃₆H₄₀N₇O₃ (M+H)⁺: m/z = 618.3; found 618.3.

### Example 38

### (S)-2-(dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one

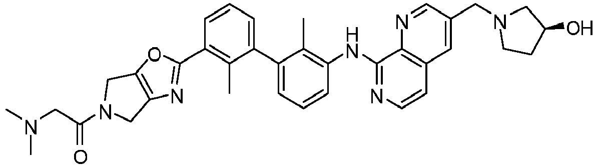

This compound was prepared using similar procedures as described for *Example 37* with (*S*)-pyrrolidin-3-ol (*Combi-Blocks, cat#SS-7948*) replacing (*R*)-pyrrolidin-3-ol in *Step 8.* LC-MS calculated for C₃₆H₄₀N₇O₃ (M+H)⁺: m/z = 618.3; found 618.3.

### Example 39

### (R)-1-(2-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)azetidine-3-carboxylic acid

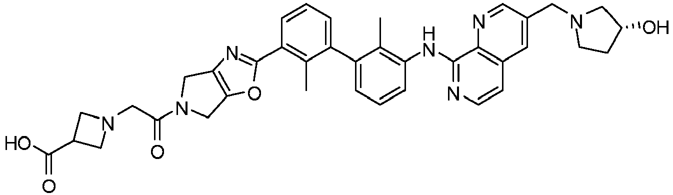

### Step 1: 1-(2-(3-bromo-2-methylphenyl)-4,6-dihydro-5H-pyrrolo[3,4-d]oxazol-5-yl)-2-chloroethan-1-one

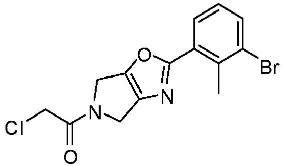

A solution of 2-(3-bromo-2-methylphenyl)-5,6-dihydro-4H-pyrrolo[3,4-d]oxazole (Example 37, Step 6: 1.04 g, 3.73 mmol) in CH₂Cl₂(18ml) was added 2-chloroacetyl chloride (0.421 g, 3.73 mmol) and *N,N-*diisopropylethylamine (1.947 ml, 11.18 mmol) at 0 °C. The reaction mixture was stirred at room temperature for 2h. The reaction was diluted with water, and the aqueous layer was extracted with DCM. The combined organic layers were dried over Na₂SO₄, filtered and concentrated. The residue was purified with flash chromatography (eluting with 0-60% ethyl acetate in hexanes) to give the desired product as white solid (0.65 g, 49%). LC-MS calculated for C₁₄H₁₃BrClN₂O₂ (M+H)⁺: m/z = 355.0, 357.0; found 355.0, 357.0.

### Step 2: 1-(2-(2-(3-bromo-2-methylphenyl)-4,6-dihydro-5H-pyrrolo[3,4-d]oxazol-5-yl)-2-oxoethyl)azetidine-3-carboxylic acid

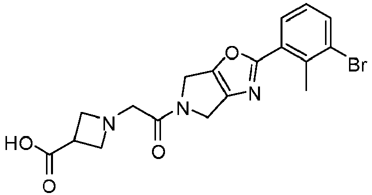

The mixture of 1-(2-(3-bromo-2-methylphenyl)-4,6-dihydro-5H-pyrrolo[3,4-d]oxazol-5-yl)-2-chloroethan-1-one (15 mg, 0.042 mmol), azetidine-3-carboxylic acid (Aldrich, cat#391131: 4.26 mg, 0.042 mmol), TEA (0.018 ml, 0.127 mmol) and *N,N-*dimethylformamide (1.0 ml) was heated at 60 °C for 2 h. The reaction mixture was diluted with methanol and 1 N HCl, then purified with prep- LC-MS (pH 2) to give the desired product C (12 mg, 67%). LC-MS calculated for C₁₈H₁₉BrN₃O₄ (M+H)⁺: m/z = 420.1; found 420.1.

### Step 3: (R)-1-(2-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)azetidine-3-carboxylic acid

This compound was prepared using similar procedures as described for Example 37, Step 10 with 1-(2-(2-(3-bromo-2-methylphenyl)-4,6-dihydro-5H-pyrrolo[3,4-d]oxazol-5-yl)-2-oxoethyl)azetidine-3-carboxylic acid replacing 1-(2-(3-bromo-2-methylphenyl)-4,6-dihydro-5H-pyrrolo[3,4-d]oxazol-5-yl)-2-(dimethylamino)ethan-1-one. LC-MS calculated for C₃₈H₄₀N₇O₅ (M+H)⁺: m/z = 674.3; found 674.3.

### Example 40

### (S)-1-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidine-3-carboxylic acid

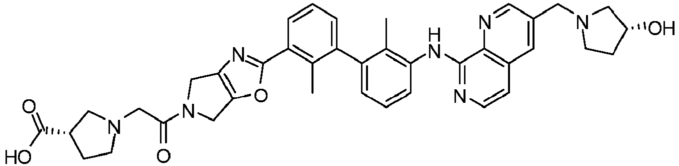

### Step 1: (S)-1-(2-(2-(3-bromo-2-methylphenyl)-4,6-dihydro-5H-pyrrolo[3,4-d]oxazol-5-yl)-2-oxoethyl)pyrrolidine-3-carboxylic acid

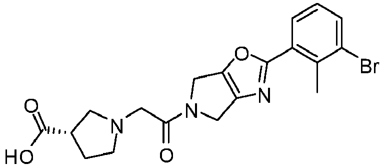

This compound was prepared using similar procedures as described for Example 39, Step 2 with (*S*)-pyrrolidine-3-carboxylic acid (Combi-Blocks, #ST-1381) replacing azetidine-3-carboxylic acid. LC-MS calculated for C₁₉H₂₁BrN₃O₄ (M+H)⁺: m/z = 434.1, 436.1; found 434.1, 436.1.

### Step 2: (S)-]-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidine-3-carboxylic acid

This compound was prepared using similar procedures as described for Example 37, Step 10 with (*S*)-1-(2-(2-(3-bromo-2-methylphenyl)-4,6-dihydro-5H-pyrrolo[3,4-d]oxazol-5-yl)-2-oxoethyl)pyrrolidine-3-carboxylic acid replacing 1-(2-(3-bromo-2-methylphenyl)-4,6-dihydro-5H-pyrrolo[3,4-d]oxazol-5-yl)-2-(dimethylamino)ethan-1-one. LC-MS calculated for C₃₉H₄₂N₇O₅ (M+H)⁺: m/z = 688.3; found 688.3.

### Example 41

### (R)-1-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo [3,4-d] oxazol-5-yl)-2-oxoethyl)pyrrolidine-3-carboxylic acid

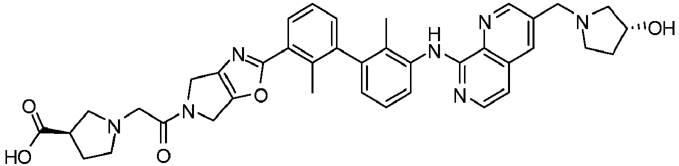

### Step 1: (R)-1-(2-(2-(3-bromo-2-methylphenyl)-4,6-dihydro-5H-pyrrolo[3,4-d]oxazol-5-yl)-2-oxoethyl)pyrrolidine-3-carboxylic acid

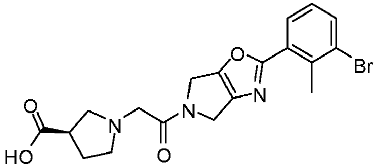

This compound was prepared using similar procedures as described for Example 39, Step 2 with (*R*)-pyrrolidine-3-carboxylic acid (Combi-Blocks, cat#ST-7698) replacing azetidine-3-carboxylic acid. LC-MS calculated for C₁₉H₂₁BrN₃O₄ (M+H)⁺: m/z = 434.1, 436.1; found 434.1, 436.1.

### Step 2: (R)-1-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidine-3-carboxylic acid

This compound was prepared using similar procedures as described for Example 37, Step 10 with (*R*)-1-(2-(2-(3-bromo-2-methylphenyl)-4,6-dihydro-5H-pyrrolo[3,4-d]oxazol-5-yl)-2-oxoethyl)pyrrolidine-3-carboxylic acid replacing 1-(2-(3-bromo-2-methylphenyl)-4,6-dihydro-5H-pyrrolo[3,4-d]oxazol-5-yl)-2-(dimethylamino)ethan-1-one. LC-MS calculated for C₃₉H₄₂N₇O₅ (M+H)⁺: m/z = 688.3; found 688.3.

### Example 42

### (S)-1-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)piperidine-2-carboxylic acid

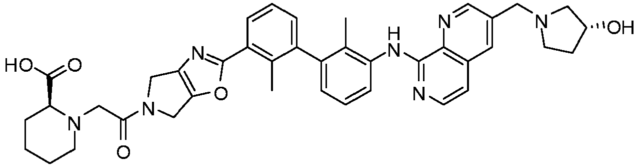

### Step 1: (S)-1-(2-(2-(3-bromo-2-methylphenyl)-4,6-dihydro-5H-pyrrolo[3,4-d]oxazol-5-yl)-2-oxoethyl)piperidine-2-carboxylic acid

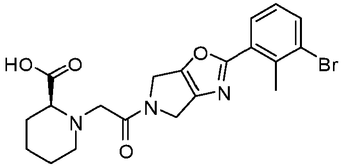

This compound was prepared using similar procedures as described for Example 39, Step 2 with (*S*)-piperidine-2-carboxylic acid (Alfa Aesar, cat#L15373) replacing azetidine-3-carboxylic acid. LC-MS calculated for C₂₀H₂₃BrN₃O₄ (M+H)⁺: m/z = 448.1, 450.1; found 448.1, 450.1.

### Step 2: (S)-1-(2-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro--5H-pyrrolo[3,4-d]oxazol-5-yl)-2-oxoethyl)piperidine-2-carboxylic acid

This compound was prepared using similar procedures as described for Example 37, Step 10 with (*S*)-1-(2-(2-(3-bromo-2-methylphenyl)-4,6-dihydro-5H-pyrrolo[3,4-d]oxazol-5-yl)-2-oxoethyl)piperidine-2-carboxylic acid replacing 1-(2-(3-bromo-2-methylphenyl)-4,6-dihydro-5H-pyrrolo[3,4-d]oxazol-5-yl)-2-(dimethylamino)ethan-1-one. LC-MS calculated for C₄₀H₄₄N₇O₅ (M+H)⁺: m/z = 702.3; found 702.3.

### Example 43

### (S)-1-(5-chloro-2-((5-cyanopyridin-3-yl)methoxy)-4-((3'-((3-(((R)-3-hydroxypyrrolidin-1-yl)methyl)-1,7-naphthyridin-8-yl)amino)-2,2'-dimethyl-[1,1'-biphenyl]-3-yl)methoxy)benzyl)piperidine-2-carboxylic acid

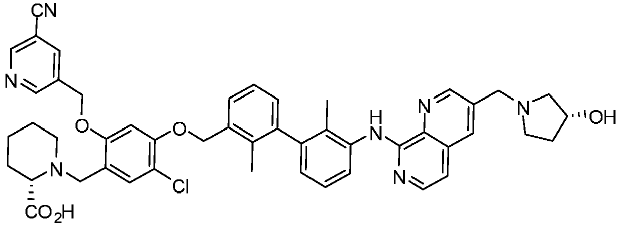

### Step 1: 4-((3-bromo-2-methylbenzyl)oxy)-5-chloro-2-hydroxybenzaldehyde

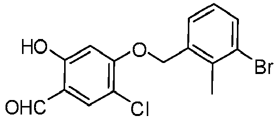

To a mixture of (3-bromo-2-methylphenyl)methanol (Ark Pharm, cat#AK162869: 2.330 g, 11.59 mmol), 5-chloro-2,4-dihydroxybenzaldehyde (Ark Pharm, cat#AK199510: 2.0 g, 11.59 mmol) and triphenylphosphine (3.65 g, 13.91 mmol) in THF (10 ml) at 0 °C was added DIAD (2.93 ml, 15.07 mmol). The mixture was stirred at room temperature overnight. The mixture was concentrated and diluted with EtOAc. The solid was collected by filtration to give 4-((3-bromo-2-methylbenzyl)oxy)-5-chloro-2-hydroxybenzaldehyde (2.0 g, 5.62 mmol, 48.5 % yield). LC-MS calculated for C₁₅H₁₃BrClO₃ (M+H)⁺: m/z = 355.0; found 355.2.

### Step 2: -5-((5-((3-bromo-2-methylbenzyl)oxy)-4-chloro-2-formylphenoxy)methyl)nicotinonitrile

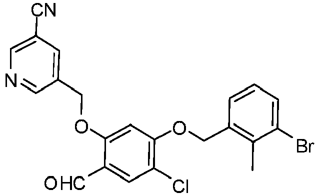

A mixture of 4-((3-bromo-2-methylbenzyl)oxy)-5-chloro-2-hydroxybenzaldehyde (2.0 g, 5.62 mmol), 5-(chloromethyl)nicotinonitrile (0.927 g, 6.07 mmol) and cesium carbonate (2.75 g, 8.44 mmol) in DMF (12 ml) was stirred at 70 °C for 3 hours. The mixture was poured into water. The solid was collected by filtration and air dried to give 5-((5-((3-bromo-2-methylbenzyl)oxy)-4-chloro-2-formylphenoxy)methyl)nicotinonitrile (2.2 g, 4.66 mmol, 83 % yield). LC-MS calculated for C₂₂H₁₇BrClN₂O₃ (M+H)+: m/z = 471.0; found 471.2.

### Step 3: (R)-5-((4-chloro-2-formyl-5-((3'-((3-((3-hydroxypyrrolidin-1-yl)methyl)-1,7-naphthyridin-8-yl)amino)-2,2'-dimethyl-[1,1'-biphenyl]-3-yl) methoxy)phenoxy)methyl)nicotinonitrile

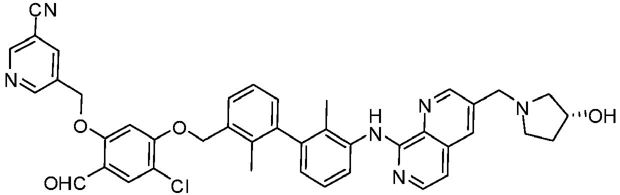

A mixture of 5-((5-((3-bromo-2-methylbenzyl)oxy)-4-chloro-2-formylphenoxy)methyl)nicotinonitrile (78 mg, 0.165 mmol), (*R*)-1-((8-((2-methyl-3-(4,4,5,5-tetramethyl-1,3,2-dioxaborolan-2-yl)phenyl)amino)-1,7-naphthyridin-3-yl)methyl)pyrrolidin-3-ol (Example 37, Step 9: 91 mg, 0.198 mmol), potassium carbonate (45.7 mg, 0.331 mmol) and (1,1'-bis(diphenylphosphino)ferrocene)-dichloropalladium(II) (12.1mg, 0.017 mmol) in 1,4-dioxane (3 mL) and water (0.600 mL) was purged with nitrogen, and heated at 95 °C for 2 hours. The mixture was purified on prep-HPLC (pH = 2, acetonitrile/water+TFA) to give (*R*)-5-((4-chloro-2-formyl-5-((3'-((3-((3-hydroxypyrrolidin-1-yl)methyl)-1,7-naphthyridin-8-yl)amino)-2,2'-dimethyl-[1,1'-biphenyl]-3-yl)methoxy)phenoxy)methyl)nicotinonitrile (60 mg, 0.083 mmol, 50.0 % yield). LC-MS calculated for C₄₂H₃₈ClN₆O₄ (M+H)⁺: m/z = 725.3; found 725.2.

### Step 4: (S)-1-(5-chloro-2-((5-cyanopyridin-3-yl)methoxy)-4-((3'-((3-(((R)-3-hydroxypyrrolidin-1-yl)methyl)-1,7-naphthyridin-8-yl)amino)-2,2'-dimethyl-[1,1'-biphenyl]-3-yl)methoxy)benzyl)piperidine-2-carboxylic acid

Sodium triacetoxyborohydride (2.192 mg, 10.34 µmol) was added to a mixture *of (R)-*5-((4-chloro-2-formyl-5-((3'-((3-((3-hydroxypyrrolidin-1-yl)methyl)-1,7-naphthyridin-8-yl)amino)-2,2'-dimethyl-[1,1'-biphenyl]-3-yl)methoxy)phenoxy)methyl)nicotinonitrile (5mg, 6.89 µmol), (*S*)-piperidine-2-carboxylic acid (1.4 mg, 10.34 µmol) and triethylamine (1.922 µL, 0.014 mmol) in DCM (1.0 mL) after stirring for 2 hours at room temperature. After stirring at room temperature overnight, the mixture was purified using prep-HPLC (pH = 2, acetonitrile/water+TFA) to give the desired product as its TFA salt. LC-MS calculated for C₄₈H₄₉ClN₇O₅ (M+H)⁺: m/z = 838.3; found 838.2.

### Example 44

### (R)-1-(5-chloro-2-((5-cyanopyridin-3-yl)methoxy)-4-((3'-((3-(((R)-3-hydroxypyrrolidin-1-yl)methyl)-1,7-naphthyridin-8-yl)amino)-2,2'-dimethyl-[1,1'-biphenyl]-3-yl)methoxy)benzyl)pyrrolidine-3-carboxylic acid

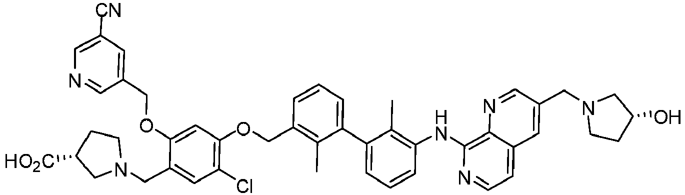

Sodium triacetoxyborohydride (2.192 mg, 10.34 µmol) was added to a mixture of (R)-5-((4-chloro-2-formyl-5-((3'-((3-((3-hydroxypyrrolidin-1-yl)methyl)-1,7-naphthyridin-8-yl)amino)-2,2'-dimethyl-[1,1'-biphenyl]-3-yl)methoxy)phenoxy)methyl)nicotinonitrile (Example 43, Step 3: 5mg, 6.9 µmol), (*R*)-pyrrolidine-3-carboxylic acid (Combi-Blocks, cat#ST-7698: 1.2 mg, 10.34 µmol) and triethylamine (1.9 µl, 0.014 mmol) in DCM (1.0 mL) after stirring for 2 h at room temperature. After stirring at room temperature for 2 h, the mixture was purified using prep-HPLC (pH = 2, acetonitrile/water+TFA) to give the desired product as its TFA salt. LC-MS calculated for C₄₇H₄₇ClN₇O₅ (M+H)⁺: m/z = 824.3; found 824.2.

### Example 45

### (R)-1-((8-(2'-Chloro-2-methyl-3'-(1-methyl-4,5,6,7-tetrahydro-1H-imidazo[4,5-c]pyridine-2-carboxamido)biphenyl-3-ylamino)-1,7-naphthyridin-3-yl)methyl)-3-methylpyrrolidine-3-carboxylic acid

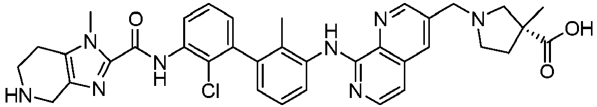

### Step 1: 8-(2-methyl-3-(4,4,5,5-tetramethyl-1,3,2-dioxaborolan-2-yl)phenylamino)-1,7-naphthyridine-3-carbaldehyde

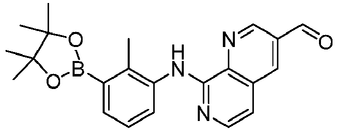

A mixture of 8-((3-bromo-2-methylphenyl)amino)-1,7-naphthyridine-3-carbaldehyde *(Example 9, Step 1:* 0.684 g, 2.0 mmol), 4,4,4',4',5,5,5',5'-octamethyl-2,2'-bi(1,3,2-dioxaborolane) (0.660 g, 2.60 mmol), potassium acetate (0.393 g, 4.00 mmol), and PdCl₂(dppf) (0.146 g, 0.200 mmol) in dioxane (10.0 mL) was vacuumed and refilled with nitrogen 3 times and then the reaction mixture was stirred at 110 °C for 7 h. The mixture was diluted with EtOAc, filtered through Celite^{®} and concentrated under reduced pressure. The residue was purified by column chromatography eluting with CH₂Cl₂ to give the desired product. LC-MS calculated for C₂₂H₂₅BN₃O₃ (M+H)⁺: m/z = 390.2; found 390.3.

### Step 2: tert-butyl 2-(2-chloro-3'-(3-formyl-1,7-naphthyridin-8-ylamino)-2'-methylbiphenyl-3-ylcarbamoyl)-1-methyl-6,7-dihydro-1H-imidazo[4,5-c]pyridine-5(4H)-carboxylate

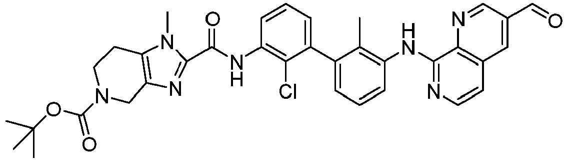

A mixture of tert-butyl 2-((3-bromo-2-chlorophenyl)carbamoyl)-1-methyl-1,4,6,7-tetrahydro-5*H*-imidazo[4,5-*c*]pyridine-5-carboxylate (*Example 31, Step 3:* 0.12 g, 0.255 mmol), 8-(2-methyl-3-(4,4,5,5-tetramethyl-1,3,2-dioxaborolan-2-yl)phenylamino)-1,7-naphthyridine-3-carbaldehyde (0.10 g, 0.26 mmol), dichloro[1,1'-bis(diphenylphosphino)ferrocene]palladium (II) dichloromethane adduct (0.019 g, 0.023 mmol) and sodium carbonate (0.049 g, 0.464 mmol) in dioxane (2.4 mL)/water (0.6 mL) was evacuated and backfilled with N₂ 3 times. The reaction mixture was stirred at 110 °C for 24 h. The mixture was diluted with ethyl acetate and washed with water, dried over Na₂SO₄, filtered and concentrated in vacuo. The product was purified by column chromatography eluting with CH₂Cl₂/EtOAc (7:3). LC-MS calculated for C₃₅H₃₅ClN₇O₄ (M+H)⁺: m/z = 652.2; found 652.2.

### Step 3: (R)-1-((8-(3'-(5-(tert-butoxycarbonyl)-1-methyl-4,5,6,7-tetrahydro-1H-imidazo[4,-5-c]pyridine-2-carboxamido)-2'-chloro-2-methylbiphenyl-3-ylamino)-1,7-naphthyridin-3-yl)methyl)-3-methylpyrrolidine-3-carboxylic acid

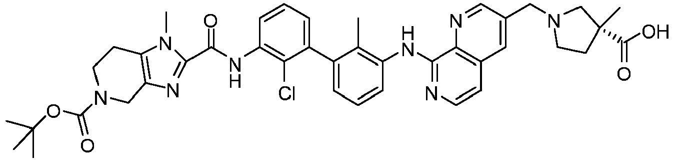

(*R*)-3-methylpyrrolidine-3-carboxylic acid (J&W PharmLab, cat#75R0495: 0.071 g, 0.552 mmol) was added to a suspension of *tert*-butyl 2-((2-chloro-3'-((3-formyl-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate (0.12 g, 0.184 mmol) in CH₂Cl₂ (1.0 mL) followed by triethylamine (0.205 mL, 1.472 mmol). The mixture was stirred at rt for 1 h. At this time sodium triacetoxyborohydride (0.117 g, 0.552 mmol) was added and then stirred at rt for 2 h. The reaction was quenched with water, extracted with CH₂Cl₂/iPrOH, and the organic phase was dried over MgSO₄, filtered, and concentrated under reduced pressure. The crude product was used directly in the next step without further purification. LC-MS calculated for C₄₁H₄₆ClN₈O₅ (M+H)⁺: m/z = 765.3; found 765.2.

### Step 4: (R)-1-((8-(2'-chloro-2-methyl-3'-(1-methyl-4,5,6,7-tetrahydro-1H-imidazo[4,5-c]pyridine-2-carboxamido)biphenyl-3-ylamino)-1,7-naphthyridin-3-yl)methyl)-3-methylpyrrolidine-3-carboxylic acid

TFA (2.0 mL) was added to a mixture of (*R*)-1-((8-((3'-(5-(tert-butoxycarbonyl)-1-methyl-4,5,6,7-tetrahydro-1H-imidazo[4,5-*c*]pyridine-2-carboxamido)-2'-chloro-2-methyl-[1,1'-biphenyl]-3-yl)amino**)-1,**7-naphthyridin-3-yl)methyl)-3-methylpyrrolidine-3-carboxylic acid (0.15 g) in CH₂Cl₂ (2.0 mL) and then stirred at rt for 30 min. The solvent was concentrated and the mixture was diluted with acetonitrile/water and purified by prep HPLC (pH = 2, acetonitrile/water+TFA) to provide the desired compound as its TFA salt. LC-MS calculated for C₃₆H₃₈ClN₈O₃ (M+H)⁺: m/z = 665.3; found 665.2.

### Example 46

### (R)-1-((8-(3'-(1,5-dimethyl-4,5,6,7-tetrahydro-1H-imidazo[4,5-c]pyridine-2-carboxamido)-2,2'-dimethylbiphenyl-3-ylamino)-1,7-naphthyridin-3-yl)methyl)pyrrolidine-3-carboxylic acid

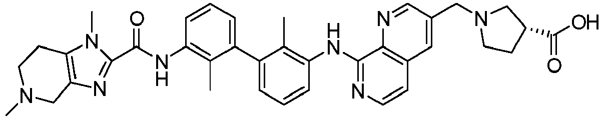

### Step 1: tert-butyl 2-(3-bromo-2-methylphenylcarbamoyl)-1-methyl-6,7-dihydro-1H-imidazo[4,5-c]pyridine-5(4H)-carboxylate

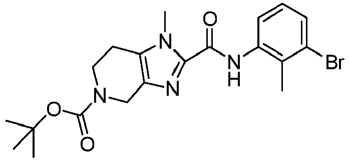

Potassium *tert*-butoxide (1.0 M THF solution, 17.61 mL, 17.61 mmol) was added to a solution of 5-*tert*-butyl 2-methyl 1-methyl-6,7-dihydro-1*H*-imidazo[4,5-*c*]pyridine-2,5(4*H*)-dicarboxylate *(Example 14, Step* 2: 2.6 g, 8.80 mmol) and 3-bromo-2-methylaniline (Aldrich, cat#530018: 1.802 g, 9.68 mmol) in THF (45 mL) at 0 °C. After being stirred at rt for 2 h, the reaction mixture was quenched with water, and extracted with ethyl acetate. The combined organic layers were washed with brine, dried over Na₂SO₄, filtered and concentrated under reduced pressure. The crude was stirred with 3:1 hexanes/EtOAc (40 mL) for 30 min and then filtered and dried to provide the desired product. LC-MS calculated for C₂₀H₂₆BrN₄O₃ (M+H)⁺: m/z = 449.1; found 449.1.

### Step 2: tert-butyl2-(3'-(3-(hydroxymethyl)-1,7-naphthyridin-8-ylamino)-2,2'-dimethylbiphenyl-3-ylcarbamoyl)-1-methyl-6,7-dihydro-1H-imidazo[4,5-c]pyridine-5(4H)-carboxylate

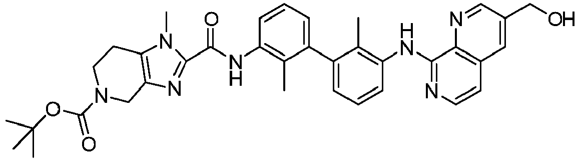

A mixture of tert-butyl 2-((3-bromo-2-methylphenyl)carbamoyl)-1-methyl-1,4,6,7-tetrahydro-5*H*-imidazo[4,5-*c*]pyridine-5-carboxylate (0.12 g, 0.267 mmol), (8-((2-methyl-3-(4,4,5,5-tetramethyl-1,3,2-dioxaborolan-2-yl)phenyl)amino)-1,7-naphthyridin-3-yl)methanol *(Example 9, Step 3:* 0.095 g, 0.243 mmol), dichloro[1,1'-bis(diphenylphosphino)ferrocene]palladium (II) dichloromethane adduct (0.020 g, 0.024 mmol) and sodium carbonate (0.051 g, 0.486 mmol) in dioxane (2.4 mL)/water (0.6 mL) was evacuated and backfilled with N₂. The evacuation/backfill sequence was repeated two additional times, and the reaction was stirred at 110 °C for 24 h. The mixture was diluted with ethyl acetate and washed with water, dried over Na₂SO₄, and concentrated under reduced pressure. The crude product was purified by column chromatography eluting with CH₂Cl₂/EtOAc (1:1). LC-MS calculated for C₃₆H₄₀N₇O₄ (M+H)⁺: m/z = 634.3; found 634.5.

### Step 3: N-(3'-(3-(hydroxymethyl)-1,7-naphthyridin-8-ylamino)-2,2'-dimethylbiphenyl-3-yl)-1-methyl-4, 5,6,7-tetrahydro-1H-imidazo[4,5-c]pyridine-2-carboxamide

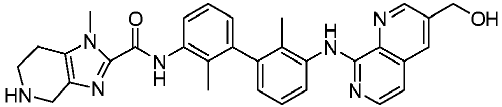

4 N HCl in dioxane (1.0 mL) was added to a mixture of *tert*-butyl 2-((3'-((3-(hydroxymethyl)-1,7-naphthyridin-8-yl)amino)-2,2'-dimethyl-[1,1'-biphenyl]-3-yl)carbamoyl)-1-methyl-1,4,6,7-tetrahydro-5*H*-imidazo[4,5-*c*]pyridine-5-carboxylate (0.10 g, 0.158 mmol) in CH₂Cl₂ (1.0 mL), and the reaction was stirred at rt for 2 h. The solvent was removed and the crude residue was used directly in the next step without further purification. LC-MS calculated for C₃₁H₃₂N₇O₂ (M+H)⁺: m/z = 534.3; found 534.3.

### Step 4: N-(3'-(3-(hydroxymethyl)-1,7-naphthyridin-8-ylamino)-2,2'-dimethylbiphenyl-3-yl)-1, 5-dimethyl-4,5,6,7-tetrahydro-1H-imidazo[4,-5-c]pyridine-2-carboxamide

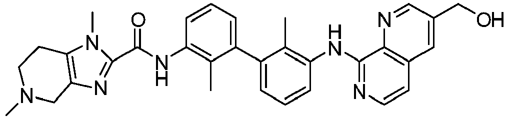

Formaldehyde (36% H₂O solution, 6.3 µL, 0.075 mmol) was added to a mixture of *N-*(3'-((3-(hydroxymethyl)-1,7-naphthyridin-8-yl)amino)-2,2'-dimethyl-[1,1'-biphenyl]-3-yl)-1-methyl-4,5,6,7-tetrahydro-1*H*-imidazo[4,5*-*c]pyridine-2-carboxamide (20.0 mg, 0.037 mmol) in CH₂Cl₂ (1.0 mL) followed by the addition of triethylamine (0.026 mL, 0.187 mmol). The mixture was stirred at rt for 10 min. At this time sodium triacetoxyborohydride (23.8 mg, 0.11 mmol) was added and then the mixture was stirred at rt for 30 min. The reaction mixture was quenched with water and then extracted with CH₂Cl₂. The combined organic phase was concentrated under reduced pressure and the crude product was used directly in the next step without further purification. LC-MS calculated for C₃₂H₃₄N₇O₂ (M+H)⁺: m/z = 548.3; found 548.4.

### Step 5: N-(3'-(3-formyl-1,7-naphthyridin-8-ylamino)-2,2'-dimethylbiphenyl-3-yl)-1,5-dimethyl-4, 5,6,7-tetrahydro-1H-imidazo[4,5-c]pyridine-2-carboxamide

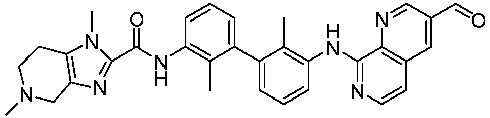

Manganese dioxide (0.143 g, 1.643 mmol) was added to a solution of *N*-(3'-((3-(hydroxymethyl)-1,7-naphthyridin-8-yl)amino)-2,2'-dimethyl-[1,1'-biphenyl]-3-yl)-1,5-dimethyl-4,5,6,7-tetrahydro-1*H*-imidazo[4,5-*c*]pyridine-2-carboxamide (0.060 g, 0.110 mmol) in CH₂Cl₂ (2.0 mL) and then the mixture was stirred at 40 °C overnight. The mixture was diluted with CH₂Cl₂, filtered through Celite^{®} and then concentrated under reduced pressure to provide the desired product which was used directly in the next step. LC-MS calculated for C₃₂H₃₂N₇O₂ (M+H)⁺: m/z = 546.3; found 546.2.

### Step 6: (R)-1-((8-(3'-(1,5-dimethyl-4,5,6,7-tetrahydro-1H-imidazo[4,5-c]pyridine-2-carboxamido)-2,2'-dimethylbiphenyl-3-ylamino)-1,7-naphthyridin-3-yl)methyl)pyrrolidine-3-carboxylic acid

To a mixture of *N*-(3'-((3-formyl-1,7-naphthyridin-8-yl)amino)-2,2'-dimethyl-[1,1'-biphenyl]-3-yl)-1,5-dimethyl-4,5,6,7-tetrahydro-1*H*-imidazo[4,5-c]pyridine-2-carboxamide (0.010 g, 0.018 mmol) and (*R*)-pyrrolidine-3-carboxylic acid (Combi-Blocks, cat#ST-7698: 6.3 mg, 0.055 mmol) in CH₂Cl₂ (1.0 mL) was added triethylamine (6.3 µL, 0.11 mmol). After stirring for 10 min sodium triacetoxyborohydride (0.012 g, 0.055 mmol) was added, and the reaction was further stirred at rt for 2 h. The reaction was concentrated and the mixture was diluted with acetonitrile/water and purified by prep HPLC (pH = 2, acetonitrile/water+TFA) to provide the desired compound as its TFA salt. LC-MS calculated for C₃₇H₄₁N₈O₃ (M+H)⁺: m/z = 645.3; found 645.4.

### Example 47

### trans-4-( (2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylic acid

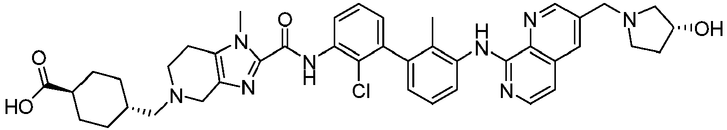

### Step 1: tert-butyl 2-(2-chloro-3-(4,4,5,5-tetramethyl-1,3,2-dioxaborolan-2-yl)phenylcarbamoyl)-1-methyl-6,7-dihydro-1H-imidazo[4,5-c]pyridine-5(4H)-carboxylate

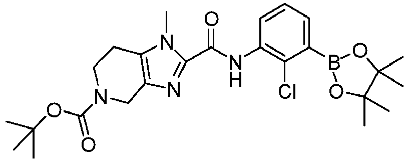

Potassium *tert*-butoxide (1.0 M in THF, 2.20 mL, 2.20 mmol) was added to a solution of 5-tert-butyl 2-methyl 1-methyl-6,7-dihydro-1H-imidazo[4,5-c]pyridine-2,5(4H)-dicarboxylate *(Example 14, Step* 2: 0.295 g, 1.0 mmol) and 2-chloro-3-(4,4,5,5-tetramethyl-1,3,2-dioxaborolan-2-yl)aniline *(Example 5, Step 1:* 0.304 g, 1.200 mmol) in THF (4.0 mL). After being stirred at rt for 2 h, the reaction mixture was quenched with water, and extracted with ethyl acetate. The combined organic layers were washed with brine, dried over Na₂SO₄, filtered and concentrated under reduced pressure. The residue was purified by flash chromatography on a silica gel column eluting with ethyl acetate in hexanes (0-50%) to afford the desired product. LC-MS calculated for C₂₅H₃₅BClN₄O₅ (M+H)⁺: m/z = 517.2; found 517.3.

### Step 2: tert-butyl 2-(2-chloro-3'-(3-formyl-1,7-naphthyridin-8-ylamino)-2'-methylbiphenyl-3-ylcarbamoyl)-1-methyl-6,7-dihydro-1H-imidazo[4,-5-c]pyridine--5(4H)-carboxylate

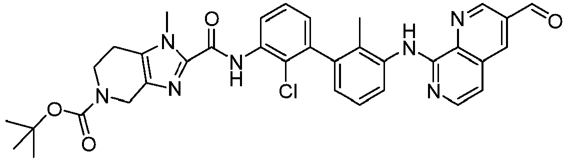

A mixture of tert-butyl 2-((2-chloro-3-(4,4,5,5-tetramethyl-1,3,2-dioxaborolan-2-yl)phenyl)carbamoyl)-1-methyl-1,4,6,7-tetrahydro-5*H*-imidazo[4,5-*c*]pyridine-5-carboxylate (0.35 g, 0.677 mmol), 8-((3-bromo-2-methylphenyl)amino)-1,7-naphthyridine-3-carbaldehyde *(Example 9, Step 1:* 0.255 g, 0.745 mmol), dichloro[1,1'-bis(dicyclohexylphosphino)ferrocene]-palladium(II) (0.051 g, 0.068 mmol) and cesium fluoride (0.514 g, 3.39 mmol) in *t*-BuOH (3.00 mL)/water (1.2 mL) was evacuated and backfilled with N₂ 3 times. The reaction was stirred at 105 °C for 2 h. The mixture was cooled to rt, diluted with ethyl acetate, and washed with water. The organic layers were washed with brine, dried over MgSO₄, filtered, and concentrated under reduced pressure. The product was purified by column chromatography eluting with CH₂Cl₂/EOAc (7:3). LC-MS calculated for C₃₅H₃₅ClN₇O₄ (M+H)⁺: m/z = 652.2; found 652.4.

### Step 3: (R)-tert-butyl 2-(2-chloro-3'-(3-((3-hydroxypyrrolidin-1-yl)methyl)-1,7-naphthyridin-8-ylamino)-21-3-ylcarbamoyl)-1-methyl-6,7-dihydro-1H-imidazo[4,5-c]pyridine--5(4H)-carboxylate

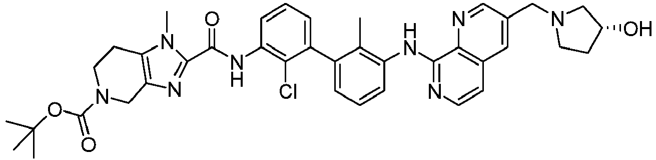

(*R*)-pyrrolidin-3-ol (Combi-Blocks, cat#AM-2005: 0.072 g, 0.828 mmol) was added to a solution of *tert-butyl* 2-((2-chloro-3'-((3-formyl-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate (0.180 g, 0.276 mmol) in CH₂Cl₂ (1.0 mL). Triethylamine (0.308 mL, 2.208 mmol) was then added and the mixture was stirred at rt for 1 h. At this time sodium triacetoxyborohydride (0.175 g, 0.828 mmol) was added and then stirred at rt for 2 h. The reaction was quenched with water, extracted with CH₂Cl₂, and the organic phase was dried over MgSO₄, filtered, and concentrated under reduced pressure. The product was purified by column chromatography eluting with CH₂Cl₂/MeOH (9:1). LC-MS calculated for C₃₉H₄₄ClN₈O₄ (M+H)⁺: m/z = 723.3; found 723.5.

### Step 4: (R)-N-(2-chloro-3'-(3-((3-hydroxypyrrolidin-1-yl)methyl)-1,7-naphthyridin-8-ylamino)-2'-methylbiphenyl-3-yl)-1-methyl-4,5,6,7-tetrahydro-1H-imidazo[4, -5-c]pyridine-2-carboxamide

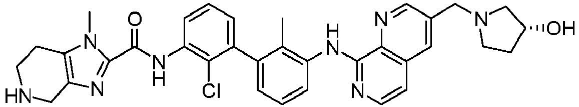

4 N HCl in dioxane (2.0 mL) was added to a mixture of *tert*-butyl (*R*)-2-((2-chloro-3'-((3-((3-hydroxypyrrolidin-1-yl)methyl)-1,7-naphthyridin-8-yl)amino)-2'-methyl-[1,1-biphenyl]-3-yl)carbamoyl)-1-methyl-1,4,6,7-tetrahydro-5*H*-imidazo[4,5-*c*]pyridine-5-carboxylate (0.18 g, 0.249 mmol) in CH₂Cl₂ (1.0 mL)/MeOH (1.0 mL) and the reaction was stirred at rt for 2 h. The solvent was removed and the crude HCl salt was used directly in the next step. LC-MS calculated for C₃₄H₃₆ClN₈O₂ (M+H)⁺: m/z = 623.3; found 623.3.

### Step 5: trans-4-((2-(2-chloro-3'-(3-(((R)-3-hydroxypyrrolidin-1-yl)methyl)-1,7-naphthyridin-8-ylamino)-2'1-3-ylcarbamoyl)-1-methyl-6, 7-dihydro-1H-imidazo[4,5-c]pyridin-5(4H)-yl)methyl)cyclohexanecarboxylic acid

To a mixture of (*R*)-*N*-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-4,5,6,7-tetrahydro-1H-imidazo[4,5-c]pyridine-2-carboxamide (0.025 g, 0.040 mmol) and methyl *trans-4-*formylcyclohexane-1-carboxylate (Ark Pharm, cat#AK-50935: 0.014 g, 0.080 mmol) in CH₂Cl₂ (1.0 mL) was added triethylamine (0.011 mL, 0.201 mmol) and the resulting mixture was stirred for 10 min. Sodium triacetoxyborohydride (0.026 g, 0.120 mmol) was added and the reaction was stirred at rt for 2 h. The solvent was removed and the crude residue was redissoved in methanol/THF/water (0.5/0.5/0.2 mL) and LiOH monohydrate (20 mg) was added. The mixture was then stirred at rt for 3 h. The mixture was diluted with acetonitrile/water, acidified to pH = 2, and purified by prep HPLC (pH = 2, acetonitrile/water+TFA) to provide the desired compound as its TFA salt. LC-MS calculated for C₄₂H₄₈ClN₈O₄ (M+H)⁺: m/z = 763.3; found 763.3.

### Example 48

### cis-4-((2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6, 7-dihydro-1H-imidazo[4,5-c]pyridin-5(4H)-yl)methyl)cyclohexanecarboxylic acid

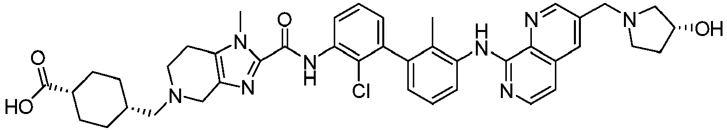

To a mixture of of (*R*)-N-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-4,5,6,7-tetrahydro-1H-imidazo[4,5-c]pyridine-2-carboxamide *(Example 47, Step 4:* 85.0 mg, 0.136 mmol) in DMF (2.0 mL) was added methyl *cis*-4-(((methylsulfonyl)oxy)methyl)cyclohexane-1-carboxylate (Aldlab Chemicals, cat#JPM2-11253: 102 mg, 0.409 mmol), potassium carbonate (56.6 mg, 0.409 mmol), potassium iodide (22.64 mg, 0.136 mmol) and benzyltriethylammonium chloride (31.1 mg, 0.136 mmol). The mixture was then stirred at 75 °C overnight. The solvent was removed and the crude residue was redissolved in methanol/THF/water (0.5/0.5/0.2 mL). LiOH hydrate (20 mg) was added and the mixture was stirred at rt for 5 h. The mixture was diluted with acetonitrile/water, acidified to pH = 2 and purified by prep HPLC (pH = 2, acetonitrile/water+TFA) to provide the desired compound as its TFA salt. LC-MS calculated for C₄₂H₄₈ClN₈O₄ (M+H)⁺: m/z = 763.3; found 763.4.

### Example 49

### cis-4-((2-(2-chloro-2'-methyl-3'-(3-(pyrrolidin-1-ylmethyl)-1,7-naphthyridin-8-ylamino)biphenyl-3-ylcarbamoyl)-1-methyl-6,7-dihydro-1H-imidazo[4,5-c]pyridin-5(4H)-yl)methyl)cyclohexanecarboxylic acid

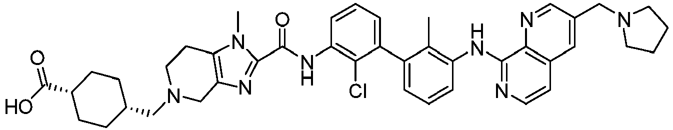

### Step 1: N-(3-bromo-2-methylphenyl)-3-(pyrrolidin-1-ylmethyl)-1,7-naphthyridin-8-amine

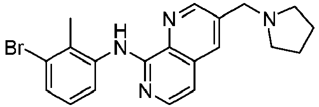

A mixture of 8-((3-bromo-2-methylphenyl)amino)-1,7-naphthyridine-3-carbaldehyde *(Example 9, Step 1:* 0.342 g, 1.0 mmol) and pyrrolidine (0.107 g, 1.500 mmol) in CH₂Cl₂ (8.0 mL) was stirred at rt for 10 min. Sodium triacetoxyborohydride (0.424 g, 2.000 mmol) was then added and the mixture was stirred at rt for 2 h. The mixture was diluted with CH₂Cl₂, washed with 1 N NaOH, water, brine, and the organic phase was separated and dried over Na₂SO₄, filtered and concentrated under reduced pressure. The product was purified by column chromatography eluting with CH₂Cl₂/MeOH (9:1). LC-MS calculated for C₂₀H₂₂BrN₄ (M+H)⁺: m/z = 397.1; found 397.2.

### Step 2: tert-butyl 2-(2-chloro-2'-methyl-3'-(3-(pyrrolidin-1-ylmethyl)-1,7-naphthyridin-8-ylamino)biphenyl-3-ylcarbamoyl)-1-methyl-6,7-dihydro-1H-imidazo[4,5-c]pyridine-5(4H)-carboxylate

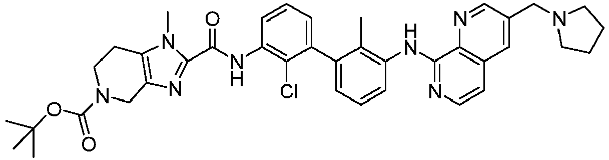

A mixture of tert-butyl 2-((2-chloro-3-(4,4,5,5-tetramethyl-1,3,2-dioxaborolan-2-yl)phenyl)carbamoyl)-1-methyl-1,4,6,7-tetrahydro-5*H*-imidazo[4,5-*c*]pyridine-5-carboxylate *(Example 47, Step 1:* 1.0 g, 1.935 mmol), *N*-(3-bromo-2-methylphenyl)-3-(pyrrolidin-1-ylmethyl)-1,7-naphthyridin-8-amine (0.846 g, 2.128 mmol), dichloro[1,1'-bis(dicyclohexylphosphino)ferrocene]palladium(II) (0.146 g, 0.193 mmol) and cesium fluoride (1.470 g, 9.67 mmol) in *t*-BuOH (3.00 mL)/water (1.2 mL) was evacuated and backfilled with N₂ 3 times. The reaction mixture was stirred at 105 °C for 2 h. The mixture was diluted with ethyl acetate and washed with water, dried over Na₂SO₄, filtered, and concentrated under reduced pressure. The product was purified by column chromatography eluting with CH₂Cl₂/MeOH (9:1). LC-MS calculated for C₃₉H₄₄ClN₈O₃ (M+H)⁺: m/z = 707.3; found 707.5.

### Step 3: N-(2-chloro-2'-methyl-3'-(3-(pyrrolidin-1-ylmethyl)-1,7-naphthyridin-8-ylamino)biphenyl-3-yl)-1-methyl-4,5,6,7-tetrahydro-1H-imidazo[4,-5-c]pyridine-2-carboxamide

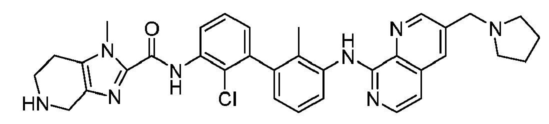

This compound was prepared using a similar procedure as described for *Example 47, Step* 4 with tert-butyl 2-(2-chloro-2'-methyl-3'-(3-(pyrrolidin-1-ylmethyl)-1,7-naphthyridin-8-ylarnino)biphenyl-3-ylcarbamoyl)-1-methyl-6,7-dihydro-1*H*-imidazo[4,5-*c*]pyridine-5(4*H*)-carboxylate replacing *tert*-butyl (*R*)-2-((2-chloro-3'-((3-((3-hydroxypyrrolidin-1-yl)methyl)-1,7-naphthyridin-8-yl)amino)-2'-methyl-[1,1'-biphenyl]-3-yl)carbamoyl)-1-methyl-1,4,6,7-tetrahydro-5*N*-imidazo[4,5-c]pyridine-5-carboxylate. LC-MS calculated for C₃₄H₃₆ClN₈O (M+H)⁺: m/z = 607.3; found 607.4.

### Step 4: cis-4-((2-(2-chloro-2'-methyl-3'-(3-(pyrrolidin-1-ylmethyl)-1,7-naphthyridin-8-ylamino)biphenyl-3-ylcarbamoyl)-1-methyl-6,7-dihydro-1H-imidazo[4,5-c]pyridin-5(4H)-yl)methyl)cyclohexanecarboxylic acid

This compound was prepared using a similar procedure as described for *Example 48* with *N-*(2-chloro-2'-methyl-3'-(3-(pyrrolidin-1-ylmethyl)-1,7-naphthyridin-8-ylamino)biphenyl-3-yl)-1-methyl-4,5,6,7-tetrahydro-1H-imidazol[4.5-*c*]pyridine-2-carboxamide replacing (*R*)-*N*-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-4,5,6,7-tetrahydro-1*H-*imidazo[4,5-*c*]pyridine-2-carboxamide. LC-MS calculated for C₄₂H₄₈ClN₈O₃ (M+H)⁺: m/z = 747.4; found 747.5.

### Example 50

### trans-4-((2-(2-chloro-3'-(3-(((S)-1-hydroxypropan-2-ylamino)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylic acid

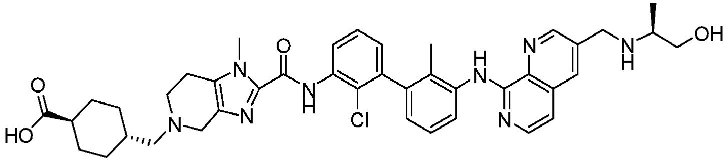

### Step 1: tert-butyl 2-(2-chloro-3 '-(3-(hydroxymethyl)-1,7-naphthyridin-8-ylamino)-2'-methylbiphenyl-3-ylcarbamoyl)-1-methyl-6,7-dihydro-1H-imidazo[4,5-c]pyridine-5(4H)-carboxylate

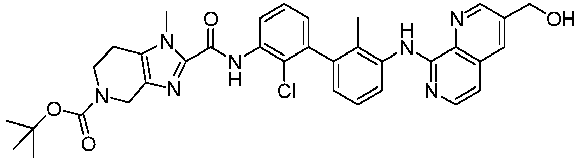

A mixture of tert-butyl 2-((2-chloro-3-(4,4,5,5-tetramethyl-1,3,2-dioxaborolan-2-yl)phenyl)carbamoyl)-1-methyl-1,4,6,7-tetrahydro-5*H*-imidazo[4,5-*c*]pyridine-5-carboxylate *(Example 47, Step 1:* 1.0 g, 1.935 mmol), (8-((3-bromo-2-methylphenyl)amino)-1,7-naphthyridin-3-yl)methanol (Affinity Research Chemicals, #ARI-0169: 0.733 g, 2.128 mmol), dichloro[1,1'-bis(dicyclohexylphosphino)ferrocene]palladium(II) (0.146 g, 0.193 mmol) and cesium fluoride (1.470 g, 9.67 mmol) in *t*-BuOH (3.00 mL) /water (1.2 mL) was evacuated and backfilled with N₂ 3 times. The reaction was stirred at 105 °C for 2 h. The mixture was diluted with ethyl acetate and washed with water, brine, dried over Na₂SO₄, and concentrated under reduced pressure. The product was purified by column chromatography eluting with CH₂Cl₂/EtOAc (1:1). LC-MS calculated for C₃₅H₃₇ClN₇O₄ (M+H)⁺: m/z = 654.2; found 654.2.

### Step 2: N-(2-chloro-3'-(3-(hydroxymethyl)-1,7-naphthyridin-8-ylamino)-2'-methylbiphenyl-3-yl)-1-methyl-4,-5,6,7-tetrahydro-1H-imidazo[4,-5-c]pyridine-2-carboxamide

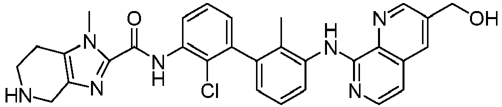

This compound was prepared using a similar procedure as described for *Example 47, Step* 4 with *tert*-butyl 2-(2-chloro-3'-(3-(hydroxymethyl)-1,7-naphthyridin-8-ylamino)-2'-methylbiphenyl-3-ylcarbamoyl)-1-methyl-6,7-dihydro-1H/-imidazol[4,5-c]pyridine-5(4H)-carboxylate replacing *tert*-butyl (*R*)-2-((2-chloro-3'-((3-((3-hydroxypyrrolidin-1-yl)methyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate. LC-MS calculated for C₃₀H₂₉ClN₇O₂ (M+H)⁺: m/z = 554.2; found 554.2.

### Step 3: trans-methyl 4-((2-(2-chloro-3'-(3-(hydroxymethyl)-1,7-naphthyridin-8-ylamino)-2'-methylbiphenyl-3-ylcarbamoyl)-1-methyl-6,7-dihydro-1H-imidazo[4,-5-c]pyridin--5(4H)-yl)methyl)cyclohexanecarboxylate

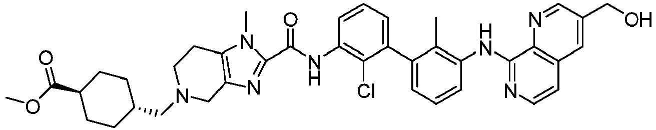

To a mixture of *N*-(2-chloro-3'-((3-(hydroxymethyl)-1,7-naphthyridin-8-yl)amino)-2'-methyl-[1,1'-biphenyl]-3-yl)-1-methyl-4,5,6,7-tetrahydro-1*H*-imidazo[4,5-*c*]pyridine-2-carboxamide (0.075 g, 0.135 mmol) and methyl *trans*-4-formylcyclohexane-1-carboxylate (Ark Pharm, cat#AK-50935: 0.046 g, 0.271 mmol) in CH₂Cl₂ (1.0 mL) was added triethylamine (0.039 mL, 0.677 mmol), and the resulting mixture was stirred at 40 °C for 30 min. Sodium triacetoxyborohydride (0.086 g, 0.406 mmol) was added and stirred at rt for 4 h. The mixture was diluted with CH₂Cl₂ and washed with 1 N NaOH, water, and brine. The organic phase was concentrated under reduced pressure and the crude product was purified by column chromatography eluting with CH₂Cl₂/MeOH (9:1). LC-MS calculated for C₃₉H₄₃ClN₇O₄ (M+H)⁺: m/z = 708.3; found 708.4.

### Step 4: trans-methyl 4-((2-(2-chloro-3'-(3-formyl-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylate

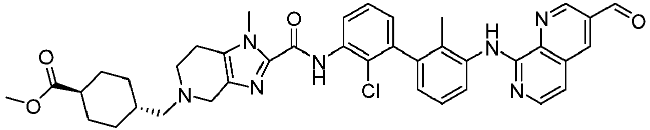

Manganese dioxide (0.313 g, 3.60 mmol) was added to a solution of methyl *trans 4-*((2-((2-chloro-3'-((3-(hydroxymethyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridin-5-yl)methyl)cyclohexane-1-carboxylate (0.17 g, 0.240 mmol) in CH₂Cl₂ (5.0 mL) and the mixture was stirred at 45 °C for 5 h. The mixture was diluted with CH₂Cl₂, filtered through Celite^{®} and then concentrated under reduced pressure. The crude product which was purified by column chromatography eluting with CH₂Cl₂/MeOH (9:1). LC-MS calculated for C₃₉H₄₁ClN₇O₄ (M+H)⁺: m/z = 706.3; found 706.4.

### Step 5: trans-4-((2-(2-chloro-3'-(3-(((S)-1-hydroxypropan-2-ylamino)methyl)-1,7-naphthyridin-8-ylamino)-2'-methylbiphenyl-3-ylcarbamoyl)-1-methyl-6,7 -dihydro-1H-imidazo[4,5-c]pyridin-5(4H)-yl)methyl)cyclohexanecarboxylic acid

A mixture of methyl *trans* 4-((2-((2-chloro-3'-((3-formyl-1,7-naphthyridin-8-yl)amino)-2'-methyl-[1,1'-biphenyl]-3-yl)carbamoyl)-1-methyl-1,4,6,7-tetrahydro-5*H-*imidazo[4,5-c]pyridin-5-yl)methyl)cyclohexane-1-carboxylate (0.010 g, 0.014 mmol) and (*S*)-2-aminopropan-1-ol (Aldrich, cat#A76206: 5.32 mg, 0.071 mmol) in CH₂Cl₂ (1.0 mL) was stirred for 30 min at rt. Sodium triacetoxyborohydride (9.0 mg, 0.042 mmol) was added and the mixture was stirred at rt overnight. The solvent was removed and the crude material was redissoved in methanol/THF/water (0.5/0.5/0.2 mL). LiOH monohydrate (40 mg) was added and the mixture was stirred at rt for 5 h. The mixture was diluted with acetonitrile/water, acidified to pH = 2 and purified by prep HPLC (pH = 2, acetonitrile/water+TFA) to provide the desired compound as its TFA salt. LC-MS calculated for C₄₁H₄₈ClN₈O₄ (M+H)⁺: m/z = 751.3; found 751.4.

### Example 51

### trans-4-( (2-(2-chloro-3'-(3-(((1S,2S)-2-hydroxycyclopentylamino)methyl)-1,7-naphthyridin-8-ylamino)-2'-methylbiphenyl-3-ylcarbamoyl)-1-methyl-6, 7-dihydro-1H-imidazo[4,5-c]pyridin-5(4H)-yt)methyl)cyclohexanecarboxylic acid

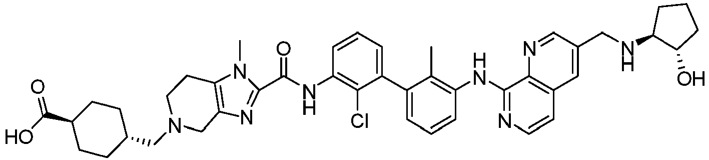

This compound was prepared using a similar procedure as described for *Example 50* with (1*S*,2*S*)-2-aminocyclopentan-1-ol (Ark Pharm, cat#AK-88109) replacing (*S*)-2-aminopropan-1-ol in *Step 5.* LC-MS calculated for C₄₃H₅₀ClN₈O₄ (M+H)⁺: m/z = 777.4; found 777.4.

### Example 52

### trans 4-(2-(2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexanecarboxylic acid

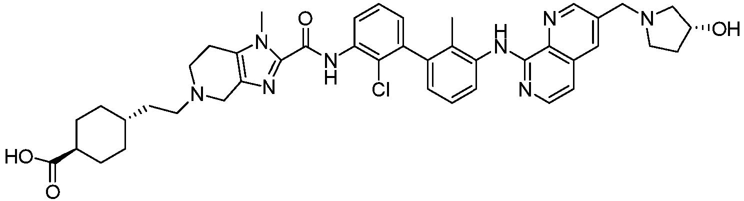

This compound was prepared using a similar procedure as described for *Example 47* with methyl 4-(2-oxoethyl)cyclohexane-1-carboxylate (Enamine, cat#EN300-198655) replacing methyl *trans*-4-formylcyclohexane-1-carboxylate in *Step* 5 as a mixture of diastereomers. The diastereomers were separated using prep HPLC (pH = 2, acetonitrile/water+TFA), with the *trans* isomer eluting first in the column, peak 1: retention time on analytical LCMS (pH=2, acetonitrile/water+TFA) *tᵣ* = 0.80 min; LC-MS calculated for C₄₃H₅₀ClN₈O₄ (M+H)⁺: m/z = 777.4; found 777.4.

### Example 53

### cis 4-(2-(2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6, 7-dihydro-1H-imidazo[4,5-c] pyridin-5(4H)-yl)ethyl)cyclohexanecarboxylic acid

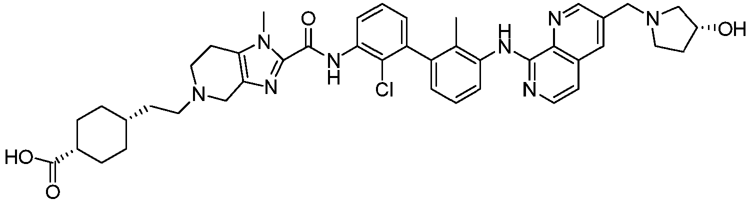

This compound was prepared using a similar procedure as described for *Example 52* The diastereomers were separated using prep HPLC (pH = 2, acetonitrile/water+TFA), with the minor *cis* isomer eluting later in the column, **peak 2**: retention time on analytical LCMS (pH=2, acetonitrile/water+TFA) *tᵣ* = 0.82 min; LC-MS calculated for C₄₃H₅₀ClN₈O₄ (M+H)⁺: m/z = 777.4; found 777.4.

### Example 54

### 3-(2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)butanoic acid

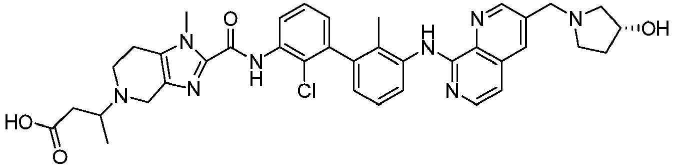

This compound was prepared using a similar procedure as described for *Example 47* with methyl 3-oxobutanoate (Aldrich, cat#537365) replacing methyl *trans-4-*formylcyclohexane-1-carboxylate in *Step 5.* LC-MS calculated for C₃₈H₄₂ClN₈O₄ (M+H)⁺: m/z = 709.3; found 709.2.

### Example 55

### cis 4-((2-(2-chloro-3'-(3-(((S)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylic acid

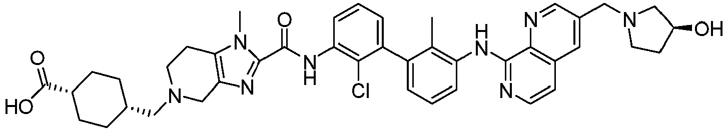

### Step 1: N-(2-chloro-3'-(3-formyl-1,7-naphthyridin-8-ylamino)-2'-methylbiphenyl-3-yl)-1-methyl-4, 5,6,7-tetrahydro-1H-imidazo[4,5-c]pyridine-2-carboxamide

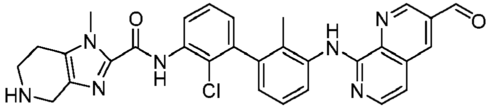

TFA (2.0 mL, 26.0 mmol) was added to a solution of *tert*-butyl 2-((2-chloro-3'-((3-formyl-1,7-naphthyridin-8-yl)amino)-2'-methyl-[1,1'-biphenyl] -3-yl)carbamoyl)-1-methyl-1,4,6,7-tetrahydro-5*H*-imidazo[4,5-*c*]pyridine-5-carboxylate *(Example 45, Step* 2: 0.20 g, 0.307 mmol) in CH₂Cl₂ (1.0 mL) at rt and the the reaction was stirred for 30 min. The solvent was removed under vacuum and the crude TFA salt was used directly in the next step without further purification. LC-MS calculated for C₃₀H₂₇ClN₇O₂ (M+H)⁺: m/z = 552.2; found 552.1.

### Step 2: cis-methyl 4-((2-(2-chloro-3'-(3-formyl-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylate

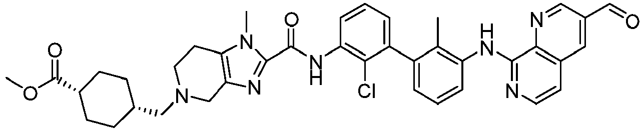

To a mixture of of *N*-(2-chloro-3'-((3-formyl-1,7-naphthyridin-8-yl)amino)-2'-methyl-[1,1'-biphenyl]-3-yl)-1-methyl-4,5,6,7-tetrahydro-1*H*-imidazo[4,5-*c*]pyridine-2-carboxamide (150.0 mg, 0.272 mmol) in DMF (2.0 mL) was added methyl *cis-4-*(((methylsulfonyl)oxy)methyl)cyclohexane-1-carboxylate (Aldlab Chemicals, cat#JPM2-11253: 136 mg, 0.543 mmol), potassium carbonate (113 mg, 0.815 mmol), potassium iodide (45.1 mg, 0.272 mmol), and benzyltriethylammonium chloride (61.9 mg, 0.272 mmol). The resulting mixture was then stirred at 75 °C overnight. The mixture was diluted with CH₂Cl₂ and then washed with water and brine. The organic phase was dried over MgSO₄, filtered, and concentrated under reduced pressure. The product was purified by column chromatography eluting with CH₂Cl₂/MeOH (9:1). LC-MS calculated for C₃₉H₄₁ClN₇O₄ (M+H)⁺: m/z = 706.3; found 706.4.

### Step 3: cis-4-((2-(2-chloro-3'-(3-(((S)-3-hydroxypyrrolidin-1-yl)methyl)-1,7-naphthyridin-8-ylamino)-2'1-3-ylcarbamoyl)-1-methyl-6, 7-dihydro-1H-imidazo[4,5-c]pyridin-5(4H)-yl)methyl)cyclohexanecarboxylic acid

A mixture of methyl *cis*-4-((2-((2-chloro-3'-((3-formyl-1,7-naphthyridin-8-yl)amino)-2'-methyl-[1,1'-biphenyl]-3-yl)carbamoyl)-1-methyl-1,4,6,7-tetrahydro-5*H*-imidazo[4,5-c]pyridin-5-yl)methyl)cyclohexane-1-carboxylate (0.010 g, 0.014 mmol) and (*S*)-pyrrolidin-3-ol (Combi-Blocks, cat#SS-7948, 1.234 mg, 0.014 mmol) in CH₂Cl₂ (1.0 mL) was stirred for 30 min and then sodium triacetoxyborohydride (9.00 mg, 0.042 mmol) was added and stirred at rt overnight. The solvent was removed and the crude was redissolved in methanol/THF/water (0.5/0.5/0.2 mL). LiOH monohydrate (40 mg) was added and the mixture was stirred at rt for 5 h. The mixture was diluted with acetonitrile/water, acidified to pH = 2 and purified by prep HPLC (pH = 2, acetonitrile/water+TFA) to provide the desired compound as its TFA salt. LC-MS calculated for C₄₂H₄₈ClN₈O₄ (M+H)⁺: m/z = 763.3; found 763.5.

### Example 56

### cis 4-((2-(2-chloro-3'-(3-(((R)-3-hydroxy-3-methylpyrrolidin-1-yl)methyl)-1,7-naphthyridin-8-ylamino)-2'-methylbiphenyl-3-ylcarbamoyl)-1-methyl-6, 7-dihydro-1H-imidazo[4,5-c]pyridin-5(4H)-yl)methyl)cyclohexanecarboxylic acid

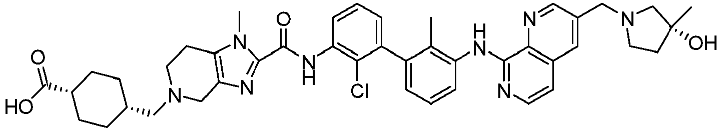

This compound was prepared using a similar procedure as described for *Example 55* with (*R*)-3-methylpyrrolidin-3-ol (Ark Pharm, cat#AK100499) replacing (*S*)-pyrrolidin-3-ol in *Step 3.* LC-MS calculated for C₄₃H₅₀ClN₈O₄ (M+H)⁺: m/z = 777.3; found 777.3.

### Example 57

### (R)-4-(2-(2-chloro-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)cyclohexanecarboxylic acid

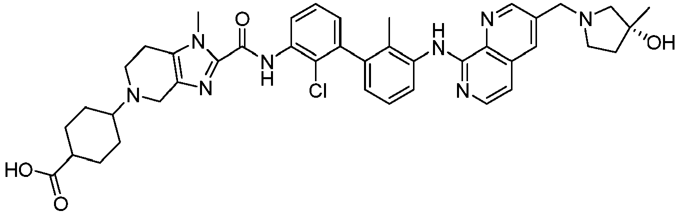

### Step 1: tert-butyl 4-(2-(2-chloro-3'-(3-(hydroxymethyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)cyclohexanecarboxylate

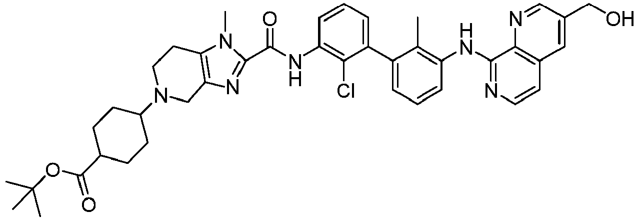

To a mixture of *N*-(2-chloro-3'-((3-(hydroxymethyl)-1,7-naphthyridin-8-yl)amino)-2'-methyl-[1,1'-biphenyl]-3-yl)-1-methyl-4,5,6,7-tetrahydro-1H-imidazo[4,5-*c*]pyridine-2-carboxamide *(Example 50, Step* 2: 0.275 g, 0.496 mmol) and tert-butyl 4-oxocyclohexane-1-carboxylate (Ark Pharm, cat#AK-40114: 0.197 g, 0.993 mmol) in CH₂Cl₂ (1.0 mL) was added triethylamine (0.142 mL, 2.482 mmol). The resulting mixture was stirred at 40 °C for 30 min and then sodium triacetoxyborohydride (0.316 g, 1.489 mmol) was added and stirred at rt overnight. The mixture was diluted with CH₂Cl₂ and washed with 1 N NaOH, water, and brine. The solvent was removed and the product was purified by column chromatography eluting with CH₂Cl₂/MeOH (9:1). LC-MS calculated for C₄₁H₄₇ClN₇O₄ (M+H)⁺: m/z = 736.3; found 736.3.

### Step 2: tert-butyl 4-(2-(2-chloro-2'-methyl-3'-(3-((methylsulfonyloxy)methyl)-1,7-naphthyridin-8-ylamino)biphenyl-3-ylcarbamoyl)-1-methyl-6,7-dihydro-1H-imidazo[4,5-c]pyridin-5(4H)-yl)cyclohexanecarboxylate

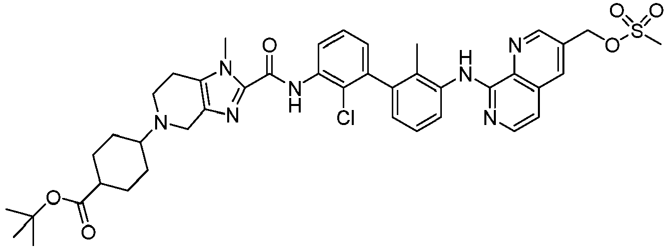

Methanesulfonyl chloride (0.023 g, 0.204 mmol) was added to a solution of *tert*-butyl 4-(2-((2-chloro-3'-((3-(hydroxymethyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridin-5-yl)cyclohexane-1-carboxylate (0.10 g, 0.136 mmol) and triethylamine (0.057 mL, 0.407 mmol) in CH₂Cl₂ (2.0 mL) at 0 °C and then the reaction was stirred at this temperature for 30 min. The mixture was quenched by adding aqueous saturated NaHCO₃, and the aqueous phase was extracted with methylene chloride. The combined organic layers were dried over Na₂SO₄, filtered, and concentrated under reduced pressure, and the crude product was used directly in the next step. LC-MS calculated for C₄₂H₄₉ClN₇O₆S (M+H)⁺: m/z = 814.3; found 814.3.

### Step 3: (R)-4-(2-(2-chloro-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)cyclohexanecarboxylic acid

(*R*)-3-methylpyrrolidin-3-ol (Ark Pharm, cat#AK100499: 2.484 mg, 0.025 mmol) was added to a solution of tert-butyl 4-(2-((2-chloro-2'-methyl-3'-((3-(((methylsulfonyl)oxy)methyl)-1,7-naphthyridin-8-yl)amino)-[1,1'-biphenyl]-3-yl)carbamoyl)-1-methyl-1,4,6,7-tetrahydro-5*H*-imidazo[4,5-*c*]pyridin-5-yl)cyclohexane-1-carboxylate (0.020 g, 0.025 mmol) and triethylamine (0.021 mL, 0.147 mmol) in CH₂Cl₂ (0.8 mL) at rt. The reaction was stirred at 30 °C for 1 h. The solvent was removed and the residue was treated with 4 N HCl in dioxane (1.0 mL) for 2 h. The mixture was diluted with acetonitrile/water, acidified to pH = 2 and purified by prep HPLC (pH = 2, acetonitrile/water+TFA) to provide the desired compound as its TFA salt. LC-MS calculated for C₄₂H₄₈ClN₈O₄ (M+H)⁺: m/z = 763.3; found 763.3.

### Example 58

### (S)-4-(2-(2-chloro-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)cyclohexanecarboxylic acid

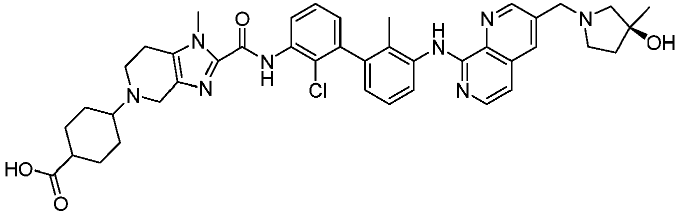

This compound was prepared using a similar procedure as described for *Example 57* with (*S*)-3-methylpyrrolidin-3-ol (J&W Pharma, cat#75R0496) replacing (R)-3-methylpyrrolidin-3-ol in *Step 3.* LC-MS calculated for C₄₂H₄₈ClN₈O₄ (M+H)⁺: m/z = 763.3; found 763.3.

### Example 59

### trans 4-(2-(2-(2-chloro-3'-(3-(((R)-1-hydroxypropan-2-ylamino)methyl)-1,7-naphthyridin-8-ylamino)-2'-methylbiphenyl-3-ylcarbamoyl)-1-methyl-6, 7-dihydro-1H-imidazo[4,5-c]pyridin-5(4H)-yl)ethyl)cyclohexanecarboxylic acid

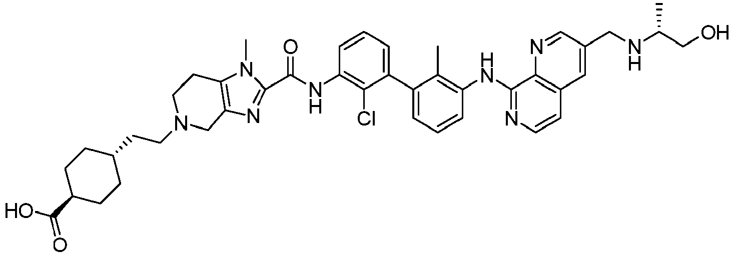

### Step 1: trans methyl 4-(2-(2-(2-chloro-3'-(3-(hydroxymethyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexanecarboxylate

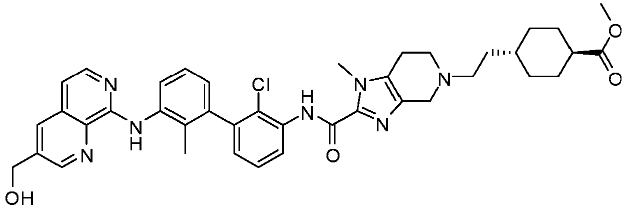

To a mixture of *N*-(2-chloro-3'-((3-(hydroxymethyl)-1,7-naphthyridin-8-yl)amino)-2'-methyl-[1,1'-biphenyl]-3-yl)-1-methyl-4,5,6,7-tetrahydro-1*H*-imidazo [4,5-*c*]pyridine-2-carboxamide *(Example 50, Step* 2: 0.075 g, 0.135 mmol) and methyl *trans 4-(2-*oxoethyl)cyclohexane-1-carboxylate (Enamine, cat#EN300-198655: 0.050 g, 0.271 mmol) in CH₂Cl₂ (1.0 mL) was added triethylamine (0.039 mL, 0.677 mmol) and the resulting mixture was stirred at 40 °C for 30 min. Sodium triacetoxyborohydride (0.086 g, 0.406 mmol) was added and stirred at rt for 4 h .The mixture was diluted with CH₂Cl₂ and washed with 1 N NaOH, water, and brine. The solvent was removed and the product was purified by column chromatography eluting with CH₂Cl₂/MeOH (9:1). LC-MS calculated for C₄₀H₄₅ClN₇O₄ (M+H)⁺: m/z = 722.3; found 722.4.

### Step 2: trans methyl 4-(2-(2-(2-chloro-2'-methyl-3'-(3-((methylsulfonyloxy)methyl)-1,7-naphthyridin-8-ylamino)biphenyl-3-ylcarbamoyl)-1-methyl-6,7-dihydro-1H-imidazo[4,5-c]pyridin-5(4H)-yl)ethyl)cyclohexanecarboxylate

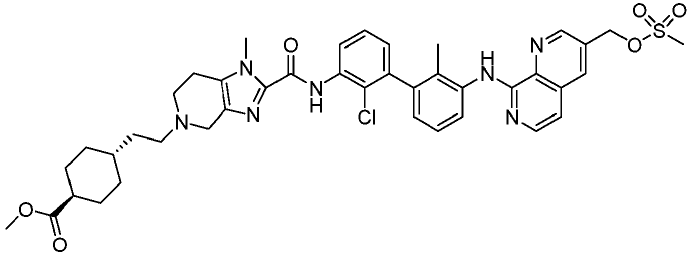

Methanesulfonyl chloride (0.024 g, 0.208 mmol) was added to a solution *of trans* methyl 4-(2-(2-((2-chloro-3'-((3-(hydroxymethyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridin-5-yl)ethyl)cyclohexane-1-carboxylate (0.10 g, 0.138 mmol) and triethylamine (0.058 mL, 0.415 mmol) in CH₂Cl₂ (2.0 mL) at 0 °C and then the reaction was stirred at this temperature for 30 min. The mixture was quenched by adding aqueous saturated NaHCO₃, and the reaction was extracted with methylene chloride. The combined organic layers were dried over Na₂SO₄, filtered, and concentrated under reduced pressure. The crude product was used directly in the next step. LC-MS calculated for C₄₁H₄₇ClN₇O₆S (M+H)⁺: m/z = 800.3; found 800.3.

### Step 3: trans-4-(2-(2-(2-chloro-3'-(3-(((R)-1-hydroxypropan-2-ylamino)methyl)-1,7-naphthyridin-8-ylamino)-2'-methylbiphenyl-3-ylcarbamoyl)-1-methyl-6,7 -dihydro-1H-imidazo[4,5-c]pyridin-5(4H)-yl)ethyl)cyclohexanecarboxylic acid

(*R*)-2-aminopropan-1-ol (Aldrich, cat#297682: 1.9 mg, 0.025 mmol) was added to a solution of *trans* methyl 4-(2-(2-((2-chloro-2'-methyl-3'-((3-(((methylsulfonyl)oxy)methyl)-1,7-naphthyridin-8-yl)amino)-[1,1'-biphenyl]-3-yl)carbamoyl)-1-methyl-1,4,6,7-tetrahydro-5*H*-imidazo[4,5-*c*]pyridin-5-yl)ethyl)cyclohexane-1-carboxylate (20 mg, 0.025 mmol) and triethylamine (0.021 mL, 0.147 mmol) in CH₂Cl₂ (0.8 mL) at rt. The reaction was stirred at 30 °C for 1 h. The solvent was removed and the residue was dissolved in MeOH/THF/water (0.4/0.4/0.2 mL). LiOH monohydrate (40 mg) was added and stirred at rt for 4 h. The mixture was diluted with acetonitrile/water, acidified to pH = 2 and purified by prep HPLC (pH = 2, acetonitrile/water+TFA) to provide the desired compound as its TFA salt. LC-MS calculated for C₄₂H₅₀ClN₈O₄ (M+H)⁺: m/z = 765.4; found 765.5.

### Example 60

### trans 4-(2-(2-(2-chloro-3'-(3-(((S)-1-hydroxypropan-2-ylamino)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexanecarboxylic acid

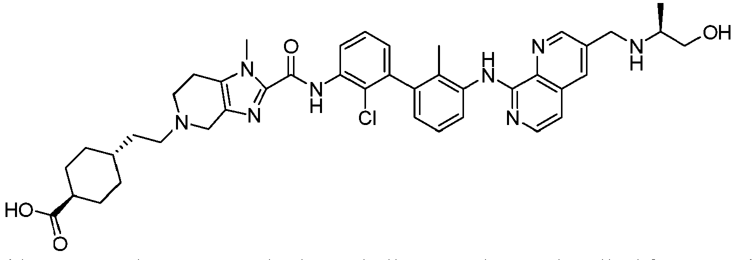

This compound was prepared using a similar procedure as described for *Example 59* with (*S*)-2-aminopropan-1-ol (Aldrich, cat#A76206) replacing (*R*)-2-aminopropan-1-ol in *Step 3.* LC-MS calculated for C₄₂H₅₀ClN₈O₄ (M+H)⁺: m/z = 765.4; found 765.5.

### Example 61

### trans-4-(2-(2-(2-chloro-3'-(3-(((R)-3-hydroxy-3-methylpyrrolidin-1-yl)methyl)-1,7-naphthyridin-8-ylamino)-2'-methylbiphenyl-3-ylcarbamoyl)-1-methyl-6, 7-dihydro-1H-imidazo[4,5-c]pyridin-5(4H)-yl)ethyl)cyclohexanecarboxylic acid

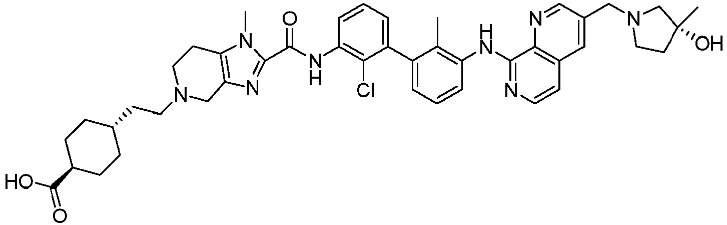

This compound was prepared using a similar procedure as described for *Example 59* with (*R*)-3-methylpyrrolidin-3-ol (Ark Pharm, cat#AK100499) replacing (*R*)-2-aminopropan-1-ol in *Step 3.* LC-MS calculated for C₄₄H₅₂ClN₈O₄ (M+H)⁺: m/z = 791.4; found 791.4.

### Example 62

### (R)-4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridin-5(4H)-yl)-1-methylcyclohexanecarboxylic acid

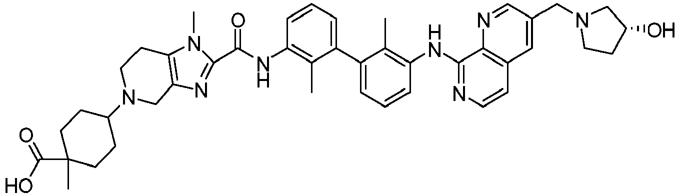

### Step 1: (R)-tert-butyl 2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridine-5(4H)-carboxylate

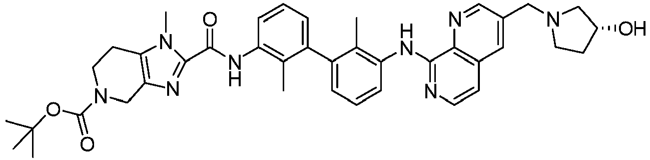

A mixture of tert-butyl 1-methyl-2-((2-methyl-3-(4,4,5,5-tetramethyl-1,3,2-dioxaborolan-2-yl)phenyl)carbamoyl)-1,4,6,7-tetrahydro-5*H*-imidazo[4,5-*c*]pyridine-5-carboxylate *(Example 14, Step 3:* 0.25 g, 0.504 mmol), (*R*)-1-((8-((3-bromo-2-methylphenyl)amino)-1,7-naphthyridin-3-yl)methyl)pyrrolidin-3-ol *(Example 9, Step* 2: 0.229 g, 0.554 mmol), dichloro[1,1'-bis(dicyclohexylphosphino)ferrocene]palladium(II), (0.038 g, 0.050 mmol), and cesium fluoride (0.383 g, 2.52 mmol) in *t*-BuOH (8.00 mL)/water (3.0 mL) was evacuated and backfilled with N₂. The evacuation/backfill sequence was repeated two more times, and then the reaction was stirred at 105 °C for 2 h. The mixture was diluted with ethyl acetate and washed with water and brine. The organic phase was dried over MgSO₄, filtered, and concentrated. The product was purified by column chromatography eluting with CH₂Cl₂/MeOH (9:1). LC-MS calculated for C₄₀H₄₇N₈O₄ (M+H)⁺: m/z = 703.4; found 703.6.

### Step 2: (R)-N-(3'-(3-((3-hydroxypyrrolidin-1-yl)methyl)-1,7-naphthyridin-8-ylatnino)-2,2'-dimethylbiphenyl-3-yl)-1-methyl-4,5,6,7-tetrahydro-1H-imidazo[4,5-c]pyridine-2-carboxamide

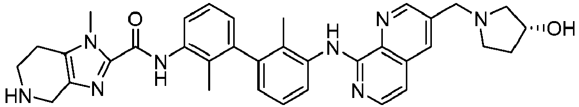

This compound was prepared using a similar procedure as described for *Example 47, Step* 4 with *(R)-tert*-butyl 2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridine-5(4*H*)-carboxylate replacing *tert-butyl* (*R*)-2-((2-chloro-3'-((3-((3-hydroxypyrrolidin-1-yl)methyl)-1,7-naphthyridin-8-yl)amino)-2'-mettiyl-[1,1'-biphenyl]-3-yl)carbamoyl)-1-methyl-1,4,6,7-tetrahydro-5*H*-imidazo[4,5-*c*]pyridine-5-carboxylate. LC-MS calculated for C₃₅H₃₉N₈O₂ (M+H)⁺: m/z = 603.3; found 603.3.

### Step 3: (R)-4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridin-5(4H)-yl)-1-methylcyclohexanecarboxylic acid

To a mixture of (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide (0.025 g, 0.041 mmol) and 1-methyl-4-oxocyclohexane-1-carboxylic acid (Aurum Pharmatech, cat#U31985: 6.48 mg, 0.041 mmol) in CH₂Cl₂ (1.0 mL) was added triethylamine (0.012 mL, 0.207 mmol), and the reaction was stirred for 30 min at 40 °C. Sodium triacetoxyborohydride (0.026 g, 0.124 mmol) was added and stirred at 40 °C for 4 h. The mixture was diluted with acetonitrile/water, acidified to pH = 2 and purified by prep HPLC (pH = 2, acetonitrile/water+TFA) to provide the two desired compounds as TFA salts.
**Peak 1:** retention time on analytical LCMS (pH=2, acetonitrile/water+TFA) *tᵣ* = 0.686 min; LC-MS calculated for C₄₃H₅₁N₈O₄ (M+H)⁺: m/z = 743.4; found 743.4.
**Peak** 2: retention time on analytical LCMS (pH=2, acetonitrile/water+TFA) *tᵣ* = 0.700 min; LC-MS calculated for C₄₃H₅₁N₈O₄ (M+H)⁺: m/z = 743.4; found 743.4.

### Example 63

### trans-4-(2-(2-(3'-(3-(((R)-3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridin-5(4H)-yl)ethyl)cyclohexanecarboxylic acid

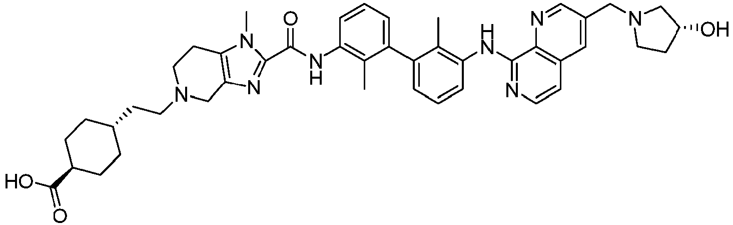

To a mixture of (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide *(Example 62, Step* 2: 0.025 g, 0.041 mmol) and methyl *trans 4-*(2-oxoethyl)cyclohexane-1-carboxylate (Enamine, cat#EN300-198655: 0.015 g, 0.083 mmol) in CH₂Cl₂ (1.0 mL) was added triethylamine (0.012 mL, 0.207 mmol). The resulting mixture was stirred for 10 min and then sodium triacetoxyborohydride (0.026 g, 0.124 mmol) was added and stirred at rt overnight. The solvent was removed and the crude was redissoved in methanol/THF/water (0.5/0.5/0.2 mL). LiOH monohydrate (20 mg) was added and the mixture was stirred at rt for 3 h. The mixture was diluted with acetonitrile/water, acidified to pH = 2, and purified by prep HPLC (pH = 2, acetonitrile/water+TFA) to provide the desired compound as its TFA salt. LC-MS calculated for C₄₄H₅₃N₈O₄ (M+H)⁺: m/z = 757.4; found 757.6.

### Example 64

### (R)-4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridin-5(4H)-yl)cyclohexanecarboxylic acid

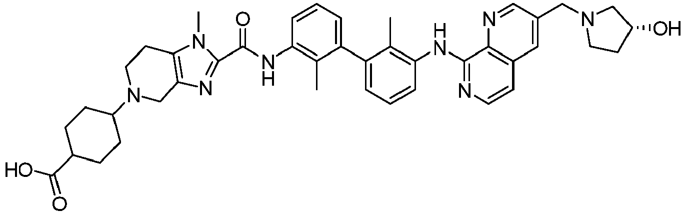

To a mixture of (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide *(Example 62, Step* 2: 0.275 g, 0.456 mmol) and tert-butyl 4-oxocyclohexane-1-carboxylate (Ark Pharm, cat#AK-40114: 0.181 g, 0.912 mmol) in CH₂Cl₂ (1.0 mL) was added triethylamine (0.131 mL, 2.281 mmol). The mixture was stirred at 40 °C for 30 min and then sodium triacetoxyborohydride (0.290 g, 1.369 mmol) was added and stirred at rt overnight to provide (*R*)-*tert*-butyl 4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridin-5(4*H*)-yl)cyclohexanecarboxylate as a mixture *of cis & trans* isomers which were separated by prep-HPLC (pH = 10, acetonitrile/water+NH₄OH): **Peak 1:** retention time on analytical LCMS (pH=10, acetonitrile/water+NH₄OH), *tᵣ* = 1.78 min; LC-MS calculated for C₄₆H₅₇N₈O₄ (M+H)⁺: m/z = 785.4; found 785.4. **Peak 2:** retention time on analytical LCMS (pH=10, acetonitrile/water+NH₄OH), *tᵣ* = 1.82 min; LC-MS calculated for C₄₆H₅₇N₈O₄ (M+H)⁺: m/z = 785.4; found 785.4.

The fractions of each peak were combined, concentrated under reduced pressure, and the resulting residues were then treated with 4 N HCl (in dioxane) for 4 h. The respective mixtures were diluted with acetonitrile/water, acidified to pH = 2 and purified by prep HPLC (pH = 2, acetonitrile/water+TFA) to provide each isomer as its TFA salt.
**Peak 1:** retention time on analytical LCMS (pH=2, acetonitrile/water+TFA), *tᵣ =* 0.656 min; LC-MS calculated for C₄₂H₄₉N₈O₄ (M+H)⁺: m/z = 729.4; found 729.4.
**Peak 2:** retention time on analytical LCMS (pH=2, acetonitrile/water+TFA), *tᵣ =* 0.663 min; LC-MS calculated for C₄₂H₄₉N₈O₄ (M+H)⁺: m/z = 729.4; found 729.4.

### Example 65

### Trans-4-(2-(2-(2,2'-dichloro-3'-(3-(((R)-3-hydroxypyrrolidin-1-yl)methyl)-1,7-naphthyridin-8-ylamino)biphenyl-3-ylcarbamoyl)-1-methyl-6,7-dihydro-1H-imidazo[4,5-c]pyridin-5(4H)-yl)ethyl)cyclohexanecarboxylic acid

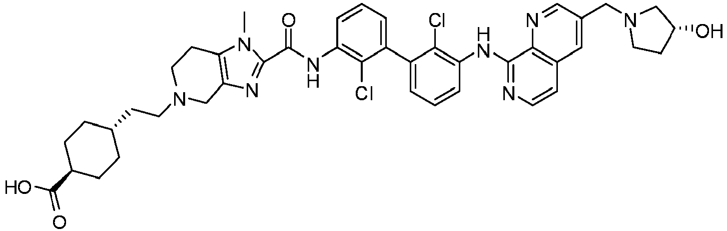

### Step 1: (R)-tert-butyl 2-(2,2'-dichloro-3'-(3-((3-hydroxypyrrolidin-l-yl)methyl)-1,7-naphthyridin-8-ylamino)biphenyl-3-ylcarbamoyl)-1-methyl-6,7-dihydro-1H-imidazo[4,5-c]pyridine-5(4H)-carboxylate

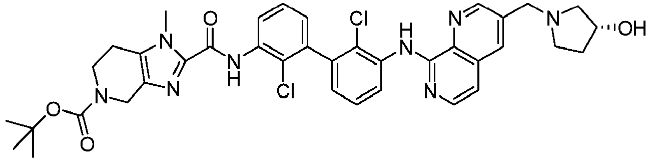

A mixture of tert-butyl 2-((2-chloro-3-(4,4,5,5-tetramethyl-1,3,2-dioxaborolan-2-yl)phenyl)carbamoyl)-1-methyl-1,4,6,7-tetrahydro-5*H*-imidazo[4,5-*c*]pyridine-5-carboxylate *(Example 47, Step 1:* 0.050 g, 0.097 mmol), (*R*)-1-((8-((3-bromo-2-chlorophenyl)amino)-1,7-naphthyridin-3-yl)methyl)pyrrolidin-3-ol *(Example 22, Step 4:* 0.046 g, 0.106 mmol), dichloro[1,1'-bis(dicyclohexylphosphino)ferrocene]palladium(II) (7.31 mg, 9.67 µmol) and cesium fluoride (0.073 g, 0.484 mmol) in *t*-BuOH (8.00 mL)/water (3.0 mL) was evacuated and flushed with N₂ 3 times. The reaction was stirred at 105 °C for 2 h. The mixture was diluted with ethyl acetate and washed with water and brine. The organic layer was separated, dried over Na₂SO₄, filtered, and concentrated under reduced pressure. The product was purified by column chromatography eluting with CH₂Cl₂/MeOH (9:1). LC-MS calculated for C₃₈H₄₁Cl₂N₈O₄ (M+H)⁺: m/z = 743.3; found 743.3.

### Step 2: (R)-N-(2,2'-dichloro-3'-(3-((3-hydroxypyrrolidin-1-yl)methyl)-1,7-naphthyridin-8-ylamino)biphenyl-3 yl)-1-methyl-4,5,6,7-tetrahydro-1H-imidazo[4, -5-c]pyridine-2-carboxamide

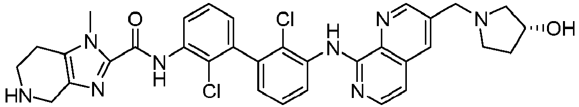

This compound was prepared using a similar procedure as described for *Example 47, Step* 4 with (*R*)-*tert*-butyl 2-(2,2'-dichloro-3'-(3-((3-hydroxypyrrolidin-1-yl)methyl)-1,7-naphthyridin-8-ylamino)biphenyl-3-ylcarbamoyl)-1-methyl-6,7-dihydro-1*H*-imidazo[4,5-*c*]pyridine-5(4*H*)-carboxylate replacing *tert-*butyl (*R*)-2-((2-chloro-3'-((3-((3-hydroxypyrrolidin-1-yl)methyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate. LC-MS calculated for C₃₃H₃₃Cl₂N₈O₂ (M+H)⁺: m/z = 643.2; found 643.2.

### Step 3: Trans 4-(2-(2-(2,2'-dichloro-3'-(3-(((R)-3-hydroxypyrrolidin-1-yl)methyl)-1,7-naphthyridin-8-ylamino)biphenyl-3-ylcarbamoyl)-1-methyl-6,7-dihydro-1H-imidazo[4,5-c]pyridin-5(4H)-yl)ethyl)cyclohexanecarboxylic acid

This compound was prepared using a similar procedure as described for *Example 63* with (R)-N-(2,2'-dichloro-3'-((3-((3-hydroxypyrrolidin-1-yl)methyl)-1,7-naphthyridin-8-yl)amino)-[1,1'-biphenyl]-3-yl)-1-methyl-4,5,6,7-tetrahydro-1*H*-imidazo[4,5-*c*]pyridine-2-carboxamide replacing (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide. LC-MS calculated for C₄₂H₄₇Cl₂N₈O₄ (M+H)⁺: m/z = 797.3; found 797.2.

### Example 66

### trans 4-(2-(2-(2'-chloro-3'-(3-(((R)-3-hydroxypyrrolidin-1-yl)methyl)-1,7-naphthyridin-8-ylamino)-2-methylbiphenyl-3-ylcarbamoyl)-1-methyl-6,7-dihydro-1H-imidazo[4,5-c]pyridin-5(4H)-yl)ethyl)cyclohexanecarboxylic acid

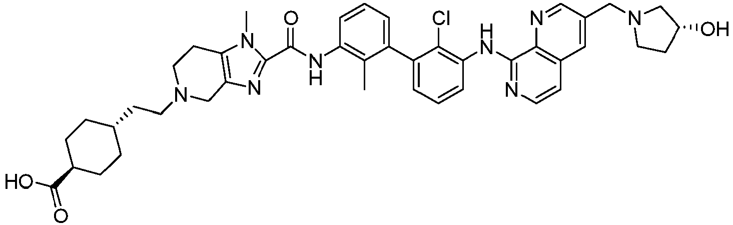

### Step 1: (R)-tert-butyl 2-(2'-chloro-3'-(3-((3-hydroxypyrrolidin-1-yl)methyl)-1,7-naphthyridin-8-ylamino)-2-methylbiphenyl-3-ylcarbamoyl)-1-methyl-6,7-dihydro-1H-imidazo[4,5-c]pyridine-5(4H)-carboxylate

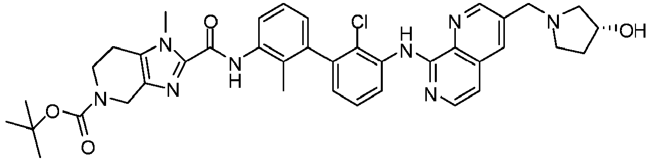

This compound was prepared using a similar procedure as described for *Example 65* with *tert*-butyl 1-methyl-2-(2-methyl-3-(4,4,5,5-tetramethyl-1,3,2-dioxaborolan-2-yl)phenylcarbamoyl)-6,7-dihydro-1*H*-imidazo[4,5-*c*]pyridine-5(4*H*)-carboxylate (*Example 14, Step 3)* replacing *tert*-butyl 2-((2-chloro-3-(4,4,5,5-tetramethyl-1,3,2-dioxaborolan-2-yl)phenyl)carbamoyl)-1-methyl-1,4,6,7-tetrahydro-5*H*-imidazo[4,5-*c*]pyridine-5-carboxylate in *Step 1.* LC-MS calculated for C₃₉H₄₄ClN₈O₄ (M+H)⁺: m/z = 723.3; found 723.3.

### Step 2: (R)-N-(2'-chloro-3'-(3-((3-hydroxypyrrolidin-1-yl)methyl)-1,7-naphthyridin-8-ylamino)-2-methylbiphenyl-3-yl)-1-methyl-4,5,6,7-tetrahydro-1H-imidazo[4,5-c]pyridine-2-carboxamide

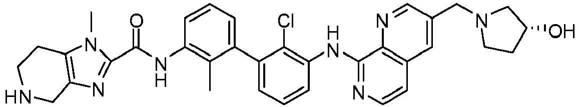

This compound was prepared using a similar procedure as described for *Example 47* with (R)-tert-butyl 2-(2'-chloro-3'-(3-((3-hydroxypyrrolidin-1-yl)methyl)-1,7-naphthyridin-8-ylamino)-2-methylbiphenyl-3-ylcarbamoyl)-1-methyl-6,7-dihydro-1*H*-imidazo[4,5-*c*]pyridine-5(4*H*)-carboxylate replacing *tert-*butyl (*R*)-2-((2-chloro-3'-((3-((3-hydroxypyrrolidin-1-yl)methyl)-1,7-naphthyridin-8-yl)amino)-2'-methyl-[1,1'-biphenyl]-3-yl)carbamoyl)-1-methyl-1,4,6,7-tetrahydro-5H-imidazo[4,5-c]pyridine-5-carboxylate in *Step* 4. LC-MS calculated for C₃₄H₃₆ClN₈O₂ (M+H)⁺: m/z = 623.3; found 623.3.

### Step 3: trans 4-(2-(2-(2'-chloro-3'-(3-(((R)-3-hydroxypyrrolidin-1-yl)methyl)-1,7-naphthyridin-8-ylamino)-2-methylbiphenyl-3-ylcarbamoyl)-1-methyl-6,7-dihydro-1H-imidazo[4,5-c]pyridin-5(4H)-yl)ethyl)cyclohexanecarboxylic acid

This compound was prepared using a similar procedure as described for *Example 63* with (*R*)-*N*-(2'-chloro-3'-(3-((3-hydroxypyrrolidin-1-yl)methyl)-1,7-naphthyridin-8-ylamino)-2-methylbiphenyl-3-yl)-1-methyl-4,5,6,7-tetrahydro-1*H*-imidazo[4,5-*c*]pyridine-2-carboxamide replacing (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide. LC-MS calculated for C₄₃H₅₀ClN₈O₄ (M+H)⁺: m/z = 777.4; found 777.4.

### Example 67

### (R)-1-((4-(2'-chloro-3'-(1,5-dimethyl-4,5,6,7-tetrahydro-1H-imidazo[4,5-c]pyridine-2-carboxamido)-2-methylbiphenyl-3-ylamino)pyrido[3,2-d]pyrimidin-7-yl)methyl)-3-methylpyrrolidine-3-carboxylic acid

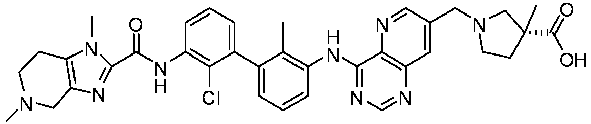

### Step 1: tert-butyl 2-(3'-amino-2-chloro-2'-methylbiphenyl-3-ylcarbamoyl)-1-methyl-6,7-dihydro-1H-imidazo[4,5-c]pyridine-5(4H)-carboxylate

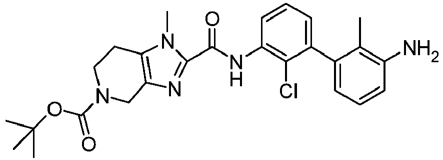

A mixture of *tert*-butyl 2-((3-bromo-2-chlorophenyl)carbamoyl)-1-methyl-1,4,6,7-tetrahydro-5*H*-imidazo[4,5-*c*]pyridine-5-carboxylate (*Example 31, Step 3:* 0.470 g, 1.0 mmol), 2-methyl-3-(4,4,5,5-tetramethyl-1,3,2-dioxaborolan-2-yl)aniline (Combi-Blocks, cat#PN-9127: 0.233 g, 1.000 mmol), dichloro[1,1'-bis(diphenylphosphino)ferrocene]palladium (II) dichloromethane adduct (0.082 g, 0.100 mmol) and sodium carbonate (0.212 g, 2.000 mmol) in dioxane (6 mL)/water (2 mL) was evacuated under vacuum and flushed with N₂ 3 times. The reaction was stirred at 110 °C overnight. The mixture was diluted with ethyl acetate and washed with saturated NaHCO₃, water, and brine. The organic phase was separated and dried over Na₂SO₄, filtered, and concentrated under reduced pressure. The product was purified by silica gel chromatography using CH₂Cl₂/EtOAc (1:1). LC-MS calculated for C₂₆H₃₁ClN₅O₃ (M+H)⁺: m/z = 496.2; found 496.1.

### Step 2: tert-butyl 2-(3'-(7-bromopyrido[3,2-d]pyrimidin-4-ylamino)-2-chloro-2'-methylbiphenyl-3-ylcarbamoyl)-1-methyl-6,7-dihydro-1H-imidazo[4,5-c]pyridine-5(4H)-carboxylate

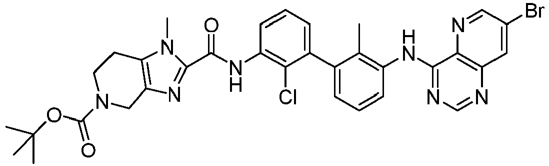

7-Bromo-4-chloropyrido[3,2-*d*]pyrimidine (Synthonix, cat#B0473: 0.217 g, 0.887 mmol) was added to a mixture of *tert*-butyl 2-((3'-amino-2-chloro-2'-methyl-[1,1'-biphenyl]-3-yl)carbamoyl)-1-methyl-1,4,6,7-tetrahydro-5*H*-imidazo[4,5-*c*]pyridine-5-carboxylate (0.40 g, 0.806 mmol) and triethylamine (0.225 mL, 1.613 mmol) in 2-propanol (5.0 mL) at rt. The reaction was stirred at 100 °C for 2 h. Diethyl ether (5.0 mL) was added to the reaction mixture and the resulting precipitate was filtered and dried to provide the crude product which was used directly in the next step without further purification. LC-MS calculated for C₃₃H₃₃BrClN₈O₃ (M+H)⁺: m/z = 703.2; found 703.3.

### Step 3: tert-butyl 2-(2-chloro-2'-methyl-3'-(7-vinylpyrido[3,2-d]pyrimidin-4-ylamino)biphenyl-3-ylcarbamoyl)-1-methyl-6,7-dihydro-1H-imidazo[4,5-c]pyridine-5(4H)-carboxylate

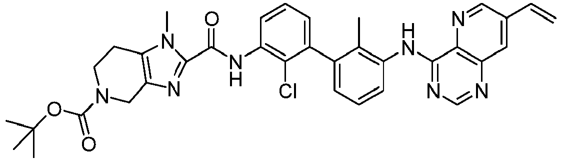

A mixture of *tert*-butyl 2-((3'-((7-bromopyrido[3,2-*d*]pyrimidin-4-yl)amino)-2-chloro-2'-methyl-[1,1'-biphenyl]-3-yl)carbamoyl)-1-methyl-1,4,6,7-tetrahydro-5*H-*imidazo[4,5-c]pyridine-5-carboxylate (0.35 g, 0.497 mmol), 4,4,5,5-tetramethyl-2-vinyl-1,3,2-dioxaborolane (Aldrich, cat#663348: 0.115 g, 0.746 mmol), dichloro[1,1'-bis(diphenylphosphino)ferrocene]-palladium (II) dichloromethane adduct (0.041 g, 0.050 mmol) and sodium carbonate (0.105 g, 0.994 mmol) in dioxane (6 mL)/water (2 mL) was evacuated under vacuum and flushed with N₂ 3 times. The reaction was stirred at 110 °C for 2 h. The mixture was diluted with ethyl acetate and washed with saturated NaHCO₃, water, and brine. The organic phase was separated, dried over Na₂SO₄, and concentrated under reduced pressure. The product was purified by column chromatography using CH₂Cl₂/EtOAc (7:3). LC-MS calculated for C₃₅H₃₆ClN₈O₃ (M+H)⁺: m/z = 651.3; found 651.2.

### Step 4: tert-butyl 2-(2-chloro-3'-(7-formylpyrido[3,2-d]pyrimidin-4-ylamino)-2'-methylbiphenyl-3-ylcarbamoyl)-1-methyl-6,7-dihydro-1H-imidazo[4,5-c]pyridine-5(4H)-carboxylate

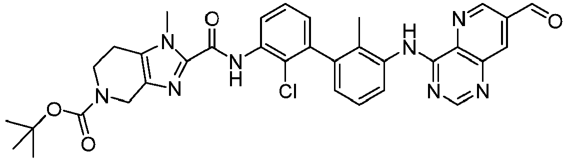

A vial was charged with *tert*-butyl 2-((2-chloro-2'-methyl-3'-((7-vinylpyrido[3,2-*d*]pyrimidin-4-yl)amino)-[1,1'-biphenyl]-3-yl)carbamoyl)-1-methyl-1,4,6,7-tetrahydro-5*H-*imidazo[4,5-*c*]pyridine-5-carboxylate (10.0 mg, 0.015 mmol), a stir bar, THF (2.0 mL) and water (0.8 mL). To this suspension was added a 4% w/w mixture of osmium tetroxide in water (12.0 µL, 1.529 µmol). The reaction was stirred for 5 min then sodium periodate (16.42 mg, 0.077 mmol) was added. After stirring at rt for 1 h, the reaction was quenched with a saturated aqueous solution of sodium thiosulfate. The mixture was then extracted with ethyl acetate (2 X 10 mL), and the combined organic layers were washed with brine, dried over Na₂SO₄, filtered, and concentrated in vacuo. The crude residue was used directly in the next step. LC-MS calculated for C₃₄H₃₄ClN₈O₄ (M+H)⁺: m/z = 653.2; found 653.2.

### Step 5: (R)-1-((4-(2'-chloro-2-methyl-3'-(1-methyl-4,5,6,7-tetrahydro-1H-imidazo[4,5-c]pyridine-2-carboxamido)biphenyl-3-ylamino)pyrido[3,2-d]pyrimidin-7-yl)methyl)-3-methylpyrrolidine-3-carboxylic acid

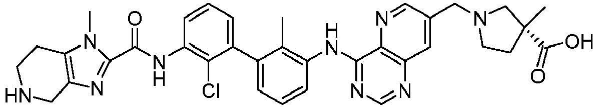

(*R*)-3-methylpyrrolidine-3-carboxylic acid (J&W PharmLab, cat#75R0495: 0.015 g, 0.115 mmol) was added to a suspension of *tert*-butyl 2-((2-chloro-3'-((7-formylpyrido[3,2-*d*]pyrimidin-4-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate (0.025 g, 0.038 mmol) in CH₂Cl₂ (1.0 mL). Triethylamine (0.043 mL, 0.306 mmol) was added and the mixture was stirred at rt for 1 h. At this time sodium triacetoxyborohydride (0.024 g, 0.115 mmol) was added and then stirred at rt for 2 h. The reaction was quenched with water, extracted with CH₂Cl₂/iPrOH, and the layers were separated. The organic phase was dried over Na₂SO₄, filtered, and concentrated under reduced pressure. The resulting crude residue was redissoved in CH₂Cl₂ (0.2 mL) and then TFA (0.5 mL) was added and the reaction was stirred at rt for 30 min. The solvent was removed and the crude product was used directly in the next step. LC-MS calculated for C₃₅H₃₇ClN₉O₃ (M+H)⁺: m/z = 666.3; found 666.5.

### Step 6: (R)-1-((4-(2'-chloro-3'-(1,5-dimethyl-4,5,6,7-tetrahydro-1H-imidazo[4,5-c]pyridine-2-carboxamido)-2-methylbiphenyl-3-ylamino)pyrido[3,2-d]pyrimidin-7-yl)methyl)-3-methylpyrrolidine-3-carboxylic acid

Formaldehyde (4.5 mg, 0.15 mmol) was added to a mixture of (*R*)-1-((4-(2'-chloro-2-methyl-3'-(1-methyl-4,5,6,7-tetrahydro-1*H*-imidazo[4,5-*c*]pyridine-2-carboxamido)biphenyl-3-ylamino)pyrido[3,2-d]pyrimidin-7-yl)methyl)-3-methylpyrrolidine-3-carboxylic acid (20. mg, 0.03 mmol) in CH₂Cl₂ (1.0 mL) followed by the addition of triethylamine (0.021 mL, 0.15 mmol). The mixture was stirred at rt for 10 min. At this time sodium triacetoxyborohydride (19 mg, 0.09 mmol) was added and then stirred at rt for 30 min. The mixture was diluted with acetonitrile/water, acidified to pH = 2 and purified by prep HPLC (pH = 2, acetonitrile/water+TFA) to provide the desired compound as its TFA salt. LC-MS calculated for C₃₆H₃₉ClN₉O₃ (M+H)⁺: m/z = 680.3; found 680.4.

### Example 68

### (R)-4-(2-(2-chloro-3'-(7-((3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1H-imidazo[4,5-c]pyridin-5(4H)-yl)-1-methylcyclohexanecarboxylic acid

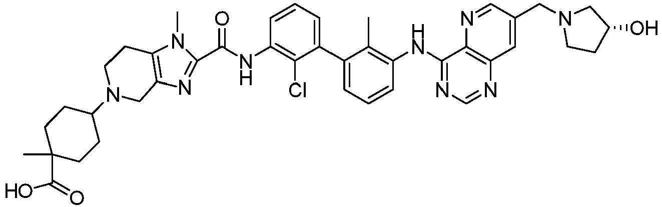

### Step 1: (R)-tert-butyl 2-(2-chloro-3'-(7-((3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1H-imidazo[4,5-c]pyridine-5(4H)-carboxylate

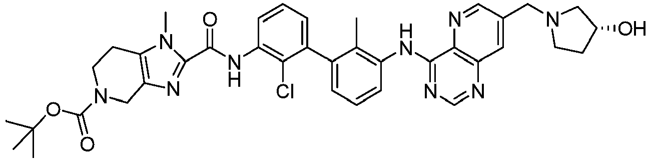

A mixture of *tert*-butyl 2-((2-chloro-3'-((7-formylpyrido[3,2-*d*]pyrimidin-4-yl)amino)-2'-methyl-[1,1'-biphenyl]-3-yl)carbamoyl)-1-methyl-1,4,6,7-tetrahydro-5*H-*imidazo[4,5-c]pyridine-5-carboxylate (*Example 67, Step 4*: 0.10 g, 0.153 mmol) and (*R*)-pyrrolidin-3-ol (Combi-Blocks, cat#AM-2005: 0.027 g, 0.306 mmol) in CH₂Cl₂ (8.0 mL) was stirred at rt for 10 min. Sodium triacetoxyborohydride (0.097 g, 0.459 mmol) was then added and the mixture was stirred at rt for 2 h. The mixture was diluted with CH₂Cl₂, washed with 1 N NaOH, water, and brine. The organic phase was dried over Na₂SO₄, filtered and concentrated. The product was purified by silica gel chromatography eluting with CH₂Cl₂/MeOH (9:1). LC-MS calculated for C₃₈H₄₃ClN₉O₄ (M+H)⁺: m/z = 724.3; found 724.5.

### Step 2: (R)-N-(2-chloro-3'-(7-((3-hydroxypyrrolidin-1-yl)methyl)pyrido[3,2-d]pyrimidin-4-ylamino)-2'-methylbiphenyl-3-yl)-1-methyl-4,5, 6, 7-tetrahydro-1H-imidazo[4,-5-c]pyridine-2-carboxamide

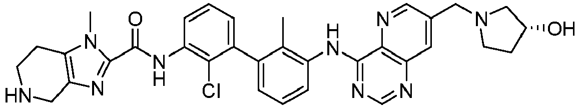

This compound was prepared using a similar procedure as described for *Example 47* with (R)-tert-butyl 2-(2-chloro-3'-(7-((3-hydroxypyrrolidin-1-yl)methyl)pyrido[3,2-*d*]pyrimidin-4-ylamino)-2'-methylbiphenyl-3-ylcarbamoyl)-1-methyl-6,7-dihydro-1*H-*imidazo[4,5-*c*]pyridine-5(4*H*)-carboxylate replacing *tert*-butyl (*R*)-2-((2-chloro-3'-((3-((3-hydroxypyrrolidin-1-yl)methyl)-1,7-naphthyridin-8-yl)amino)-2'-methyl-[1,1'-biphenyl]-3-yl)carbamoyl)-1-methyl-1,4,6,7-tetrahydro-5*H*-imidazo[4,5-*c*]pyridine-5-carboxylate in *Step* 4. LC-MS calculated for C₃₃H₃₅ClN₉O₂ (M+H)⁺: m/z = 624.3; found 624.2.

### Step 3: (R)-4-(2-(2-chloro-3'-(7-((3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1H-imidazo[4,5-c]pyridin-5(4H)-yl)-1-methylcyclohexanecarboxylic acid

This compound was prepared using a similar procedure as described for Example 62 with (*R*)-N-(2-chloro-3'-(7-((3-hydroxypyrrolidin-1-yl)methyl)pyrido[3,2-*d*]pyrimidin-4-ylamino)-2'-methylbiphenyl-3-yl)-1-methyl-4,5,6,7-tetrahydro-1*H*-imidazo[4,5-*c*]pyridine-2-carboxamide replacing (*R*)-*N*-(3'-((3-((3-hydroxypyrrolidin-1-yl)methyl)-1,7-naphthyridin-8-yl)amino)-2,2'-dimethyl-[1,1'-biphenyl]-3-yl)-1-methyl-4,5,6,7-tetrahydro-1*H*-imidazo[4,5-c]pyridine-2-carboxamide in *Step 3.*
**Peak 1:** retention time on analytical LCMS (pH=2, acetonitrile/water+TFA) *tᵣ* = 0.796 min; LC-MS calculated for C₄₁H₄₇ClN₉O₄ (M+H)⁺: m/z = 764.3; found 764.4.
**Peak 2:** retention time on analytical LCMS (pH=2, acetonitrile/water+TFA) *tᵣ* = 0.805 min; LC-MS calculated for C₄₁H₄₇ClN₉O₄ (M+H)⁺: m/z = 764.3; found 764.4.

### Example 69

### trans 4-((2-(2-chloro-3'-(7-(((R)-3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylic acid

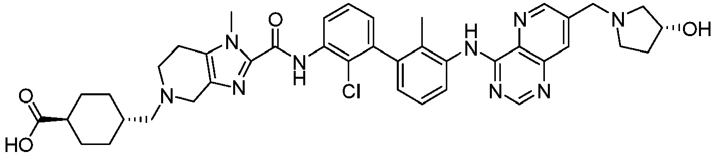

This compound was prepared using a similar procedure as described for *Example 47* with (*R*)-*N*-(2-chloro-3'-(7-((3-hydroxypyrrolidin-1-yl)methyl)pyrido[3,2-*d*]pyrimidin-4-ylamino)-2'-methylbiphenyl-3-yl)-1-methyl-4,5,6,7-tetrahydro-1*H*-imidazo[4,5-*c*]pyridine-2-carboxamide (*Example 68, Step* 2) replacing (*R*)-*N*-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-4,5,6,7-tetrahydro-1*H*-imidazo[4,5-*c*]pyridine-2-carboxamide in *Step 5.* LC-MS calculated for C₄₁H₄₇ClN₉O₄ (M+H)⁺: m/z = 764.3; found 764.5.

### Example 70

### (R)-1-((5-(3'-(3-(((R)-3-hydroxypyrrolidin-1-yl)methyl)-1,7-naphthyridin-8-ylamino)-2,2'-dimethylbiphenyl-3-ylamino)pyrido[4,3-b]pyrazin-2-yl)methyl)pyrrolidine-3-carboxylic acid

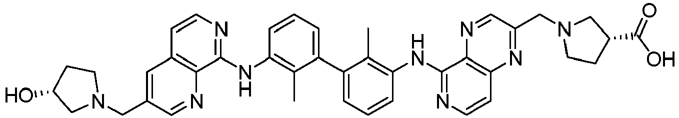

### Step 1: 5-(3-chloro-2-methylphenylamino)pyrido[4,3-b]pyrazin-2(1H)-one

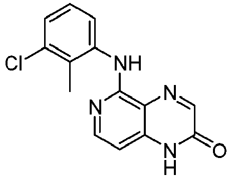

In a vial was combined 3-chloro-2-methylaniline (Aldrich, cat#101621: 351 mg, 2.478 mmol), 5-chloropyrido[3,4-*b*]pyrazin-2(1*H*)-one (Ark Pharm, cat#AK329687: 500mg, 2.75 mmol), isopropanol (5.0 mL), and sulfuric acid (0.147 mL, 2.75 mmol). The vial was sealed, then the reaction was heated to 100 °C for 1 hour. The mixture was cooled to rt, quenched with sat. NaHCO₃, diluted with ethyl acetate and the layers were separated. The aqueous layer was further extracted with ethyl acetate, and the combined organic layers were washed with brine, dried over MgSO₄, and filtered. The filtrate was concentrated in vacuo and the crude residue was purified by silica gel chromatography (50% EtOAc/hexanes) to provide the desired compound as a yellow oil. LC-MS calculated for C₁₄H₁₂ClN₄O (M+H)⁺: m/z = 287.1; found 287.1.

### Step 2: 2-bromo-N-(3-chloro-2-methylphenyl)pyrido[4,3-b]pyrazin-5-amine

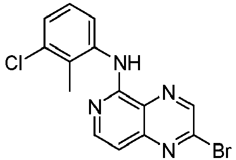

In a vial, a mixture of 5-(3-chloro-2-methylphenylamino)pyrido[4,3-*b*]pyrazin-2(1*H*)-one (200 mg, 0.698 mmol), phosphorus (V) oxybromide (1000 mg, 3.49 mmol), and MeCN (6.0 mL) was stirred at 80 °C for 4 hours. The mixture was cooled to rt, quenched with sat. NaHCO₃, diluted with ethyl acetate and the layers were separated. The aqueous layer was further extracted with ethyl acetate, and the combined organic layers were washed with brine, dried over MgSO₄, and filtered. The filtrate was concentrated in vacuo and the crude residue was purified by silica gel chromatography (20% EtOAc/hexanes) to provide the desired compound as a brown oil. LC-MS calculated for C₁₄H₁₁ClBrN₄ (M+H)⁺: m/z = 349.0; found 349.0.

### Step 3: N-(3-chloro-2-methylphenyl)-2-vinylpyrido[4,3-b]pyrazin-5-amine

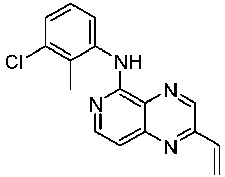

In a vial, a mixture of 2-bromo-*N*-(3-chloro-2-methylphenyl)pyrido[4,3-*b*]pyrazin-5-amine (30mg, 0.086 mmol), 4,4,5,5-tetramethyl-2-vinyl-1,3,2-dioxaborolane (Aldrich, cat#663348: 29.1 µL, 0.172 mmol), sodium carbonate (27.3 mg, 0.257 mmol), palladiumtetrakis (9.92 mg, 8.58 µmol) and 1,4-dioxane (2.0 mL) was stirred at 90 °C for 2 hours. The mixture was cooled to rt, diluted with ethyl acetate and washed with water and brine, dried over MgSO₄, and filtered. The filtrate was concentrated in vacuo and the crude residue was purified by silica gel chromatography (20% EtOAc/hexanes) to provide the desired compound as a brown oil. LC-MS calculated for C₁₆H₁₄ClN₄ (M+H)⁺: m/z = 297.1; found 297.1.

### Step 4: 5-(3-chloro-2-methylphenylamino)pyrido[4,3-b]pyrazine-2-carbaldehyde

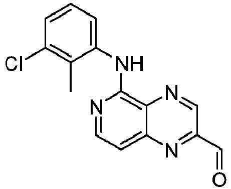

A 10 mL vial was charged with *N*-(3-chloro-2-methylphenyl)-2-vinylpyrido[4,3-*b*]pyrazin-5-amine (25.6 mg, 0.086 mmol) 1,4-dioxane (2 mL) and water (2 mL). A 4 % osmium tetroxide solution in water (38.2 µL, 6.01 µmol) was added to the reaction mixture. After 5 min, sodium periodate (147 mg, 0.686 mmol) was added. The reaction was stirred at rt for 2 hours before being quenched with sat. NaHCO₃. The resulting mixture was extracted with DCM, and the combined organic layers were washed with water and brine, dried over MgSO₄, and filtered. The filtrate was concentrated in vacuo and the crude residue was used directly in next step without further purification. LC-MS calculated for C₁₅H₁₂ClN₄O (M+H)⁺: m/z = 299.1; found 299.1.

### Step 5: (R)-1-((5-(3-chloro-2-methylphenylamino)pyrido[4,3-b]pyrazin-2-yl)methyl)pyrrolidine-3-carboxylic acid

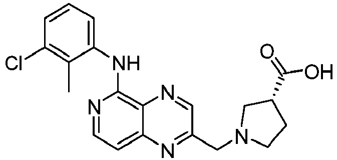

A 10 mL vial was charged with 5-(3-chloro-2-methylphenylamino)pyrido[4,3-*b*]pyrazine-2-carbaldehyde (10.0 mg, 0.033 mmol), (*R*)-pyrrolidine-3-carboxylic acid (Combi-Blocks, cat#ST-7698: 5.8 mg, 0.050 mmol) and DCM (1 mL). Triethylamine (9.3 µl, 0.067 mmol) and sodium triacetoxyborohydride (14.2 mg, 0.067 mmol) were added subsequently. The resulting reaction mixture was stirred at rt overnight before being quenched with sat. NaHCO₃. The resulting mixture was extracted with a 3:1 DCM/IPA mixture, and the combined organic layers were washed with water and brine, dried over MgSO₄, and filtered. The filtrate was concentrated in vacuo and the crude residue was used directly in next step without further purification. LC-MS calculated for C₂₀H₂₁ClN₅O₂ (M+H)⁺: m/z = 398.1; found 398.1.

### Step 6: (R)-1-((5-(3'-(3-(((R)-3-hydroxypyrrolidin-1-yl)methyl)-1,7-naphthyridin-8-ylamino)-2,2'-dimethylbiphenyl-3-ylamino)pyrido[4,3-b]pyrazin-2-yl)methyl)pyrrolidine-3-carboxylic acid

A mixture of (*R*)-1-((5-(3-chloro-2-methylphenylamino)pyrido[4,3-*b*]pyrazin-2-yl)methyl)pyrrolidine-3-carboxylic acid (10.0 mg, 0.025 mmol), (*R*)-1-((8-((2-methyl-3-(4,4,5,5-tetramethyl-1,3,2-dioxaborolan-2-yl)phenyl)amino)-1,7-naphthyridin-3-yl)methyl)pyrrolidin-3-ol (*Example 37, step 9:* 23.14 mg, 0.050 mmol), XPhos Pd G2 (2.0 mg, 2.51 µmol), and sodium carbonate (5.3 mg, 0.050 mmol) in 1,4-dioxane (1 mL) and water (0.2 mL) was degassed and sealed. It was stirred at 90 °C overnight. The reaction mixture was cooled then diluted with methanol, then purified with prep- LC-MS (pH = 2, acetonitrile/water+TFA) to give the desired product as its TFA salt. LC-MS calculated for C₄₀H₄₂N₉O₃ (M+H)⁺: m/z = 696.3; found 696.3.

### Example 71

### (3R)-1-((8-(2,2'-dimethyl-3'-(3-(pyrrolidin-2-yl)-1,7-naphthyridin-8-ylamino)biphenyl-3-ylamino)-1,7-naphthyridin-3-yl)methyl)pyrrolidine-3-carboxylic acid

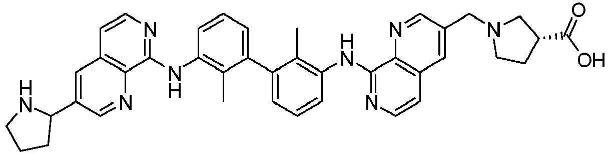

### Step 1: tert-butyl 4-(8-chloro-1,7-naphthyridin-3-yl)-4-oxobutylcarbamate

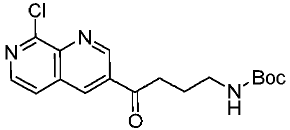

To a solution of 3-bromo-8-chloro-1,7-naphthyridine (PharmaBlock, cat#PBLJ2743: 100.2 mg, 0.411 mmol) in THF (10 mL) was added *n*-butyllithium (1.6 M, 0.26 mL, 0.411 mmol) dropwise at -78 °C. After stirring at this temperature for 1 hour, *tert*-butyl 2-oxopyrrolidine-1-carboxylate (0.14 mL, 0.821 mmol) was added. The reaction was further stirred at -78 °C for 2 hours. After completion, the reaction mixture was quenched by adding sat. NH₄Cl, which was then extracted with EtOAc. The combined organic layers were washed with water and brine, dried over MgSO₄, and filtered. The filtrate was concentrated in vacuo and the crude residue was purified by silica gel chromatography (80% EtOAc/hexanes) to provide the desired compound as a brown oil. LC-MS calculated for C₁₇H₂₁ClN₃O₃ (M+H)⁺: m/z = 350.1; found 350.1.

### Step 2: tert-butyl 2-(8-(3-bromo-2-methylphenylamino)-1,7-naphthyridin-3-yl)pyrrolidine-1-carboxylate

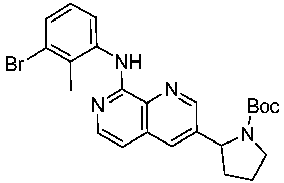

In a vial was combined 3-bromo-2-methylaniline (Aldrich, cat#530018: 51.1 mg, 0.274 mmol), *tert-butyl* (4-(8-chloro-1,7-naphthyridin-3-yl)-4-oxobutyl)carbamate (80.0 mg, 0.229 mmol), isopropanol (2.0 mL), and sulfuric acid (13.4 µl, 0.252 mmol). The vial was sealed, then the reaction was heated to 100 °C for 1 hour. The mixture was cooled to rt, quenched with solid NaHCO₃, diluted with ethyl acetate and filtered. The filtrate was concentrated in vacuo and the crude residue was dissolved in DCM (2.0 mL). Triethylamine (63.8 µl, 0.457 mmol) and sodium triacetoxyborohydride (72.7 mg, 0.343 mmol) were added to the above solution. The reaction was stirred at rt overnight before being quenched with sat. NaHCO₃. The resulting mixture was extracted with 3:1 DCM/IPA mixture, and the combined organic layers were washed with water and brine, dried over MgSO₄, and filtered. The filtrate was concentrated in vacuo and the crude residue was dissolved in dry DCM (4.0 mL) followed by addition of triethylamine (0.064 mL, 0.458 mmol) and Boc-anhydride (0.10 g, 0.458 mmol). The reaction mixture was stirred at rt for 2 hours before being quenched with sat. NaHCO₃. The resulting mixture was extracted with DCM, and the combined organic layers were washed with water and brine, dried over MgSO₄, and filtered. The filtrate was concentrated in vacuo and the residue was purified by silica gel chromatography (50% EtOAc/hexanes) to provide the desired compound as a yellow oil. LC-MS calculated for C₂₄H₂₈BrN₄O₂ (M+H)⁺: m/z = 483.1; found 483.1.

### Step 3: tert-butyl 2-(8-(3'-(3-(hydroxymethyl)-1,7-naphthyridin-8-ylamino)-2,2'-dimethylbiphenyl-3-ylamino)-1,7-naphthyridin-3-yl)pyrrolidine-1-carboxylate

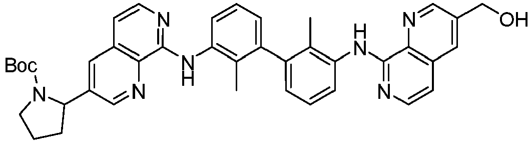

A mixture of *tert*-butyl 2-(8-(3-bromo-2-methylphenylamino)-1,7-naphthyridin-3-yl)pyrrolidine-1-carboxylate (200 mg, 0.414 mmol), (8-(2-methyl-3-(4,4,5,5-tetramethyl-1,3,2-dioxaborolan-2-yl)phenylamino)-1,7-naphthyridin-3-yl)methanol (*Example 20, step 3* 23.1 mg, 0.050 mmol), tetrakis(triphenylphosphine)palladium(0) (47.8 mg, 0.041 mmol), and sodium carbonate (88 mg, 0.827 mmol) in 1,4-dioxane (10 mL) and water (2 mL) was degassed and sealed. It was stirred at 100 °C overnight. The reaction mixture was cooled and then diluted with EtOAc. The organic layer was washed with water and brine, dried over MgSO₄, and filtered. The filtrate was concentrated in vacuo and the crude residue was purified by silica gel chromatography (90% EtOAc/hexanes) to provide the desired compound as a brown oil. LC-MS calculated for C₄₀H₄₂N₇O₃ (M+H)⁺: m/z = 668.3; found 668.3.

### Step 4: tert-butyl 2-(8-(3'-(3-formyl-1,7-naphthyridin-8-ylamino)-2,2'-dimethylbiphenyl-3-ylamino)-1,7-naphthyridin-3-yl)pyrrolidine-1-carboxylate

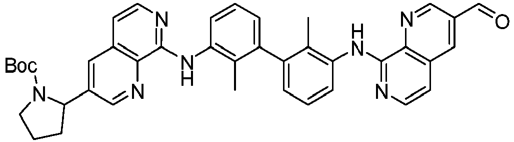

To a stirred solution of *tert*-butyl 2-(8-(3'-(3-(hydroxymethyl)-1,7-naphthyridin-8-ylamino)-2,2'-dimethylbiphenyl-3-ylamino)-1,7-naphthyridin-3-yl)pyrrolidine-1-carboxylate (276 mg, 0.414 mmol) in DCM (10.0 mL) was added manganese dioxide (719 mg, 8.27 mmol). The resulted mixture was stirred at 45 °C for 2 hours, then filtered. The filtrate was concentrated under reduced pressure. The residue was used in the next step directly without further purification. LC-MS calculated for C₄₀H₄₀N₇O₃ (M+H)⁺: m/z = 666.3; found 666.3.

### Step 5: (3R)-1-((8-(2,2'-dimethyl-3'-(3-(pyrrolidin-2-yl)-1,7-naphthyridin-8-ylamino)biphenyl-3-ylamino)-1,7-naphthyridin-3-yl)methyl)pyrrolidine-3-carboxylic acid

To a solution of *tert-butyl* 2-(8-(3'-(3-formyl-1,7-naphthyridin-8-ylamino)-2,2'-dimethylbiphenyl-3-ylamino)-1,7-naphthyridin-3-yl)pyrrolidine-1-carboxylate (20 mg, 0.030 mmol) in DCM (1 mL) was added (*R*)-pyrrolidine-3-carboxylic acid (Combi-Blocks, cat#ST-7698: 3.5 mg, 0.030 mmol) and triethylamine (8.4 µl, 0.060 mmol). The mixture was stirred at rt for 60 min, then sodium triacetoxyborohydride (9.6 mg, 0.045 mmol) was added. The resulting mixture was stirred at rt overnight before 1 mL of TFA was added. The reaction mixture was further stirred for 1 h. The reaction mixture was concentrated then purified with prep- LC-MS (pH 2, acetonitrile/water+TFA) to give the desired product as its TFA salt. LC-MS calculated for C₄₀H₄₁N₈O₂ (M+H)⁺: m/z = 665.3; found 665.3.

### Example 72

### (R)-1-((8-(2,2'-dichloro-3'-(3-((2-hydroxyethylamino)methyl)imidazo[1,2-a]pyrazin-8-ylamino)biphenyl-3-ylamino)-1,7-naphthyridin-3-yl)methyl)pyrrolidin-3-ol

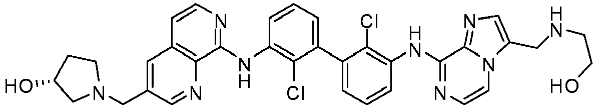

### Step 1: (R)-1-((8-(3'-amino-2,2'-dichlorobiphenyl-3ylamino)-1,7-naphthyridin-3-yl)methyl)pyrrolidin-3-ol

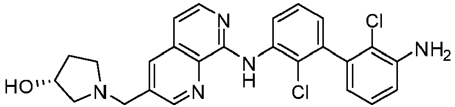

In a vial was combined 2-chloro-3-(4,4,5,5-tetramethyl-1,3,2-dioxaborolan-2-yl)aniline (*Example 5, step 1*: 0.474 g, 1.870 mmol), (*R*)-1-((8-((3-bromo-2-chlorophenyl)amino)-1,7-naphthyridin-3-yl)methyl)pyrrolidin-3-ol (*Example 22, step 4*: 0.676 g, 1.559 mmol), sodium carbonate (0.330 g, 3.12 mmol), (1,1'-bis(di-cyclohexylphosphino)ferrocene)-dichloropalladium(II) (Aldrich, cat#701998: 0.023 g, 0.031 mmol), 1,4-dioxane (2.92 mL) and water (0.974 mL). The mixture was degassed, sealed, and heated to 90 °C whilst stirring for 2 h. The mixture was cooled, diluted with EtOAc and filtered through celite. The filtrate was concentrated and purified using flash chromatography (0 → 15% MeOH/DCM). LC-MS calculated for C₂₅H₂₄Cl₂N₅O (M+H)⁺: m/z = 480.1; found 480.2.

### Step 2: (R)-1-((8-(3'-(3-bromoimidazo[1,2-a]pyrazin-8ylamino)-2,2'-dichlorobiphenyl-3-ylamino)-1,7-naphthyridin-3-yl)methyl)pyrrolidin-3-ol

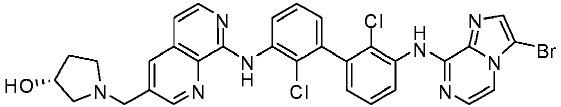

In a vial was combined (*R*)-1-((8-((3'-amino-2,2'-dichloro-[1,1'-biphenyl]-3-yl)amino)-1,7-naphthyridin-3-yl)methyl)pyrrolidin-3-ol (497 mg, 1.035 mmol) and 3-bromo-8-chloroimidazo[1,2-a]pyrazine (Combi-Blocks, cat# QA-2223: 361 mg, 1.552 mmol). The reactants were diluted with 2-propanol (5173 µl). To this was then added sulfuric acid (83 µl, 1.552 mmol) drop-wise. The reaction mixture was heated to 100 °C overnight. The solvent was removed under reduced pressure. The crude residue was taken back up in a 3:1 chloroform/IPA mixture and was neutralized with a saturated solution of sodium bicarbonate. The aqueous layer was extracted once more with 3:1 chloroform/IPA. The combined organic layers were washed with brine, dried over magnesium sulfate and filtered. The filtrate was concentrated and purified using flash chromatography (0 → 40% MeOH/DCM). LC-MS calculated for C₃₁H₂₆BrCl₂N₈O (M+H)⁺: m/z = 675.1; found 675.1.

### Step 3: (R)-1-((8-(2,2'-dichloro-3'-(3-vinylimidazo[1,2-a]pyrazin-8ylamino)biphenyl-3-ylamino)-1,7-naphthyridin-3-yl)methyl)pyrrolidin-3-ol

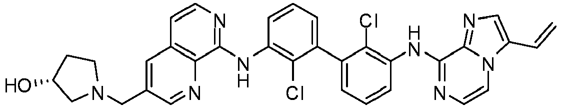

A mixture of (*R*)-1-((8-((3'-((3-bromoimidazo[1,2-a]pyrazin-8-yl)amino)-2,2'-dichloro-[1,1'-biphenyl]-3-yl)amino)-1,7-naphthyridin-3-yl)methyl)pyrrolidin-3-ol (458 mg, 0.677 mmol), 4,4,5,5-tetramethyl-2-vinyl-1,3,2-dioxaborolane (Sigma-Aldrich, cat#633348: 126 µl, 0.745 mmol), sodium carbonate (2.031 mmol) and [1,1'-bis(di-cyclohexylphosphino)ferrocene]dichloropalladium(II) (Aldrich, cat#701998: 25.7 mg, 0.034 mmol) in 1,4-dioxane (3224 µL) and water (1290 µL) was degassed and sealed. It was stirred at 90 °C for 1.5 h. The crude reaction mixture was diluted with water and extracted with ethyl acetate. The combined organic layers were washed with brine, dried over magnesium sulfate and filtered. The filtrate was concentrated under reduced pressure and used without further purification. LC-MS calculated for C₃₃H₂₉Cl₂N₈O (M+H)⁺: m/z = 623.2; found 623.4.

### Step 4: (R)-8-(2,2'-dichloro-3'-(3-((3-hydroxypyrrolidin-1-yl)methyl)-1,7-naphthyridin-8-ylamino)biphenyl-3-ylamino)imidazo[1,2-a]pyrazine-3-carbaldehyde

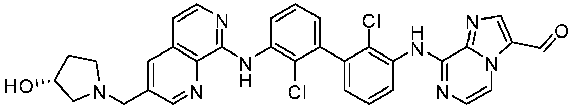

In a vial was combined (*R*)-1-((8-((2,2'-dichloro-3'-((3-vinylimidazo[1,2-a]pyrazin-8-yl)amino)-[1,1'-biphenyl]-3-yl)amino)-1,7-naphthyridin-3-yl)methyl)pyrrolidin-3-ol (200 mg, 0.321 mmol) and THF (2053 µl). The material was sonicated until it was fully in solution. To this, in order, was then added water (513 µl), 2,6-lutidine (191 µl, 1.636 mmol), sodium periodate (343 mg, 1.604 mmol) and potassium osmate dihydrate (17.73 mg, 0.048 mmol). The reaction was allowed to stir at rt for 30 min. The reaction was diluted with water and was extracted with ethyl acetate. The combined organic layers were washed with brine, dried over magnesium sulfate, and was filtered. The filtrate was concentrated and purified using flash chromatography (0 → 40% MeOH/DCM). LC-MS calculated for C₃₂H₂₇Cl₂N₈O₂ (M+H)⁺: m/z = 625.2; found 625.2.

### Step 5: (R)-1-((8-(2,2'-dichloro-3'-(3-((2-hydroxyethylamino)methyl)imidazo[1,2-a]pyrazin-8-ylamino)biphenyl-3-ylamino)-1,7-naphthyridin-3yl)methyl)pyrrolidin-3-ol

In a vial (*R*)-8-((2,2'-dichloro-3'-((3-((3-hydroxypyrrolidin-1-yl)methyl)-1,7-naphthyridin-8-yl)amino)-[1,1'-biphenyl]-3-yl)amino)imidazo[1,2-a]pyrazine-3-carbaldehyde (10 mg, 0.016 mmol) was combined with ethanolamine (9.67 µl, 0.160 mmol) and was diluted with methanol (160 µl). To this was then added acetic acid (13.73 µl, 0.240 mmol) followed by sodium cyanoborohydride (2.009 mg, 0.032 mmol) as a solution in methanol (160 µl). The reaction was allowed to stir at room temperature for 15 minutes after which time the reaction mixture was further diluted to a final volume of 5 mL with methanol and purified by prep HPLC (pH = 2, acetonitrile/water+TFA) to provide the desired compound as the TFA salt. LC-MS calculated for C₃₄H₃₄Cl₂N₉O₂ (M+H)⁺: m/z = 670.2; found 670.5.

### Example 73

### (R)-1-((8-((2,2'-dimethyl-3'-((3-(pyrrolidin-1-ylmethyl)-1,7-naphthyridin-8-yl)amino)-[1,1'-biphenyl]-3-yl)amino)-1,7-naphthyridin-3-yl)methyl)pyrrolidin-3-ol

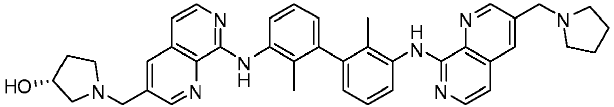

This compound was prepared using similar procedures as described for *Example 20* with pyrrolidine (Aldrich, cat#394238) replacing (*R*)-pyrrolidin-3-ol in *Step 6.* The reaction mixture was diluted with methanol and purified by prep HPLC (pH = 2, acetonitrile/water+TFA) to provide the desired compound as its TFA salt. LC-MS calculated for C₄₀H₄₃N₈O (M+H)⁺: m/z = 651.4; found 651.3.

### Example 74

### (S)-1-((8-((2'-chloro-3'-((3-(((R)-3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)pyrrolidin-3-ol

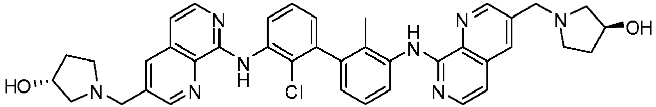

### Step 1: (R)-1-((8-((2-chloro-3'-((3-(hydroxymethyl)-1,7-naphthyridin-8-yl)amino)-2'-methyl-[1,1'-biphenyl]-3-yl)amino)-1,7-naphthyridin-3-yl)methyl)pyrrolidin-3-ol

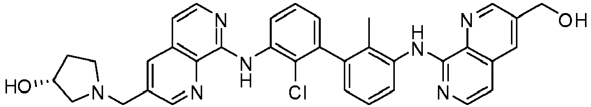

To a vial was added (8-((2-methyl-3-(4,4,5,5-tetramethyl-1,3,2-dioxaborolan-2-yl)phenyl)amino)-1,7-naphthyridin-3-yl)methanol (*Example 9, Step 3:* 0.166 g, 0.424 mmol), (*R*)-1-((8-((3-bromo-2-chlorophenyl)amino)-1,7-naphthyridin-3-yl)methyl)pyrrolidin-3-ol *(Example 22, Step 4*: 0.184g, 0.424 mmol), 1 M aqueous sodium carbonate (0.848 mmol), tetrakis (0.049 g, 0.042 mmol), and 1,4-dioxane (3.74 mL). The mixture was degassed, sealed, and heated to 110°C whilst stirring for 4 h. The mixture was cooled, diluted with EtOAc and filtered through celite. The filtrate was concentrated and purified by silica gel chromatography (15% MeOH/DCM) to provide the desired product as a yellow solid. LC-MS calculated for C₃₅H₃₃ClN₇O₂ (M+H)⁺: m/z = 618.2; found 618.3.

### Step 2: (R)-8-((2'-chloro-3'-((3-((3-hydroxypyrrolidin-1-yl)methyl)-1,7-naphthyridin-8-yl)amino)-2-methyl-[1,1'-biphenyl]-3-yl)amino)-1,7-naphthyridine-3-carbaldehyde

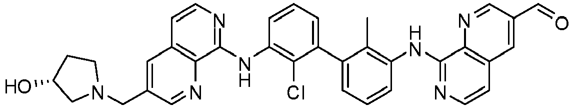

This compound was prepared using similar procedures as described for *Example 9* with (*R*)-1-((8-((2-chloro-3'-((3-(hydroxymethyl)-1,7-naphthyridin-8-yl)amino)-2'-methyl-[1,1'-biphenyl]-3-yl)amino)-1,7-naphthyridin-3-yl)methyl)pyrrolidin-3-ol replacing (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol in *Step 5.* LC-MS calculated for C₃₅H₃₁ClN₇O₂ (M+H)⁺: m/z = 616.2; found 616.3.

### Step 3: (S)-1-((8-((2'-chloro-3'-((3-(((R)-3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)pyrrolidin-3-ol

To a vial was added (*R*)-8-((2'-chloro-3'-((3-((3-hydroxypyrrolidin-1-yl)methyl)-1,7-naphthyridin-8-yl)amino)-2-methyl-[1,1'-biphenyl]-3-yl)amino)-1,7-naphthyridine-3-carbaldehyde (0.015 g, 0.024 mmol), (*S*)-pyrrolidin-3-ol (Combi-Blocks, cat#SS-7948: 6.4 mg, 0.073 mmol), a stir bar, and 1,2-dichloroethane (0.122 mL). The mixture was stirred for 5 min, then sodium triacetoxyborohydride (0.015 g, 0.073 mmol) and acetic acid (0.011 mL, 0.195 mmol) were added. The mixture was stirred for 1 h, then was diluted with methanol and purified by prep HPLC (pH = 2, acetonitrile/water+TFA) to provide the desired compound as its TFA salt. LC-MS calculated for C₃₉H₄₀ClN₈O₂ (M+H)⁺: m/z = 687.3; found 687.4.

### Example 75

### (R)-1-((8-((2'-chloro-3'-((3-((3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)azetidine-3-carboxylic acid

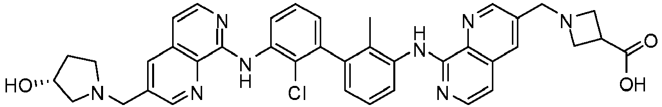

This compound was prepared using similar procedures as described for *Example 74* with azetidine-3-carboxylic acid (Aldrich, cat#391131) replacing (*S*)-pyrrolidin-3-ol in *Step* 3. The reaction mixture was diluted with methanol and purified by prep HPLC (pH = 2, acetonitrile/water+TFA) to provide the desired compound as its TFA salt. LC-MS calculated for C₃₉H₃₈ClN₈O₃ (M+H)⁺: m/z = 701.3; found 701.3.

### Example 76

### (R)-1-((8-((2,2'-dichloro-3'-((3-(((R)-3-hydroxypyrrolidin-1-yl)methyl)-1,7-naphthyridin-8-yl)amino)-[1,1'-biphenyl]-3-yl)amino)-1,7-naphthyridin-3-yl)methyl)-3-methylpyrrolidin-3-ol

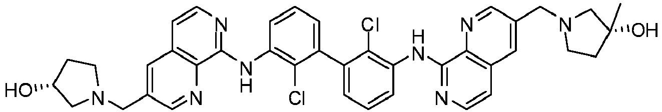

### Step 1: (8-((3-bromo-2-chlorophenyl)amino)-1,7-naphthyridin-3-yl)methanol

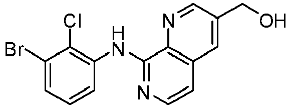

A flask was charged with 8-((3-bromo-2-chlorophenyl)amino)-1,7-naphthyridine-3-carbaldehyde (*Example 14, Step 6*: 0.586 g, 1.625 mmol), methanol (6.7 mL), and a stir bar. The mixture was cooled to 0 °C, and sodium borohydride (0.255 g, 6.74 mmol) was added portionwise over 1 h. After the final addition, the mixture was warmed to rt and stirred for 1 h. Another portion of sodium borohydride (0.050 g, 1.349 mmol) was added and stirred for 30 min. Saturated aqueous sodium bicarbonate was added (5 mL), and the mixture was diluted with DCM (10 mL). The layers were separated, and the aqueous layer was further extracted with DCM (2 X 10 mL). The combined organic extracts were dried over MgSO₄, filtered, and concentrated in vacuo. The crude product was purified by silica gel chromatography (0→ 46% EtOAc/hexanes). LC-MS calculated for C₁₅H₁₂BrClN₃O (M+H)⁺: m/z = 364.0; found 364.0.

### Step 2: (8-((2-chloro-3-(4,4,5,5-tetramethyl-1,3,2-dioxaborolan-2-yl)phenyl)amino)-1,7-naphthyridin-3-yl)methanol

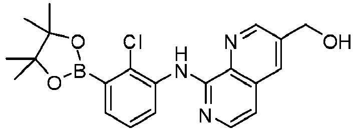

This compound was prepared using similar procedures as described for *Example 9* with (8-((3-bromo-2-chlorophenyl)amino)-1,7-naphthyridin-3-yl)methanol replacing (8-((3-bromo-2-methylphenyl)amino)-1,7-naphthyridin-3-yl)methanol in *Step 3.* LC-MS calculated for C₂₁H₂₄BClN₃O₃ (M+H)⁺: m/z = 412.2; found 412.2.

### Step 3: (R)-1-((8-((2,2'-dichloro-3'-((3-(hydroxymethyl)-1,7-naphthyridin-8-yl)amino)-[1,1'-biphenyl]-3-yl)amino)-1,7-naphthyridin-3-yl)methyl)pyrrolidin-3-ol

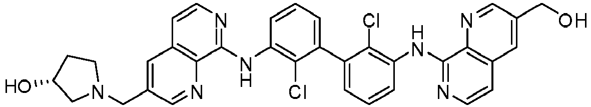

This compound was prepared using similar procedures as described for *Example 74* with (8-((2-chloro-3-(4,4,5,5-tetramethyl-1,3,2-dioxaborolan-2-yl)phenyl)amino)-1,7-naphthyridin-3-yl)methanol replacing (8-((2-methyl-3-(4,4,5,5-tetramethyl-1,3,2-dioxaborolan-2-yl)phenyl)amino)-1,7-naphthyridin-3-yl)methanol in *Step 1.* LC-MS calculated for C₃₄H₃₀Cl₂N₇O₂ (M+H)⁺: m/z = 638.2; found 638.2.

### Step 4: (R)-8-((2,2'-dichloro-3'-((3-((3-hydroxypyrrolidin-1-yl)methyl)-1,7-naphthyridin-8-yl)amino)-[1,1'-biphenyl]-3-yl)amino)-1,7-naphthyridine-3-carbaldehyde

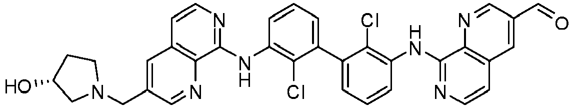

This compound was prepared using similar procedures as described for *Example 9* with (*R*)-1-((8-((2,2'-dichloro-3'-((3-(hydroxymethyl)-1,7-naphthyridin-8-yl)amino)-[1,1'-biphenyl]-3-yl)amino)-1,7-naphthyridin-3-yl)methyl)pyrrolidin-3-ol replacing (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol in *Step 5.* LC-MS calculated for C₃₄H₂₈Cl₂N₇O₂ (M+H)⁺: m/z = 636.2; found 636.2.

### Step 5: (R)-1-((8-((2,2'-dichloro-3'-((3-(((R)-3-hydroxypyrrolidin-1-yl)methyl)-1,7-naphthyridin-8-yl)amino)-[1,1'-biphenyl]-3-yl)amino)-1,7-naphthyridin-3yl)methyl)-3-methylpyrrolidin-3-ol

To a vial was added (*R*)-8-((2,2'-dichloro-3'-((3-((3-hydroxypyrrolidin-1-yl)methyl)-1,7-naphthyridin-8-yl)amino)-[1,1'-biphenyl]-3-yl)amino)-1,7-naphthyridine-3-carbaldehyde (.0150 g, 0.024 mmol), (*R*)-3-methylpyrrolidin-3-ol (Ark Pharm, cat#AK100499: 7.15 mg, 0.071 mmol), a stir bar, and 1,2-dichloroethane (0.236 mL). The mixture was stirred for 5 min, then sodium triacetoxyborohydride (0.015 g, 0.071 mmol) and acetic acid (4.05 µl, 0.071 mmol) were added. The reaction was stirred for 1 h, and the mixture was diluted with methanol and purified by prep HPLC (pH = 2, acetonitrile/water+TFA) to provide the desired compound as its TFA salt. LC-MS calculated for C₃₉H₃₉Cl₂N₈O₂ (M+H)⁺: m/z = 721.3; found 721.3.

### Example 77

### (R)-1-((8-((2,2'-dichloro-3'-((3-(((2-hydroxyethyl)amino)methyl)-1,7-naphthyridin-8-yl)amino)-[1,1'-biphenyl]-3-yl)amino)-1,7-naphthyridin-3-yl)methyl)pyrrolidin-3-ol

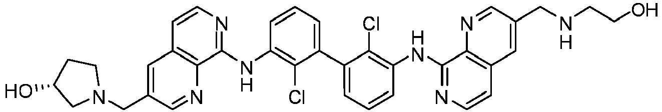

This compound was prepared using similar procedures as described for *Example 76* with ethanolamine (Aldrich, cat#411000) replacing (*R*)-3-methylpyrrolidin-3-ol in *Step 5.* LC-MS calculated for C₃₆H₃₅Cl₂N₈O₂ (M+H)⁺: m/z = 681.2; found 681.2.

### Example 78

### (R)-1-((8-((2,2'-dichloro-3'-((3-(((R)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid

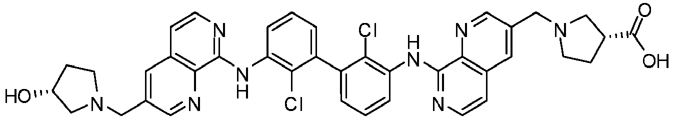

This compound was prepared using similar procedures as described for *Example 76* with (*R*)-pyrrolidine-3-carboxylic acid (Combi-Blocks, cat#ST-7698) replacing (R)-3-methylpyrrolidin-3-ol in *Step 5.* LC-MS calculated for C₃₉H₃₇Cl₂N₈O₃ (M+H)⁺: m/z = 735.2; found 735.2.

### Example 79

### (R)-1-((8-((2-chloro-3'-((3-(((R)-3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)pyrrolidine-3-carboxylic acid

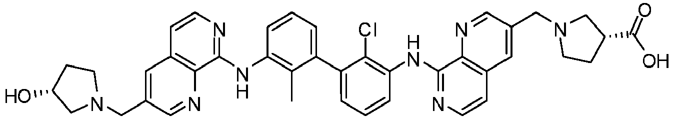

### Step 1: (R)-1-((8-((2'-chloro-2-methyl-3'-((3-vinyl-1,7-naphthyridin-8-yl)amino)-[1,1'-biphenyl]-3-yl)amino)-1,7-naphthyridin-3-yl)methyl)pyrrolidin-3-ol

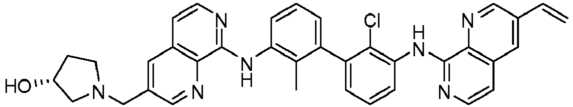

To a vial was added *N*-(3-bromo-2-chlorophenyl)-3-vinyl-1,7-naphthyridin-8-amine *(Example 14, Step 5:* 0.141 g, 0.391 mmol), (*R*)-1-((8-((2-methyl-3-(4,4,5,5-tetramethyl-1,3,2-dioxaborolan-2-yl)phenyl)amino)-1,7-naphthyridin-3-yl)methyl)pyrrolidin-3-ol *(Example 37, Step 9:* 0.150 g, 0.326 mmol), sodium carbonate (0.069 g, 0.652 mmol), tetrakis (0.038 g, 0.033 mmol), 1,4-dioxane (2.444 mL), and water (0.815 mL). The mixture was degassed, sealed, and heated to 110 °C whilst stirring for 4 h. The mixture was cooled, diluted with EtOAc and filtered through celite. The filtrate was concentrated and purified using flash chromatography (0→15% MeOH/DCM). LC-MS calculated for C₃₆H₃₃ClN₇O (M+H)⁺: m/z = 614.2; found 614.4.

### Step 2: (R)-8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridine-3-carbaldehyde

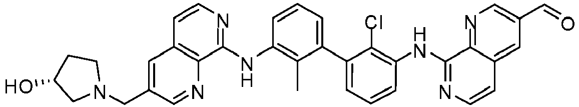

To a vial was added (*R*)-1-((8-((2'-chloro-2-methyl-3'-((3-vinyl-1,7-naphthyridin-8-yl)amino)-[1,1'-biphenyl]-3-yl)amino)-1,7-naphthyridin-3-yl)methyl)pyrrolidin-3-ol (0.108 g, 0.176 mmol), tetrahydrofuran (1.125 mL), water (0.281 mL), 2,6-lutidine (0.107 mL, 0.914 mmol), sodium periodate (0.188 g, 0.879 mmol), then potassium osmate dihydrate (9.72 mg, 0.026 mmol). The mixture was stirred for 30 min at rt. The mixture was diluted with 3:1 CHCl₃/IPA (5 mL) and water (2 mL), and the layers were separated. The aqueous layer was further extracted with 3:1 CHCl₃/IPA, dried over MgSO₄, filtered and concentrated under reduced pressure. The solid was slurried with 5:1 Et₂O/DCM, and filtered to provide the desired product as a beige solid. LC-MS calculated for C₃₅H₃₁ClN₇O₂ (M+H)⁺: m/z = 616.2; found 616.2.

### Step 3: (R)-1-((8-((2-chloro-3'-((3-(((R)-3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)pyrrolidine-3-carboxylic acid

To a vial was added (*R*)-8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridine-3-carbaldehyde (0.0350 g, 0.057 mmol), (*R*)-pyrrolidine-3-carboxylic acid (Combi-Blocks, cat#ST-7698: 0.020 g, 0.170 mmol), a stir bar, and 1,2-dichloroethane (0.568 mL). The mixture was stirred for 5 min, then sodium triacetoxyborohydride (0.036 g, 0.170 mmol) and acetic acid (0.020 mL, 0.341 mmol) were added. The reaction was stirred for 1 h, and the mixture was diluted with methanol and purified by prep HPLC (pH = 2, acetonitrile/water+TFA) to provide the desired compound as its TFA salt. LC-MS calculated for C₄₀H₄₀ClN₈O₃ (M+H)⁺: m/z = 715.3; found 715.3.

### Example 80

### (R)-1-((8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)azetidine-3-carboxylic acid

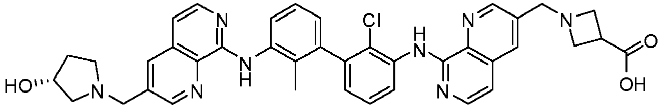

This compound was prepared using similar procedures as described for *Example 79* with azetidine-3-carboxylic acid (Aldrich, cat#391131) replacing (*R*)-pyrrolidine-3-carboxylic acid in *Step 3.* LC-MS calculated for C₃₉H₃₈ClN₈O₃ (M+H)⁺: m/z = 701.3; found 701.3.

### Example 81

### (R)-3-(((8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)amino)propanoic acid

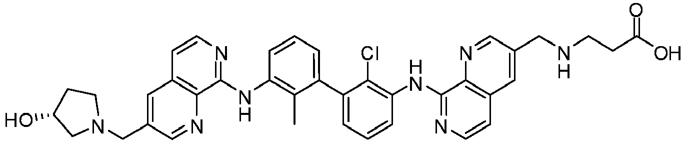

This compound was prepared using similar procedures as described for *Example 79* with β-alanine (Aldrich, cat#146064) replacing (*R*)-pyrrolidine-3-carboxylic acid in *Step 3.* LC-MS calculated for C₃₈H₃₈ClN₈O₃ (M+H)⁺: m/z = 689.3; found 689.3.

### Example 82

### (R)-1-((8-((2,2'-dichloro-3'-((3-(((S)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid

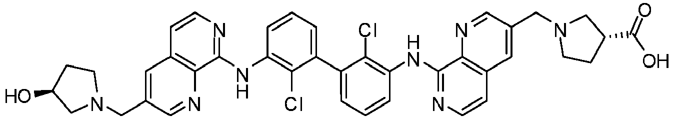

### Step 1: (S)-1-((8-((3-bromo-2-chlorophenyl)amino)-1,7-naphthyridin-3-yl)methyl)pyrrolidin-3-ol

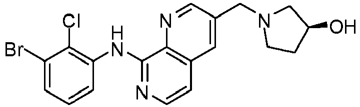

To a vial was added 8-((3-bromo-2-chlorophenyl)amino)-1,7-naphthyridine-3-carbaldehyde (*Example 14, Step 6:* 0.200 g, 0.552 mmol), (*S*)-3-hydroxypyrrolidine (Combi-Blocks, cat#SS-7948: 0.144 g, 1.655 mmol), DCE (2.76 mL), and a stir bar. The mixture was stirred at rt for 15 min, then sodium cyanoborohydride (0.104 g, 1.655 mmol) and acetic acid (0.120 mL, 2.096 mmol) were added. The mixture was stirred at rt for 1 h, then the reaction was quenched with aqueous saturated sodium bicarbonate (5 mL). 3:1 CHCl₃/IPA was added (5 mL), and the layers were separated. The aqueous layer was further extracted with 3:1 CHCl₃/IPA (2 × 5 mL), and the combined organic layers were dried over MgSO₄, filtered, and concentrated under reduced pressure. The resulting brown residue was purified by silica gel chromatography (0 → 15% MeOH/DCM) to provide the desired product as a brown solid. LC-MS calculated for C₁₉H₁₉BrClN₄O (M+H)⁺: m/z = 433.0; found 433.2.

### Step 2: (S)-1-((8-((2,2'-dichloro-3'-((3-(hydroxymethyl)-1,7-naphthyridin-8-yl)amino)-[1,1'-biphenyl]-3-yl)amino)-1,7-naphthyridin-3-yl)methyl)pyrrolidin-3-ol

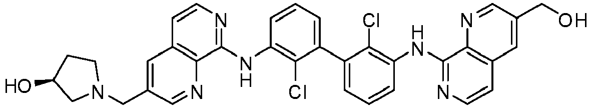

To a vial was added (8-((2-chloro-3-(4,4,5,5-tetramethyl-1,3,2-dioxaborolan-2-yl)phenyl)amino)-1,7-naphthyridin-3-yl)methanol (*Example 76, Step 2:* 0.163 g, 0.396 mmol), (*S*)-1-((8-((3-bromo-2-chlorophenyl)amino)-1,7-naphthyridin-3-yl)methyl)pyrrolidin-3-ol (0.172 g, 0.396 mmol), 1 M aqueous sodium carbonate (0.792 mmol), tetrakis (0.046 g, 0.040 mmol), and 1,4-dioxane (2.97 mL). The mixture was degassed, sealed, and heated to 110 °C whilst stirring for 4 h. The mixture was cooled, diluted with EtOAc and filtered through celite. The filtrate was concentrated and purified by silica gel chromatography (15% MeOH/DCM) to provide the desired product as a yellow solid. LC-MS calculated for C₃₄H₃₀Cl₂N₇O₂ (M+H)⁺: m/z = 638.2; found 638.2.

### Step 3: (S)-8-((2,2'-dichloro-3'-((3-((3-hydroxypyrrolidin-1-yl)methyl)-1,7-naphthyridin-8-yl)amino)-[1,1'-biphenyl]-3-yl)amino)-1,7-naphthyridine-3-carbaldehyde

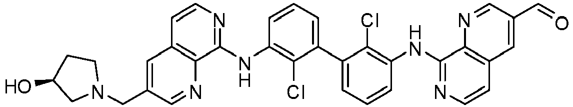

This compound was prepared using similar procedures as described for *Example 9* with (*S*)-1-((8-((2,2'-dichloro-3'-((3-(hydroxymethyl)-1,7-naphthyridin-8-yl)amino)-[1,1'-biphenyl]-3-yl)amino)-1,7-naphthyridin-3-yl)methyl)pyrrolidin-3-ol replacing (*R*)-1-((8-((3'-((3-(hydroxymethyl)-1,7-naphthyridin-8-yl)amino)-2,2'-dimethyl-[1,1'-biphenyl]-3-yl)amino)-1,7-naphthyridin-3-yl)methyl)pyrrolidin-3-ol in *Step 5.* LC-MS calculated for C₃₄H₂₈Cl₂N₇O₂ (M+H)⁺: m/z = 636.2; found 636.2.

### Step 4: (R)-1-((8-((2,2'-dichloro-3'-((3-(((S)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid

To a vial was added (*S*)-8-((2,2'-dichloro-3'-((3-((3-hydroxypyrrolidin-1-yl)methyl)-1,7-naphthyridin-8-yl)amino)-[1,1'-biphenyl]-3-yl)amino)-1,7-naphthyridine-3-carbaldehyde (0.009 g, 0.014 mmol), (*R*)-pyrrolidine-3-carboxylic acid (Combi-Blocks, cat#ST-7698: 4.88 mg, 0.042 mmol), a stir bar, *N*,*N*-dimethylformamide (0.141 mL), and DIPEA (7.41 µl, 0.042 mmol). The mixture was stirred for 5 min, then sodium cyanoborohydride (2.67 mg, 0.042 mmol) and was added. The reaction was stirred for 1 h, then the mixture was diluted with methanol and purified by prep HPLC (pH = 2, acetonitrile/water+TFA) to provide the desired compound as its TFA salt. LC-MS calculated for C₃₉H₃₇Cl₂N₈O₃ (M+H)⁺: m/z = 735.2; found 735.2.

### Example 83

### (S)-1-((8-((2,2'-dichloro-3'-((3-(((S)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid

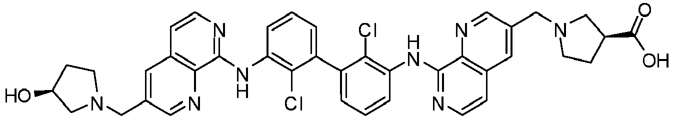

This compound was prepared using similar procedures as described for *Example 82* with (*S*)-pyrrolidine-3-carboxylic acid (Combi-Blocks, cat#ST-1381) replacing (*R*)-pyrrolidine-3-carboxylic acid in *Step 4.* LC-MS calculated for C₃₉H₃₇Cl₂N₈O₃ (M+H)⁺: m/z = 735.2; found 735.2.

### Example 84

### (R)-1-((8-((3'-(5-(dimethylglycyl)-5,6-dihydro-4H-pyrrolo[3,4-d]oxazol-2-yl)-2,2'-dimethyl-[1,1'-biphenyl]-3-yl)amino)-1,7-naphthyridin-3-yl)methyl)pyrrolidine-3-carboxylic acid

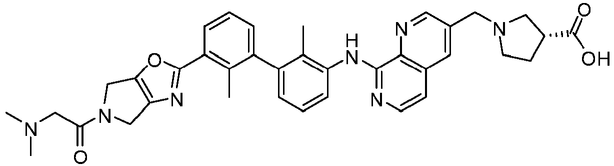

### Step 1: 2-(dimethylamino)-1-(2-(3'-((3-(hydroxymethyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one

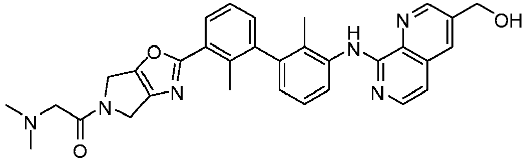

A mixture of 1-(2-(3-bromo-2-methylphenyl)-4,6-dihydro-5H-pyrrolo[3,4-*d*]oxazol-5-yl)-2-(dimethylamino)ethan-1-one (*Example 37, Step* 7: 112 mg, 0.307 mmol), (8-((2-methyl-3-(4,4,5,5-tetramethyl-1,3,2-dioxaborolan-2-yl)phenyl)amino)-1,7-naphthyridin-3-yl)methanol (*Example 9, Step 3:* 120 mg, 0.307 mmol), dicyclohexyl(2',4',6'-triisopropylbiphenyl-2-yl)phosphine-(2'-aminobiphenyl-2-yl)(chloro)palladium (1:1) (24.13 mg, 0.031 mmol) and tripotassium phosphate hydrate (155 mg, 0.675 mmol) in 1,4-dioxane (3mL)/water (1mL) was stirred at 80 °C for 1 h. The residue was dissolved in methanol and 1 N HCl and purified with prep-LCMS (pH 2, acetonitrile/water+TFA) to give the desired compound as light yellow solid. LC-MS calculated for C₃₂H₃₃N₆O₃ (M+H)⁺: m/z = 549.3; found 549.3.

### Step 2: 8-((3'-(5-(dimethylglycyl)-5,6-dihydro-4H-pyrrolo[3,4-d]oxazol-2-yl)-2,2'-dimethyl-[1,1'-biphenyl]-3-yl)amino)-1,7-naphthyridine-3-carbaldehyde

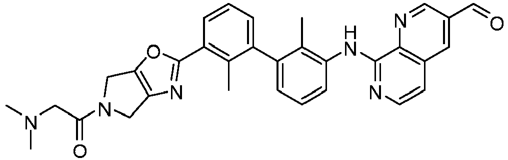

This compound was prepared using similar procedures as described for *Example 34,* with 2-(dimethylamino)-1-(2-(3'-((3-(hydroxymethyl)-1,7-naphthyridin-8-yl)amino)-2,2'-dimethyl-[1,1'-biphenyl]-3-yl)-4,6-dihydro-5*H*-pyrrolo[3,4-*d*]oxazol-5-yl)ethan-1-one replacing *tert*-butyl 2-(3'-((3-(hydroxymethyl)-1,7-naphthyridin-8-yl)amino)-2,2'-dimethyl-[1,1'-biphenyl]-3-yl)-6,7-dihydrothiazolo[5,4-*c*]pyridine-5(4*H*)-carboxylate in *Step 2.* LC-MS calculated for C₃₂H₃₁N₆O₃ (M+H)⁺: m/z = 547.2; found 547.3.

### Step 3. (R)-1-((8-((3'-(5-(dimethylglycyl)-5,6-dihydro-4H-pyrrolo[3,4-d]oxazol-2-yl)-2,2'-dimethyl-[1,1'-biphenyl]-3-yl)amino)-1,7-naphthyridin-3-yl)methyl)pyrrolidine-3-carboxylic acid

This compound was prepared using similar procedures as described for *Example 31* with 8-((3'-(5-(dimethylglycyl)-5,6-dihydro-4*H*-pyrrolo[3,4-*d*]oxazol-2-yl)-2,2'-dimethyl-[1,1'-biphenyl]-3-yl)amino)-1,7-naphthyridine-3-carbaldehyde replacing *N*-(2-chloro-3'-((3-formyl-1,7-naphthyridin-8-yl)amino)-2'-methyl-[1,1'-biphenyl]-3-yl)-1,5-dimethyl-4,5,6,7-tetrahydro-1*H*-imidazo[4,5-*c*]pyridine-2-carboxamide in *Step* 7. LC-MS calculated for C₃₇H₄₀N₇O₄ (M+H)⁺: m/z = 646.3; found 646.3.

### Example 85

### (R)-1-((8-((3'-(5-(dimethylglycyl)-5,6-dihydro-4H-pyrrolo[3,4-d]thiazol-2-yl)-2,2'-dimethyl-[1,1'-biphenyl]-3-yl)amino)-1,7-naphthyridin-3-yl)methyl)pyrrolidine-3-carboxylic acid

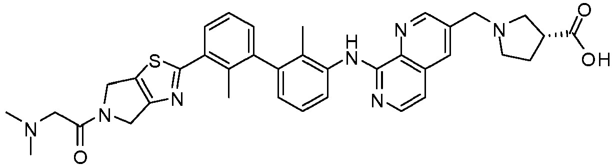

### Step1: tert-butyl 2-bromo-4H-pyrrolo[3,4-d]thiazole-5(6H)-carboxylate

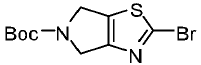

To a stirred solution of 2-bromo-5,6-dihydro-4*H*-pyrrolo[3,4-*d*]thiazole, HBr salt (Aurum Pharm, cat# *MR22320:* 220.0 mg, 0.769 mmol) and *N*,*N*-diisopropylethylamine (0.269 mL, 1.539 mmol) in DCM (5.0 mL), Boc-anhydride (201 mg, 0.923 mmol) was added at room temperature. After 1 hour, the reaction mixture was diluted with EtOAc (100 mL), and washed with water (3 × 15 mL). The organic layer was dried over Na₂SO₄, filtered and the filtrate was concentrated to afford crude *tert*-butyl 2-bromo-4*H*-pyrrolo[3,4-*d*]thiazole-5(6*H*)-carboxylate (220 mg, 0.724 mmol, 93.6 % yield), which was used directly in the next step without further purification. LC-MS calculated for C₁₀H₁₄BrN₂O₂S (M+H)⁺: m/z = 305.0/307.0; found 305.0/307.0.

### Step 2: tert-butyl 2-(3-chloro-2-methylphenyl)-4,6-dihydro-5H-pyrrolo[3,4-d]thiazole-5-carboxylate

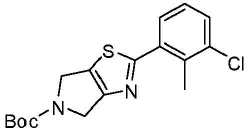

(3-Chloro-2-methylphenyl)boronic acid (344 mg, 2.02 mmol) (Combi-blocks, cat#BB-2035), tert-butyl 2-bromo-4,6-dihydro-5*H*-pyrrolo[3,4-*d*]thiazole-5-carboxylate (616 mg, 2.02 mmol), sodium carbonate (428 mg, 4.04 mmol) in 1,4-dioxane (8 mL) and water (2 mL) was added palladiumtetrakis (233 mg, 0.202 mmol). The resulting mixture was purged with N₂, then heated at 100°C. After 3 h, the reaction was concentrated, and diluted with DCM. The crude product was added to a silica gel column and was eluted with ethyl acetate/hexane from 0% to 40% to give *tert*-butyl 2-(3-chloro-2-methylphenyl)-4,6-dihydro-5*H*-pyrrolo[3,4-*d*]thiazole-5-carboxylate (541 mg, 76 % yield). LC-MS calculated for C₁₇H₂₀ClN₂O₂S (M+H)⁺: m/z = 351.1; found 351.0.

### Step 3: tert-butyl 2-(2-methyl-3-(4,4,5,-tetramethyl-1,3,2-dioxaborolan-2-yl)phenyl)-4,6-dihydro-5H-pyrrolo[3,4-d]thiazole-5-carboxylate

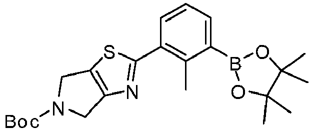

A mixture of *tert-*butyl 2-(3-chloro-2-methylphenyl)-4,6-dihydro-5*H*-pyrrolo[3,4-*d*]thiazole-5-carboxylate (261 mg, 0.715 mmol), 4,4,5,5,4',4',5',5'-octamethyl-[2,2']bi[[1,3,2]dioxaborolanyl] (Aldrich, cat#473294: 545 mg, 2.14 mmol), palladium acetate (6.42 mg, 0.0286 mmol), K₃PO₄ (455 mg, 2.14 mmol) and 2-(dicyclohexylphosphino)-2',6'-dimethoxy-1,1'-biphenyl (Strem Chemicals, cat#15-1143: 29.4 mg, 0.0715 mmol) in 1,4-dioxane was degassed and stirred at rt for 16 h. The mixture was diluted with DCM, and washed with water. The organic layer was concentrated in vacuo and purified by silica-gel chromatography (5% EtOAc/DCM). LC-MS calculated for C₂₃H₃₂BN₂O₄S (M+H)⁺: m/z = 443.2; found 443.3.

### Step 4: tert-butyl 2-(3'-((3-formyl-1,7-naphthyridin-8-yl)amino)-2,2'-dimethyl-[1,1'-biphenyl]-3-yl)-4,6-dihydro-5H-pyrrolo[3,4-d]thiazole-5-carboxylate

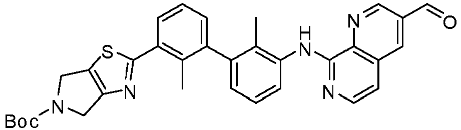

To a vial was added *tert*-butyl 2-(2-methyl-3-(4,4,5,5-tetramethyl-1,3,2-dioxaborolan-2-yl)phenyl)-4,6-dihydro-5*H*-pyrrolo[3,4-d]thiazole-5-carboxylate (0.013 g, 0.029 mmol), 8-((3-bromo-2-methylphenyl)amino)-1,7-naphthyridine-3-carbaldehyde (*Example 15, Step 2:* 7 mg, 0.020 mmol), sodium carbonate (6.24 mg, 0.059 mmol), [1,1'-bis(diphenylphosphino)ferrocene]dichloropalladium(II) (1.436 mg, 1.962 µmol), 1,4-dioxane (0.346 mL), and water (0.046 mL). The mixture was degassed, sealed, and heated to 90 °C whilst stirring for 4 h. After cooling, the mixture was diluted with DCM and water. The layers were separated and the aqueous layer was further extracted. The combined organic layers were dried over magnesium sulfate, filtered, concentrated in vacuo, and purified by silica gel chromatography (MeOH/DCM). LC-MS calculated for C₃₃H₃₂N₅O₃S (M+H)⁺: m/z = 578.2; found 578.4.

### Step 5: (R)-1-((8-((3'-(5-(tert-butoxycarbonyl)-5,6-dihydro-4H-pyrrolo[3, 4-d]thiazol-2-yl)-2,2'-dimethyl-[1,1'-biphenyl]-3-yl)amino)-1,7-naphthyridin-3-yl)methyl)pyrrolidine-3-carboxylic acid

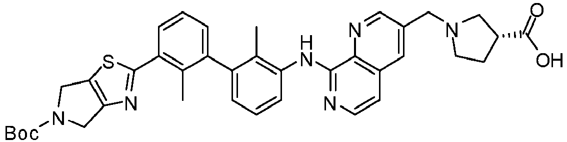

To a vial was added *tert*-butyl 2-(3'-((3-formyl-1,7-naphthyridin-8-yl)amino)-2,2'-dimethyl-[1,1'-biphenyl]-3-yl)-4,6-dihydro-5*H-*pyrrolo[3,4-*d*]thiazole-5-carboxylate (9 mg, 0.02 mmol), (*R*)-pyrrolidine-3-carboxylic acid (Combi-Blocks, cat#ST-7698: 0.017 g, 0.152 mmol), dichloromethane (0.829 mL) and triethylamine (0.016 mL, 0.115 mmol). The reaction was stirred at rt for 2 h, then sodium triacetoxyborohydride (0.054 g, 0.253 mmol) and acetic acid (8.7 µl, 0.15 mmol) were added. The reaction was stirred at rt for 2 h, then quenched with a saturated aqueous solution of sodium bicarbonate. The mixture was then extracted with a 3:1 mixture of chloroform/isopropanol. The combined organic layers were dried over sodium sulfate, then concentrated in vacuo to provide the desired compound. LC-MS calculated for C₃₈H₄₁N₆O₄S (M+H)⁺: m/z = 677.3; found 677.2.

### Step 6: (R)-1-((8-((3'-(5,6-dihydro-4H-pyrrolo[3, 4-d]thiazol-2-yl)-2,2'-dimethyl-[1,1'-biphenyl]-3-yl)amino)-1,7-naphthyridin-3-yl)methyl)pyrrolidine-3-carboxylic acid

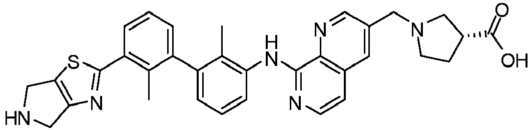

To a solution of (*R*)-1-((8-((3'-(5-(*tert*-butoxycarbonyl)-5,6-dihydro-4*H*-pyrrolo[3,4-*d*]thiazol-2-yl)-2,2'-dimethyl-[1,1'-biphenyl]-3-yl)amino)-1,7-naphthyridin-3-yl)methyl)pyrrolidine-3-carboxylic acid (7 mg, 0.02 mmol) in DCM (0.5 mL) was added TFA (0.2 mL). After 2 h, the reaction mixture was concentrated, and then the crude product was used directly in the next step. LC-MS calculated for C₃₃H₃₃N₆O₂S (M+H)⁺: m/z = 577.2; found 577.3.

### Step 7: (R)-1-((8-((3'-(5-(dimethylglycyl)-5,6-dihydro-4H-pyrrolo[3,4-d]thiazol-2-yl)-2,2'-dimethyl-[1,1'-biphenyl]-3-yl)amino)-1,7-naphthyridin-3-yl)methyl)pyrrolidine-3-carboxylic acid

In a 1 dram vial (*R*)-1-((8-((3'-(5,6-dihydro-4H-pyrrolo[3,4-*d*]thiazol-2-yl)-2,2'-dimethyl-[1,1'-biphenyl]-3-yl)amino)-1,7-naphthyridin-3-yl)methyl)pyrrolidine-3-carboxylic acid (6 mg, 0.02 mmol) and *N,N*-Dimethylglycine (6 mg, 0.06 mmol) were dissolved in DMF (0.2 mL). DIPEA (14 µl, 0.08 mmol) and HATU (18 mg, 0.05 mmol) were added to the reaction mixture in one portion. After 5 h, the reaction mixture was diluted with MeOH then purified by prep-HPLC (pH = 10, acetonitrile/water+NH₄OH) to give the desired product. LC-MS calculated for C₃₇H₄₀N₇O₃S (M+H)⁺: m/z = 662.3; found 662.2. ¹H NMR (600 MHz, DMSO-*d₆*) δ 9.30 (s, 1H), 8.85 (d, *J* = 2.0Hz, 1H), 8.43 (d, *J* = 8.1 Hz, 1H), 8.17 (d, *J* = 1.7 Hz, 1H), 8.05 (d, *J*= 5.8 Hz, 1H), 7.66 (d, *J* = 7.8 Hz, 1H), 7.42 (td, *J* = 7.6, 2.4 Hz, 1H), 7.32 (t, *J* = 7.8 Hz, 1H), 7.28 (d, *J* = 7.7 Hz, 1H), 7.17 (d, *J* = 5.8 Hz, 1H), 6.89 (d, *J* = 7.5 Hz, 1H), 5.06 - 4.96 (m, 1H), 4.88 (t, *J*= 2.8 Hz, 1H), 4.77 - 4.68 (m, 1H), 4.63 - 4.54 (m, 1H), 3.81 (q, *J*= 13.8 Hz, 2H), 3.16 (d, J= 1.9 Hz, 2H), 2.97 - 2.85 (m, 1H), 2.75 (t, *J* = 8.7 Hz, 1H), 2.66 (dd, *J* = 9.1, 6.5 Hz, 1H), 2.61 - 2.52 (m, 2H), 2.25 (s, 6H), 2.21 (s, 3H), 2.08 (s, 3H), 1.96 (q, *J* = 7.2 Hz, 2H).

### Example 86

### 2-((R)-3-hydroxypyrrolidin-1-yl)-1-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo [3,4-d]thiazol-5-yl)ethan-1-one

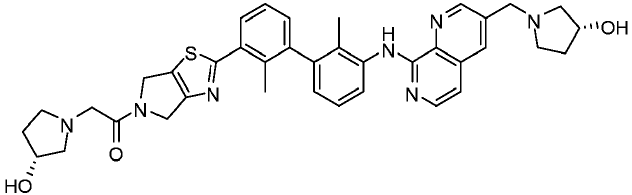

### Step 1: tert-butyl (R)-2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]thiazole-5-carboxylate

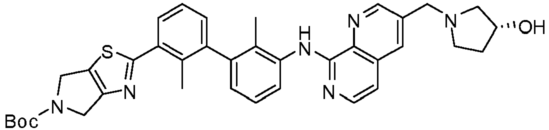

This compound was prepared using a similar procedure as described for *Example 85, Step* 4 with (*R*)-1-((8-((3-bromo-2-methylphenyl)amino)-1,7-naphthyridin-3-yl)methyl)pyrrolidin-3-ol (*Example 20, Step* 2) replacing 8-((3-bromo-2-methylphenyl)amino)-1,7-naphthyridine-3-carbaldehyde. The crude compound was diluted with DCM and water. The layers were separated and the aqueous layer was further extracted. The combined organic layers were dried over magnesium sulfate, filtered, and concentrated in vacuo. LC-MS calculated for C₃₇H₄₁N₆O₃S (M+H)⁺: m/z = 649.3; found 649.2.

### Step 2: (R)-1-((8-((3'-(5,6-dihydro-4H-pyrrolo[3,4-d]thiazol-2-yl)-2,2'-dimethyl-[1,1'-biphenyl]-3-yl)amino)-1,7-naphthyridin-3-yl)methyl)pyrrolidin-3-ol

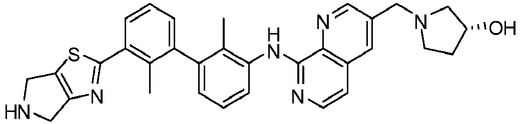

To a solution of *tert*-butyl (*R*)-2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5*H*-pyrrolo[3,4-*d*]thiazole-5-carboxylate (147 mg, 0.226 mmol) in DCM (3 mL) was added TFA (1 mL). After 2 h, the reaction mixture was concentrated, and then the crude product was used directly in the next step. LC-MS calculated for C₃₂H₃₃N₆OS (M+H)⁺: m/z = 549.2; found 549.3.

### Step 3: (R)-2-chloro-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]thiazol-5-yl)ethan-1-one

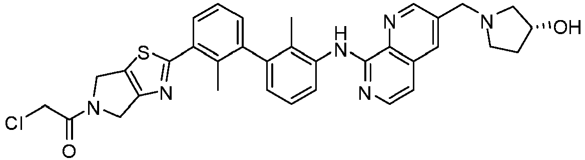

To a solution of the above crude product and DIPEA (118 uL, 0.678 mmol) in DCM (3 mL) was added chloroacetyl chloride (20 uL, 0.25 mmol) at -78°C. After 15 min, the reaction mixture was warmed to room temperature slowly. After 30 min, the reaction mixture was concentrated and diluted with MeOH then purified by prep-HPLC (pH = 2, acetonitrile/water+TFA) to give the desired product as the TFA salt. LC-MS calculated for C₃₄H₃₄ClN₆O₂S (M+H)+: m/z = 625.2; found 625.2.

### Step 4: 2-((R)-3-hydroxypyrrolidin-1-yl)-1-(2-(3'-((3-(((R)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]thiazol-5-yl)ethan-1-one

In a 1 dram vial (*R*)-2-chloro-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5*H*-pyrrolo[3,4-*d*]thiazol-5-yl)ethan-1-one (5 mg, 8.00 µmol) was dissolved in acetonitrile (400 µL) to give a yellow solution. (*R*)-pyrrolidin-3-ol (Combi-Blocks, cat#AM-2005: 5 mg) and DIPEA (1.5 µl, 8.0 µmol) were added to the reaction mixture. The reaction mixture was heated to 60 °C. After 12h, the reaction mixture was diluted with MeOH then purified by prep-HPLC (pH = 2, acetonitrile/water+TFA) to give the desired product as the TFA salt. LC-MS calculated for C₃₈H₄₂N₇O₃S (M+H)⁺: m/z = 676.3; found 676.3.

### Example 87

### (R)-1-((8-((3'-(5-(dimethylglycyl)-5,6-dihydro-4H-pyrrolo[3,4-d]thiazol-2-yl)-2,2'-dimethyl-[1,1'-biphenyl]-3-yl)amino)-1,7-naphthyridin-3-yl)methyl)-3-methylpyrrolidine-3-carboxylic acid

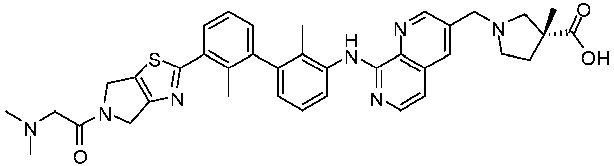

This compound was prepared using a similar procedure as described for *Example 85, Step* 5 with (*R*)-3-methylpyrrolidine-3-carboxylic acid (J&W PharmLab, cat#75R0495) replacing (*R*)-pyrrolidine-3-carboxylic acid. LC-MS calculated for C₃₈H₄₂N₇O₃S (M+H)⁺: m/z = 676.3; found 676.3.

### Example 88

### 1-((8-((3'-(5-(dimethylglycyl)-5,6-dihydro-4H-pyrrolo[3,4-d]thiazol-2-yl)-2,2'-dimethyl-[1,1'-biphenyl]-3-yl)amino)-1,7-naphthyridin-3-yl)methyl)azetidine-3-carboxylic acid

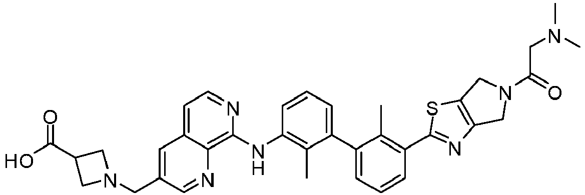

This compound was prepared using a similar procedure as described for *Example 85, Step* 5 with azetidine-3-carboxylic acid (Aldrich, cat#391131) replacing (*R*)-pyrrolidine-3-carboxylic acid. LC-MS calculated for C₃₆H₃₈N₇O₃S (M+H)⁺: m/z = 648.3; found 648.3.

### Example 89

### (R)-1-((8-((2-chloro-3'-(5-(dimethylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidine-3-carboxylic acid

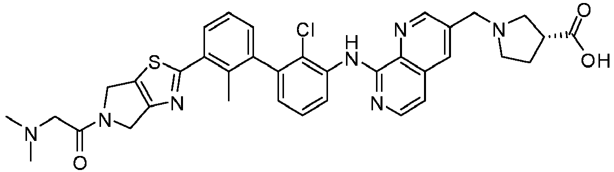

### Step 1: tert-butyl 2-(2'-chloro-3'-((3-formyl-1,7-naphthyridin-8-yl)amino)-2-methyl-[1,1'-biphenyl]-3-yl)-4,6-dihydro-5H-pyrrolo[3,4-d]thiazole-5-carboxylate

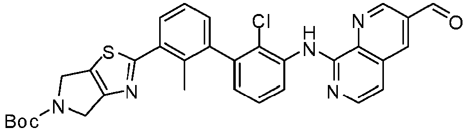

This compound was prepared using a similar procedure as described for *Example 85, Step 4* with 8-((3-bromo-2-chlorophenyl)amino)-1,7-naphthyridine-3-carbaldehyde (*Example 14, Step 6*) replacing 8-((3-bromo-2-methylphenyl)amino)-1,7-naphthyridine-3-carbaldehyde. The crude compound was diluted with DCM and water. The layers were separated and the aqueous layer was further extracted. The combined organic layers were dried over magnesium sulfate, filtered, and concentrated in vacuo. LC-MS calculated for C₃₂H₂₉ClN₅O₃S (M+H)⁺: m/z = 598.2; found 598.3.

### Step 2: (R)-1-((8-((2-chloro-3'-(5-(dimethylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidine-3-carboxylic acid

This compound was prepared using a similar procedure as described for *Example 85, Steps 5-7* with *tert-butyl* 2-(2'-chloro-3'-((3-formyl-1,7-naphthyridin-8-yl)amino)-2-methyl-[1,1'-biphenyl]-3-yl)-4,6-dihydro-5*H*-pyrrolo[3,4-*d*]thiazole-5-carboxylate replacing *tert-*butyl 2-(3'-((3-formyl-1,7-naphthyridin-8-yl)amino)-2,2'-dimethyl-[1,1'-biphenyl]-3-yl)-4,6-dihydro-5*H*-pyrrolo[3,4-*d*]thiazole-5-carboxylate. LC-MS calculated for C₃₆H₃₇ClN₇O₃S (M+H)⁺: m/z = 682.2; found 682.3.

### Example 90

### (R)-1-((8-((2-chloro-3'-(5-(N-ethyl-N-methylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidine-3-carboxylic acid

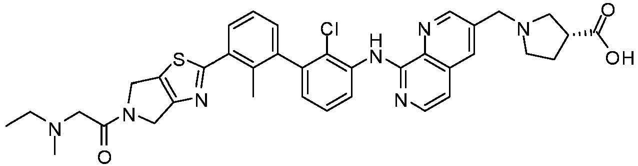

This compound was prepared using a similar procedure as described for *Example 85* with 8-((3-bromo-2-chlorophenyl)amino)-1,7-naphthyridine-3-carbaldehyde (*Example 14, Step 6*) replacing 8-((3-bromo-2-methylphenyl)amino)-1,7-naphthyridine-3-carbaldehyde in *Step* 4 and *N*-ethyl-*N*-methylglycine replacing *N*,*N*-dimethylglycine in *Step* 7. LC-MS calculated for C₃₇H₃₉ClN₇O₃S (M+H)⁺: m/z = 696.2; found 696.3.

### Example 91

### (R)-2-(1-((8-((2-chloro-3'-(5-(dimethylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidin-3-yl)acetic acid

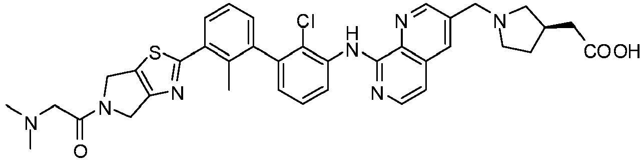

This compound was prepared using a similar procedure as described for *Example 85* with 8-((3-bromo-2-chlorophenyl)amino)-1,7-naphthyridine-3-carbaldehyde (*Example 14, Step* 6) replacing 8-((3-bromo-2-methylphenyl)amino)-1,7-naphthyridine-3-carbaldehyde in *Step* 4 and (*R*)-2-(pyrrolidin-3-yl)acetic acid (Combi-Blocks, cat#QE6116) replacing (*R*)-pyrrolidine-3-carboxylic acid in *Step 5.* LC-MS calculated for C₃₇H₃₉ClN₇O₃S (M+H)⁺: m/z = 696.2; found 696.3.

### Example 92

### 2-((8-((2-chloro-3'-(5-(dimethylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)-2-azabicyclo [2.2.1] heptane-5-carboxylic acid

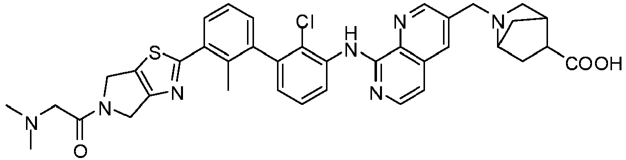

This compound was prepared using a similar procedure as described for *Example 85* with 8-((3-bromo-2-chlorophenyl)amino)-1,7-naphthyridine-3-carbaldehyde (*Example 14, Step* 6) replacing 8-((3-bromo-2-methylphenyl)amino)-1,7-naphthyridine-3-carbaldehyde in *Step* 4 and 2-azabicyclo[2.2.1]heptane-5-carboxylic acid (Aurora Fine Chemicals, cat#A30.309.242) replacing (*R*)-pyrrolidine-3-carboxylic acid in *Step 5.* LC-MS calculated for C₃₈H₃₉ClN₇O₃S (M+H)⁺: m/z = 708.2; found 708.3.

### Example 93

### (R)-2-(1-((8-(2-chloro-3'-(5-(2-(ethyl(methyl)amino)acetyl)-5,6-dihydro-4H-pyrrolo[3,4-d]thiazol-2-yl)-2'-methylbiphenyl-3-ylamino)-1,7-naphthyridin-3-yl)methyl)pyrrolidin-3-yl)acetic acid

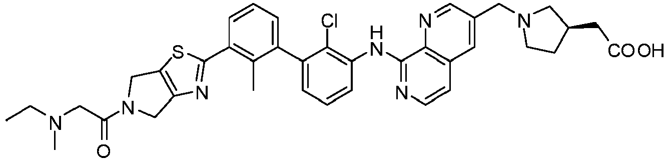

This compound was prepared using a similar procedure as described for *Example 85* with 8-((3-bromo-2-chlorophenyl)amino)-1,7-naphthyridine-3-carbaldehyde (*Example 14, Step 6*) replacing 8-((3-bromo-2-methylphenyl)amino)-1,7-naphthyridine-3-carbaldehyde in *Step 4,* (*R*)-2-(pyrrolidin-3-yl)acetic acid (Combi-Blocks, cat#QE6116) replacing (R)-pyrrolidine-3-carboxylic acid in *Step* 5 and *N*-ethyl-*N*-methylglycine replacing *N,N-*dimethylglycine in *Step 7*. LC-MS calculated for C₃₈H₄₁ClN₇O₃S (M+H)⁺: m/z = 710.3; found 710.3.

### Example 94

### 2-((8-(2-chloro-3'-(5-(2-(ethyl(methyl)amino)acetyl)-5,6-dihydro-4H-pyrrolo[3,4-d]thiazol-2-yl)-2'-methylbiphenyl-3-ylamino)-1,7-naphthyridin-3-yl)methyl)-2-azabicyclo [2.2.1] heptane-5-carboxylic acid

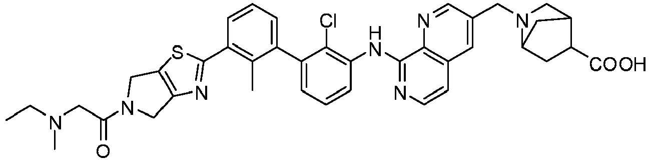

This compound was prepared using a similar procedure as described for *Example 85* with 8-((3-bromo-2-chlorophenyl)amino)-1,7-naphthyridine-3-carbaldehyde (*Example 14, Step 6*) replacing 8-((3-bromo-2-methylphenyl)amino)-1,7-naphthyridine-3-carbaldehyde in *Step 4,* 2-azabicyclo[2.2.1]heptane-5-carboxylic acid (Aurora Fine Chemicals, cat#A30.309.242) replacing (*R*)-pyrrolidine-3-carboxylic acid in *Step* 5 and *N*-ethyl-*N-*methylglycine replacing *N,N*-dimethylglycine in *Step 7*. LC-MS calculated for C₃₉H₄₁ClN₇O₃S (M+H)⁺: m/z = 722.3; found 722.3.

### Example 95

### (R)-1-((8-((2-chloro-3'-(5-(2-((R)-3-hydroxypyrrolidin-1-yl)acetyl)-5,6-dihydro-4H-pyrrolo[3,4-d]thiazol-2-yl)-2'-methyl-[1,1'-biphenyl]-3-yl)amino)-1,7-naphthytidin-3-yl)methyl)pyrrolidine-3-carboxylic acid

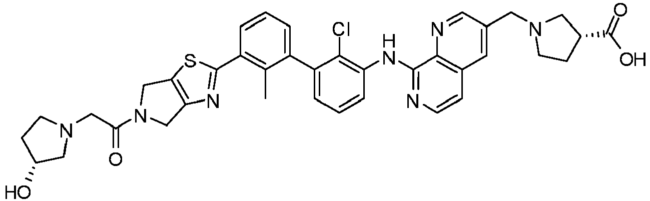

### Step 1: (R)-1-((8-((2-chloro-3'-(5-(2-chloroacetyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidine-3-carboxylic acid

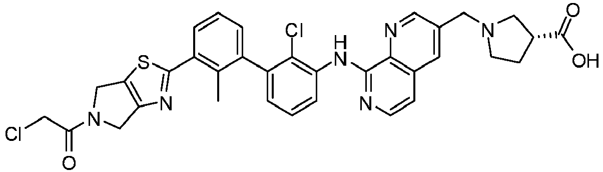

This compound was prepared using a similar procedure as described for *Example 90* with chloracetyl acid replacing *N*-ethyl-*N*-methylglycine. The reaction mixture was diluted with MeOH then purified by prep-HPLC (pH = 2, acetonitrile/water+TFA) to give the desired product as the TFA salt. LC-MS calculated for C₃₄H₃₁Cl₂N₆O₃S (M+H)⁺: m/z = 673.2; found 673.3.

### Step 2: (R)-1-((8-((2-chloro-3'-(5-(2-((R)-3-hydroxypyrrolidin-1-yl)acetyl)-5,6-dihydro-4H-pyrrolo[3, 4-d]thiazol-2-yl)-2'-methyl-[1,1'-biphenyl]-3-yl)amino)-1,7-naphthyridin-3-yl)methyl)pyrrolidine-3-carboxylic acid

In a 1 dram vial (*R*)-1-((8-((2-chloro-3'-(5-(2-chloroacetyl)-5,6-dihydro-4*H-*pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid (5 mg, 8.00 µmol) was dissolved in acetonitrile (400 µL) to give a yellow solution. (*R*)-pyrrolidin-3-ol (Combi-Blocks, cat#AM-2005: 5 mg) and DIPEA (1.5 µl, 8.0 µmol) were added to the reaction mixture. The reaction mixture was heated to 60 °C. After 12h, the reaction mixture was diluted with MeOH then purified by prep-HPLC (pH = 2, acetonitrile/water+TFA) to give the desired product as the TFA salt. LC-MS calculated for C₃₈H₃₉ClN₇O₄S (M+H)⁺: m/z = 724.3; found 724.2.

### Example 96

### (R)-1-((8-((2-chloro-3'-(5-(N-(2-hydroxyethyl)-N-methylglycyl)-5,6-dihydro-4H-pyrrolo[3,4-d]thiazol-2-yl)-2'-methyl-[1,1'-biphenyl]-3-yl)amino)-1,7-naphthytidin-3-yl)methyl)pyrrolidine-3-carboxylic acid

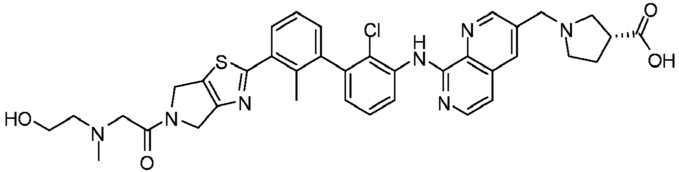

This compound was prepared using a similar procedure as described for *Example 95* with *N*-(2-hydroxyethyl)-*N*-methylamine replacing (*R*)-pyrrolidin-3-ol in *Step 2.* LC-MS calculated for C₃₇H₃₉ClN₇O₄S (M+H)⁺: m/z = 712.3; found 712.3.

### Example 97

### (R)-1-((8-((2-chloro-3'-(5-(2-((S)-3-hydroxypyrrolidin-1-yl)acetyl)-5,6-dihydro-4H-pyrrolo[3,4-d]thiazol-2-yl)-2'-methyl-[1,1'-biphenyl]-3-yl)amino)-1,7-naphthytidin-3-yl)methyl)pyrrolidine-3-carboxylic acid

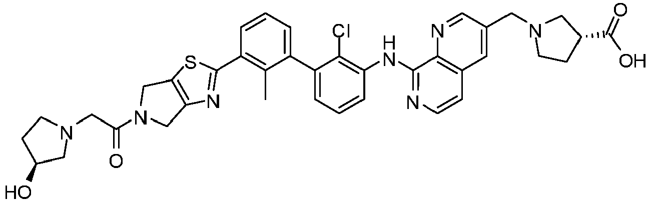

This compound was prepared using a similar procedure as described for *Example 95* with (*S*)-pyrrolidin-3-ol (Combi-Blocks, cat#SS-7948) replacing (*R*)-pyrrolidin-3-ol in *Step 2.* LC-MS calculated for C₃₈H₃₉ClN₇O₄S (M+H)⁺: m/z = 724.3; found 724.3.

### Example 98

### (R)-1-((8-((2-chloro-3'-(5-(2-(3-hydroxyazetidin-1-yl)acetyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidine-3-carboxylic acid

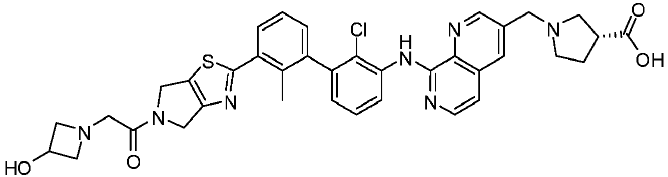

This compound was prepared using a similar procedure as described for *Example 95* with 3-hydroxyazetidine hydrochloride (Ark Pharm, cat#AK-25536) replacing (*R*)-pyrrolidin-3-ol in *Step 2.* LC-MS calculated for C₃₇H₃₇ClN₇O₄S (M+H)⁺: m/z = 710.3; found 710.3.

### Example 99

### Cis-4-((2-(3'-(3-(((R)-3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c] pyridin-5(4H)-yl)methyl)cyclohexanecarboxylic acid

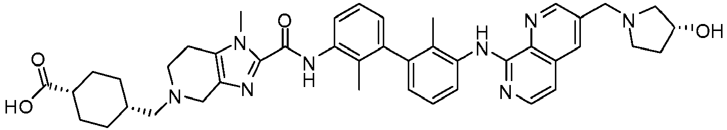

This compound was prepared using a similar procedure as described for *Example 48* with (R)-N-(3'-(3-((3-hydroxypyrrolidin-1-yl)methyl)-1,7-naphthyridin-8-ylamino)-2,2'-dimethylbiphenyl-3-yl)-1-methyl-4,5,6,7-tetrahydro-1H-imidazo[4,5-c]pyridine-2-carboxamide (Example 62, Step 2) replacing (*R*)-*N*-(2-chloro-3'-((3-((3-hydroxypyrrolidin-1-yl)methyl)-1,7-naphthyridin-8-yl)amino)-2'-methyl-[1,1'-biphenyl]-3-yl)-1-methyl-4,5,6,7-tetrahydro-1*H*-imidazo[4,5-*c*]pyridine-2-carboxamide. The reaction mixture was purified by prep HPLC (pH = 2, acetonitrile/water+TFA) to give the desired compound as its TFA salt. LC-MS calculated for C₄₃H₅₁N₈O₄ (M+H)⁺: m/z = 743.4; found 743.4.

### Example A. PD-1/PD-L1 Homogeneous Time-Resolved Fluorescence (HTRF) binding assay

The assays were conducted in a standard black 384-well polystyrene plate with a final volume of 20 µL. Inhibitors were first serially diluted in DMSO and then added to the plate wells before the addition of other reaction components. The final concentration of DMSO in the assay was 1%. The assays were carried out at 25° C in the PBS buffer (pH 7.4) with 0.05% Tween-20 and 0.1% BSA. Recombinant human PD-L1 protein (19-238) with a Histag at the C-terminus was purchased from AcroBiosystems (PD1-H5229). Recombinant human PD-1 protein (25-167) with Fc tag at the C-terminus was also purchased from AcroBiosystems (PD1-H5257). PD-L1 and PD-1 proteins were diluted in the assay buffer and 10 µL was added to the plate well. Plates were centrifuged and proteins were preincubated with inhibitors for 40 minutes. The incubation was followed by the addition of 10 µL of HTRF detection buffer supplemented with Europium cryptate-labeled anti-human IgG (PerkinElmer-AD0212) specific for Fc and anti-His antibody conjugated to SureLight^{®}-Allophycocyanin (APC, PerkinElmer-AD0059H). After centrifugation, the plate was incubated at 25° C for 60 min. before reading on a PHERAstar FS plate reader (665nm/620nm ratio). Final concentrations in the assay were - 3 nM PD1, 10 nM PD-L1, 1 nM europium anti-human IgG and 20 nM anti-His-Allophycocyanin. IC₅₀ determination was performed by fitting the curve of percent control activity versus the log of the inhibitor concentration using the GraphPad Prism 5.0 software.

Compounds of the present disclosure, as exemplified in the Examples, showed IC₅₀ values in the following ranges: + = IC₅₀ ≤ 10 nM; ++ = 10 nM < IC₅₀ ≤ 100 nM; +++ = 100 nM < IC₅₀ ≤ 1000 nM.

Data obtained for the Example compounds using the PD-1/PD-L1 homogenous time-resolved fluorescence (HTRF) binding assay described in Example A is provided in Table 1.

**Table 1**

| **Example** | **PD-1/PD-L1 HTRF IC₅₀ (nM)** |
|---|---|
| 1 | + |
| 2 | + |
| 3 | + |
| 4 | + |
| 5 | + |
| 6 | + |
| 7 | + |
| 8 | + |
| 9 | + |
| 10 | + |
| 11 | + |
| 12 | + |
| 13 | + |
| 14 | + |
| 15 | + |
| 16 | + |
| 17 | + |
| 18 | + |
| 19 | + |
| 20 | + |
| 21 | + |
| 22 | + |
| 23 | + |
| 24 | + |
| 25 | + |
| 26 | + |
| 27 | + |
| 28 | + |
| 29 | + |
| 30 | + |
| 31 | + |
| 32 | + |
| 33 | + |
| 34 | + |
| 35 | + |
| 36 | + |
| 37 | + |
| 38 | + |
| 39 | + |
| 40 | + |
| 41 | + |
| 42 | + |
| 43 | + |
| 44 | + |
| 45 | + |
| 46 | + |
| 47 | + |
| 48 | + |
| 49 | + |
| 50 | + |
| 51 | + |
| 52 | + |
| 53 | + |
| 54 | + |
| 55 | + |
| 56 | + |
| 57 | + |
| 58 | + |
| 59 | + |
| 60 | + |
| 61 | + |
| 62-peak1 | + |
| 62-peak2 | + |
| 63 | + |
| 64-peak1 | + |
| 64-peak2 | + |
| 65 | + |
| 66 | + |
| 67 | + |
| 68-peak1 | + |
| 68-peak2 | + |
| 69 | + |
| 70 | + |
| 71 | + |
| 72 | + |
| 73 | + |
| 74 | + |
| 75 | + |
| 76 | + |
| 77 | + |
| 78 | + |
| 79 | + |
| 80 | + |
| 81 | + |
| 82 | + |
| 83 | + |
| 84 | + |
| 85 | + |
| 86 | + |
| 87 | + |
| 88 | + |
| 89 | + |
| 90 | + |
| 91 | + |
| 92 | + |
| 93 | ++ |
| 94 | + |
| 95 | + |
| 96 | + |
| 97 | + |
| 98 | + |
| 99 | + |

## Claims

1. A compound of Formula (I): 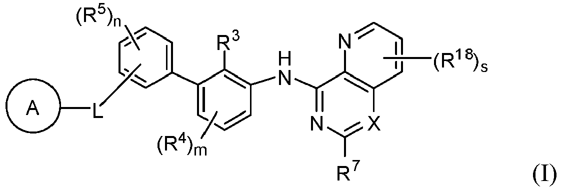 or a pharmaceutically acceptable salt thereof, wherein:
ring A is 5- to 10-membered heteroaryl, 4- to 11-membered heterocycloalkyl, C₆₋₁₀ aryl or C₃₋₁₀ cycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, 3, 4 or 5 R⁶ substituents;
L is a bond, -C(O)NR¹³-, -NR¹³C(O)-, O, -(CR¹⁴R¹⁵)_{q}-, -(CR¹⁴R¹⁵)_{q}-O- -O(CR¹⁴R¹⁵)_{q}-,-NR¹³-, -(CR¹⁴R¹⁵)_{q}-NR¹³-, -NR¹³-(CR¹⁴R¹⁵)_{q}-, -CH=CH-, -C≡C-, -SO₂NR¹³-, -NR¹³SO₂-,-NR¹³C(O)O- or -NR¹³C(O)NR¹³-;
X is N or CR¹⁷;
R³ is methyl, halo, CN or C₁₋₄ haloalkyl;
R⁴ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁵ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁶, R⁷, R¹⁷ and R¹⁸ are each independently selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-10 membered heterocycloalkyl)-C₁₋₄ alkyl-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, and S(O)₂NR^{a}R^{a}, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-10 membered heterocycloalkyl)-C₁₋₄ alkyl- of R⁶, R⁷, R¹⁷ and R¹⁸ are each optionally substituted with 1, 2, 3, 4 or 5 R^{b} substituents;
or two R⁶ substituents attached to the same ring carbon atom taken together with the ring carbon atom to which they are attached form spiro C₃₋₆ cycloalkyl or spiro 4- to 7-membered heterocycloalkyl, each of which is optionally substituted with 1, 2, or 3 independently selected R^{f} substituents;
each R¹³ is independently H, C₁₋₆ haloalkyl or C₁₋₆ alkyl optionally substituted with a substituent selected from C₁₋₄ alkyl, C₁₋₄ alkoxy, C₁₋₄ haloalkyl, C₁₋₄ haloalkoxy, CN, halo, OH,-COOH, NH₂, -NHC₁₋₄ alkyl and -N(C₁₋₄ alkyl)₂;
R¹⁴ and R¹⁵ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R¹⁴ or R¹⁵ are each optionally substituted with 1, 2, or 3 independently selected R^{q} substituents;
or R¹⁴ and R¹⁵ taken together with the carbon atom to which they are attached form 3-, 4-, 5- or 6-membered cycloalkyl or 3-, 4-, 5- or 6-membered heterocycloalkyl, each of which is optionally substituted with 1 or 2 R^{q} substituents;
each R^{a} is independently selected from H, CN, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{a} are each optionally substituted with 1, 2, 3, 4, or 5 R^{d} substituents;
each R^{d} is independently selected from C₁₋₆ alkyl, C₁₋₆ haloalkyl, halo, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, NH2, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}Re, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e}, and S(O)₂NR^{e}R^{e}, wherein the C₁₋₆ alkyl, C₁₋₆ haloalkyl, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{d} are each optionally substituted with 1, 2, or 3 independently selected R^{f} substituents;
each R^{e} is independently selected from H, C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{e} are each optionally substituted with 1, 2 or 3 independently selected R^{f} substituents;
each R^{b} substituent is independently selected from halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, OH, NH₂, NO₂, NHOR^{c}, OR^{c}, SR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} and S(O)₂NR^{c}R^{c}; wherein the C₁₋₆ alkyl, C₁₋₆ haloalkyl, C₁₋₆ haloalkoxy, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-and (4-10 membered heterocycloalkyl)-C₁₋₄ alkyl- of R^{b} are each further optionally substituted with 1, 2, or 3 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{c} are each optionally substituted with 1, 2, 3, 4, or 5 R^{f} substituents;
each R^{f} is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR⁹, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g}, and S(O)₂NR^{g}R^{g}; wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{f} are each optionally substituted with 1, 2, 3, 4, or 5 Rⁿ substituents;
each Rⁿ is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{o}, OR^{o}, SR^{o}, C(O)R^{o}, C(O)NR^{o}R^{o}, C(O)OR^{o}, OC(O)R^{o}, OC(O)NR^{o}R^{o}, NHR^{o}, NR^{o}R^{o}, NR^{o}C(O)R^{o}, NR^{o}C(O)NR^{o}R^{o}, NR^{o}C(O)OR^{o}, C(=NR^{o})NR^{o}R^{o}, NR^{o}C(=NR^{o})NR^{o}R^{o}, S(O)R^{o}, S(O)NR^{o}R^{o}, S(O)₂R^{o}, NR^{o}S(O)₂R^{o}, NR^{o}S(O)₂NR^{o}R^{o}, and S(O)₂NR^{o}R^{o}, wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of Rⁿ are each optionally substituted with 1, 2 or 3 independently selected R^{q} substituents;
each R^{g} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{g} are each optionally substituted with 1, 2, or 3 R^{p} substituents;
each R^{p} is independently selected from C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} and S(O)₂NR^{r}W^{r}, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{p} is optionally substituted with 1, 2 or 3 R^{q} substituents;
or any two R^{a} substituents together with the nitrogen atom to which they are attached form a 4-, 5-, 6-, 7-, 8-, 9- or 10-membered heterocycloalkyl group optionally substituted with 1, 2 or 3 R^{h} substituents;
each R^{h} is independently selected from C₁₋₆ alkyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl-, C₁₋₆ haloalkyl, C₁₋₆ haloalkoxy, C₂₋₆ alkenyl, C₂₋₆ alkynyl, halo, CN, ORⁱ, SRⁱ, NHORⁱ, C(O)Rⁱ, C(O)NRⁱRⁱ, C(O)ORⁱ OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ, and S(O)₂NRⁱRⁱ, wherein the C₁₋₆ alkyl, C₂₋₆ alkenyl, C₂₋₆ alkynyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl- of R^{h} are each further optionally substituted by 1, 2, or 3 R^{j} substituents;
each R^{j} is independently selected from C₃₋₆ cycloalkyl, C₆₋₁₀ aryl, 5 or 6-membered heteroaryl, 4-7 membered heterocycloalkyl, C₂₋₄ alkenyl, C₂₋₄ alkynyl, halo, C₁₋₄ alkyl, C₁₋₄ haloalkyl, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k}, and S(O)₂NR^{k}R^{k}, wherein the C₁₋₄ alkyl, C₃₋₆ cycloalkyl, C₆₋₁₀ aryl, 5- or 6-membered heteroaryl, 4-6 membered heterocycloalkyl, C₂₋₄ alkenyl, C₂₋₄ alkynyl, C₁₋₄ haloalkyl, and C₁₋₄ haloalkoxy of R^{j} are each optionally substituted with 1, 2 or 3 independently selected R^{q} substituents;
or two R^{h} groups attached to the same carbon atom of the 4- to 10-membered heterocycloalkyl taken together with the carbon atom to which they are attached form a C₃₋₆ cycloalkyl or 4- to 6-membered heterocycloalkyl having 1-2 heteroatoms as ring members selected from O, N or S;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
or any two R^{e} substituents together with the nitrogen atom to which they are attached form a 4-, 5-, 6-, or 7-membered heterocycloalkyl group optionally substituted with 1, 2, or 3 independently selected R^{h} substituents;
or any two R^{g} substituents together with the nitrogen atom to which they are attached form a 4-, 5-, 6-, or 7-membered heterocycloalkyl group optionally substituted with 1, 2, or 3 independently selected R^{h} substituents;
or any two R^{o} substituents together with the nitrogen atom to which they are attached form a 4-, 5-, 6-, or 7-membered heterocycloalkyl group optionally substituted with 1, 2, or 3 independently selected R^{h} substituents;
or any two R^{r} substituents together with the nitrogen atom to which they are attached form a 4-, 5-, 6-, or 7-membered heterocycloalkyl group optionally substituted with 1, 2, or 3 independently selected R^{h} substituents;
each Rⁱ, R^{k}, R^{o} or R^{r} is independently selected from H, C₁₋₄ alkyl, C₃₋₆ cycloalkyl, C₆₋₁₀ aryl, 5 or 6-membered heteroaryl, 4-7 membered heterocycloalkyl, C₁₋₆ haloalkyl, C₁₋₆ haloalkoxy, C₂₋₄ alkenyl, and C₂₋₄ alkynyl, wherein the C₁₋₄ alkyl, C₃₋₆ cycloalkyl, C₆₋₁₀ aryl, 5 or 6-membered heteroaryl, 4-7 membered heterocycloalkyl, C₂₋₄ alkenyl, and C₂₋₄ alkynyl of Rⁱ, R^{k}, R^{o} or R^{r} are each optionally substituted with 1, 2 or 3 R^{q} substituents;
each R^{q} is independently selected from halo, OH, CN, -COOH, NH₂, -NH-C₁₋₆ alkyl, - N(C₁₋₆ alkyl)₂, C₁₋₆ alkyl, C₁₋₆ alkoxy, C₁₋₆ alkylthio, C₁₋₆ haloalkyl, C₁₋₆ haloalkoxy, phenyl, 5-6 membered heteroaryl, 4-6 membered heterocycloalkyl and C₃₋₆ cycloalkyl, wherein the C₁₋₆ alkyl, phenyl, C₃₋₆ cycloalkyl, 4-6 membered heterocycloalkyl, and 5-6 membered heteroaryl of R^{q} are each optionally substituted with 1, 2, or 3 substituents selected from halo, OH, CN, -COOH, NH₂, C₁₋₄ alkyl, C₁₋₄ alkoxy, C₁₋₄ haloalkyl, C₁₋₄ haloalkoxy, phenyl, C₃₋₁₀ cycloalkyl, 5-6 membered heteroaryl and 4-6 membered heterocycloalkyl;
the subscript m is an integer of 0, 1, 2 or 3;
the subscript n is an integer of 0, 1, 2 or 3;
each subscript q is independently an integer of 1, 2, 3 or 4; and
the subscript s is an integer of 1, 2, or 3.

2. A compound of Formula (Ia): 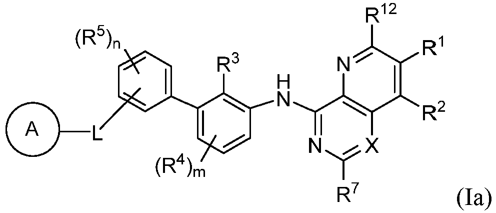 or a pharmaceutically acceptable salt thereof, wherein:
ring A is 5- to 10-membered heteroaryl, 4- to 11-membered heterocycloalkyl, C₆₋₁₀ aryl or C₃₋₁₀ cycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2, 3, 4 or 5 R⁶ substituents;
L is a bond, -C(O)NR¹³-, -NR¹³C(O)-, O, -(CR¹⁴R¹⁵)_{q}-, -(CR¹⁴R¹⁵)_{q}-O-, -O(CR¹⁴R¹⁵)_{q}-,-NR¹³-, -(CR¹⁴R¹⁵)_{q}-NR¹³-, -NR¹³-(CR¹⁴R¹⁵)_{q}-, -CH=CH-, -C≡C-, -SO₂NR¹³-, -NR¹³SO₂-,-NR¹³C(O)O- or -NR¹³C(O)NR¹³-;
X is N or CR¹⁷;
R³ is methyl, halo, CN or C₁₋₄ haloalkyl;
R⁴ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁵ is C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂;
R⁶ is selected from H, halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl-, (4-10 membered heterocycloalkyl)-C₁₋₄ alkyl-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a}, and S(O)₂NR^{a}R^{a}, wherein the C₁₋₆ alkyl, C₂₋₆ alkenyl, C₂₋₆ alkynyl, C₆₋₁₀ aryl, C₃₋₁₀ cycloalkyl, 5-14 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-14 membered heteroaryl)-C₁₋₄ alkyl- and (4-10 membered heterocycloalkyl)-C₁₋₄ alkyl- of R⁶ is optionally substituted with 1, 2, 3, 4 or 5 R^{b} substituents;
or two R⁶ substituents attached to the same ring carbon atom taken together with the ring carbon atom to which they are attached form spiro C₃₋₆ cycloalkyl or spiro 4- to 7-membered heterocycloalkyl, each of which is optionally substituted with 1, 2, or 3 independently selected R^{f} substituents;
each R¹³ is independently H, C₁₋₆ haloalkyl or C₁₋₆ alkyl optionally substituted with a substituent selected from C₁₋₄ alkyl, C₁₋₄ alkoxy, C₁₋₄ haloalkyl, C₁₋₄ haloalkoxy, CN, halo, OH,-COOH, NH₂, -NHC₁₋₄ alkyl and -N(C₁₋₄ alkyl)₂;
R¹⁴ and R¹⁵ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R¹⁴ or R¹⁵ are each optionally substituted with 1, 2, or 3 independently selected R^{q} substituents;
or R¹⁴ and R¹⁵ taken together with the carbon atom to which they are attached form 3-, 4-, 5- or 6-membered cycloalkyl or 3-, 4-, 5- or 6-membered heterocycloalkyl, each of which is optionally substituted with 1 or 2 R^{q} substituents;
R¹⁷ is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, OH, -COOH,-C(O)NH₂, NH₂, -NHC₁₋₄ alkyl and -N(C₁₋₄ alkyl)₂;
R⁷ is H, C₁₋₄ alkyl, C₁₋₄ alkoxy, C₁₋₄ haloalkyl, C₁₋₄ haloalkoxy, CN, halo, OH, -COOH, NH₂, -NHC₁₋₄ alkyl or -N(C₁₋₄ alkyl)₂, wherein the C₁₋₄ alkyl and C₁₋₄ alkoxy are each optionally substituted with 1 or 2 substituents independently selected from CN, halo or -C(O)NH₂;
R⁸ and R⁹ are each independently selected from H, halo, CN, OH, -COOH, C₁₋₄ alkyl, C₁₋₄ alkoxy, -NHC₁₋₄ alkyl, -N(C₁₋₄ alkyl)₂, C₁₋₄ haloalkyl, C₁₋₄ haloalkoxy, C₃₋₆ cycloalkyl, phenyl, 5-6 membered heteroaryl and 4-6 membered heterocycloalkyl, wherein the C₁₋₄ alkyl, C₁₋₄ alkoxy, C₁₋₄ haloalkyl, C₁₋₄ haloalkoxy, C₃₋₆ cycloalkyl, phenyl, 5-6 membered heteroaryl and 4-6 membered heterocycloalkyl of R⁸ or R⁹ are each optionally substituted with 1, 2 or 3 independently selected R^{q} substituents;
or R⁸ and R⁹ taken together with the carbon atom to which they are attached form 3-, 4-, 5- or 6-membered cycloalkyl or 4-, 5-, 6- or 7-membered heterocycloalkyl, each of which is optionally substituted with 1 or 2 R^{q} substituents;
or R⁸ and R¹⁰ taken together with the atoms to which they are attached form 4-, 5-, 6- or 7-membered heterocycloalkyl, having zero to one additional heteroatoms as ring members selected from O, N or S, wherein the 4-, 5-, 6- or 7-membered heterocycloalkyl formed by R⁸ and R¹⁰ are each optionally substituted with 1 or 2 R^{q} substituents;
R¹⁰ and R¹¹ are each independently selected from H, C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, (4-10 membered heterocycloalkyl)-C₁₋₄ alkyl-, -C(O)R^{g}, -C(O)OR^{g}, -C(O)NR^{g}R^{g}, -SO₂R^{g} and -SO₂NR^{g}R^{g}, wherein the C₁₋₆ alkyl, C₁₋₆ haloalkyl, C₃₋₆ cycloalkyl, C₆₋₁₀ aryl, 5-10 membered heteroaryl, 4-10 membered heterocycloalkyl, C₆₋₁₀ aryl-C₁₋₄ alkyl-, C₃₋₆ cycloalkyl-C₁₋₄ alkyl-, (5-10 membered heteroaryl)-C₁₋₄ alkyl-, and (4-10 membered heterocycloalkyl)-C₁₋₄ alkyl- of R¹⁰ or R¹¹ are each optionally substituted with 1, 2, or 3 independently selected R^{d} substituents;
or R¹⁰ and R¹¹ taken together with the nitrogen atom to which they are attached form 4-, 5-, 6-, 7-, 8-, 9-, 10-, or 11-membered heterocycloalkyl, wherein the 4-11 membered heterocycloalkyl is each optionally substituted with 1, 2 or 3 R^{f} substituents;
R¹² is H, C₁₋₄ alkyl, C₁₋₄ alkoxy, C₁₋₄ haloalkyl, C₁₋₄ haloalkoxy, CN, halo, OH,-COOH, NH₂, -NHC₁₋₄ alkyl or -N(C₁₋₄ alkyl)₂;
each R^{a} is independently selected from H, CN, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{a} are each optionally substituted with 1, 2, 3, 4, or 5 R^{d} substituents;
each R^{d} is independently selected from C₁₋₆ alkyl, C₁₋₆ haloalkyl, halo, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R, NR^{e}S(O)₂R^{e}, NR^{e}S(O)₂NR^{e}R^{e}, and S(O)₂NR^{e}R^{e}, wherein the C₁₋₆ alkyl, C₁₋₆ haloalkyl, C₆₋₁₀ aryl, 5-10 membered heteroaryl, C₃₋₁₀ cycloalkyl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{d} are each optionally substituted with 1, 2, or 3 independently selected R^{f} substituents;
each R^{e} is independently selected from H, C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{e} are each optionally substituted with 1, 2 or 3 independently selected R^{f} substituents;
each R^{b} substituent is independently selected from halo, C₁₋₆ alkyl, C₂₋₆ alkenyl, C₂₋₆ alkynyl, C₁₋₆ haloalkyl, C₁₋₆ haloalkoxy, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, CN, OH, NH₂, NO₂, NHOR^{c}, OR^{c}, SR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} and S(O)₂NR^{c}R^{c}; wherein the C₁₋₆ alkyl, C₁₋₆ haloalkyl, C₁₋₆ haloalkoxy, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-and (4-10 membered heterocycloalkyl)-C₁₋₄ alkyl- of R^{b} are each further optionally substituted with 1, 2, or 3 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{c} are each optionally substituted with 1, 2, 3, 4, or 5 R^{f} substituents;
each R^{f} is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g}, and S(O)₂NR^{g}R^{g}; wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of R^{f} are each optionally substituted with 1, 2, 3, 4, or 5 Rⁿ substituents;
each Rⁿ is independently selected from C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{o}, OR^{o}, SR^{o}, C(O)R^{o}, C(O)NR^{o}R^{o}, C(O)OR^{o}, OC(O)R^{o}, OC(O)NR^{o}R^{o}, NHR^{o}, NR^{o}R^{o}, NR^{o}C(O)R^{o}, NR^{o}C(O)NR^{o}R^{o}, NR°C(O)OR°, C(=NR^{o})NR^{o}R^{o}, NR^{o}C(=NR^{o})NR^{o}R^{o}, S(O)R^{o}, S(O)NR^{o}R^{o}, S(O)₂R^{o}, NR^{o}S(O)₂R^{o}, NR^{o}S(O)₂NR^{o}R^{o}, and S(O)₂NR^{o}R^{o}, wherein the C₁₋₄ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl- of Rⁿ are each optionally substituted with 1, 2 or 3 independently selected R^{q} substituents;
each R^{g} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{g} are each optionally substituted with 1, 2, or 3 R^{p} substituents;
each R^{p} is independently selected from C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl-, (4-10 membered heterocycloalkyl)-C₁₋₄ alkyl-, halo, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} and S(O)₂NR^{r}R^{r}, wherein the C₁₋₆ alkyl, C₁₋₆ haloalkyl, C₂₋₆ alkenyl, C₂₋₆ alkynyl, C₆₋₁₀ aryl, C₃₋₁₀ cycloalkyl, 5-10 membered heteroaryl, 4-10 membered heterocycloalkyl, C₆₋₁₀ aryl-C₁₋₄ alkyl-, C₃₋₁₀ cycloalkyl-C₁₋₄ alkyl-, (5-10 membered heteroaryl)-C₁₋₄ alkyl- and (4-10 membered heterocycloalkyl)-C₁₋₄ alkyl- of R^{p} is optionally substituted with 1, 2 or 3 R^{q} substituents;
or any two R^{a} substituents together with the nitrogen atom to which they are attached form a 4-, 5-, 6-, 7-, 8-, 9- or 10-membered heterocycloalkyl group optionally substituted with 1, 2 or 3 R^{h} substituents;
each R^{h} is independently selected from C₁₋₆ alkyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl-, C₁₋₆ haloalkyl, C₁₋₆ haloalkoxy, C₂₋₆ alkenyl, C₂₋₆ alkynyl, halo, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ, and S(O)₂NRⁱRⁱ, wherein the C₁₋₆ alkyl, C₂₋₆ alkenyl, C₂₋₆ alkynyl, C₃₋₁₀ cycloalkyl, 4-7 membered heterocycloalkyl, C₆₋₁₀ aryl, 5-6 membered heteroaryl, C₆₋₁₀ aryl-C₁₋₄ alkyl-, C₃₋₁₀ cycloalkyl-C₁₋₄ alkyl-, (5-6 membered heteroaryl)-C₁₋₄ alkyl-, (4-7 membered heterocycloalkyl)-C₁₋₄ alkyl- of R^{h} are each further optionally substituted by 1, 2, or 3 R^{j} substituents;
each R^{j} is independently selected from C₃₋₆ cycloalkyl, C₆₋₁₀ aryl, 5 or 6-membered heteroaryl, 4-7 membered heterocycloalkyl, C₂₋₄ alkenyl, C₂₋₄ alkynyl, halo, C₁₋₄ alkyl, C₁₋₄ haloalkyl, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{K}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k}, and S(O)₂NR^{k}R^{k}, wherein the C₁₋₄ alkyl, C₃₋₆ cycloalkyl, C₆₋₁₀ aryl, 5- or 6-membered heteroaryl, 4-6 membered heterocycloalkyl, C₂₋₄ alkenyl, C₂₋₄ alkynyl, C₁₋₄ haloalkyl, and C₁₋₄ haloalkoxy of R^{j} are each optionally substituted with 1, 2 or 3 independently selected R^{q} substituents;
or two R^{h} groups attached to the same carbon atom of the 4- to 10-membered heterocycloalkyl taken together with the carbon atom to which they are attached form a C₃₋₆ cycloalkyl or 4- to 6-membered heterocycloalkyl having 1-2 heteroatoms as ring members selected from O, N or S;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
or any two R^{e} substituents together with the nitrogen atom to which they are attached form a 4-, 5-, 6-, or 7-membered heterocycloalkyl group optionally substituted with 1, 2, or 3 independently selected R^{h} substituents;
or any two R^{g} substituents together with the nitrogen atom to which they are attached form a 4-, 5-, 6-, or 7-membered heterocycloalkyl group optionally substituted with 1, 2, or 3 independently selected R^{h} substituents;
or any two R° substituents together with the nitrogen atom to which they are attached form a 4-, 5-, 6-, or 7-membered heterocycloalkyl group optionally substituted with 1, 2, or 3 independently selected R^{h} substituents;
or any two R^{r} substituents together with the nitrogen atom to which they are attached form a 4-, 5-, 6-, or 7-membered heterocycloalkyl group optionally substituted with 1, 2, or 3 independently selected R^{h} substituents;
each Rⁱ, R^{k}, R° or R^{r} is independently selected from H, C₁₋₄ alkyl, C₃₋₆ cycloalkyl, C₆₋₁₀ aryl, 5 or 6-membered heteroaryl, 4-7 membered heterocycloalkyl, C₁₋₆ haloalkyl, C₁₋₆ haloalkoxy, C₂₋₄ alkenyl, and C₂₋₄ alkynyl, wherein the C₁₋₄ alkyl, C₃₋₆ cycloalkyl, C₆₋₁₀ aryl, 5 or 6-membered heteroaryl, 4-7 membered heterocycloalkyl, C₂₋₄ alkenyl, and C₂₋₄ alkynyl of Rⁱ, R^{k}, R^{o} or R^{r} are each optionally substituted with 1, 2 or 3 R^{q} substituents;
each R^{q} is independently selected from halo, OH, CN, -COOH, NH₂, -NH-C₁₋₆ alkyl,-N(C₁₋₆ alkyl)₂, C₁₋₆ alkyl, C₁₋₆ alkoxy, C₁₋₆ alkylthio, C₁₋₆ haloalkyl, C₁₋₆ haloalkoxy, phenyl, 5-6 membered heteroaryl, 4-6 membered heterocycloalkyl and C₃₋₆ cycloalkyl, wherein the C₁₋₆ alkyl, phenyl, C₃₋₆ cycloalkyl, 4-6 membered heterocycloalkyl, and 5-6 membered heteroaryl of R^{q} are each optionally substituted with 1, 2, or 3 substituents selected from halo, OH, CN, -COOH, NH₂, C₁₋₄ alkyl, C₁₋₄ alkoxy, C₁₋₄ haloalkyl, C₁₋₄ haloalkoxy, phenyl, C₃₋₁₀ cycloalkyl, 5-6 membered heteroaryl and 4-6 membered heterocycloalkyl;
the subscript m is an integer of 0, 1, 2 or 3;
the subscript n is an integer of 0, 1, 2 or 3;
each subscript q is independently an integer of 1, 2, 3 or 4; and
the subscript p is an integer of 1, 2, 3 or 4.

3. The compound of claim 2, having:
(a) Formula (II): 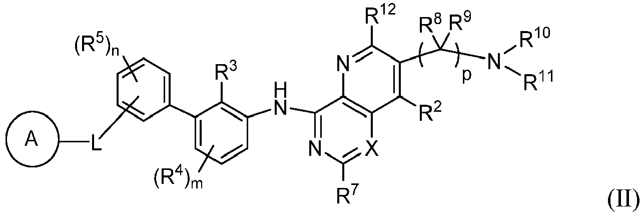 or a pharmaceutically acceptable salt thereof; or
(b) Formula (IIa): 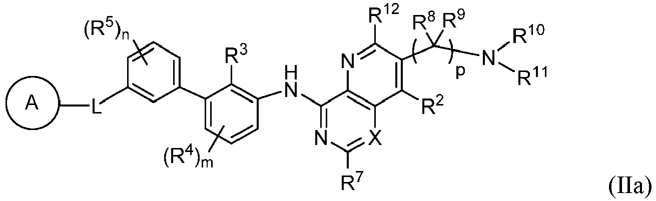 or a pharmaceutically acceptable salt thereof; or
(c) Formula (IIb): 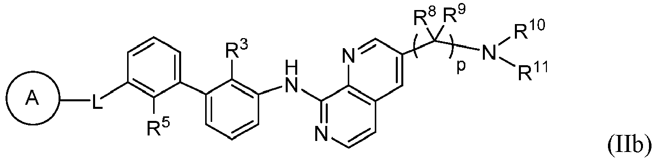 or a pharmaceutically acceptable salt thereof.

4. The compound of claim 2, having:
(a) Formula (III): 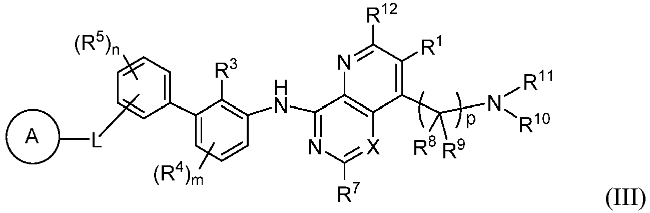 or a pharmaceutically acceptable salt thereof; or
(b) Formula (IIIa): 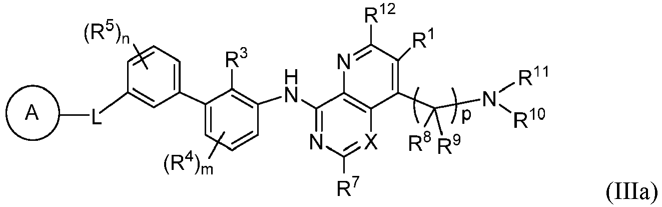 or a pharmaceutically acceptable salt thereof; or
(c) Formula (IIIb): 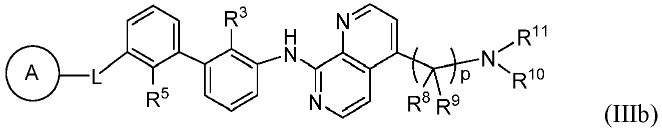 or a pharmaceutically acceptable salt thereof.

5. The compound of any one of claims 1-4, or a pharmaceutically acceptable salt thereof, wherein ring A is selected from: 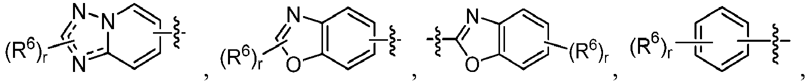 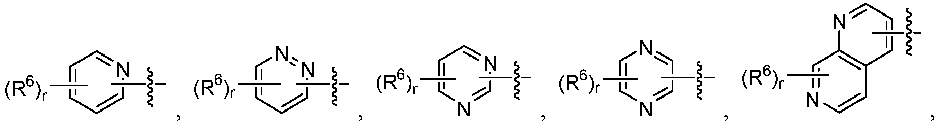 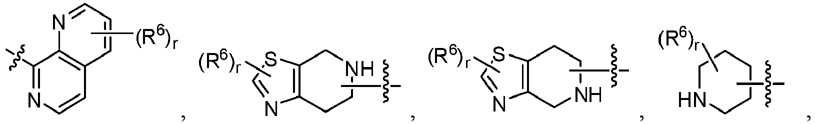 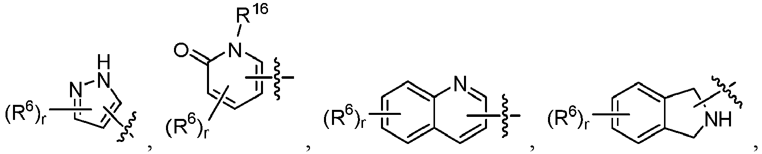 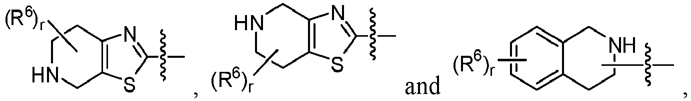 wherein each subscript r is an integer of 1, 2, 3, 4 or 5; R¹⁶ is C₁₋₆ alkyl; and the wavy line indicates the point of attachment to L.

6. The compound of any one of claims **1-5**, or a pharmaceutically acceptable salt thereof, wherein L is a bond, -NH-, -CH=CH- or -C(O)NH-, wherein the carbonyl group in the -C(O)NH- linkage is attached to ring A.

7. The compound of any one of claims **1-6**, or a pharmaceutically acceptable salt thereof, wherein the subscript m is 0.

8. The compound of any one of claims **1-7**, or a pharmaceutically acceptable salt thereof, wherein the subscript n is 1 and R⁵ is halo or C₁₋₄ alkyl.

9. The compound of any one of claims **1-8**, or a pharmaceutically acceptable salt thereof, wherein R³ is methyl, CN or Cl.

10. The compound of any one of claims **2-9**, or a pharmaceutically acceptable salt thereof, wherein R¹² is H, halo, CN, C₁₋₄ alkyl or C₁₋₄ alkoxy.

11. The compound of any one of claims **2-10**, or a pharmaceutically acceptable salt thereof, wherein R⁷ is H, halo, CN, C₁₋₄ alkyl, C₁₋₄ alkoxy or C₁₋₄ haloalkoxy, wherein the C₁₋₄ alkyl and C₁₋₄ alkoxy of R⁷ are each optionally substituted with CN.

12. The compound of any one of claims **2-11**, or a pharmaceutically acceptable salt thereof, wherein R² is H.

13. The compound of any one of claims **2-12**, or a pharmaceutically acceptable salt thereof, wherein R¹ is H.

14. The compound of any one of claims **2-13**, or a pharmaceutically acceptable salt thereof, wherein the subscript p is 1.

15. The compound of any one of claims **2-14**, or a pharmaceutically acceptable salt thereof, wherein R⁸ and R⁹ are each H.

16. The compound of any one of claims **2-15**, or a pharmaceutically acceptable salt thereof, wherein R¹⁰ is H.

17. The compound of any one of claims **2-16**, or a pharmaceutically acceptable salt thereof, wherein R¹¹ is 2-hydroxyethyl, [1-(hydroxymethyl)cyclopropyl]methyl, [1-(hydroxymethyl)cyclobutyl]methyl or 2-(dimethylamino)-2-oxo-ethyl.

18. The compound of any one of claims **2-15**, or a pharmaceutically acceptable salt thereof, wherein -NR¹⁰R¹¹ is (2-hydroxyethyl)amino, 3-hydroxypyrrolidin-1-yl, 3-carboxypyrrolidin-1-yl, 3-carboxyazetidin-1-yl, 2-carboxy-1-piperidinyl, 2-oxooxazolidin-3-yl, [1-(hydroxymethyl)cyclopropyl]methylamino, [1-(hydroxymethyl)cyclobutyl]methylamino or [2-(dimethylamino)-2-oxo-ethyl]amino.

19. The compound of any one of claims **1-18**, or a pharmaceutically acceptable salt thereof, wherein ring A is 2-pyridyl, optionally substituted with 1, 2, 3, or 4 independently selected R⁶ substituents.

20. The compound of any one of claims **1-19**, or a pharmaceutically acceptable salt thereof, wherein X is N or CH.

21. The compound of claim **1** selected from:
2-(((8-((2-chloro-2'methyl-3'(4,5,6,7-tetrahydrothiazolo[5,4-c]pyridin-2-yl)-[1,1'-biphenyl]-3-yl)amino)-1,7-naphthyridin-3-yl)methyl)amino)ethan-1-ol;
1-(((6-(2-fluoro-3'-(3-((2-hydroxyethylamino)methyl)-1,7-naphthyridin-8-ylamino)-2'-methylbiphenyl-3-ylcarbamoyl)pyridin-3-yl)methyl)amino)cyclobutanecarboxylic acid;
(S)-1-((6-((2-fluoro-3'-((3-(((2-hydroxyethyl)amino)methyl)-1,7-naphthyridin-8-yl)amino)-2'-methyl-[1,1-biphenyl]-3-yl)carbamoyl)pyridin-3-yl)methyl)piperidine-2-carboxylic acid;
N-(2-fluoro-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamide;
N-(2-chloro-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamide;
N-(2-chloro-3'-((3-(((R)-3-hydroxypyrrolidin-1-yl)methyl)-1,7-naphthyridin-8-yl)amino)-2'-methyl-[1,1'-biphenyl]-3-yl)-5-(((R)-3-hydroxypyrrolidin-1-yl)methyl)picolinamide;
(R)-1-((8-((2'chloro-3'(5-(((R)-3-hydroxypyrrolidin-1-yl)methyl)picolinamido)-2-methyl-[1,1'-biphenyl]-3-yl)amino)-1,7-naphthyridin-3-yl)methyl)pyrrolidine-3-carboxylic acid; and
(R)-1-((8-((2'-chloro-3'-(5-((3-hydroxypyrrolidin-1-yl)methyl)picolinamido)-2-methyl-[1,1'-biphenyl]-3-yl)amino)-1,7-naphthyridin-3-yl)methyl)azetidine-3-carboxylic acid;
or a pharmaceutically acceptable salt thereof.

22. The compound of claim **1** selected from:
(*R*)-1-((8-((3'-((3-(((*R*)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid;
(*S*)-1-((8-((3'-((3-(((*R*)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid;
(*R*)-1-((8-((3'-((3-(((2-hydroxyethyl)amino)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid;
(*R*)*-*1-((8-((3'-((3-(((*S*)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid; and
(*S*)-1-((8-((3'-((3-(((*S*)-3-hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidine-3-carboxylic acid;
or a pharmaceutically acceptable salt thereof.

23. The compound of claim **1** selected from:
1-((8-(2-chloro-3'-(1,5-dimethyl-4,5,6,7-tetrahydro-1H-imidazo[4,5-c]pyridine-2-carboxamido)-2'-methylbiphenyl-3-ylamino)-1,7-naphthyridin-3-yl)methyl)azetidine-3-carboxylic acid;
(*R*)-1-((5-(2-chloro-3'(3-(((*R*)-3-hydroxypyrrolidin-1-yl)methyl)-1,7-naphthyridin-8-ylamino)-2'-methylbiphenyl-3-ylcarbamoyl)-1-methyl-6-oxo-1,6-dihydropyridin-3-yl)methyl)pyrrolidine-3-carboxylic acid;
(*R*)-1-((8-(2,2'-dichloro-3'-(5-((3-hydroxypyrrolidin-1-yl)methyl)-1-methyl-2-oxo-1,2-dihydropyridine-3-carboxamido)biphenyl-3-ylamino)-1,7-naphthyridin-3-yl)methyl)azetidine-3-carboxylic acid;
1-((8-(2,2'-dichloro-3'-(5-((2-hydroxyethylamino)methyl)-1-methyl-2-oxo-1,2-dihydropyridine-3-carboxamido)biphenyl-3-ylamino)-1,7-naphthyridin-3-yl)methyl)azetidine-3-carboxylic acid;
1-((8-(2,2'-dichloro-3'-(5-((2-hydroxy-2-methylpropylamino)methyl)-1-methyl-2-oxo-1,2-dihydropyridine-3-carboxamido)biphenyl-3-ylamino)-1,7-naphthyridin-3-yl)methyl)azetidine-3-carboxylic acid;
2,2'-(((((2,2'-dimethyl-[1,1'-biphenyl]-3,3'-diyl)bis(azanediyl))bis(1,7-naphthyridine-8,3-diyl))bis(methylene))bis(azanediyl))bis(ethan-1-ol);
(3*R*,3'*R*)-1,1'-((((2,2'-dimethyl-[1,1'-biphenyl]-3,3'-diyl)bis(azanediyl))bis(1,7-naphthyridine-8,3-diyl))bis(methylene))bis(pyrrolidin-3-ol);
(*R*)-1-((8-(3'-(3-((2-hydroxyethylamino)methyl)-1,7-naphthyridin-8-ylamino)-2,2'-dimethylbiphenyl-3-ylamino)-1,7-naphthyridin-3-yl)methyl)pyrrolidin-3-ol;
(3*R*,3'*R*)-1,1'-((((2,2'-dichloro-[1,1'-biphenyl]-3,3'-diyl)bis(azanediyl))bis(1,7-naphthyridine-8,3-diyl))bis(methylene))bis(pyrrolidin-3-ol);
(*R*)-1-((4-(3'-(3-(((*R*)-3-hydroxypyrrolidin-1-yl)methyl)-1,7-naphthyridin-8-ylamino)-2,2'-dimethylbiphenyl-3-ylamino)pyrido[3,2-d]pyrimidin-7-yl)methyl)pyrrolidin-3-ol;
(*R*)-1-((8-(3'-(7-((2-hydroxyethylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(7-(((2-hydroxyethyl)(methyl)amino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(3-(((*R*)-3-hydroxypyrrolidin-1-yl)methyl)-1,7-naphthyridin-8-ylamino)-2,2'-dimethylbiphenyl-3-ylamino)-1,7-naphthyridin-3-yl)methyl)-N,N-dimethylpyrrolidine-3-carboxamide
(*R*)-1-((8-(3'-(7-(((*S*)-2-hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'(5-(((*R*)-3-hydroxypyrrolidin-1-yl)methyl)-1-methyl-2-oxo-1,2-dihydropyridine-3-carboxamido)-2,2'-dimethylbiphenyl-3-ylamino)-1,7-naphthyridin-3-yl)methyl)pyrrolidine-3-carboxylic acid;
(*R*)-1-((8-(3'-(7-(((*R*)-2-hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(7-((2-hydroxy-2-methylpropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(2'-chloro-3'-(1,5-dimethyl-4,5,6,7-tetrahydro-1H-imidazo[4,5-c]pyridine-2-carboxamido)-2-methylbiphenyl-3-ylamino)-1,7-naphthyridin-3-yl)methyl)pyrrolidine-3-carboxylic acid;
(*S*)-N-(2-chloro-3'-(3-((3-hydroxypyrrolidin-1-yl)methyl)-1,7-naphthyridin-8-ylamino)-2'-methylbiphenyl-3-yl)-1,5-dimethyl-4,5,6,7-tetrahydro-1H-imidazo[4,5-c]pyridine-2-carboxamide;
(*R*)-1-((8-(2'-chloro-3'-(1,5-dimethyl-4,5,6,7-tetrahydro-1H-imidazo[4,5-c]pyridine-2-carboxamido)-2-methylbiphenyl-3-ylamino)-1,7-naphthyridin-3-yl)methyl)-3-methylpyrrolidine-3-carboxylic acid;
(*R*)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidine-3-carboxylic acid;
(*R*)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol;
(*S*)-1-((8-((2,2'-dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol;
(*R*)-2-(dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one;
(*S*)-2-(dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-one;
(*R*)-1-(2-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)azetidine-3-carboxylic acid;
(*S*)-1-(2-(2-(3'-((3-(((*R*)-3-hydroxypyrrolidin-1-yl)methyl)-1,7-naphthyridindin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidine-3-carboxylic acid;
(*R*)-1-(2-(2-(3'-((3-(((*R*)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidine-3-carboxylic acid;
(*S*)-1-(2-(2-(3'-((3-(((*R*)-3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)piperidine-2-carboxylic acid;
(*S*)-1-(5-chloro-2-((5-cyanopyridin-3-yl)methoxy)-4-((3'-((3-(((*R*)-3-hydroxypyrrolidin-1-yl)methyl)-1,7-naphthyridin-8-yl)amino)-2,2'-dimethyl-[1,1'-biphenyl]-3-yl)methoxy)benzyl)piperidine-2-carboxylic acid; and
(*R*)-1-(5-chloro-2-((5-cyanopyridin-3-yl)methoxy)-4-((3'-((3-(((*R*)-3-hydroxypyrrolidin-1-yl)methyl)-1,7-naphthyridin-8-yl)amino)-2,2'-dimethyl-[1,1'-biphenyl]-3-yl)methoxy)benzyl)pyrrolidine-3-carboxylic acid;
or a pharmaceutically acceptable salt thereof.

24. The compound of claim **1** selected from:
(*R*)-1-((8-(2'-Chloro-2-methyl-3'-(1-methyl-4,5,6,7-tetrahydro-1*H*-imidazo[4,5-*c*]pyridine-2-carboxamido)biphenyl-3-ylamino)-1,7-naphthyridin-3-yl)methyl)-3-methylpyrrolidine-3-carboxylic acid;
(*R*)-1-((8-(3'-(1,5-dimethyl-4,5,6,7-tetrahydro-1*H*-imidazo[4,5-c]pyridine-2-carboxamido)-2,2'-dimethylbiphenyl-3-ylamino)-1,7-naphthyridin-3-yl)methyl)pyrrolidine-3-carboxylic acid;
*trans*-4-((2-(2-chloro-3'-(3-(((*R*)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-*c*]pyridin-5(4*H*)-yl)methyl)cyclohexanecarboxylic acid;
*cis*-4-((2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexanecarboxylic acid;
*cis*-4-((2-(2-chloro-2'-methyl-3'-(3-(pyrrolidin-1-ylmethyl)-1,7-naphthyridin-8-ylamino)biphenyl-3-ylcarbamoyl)-1-methyl-6,7-dihydro-1H-imidazo[4,5-c]pyridin-5(4H)-yl)methyl)cyclohexanecarboxylic acid;
*trans*-4-((2-(2-chloro-3'-(3-(((*S*)-1-hydroxypropan-2-ylamino)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)methyl)cyclohexanecarboxylic acid;
*trans*-4-((2-(2-chloro-3'-(3-(((1S,2S)-2-hydroxycyclopentylamino)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)methyl)cyclohexanecarboxylic acid;
*trans* 4-(2-(2-(2-chloro-3'-(3-(((*R*)-3-hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-*c*]pyridin-5(4*H*)-yl)ethyl)cyclohexanecarboxylic acid;
*cis* 4-(2-(2-(2-chloro-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexanecarboxylic acid;
3-(2-(2-chloro-3'-(3-(((*R*)-3-hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-*c*]pyridin-5(4*H*)-yl)butanoic acid;
*cis* 4-((2-(2-chloro-3'-(3-(((*S*)-3-hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-*c*]pyridin-5(4*H*)-yl)methyl)cyclohexanecarboxylic acid;
*cis* 4-((2-(2-chloro-3'-(3-(((*R*)-3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)methyl)cyclohexanecarboxylic acid;
(*R*)-4-(2-(2-chloro-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)cyclohexanecarboxylic acid;
(*S*)-4-(2-(2-chloro-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)cyclohexanecarboxylic acid;
*trans* 4-(2-(2-(2-chloro-3'-(3-(((*R*)-1-hydroxypropan-2-ylamino)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)ethyl)cyclohexanecarboxylic acid;
*trans* 4-(2-(2-(2-chloro-3'-(3-(((*S*)-1-hydroxypropan-2-ylamino)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)ethyl)cyclohexanecarboxylic acid;
*trans*-4-(2-(2-(2-chloro-3'-(3-(((*R*)-3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H-*imidazo[4,5-*c*]pyridin-5(4*H*)-yl)ethyl)cyclohexanecarboxylic acid;
(*R*)-4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyndin-5(4*H*)-yl)-1-methylcyclohexanecarboxylic acid;
*trans-*4-(2-(2-(3'-(3-(((*R*)-3-hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridin-5(4*H*)-yl)ethyl)cyclohexanecarboxylic acid;
(*R*)-4-(2-(3'-(3-((3-hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridin-5(4*H*)-yl)cyclohexanecarboxylic acid;
*Trans*-4-(2-(2-(2,2'-dichloro-3'-(3-(((*R*)-3-hydroxypyrrolidin-1-yl)methyl)-1,7-naphthyridin-8-ylamino)biphenyl-3-ylcarbamoyl)-1-methyl-6,7-dihydro-1*H*-imidazo[4,5-*c*]pyridin-5(4*H*)-yl)ethyl)cyclohexanecarboxylic acid;
*trans* 4-(2-(2-(2'-chloro-3'-(3-(((*R*)-3-hydroxypyrrolidin-1-yl)methyl)-1,7-naphthyridin-8-ylamino)-2-methylbiphenyl-3-ylcarbamoyl)-1-methyl-6,7-dihydro-1*H-*imidazo[4,5-*c*]pyridin-5(4*H*)-yl)ethyl)cyclohexanecarboxylic acid;
(*R*)-1-((4-(2'-chloro-3'-(1,5-dimethyl-4,5,6,7-tetrahydro-1*H*-imidazo[4,5-*c*]pyridine-2-carboxamido)-2-methylbiphenyl-3-ylamino)pyrido[3,2-*d*]pyrimidin-7-yl)methyl)-3-methylpyrrolidine-3-carboxylic acid
(*R*)-4-(2-(2-chloro-3'-(7-((3-hydroxypyrrolidin-1-yl)methyl)pyrido[3,2-*d*]pyrimidin-4-ylamino)-2'-methylbiphenyl-3-ylcarbamoyl)-1-methyl-6,7-dihydro-1*H*-imidazo[4,5-*c*]pyridin-5(4*H*)-yl)-1-methylcyclohexanecarboxylic acid;
*trans* 4-((2-(2-chloro-3'-(7-(((*R*)-3-hydroxypyrrolidin-I-yl)methyl)pyrido[3,2-*d*]pyrimidin-4-ylamino)-2'-methylbiphenyl-3-ylcarbamoyl)-1-methyl-6,7-dihydro-1*H-*imidazo[4,5-*c*]pyridin-5(4*H*)-yl)methyl)cyclohexanecarboxylic acid;
(*R*)-1-((5-(3'-(3-(((*R*)-3-hydroxypyrrolidin-1-yl)methyl)-1,7-naphthyridin-8-ylamino)-2,2'-dimethylbiphenyl-3-ylamino)pyrido[4,3-*b*]pyrazin-2-yl)methyl)pyrrolidine-3-carboxylic acid;
(3*R*)-1-((8-(2,2'-dimethyl-3'-(3-(pyrrolidin-2-yl)-1,7-naphthyridin-8-ylamino)biphenyl-3-ylamino)-1,7-naphthyridin-3-yl)methyl)pyrrolidine-3-carboxylic acid;
(*R*)-1-((8-(2,2'-dichloro-3'-(3-((2-hydroxyethylamino)methyl)imidazo[1,2-*a*]pyrazin-8-ylamino)biphenyl-3-ylamino)-1,7-naphthyridin-3-yl)methyl)pyrrolidin-3-ol;
(*R*)-1-((8-((2,2'-dimethyl-3'-((3-(pyrrolidin-1-ylmethyl)-1,7-naphthyridin-8-yl)amino)-[1,1'-biphenyl]-3-yl)amino)-1,7-naphthyridin-3-yl)methyl)pyrrolidin-3-ol;
(*S*)-1-((8-((2'-chloro-3'-((3-(((*R*)-3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)pyrrolidin-3-ol;
(*R*)-1-((8-((2'-chloro-3'-((3-((3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)azetidine-3-carboxylic acid;
(*R*)-1-((8-((2,2'-dichloro-3'-((3-(((R)-3-hydroxypyrrolidin-1-yl)methyl)-1,7-naphthyridin-8-yl)amino)-[1,1'-biphenyl]-3-yl)amino)-1,7-naphthyridin-3-yl)methyl)-3-methylpyrrolidin-3-ol;
(*R*)-1-((8-((2,2'-dichloro-3'-((3-(((2-hydroxyethyl)amino)methyl)-1,7-naphthyridin-8-yl)amino)-[1,1'-biphenyl]-3-yl)amino)-1,7-naphthyridin-3-yl)methyl)pyrrolidin-3-ol;
(*R*)-1-((8-((2,2'-dichloro-3'-((3-(((*R*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid;
(*R*)-1-((8-((2-chloro-3'-((3-(((*R*)-3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)pyrrolidine-3-carboxylic acid;
(*R*)-1-((8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)azetidine-3-carboxylic acid;
(*R*)-3-(((8-((2-chloro-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)amino)propanoic acid;
(*R*)-1-((8-((2,2'-dichloro-3'-((3-(((*S*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid;
(*S*)-1-((8-((2,2'-dichloro-3'-((3-(((*S*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidine-3-carboxylic acid;
(*R*)-1-((8-((3'-(5-(dimethylglycyl)-5,6-dihydro-4*H*-pyrrolo[3,4-*d*]oxazol-2-yl)-2,2'-dimethyl-[1,1'-biphenyl]-3-yl)amino)-1,7-naphthyridin-3-yl)methyl)pyrrolidine-3-carboxylic acid;
(*R*)-1-((8-((3'-(5-(dimethylglycyl)-5,6-dihydro-4*H*-pyrrolo[3,4-*d*]thiazol-2-yl)-2,2'-dimethyl-[1,1'-biphenyl]-3-yl)amino)-1,7-naphthyridin-3-yl)methyl)pyrrolidine-3-carboxylic acid;
2-((*R*)-3-hydroxypyrrolidin-1-yl)-1-(2-(3'-((3-(((*R*)-3-hydroxypyrrolidin-1-yl)methyl)-1,7-naphthyridin-8-yl)amino)-2,2'-dimethyl-[1,1'-biphenyl]-3-yl)-4,6-dihydro-5*H-*pyrrolo[3,4-*d*]thiazol-5-yl)ethan-1-one;
(*R*)-1-((8-((3'-(5-(dimethylglycyl)-5,6-dihydro-4*H*-pyrrolo[3,4-*d*]thiazol-2-yl)-2,2'-dimethyl-[1,1'-biphenyl]-3-yl)amino)-1,7-naphthyridin-3-yl)methyl)-3-methylpyrrolidine-3-carboxylic acid;
1-((8-((3'-(5-(dimethylglycyl)-5,6-dihydro-4*H*-pyrrolo[3,4-*d*]thiazol-2-yl)-2,2'-dimethyl-[1,1'-biphenyl]-3-yl)amino)-1,7-naphthyridin-3-yl)methyl)azetidine-3-carboxylic acid;
(*R*)-1-((8-((2-chloro-3'-(5-(dimethylglycyl)-5,6-dihydro-4*H*-pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid;
(*R*)-1-((8-((2-chloro-3'-(5-(*N*-ethyl-*N*-methylglycyl)-5,6-dihydro-4*H*-pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid;
(*R*)-2-(1-((8-((2-chloro-3'-(5-(dimethylglycyl)-5,6-dihydro-4*H*-pyrrolo[3,4-*d*]thiazol-2-yl)-2'-methyl-[1,1'-biphenyl]-3-yl)amino)-1,7-naphthyridin-3-yl)methyl)pyrrolidin-3-yl)acetic acid;
2-((8-((2-chloro-3'-(5-(dimethylglycyl)-5,6-dihydro-4*H*-pyrrolo[3,4-*d*]thiazol-2-yl)-2'-methyl-[1,1'-biphenyl]-3-yl)amino)-1,7-naphthyridin-3-yl)methyl)-2-azabicyclo[2.2.1]heptane-5-carboxylic acid;
(*R*)-2-(1-((8-(2-chloro-3'-(5-(2-(ethyl(methyl)amino)acetyl)-5,6-dihydro-4*H-*pyrrolo[3,4-*d*]thiazol-2-yl)-2'-methylbiphenyl-3-ylamino)-1,7-naphthyridin-3-yl)methyl)pyrrolidin-3-yl)acetic acid;
2-((8-(2-chloro-3'-(5-(2-(ethyl(methyl)amino)acetyl)-5,6-dihydro-4H-pyrrolo[3,4-d]thiazol-2-yl)-2'-methylbiphenyl-3-ylamino)-1,7-naphthyridin-3-yl)methyl)-2-azabicyclo[2.2.1]heptane-5-carboxylic acid;
(*R*)-1-((8-((2-chloro-3'-(5-(2-((*R*)-3-hydroxypyrrolidin-1-yl)acetyl)-5,6-dihydro-4*H-*pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid;
(*R*)-1-((8-((2-chloro-3'-(5-(*N*-(2-hydroxyethyl)-*N*-methylglycyl)-5,6-dihydro-4*H-*pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid;
(*R*)-1-((8-((2-chloro-3'-(5-(2-((*S*)-3-hydroxypyrrolidin-1-yl)acetyl)-5,6-dihydro-4*H-*pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid;
(*R*)-1-((8-((2-chloro-3'-(5-(2-(3-hydroxyazetidin-1-yl)acetyl)-5,6-dihydro-4*H-*pyrrolo[3,4-*d*]thiazol-2-yl)-2'-methyl-[1,1'-biphenyl]-3-yl)amino)-1,7-naphthyridin-3-yl)methyl)pyrrolidine-3-carboxylic acid; and
Cis-4-((2-(3'-(3-(((R)-3-hydroxypyrrolidin-1-yl)methyl)-1,7-naphthyridin-8-ylamino)-2,2'-dimethylbiphenyl-3-ylcarbamoyl)-1-methyl-6,7-dihydro-1H-imidazo[4,5-c]pyridin-5(4H)-yl)methyl)cyclohexanecarboxylic acid;
or a pharmaceutically acceptable salt thereof.

25. The compound of claim 2, or a pharmaceutically acceptable salt thereof, wherein:
ring A is 5- to 10-membered heteroaryl or 4- to 11-membered heterocycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³- , -NR³C(O)-, -NR¹³-, or CH=CH-;
X is N or CR¹⁷, wherein R¹⁷ is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy are each optionally substituted with 1 or 2 substituents independently selected from CN, halo and -C(O)NH₂;
one of R¹ and R² is -(CR⁸R⁹)ₚ-NR¹⁰R¹¹ and the other is H, C₁₋₄ alkyl, C₁₋₄ alkoxy, C₁₋₄ haloalkyl, C₁₋₄ haloalkoxy, CN, halo, or OH, wherein the C₁₋₄ alkyl and C₁₋₄ alkoxy of R¹ or R² is optionally substituted with 1 or 2 substituents independently selected from C₁₋₄ alkoxy, C₁₋₄ haloalkyl, C₁₋₄ haloalkoxy, CN, halo, and OH;
R³ is methyl, halo, CN or C₁₋₄ haloalkyl;
R⁴ is C₁₋₄ alkyl, C₁₋₄ alkoxy, or C₁₋₄ haloalkyl;
R⁵ is C₁₋₄ alkyl, C₁₋₄ alkoxy, C₁₋₄ haloalkyl, C₁₋₄ haloalkoxy, CN, halo, or OH;
each R⁶ is independently selected from H, halo, C₁₋₆ alkyl, C₂₋₆ alkenyl, C₂₋₆ alkynyl, C₁₋₆ haloalkyl, C₁₋₆ haloalkoxy, 5-14 membered heteroaryl, 4-10 membered heterocycloalkyl, (5-14 membered heteroaryl)-C₁₋₄ alkyl-, (4-10 membered heterocycloalkyl)-C₁₋₄ alkyl-, CN, NO₂, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, or NR^{a}C(O)OR^{a}, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H, C₁₋₄ alkyl, C₁₋₄ alkoxy, C₁₋₄ haloalkyl, C₁₋₄ haloalkoxy, CN, halo, or OH;
R⁸ and R⁹ are each independently selected from H, halo, CN, OH, -COOH, C₁₋₄ alkyl, C₁₋4 alkoxy, -NHC₁₋₄ alkyl, -N(C₁₋₄ alkyl)₂, and C₁₋₄ haloalkyl;
R¹⁰ and R¹¹ are each independently selected from H, C₁₋₆ alkyl, C₁₋₆ haloalkyl, -C(O)R^{g},-C(O)OR^{g}, and -C(O)NR^{g}R^{g}, wherein the C₁₋₆ alkyl and C₁₋₆ haloalkyl of R¹⁰ or R¹¹ are each optionally substituted with 1 or 2 independently selected R^{f} substituents;
or R¹⁰ and R ¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H, C₁₋₄ alkyl, C₁₋₄ alkoxy, C₁₋₄ haloalkyl, C₁₋₄ haloalkoxy, CN, halo, or OH;
each R¹³ is independently H, C₁₋₆ haloalkyl or C₁₋₆ alkyl;
each R^{a} is independently selected from H, CN, C₁₋₆ alkyl, C₁₋₄ haloalkyl, C₂₋₆ alkenyl, and C₂₋₆ alkynyl;
each R^{d} is independently selected from C₁₋₆ alkyl, C₁₋₆ haloalkyl, halo, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, and NR^{e}C(O)R^{e};
each R^{e} is independently selected from H, C₁₋₆ alkyl, C₁₋₆ haloalkyl, C₂₋₆ alkenyl, and C₂₋₆ alkynyl;
each R^{b} substituent is independently selected from halo, C₁₋₆ alkyl, C₁₋₆ haloalkyl, C₁₋₆ haloalkoxy, CN, OH, NH₂, NO₂, OR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, and NR^{c}C(O)OR^{c}; wherein the C₁₋₄ alkyl, C₁₋₄ haloalkyl, and C₁₋₄ haloalkoxy of R^{b} are each further optionally substituted with 1 or 2 independently selected R^{d} substituents;
each R^{c} is independently selected from H, C₁₋₆ alkyl, C₁₋₄ haloalkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl-, wherein the C₁₋₆ alkyl, C₂₋₆ alkenyl, C₂₋₆ alkynyl, C₆₋₁₀ aryl, C₃₋₁₀ cycloalkyl, C₆₋₁₀ aryl-C₁₋₄ alkyl-, and C₃₋₁₀ cycloalkyl-C₁₋₄ alkyl- of R^{c} are each optionally substituted with 1, 2, or 3 R^{f} substituents;
each R^{f} is independently selected from C₁₋₄ alkyl, C₁₋₄ haloalkyl, C₂₋₆ alkenyl, C₂₋₆ alkynyl, halo, CN, OR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, and NR^{g}C(O)OR^{g};
each R^{g} is independently selected from H, C₁₋₆ alkyl, C₁₋₄ haloalkyl, C₂₋₆ alkenyl, and C₂₋₆ alkynyl;
each R^{h} is independently selected from C₁₋₆ alkyl, C₁₋₆ haloalkyl, C₁₋₆ haloalkoxy, C₂₋₆ alkenyl, C₂₋₆ alkynyl, halo, CN, ORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, and NRⁱC(O)ORⁱ, wherein the C₁₋₆ alkyl, C₂₋₆ alkenyl, and C₂₋₆ alkynyl of R^{h} are each further optionally substituted by 1, 2, or 3 R^{j} substituents;
each R^{j} is independently selected from C₂₋₄ alkenyl, C₂₋₄ alkynyl, halo, C₁₋₄ alkyl, C₁₋₄ haloalkyl, and CN;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each Rⁱ is independently selected from H, C₁₋₄ alkyl, C₁₋₆ haloalkyl, C₁₋₆ haloalkoxy, C₂₋₄ alkenyl, and C₂₋₄ alkynyl;
the subscript m is an integer of 0, 1, or 2;
the subscript n is an integer of 0, 1, or 2; and
the subscript p is an integer of 1, 2, or 3.

26. The compound of claim **2,** or a pharmaceutically acceptable salt thereof, wherein:
ring A is 5- to 10-membered heteroaryl or 4- to 11-membered heterocycloalkyl, wherein the 5- to 10-membered heteroaryl and 4- to 11-membered heterocycloalkyl each has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1, 2 or 3 R⁶ substituents;
L is a bond, -C(O)NR¹³- or -NR¹³C(O)-;
X is CR¹⁷, wherein R¹⁷ is H or C₁₋₄ alkyl;
one of R¹ and R² is -(CR⁸R⁹)ₚ-NR¹⁰R¹¹ and the other is H, C₁₋₄ alkyl, or C₁₋₄ alkoxy;
R³ is methyl, or halo;
R⁴ is C₁₋₄ alkyl or C₁₋₄ alkoxy;
R⁵ is C₁₋₄ alkyl, C₁₋₄ alkoxy, or halo;
each R⁶ is independently selected from H, halo, C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl-, and (4-10 membered heterocycloalkyl)-C₁₋₄ alkyl-, wherein the C₁₋₆ alkyl, C₂₋₆ alkenyl, C₂₋₆ alkynyl, 5-14 membered heteroaryl, 4-10 membered heterocycloalkyl, (5-14 membered heteroaryl)-C₁₋₄ alkyl- and (4-10 membered heterocycloalkyl)-C₁₋₄ alkyl- of R⁶ are each optionally substituted with 1, 2, or 3 R^{b} substituents;
R⁷ is H or C₁₋₄ alkyl;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H or C₁₋₄ alkyl;
each R¹³ is independently H or C₁₋₆ alkyl;
each R^{b} substituent is independently selected from halo, C₁₋₆ alkyl, OH, NH₂, C(O)OR^{c}, NHR^{c}, and NR^{c}R^{c};
each R^{c} is independently selected from H, C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl-, wherein the C₁₋₆ alkyl, C₂₋₆ alkenyl, C₂₋₆ alkynyl, C₃₋₁₀ cycloalkyl, and C₃₋₁₀ cycloalkyl-C₁₋₄ alkyl- of R^{c} are each optionally substituted with 1 or 2 R^{f} substituents;
each R^{f} is independently selected from C₁₋₄ alkyl, C₂₋₆ alkenyl, C₂₋₆ alkynyl, halo, OR^{g}, and C(O)OR^{g};
each R^{g} is independently selected from H and C₁₋₆ alkyl;
each R^{h} is independently selected from C₁₋₆ alkyl, C₂₋₆ alkenyl, C₂₋₆ alkynyl, halo, CN, ORⁱ, and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
each Rⁱ is independently selected from H and C₁₋₄ alkyl;
the subscript m is an integer of 0 or 1;
the subscript n is an integer of 0 or 1; and
the subscript p is an integer of 1 or 2.

27. The compound of claim **2,** or a pharmaceutically acceptable salt thereof, wherein:
ring A is 5- to 10-membered heteroaryl, wherein the 5- to 10-membered heteroaryl has 1-4 heteroatoms as ring members selected from N, O and S, wherein the N or S atom as ring members is optionally oxidized and one or more carbon atoms as ring members are each optionally replaced by a carbonyl group; and wherein ring A is optionally substituted with 1 or 2 R⁶ substituents;
L is a bond, -C(O)NR¹³- or -NR¹³C(O)-;
X is CR¹⁷, wherein R¹⁷ is H;
one of R¹ and R² is -(CR⁸R⁹)ₚ-NR¹⁰R¹¹ and the other is H;
R³ is methyl, or halo;
R⁴ is C₁₋₄ alkyl or C₁₋₄ alkoxy;
R⁵ is C₁₋₄ alkyl or halo;
each R⁶ is independently selected from H, C₁₋₆ alkyl, and (4-10 membered heterocycloalkyl)-C₁₋₄ alkyl-, wherein the C₁₋₆ alkyl and (4-10 membered heterocycloalkyl)-C₁₋₄ alkyl- of R⁶ are each optionally substituted with 1 or 2 R^{b} substituents;
R⁷ is H;
R⁸ and R⁹ are each independently selected from H and C₁₋₄ alkyl;
R¹⁰ and R¹¹ are each independently selected from H and C₁₋₆ alkyl optionally substituted with 1 or 2 independently selected R^{f} substituents;
or R¹⁰ and R¹¹ taken together with the nitrogen atom to which they are attached form 4-, 5-, 6- or 7-membered heterocycloalkyl, wherein the 4-, 5-, 6- or 7-membered heterocycloalkyl is optionally substituted with 1, 2 or 3 R^{h} substituents;
R¹² is H;
R¹³ is H;
each R^{b} substituent is independently selected from OH, C(O)OR^{c}, NHR^{c}, and NR^{c}R^{c};
each R^{c} is independently selected from H, C₁₋₆ alkyl, and C₃₋₁₀ cycloalkyl, wherein the C₁₋₆ alkyl, and C₃₋₁₀ cycloalkyl of R^{c} are each optionally substituted with 1 or 2 R^{f} substituents;
each R^{f} is independently selected from OR^{g}, and C(O)OR^{g};
R^{g} is H;
each R^{h} is independently selected from ORⁱ and C(O)ORⁱ;
or any two R^{c} substituents together with the nitrogen atom to which they are attached form a 4-, 5-, 6-, or 7-membered heterocycloalkyl group optionally substituted with 1, 2, or 3 independently selected R^{h} substituents;
Rⁱ is H;
the subscript m is an integer of 0 or 1;
the subscript n is an integer of 0 or 1; and
the subscript p is an integer of 1 or 2.

28. A pharmaceutical composition comprising a compound of any one of claims **1-21** and **25-27,** or a pharmaceutically acceptable salt thereof, and one or more pharmaceutically acceptable excipients or carriers.

29. A pharmaceutical composition comprising a compound of any one of claims **22-24,** or a pharmaceutically acceptable salt thereof, and one or more pharmaceutically acceptable excipients or carriers.

30. A compound of any one of claims 1-21 and 25-27, or a pharmaceutically acceptable salt thereof, or a composition of claim **28,** for use in a method of treating a disease or disorder associated with inhibition of PD-1/PD-L1 interaction, wherein the disease or disorder is cancer or an infection.

31. The compound, salt or composition for use according to claim **30,** wherein the disease or disorder is an infection, and the infection is a viral infection.

32. The compound, salt or composition for use according to claim **30,** wherein the disease or disorder is cancer.

33. The compound, salt or composition for use according to claim **32,** wherein the cancer is selected from:
(a) bone cancer, pancreatic cancer, skin cancer, cancer of the head or neck, cutaneous malignant melanoma, intraocular malignant melanoma, uterine cancer, ovarian cancer, rectal cancer, cancer of the anal region, stomach cancer, testicular cancer, carcinoma of the fallopian tubes, carcinoma of the endometrium, endometrial cancer, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic leukemias, acute leukemias, solid tumors of childhood, lymphocytic lymphoma, cancer of the bladder, cancer of the kidney, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, melanoma, metastatic malignant melanoma, renal cancer, clear cell carcinoma, prostate cancer, hormone refractory prostate adenocarcinoma, breast cancer, colon cancer, lung cancer, non-small cell lung cancer, small cell lung cancer, solid tumors, hepatic cancer, gastric cancer, glioblastoma, sarcoma, hematological cancers, lymphoma, leukemia, acute lymphoblastic leukemia (ALL), acute myelogenous leukemia (AML), chronic lymphocytic leukemia (CLL), chronic myelogenous leukemia (CML), DLBCL, mantle cell lymphoma, relapsed NHL, refractory NHL, recurrent follicular NHL, multiple myeloma, cholangiocarcinoma, bile duct cancer, triple negative breast cancer, rhabdomyosarcoma, leiomyosarcoma, hepatocellular carcinoma, Ewing's sarcoma, brain cancer, brain tumor, astrocytoma, neuroblastoma, neurofibroma, basal cell carcinoma, chondrosarcoma, epithelioid sarcoma, eye cancer, Fallopian tube cancer, gastrointestinal cancer, gastrointestinal stromal tumors, hairy cell leukemia, intestinal cancer, islet cell cancer, oral cancer, mouth cancer, throat cancer, laryngeal cancer, lip cancer, mesothelioma, nasal cavity cancer, ocular cancer, ocular melanoma, pelvic cancer, renal cell carcinoma, salivary gland cancer, sinus cancer, spinal cancer, tongue cancer, tubular carcinoma, and ureteral cancer; or
(b) a metastatic cancer that expresses PD-L1; or
(c) lung cancer; or
(d) small cell lung cancer; or
(e) non-small cell lung cancer; or
(f) kidney cancer; or
(g) hepatic cancer; or
(h) hepatocellular carcinoma; or
(i) melanoma; or
(j) cancer of the bladder; or
(k) cancer of the urethra; or
(l) renal cancer; or
(m) renal cell carcinoma.

34. A compound of any one of claims **1-21** and **25-27,** or a pharmaceutically acceptable salt thereof, or a composition of claim **28,** for use in a method of enhancing, stimulating and/or increasing the immune response in a patient, wherein the patient has cancer or an infection.

35. A compound of any one of claims **22-24,** or a pharmaceutically acceptable salt thereof, or a composition of claim **29,** for use in a method of treating a disease or disorder associated with inhibition of PD-1/PD-L1 interaction, wherein the disease or disorder is cancer or an infection.

36. The compound, salt or composition for use according to claim **35,** wherein the disease or disorder is cancer.

37. The compound, salt or composition for use according to claim **36,** wherein the cancer is selected from urothelial cancer, cancers with high microsatellite instability (MSI^{high}), genitourinary tract cancers, liver cancers, nervous system cancers, gynecological cancers, acute promyelocytic leukemia (APL), myeloproliferative diseases, primary myelofibrosis (PMF), polycythemia vera (PV), essential thrombocytosis (ET), myelodysplasia syndrome (MDS), T-cell acute lymphoblastic lymphoma (T-ALL), osteosarcoma, angiosarcoma, fibrosarcoma, liposarcoma, myxoma, rhabdomyoma, rhabdosarcoma, fibroma, lipoma, harmatoma, teratoma, bronchogenic carcinoma, squamous cell carcinoma, undifferentiated small cell carcinoma, undifferentiated large cell carcinoma, adenocarcinoma, alveolar (bronchiolar) carcinoma, bronchial adenoma, chondromatous hamartoma, carcinoma, ductal adenocarcinoma, insulinoma, glucagonoma, gastrinoma, carcinoid tumors, vipoma, cancers of the small bowel, leiomyoma, hemangioma, cancers of the large bowel, tubular adenoma, villous adenoma, hamartoma, colorectal cancer, Wilm's tumor [nephroblastoma], transitional cell carcinoma, seminoma, embryonal carcinoma, teratocarcinoma, choriocarcinoma, sarcoma, interstitial cell carcinoma, fibroadenoma, adenomatoid tumors, hepatoma, hepatoblastoma, hepatocellular adenoma, osteogenic sarcoma, malignant fibrous histiocytoma, malignant lymphoma, reticulum cell sarcoma, malignant giant cell tumor chordoma, osteochronfroma, osteocartilaginous exostoses, benign chondroma, chondroblastoma, chondromyxofibroma, osteoid osteoma, giant cell tumors, cancers of the skull, osteoma, granuloma, xanthoma, osteitis deformans, cancers of the meninges, meningioma, meningiosarcoma, gliomatosis, meduoblastoma, glioma, ependymoma, germinoma, pinealoma, glioblastoma multiform, oligodendroglioma, schwannoma, retinoblastoma, congenital tumors, cancers of the spinal cord, Lhermitte-Duclos disease, endometrial carcinoma, cancers of the cervix, pre-tumor cervical dysplasia, ovarian carcinoma, serous cystadenocarcinoma, mucinous cystadenocarcinoma, unclassified carcinoma, granulosa-thecal cell tumors, Sertoli-Leydig cell tumors, dysgerminoma, malignant teratoma, cancers of the vulva, intraepithelial carcinoma, cancers of the vagina, botryoid sarcoma, embryonal rhabdomyosarcoma, moles dysplastic nevi, angioma, dermatofibroma, keloids, myelodysplastic syndromes, and urothelial carcinoma.

## Patentansprüche

1. Verbindung der Formel (I): 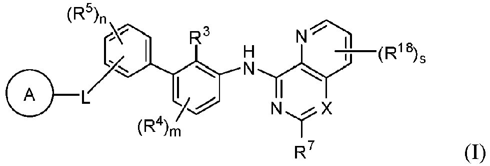 oder ein pharmazeutisch unbedenkliches Salz davon, wobei:
Ring A für 5- bis 10-gliedriges Heteroaryl, 4- bis 11-gliedriges Heterocycloalkyl, C₆₋₁₀-Aryl oder
C₃₋₁₀-Cycloalkyl steht, wobei das 5- bis 10-gliedrige Heteroaryl und das 4- bis 11-gliedrige Heterocycloalkyl jeweils 1-4 Heteroatome als Ringglieder, die aus N, O und S ausgewählt sind, aufweisen, wobei das N- oder S-Atom als Ringglied gegebenenfalls oxidiert ist und ein oder mehrere Kohlenstoffatome als Ringglieder jeweils gegebenenfalls durch eine Carbonylgruppe ersetzt sind; und wobei Ring A gegebenenfalls durch 1, 2, 3, 4 oder 5 R⁶-Substituenten substituiert ist;
L für eine Bindung, -C(O)NR¹³-, -NR¹³C(O)-, O, -(CR¹⁴R¹⁵)_{q}-, -(CR¹⁴R¹⁵)_{q}-O-, -O(CR¹⁴R¹⁵)_{q}-, -NR¹³-, -(CR¹⁴R¹⁵)_{q}-NR¹³-, -NR¹³-(CR¹⁴R¹⁵)_{q}-, -CH=CH-, -C≡C-, -SO₂NR¹³-, -NR¹³SO₂-, -NR¹³C(O)O- oder -NR¹³C(O)NR¹³-steht;
X für N oder CR¹⁷ steht;
R³ für Methyl, Halogen, CN oder C₁₋₄-Halogenalkyl steht;
R⁴ für C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl oder -N(C₁₋₄-Alkyl)₂ steht;
R⁵ für C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl oder -N(C₁₋₄-Alkyl)₂ steht;
R⁶, R⁷, R¹⁷ und R¹⁸ jeweils unabhängig aus H, Halogen, C₁₋₆-Alkyl, C₂₋₆-Alkenyl, C₂₋₆-Alkinyl, C₁₋₆-Halogenalkyl, C₁₋₆-Halogenalkoxy, C₆₋₁₀-Aryl, C₃₋₁₀-Cycloalkyl, 5-14-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-14-gliedrigem Heteroaryl)-C₁₋₄-alkyl-, (4-10-gliedrigem Heterocycloalkyl) -C₁₋₄-alkyl-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a} und S(O)₂NR^{a}R^{a} ausgewählt sind, wobei das C₁₋₆-Alkyl, C₂₋₆-Alkenyl, C₂₋₆-Alkinyl, C₆₋₁₀-Aryl, C₃₋₁₀-Cycloalkyl, 5-14-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-14-gliedrige Heteroaryl)-C₁₋₄-alkyl- und (4-10-gliedrige Heterocycloalkyl)-C₁₋₄-alkyl- von R⁶, R⁷, R¹⁷ und R¹⁸ jeweils gegebenenfalls durch 1, 2, 3, 4 oder 5 R^{b}-Substituenten substituiert ist;
oder zwei R⁶-Substituenten, die an dasselbe Ringkohlenstoffatom gebunden sind, zusammen mit dem Ringkohlenstoffatom, an das sie gebunden sind, C₃₋₆-Spirocycloalkyl oder 4- bis 7-gliedriges Spiroheterocycloalkyl bilden, wobei jede dieser Gruppen gegebenenfalls durch 1, 2 oder 3 unabhängig ausgewählte R^{f}-Substituenten substituiert ist;
R¹³ jeweils unabhängig für H, C₁₋₆-Halogenalkyl oder C₁₋₆-Alkyl, das gegebenenfalls durch einen Substituenten, der aus C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl und -N(C₁₋₄-Alkyl)₂ ausgewählt ist, substituiert ist, steht;
R¹⁴ und R¹⁵ jeweils unabhängig aus H, Halogen, CN, OH, -COOH, C₁₋₄-Alkyl, C₁₋₄-Alkoxy, -NH-C₁₋₄-Alkyl, -N (C₁₋₄-Alkyl)₂, C₁₋₄-Halogenalkyl, C₁₋₄-Halogen-alkoxy, C₃₋₆-Cycloalkyl, Phenyl, 5-6-gliedrigem Heteroaryl und 4-6-gliedrigem Heterocycloalkyl ausgewählt sind, wobei das C₁₋₄-Alkyl, C₁₋₄-Alkoxy, C₁₋₄-Halogenalkyl, C₁₋₄-Halogenalkoxy, C₃₋₆-Cycloalkyl, Phenyl, 5-6-gliedrige Heteroaryl und 4-6-gliedrige Heterocycloalkyl von R¹⁴ oder R¹⁵ jeweils gegebenenfalls durch 1, 2 oder 3 unabhängig ausgewählte R^{q}-Substituenten substituiert ist;
oder R¹⁴ und R¹⁵ zusammen mit dem Kohlenstoffatom, an das sie gebunden sind, 3-, 4-, 5- oder 6-gliedriges Cycloalkyl oder 3-, 4-, 5- oder 6-gliedriges Heterocycloalkyl bilden, wobei jede dieser Gruppen gegebenenfalls durch 1 oder 2 R^{q}-Substituenten substituiert ist;
R^{a} jeweils unabhängig aus H, CN, C₁₋₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{a} jeweils gegebenenfalls durch 1, 2, 3, 4 oder 5 R^{d}-Substituenten substituiert sind;
R^{d} jeweils unabhängig aus C₁₋₆-Alkyl, C₁₋₆-Halogenalkyl, Halogen, C₆₋₁₀-Aryl, 5-10-gliedrigem Heteroaryl, C₃₋₁₀-Cycloalkyl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁-₄-alkyl-, (4-10-gliedrigem Heterocycloalkyl) -C₁₋₄-alkyl-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e} und S(O)₂NR^{e}R^{e} ausgewählt ist, wobei das C₁₋₆-Alkyl, C₁₋₆-Halogenalkyl, C₆₋₁₀-Aryl, 5-10-gliedrige Heteroaryl, C₃₋₁₀-Cycloalkyl, 4-10-gliedrige Heterocycloalkyl, C₆₋₁₀-Aryl-C₁₋₄-alkyl, C₃₋₁₀-Cycloalkyl-C₁₋₄-alkyl-, (5-10-gliedrige Heteroaryl)-C₁₋₄-alkyl und (4-10-gliedrige Heterocycloalkyl)-C₁₋₄-alkyl- von R^{d} jeweils gegebenenfalls durch 1, 2 oder 3 unabhängig ausgewählte R^{f}-Substituenten substituiert sind;
R^{e} jeweils unabhängig aus H, C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{e} jeweils gegebenenfalls durch 1, 2 oder 3 unabhängig ausgewählte R^{f}-Substituenten substituiert sind;
jeder R^{b}-Substituent unabhängig aus Halogen, C₁₋₆-Alkyl, C₂₋₆-Alkenyl, C₂₋₆-Alkinyl, C₁₋₆-Halogenalkyl, C₁₋₆-Halogenalkoxy, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, CN, OH, NH₂, NO₂, NHOR^{c}, OR^{c}, SR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(O)OR^{c}, NR^{c}C(O)NR^{c}R^{c}, , NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} oder S(O)₂NR^{c}R^{c} ausgewählt ist, wobei das C₁₋₆-Alkyl, C₁₋₆-Halogenalkyl, C₁₋₆-Halogenalkoxy, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{b} jeweils gegebenenfalls durch 1, 2 oder 3 unabhängig ausgewählte R^{d}-Substituenten substituiert sind;
R^{c} jeweils unabhängig aus H, C₁₋₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{c} jeweils gegebenenfalls durch 1, 2, 3, 4 oder 5 R^{f}-Substituenten substituiert sind;
R^{f} jeweils unabhängig aus C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, Halogen, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g} und S(O)₂NR^{g}R^{g} ausgewählt ist; wobei das C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl) -C₁₋₄-alkyl- und (4-10-gliedrige Heterocycloalkyl)-C₁₋₄-alkyl- von R^{f} jeweils gegebenenfalls durch 1, 2, 3, 4 oder 5 Rⁿ-Substituenten substituiert sind;
Rⁿ jeweils unabhängig aus C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl) -C₁₋₄-alkyl-, (4-10-gliedrige Heterocycloalkyl) -C₁₋₄-alkyl-, Halogen, CN, R^{o}, NHOR^{o}, OR^{o}, SR^{o}, C(O)R^{o}, C(O)NR^{o}R^{o}, C(O)OR^{o}, OC(O)R^{o}, OC(O)NR^{o}R^{o}, NHR^{o}, NR^{o}R^{o}, NR^{o}C(O)R^{o}, NR^{o}C(O)NR^{o}R^{o}, NR^{o}C(O)OR^{o}, C(=NR^{o})NR^{o}R^{o}, NR^{o}C(=NR^{o})NR^{o}R^{o}, S(O)R^{o}, S(O)NR^{o}R^{o}, S(O)₂R^{o}, NR^{o}S(O)₂R^{o}, NR^{o}S(O)₂NR^{o}R^{o} und S(O)₂NR^{o}R^{o} ausgewählt ist, wobei das C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von Rⁿ jeweils gegebenenfalls durch 1, 2 oder 3 unabhängig ausgewählte R^{q}-Substituenten substituiert sind;
R^{g} jeweils unabhängig aus H, C₁₋₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{g} jeweils gegebenenfalls durch 1, 2 oder 3 R^{p}-Substituenten substituiert sind,
R^{p} jeweils unabhängig aus C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, Halogen, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r}) NR^{r}R^{r}, NR^{r}C(=NR^{r}) NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)2R^{r}, NR^{r}S(O)₂NR^{r}R^{r} und S(O)₂NR^{r}R^{r} ausgewählt ist, wobei das C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{p} jeweils gegebenenfalls durch 1, 2 oder 3 R^{q}-Substituenten substituiert sind;
oder beliebige zwei R^{a}-Substituenten zusammen mit dem Stickstoffatom, an das sie gebunden sind, eine 4-, 5-, 6-, 7-, 8-, 9- oder 10-gliedrige Heterocycloalkylgruppe bilden, die gegebenenfalls durch 1, 2 oder 3 R^{h}-Substituenten substituiert ist;
R^{h} jeweils unabhängig aus C₁₋₆-Alkyl, C₃₋₁₀-Cycloalkyl, 4-7-gliedrigem Heterocycloalkyl, C₆₋₁₀-Aryl, 5-6-gliedrigem Heteroaryl, C₆₋₁₀-Aryl-C₁₋₄-alkyl-, C₃₋₁₀-Cycloalkyl-C₁₋₄-alkyl-, (5-6-gliedrigem Heteroaryl) -C₁₋₄-alkyl-, (4-7-gliedrigem Heterocycloalkyl) -C₁₋₄-alkyl-, C₁₋₆-Halogenalkyl, C₁₋₆-Halogenalkoxy, C₂₋₆-Alkenyl, C₂₋₆-Alkinyl, Halogen, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ und S(O)₂NRⁱRⁱ ausgewählt ist, wobei das C₁₋₆-Alkyl, C₂₋₆-Alkenyl, C₂₋₆-Alkinyl, C₃₋₁₀-Cycloalkyl, 4-7-gliedrige Heterocycloalkyl, C₆₋₁₀-Aryl, 5-6-gliedrige Heteroaryl, C₆₋₁₀-Aryl-C₁₋₄-alkyl-, C₃₋₁₀-Cycloalkyl-C₁₋₄-alkyl-, (5-6-gliedrige Heteroaryl)-C₁₋₄-alkyl- und (4-7-gliedrige Heterocycloalkyl)-C₁₋₄-alkyl- von R^{h} jeweils ferner gegebenenfalls durch 1, 2 oder 3 R^{j}-Substituenten substituiert sind;
R^{j} jeweils unabhängig aus C₃₋₆-Cycloalkyl, C₆₋₁₀-Aryl, 5- oder 6-gliedrigem Heteroaryl, 4-7-gliedrigem Heterocycloalkyl, C₂₋₄-Alkenyl, C₂₋₄-Alkinyl, Halogen, C₁₋₄-Alkyl, C₁₋₄-Halogenalkyl, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k} und S(O)₂NR^{k}R^{k} ausgewählt ist; wobei das C₁₋₄-Alkyl, C₃₋₆-Cycloalkyl, C₆₋₁₀-Aryl, 5- oder 6-gliedrige Heteroaryl, 4-6-gliedrige Heterocycloalkyl, C₂₋₄-Alkenyl, C₂₋₄-Alkinyl, C₁₋₄-Halogenalkyl und C₁₋₄-Halogenalkoxy von R^{j} jeweils ferner gegebenenfalls durch 1, 2 oder 3 unabhängig ausgewählte R^{q}-Substituenten substituiert sind;
oder zwei R^{h}-Gruppen, die an dasselbe Kohlenstoffatom des 4- bis 10-gliedrigen Heterocycloalkyls gebunden sind, zusammen mit dem Kohlenstoffatom, an das sie gebunden sind, ein C₃₋₆-Cycloalkyl oder 4- bis 6-gliedriges Heterocycloalkyl mit 1-2 Heteroatomen als Ringgliedern, die aus O, N und S ausgewählt sind, bilden;
oder beliebige zwei R^{c}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{e}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{g}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{o}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{r}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
Rⁱ, R^{k}, R^{o} oder R^{r} jeweils unabhängig aus H, C₁₋₄-Alkyl, C₃₋₆-Cycloalkyl, C₆₋₁₀-Aryl, 5- oder 6-gliedrigem Heteroaryl, 4-7-gliedrigem Heterocycloalkyl, C₁₋₆-Halogenalkyl, C₁₋₆-Halogenalkoxy, C₂₋₄-Alkenyl und C₂₋₄-Alkinyl ausgewählt ist, wobei das C₁₋₄-Alkyl, C₃₋₆-Cycloalkyl, C₆₋₁₀-Aryl, 5- oder 6-gliedrige Heteroaryl, 4-7-gliedrige Heterocycloalkyl, C₂₋₄-Alkenyl und C₂₋₄-Alkinyl von Rⁱ, R^{k}, R^{o} oder R^{r} jeweils gegebenenfalls durch 1, 2 oder 3 R^{q}-Substituenten substituiert sind;
R^{q} jeweils unabhängig aus Halogen, OH, CN, -COOH, NH₂, -NH-C₁₋₆-Alkyl, -N(C₁₋₆-Alkyl)₂, C₁₋₆-Alkyl, C₁₋₆-Alkoxy, C₁₋₆-Alkylthio, C₁-₆-Halogenalkyl, C₁₋₆-Halogenalkoxy, Phenyl, 5-6-gliedrigem Heteroaryl, 4-6-gliedrigem Heterocycloalkyl und C₃-₆-Cycloalkyl ausgewählt ist, wobei das C₁₋₆-Alkyl, Phenyl, C₃₋₆-Cycloalkyl, 4-6-gliedrige Heterocycloalkyl und 5-6-gliedrige Heteroaryl von R^{q} jeweils gegebenenfalls durch 1, 2 oder 3 Substituenten substituiert sind, die aus Halogen, OH, CN, -COOH, NH₂, C₁₋₄-Alkyl, C₁₋₄-Alkoxy, C₁₋₄-Halogenalkyl, C₁₋₄-Halogen-alkoxy, Phenyl, C₃₋₁₀-Cycloalkyl, 5-6-gliedrigem Heteroaryl und 4-6-gliedrigem Heterocycloalkyl ausgewählt sind;
der tiefgestellte Index m für eine ganze Zahl mit einem Wert von 0, 1, 2 oder 3 steht;
der tiefgestellte Index n für eine ganze Zahl mit einem Wert von 0, 1, 2 oder 3 steht;
der tiefgestellte Index q jeweils für eine ganze Zahl mit einem Wert von 0, 1, 2, 3 oder 4 steht und
der tiefgestellte Index s für eine ganze Zahl mit einem Wert von 1, 2 oder 3 steht.

2. Verbindung der Formel (Ia): 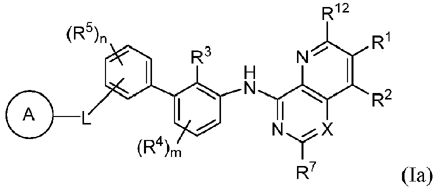 oder ein pharmazeutisch unbedenkliches Salz davon, wobei:
Ring A für 5- bis 10-gliedriges Heteroaryl, 4- bis 11-gliedriges Heterocycloalkyl, C₆₋₁₀-Aryl oder C₃₋₁₀-Cycloalkyl steht, wobei das 5- bis 10-gliedrige Heteroaryl und das 4- bis 11-gliedrige Heterocycloalkyl jeweils 1-4 Heteroatome als Ringglieder, die aus N, O und S ausgewählt sind, aufweisen, wobei das N- oder S-Atom als Ringglied gegebenenfalls oxidiert ist und ein oder mehrere Kohlenstoffatome als Ringglieder jeweils gegebenenfalls durch eine Carbonylgruppe ersetzt sind; und wobei Ring A gegebenenfalls durch 1, 2, 3, 4 oder 5 R⁶-Substituenten substituiert ist;
L für eine Bindung, -C(O)NR¹³-, -NR¹³C(O)-, O, -(CR¹⁴R¹⁵)_{q}-, -(CR¹⁴R¹⁵)_{q}-O-, -O(CR¹⁴R¹⁵)_{q}- , -NR¹³- , -(CR¹⁴R¹⁵)_{q}-NR¹³-, -NR¹³-(CR¹⁴R¹⁵)_{q}-, -CH=CH-, -C=C-, -SO₂NR¹³-, -NR¹³SO₂₋, -NR¹³C(O)O- oder -NR¹³C(O)NR¹³-steht;
X für N oder CR¹⁷ steht;
R³ für Methyl, Halogen, CN oder C₁₋₄-Halogenalkyl steht;
R⁴ für C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl oder -N(C₁₋₄-Alkyl)₂ steht;
R⁵ für C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl oder -N(C₁₋₄-Alkyl)₂ steht;
R⁶ aus H, Halogen, C₁-₆-Alkyl, C₂₋₆-Alkenyl, C₂₋₆-Alkinyl, C₁₋₆-Halogenalkyl, C₁₋₆-Halogenalkoxy, C₆₋₁₀-Aryl, C₃₋₁₀-Cycloalkyl, 5-14-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-14-gliedrigem Heteroaryl)-C₁₋₄-alkyl-, (4-10-gliedrigem Heterocycloalkyl) -C₁₋₄-alkyl-, CN, N0₂, OR^{a}, SR^{a}, NHOR^{a}, C(O)R^{a}, C(O)NR^{a}R^{a}, C(O)OR^{a}, OC(O)R^{a}, OC (O) NR^{a}R^{a}, NHR^{a}, NR^{a}R^{a}, NR^{a}C (O) R^{a}, NR^{a}C(O)OR^{a}, NR^{a}C (O) NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S (O) R^{a}, S (O) NR^{a}R^{a}, S (O)₂R^{a} und S (O)₂NR^{a}R^{a} ausgewählt ist, wobei das C₁-₆-Alkyl, C₂₋₆-Alkenyl, C₂₋₆-Alkinyl, C₆₋₁₀-Aryl, C₃₋₁₀-Cycloalkyl, 5-14-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-14-gliedrige Heteroaryl)-C₁₋₄-alkyl- und (4-10-gliedrige Heterocycloalkyl)-C₁₋₄-alkyl- von R⁶ gegebenenfalls durch 1, 2, 3, 4 oder 5 R^{b}-Substituenten substituiert ist;
oder zwei R⁶-Substituenten, die an dasselbe Ringkohlenstoffatom gebunden sind, zusammen mit dem Ringkohlenstoffatom, an das sie gebunden sind, C₃₋₆-Spirocycloalkyl oder 4- bis 7-gliedriges Spiroheterocycloalkyl bilden, wobei jede dieser Gruppen gegebenenfalls durch 1, 2 oder 3 unabhängig ausgewählte R^{f}-Substituenten substituiert ist;
R¹³ jeweils unabhängig für H, C₁₋₆-Halogenalkyl oder C₁-₆-Alkyl, das gegebenenfalls durch einen Substituenten, der aus C₁₋₄-Alkyl, C₁₋₄-Alkoxy, C₁₋₄-Halogenalkyl, C₁₋₄-Halogenalkoxy, CN, Halogen, OH, -COOH, NH₂, -NH-C₁₋₄-Alkyl und -N(C₁₋₄-Alkyl)₂ ausgewählt ist, substituiert ist, steht;
R¹⁴ und R¹⁵ jeweils unabhängig aus H, Halogen, CN, OH, -COOH, C₁₋₄-Alkyl, C₁₋₄-Alkoxy, -NH-C₁₋₄-Alkyl, -N(C₁₋₄-Alkyl)₂, C₁₋₄-Halogenalkyl, C₁₋₄-Halogen-alkoxy, C₃₋₆-Cycloalkyl, Phenyl, 5-6-gliedrigem Heteroaryl und 4-6-gliedrigem Heterocycloalkyl ausgewählt sind, wobei das C₁₋₄-Alkyl, C₁₋₄-Alkoxy, C₁₋₄-Halogenalkyl, C₁₋₄-Halogenalkoxy, C₃₋₆-Cycloalkyl, Phenyl, 5-6-gliedrige Heteroaryl und 4-6-gliedrige Heterocycloalkyl von R¹⁴ oder R¹⁵ jeweils gegebenenfalls durch 1, 2 oder 3 unabhängig ausgewählte R^{q}-Substituenten substituiert ist;
oder R¹⁴ und R¹⁵ zusammen mit dem Kohlenstoffatom, an das sie gebunden sind, 3-, 4-, 5- oder 6-gliedriges Cycloalkyl oder 3-, 4-, 5- oder 6-gliedriges Heterocycloalkyl bilden, wobei jede dieser Gruppen gegebenenfalls durch 1 oder 2 R^{q}-Substituenten substituiert ist;
R¹⁷ für H, C₁₋₄-Alkyl, C₁₋₄-Alkoxy, C₁₋₄-Halogenalkyl, C₁₋₄-Halogenalkoxy, CN, Halogen, OH, -COOH, -NH₂, -NH-C₁₋₄-Alkyl oder -N(C₁₋₄-Alkyl)₂ steht, wobei das C₁₋₄-Alkyl und C₁₋₄-Alkoxy jeweils gegebenenfalls durch 1 oder 2 Substituenten, die unabhängig aus CN, Halogen und -C(O)NH₂ ausgewählt sind, substituiert sind;
eines von R¹ und R² für -(CR⁸R⁹)ₚ-NR¹⁰R¹¹ steht und das andere für H, C₁₋₄-Alkyl, C₁₋₄-Alkoxy, C₁₋₄-Halogenalkyl, C₁₋₄-Halogenalkoxy, CN, Halogen, OH, -COOH, -NH₂, -NH-C₁₋₄-Alkyl oder -N(C₁₋₄-Alkyl)₂ steht, wobei das C₁₋₄-Alkyl und C₁₋₄-Alkoxy von R¹ oder R² gegebenenfalls durch 1 oder 2 Substituenten, die unabhängig aus C₁₋₄-Alkoxy, C₁₋₄-Halogenalkyl, C₁₋₄-Halogenalkoxy, CN, Halogen, OH, -COOH, -C(O)NH₂, -NH₂, -NH-C₁₋₄-Alkyl und -N(C₁₋₄-Alkyl) ₂ ausgewählt sind, substituiert sind;
R⁷ für H, C₁₋₄-Alkyl, C₁₋₄-Alkoxy, C₁₋₄-Halogenalkyl, C₁₋₄-Halogenalkoxy, CN, Halogen, OH, -COOH, -NH₂, -NH-C₁₋₄-Alkyl oder -N(C₁₋₄-Alkyl)₂ steht, wobei das C₁₋₄-Alkyl und C₁₋₄-Alkoxy jeweils gegebenenfalls durch 1 oder 2 Substituenten, die unabhängig aus CN, Halogen oder -C(O)NH₂ ausgewählt sind, substituiert sind;
R⁸ und R⁹ jeweils unabhängig aus H, Halogen, CN, OH, -COOH, C₁₋₄-Alkyl, C₁₋₄-Alkoxy, -NH-C₁₋₄-Alkyl, -N(C₁₋₄-Alkyl)₂, C₁₋₄-Halogenalkyl, C₁₋₄-Halogen-alkoxy, C₃₋₆-Cycloalkyl, Phenyl, 5-6-gliedrigem Heteroaryl und 4-6-gliedrigem Heterocycloalkyl ausgewählt sind, wobei das C₁₋₄-Alkyl, C₁₋₄-Alkoxy, C₁₋₄-Halogenalkyl, C₁₋₄-Halogenalkoxy, C₃₋₆-Cycloalkyl, Phenyl, 5-6-gliedrige Heteroaryl und 4-6-gliedrige Heterocycloalkyl von R⁸ oder R⁹ jeweils gegebenenfalls durch 1, 2 oder 3 unabhängig ausgewählte R^{q}-Substituenten substituiert sind;
oder R⁸ und R⁹ zusammen mit dem Kohlenstoffatom, an das sie gebunden sind, 3-, 4-, 5- oder 6-gliedriges Cycloalkyl oder 4-, 5-, 6- oder 7-gliedriges Heterocycloalkyl bilden, wobei jede dieser Gruppen gegebenenfalls durch 1 oder 2 R^{q}-Substituenten substituiert ist;
oder R⁸ und R¹⁰ zusammen mit den Atomen, an die sie gebunden sind, 4-, 5-, 6- oder 7-gliedriges Heterocycloalkyl mit null bis eins zusätzlichen Heteroatomen als Ringgliedern, die aus O, N und S ausgewählt sind, wobei das durch R⁸ und R¹⁰ gebildete 4-, 5-, 6- oder 7-gliedrige Heterocycloalkyl jeweils gegebenenfalls durch 1 oder 2 R^{q}-Substituenten substituiert ist;
R¹⁰ und R¹¹ jeweils unabhängig aus H, C₁₋₆-Alkyl, C₁₋₆-Halogenalkyl, C₃₋₆-Cycloalkyl, C₆₋₁₀-Aryl, 5-10-gliedrigem Heteroaryl, 4-10-gliedrigem Heterocycloalkyl, C₆₋₁₀-Aryl-C₁₋₄-alkyl-, C₃₋₆-Cycloalkyl-C₁₋₄-alkyl-, (5-10-gliedrigem Heteroaryl) -C₁₋₄-alkyl-, (4-10-gliedrigem Heterocycloalkyl)-C₁₋₄-alkyl-, -C(O)R^{g}, -C(O)OR^{g}, -C(O)NR^{g}R^{g}, -S(O)₂R^{g} und -S (0) ₂NR^{g}R^{g} ausgewählt sind, wobei das C₁₋₆-Alkyl, C₁₋₆-Halogenalkyl, C₃₋₆-Cycloalkyl, C₆₋₁₀-Aryl, 5-10-gliedrige Heteroaryl, 4-10-gliedrige Heterocycloalkyl, C₆₋₁₀-Aryl-C₁₋₄-alkyl-, C₃₋₆-Cycloalkyl-C₁₋₄-alkyl-, (5-10-gliedrige Heteroaryl)-C₁₋₄-alkyl-, (4-10-gliedrige Heterocycloalkyl)-C₁₋₄-alkyl-, von R¹⁰ und R¹¹ jeweils gegebenenfalls durch 1, 2 oder 3 unabhängig ausgewählte R^{d}-Substituenten substituiert sind;
oder R¹⁰ und R¹¹ zusammen mit dem Stickstoffatom, an das sie gebunden sind, 4-, 5-, 6-, 7-, 8-, 9-, 10-oder 11-gliedriges Heterocycloalkyl bilden, wobei das 4-11-gliedrige Heterocycloalkyl gegebenenfalls durch 1, 2 oder 3 R^{f}-Substituenten substituiert ist;
R¹² für H, C₁₋₄-Alkyl, C₁₋₄-Alkoxy, C₁₋₄-Halogenalkyl, C₁₋₄-Halogenalkoxy, CN, Halogen, OH, -COOH, -NH₂, -NH-C₁₋₄-Alkyl oder -N(C₁₋₄-Alkyl)₂ steht;
R^{a} jeweils unabhängig aus H, CN, C₁-₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁-₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl) -C₁₋₄-alkyl- und (4-10-gliedrige Heterocycloalkyl)-C₁₋₄-alkyl- von R^{a} jeweils gegebenenfalls durch 1, 2, 3, 4 oder 5 R^{d}-Substituenten substituiert sind;
R^{d} jeweils unabhängig aus C₁-₆-Alkyl, C₁₋₆-Halogenalkyl, Halogen, C₆₋₁₀-Aryl, 5-10-gliedrigem Heteroaryl, C₃₋₁₀-Cycloalkyl, 4-10-gliedrigem Heterocycloalkyl, C₆₋₁₀-Aryl-C₁₋₄ -alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, CN, NH₂, NHOR^{e}, OR^{e}, SR^{e}, C (O) R^{e}, C(O)NR^{e}R^{e}, C(O)OR^{e}, OC (O) R^{e}, OC(O)NR^{e}R^{e}, NHR^{e}, NR^{e}R^{e}, NR^{e}C (O) R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S (O) R^{e}, S(O)NR^{e}R^{e}, S(O)₂R^{e}, NR^{e}S(O)₂R^{e}, NR^{e}S(O)₂NR^{e}R^{e} und S(O)₂NR^{e}R^{e} ausgewählt ist, wobei das C₁-₆-Alkyl, C₁₋₆-Halogenalkyl, C₆₋₁₀-Aryl, 5-10-gliedrige Heteroaryl, C₃₋₁₀-Cycloalkyl, 4-10-gliedrige Heterocycloalkyl, C₆₋₁₀-Aryl-C₁₋₄-alkyl, C₃₋₁₀-Cycloalkyl-C₁₋₄-alkyl-, (5-10-gliedrige Heteroaryl)-C₁₋₄-alkyl und (4-10-gliedrige Heterocycloalkyl)-C₁₋₄-alkyl- von R^{d} jeweils gegebenenfalls durch 1, 2 oder 3 unabhängig ausgewählte R^{f}-Substituenten substituiert sind;
R^{e} jeweils unabhängig aus H, C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀)-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁-₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{e} jeweils gegebenenfalls durch 1, 2 oder 3 unabhängig ausgewählte R^{f}-Substituenten substituiert sind;
jeder R^{b}-Substituent unabhängig aus Halogen, C₁₋₆-Alkyl, C₂₋₆-Alkenyl, C₂₋₆-Alkinyl, C₁₋₆-Halogenalkyl, C₁₋₆-Halogenalkoxy, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄ -alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, CN, OH, NH₂, NO2, NHOR^{c}, OR^{c}, SR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C (O) R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} oder S(O)₂NR^{c}R^{c} ausgewählt ist, wobei das C₁₋₆-Alkyl, C₁₋₆-Halogenalkyl, C₁₋₆-Halogenalkoxy, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀)-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{b} jeweils gegebenenfalls durch 1, 2 oder 3 unabhängig ausgewählte R^{d}-Substituenten substituiert sind;
R^{c} jeweils unabhängig aus H, C₁-₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀)-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁-₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{c} jeweils gegebenenfalls durch 1, 2, 3, 4 oder 5 R^{f}-Substituenten substituiert sind;
R^{f} jeweils unabhängig aus C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀-Aryl-C₁₋₄alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl)-C₁₋₄-alkyl-, Halogen, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g} und S (O)₂NR^{g}R^{g} ausgewählt ist; wobei das C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄alkyl-, C₃₋₁₀-Cycloalkyl-C₁₋₄-alkyl-, (5-10-gliedrige Heteroaryl) -C₁₋₄-alkyl- und (4-10-gliedrige Heterocycloalkyl)-C₁₋₄-alkyl- von R^{f} jeweils gegebenenfalls durch 1, 2, 3, 4 oder 5 Rⁿ-Substituenten substituiert sind;
Rⁿ jeweils unabhängig aus C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀)-Aryl-C₁₋₄-alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, Halogen, CN, NHOR°, OR°, SR°, C(O)R°, C(O)NR°R°, C(O)OR°, OC(O)R°, OC(O)NR°R°, NHR°, NR°R°, NR°C (O) R°, NR°C (O) NR°R°, NR°C (O) OR°, C (=NR°) NR°R°, NR°C (=NR°) NR°R°, S (O) R°, S (O) NR°R°, S(O)₂R°, NR°S (O)₂R°, NR°S (O)₂NR°R° und S (O)₂NR°R° ausgewählt ist, wobei das C₁₋₄-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄alkyl-, C₃₋₁₀-Cycloalkyl-C₁₋₄-alkyl-, (5-10-gliedrige Heteroaryl) -C₁₋₄-alkyl- und (4-10-gliedrige Heterocycloalkyl)-C₁₋₄-alkyl- von Rⁿ jeweils gegebenenfalls durch 1, 2 oder 3 unabhängig ausgewählte R^{q}-Substituenten substituiert sind;
R^{g} jeweils unabhängig aus H, C₁-₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀)-Aryl-C₁₋₄-alkyl-, C₃₋₁₀-Cycloalkyl-C₁₋₄-alkyl-, (5-10-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄-alkyl-, C₃₋₁₀-Cycloalkyl-C₁₋₄-alkyl-, (5-10-gliedrige Heteroaryl)-C₁₋₄-alkyl- und (4-10-gliedrige Heterocycloalkyl)-C₁₋₄-alkyl- von R^{g} jeweils gegebenenfalls durch 1, 2 oder 3 R^{p}-Substituenten substituiert sind,
R^{p} jeweils unabhängig aus C₁-₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrigem Heteroaryl, 4-10-gliedrigem Heterocycloalkyl, C₆₋₁₀)-Aryl-C₁₋₄-alkyl-, C₃₋₁₀-Cycloalkyl-C₁₋₄-alkyl-, (5-10-gliedrigem Heteroaryl)-C₁₋₄-alkyl-, (4-10-gliedrigem Heterocycloalkyl) -C₁₋₄-alkyl-, Halogen, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} und S(O)₂NR^{r}R^{r} ausgewählt ist, wobei das C₁₋₆-Alkyl, C₁₋₆-Halogenalkyl, C₂₋₆-Alkenyl, C₂₋₆-Alkinyl, C₆₋₁₀-Aryl, C₃₋₁₀-Cycloalkyl, 5-10-gliedrige Heteroaryl, 4-10-gliedrige Heterocycloalkyl, C₆₋₁₀-Aryl-C₁₋₄ -alkyl-, C₃₋₁₀-Cycloalkyl-C₁₋₄-alkyl-, (5-10-gliedrige Heteroaryl) -C₁₋₄-alkyl- und (4-10-gliedrige Heterocycloalkyl)-C₁₋₄-alkyl- von R^{p} jeweils gegebenenfalls durch 1, 2 oder 3 R^{q}-Substituenten substituiert sind;
oder beliebige zwei R^{a}-Substituenten zusammen mit dem Stickstoffatom, an das sie gebunden sind, eine 4-, 5-, 6-, 7-, 8-, 9- oder 10-gliedrige Heterocycloalkylgruppe bilden, die gegebenenfalls durch 1, 2 oder 3 R^{h}-Substituenten substituiert ist;
R^{h} jeweils unabhängig aus C₁-₆-Alkyl, C₃₋₁₀-Cycloalkyl, 4-7-gliedrigem Heterocycloalkyl, C₆₋₁₀-Aryl, 5-6-gliedrigem Heteroaryl, C₆₋₁₀-Aryl-C₁₋₄-alkyl-, C₃₋₁₀-Cycloalkyl-C₁₋₄-alkyl-, (5-6-gliedrigem Heteroaryl)-C₁₋₄-alkyl-, (4-7-gliedrigem Heterocycloalkyl) -C₁₋₄-alkyl-, C₁₋₆-Halogenalkyl, C₁₋₆-Halogenalkoxy, C₂₋₆-Alkenyl, C₂₋₆-Alkinyl, Halogen, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ und S(O)₂NRⁱRⁱ ausgewählt ist, wobei das C₁-₆-Alkyl, C₂₋₆-Alkenyl, C₂₋₆-Alkinyl, C₃₋₁₀-Cycloalkyl, 4-7-gliedrige Heterocycloalkyl, C₆₋₁₀-Aryl, 5-6-gliedrige Heteroaryl, C₆₋₁₀-Aryl-C₁₋₄-alkyl-, C₃₋₁₀-Cycloalkyl-C₁₋₄-alkyl-, (5-6-gliedrige Heteroaryl)-C₁₋₄-alkyl- und (4-7-gliedrige Heterocycloalkyl)-C₁₋₄-alkyl- von R^{h} jeweils ferner gegebenenfalls durch 1, 2 oder 3 R^{j}-Substituenten substituiert sind;
R^{j} jeweils unabhängig aus C₃₋₆-Cycloalkyl, C₆₋₁₀-Aryl, 5- oder 6-gliedrigem Heteroaryl, 4-7-gliedrigem Heterocycloalkyl, C₂₋₄-Alkenyl, C₂₋₄-Alkinyl, Halogen, C₁₋₄-Alkyl, C₁₋₄-Halogenalkyl, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k} und S(O)₂NR^{k}R^{k} ausgewählt ist; wobei das C₁₋₄-Alkyl, C₃₋₆-Cycloalkyl, C₆₋₁₀-Aryl, 5- oder 6-gliedrige Heteroaryl, 4-6-gliedrige Heterocycloalkyl, C₂₋₄-Alkenyl, C₂₋₄-Alkinyl, C₁₋₄-Halogenalkyl und C₁₋₄-Halogenalkoxy von R^{j} jeweils ferner gegebenenfalls durch 1, 2 oder 3 unabhängig ausgewählte R^{q}-Substituenten substituiert sind;
oder zwei R^{h}-Gruppen, die an dasselbe Kohlenstoffatom des 4- bis 10-gliedrigen Heterocycloalkyls gebunden sind, zusammen mit dem Kohlenstoffatom, an das sie gebunden sind, ein C₃-₆-Cycloalkyl oder 4- bis 6-gliedriges Heterocycloalkyl mit 1-2 Heteroatomen als Ringgliedern, die aus O, N und S ausgewählt sind, bilden;
oder beliebige zwei R^{c}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{e}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{g}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{o}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
oder beliebige zwei R^{r}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
Rⁱ, R^{k}, R^{o} oder R^{r} jeweils unabhängig aus H, C₁₋₄-Alkyl, C₃₋₆-Cycloalkyl, C₆₋₁₀-Aryl, 5- oder 6-gliedrigem Heteroaryl, 4-7-gliedrigem Heterocycloalkyl, C₁₋₆-Halogenalkyl, C₁₋₆-Halogenalkoxy, C₂₋₄-Alkenyl und C₂₋₄-Alkinyl ausgewählt ist, wobei das C₁₋₄-Alkyl, C₃₋₆-Cycloalkyl, C₆₋₁₀-Aryl, 5- oder 6-gliedrige Heteroaryl, 4-7-gliedrige Heterocycloalkyl, C₂₋₄-Alkenyl und C₂₋₄-Alkinyl von Rⁱ, R^{k}, R^{o} oder R^{r} jeweils gegebenenfalls durch 1, 2 oder 3 R^{q}-Substituenten substituiert sind;
R^{q} jeweils unabhängig aus Halogen, OH, CN, -COOH, NH₂, -NH-C₁₋₆-Alkyl, -N (C₁₋₆-Alkyl)₂, C₁-₆-Alkyl, C₁₋₆-Alkoxy, C₁₋₆-Alkyl thio, C₁₋₆-Halogenalkyl, C₁₋₆-Halogenalkoxy, Phenyl, 5-6-gliedrigem Heteroaryl, 4-6-gliedrigem Heterocycloalkyl und C₃-₆-Cycloalkyl ausgewählt ist, wobei das C₁-₆-Alkyl, Phenyl, C₃₋₆-Cycloalkyl, 4-6-gliedrige Heterocycloalkyl und 5-6-gliedrige Heteroaryl von R^{q} jeweils gegebenenfalls durch 1, 2 oder 3 Substituenten substituiert sind, die aus Halogen, OH, CN, -COOH, NH₂, C₁₋₄-Alkyl, C₁₋₄-Alkoxy, C₁₋₄-Halogenalkyl, C₁₋₄-Halogen-alkoxy, Phenyl, C₃₋₁₀-Cycloalkyl, 5-6-gliedrigem Heteroaryl und 4-6-gliedrigem Heterocycloalkyl ausgewählt sind;
der tiefgestellte Index m für eine ganze Zahl mit einem Wert von 0, 1, 2 oder 3 steht;
der tiefgestellte Index n für eine ganze Zahl mit einem Wert von 0, 1, 2 oder 3 steht;
der tiefgestellte Index q jeweils für eine ganze Zahl mit einem Wert von 1, 2, 3 oder 4 steht und
der tiefgestellte Index p für eine ganze Zahl mit einem Wert von 1, 2, 3 oder 4 steht.

3. Verbindung nach Anspruch 2 mit:
(a) Formel (II): 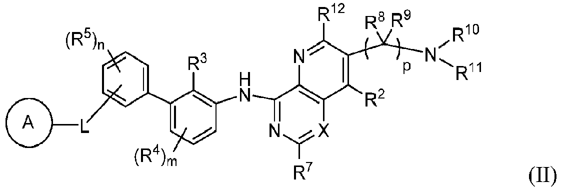 oder ein pharmazeutisch unbedenkliches Salz davon; oder
(b) Formel (IIa): 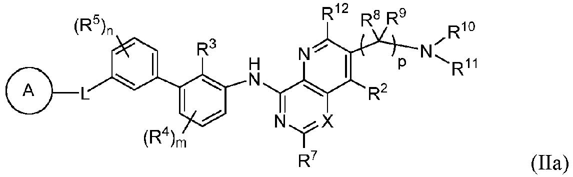 oder ein pharmazeutisch unbedenkliches Salz davon; oder
(c) Formel (IIb): 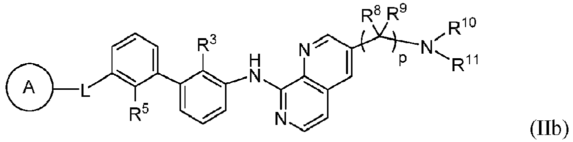 oder ein pharmazeutisch unbedenkliches Salz davon.

4. Verbindung der Formel 2 mit:
(a) Formel (III): 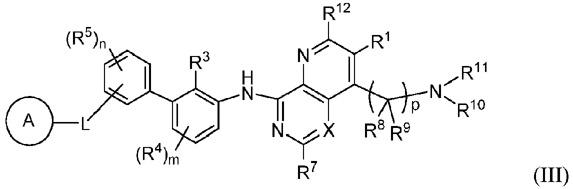 oder ein pharmazeutisch unbedenkliches Salz davon; oder
(b) Formel (IIIa): 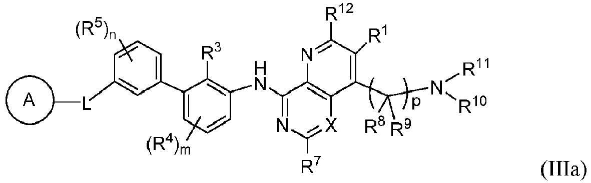 oder ein pharmazeutisch unbedenkliches Salz davon; oder
(c) Formel (IIIb): 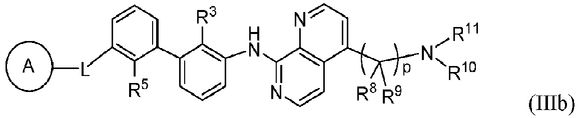 oder ein pharmazeutisch unbedenkliches Salz davon.

5. Verbindung nach einem der Ansprüche 1-4 oder ein pharmazeutisch unbedenkliches Salz davon, wobei Ring A aus 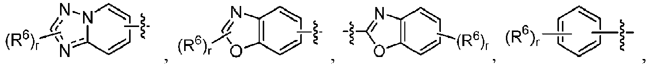 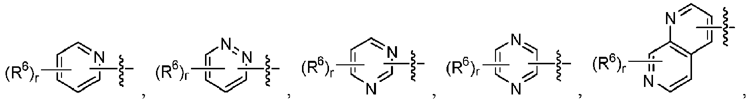 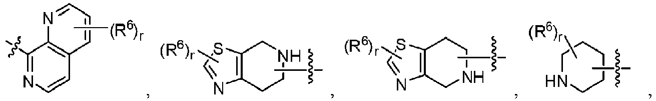 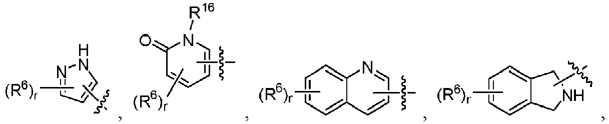 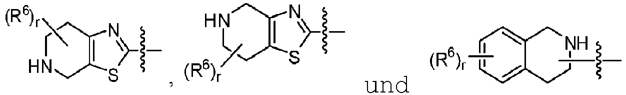 ausgewählt ist, wobei jeder tiefgestellte Index r für eine ganze Zahl mit einem Wert von 0, 1, 2, 3, 4 oder 5 steht; R¹⁶ für C₁₋₆-Alkyl steht und die Wellenlinie den Punkt der Verknüpfung mit L anzeigt.

6. Verbindung nach einem der Ansprüche 1-5 oder ein pharmazeutisch unbedenkliches Salz davon, wobei L für eine Bindung, -NH-, -CH=CH- oder -C(O)NH-steht, wobei die Carbonylgruppe in der -C(O)NH-Verknüpfung an Ring A gebunden ist.

7. Verbindung nach einem der Ansprüche 1-6 oder ein pharmazeutisch unbedenkliches Salz davon, wobei der tiefgestellte Index m für 0 steht.

8. Verbindung nach einem der Ansprüche 1-7 oder ein pharmazeutisch unbedenkliches Salz davon, wobei der tiefgestellte Index n für 1 steht und R⁵ für Halogen oder C₁₋₄-Alkyl steht.

9. Verbindung nach einem der Ansprüche 1-8 oder ein pharmazeutisch unbedenkliches Salz davon, wobei R³ für Methyl, CN oder Cl steht.

10. Verbindung nach einem der Ansprüche 2-9 oder ein pharmazeutisch unbedenkliches Salz davon, wobei R¹² für H, Halogen, CN, C₁₋₄-Alkyl oder C₁₋₄-Alkoxy steht.

11. Verbindung nach einem der Ansprüche 2-10 oder ein pharmazeutisch unbedenkliches Salz davon, wobei R⁷ für H, Halogen, CN, C₁₋₄-Alkyl, C₁₋₄-Alkoxy oder C₁₋₄-Halogenalkoxy steht, wobei das C₁₋₄-Alkyl und C₁₋₄-Alkoxy von R⁷ jeweils gegebenenfalls durch CN substituiert sind.

12. Verbindung nach einem der Ansprüche 2-11 oder ein pharmazeutisch unbedenkliches Salz davon, wobei R² für H steht.

13. Verbindung nach einem der Ansprüche 2-12 oder ein pharmazeutisch unbedenkliches Salz davon, wobei R¹ für H steht.

14. Verbindung nach einem der Ansprüche 2-13 oder ein pharmazeutisch unbedenkliches Salz davon, wobei der tiefgestellte Index p für 1 steht.

15. Verbindung nach einem der Ansprüche 2-14 oder ein pharmazeutisch unbedenkliches Salz davon, wobei R⁸ und R⁹ jeweils für H stehen.

16. Verbindung nach einem der Ansprüche 2-15 oder ein pharmazeutisch unbedenkliches Salz davon, wobei R¹⁰ für H steht.

17. Verbindung nach einem der Ansprüche 2-16 oder ein pharmazeutisch unbedenkliches Salz davon, wobei R¹¹ für 2-Hydroxyethyl, [1-(Hydroxymethyl)cyclopropyl]methyl, [1-(Hydroxymethyl)cyclobutyl]methyl oder 2-(Dimethylamino)-2-oxoethyl steht.

18. Verbindung nach einem der Ansprüche 2-15 oder ein pharmazeutisch unbedenkliches Salz davon, wobei -NR¹⁰R¹¹ für (2-Hydroxyethyl) amino, 3-Hydroxy-pyrrolidin-1-yl, 3-Carboxypyrrolidin-1-yl, 3-Carboxyazetidin-1-yl, 2-Carboxy-1-piperidinyl, 2-Oxooxazolidin-3-yl, [1-(Hydroxymethyl)cyclo-propyl]methylamino, [1-(Hydroxymethyl)cyclobutyl]-methylamino oder [2-(Dimethylamino)-2-oxoethyl]-amino steht.

19. Verbindung nach einem der Ansprüche 1-18 oder ein pharmazeutisch unbedenkliches Salz davon, wobei Ring A für 2-Pyridyl, das gegebenenfalls durch 1, 2, 3 oder 4 unabhängig ausgewählte R⁶-Substituenten substituiert ist, steht.

20. Verbindung nach einem der Ansprüche 1-19 oder ein pharmazeutisch unbedenkliches Salz davon, wobei X für N oder CH steht.

21. Verbindung nach Anspruch 1, ausgewählt aus:
2-(((8-((2-Chlor-2'-methyl-3'-(4,5,6,7-tetrahydrothiazolo[5,4-c]pyridin-2-yl)-[1,1'-biphenyl]-3-yl)amino)-1,7-naphthyridin-3-yl)methyl) amino)ethan-1-ol; 1-(((6-(2-Fluor-3'-(3-((2-hydroxyethylamino)methyl)-1,7-naphthyridin-8-ylamino)-2'-methylbiphenyl-3-ylcarbamoyl)pyridin-3-yl)methyl) amino)cyclobutancarbonsäure; (S)-1-((6-((2-Fluor-3'-((3-(((2-hydroxyethyl)amino)methyl)-1,7-naphthyridin-8-yl)amino)-2'-methyl-[1,1'-biphenyl]-3-yl)carbamoyl)pyridin-3-yl)methyl)piperidin-2-carbonsäure;
N-(2-Fluor-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamid; N-(2-Chlor-3'-((3-(((2-hydroxyethyl)amino)methyl)-1,7-naphthyridin-8-yl)amino)-2'-methyl-[1,1'-biphenyl]-3-yl)-5-(((2-hydroxyethyl)amino)methyl)picolinamid; N-(2-Chlor-3'-((3-(((R)-3-hydroxypyrrolidin-1-yl)methyl)-1,7-naphthyridin-8-yl)amino)-2'-methyl-[1,1'-biphenyl]-3-yl)-5-(((R)-3-hydroxypyrrolidin-1-yl)methyl)picolinamid;
(R)-1-((8-((2'-Chlor-3'-(5-(((R)-3-hydroxypyrrolidin-1-yl)methyl)picolinamido)-2-methyl-[1,1'-biphenyl]-3-yl)amino)-1,7-naphthyridin-3-yl)methyl)pyrrolidin-3-carbonsäure und
(R)-1-((8-((2'-Chlor-3'-(5-((3-hydroxypyrrolidin-1-yl)methyl)picolinamido)-2-methyl-[1,1'-biphenyl]-3-yl)amino)-1,7-naphthyridin-3-yl)methyl)azetidin-3-carbonsäure;
oder ein pharmazeutisch unbedenkliches Salz davon.

22. Verbindung nach Anspruch 1, ausgewählt aus:
(R)-1-((8-((3'-((3-(((R)-3-Hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidin-3-carbonsäure; (S)-1-((8-((3'-((3-(((R)-3-Hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidin-3-carbonsäure; (R)-1-((8-((3'-((3-(((2-Hydroxyethyl)amino)methyl)-1,7-naphthyridin-8-yl)amino)-2,2'-dimethyl-[1,1'-biphenyl]-3-yl)amino)-1,7-naphthyridin-3-yl)methyl)pyrrolidin-3-carbonsäure;
(R)-1-((8-((3'-((3-(((S)-3-Hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidin-3-carbonsäure; and
(S)-1-((8-((3'-((3-(((S)-3-Hydroxypyrrolidin-1-yl)methyl)-1,7-naphthyridin-8-yl)amino)-2,2'-dimethyl-[1,1'-biphenyl]-3-yl)amino)-1,7-naphthyridin-3-yl)methyl)pyrrolidin-3-carbonsäure; oder ein pharmazeutisch unbedenkliches Salz davon.

23. Verbindung nach Anspruch 1, ausgewählt aus:
1-((8-(2-Chlor-3'-(1,5-dimethyl-4,5,6,7-tetrahydro-1H-imidazo[4,5-c]pyridin-2-carboxamido)-2'-methylbiphenyl-3-ylamino)-1,7-naphthyridin-3-yl)methyl)azetidin-3-carbonsäure;
(R)-1-((5-(2-Chlor-3'-(3-(((R)-3-hydroxypyrrolidin-1-yl)methyl)-1,7-naphthyridin-8-ylamino)-2'-methylbiphenyl-3-ylcarbamoyl)-1-methyl-6-oxo-1,6-dihydropyridin-3-yl)methyl)pyrrolidin-3-carbonsäure;
(R)-1-((8-(2,2'-Dichlor-3'-(5-((3-hydroxypyrrolidin-1-yl)methyl)-1-methyl-2-oxo-1,2-dihydropyridin-3-carboxamido)biphenyl-3-ylamino)-1,7-naphthyridin-3-yl)methyl)azetidin-3-carbonsäure;
1-((8-(2,2'-Dichlor-3'-(5-((2-hydroxyethylamino)methyl)-1-methyl-2-oxo-1,2-dihydropyridin-3-carboxamido)biphenyl-3-ylamino)-1,7-naphthyridin-3-yl)methyl)azetidin-3-carbonsäure;
1-((8-(2,2'-Dichlor-3'-(5-((2-hydroxy-2-methylpropylamino)methyl)-1-methyl-2-oxo-1,2-dihydropyridin-3-carboxamido)biphenyl-3-ylamino)-1,7-naphthyridin-3-yl)methyl)azetidin-3-carbonsäure;
2,2'-(((((2,2'-Dimethyl-[1,1'-biphenyl]-3,3'-diyl)bis(azandiyl))bis(1,7-naphthyridin-8,3-diyl))bis(methylen))bis(azandiyl))bis(ethan-1-ol);
(3*R*,3'*R*)-1,1'-((((2,2'-Dimethyl-[1,1'-biphenyl]-3,3'-diyl)bis(azandiyl))bis(1,7-naphthyridin-8,3-diyl))bis(methylen))bis(pyrrolidin-3-ol);
(*R*)-1-((8-(3'-(3-((2-Hydroxyethylamino)methyl)-1,7-naphthyridin-8-ylamino)-2,2'-dimethylbiphenyl-3-ylamino)-1,7-naphthyridin-3-yl)methyl)pyrrolidin-3-ol;
(3*R*,3'*R*)-1,1'-((((2,2'-Dichlor-[1,1'-biphenyl]-3,3'-diyl)bis(azandiyl))bis(1,7-naphthyridin-8,3-diyl))bis(methylen))bis(pyrrolidin-3-ol);
(R)-1-((4-(3'-(3-(((R)-3-Hydroxypyrrolidin-l-yl)methyl)-1,7-naphthyridin-8-ylamino)-2,2'-dimethylbiphenyl-3-ylamino)pyrido[3,2-d]pyrimidin-7-yl)methyl)pyrrolidin-3-ol;
(*R*)-1-((8-(3'-(7-((2-Hydroxyethylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(7-(((2-Hydroxyethyl)(methyl)amino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(3-(((*R*)-3-Hydroxypyrrolidin-1-yl)methyl)-1,7-naphthyridin-8-ylamino)-2,2'-dimethylbiphenyl-3-ylamino)-1,7-naphthyridin-3-yl)methyl)-N,N- dimethylpyrrolidin-3-carboxamid;
(*R*)-1-((8-(3'-(7-(((*S*)-2-Hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(5-(((*R*)-3-Hydroxypyrrolidin-1-yl)methyl)-1-methyl-2-oxo-1,2-dihydropyridin-3-carboxamido)-2,2'-dimethylbiphenyl-3-ylamino)-1,7-naphthyridin-3-yl)methyl)pyrrolidin-3-carbonsäure;
(*R*)-1-((8-(3'-(7-(((*R*)-2-Hydroxypropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(3'-(7-((2-Hydroxy-2-methylpropylamino)methyl)pyrido[3,2-d]pyrimidin-4-ylamino)-2,2'-dimethylbiphenyl-3-ylamino)-1,7-naphthyridin-3-yl)methyl)pyrrolidin-3-ol;
(*R*)-1-((8-(2'-Chlor-3'-(1,5-dimethyl-4,5,6,7-tetrahydro-1H-imidazo[4,5-c]pyridin-2-carboxamido)-2-methylbiphenyl-3-ylamino)-1,7-naphthyridin-3-yl)methyl)pyrrolidin-3-carbonsäure;
(*S*)-N-(2-Chlor-3'-(3-((3-hydroxypyrrolidin-1-yl)methyl)-1,7-naphthyridin-8-ylamino)-2'-methylbiphenyl-3-yl)-1,5-dimethyl-4,5,6,7-tetrahydro-1H-imidazo[4,5-c]pyridin-2-carboxamid;
(*R*)-1-((8-(2'-Chlor-3'-(1,5-dimethyl-4,5,6,7-tetrahydro-1H-imidazo[4,5-c]pyridin-2-carboxamido)-2-methylbiphenyl-3-ylamino)-1,7-naphthyridin-3-yl)methyl)-3-methylpyrrolidin-3-carbonsäure;
(*R*)-1-((8-((2,2'-Dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-carbonsäure;
(*R*)-1-((8-((2,2'-Dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol;
(*S*)-1-((8-((2,2'-Dimethyl-3'-(4,5,6,7-tetrahydrothiazolo[5,4-c]pyridin-2-yl)-[1,1'-biphenyl]-3-yl)amino)-1,7-naphthyridin-3-yl)methyl)pyrrolidin-3-ol;
(*R*)-2-(Dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-on;
(*S*)-2-(Dimethylamino)-1-(2-(3'-((3-((3-hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)ethan-1-on;
(*R*)-1-(2-(2-(3'-((3-((3-Hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)azetidin-3-carbonsäure;
(*S*)-1-(2-(2-(3'-((3-(((*R*)-3-Hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidin-3-carbonsäure;
(*R*)-1-(2-(2-(3'-((3-(((*R*)-3-Hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)pyrrolidin-3-carbonsäure;
(*S*)-1-(2-(2-(3'-((3-(((*R*)-3-Hydroxypyrrolidin-1-yl)methyl)-1,7-naphthyridin-8-yl)amino)-2,2'-dimethyl-[1,1'-biphenyl]-3-yl)-4,6-dihydro-5H-pyrrolo[3,4-d]oxazol-5-yl)-2-oxoethyl)piperidin-2-carbonsäure;
(*S*)-1-(5-Chlor-2-((5-cyanopyridin-3-yl)methoxy)-4-((3'-((3-(((*R*)-3-hydroxypyrrolidin-1-yl)methyl)-1,7-naphthyridin-8-yl)amino)-2,2'-dimethyl-[1,1'-biphenyl]-3-yl)methoxy)benzyl)piperidin-2-carbonsäure und
(*R*)-1-(5-Chlor-2-((5-cyanopyridin-3-yl)methoxy)-4-((3'-((3-(((*R*)-3-hydroxypyrrolidin-1-yl)methyl)-1,7-naphthyridin-8-yl)amino)-2,2'-dimethyl-[1,1'-biphenyl]-3-yl)methoxy)benzyl)pyrrolidin-3-carbonsäure;
oder ein pharmazeutisch unbedenkliches Salz davon.

24. Verbindung nach Anspruch 1, ausgewählt aus:
(*R*)-1-((8-(2'-Chlor-2-methyl-3'-(1-methyl-4,5,6,7-tetrahydro-1H-imidazo[4,5-c]pyridin-2-carboxamido)biphenyl-3-ylamino)-1,7-naphthyridin-3-yl)methyl)-3-methylpyrrolidin-3-carbonsäure;
(*R*)-1-((8-(3'-(1,5-Dimethyl-4,5,6,7-tetrahydro-1H-imidazo[4,5-c]pyridin-2-carboxamido)-2,2'-dimethylbiphenyl-3-ylamino)-1,7-naphthyridin-3-yl)methyl)pyrrolidin-3-carbonsäure;
*trans-4-((2-(2-Chlor-3'-(3-(((R)-3-*hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexancarbonsäure;
*cis-4-((2-(2-Chlor-3'-(3-(((R)-3-*hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexancarbonsäure;
*cis-4-*((2-(2-Chlor-2'-methyl-3'-(3-(pyrrolidin-1-ylmethyl)-1,7-naphthyridin-8-ylamino)biphenyl-3-ylcarbamoyl)-1-methyl-6,7-dihydro-1H-imidazo[4,5-c]pyridin-5(4H)-yl)methyl)cyclohexancarbonsäure;
*trans*-4-((2-(2-Chlor-3'-(3-(((*S*)-1-hydroxypropan-2-ylamino)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)methyl)cyclohexancarbonsäure;
trans-4-((2-(2-Chlor-3'-(3-(((1S,2S)-2-hydroxycyclopentylamino)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)methyl)cyclohexancarbonsäure;
*trans-4-(2-(2-(2-Chlor-3'-(3-(((R)-3-*hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)ethyl)cyclohexancarbonsäure;
*cis*-4-(2-(2-(2-Chlor-3'-(3-(((R)-3-hydroxypyrrolidin-1-yl)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexancarbonsäure;
3-(2-(2-Chlor-3'-(3-(((*R*)-3-hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-*c*]pyridin-5(4*H*)-yl)butan-säure;
*cis-4-((2-(2-Chlor-3'-(3-(((S)-3-*hydroxypyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)methyl)cyclohexancarbonsäure;
*cis*-4-((2-(2-Chlor-3'-(3-(((R)-3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)methyl)cyclohexancarbonsäure;
(*R*)-4-(2-(2-Chlor-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)cyclohexancarbonsäure;
(*S*)-4-(2-(2-Chlor-3'-(3-((3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-c]pyridin-5(4*H*)-yl)cyclohexancarbonsäure;
*trans-4*-(2-(2-(2-Chlor*-*3*'*-(3-((*(R*)-1-hydroxypropan-2-ylamino)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexancarbonsäure;
*trans-*4-(2-(2-(2-Chlor-3'-(3-(((5)-1-hydroxypropan-2-ylamino)methyl)-1,7-naphthyridin-8-ylamino)-2'-methylbiphenyl-3-ylcarbamoyl)-1-methyl-6,7-dihydro-1H-imidazo[4,5-c]pyridin-5(4H)-yl)ethyl)cyclohexancarbonsäure;
*trans*-4-(2-(2-(2-Chlor-3'-(3-(((*R*)-3-hydroxy-3-methylpyrrolidin-1-yl)methyl)-1,7-naphthyridin-8-ylamino)-2'-methylbiphenyl-3-ylcarbamoyl)-1-methyl-6,7-dihydro-1*H*-imidazo[4,5-*c*]pyridin-5(4H)-yl)ethyl)cyclohexancarbonsäure;
(R)-4-(2-(3'-(3-((3-Hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridin-5(4*H*)-yl)-1-methylcyclohexancarbonsäure;
*trans*-4-(2-(2-(3'-(3-(((*R*)-3-Hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-c]pyridin-5(4*H*)-yl)ethyl)cyclohexancarbonsäure;
(*R*)-4-(2-(3'-(3-((3-Hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-c]pyridin-5(4*H*)-yl)cyclohexancarbonsäure;
*trans*-4-(2-(2-(2,2'-Dichlor-3'-(3-(((*R*)-3-hydroxypyrrolidin-1-yl)methyl)-1,7-naphthyridin-8-ylamino)biphenyl-3-ylcarbamoyl)-1-methyl-6,7-dihydro-1*H*-imidazo[4,5-c]pyridin-5(4*H*)-yl)ethyl)cyclohexancarbonsäure;
*trans-*4-(2-(2-(2'-Chlor-3'-(3-(((*R*)-3-hydroxypyrrolidin-1-yl)methyl)-1,7-naphthyridin-8-ylamino)-2-methylbiphenyl-3-ylcarbamoyl)-1-methyl-6,7-dihydro-1*H*-imidazo[4,5-*c*]pyridin-5(4*H*)-yl)ethyl)cyclohexancarbonsäure;
(*R*)-1-((4-(2'-Chlor-3'-(1,5-dimethyl-4,5,6,7-tetrahydro-1*H*-imidazo[4,5-*c*]pyridin-2-carboxamido)-2-methylbiphenyl-3-ylamino)pyrido[3,2-d]pyrimidin-7-yl)methyl)-3-methylpyrrolidin-3-carbonsäure;
(*R*)-4-(2-(2-Chlor-3'-(7-((3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1*H*-imidazo[4,5-*c*]pyridin-5(4*H*)-yl)-1-methylcyclohexancarbonsäure;
*trans*-4-((2-(2-Chlor-3'-(7-(((*R*)-3-hydroxypyrrolidin-1-yl)methyl)pyrido[3,2-d]pyrimidin-4-ylamino)-2'-methylbiphenyl-3-ylcarbamoyl)-1-methyl-6,7-dihydro-1*H*-imidazo[4,5-c]pyridin-5(4H)-yl)methyl)cyclohexancarbonsäure;
(*R*)-1-((5-(3'-(3-(((*R*)-3-Hydroxypyrrolidin-1-yl)methyl)-1,7-naphthyridin-8-ylamino)-2,2'-dimethylbiphenyl-3-ylamino)pyrido[4,3-*b*]pyrazin-2-yl)methyl)pyrrolidin-3-carbonsäure;
(3*R*)-1-((8-(2,2'-Dimethyl-3'-(3-(pyrrolidin-2-yl)-1,7-naphthyridin-8-ylamino)biphenyl-3-ylamino)-1,7-naphthyridin-3-yl)methyl)pyrrolidin-3-carbonsäure;
(*R*)-1-((8-(2,2'-Dichlor-3'-(3-((2-hydroxyethylamino)methyl)imidazo[1,2-a]pyrazin-8-ylamino)biphenyl-3-ylamino)-1,7-naphthyridin-3-yl)methyl)pyrrolidin-3-ol;
(*R*)-1-((8-((2,2'-Dimethyl-3'-((3-(pyrrolidin-1-ylmethyl)-1,7-naphthyridin-8-yl)amino)-[1,1'-biphenyl]-3-yl)amino)-1,7-naphthyridin-3-yl)methyl)pyrrolidin-3-ol;
(*S*)-1-((8-((2'-Chlor-3'-((3-(((*R*)-3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)pyrrolidin-3-ol;
(*R*)-1-((8-((2'-Chlor-3'-((3-((3-hydroxypyrrolidin-1-yl)methyl)-1,7-naphthyridin-8-yl)amino)-2-methyl-[1,1'-biphenyl]-3-yl)amino)-1,7-naphthyridin-3-yl)methyl)azetidin-3-carbonsäure;
(*R*)-1-((8-((2,2'-Dichlor-3'-((3-(((*R*)-3-hydroxypyrrolidin-1-yl)methyl)-1,7-naphthyridin-8-yl)amino)-[1,1'-biphenyl]-3-yl)amino)-1,7-naphthyridin-3-yl)methyl)-3-methylpyrrolidin-3-ol;
(*R*)-1-((8-((2,2'-Dichlor-3'-((3-(((2-hydroxyethyl)amino)methyl)-1,7-naphthyridin-8-yl)amino)-[1,1'-biphenyl]-3-yl)amino)-1,7-naphthyridin-3-yl)methyl)pyrrolidin-3-ol;
(*R*)-1-((8-((2,2'-Dichlor-3'-((3-(((*R*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidin-3-carbonsäure;
(*R*)-1-((8-((2-Chlor-3'-((3-(((*R*)-3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)pyrrolidin-3-carbonsäure;
(*R*)-1-((8-((2-Chlor-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)azetidin-3-carbonsäure;
(*R*)-3-(((8-((2-Chlor-3'-((3-((3-hydroxypyrrolidin-1-yl)methyl)-1,7-naphthyridin-8-yl)amino)-2'-methyl-[1,1'-biphenyl]-3-yl)amino)-1,7-naphthyridin-3-yl)methyl)amino)propansäure;
(*R*)-1-((8-((2,2'-Dichlor-3'-((3-(((*S*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidin-3-carbonsäure;
(*S*)-1-((8-((2,2'-Dichlor-3'-((3-(((*S*)-3-hydroxypyrrolidin-1-yl)methyl)-1,7-naphthyridin-8-yl)amino)-[1,1'-biphenyl]-3-yl)amino)-1,7-naphthyridin-3-yl)methyl)pyrrolidin-3-carbonsäure;
(*R*)-1-((8-((3'-(5-(Dimethylglycyl)-5,6-dihydro-4*H-*pyrrolo[3,4-d]oxazol-2-yl)-2,2'-dimethyl-[1,1'-biphenyl]-3-yl)amino)-1,7-naphthyridin-3-yl)methyl)pyrrolidin-3-carbonsäure;
(*R*)-1-((8-((3'-(5-(Dimethylglycyl)-5,6-dihydro-4*H-*pyrrolo[3,4-d]thiazol-2-yl)-2,2'-dimethyl-[1,1'-biphenyl]-3-yl)amino)-1,7-naphthyridin-3-yl)methyl)pyrrolidin-3-carbonsäure;
2-((*R*)-3-Hydroxypyrrolidin-1-yl)-1-(2-(3'-((3-(((*R*)-3-hydroxypyrrolidin-1-yl)methyl)-1,7-naphthyridin-8-yl)amino)-2,2'-dimethyl-[1,1'-biphenyl]-3-yl)-4,6-dihydro-5*H*-pyrrolo[3,4-d]thiazol-5-yl)ethan-1-on;
(*R*)-1-((8-((3'-(5-(Dimethylglycyl)-5,6-dihydro-4*H-*pyrrolo[3,4-d]thiazol-2-yl)-2,2'-dimethyl-[1,1'-biphenyl]-3-yl)amino)-1,7-naphthyridin-3-yl)methyl)-3-methylpyrrolidin-3-carbonsäure;
1-((8-((3'-(5-(Dimethylglycyl)-5,6-dihydro-4*H-*pyrrolo[3,4-d]thiazol-2-yl)-2,2'-dimethyl-[1,1'-biphenyl]-3-yl)amino)-1,7-naphthyridin-3-yl)methyl)azetidin-3-carbonsäure;
(*R*)-1-((8-((2-Chlor-3'-(5-(dimethylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidin-3-carbonsäure;
(*R*)-1-((8-((2-Chlor-3'-(5-(N-ethyl-N-methylglycyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidin-3-carbonsäure;
(*R*)-2-(1-((8-((2-Chlor-3'-(5-(dimethylglycyl)-5,6-dihydro-4*H*-pyrrolo[3,4-d]thiazol-2-yl)-2'-methyl-[1,1'-biphenyl]-3-yl)amino)-1,7-naphthyridin-3-yl)methyl)pyrrolidin-3-yl)essigsäure;
2-((8-((2-Chlor-3'-(5-(dimethylglycyl)-5,6-dihydro-4*H*-pyrrolo[3,4-*d*]thiazol-2-yl)-2'-methyl-[1,1'-biphenyl]-3-yl)amino)-1,7-naphthyridin-3-yl)methyl)-2-azabicyclo[2.2.1]heptan-5-carbonsäure;
(*R*)-2-(1-((8-(2-Chlor-3'-(5-(2-(ethyl(methyl)amino)acetyl)-5,6-dihydro-4*H-*pyrrolo[3,4-d]thiazol-2-yl)-2'-methylbiphenyl-3-ylamino)-1,7-naphthyridin-3-yl)methyl)pyrrolidin-3-yl)essigsäure;
2-((8-(2-Chlor-3'-(5-(2-(ethyl(methyl)amino)acetyl)-5,6-dihydro-4*H-*pyrrolo[3,4-d]thiazol-2-yl)-2'-methylbiphenyl-3-ylamino)-1,7-naphthyridin-3-yl)methyl)-2-azabicyclo[2.2.1]heptan-5-carbonsäure;
(*R*)-1-((8-((2-Chlor-3'-(5-(2-((*R*)-3-hydroxypyrrolidin-1-yl)acetyl)-5,6-dihydro-4*H-*pyrrolo[3,4-d]thiazol-2-yl)-2'-methyl-[1,1'-biphenyl]-3-yl)amino)-1,7-naphthyridin-3-yl)methyl)pyrrolidin-3-carbonsäure;
(*R*)-1-((8-((2-Chlor-3'-(5-(N-(2-hydroxyethyl)-N-methylglycyl)-5,6-dihydro-4*H*-pyrrolo[3,4-d]thiazol-2-yl)-2'-methyl-[1,1'-biphenyl]-3-yl)amino)-1,7-naphthyridin-3-yl)methyl)pyrrolidin-3-carbonsäure;
(*R*)-1-((8-((2-Chlor-3'-(5-(2-((*S*)-3-hydroxypyrrolidin-1-yl)acetyl)-5,6-dihydro-4*H-*pyrrolo[3,4-d]thiazol-2-yl)-2'-methyl-[1,1'-biphenyl]-3-yl)amino)-1,7-naphthyridin-3-yl)methyl)pyrrolidin-3-carbonsäure;
(*R*)-1-((8-((2-Chlor-3'-(5-(2-(3-hydroxyazetidin-1-yl)acetyl)-5,6-dihydro-4H-pyrrolo[3,4-d]thiazol-2-yl)-2'-methyl-[1,1'-biphenyl]-3-yl)amino)-1,7-naphthyridin-3-yl)methyl)pyrrolidin-3-carbonsäure und
*cis*-4-((2-(3'-(3-(((R)-3-Hydroxypyrrolidin-1-yl)methyl)-1,7-naphthyridin-8-ylamino)-2,2'-dimethylbiphenyl-3-ylcarbamoyl)-1-methyl-6,7-dihydro-1*H*-imidazo[4,5-*c*]pyridin-5(4*H*)-yl)methyl)cyclohexancarbonsäure;
oder ein pharmazeutisch unbedenkliches Salz davon.

25. Verbindung nach Anspruch 2 oder ein pharmazeutisch unbedenkliches Salz davon, wobei:
Ring A für 5- bis 10-gliedriges Heteroaryl oder 4-bis 11-gliedriges Heterocycloalkyl steht, wobei das 5- bis 10-gliedrige Heteroaryl und das 4- bis 11-gliedrige Heterocycloalkyl jeweils 1-4 Heteroatome als Ringglieder, die aus N, O und S ausgewählt sind, aufweisen, wobei das N- oder S-Atom als Ringglied gegebenenfalls oxidiert ist und ein oder mehrere Kohlenstoffatome als Ringglieder jeweils gegebenenfalls durch eine Carbonylgruppe ersetzt sind; und wobei Ring A gegebenenfalls durch 1, 2 oder 3 R⁶-Substituenten substituiert ist;
L für eine Bindung, -C(O)NR¹³-, -NR¹³C(O)-, -NR¹³- oder CH=CH- steht;
X für N oder CR¹⁷ steht, wobei R¹⁷ für H, C₁₋₄-Alkyl, C₁₋₄-Alkoxy, C₁₋₄-Halogenalkyl, C₁₋₄-Halogenalkoxy, CN, Halogen oder OH steht, wobei das C₁₋₄-Alkyl und C₁₋₄-Alkoxy jeweils gegebenenfalls durch 1 oder 2 Substituenten, die unabhängig aus CN, Halogen und -C(O)NH₂ ausgewählt sind, substituiert sind;
eines von R¹ und R² für -(CR⁸R⁹)p-NR¹⁰R¹¹ steht und das andere für H, C₁₋₄-Alkyl, C₁₋₄-Alkoxy, C₁₋₄-Halogenalkyl, C₁₋₄-Halogenalkoxy, CN, Halogen oder OH steht, wobei das C₁₋₄-Alkyl und C₁₋₄-Alkoxy von R¹ oder R² gegebenenfalls durch 1 oder 2 Substituenten, die unabhängig aus C₁₋₄-Alkoxy, C₁₋₄-Halogenalkyl, C₁₋₄-Halogenalkoxy, CN, Halogen und OH ausgewählt sind, substituiert sind;
R³ für Methyl, Halogen, CN oder C₁₋₄-Halogenalkyl steht;
R⁴ für C₁₋₄-Alkyl, C₁₋₄-Alkoxy oder C₁₋₄-Halogenalkyl steht;
R⁵ für C₁₋₄-Alkyl, C₁₋₄-Alkoxy, C₁₋₄-Halogenalkyl, C₁₋₄-Halogenalkoxy, CN, Halogen oder OH steht;
R⁶ jeweils aus H, Halogen, C₁₋₆-Alkyl, C₂₋₆-Alkenyl, C₂₋₆-Alkinyl, C₁₋₆-Halogenalkyl, C₁₋₆-Halogenalkoxy, 5-14-gliedrigem Heteroaryl, 4-10-gliedrigem Heterocycloalkyl, (5-14-gliedrigem Heteroaryl)-C₁₋₄-alkyl-, (4-10-gliedrigem Heterocycloalkyl) -C₁-₄-alkyl-, CN, NO₂, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a} oder NR^{a}C(O)OR^{a} ausgewählt ist, wobei das C₁₋₆-Alkyl, C₂₋₆-Alkenyl, C₂₋₆-Alkinyl, 5-14-gliedrige Heteroaryl, 4-10-gliedrige Heterocycloalkyl, (5-14-gliedrige Heteroaryl)-C₁₋₄-alkyl- und (4-10-gliedrige Heterocycloalkyl)-C₁₋₄-alkyl- von R⁶ jeweils gegebenenfalls durch 1, 2 oder 3 R^{b}-Substituenten substituiert ist;
R⁷ für H, C₁₋₄-Alkyl, C₁₋₄-Alkoxy, C₁₋₄-Halogenalkyl, C₁₋₄-Halogenalkoxy, CN, Halogen oder OH steht;
R⁸ und R⁹ jeweils unabhängig aus H, Halogen, CN, OH, -COOH, C₁₋₄-Alkyl, C₁₋₄-Alkoxy, -NH-C₁₋₄-Alkyl, -N (C₁₋₄-Alkyl)₂ und C₁₋₄-Halogenalkyl ausgewählt sind;
R¹⁰ und R¹¹ jeweils unabhängig aus H, C₁₋₆-Alkyl, C₁₋₆-Halogenalkyl, -C(O)R^{g}, -C(O)OR^{g} und -C(O)NR^{g}R^{g} ausgewählt sind, wobei das C₁₋₆-Alkyl und C₁₋₆-Halogenalkyl von R¹⁰ oder R¹¹ jeweils gegebenenfalls durch 1 oder 2 unabhängig ausgewählte R^{f}-Substituenten substituiert sind;
oder R¹⁰ und R¹¹ zusammen mit dem Stickstoffatom, an das sie gebunden sind, 4-, 5-, 6- oder 7-gliedriges Heterocycloalkyl bilden, wobei das 4-, 5-, 6- oder 7-gliedrige Heterocycloalkyl gegebenenfalls durch 1, 2 oder 3 R^{h}-Substituenten substituiert ist;
R¹² für H, C₁₋₄-Alkyl, C₁₋₄-Alkoxy, C₁₋₄-Halogenalkyl, C₁₋₄-Halogenalkoxy, CN, Halogen oder OH steht;
R¹³ jeweils unabhängig für H, C₁₋₆-Halogenalkyl oder C₁₋₆-Alkyl steht;
R^{a} jeweils unabhängig aus H, CN, C₁₋₆-Alkyl, C₁₋₄-Halogenalkyl, C₂₋₆-Alkenyl und C₂₋₆-Alkinyl ausgewählt ist;
R^{d} jeweils unabhängig aus C₁₋₆-Alkyl, C₁₋₆-Halogenalkyl, Halogen, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e} und NR^{e}C(O)R^{e} ausgewählt ist;
R^{e} jeweils unabhängig aus H, C₁₋₆-Alkyl, C₁₋₆-Halogenalkyl, C₂₋₆-Alkenyl und C₂₋₆-Alkinyl ausgewählt ist;
jeder R^{b}-Substituent unabhängig aus Halogen, C₁₋₆-Alkyl, C₁₋₆-Halogenalkyl, C₁₋₆-Halogenalkoxy, CN, OH, NH₂, NO₂, OR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c} und NR^{c}C(O)OR^{c} ausgewählt ist; wobei das C₁₋₄-Alkyl, C₁₋₄-Halogenalkyl und C₁₋₄-Halogenalkoxy von R^{b} jeweils gegebenenfalls durch 1 oder 2 unabhängig ausgewählte R^{d}-Substituenten substituiert sind;
R^{c} jeweils unabhängig aus H, C₁₋₆-Alkyl, C₁₋₄-Halogenalkyl, C₂₋₆-Alkenyl, C₂₋₆-Alkinyl, C₆₋₁₀-Aryl, C₃₋₁₀-Cycloalkyl, C₆₋₁₀-Aryl-C₁₋₄-alkyl- und C₃₋₁₀-Cycloalkyl-C₁₋₄-alkyl- ausgewählt ist, wobei das C₁₋₆-Alkyl, C₂₋₆-Alkenyl, C₂₋₆-Alkinyl, C₆₋₁₀-Aryl, C₃₋₁₀-Cycloalkyl, C₆₋₁₀-Aryl-C₁₋₄-alkyl- und C₃₋₁₀-Cycloalkyl-C₁₋₄-alkyl- von R^{c} jeweils gegebenenfalls durch 1, 2 oder 3 R^{f}-Substituenten substituiert sind;
R^{f} jeweils unabhängig aus C₁₋₄-Alkyl, C₁₋₄-Halogenalkyl, C₂₋₆-Alkenyl, C₂₋₆-Alkinyl, Halogen, CN, OR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g} und NR^{g}C(O)R^{g} ausgewählt ist;
R^{g} jeweils unabhängig aus H, C₁₋₆-Alkyl, C₁₋₄-Halogenalkyl, C₂₋₆-Alkenyl und C₂₋₆-Alkinyl ausgewählt ist;
R^{h} jeweils unabhängig aus C₁₋₆-Alkyl, C₁₋₆-Halogenalkyl, C₁₋₆-Halogenalkoxy, C₂₋₆-Alkenyl, C₂₋₆-Alkinyl, Halogen, CN, ORⁱ, C(O)Rⁱ, C(O)NRⁱRⁱ, C(O)OR^{i,} OC(O)R^{i,} OC(O)NRⁱRⁱ, NHRⁱ NRⁱRⁱ, NRⁱC(O)Rⁱ und NRⁱC(O)Rⁱ ausgewählt ist, wobei das C₁₋₆-Alkyl, C₂₋₆-Alkenyl und C₂₋₆-Alkinyl von R^{h} jeweils ferner gegebenenfalls durch 1, 2 oder 3 R^{j}-Substituenten substituiert sind;
R^{j} jeweils unabhängig aus C₂₋₄-Alkenyl, C₂₋₄-Alkinyl, Halogen, C₁₋₄-Alkyl, C₁₋₄-Halogenalkyl und CN ausgewählt ist;
oder beliebige zwei R^{c}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
R¹ jeweils unabhängig aus H, C₁₋₄-Alkyl, C₁₋₆-Halogenalkyl, C₁₋₆-Halogenalkoxy, C₂₋₄-Alkenyl und C₂₋₄-Alkinyl ausgewählt ist;
der tiefgestellte Index m für eine ganze Zahl mit einem Wert von 0, 1 oder 2 steht;
der tiefgestellte Index n für eine ganze Zahl mit einem Wert von 0, 1 oder 2 steht und
der tiefgestellte Index p für eine ganze Zahl mit einem Wert von 1, 2 oder 3 steht.

26. Verbindung nach Anspruch 2 oder ein pharmazeutisch unbedenkliches Salz davon, wobei:
Ring A für 5- bis 10-gliedriges Heteroaryl oder 4-bis 11-gliedriges Heterocycloalkyl steht, wobei das 5- bis 10-gliedrige Heteroaryl und das 4- bis 11-gliedrige Heterocycloalkyl jeweils 1-4 Heteroatome als Ringglieder, die aus N, O und S ausgewählt sind, aufweisen, wobei das N- oder S-Atom als Ringglied gegebenenfalls oxidiert ist und ein oder mehrere Kohlenstoffatome als Ringglieder jeweils gegebenenfalls durch eine Carbonylgruppe ersetzt sind; und wobei Ring A gegebenenfalls durch 1, 2 oder 3 R⁶-Substituenten substituiert ist;
L für eine Bindung, -C(O)NR¹³- oder -NR¹³C(O)-steht;
X für CR¹⁷ steht, wobei R¹⁷ für H oder C₁₋₄-Alkyl steht;
eines von R¹ und R² für -(CR⁸R⁹)ₚ-NR¹⁰R¹¹ steht und das andere für H, C₁₋₄-Alkyl oder C₁₋₄-Alkoxy steht;
R³ für Methyl oder Halogen steht;
R⁴ für C₁₋₄-Alkyl oder C₁₋₄-Alkoxy steht;
R⁵ für C₁₋₄-Alkyl, C₁₋₄-Alkoxy oder Halogen steht;
R⁶ jeweils aus H, Halogen, C₁₋₆-Alkyl, C₂₋₆-Alkenyl, C₂₋₆-Alkinyl, 5-14-gliedrigem Heteroaryl, 4-10-gliedrigem Heterocycloalkyl, (5-14-gliedrigem Heteroaryl)-C₁₋₄-alkyl- und (4-10-gliedrigem Heterocycloalkyl)-C₁₋₄-alkyl- ausgewählt ist, wobei das C₁₋₆-Alkyl, C₂₋₆-Alkenyl, C₂₋₆-Alkinyl, 5-14-gliedrige Heteroaryl, 4-10-gliedrige Heterocycloalkyl, (5-14-gliedrige Heteroaryl)-C₁₋₄-alkyl- und (4-10-gliedrige Heterocycloalkyl)-C₁₋₄-alkyl- von R⁶ jeweils gegebenenfalls durch 1, 2 oder 3 R^{b}-Substituenten substituiert ist;
R⁷ für H oder C₁₋₄-Alkyl steht;
R⁸ und R⁹ jeweils unabhängig aus H und C₁₋₄-Alkyl ausgewählt sind;
R¹⁰ und R¹¹ jeweils unabhängig aus H und C₁₋₆-Alkyl, das gegebenenfalls durch 1 oder 2 unabhängig ausgewählte R^{f}-Substituenten substituiert ist, ausgewählt sind;
oder R¹⁰ und R¹¹ zusammen mit dem Stickstoffatom, an das sie gebunden sind, 4-, 5-, 6- oder 7-gliedriges Heterocycloalkyl bilden, wobei das 4-, 5-, 6- oder 7-gliedrige Heterocycloalkyl gegebenenfalls durch 1, 2 oder 3 R^{h}-Substituenten substituiert ist;
R¹² für H oder C₁₋₄-Alkyl steht;
R¹³ jeweils unabhängig für H oder C₁₋₆-Alkyl steht;
jeder R^{b}-Substituent unabhängig aus Halogen, C₁₋₆-Alkyl, OH, NH₂, C(O)OR^{c}, NHR^{c} und NR^{c}R^{c} ausgewählt ist;
R^{c} jeweils unabhängig aus H, C₁₋₆-Alkyl, C₂₋₆-Alkenyl, C₂₋₆-Alkinyl, C₃₋₁₀)-Cycloalkyl und C₃₋₁₀-Cycloalkyl-C₁₋₄-alkyl- ausgewählt ist, wobei das C₁₋₆-Alkyl, C₂₋₆-Alkenyl, C₂₋₆-Alkinyl, C₃₋₁₀-Cyclo-alkyl und C₃₋₁₀-Cycloalkyl-C₁₋₄-alkyl- von R^{c} jeweils gegebenenfalls durch 1 oder 2 R^{f}-Substituenten substituiert sind;
R^{f} jeweils unabhängig aus C₁₋₄-Alkyl, C₂₋₆-Alkenyl, C₂₋₆-Alkinyl, Halogen, OR^{g} und C(O)R^{g} ausgewählt ist;
R^{g} jeweils unabhängig aus H und C₁₋₆-Alkyl ausgewählt ist;
R^{h} jeweils unabhängig aus C₁₋₆-Alkyl, C₂₋₆-Alkenyl, C₂₋₆-Alkinyl, Halogen, CN, ORⁱ und C(O)ORⁱ ausgewählt ist;
oder beliebige zwei R^{c}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
Rⁱ jeweils unabhängig aus H und C₁₋₄-Alkyl ausgewählt ist;
der tiefgestellte Index m für eine ganze Zahl mit einem Wert von 0 oder 1 steht;
der tiefgestellte Index n für eine ganze Zahl mit einem Wert von 0 oder 1 steht und
der tiefgestellte Index p für eine ganze Zahl mit einem Wert von 1 oder 2 steht.

27. Verbindung nach Anspruch 2 oder ein pharmazeutisch unbedenkliches Salz davon, wobei:
Ring A für 5- bis 10-gliedriges Heteroaryl steht, wobei das 5- bis 10-gliedrige Heteroaryl 1-4 Heteroatome als Ringglieder, die aus N, O und S ausgewählt sind, aufweist, wobei das N- oder S-Atom als Ringglied gegebenenfalls oxidiert ist und ein oder mehrere Kohlenstoffatome als Ringglieder jeweils gegebenenfalls durch eine Carbonylgruppe ersetzt sind; und wobei Ring A gegebenenfalls durch 1 oder 2 R⁶-Substituenten substituiert ist;
L für eine Bindung, -C(O)NR¹³- oder -NR¹³C(O)-steht;
X für CR¹⁷ steht, wobei R¹⁷ für H steht;
eines von R¹ und R² für - (CR⁸R⁹) ₚ-NR¹⁰R¹¹ steht und
das andere für H steht;
R³ für Methyl oder Halogen steht;
R⁴ für C₁₋₄-Alkyl oder C₁₋₄-Alkoxy steht;
R⁵ für C₁₋₄-Alkyl oder Halogen steht;
R⁶ jeweils aus H, C₁₋₆-Alkyl und (4-10-gliedrigem Heterocycloalkyl)-C₁₋₄-alkyl- ausgewählt ist, wobei das C₁₋₆-Alkyl und (4-10-gliedrige Heterocycloalkyl)-C₁₋₄-alkyl- von R⁶ jeweils gegebenenfalls durch 1 oder 2 R^{b}-Substituenten substituiert ist;
R⁷ für H steht;
R⁸ und R⁹ jeweils unabhängig aus H und C₁₋₄-Alkyl ausgewählt sind;
R¹⁰ und R¹¹ jeweils unabhängig aus H und C₁₋₆-Alkyl, das gegebenenfalls durch 1 oder 2 unabhängig ausgewählte R^{f}-Substituenten substituiert ist, ausgewählt sind;
oder R¹⁰ und R¹¹ zusammen mit dem Stickstoffatom, an das sie gebunden sind, 4-, 5-, 6- oder 7-gliedriges Heterocycloalkyl bilden, wobei das 4-, 5-, 6- oder 7-gliedrige Heterocycloalkyl gegebenenfalls durch 1, 2 oder 3 R^{h}-Substituenten substituiert ist;
R¹² für H steht;
R¹³ für H steht;
jeder R^{b}-Substituent unabhängig aus OH, C(O)OR^{c}, NHR^{c} und NR^{c}R^{c} ausgewählt ist;
R^{c} jeweils unabhängig aus H, C₁₋₆-Alkyl und C₃₋₁₀-Cycloalkyl ausgewählt ist, wobei das C₁₋₆-Alkyl und C₃₋₁₀-Cycloalkyl von R^{c} jeweils gegebenenfalls durch 1 oder 2 R^{f}-Substituenten substituiert sind;
R^{f} jeweils unabhängig aus OR^{g} und C(O)R^{g} ausgewählt ist;
R^{g} für H steht;
R^{h} jeweils unabhängig aus ORⁱ und C(O)ORⁱ ausgewählt ist;
oder beliebige zwei R^{c}-Substituenten zusammen mit dem Stickstoffatom, an das sie gebunden sind, eine 4-, 5-, 6- oder 7-gliedrige Heterocycloalkylgruppe bilden, die gegebenenfalls durch 1, 2 oder 3 unabhängig ausgewählte R^{h}-Substituenten substituiert ist;
R¹ für H steht;
der tiefgestellte Index m für eine ganze Zahl mit einem Wert von 0 oder 1 steht;
der tiefgestellte Index n für eine ganze Zahl mit einem Wert von 0 oder 1 steht und
der tiefgestellte Index p für eine ganze Zahl mit einem Wert von 1 oder 2 steht.

28. Pharmazeutische Zusammensetzung, umfassend eine Verbindung nach einem der Ansprüche 1-21 und 25-27 oder ein pharmazeutisch unbedenkliches Salz davon und einen oder mehrere pharmazeutisch unbedenkliche Hilfsstoffe oder Träger.

29. Pharmazeutische Zusammensetzung, umfassend eine Verbindung nach einem der Ansprüche 22-24 oder ein pharmazeutisch unbedenkliches Salz davon und einen oder mehrere pharmazeutisch unbedenkliche Hilfsstoffe oder Träger.

30. Verbindung nach einem der Ansprüche 1-21 und 25-27 oder pharmazeutisch unbedenkliches Salz davon oder Zusammensetzung nach Anspruch 28 zur Verwendung bei einem Verfahren zur Behandlung einer Erkrankung oder Störung, die mit PD-1/PD-L1-Wechselwirkung assoziiert ist, wobei es sich bei der Erkrankung oder Störung um Krebs oder eine Infektion handelt.

31. Verbindung, Salz oder Zusammensetzung zur Verwendung nach Anspruch 30, wobei es sich bei der Erkrankung oder Störung um eine Infektion handelt und es sich bei der Infektion um eine Virusinfektion handelt.

32. Verbindung, Salz oder Zusammensetzung zur Verwendung nach Anspruch 30, wobei es sich bei der Erkrankung oder Störung um Krebs handelt.

33. Verbindung, Salz oder Zusammensetzung zur Verwendung nach Anspruch 32, wobei der Krebs aus
(a) Knochenkrebs, Bauchspeicheldrüsenkrebs, Hautkrebs, Kopf- oder Halskrebs, kutanem malignem Melanom, intraokulärem malignem Melanom, Gebärmutterkrebs, Eierstockkrebs, Rektumkrebs, Krebs der Analregion, Magenkrebs, Hodenkrebs, Eileiterkarzinom, Endometriumkarzinom, Endometriumkrebs, Zervixkarzinom, Vaginakarzinom, Vulvakarzinom, Hodgkin-Krankheit, Non-Hodgkin-Lymphom, Speiseröhrenkrebs, Dünndarmkrebs, Krebs des endokrinen Systems, Schilddrüsenkrebs, Nebenschilddrüsenkrebs, Nebennierenkrebs, Weichteilsarkom, Harnröhrenkrebs, Peniskrebs, chronischen Leukämien, akuten Leukämien, soliden Tumoren der Kindheit, lymphozytischem Lymphom, Blasenkrebs, Nierenkrebs, Nierenbeckenkarzinom, Neoplasma des Zentralnervensystems (ZNS), primärem ZNS-Lymphom, Tumorangiogenese, Wirbelsäulentumor, Hirnstammgliom, Hypophysenadenom, Kaposi-Sarkom, Epidermoidkrebs, Plattenepithelkrebs, T-Zell-Lymphom, durch Umwelteinflüsse verursachten Krebserkrankungen einschließlich der durch Asbest induzierten, Melanom, metastatischem malignem Melanom, Nierenkrebs, Klarzellkarzinom, Prostatakrebs, hormonrefraktärem Prostataadenokarzinom, Brustkrebs, Kolonkrebs, Lungenkrebs, nichtkleinzelligem Lungenkrebs, kleinzelligem Lungenkrebs, soliden Tumoren, Leberkrebs, Magenkrebs, Glioblastom, Sarkom, hämatologischen Krebserkrankungen, Lymphom, Leukämie, akuter lymphoblastischer Leukämie (ALL), akuter myeloischer Leukämie (AML), chronischer lymphozytischer Leukämie (CLL), chronischer myeloischer Leukämie (CML), DLBCL, Mantelzell-Lymphom, relapsiertem NHL, refraktärem NHL, rezidivierendem follikulärem NHL, multiplem Myelom, Cholangiokarzinom, Gallengangkrebs, dreifach negativem Brustkrebs, Rhabdomyosarkom, Leiomyosarkom, Leberzellkarzinom, Ewing-Sarkom, Gehirnkrebs, Gehirntumor, Astrozytom, Neuroblastom, Neurofibrom, Basalzellkarzinom, Chondrosarkom, Epitheloidsarkom, Augenkrebs, Eileiterkrebs, Magen-Darm-Krebs, gastrointestinalen Stromatumoren, Haarzellleukämie, Darmkrebs, Inselzellkrebs, oralem Krebs, Mundkrebs, Rachenkrebs, Kehlkopfkrebs, Lippenkrebs, Mesotheliom, Nasennebenhöhlenkrebs, okularem Krebs, okularem Melanom, Beckenkrebs, Nierenzellkarzinom, Speicheldrüsenkrebs, Sinuskrebs, Wirbelsäulenkrebs, Zungenkrebs, tubulärem Karzinom und Urethrakrebs oder
(b) einem metastatischen Krebs, der PD-L1 exprimiert; oder
(c) Lungenkrebs oder
(d) kleinzelligem Lungenkrebs oder
(e) nichtkleinzelligem Lungenkrebs oder
(f) Nierenkrebs oder
(g) Leberkrebs oder
(h) Leberzellkarzinom oder
(i) Melanom oder
(j) Blasenkrebs oder
(k) Harnröhrenkrebs oder
(l) renalem Krebs oder
(m) Nierenzellkarzinom
ausgewählt ist.

34. Verbindung nach einem der Ansprüche 1-21 und 25-27 oder pharmazeutisch unbedenkliches Salz davon oder Zusammensetzung nach Anspruch 28 zur Verwendung bei einem Verfahren zur Verbesserung, Stimulierung und/oder Verstärkung der Immunreaktion bei einem Patienten, wobei der Patient an Krebs oder einer Infektion leidet.

35. Verbindung nach einem der Ansprüche 22-24 oder pharmazeutisch unbedenkliches Salz davon oder Zusammensetzung nach Anspruch 29 zur Verwendung bei einem Verfahren zur Behandlung einer Erkrankung oder Störung, die mit PD-1/PD-L1-Wechselwirkung assoziiert ist, wobei es sich bei der Erkrankung oder Störung um Krebs oder eine Infektion handelt.

36. Verbindung, Salz oder Zusammensetzung zur Verwendung nach Anspruch 35, wobei es sich bei der Erkrankung oder Störung um Krebs handelt.

37. Verbindung, Salz oder Zusammensetzung zur Verwendung nach Anspruch 36, wobei der Krebs aus Urothelkrebs, Krebserkrankungen mit hoher Mikrosatelliteninstabilität (MSI^{high}) , Krebs des Urogenitaltrakts, Leberkrebserkrankungen, Nervensystemkrebserkrankungen, gynäkologischen Krebserkrankungen, akuter promyelozytärer Leukämie (APL), myeloproliferativen Erkrankungen, primärer Myelofibrose (PMF), Polycythemia vera (BV), essentieller Thrombozytose (ET), myelodysplastischem Syndrom (MDS), akutem lymphoblastischem T-Zelllymphom (T-ALL), Osteosarkom, Angiosarkom, Fibrosarkom, Liposarkom, Myxom, Rhabdomyom, Rhabdosarkom, Fibrom, Lipom, Hamartom, Teratom, bronchogenem Karzinom, Plattenepithelkarzinom, undifferenziertem kleinzelligem Karzinom, undifferenziertem großzelligem Karzinom, Adenokarzinom, alveolärem (bronchiolärem) Karzinom, Bronchialkarzinom, chondromatösem Hamartom, Karzinom, duktalem Adenokarzinom, Insulinom, Glukagonom, Gastrinom, Karzinoiden, Fibrom, Krebserkrankungen des Dünndarms, Leiomyom, Hämangiom, Krebserkrankungen des Dickdarms, tubulärem Adenom, villösem Adenom, Hamartom, Kolorektalkrebs, Wilms-Tumor [Nephroblastom], Übergangszellkarzinom, Seminom, embryonalem Karzinom, Teratokarzinom, Choriokarzinom, Sarkom, Interstitialzellkarzinom, Fibroadenom, Adenomatoidtumoren, Heteroatom, Hepatoblastom, Leberzelladenom, osteogenem Sarkom, malignem fibrösem Histiozytom, malignem Lymphom, Retikulumzellsarkom, malignem Riesenzelltumor, Chordom, Osteochondrom, osteokartilaginären Exostosen, benignem Chondrom, Chondroblastom, Chondromyxofibrom, Osteoidosteom, Riesenzelltumoren, Krebserkrankungen des Schädels, Osteom, Granulom, Xanthom, und Osteitis deformans, Krebserkrankungen der Meningen, Meningiom, Meningiosarkom, Gliomatose, Medulloblastom, Gliom, Ependymom, Germinom, Pinealom, Glioblastoma multiforme, Oligodendrogliom, Schwannom, Retinoblastom, kongenitalen Tumoren, Krebserkrankungen der Wirbelsäule, Lhermitte-Duclos-Krankheit, Endometriumkarzinom, Krebserkrankungen des Gebärmutterhalses, prätumoröser zervikaler Dysplasie, Ovarialkarzinom, serösem Zystadenokarzinom, muzinösem Zystadenokarzinom, unklassifiziertem Karzinom, Granulosa-Theka-Zelltumoren, Sertoli-Leydig-Zelltumoren, Dysgerminom, malignem Teratom, Krebserkrankungen der Vulva, intraepithelialem Karzinom, Krebserkrankungen der Vagina, Sarkoma botryoides, embryonalem Rhabdomyosarkom, Muttermalen, dysplastischen Naevi, Angiom, Dermatofibrom, Keloiden, myelodysplastischen Syndromen und Urothelkarzinom ausgewählt ist.

## Revendications

1. Composé de Formule (I) : 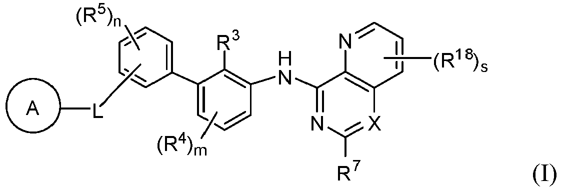 ou un sel pharmaceutiquement acceptable de celui-ci, dans laquelle :
le cycle A représente un groupement hétéroaryle comportant 5 à 10 chaînons, hétérocycloalkyle comportant 4 à 11 chaînons, aryle en C₆₋₁₀ ou cycloalkyle en C₃₋₁₀, dans lequel les groupements hétéroaryle comportant 5 à 10 chaînons et hétérocycloalkyle comportant 4 à 11 chaînons comportent chacun 1 à 4 hétéroatomes en tant que chaînons choisis parmi N, O et S, dans lequel l'atome N ou S en tant que chaînon est éventuellement oxydé et un ou plusieurs atomes de carbone en tant que chaînons sont chacun éventuellement remplacés par un groupement carbonyle ; et dans lequel le cycle A est éventuellement substitué par 1, 2, 3, 4 ou 5 substituants R⁶ ;
L représente une liaison, -C(O)NR¹³-, -NR¹³C(O)-, O,-(CR¹⁴R¹⁵)_{q}-, -(CR¹⁴R¹⁵)_{q}-O-, -O(CR¹⁴R¹⁵)_{q}-, -NR¹³-,-(CR¹⁴R¹⁵)_{q}-NR¹³-, -NR¹³-(CR¹⁴R¹⁵)_{q}-, -CH=CH-, -C≡C-,-SO₂NR¹³-, -NR¹³SO₂-, -NR¹³C(O)O- ou -NR¹³C(O)NR¹³- ;
X représente N ou CR¹⁷ ;
R³ représente un groupement méthyle, halogéno, CN ou halogénoalkyle en C₁₋₄ ;
R⁴ représente un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou -N(alkyle en C₁₋₄)₂ ;
R⁵ représente un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou -N(alkyle en C₁₋₄)₂ ;
chacun des radicaux R⁶, R⁷, R¹⁷ et R¹⁸ est indépendamment choisi parmi H et les groupements halogéno, alkyle en C₁₋₆, alcényle en C₂₋₆, alcynyle en C₂₋₆, halogénoalkyle en C₁₋₆, halogénoalkoxy en C₁₋₆, aryle en C₆₋₁₀, cycloalkyle en C₃₋₁₀, hétéroaryle comportant 5 à 14 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 14 chaînons)- (alkyle en C₁₋₄)-, (hétérocycloalkyle comportant 4 à 10 chaînons)-(alkyle en C₁₋₄)-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a} et S(O)₂NR^{a}R^{a}, dans lesquels chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 14 chaînons, hétérocycloalkyle comportant 4 à 10 chaînons, (aryle en C₅₋₁₀)-(alkyle en C₁₋₄)-, (cycloalkyle en C₃₋₁₀)-(alkyle en C₁₋₄)-, (hétéroaryle comportant 5 à 14 chaînons) - (alkyle en C₁₋₄)- et (hétérocycloalkyle comportant 4 à 10 chaînons)-(alkyle en C₁₋₄)- de R⁶, R⁷, R¹⁷ et R¹⁸ est éventuellement substitué par 1, 2, 3, 4 ou 5 R^{b} substituants ;
ou deux substituants R⁶ liés au même atome de carbone de cycle forment conjointement avec l'atome de carbone de cycle auquel ils sont liés un groupement spiro-cycloalkyle en C₃₋₆ ou spiro-hétérocycloalkyle comportant 4 à 7 chaînons, chacun étant éventuellement substitué par 1, 2 ou 3 substituants R^{f} indépendamment choisis ;
chacun des radicaux R¹³ représente indépendamment H ou un groupement halogénoalkyle en C₁₋₆ ou alkyle en C₁₋₆ éventuellement substitué par un substituant choisi parmi les groupements alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ et -N(alkyle en C₁₋₄)₂ ;
chacun des radicaux R¹⁴ et R¹⁵ est indépendamment choisi parmi H et les groupements halogéno, CN, OH, -COOH, alkyle en C₁₋₄, alkoxy en C₁₋₄, -NH-alkyle en C₁₋₄,-N(alkyle en C₁₋₄)₂, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons, dans lequel chacun des groupements alkyle en C₁₋₄, alkoxy en C₁₋₄, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons de R¹⁴ ou R¹⁵ est éventuellement substitué par 1, 2 ou 3 substituants R^{q} indépendamment choisis ;
ou R¹⁴ et R¹⁵ forment conjointement avec l'atome de carbone auquel ils sont liés un groupement cycloalkyle comportant 3, 4, 5 ou 6 chaînons ou hétérocycloalkyle comportant 3, 4, 5 ou 6 chaînons, chacun étant éventuellement substitué par 1 ou 2 substituants R^{q} ;
chacun des radicaux R^{a} est indépendamment choisi parmi H et les groupements CN, alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{a} est éventuellement substitué par 1, 2, 3, 4 ou 5 substituants R^{d} ;
chacun des radicaux R^{d} est indépendamment choisi parmi les groupements alkyle en C₁₋₆, halogénoalkyle en C₁₋₆, halogéno, aryle en C₆₋₁₀, hétéroaryle comportant 5 à 10 chaînons, cycloalkyle en C₃₋₁₀, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons) - (alkyle en C₁₋₄)-, (hétérocycloalkyle comportant 4 à 10 chaînons)-(alkyle en C₁₋₄)-, CN, NH₂, NHOR^{e}, OR^{e}, SR^{e}, C(O)R^{e} C(O)NR^{e}R^{e} , C(O)OR^{e} OC(O)R^{e}, OC(O)NR^{e}R^{e}, NHR^{e}, NR^{e}R^{e}, NR^{e}C(O)R^{e}, NR^{e}C (O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e} , S(O)₂R^{e}, NR^{e}S(O)₂R^{e}, NR^{e}S(O)₂NR^{e}R^{e} et S(O)₂NR^{e}R^{e}, dans lequel chacun des groupements alkyle en C₁₋₆, halogénoalkyle en C₁₋₆, aryle en C₆₋₁₀, hétéroaryle comportant 5 à 10 chaînons, cycloalkyle en C₃₋₁₀, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{d} est éventuellement substitué par 1, 2 ou 3 substituants R^{f} indépendamment choisis ;
chacun des radicaux R^{e} est indépendamment choisi parmi H et les groupements alkyle en C₁₋₆, halogénoalkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, halogénoalkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons) - (alkyle en C₁₋₄)- et (hétérocycloalkyle comportant 4 à 10 chaînons)-(alkyle en C₁₋₄)- de R^{e} est éventuellement substitué par 1, 2 ou 3 substituants R^{f} indépendamment choisis ;
chacun des substituants R^{b} est indépendamment choisi parmi les groupements halogéno, alkyle en C₁₋₆, alcényle en C₂₋₆, alcynyle en C₂₋₆, halogénoalkyle en C₁₋₆, halogénoalkoxy en C₁₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)-, (hétérocycloalkyle comportant 4 à 10 chaînons) - (alkyle en C₁₋₄)-, CN, OH, NH₂, NO₂, NHOR^{c}, OR^{c}, SR^{c}, C(O)R^{c}, C(O)NR^{c}R^{c}, C(O)OR^{c}, OC(O)R^{c}, OC(O)NR^{c}R^{c}, C(=NR^{c}) NR^{c}R^{c}, NR^{c}C(=NR^{c}) NR^{c}R^{c}, NHR^{c}, NR^{c}R^{c}, NR^{c}C(O)R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{c}R^{c}, S(O)₂R^{c} et S(O)₂NR^{c}R^{c} ; dans lequel chacun des groupements alkyle en C₁₋₆, halogénoalkyle en C₁₋₆, halogénoalkoxy en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{b} est en outre éventuellement substitué par 1, 2 ou 3 substituants R^{d} indépendamment choisis ;
chacun des radicaux R^{c} est indépendamment choisi parmi H et les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄) -, (cycloalkyle en C₃₋₁₀) - (alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{c} est éventuellement substitué par 1, 2, 3, 4 ou 5 substituants R^{f} ;
chacun des radicaux R^{f} est indépendamment choisi parmi les groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀) - (alkyle en C₁₋₄)-, (hétéroaryle comportant 5 à 10 chaînons)- (alkyle en C₁₋₄) -, (hétérocycloalkyle comportant 4 à 10 chaînons)-(alkyle en C₁₋₄)-, halogéno, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g} et S(O)₂NR^{g}R^{g} ; dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄) -, (cycloalkyle en C₃₋₁₀) - (alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{f} est éventuellement substitué par 1, 2, 3, 4 ou 5 substituants Rⁿ ;
chacun des radicaux Rⁿ est indépendamment choisi parmi les groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)- (alkyle en C₁₋₄)-, (cycloalkyle en C₃₋₁₀)-(alkyle en C₁₋₄)-, (hétéroaryle comportant 5 à 10 chaînons)- (alkyle en C₁₋₄)-, (hétérocycloalkyle comportant 4 à 10 chaînons)-(alkyle en C₁₋₄)-, halogéno, CN, NHOR^{o}, OR^{o}, SR^{o}, C(O)R^{o}, C(O)NR^{o}R^{o}, C(O)OR^{o}, OC(O)R^{o}, OC(O)NR^{o}R^{o}, NHR^{o}, NR^{o}R^{o}, NR^{o}C(O)R^{o}, NR^{o}C(O)NR^{o}R^{o}, NR^{o}C(O)OR^{o}, C(=NR^{o})NR^{o}R^{o}, NR^{OC} (=NR^{o})NR^{o}R^{o}, S(O)R^{o}, S(O)NR^{o}R^{o}, S(O)₂R^{o}, NR^{o}S(O)₂R^{o}, NR^{o}S(O)₂NR^{o}R^{o} et S(O)₂NR^{o}R^{o}, dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)- (alkyle en C₁₋₄)-, (cycloalkyle en C₃₋₁₀)-(alkyle en C₁₋₄)-, (hétéroaryle comportant 5 à 10 chaînons) - (alkyle en C₁₋₄)- et (hétérocycloalkyle comportant 4 à 10 chaînons)-(alkyle en C₁₋₄)- de Rⁿ est éventuellement substitué par 1, 2 ou 3 substituants R^{q} indépendamment choisis ;
chacun des radicaux R^{g} est indépendamment choisi parmi H et les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)- (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)- (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)- (alkyle en C₁₋₄)- de R^{g} est éventuellement substitué par 1, 2 ou 3 substituants RP ;
chacun des radicaux RP est indépendamment choisi parmi les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)-, (hétérocycloalkyle comportant 4 à 10 chaînons)-(alkyle en C₁₋₄)-, halogéno, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} et S(O)₂NR^{r}R^{r}, dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₆)-, (hétéroaryle comportant 5 à 10 chaînons)-(alkyle en C₁₋₄)- et (hétérocycloalkyle comportant 4 à 10 chaînons)- (alkyle en C₁₋₄)- de RP est éventuellement substitué par 1, 2 ou 3 substituants R^{q}; ou deux substituants R^{a} quelconques forment conjointement avec l'atome d'azote auquel ils sont liés un groupement hétérocycloalkyle comportant 4, 5, 6, 7, 8, 9 ou 10 chaînons éventuellement substitué par 1, 2 ou 3 substituants R^{h} ;
chacun des radicaux R^{h} est indépendamment choisi parmi les groupements alkyle en C₁₋₆, cycloalkyle en C₃₋₁₀, hétérocycloalkyle comportant 4 à 7 chaînons, aryle en C₆₋₁₀, hétéroaryle comportant 5 à 6 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 6 chaînons)- (alkyle en C₁₋₄)-, (hétérocycloalkyle comportant 4 à 7 chaînons)-(alkyle en C₁₋₄)-, halogénoalkyle en C₁₋₆, halogénoalkoxy en C₁₋₆, alcényle en C₂₋₆, alcynyle en C₂₋₆, halogéno, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ et S(O)2NRⁱRⁱ, dans lequel chacun des groupements alkyle en C₁₋₆, alcényle en C₂₋₆, alcynyle en C₂₋₆, cycloalkyle en C₃₋₁₀, hétérocycloalkyle comportant 4 à 7 chaînons, aryle en C₆₋₁₀, hétéroaryle comportant 5 à 6 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 6 chaînons)-(alkyle en C₁₋₄)-, (hétérocycloalkyle comportant 4 à 7 chaînons) - (alkyle en C₁₋₄)- de R^{h} est éventuellement en outre substitué par 1, 2 ou 3 substituants R^{j} ;
chacun des radicaux R^{j} est indépendamment choisi parmi les groupements cycloalkyle en C₃₋₆, aryle en C₆₋₁₀, hétéroaryle comportant 5 ou 6 chaînons, hétérocycloalkyle comportant 4 à 7 chaînons, alcényle en C₂₋₄, alcynyle en C₂₋₄, halogéno, alkyle en C₁₋₄, halogénoalkyle en C₁₋₄, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k} et S(O)₂NR^{k}R^{k}, dans lequel chacun des groupements alkyle en C₁₋₄, cycloalkyle en C₃₋₆, aryle en C₆₋₁₀, hétéroaryle comportant 5 ou 6 chaînons, hétérocycloalkyle comportant 4 à 6 chaînons, alcényle en C₂₋₄, alcynyle en C₂₋₄, halogénoalkyle en C₁₋₄ et halogénoalkoxy en C₁₋₄ de R^{j} est éventuellement substitué par 1, 2 ou 3 substituants R^{q} indépendamment choisis ;
ou deux groupements R^{h} liés au même atome de carbone du groupement hétérocycloalkyle comportant 4 à 10 chaînons forment conjointement avec l'atome de carbone auquel ils sont liés un groupement cycloalkyle en C₃₋₆ ou hétérocycloalkyle comportant 4 à 6 chaînons ayant 1 à 2 hétéroatomes en tant que chaînons choisis parmi O, N ou S ;
ou deux substituants R^{c} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{e} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{g} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{o} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{r} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
chacun des radicaux Rⁱ, R^{k}, R^{o} ou R^{r} est indépendamment choisi parmi H et les groupements alkyle en C₁₋₄, cycloalkyle en C₃₋₆, aryle en C₆₋₁₀, hétéroaryle comportant 5 ou 6 chaînons, hétérocycloalkyle comportant 4 à 7 chaînons, halogénoalkyle en C₁₋₆, halogénoalkoxy en C₁₋₆, alcényle en C₂₋₄ et alcynyle en C₂₋₄, dans lequel chacun des groupements alkyle en C₁₋₄, cycloalkyle en C₃₋₆, aryle en C₆₋₁₀, hétéroaryle comportant 5 ou 6 chaînons, hétérocycloalkyle comportant 4 à 7 chaînons, alcényle en C₂₋₄ et alcynyle en C₂₋₄ de Rⁱ, R^{k}, R^{o} ou R^{r} est éventuellement substitué par 1, 2 ou 3 substituants R^{q} ;
chacun des radicaux R^{q} est indépendamment choisi parmi les groupements halogéno, OH, CN, -COOH, NH₂, -NH-(alkyle en C₁₋₆), -N (alkyle en C₁₋₆)₂, alkyle en C₁₋₆, alkoxy en C₁₋₆, alkylthio en C₁₋₆, halogénoalkyle en C₁₋₆, halogénoalkoxy en C₁₋₆, phényle, hétéroaryle comportant 5 à 6 chaînons, hétérocycloalkyle comportant 4 à 6 chaînons et cycloalkyle en C₃₋₆, dans lequel chacun des groupements alkyle en C₁₋₆, phényle, cycloalkyle en C₃₋₆, hétérocycloalkyle comportant 4 à 6 chaînons et hétéroaryle comportant 5 à 6 chaînons de R^{q} est éventuellement substitué par 1, 2 ou 3 substituants choisis parmi les groupements halogéno, OH, CN, -COOH, NH₂, alkyle en C₁₋₄, alkoxy en C₁₋₄, halogénoalkyle en C₁₋₄, halogénoalkoxy en C₁₋₄, phényle, cycloalkyle en C₃₋₁₀, hétéroaryle comportant 5 à 6 chaînons et hétérocycloalkyle comportant 4 à 6 chaînons ;
l'indice m représente un entier choisi parmi 0, 1, 2 ou 3 ;
l'indice n représente un entier choisi parmi 0, 1, 2 ou 3 ;
chaque indice q représente indépendamment un entier choisi parmi 1, 2, 3 ou 4 ; et
l'indice s représente un entier choisi parmi 1, 2 ou 3.

2. Composé de Formule (Ia) : 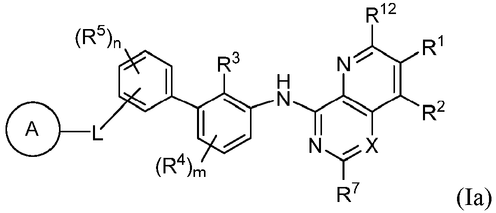 ou un sel pharmaceutiquement acceptable de celui-ci, dans laquelle :
le cycle A représente un groupement hétéroaryle comportant 5 à 10 chaînons, hétérocycloalkyle comportant 4 à 11 chaînons, aryle en C₆₋₁₀ ou cycloalkyle en C₃₋₁₀, dans lequel les groupements hétéroaryle comportant 5 à 10 chaînons et hétérocycloalkyle comportant 4 à 11 chaînons comportent chacun 1 à 4 hétéroatomes en tant que chaînons choisis parmi N, O et S, dans lequel l'atome N ou S en tant que chaînon est éventuellement oxydé et un ou plusieurs atomes de carbone en tant que chaînons sont chacun éventuellement remplacés par un groupement carbonyle ; et dans lequel le cycle A est éventuellement substitué par 1, 2, 3, 4 ou 5 substituants R⁶ ;
L représente une liaison, -C(O)NR¹³-, -NR¹³C(O)-, O, - (CR¹⁴R¹⁵)_{q}-, -(CR¹⁴R¹⁵)_{q}-O-, -O(CR¹⁴R¹⁵)_{q}-, -NR¹³-, - (CR¹⁴R¹⁵)_{q}-NR¹³-, -NR¹³-(CR¹⁴R¹⁵)_{q}-, -CH=CH-, -C≡C-, - SO₂NR¹³-, -NR¹³SO₂-, -NR¹³C(O)O- ou -NR¹³C(O)NR¹³- ;
X représente N ou CR¹⁷ ;
R³ représente un groupement méthyle, halogéno, CN ou halogénoalkyle en C₁₋₄ ;
R⁴ représente un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou -N(alkyle en C₁₋₄)₂ ;
R⁵ représente un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou -N(alkyle en C₁₋₄)₂ ;
R⁶ est choisi parmi H et les groupements halogéno, alkyle en C₁₋₆, alcényle en C₂₋₆, alcynyle en C₂₋₆, halogénoalkyle en C₁₋₆, halogénoalkoxy en C₁₋₆, aryle en C₆₋₁₀, cycloalkyle en C₃₋₁₀, hétéroaryle comportant 5 à 14 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 14 chaînons)- (alkyle en C₁₋₄)-, (hétérocycloalkyle comportant 4 à 10 chaînons)-(alkyle en C₁₋₄)-, CN, NO₂, OR^{a}, SR^{a}, NHOR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a}, NR^{a}C(O)OR^{a}, NR^{a}C(O)NR^{a}R^{a}, C(=NR^{a})R^{a}, C(=NR^{a})NR^{a}R^{a}, NR^{a}C(=NR^{a})NR^{a}R^{a}, NR^{a}S(O)R^{a}, NR^{a}S(O)₂R^{a}, NR^{a}S(O)₂NR^{a}R^{a}, S(O)R^{a}, S(O)NR^{a}R^{a}, S(O)₂R^{a} et S(O)₂NR^{a}R^{a}, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 14 chaînons, hétérocycloalkyle comportant 4 à 10 chaînons, (aryle en C₅₋₁₀) - (alkyle en C₁₋₄)-, (cycloalkyle en C₃₋₁₀)-(alkyle en C₁₋₄)-, (hétéroaryle comportant 5 à 14 chaînons)-(alkyle en C₁₋₄)- et (hétérocycloalkyle comportant 4 à 10 chaînons)- (alkyle en C₁₋₄)- de R⁶ est éventuellement substitué par 1, 2, 3, 4 ou 5 R^{b} substituants ;
ou deux substituants R⁶ liés au même atome de carbone de cycle forment conjointement avec l'atome de carbone de cycle auquel ils sont liés un groupement spiro-cycloalkyle en C₃₋₆ ou spiro-hétérocycloalkyle comportant 4 à 7 chaînons, chacun étant éventuellement substitué par 1, 2 ou 3 substituants R^{f} indépendamment choisis ;
chacun des radicaux R¹³ représente indépendamment H ou un groupement halogénoalkyle en C₁₋₆ ou alkyle en C₁₋₆ éventuellement substitué par un substituant choisi parmi les groupements alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ et -N(alkyle en C₁₋₄)₂ ;
chacun des radicaux R¹⁴ et R¹⁵ est indépendamment choisi parmi H et les groupements halogéno, CN, OH, -COOH, alkyle en C₁₋₄, alkoxy en C₁₋₄, -NH-alkyle en C₁₋₄, - N(alkyle en C₁₋₄)₂, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons, dans lequel chacun des groupements alkyle en C₁₋₄, alkoxy en C₁₋₄, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons de R¹⁴ ou R¹⁵ est éventuellement substitué par 1, 2 ou 3 substituants R^{q} indépendamment choisis ;
ou R¹⁴ et R¹⁵ forment conjointement avec l'atome de carbone auquel ils sont liés un groupement cycloalkyle comportant 3, 4, 5 ou 6 chaînons ou hétérocycloalkyle comportant 3, 4, 5 ou 6 chaînons, chacun étant éventuellement substitué par 1 ou 2 substituants R^{q} ;
R¹⁷ représente H ou un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou - N(alkyle en C₁₋₄)₂, dans lequel chacun des groupements alkyle en C₁₋₄ et alkoxy en C₁₋₄ est éventuellement substitué par 1 ou 2 substituants indépendamment choisis parmi les groupements CN, halogéno et -C(O)NH₂ ;
l'un des radicaux R¹ et R² représente -(CR⁸R⁹)ₚ-NR¹⁰R¹¹ et l'autre représente H ou un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou -N(alkyle en C₁₋₄)₂, dans lesquels le groupement alkyle en C₁₋₄ et alkoxy en C₁₋₄ de R¹ ou R² est éventuellement substitué par 1 ou 2 substituants indépendamment choisis parmi les groupements alkoxy en C₁₋₄, halogénoalkyle en C₁₋₄, halogénoalkoxy en C₁₋₄, CN, halogéno, OH, -COOH,-C(O)NH₂, NH₂, -NH-alkyle en C₁₋₄ et -N(alkyle en C₁₋₄)₂ ;
R⁷ représente H ou un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou - N(alkyle en C₁₋₄)₂, dans lequel chacun des groupements alkyle en C₁₋₄ et alkoxy en C₁₋₄ est éventuellement substitué par 1 ou 2 substituants indépendamment choisis parmi les groupements CN, halogéno ou -C(O)NH₂ ;
chacun des radicaux R⁸ et R⁹ est indépendamment choisi parmi H et les groupements halogéno, CN, OH, -COOH, alkyle en C₁₋₄, alkoxy en C₁₋₄, -NH-alkyle en C₁₋₄, - N(alkyle en C₁₋₄)₂, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons, dans lequel chacun des groupements alkyle en C₁₋₄, alkoxy en C₁₋₄, halogénoalkyle en C₁₋₄, halogénoalkoxy en C₁₋₄, cycloalkyle en C₃₋₆, phényle, hétéroaryle comportant 5 à 6 chaînons et hétérocycloalkyle comportant 4 à 6 chaînons de R⁸ ou R⁹ est éventuellement substitué par 1, 2 ou 3 substituants R^{q} indépendamment choisis ;
ou R⁸ et R⁹ forment conjointement avec l'atome de carbone auquel ils sont liés un groupement cycloalkyle comportant 3, 4, 5 ou 6 chaînons ou hétérocycloalkyle comportant 4, 5, 6 ou 7 chaînons, chacun étant éventuellement substitué par 1 ou 2 substituants R^{q} ;
ou R⁸ et R¹⁰ forment conjointement avec les atomes auxquels ils sont liés un groupement hétérocycloalkyle comportant 4, 5, 6 ou 7 chaînons, ayant zéro à un hétéroatome supplémentaire en tant que chaînon choisi parmi O, N ou S, dans lequel chacun des groupements hétérocycloalkyle comportant 4, 5, 6 ou 7 chaînons formés par R⁸ et R¹⁰ est éventuellement substitué par 1 ou 2 substituants R^{q} ;
chacun des radicaux R¹⁰ et R¹¹ est indépendamment choisi parmi H et les groupements alkyle en C₁₋₆, halogénoalkyle en C₁₋₆, cycloalkyle en C₃₋₆, aryle en C₆₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₆)-(alkyle en C₁₋₄)-, (hétéroaryle comportant 5 à 10 chaînons)- (alkyle en C₁₋₄)-, (hétérocycloalkyle comportant 4 à 10 chaînons)-(alkyle en C₁₋₄)-, -C(O)R^{g}, -C(O)OR^{g}, - C(O)NR^{g}R^{g}, -SO₂R^{g} et -SO₂NR^{g}R^{g}, dans lesquels chacun des groupements alkyle en C₁₋₆, halogénoalkyle en C₁₋₆, cycloalkyle en C₃₋₆, aryle en C₆₋₁₀, hétéroaryle comportant 5 à 10 chaînons, hétérocycloalkyle comportant 4 à 10 chaînons, (aryle en C₆₋₁₀) - (alkyle en C₁₋₄) -, (cycloalkyle en C₃₋₆)-(alkyle en C₁₋₄)-, (hétéroaryle comportant 5 à 10 chaînons) - (alkyle en C₁₋₄)- et (hétérocycloalkyle comportant 4 à 10 chaînons)-(alkyle en C₁₋₄)- de R¹⁰ ou R¹¹ est éventuellement substitué par 1, 2 ou 3 substituants R^{d} indépendamment choisis ;
ou R¹⁰ et R¹¹ forment conjointement avec l'atome d'azote auquel ils sont liés un groupement hétérocycloalkyle comportant 4, 5, 6, 7, 8, 9, 10 ou 11 chaînons, dans lequel le groupement hétérocycloalkyle comportant 4 à 11 chaînons est éventuellement substitué par 1, 2 ou 3 substituants R^{f} ;
R¹² représente H ou un groupement alkyle en C₁₋₄, alkoxy en C₁₋₄, halogénoalkyle en C₁₋₄, halogénoalkoxy en C₁₋₄, CN, halogéno, OH, -COOH, NH₂, -NH-alkyle en C₁₋₄ ou - N(alkyle en C₁₋₄)₂ ;
chacun des radicaux R^{a} est indépendamment choisi parmi H et les groupements CN, alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{a} est éventuellement substitué par 1, 2, 3, 4 ou 5 substituants R^{d} ;
chacun des radicaux R^{d} est indépendamment choisi parmi les groupements alkyle en C₁₋₆, halogénoalkyle en C₁₋₆, halogéno, aryle en C₆₋₁₀, hétéroaryle comportant 5 à 10 chaînons, cycloalkyle en C₃₋₁₀, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons) - (alkyle en C₁₋₄)-, (hétérocycloalkyle comportant 4 à 10 chaînons)-(alkyle en C₁₋₄)-, CN, NH₂, NHOR^{e}, OR^{e}, SR^{e}, C(O)R^{e}, C(O)NR^{e}R^{e}, C(O)OR^{e}, OC(O)R^{e}, OC(O)NR^{e}R^{e}, NHR^{e}, NR^{e}R^{e}, NR^{e}C(O)R^{e}, NR^{e}C(O)NR^{e}R^{e}, NR^{e}C(O)OR^{e}, C(=NR^{e})NR^{e}R^{e}, NR^{e}C(=NR^{e})NR^{e}R^{e}, NR^{e}C(=NOH)NR^{e}R^{e}, NR^{e}C(=NCN)NR^{e}R^{e}, S(O)R^{e}, S(O)NR^{e}R^{e}, S(O)₂R^{e}, NR^{e}S(O)₂R^{e}, NR^{e}S(O)₂NR^{e}R^{e} et S(O)₂NR^{e}R^{e}, dans lequel chacun des groupements alkyle en C₁₋₆, halogénoalkyle en C₁₋₆, aryle en C₆₋₁₀, hétéroaryle comportant 5 à 10 chaînons, cycloalkyle en C₃₋₁₀, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{d} est éventuellement substitué par 1, 2 ou 3 substituants R^{f} indépendamment choisis ;
chacun des radicaux R^{e} est indépendamment choisi parmi H et les groupements alkyle en C₁₋₆, halogénoalkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, halogénoalkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons) - (alkyle en C₁₋₄)- et (hétérocycloalkyle comportant 4 à 10 chaînons)-(alkyle en C₁₋₄)- de R^{e} est éventuellement substitué par 1, 2 ou 3 substituants R^{f} indépendamment choisis ;
chacun des substituants R^{b} est indépendamment choisi parmi les groupements halogéno, alkyle en C₁₋₆, alcényle en C₂₋₆, alcynyle en C₂₋₆, halogénoalkyle en C₁₋₆, halogénoalkoxy en C₁₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)-, (hétérocycloalkyle comportant 4 à 10 chaînons) - (alkyle en C₁₋₄)-, CN, OH, NH₂, NO₂, NHOR^{c}, OR^{c}, SR^{c}, C(O)R^{c}, C(O)NR^{e}R^{e}, C(O)OR^{c}, OC(O)R^{c}, OC(O)NR^{e}R^{e}, C(=NR^{c})NR^{c}R^{c}, NR^{c}C(=NR^{c})NR^{c}R^{c}, NHR^{c}, NR^{c}R^{c}, NR^{c}C(O)R^{c}, NR^{c}C(O)OR^{c}, NR^{c}C(O)NR^{c}R^{c}, NR^{c}S(O)R^{c}, NR^{c}S(O)₂R^{c}, NR^{c}S(O)₂NR^{c}R^{c}, S(O)R^{c}, S(O)NR^{e}R^{e}, S(O)₂R^{c} et S(O)₂NR^{c}R^{c} ; dans lequel chacun des groupements alkyle en C₁₋₆, halogénoalkyle en C₁₋₆, halogénoalkoxy en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)-(alkyle en C₁₋₄)- de R^{b} est en outre éventuellement substitué par 1, 2 ou 3 substituants R^{d} indépendamment choisis ;
chacun des radicaux R^{c} est indépendamment choisi parmi H et les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{c} est éventuellement substitué par 1, 2, 3, 4 ou 5 substituants R^{f} ;
chacun des radicaux R^{f} est indépendamment choisi parmi les groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)-, (hétérocycloalkyle comportant 4 à 10 chaînons)-(alkyle en C₁₋₄)-, halogéno, CN, NHOR^{g}, OR^{g}, SR^{g}, C(O)R^{g}, C(O)NR^{g}R^{g}, C(O)OR^{g}, OC(O)R^{g}, OC(O)NR^{g}R^{g}, NHR^{g}, NR^{g}R^{g}, NR^{g}C(O)R^{g}, NR^{g}C(O)NR^{g}R^{g}, NR^{g}C(O)OR^{g}, C(=NR^{g})NR^{g}R^{g}, NR^{g}C(=NR^{g})NR^{g}R^{g}, S(O)R^{g}, S(O)NR^{g}R^{g}, S(O)₂R^{g}, NR^{g}S(O)₂R^{g}, NR^{g}S(O)₂NR^{g}R^{g} et S(O)₂NR^{g}R^{g} ; dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{f} est éventuellement substitué par 1, 2, 3, 4 ou 5 substituants Rⁿ ;
chacun des radicaux Rⁿ est indépendamment choisi parmi les groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₅₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)-, (hétérocycloalkyle comportant 4 à 10 chaînons)-(alkyle en C₁₋₄)-, halogéno, CN, NHOR^{o}, OR^{o}, SR^{o}, C(O)R^{o}, C(O)NR^{o}R^{o}, C(O)OR^{o}, OC(O)R^{o}, OC (O)NR^{o}R^{o}, NHR°, NR^{o}R^{o}, NR^{o}C(O)R^{o}, NR^{o}C(O)NR^{o}R^{o}, NR^{o}C(O)OR^{o}, C(=NR^{o})NR^{o}R^{o}, NR^{OC}(=NR^{o})NR^{o}R^{o}, S(O)R^{o}, S(O)NR^{o}R^{o}, S(O)₂R^{o}, NR^{o}(O)₂R^{o}, NR^{o}S(O)₂NR^{o}R^{o} et S(O)₂NR^{o}R^{o}, dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄) -, (cycloalkyle en C₃₋₁₀)-(alkyle en C₁₋₄)-, (hétéroaryle comportant 5 à 10 chaînons) - (alkyle en C₁₋₄)- et (hétérocycloalkyle comportant 4 à 10 chaînons)-(alkyle en C₁₋₄)- de Rⁿ est éventuellement substitué par 1, 2 ou 3 substituants R^{q} indépendamment choisis ;
chacun des radicaux R^{g} est indépendamment choisi parmi H et les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 10 chaînons)-(alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 10 chaînons)- (alkyle en C₁₋₄)- et (hétérocycloalkyle comportant 4 à 10 chaînons)- (alkyle en C₁₋₄)- de R^{g} est éventuellement substitué par 1, 2 ou 3 substituants RP ;
chacun des radicaux RP est indépendamment choisi parmi les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C₆₋₁₀) - (alkyle en C₁₋₄)-, (cycloalkyle en C₃₋₁₀) - (alkyle en C₁₋₄)-, (hétéroaryle comportant 5 à 10 chaînons)- (alkyle en C₁₋₄) -, (hétérocycloalkyle comportant 4 à 10 chaînons)-(alkyle en C₁₋₄)-, halogéno, CN, NHOR^{r}, OR^{r}, SR^{r}, C(O)R^{r}, C(O)NR^{r}R^{r}, C(O)OR^{r}, OC(O)R^{r}, OC(O)NR^{r}R^{r}, NHR^{r}, NR^{r}R^{r}, NR^{r}C(O)R^{r}, NR^{r}C(O)NR^{r}R^{r}, NR^{r}C(O)OR^{r}, C(=NR^{r})NR^{r}R^{r}, NR^{r}C(=NR^{r})NR^{r}R^{r}, NR^{r}C(=NOH)NR^{r}R^{r}, NR^{r}C(=NCN)NR^{r}R^{r}, S(O)R^{r}, S(O)NR^{r}R^{r}, S(O)₂R^{r}, NR^{r}S(O)₂R^{r}, NR^{r}S(O)₂NR^{r}R^{r} et S(O)₂NR^{r}R^{r}, dans lequel chacun des groupements alkyle en C₁₋₄, halogénoalkyle en C₁₋₄, alcényle en C₂₋₆, alcynyle en C₂₋₆, aryle en C₆₋₁₀, cycloalkyle en C₃₋₁₀, hétéroaryle comportant 5 à 10 chaînons, hétérocycloalkyle comportant 4 à 10 chaînons, (aryle en C5₋₁₀)-(alkyle en C₁₋₄)-, (cycloalkyle en C₃₋₁₀)-(alkyle en C₁₋₆)-, (hétéroaryle comportant 5 à 10 chaînons)-(alkyle en C₁₋₄)- et (hétérocycloalkyle comportant 4 à 10 chaînons)- (alkyle en C₁₋₄)- de RP est éventuellement substitué par 1, 2 ou 3 substituants R^{q}; ou deux substituants R^{a} quelconques forment conjointement avec l'atome d'azote auquel ils sont liés un groupement hétérocycloalkyle comportant 4, 5, 6, 7, 8, 9 ou 10 chaînons éventuellement substitué par 1, 2 ou 3 substituants R^{h} ;
chacun des radicaux R^{h} est indépendamment choisi parmi les groupements alkyle en C₁₋₆, cycloalkyle en C₃₋₁₀, hétérocycloalkyle comportant 4 à 7 chaînons, aryle en C₆₋₁₀, hétéroaryle comportant 5 à 6 chaînons, (aryle en C₆₋₁₀)-(alkyle en C₁₋₄)-, (cycloalkyle en C₃₋₁₀)-(alkyle en C₁₋₄)-, (hétéroaryle comportant 5 à 6 chaînons) - (alkyle en C₁₋₄)-, (hétérocycloalkyle comportant 4 à 7 chaînons)- (alkyle en C₁₋₄)-, halogénoalkyle en C₁₋₆, halogénoalkoxy en C₁₋₆, alcényle en C₂₋₆, alcynyle en C₂₋₆, halogéno, CN, ORⁱ, SRⁱ, NHORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ, NRⁱC(O)NRⁱRⁱ, NRⁱC(O)ORⁱ, C(=NRⁱ)NRⁱRⁱ, NRⁱC(=NRⁱ)NRⁱRⁱ, S(O)Rⁱ, S(O)NRⁱRⁱ, S(O)₂Rⁱ, NRⁱS(O)₂Rⁱ, NRⁱS(O)₂NRⁱRⁱ et S(O)2NRⁱRⁱ, dans lequel chacun des groupements alkyle en C₁₋₆, alcényle en C₂₋₆, alcynyle en C₂₋₆, cycloalkyle en C₃₋₁₀, hétérocycloalkyle comportant 4 à 7 chaînons, aryle en C₆₋₁₀, hétéroaryle comportant 5 à 6 chaînons, (aryle en C₆₋₁₀) - (alkyle en C₁₋₄)-, (cycloalkyle en C₃₋₁₀)-(alkyle en C₁₋₄)-, (hétéroaryle comportant 5 à 6 chaînons)-(alkyle en C₁₋₄)-, (hétérocycloalkyle comportant 4 à 7 chaînons) - (alkyle en C₁₋₄)- de R^{h} est éventuellement en outre substitué par 1, 2 ou 3 substituants R^{j} ;
chacun des radicaux R^{j} est indépendamment choisi parmi les groupements cycloalkyle en C₃₋₆, aryle en C₆₋₁₀, hétéroaryle comportant 5 ou 6 chaînons, hétérocycloalkyle comportant 4 à 7 chaînons, alcényle en C₂₋₄, alcynyle en C₂₋₄, halogéno, alkyle en C₁₋₄, halogénoalkyle en C₁₋₄, CN, NHOR^{k}, OR^{k}, SR^{k}, C(O)R^{k}, C(O)NR^{k}R^{k}, C(O)OR^{k}, OC(O)R^{k}, OC(O)NR^{k}R^{k}, NHR^{k}, NR^{k}R^{k}, NR^{k}C(O)R^{k}, NR^{k}C(O)NR^{k}R^{k}, NR^{k}C(O)OR^{k}, C(=NR^{k})NR^{k}R^{k}, NR^{k}C(=NR^{k})NR^{k}R^{k}, S(O)R^{k}, S(O)NR^{k}R^{k}, S(O)₂R^{k}, NR^{k}S(O)₂R^{k}, NR^{k}S(O)₂NR^{k}R^{k} et S(O)₂NR^{k}R^{k}, dans lequel chacun des groupements alkyle en C₁₋₄, cycloalkyle en C₃₋₆, aryle en C₆₋₁₀, hétéroaryle comportant 5 ou 6 chaînons, hétérocycloalkyle comportant 4 à 6 chaînons, alcényle en C₂₋₄, alcynyle en C₂₋₄, halogénoalkyle en C₁₋₄ et halogénoalkoxy en C₁₋₄ de R^{j} est éventuellement substitué par 1, 2 ou 3 substituants R^{q} indépendamment choisis ;
ou deux groupements R^{h} liés au même atome de carbone du groupement hétérocycloalkyle comportant 4 à 10 chaînons forment conjointement avec l'atome de carbone auquel ils sont liés un groupement cycloalkyle en C₃₋₆ ou hétérocycloalkyle comportant 4 à 6 chaînons ayant 1 à 2 hétéroatomes en tant que chaînons choisis parmi O, N ou S ;
ou deux substituants R^{c} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{e} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{g} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R° quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
ou deux substituants R^{r} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
chacun des radicaux Rⁱ, R^{k}, R° ou R^{r} est indépendamment choisi parmi H et les groupements alkyle en C₁₋₄, cycloalkyle en C₃₋₆, aryle en C₆₋₁₀, hétéroaryle comportant 5 ou 6 chaînons, hétérocycloalkyle comportant 4 à 7 chaînons, halogénoalkyle en C₁₋₆, halogénoalkoxy en C₁₋₆, alcényle en C₂₋₄ et alcynyle en C₂₋₄, dans lequel chacun des groupements alkyle en C₁₋₄, cycloalkyle en C₃₋₆, aryle en C₆₋₁₀, hétéroaryle comportant 5 ou 6 chaînons, hétérocycloalkyle comportant 4 à 7 chaînons, alcényle en C₂₋₄ et alcynyle en C₂₋₄ de Rⁱ, R^{k}, R° ou R^{r} est éventuellement substitué par 1, 2 ou 3 substituants R^{q} ;
chacun des radicaux R^{q} est indépendamment choisi parmi les groupements halogéno, OH, CN, -COOH, NH₂, -NH-(alkyle en C₁₋₆), -N(alkyle en C₁₋₆)₂, alkyle en C₁₋₆, alkoxy en C₁₋₆, alkylthio en C₁₋₆, halogénoalkyle en C₁₋₆, halogénoalkoxy en C₁₋₆, phényle, hétéroaryle comportant 5 à 6 chaînons, hétérocycloalkyle comportant 4 à 6 chaînons et cycloalkyle en C₃₋₆, dans lequel chacun des groupements alkyle en C₁₋₆, phényle, cycloalkyle en C₃₋₆, hétérocycloalkyle comportant 4 à 6 chaînons et hétéroaryle comportant 5 à 6 chaînons de R^{q} est éventuellement substitué par 1, 2 ou 3 substituants choisis parmi les groupements halogéno, OH, CN, -COOH, NH₂, alkyle en C₁₋₄, alkoxy en C₁₋₄, halogénoalkyle en C₁₋₄, halogénoalkoxy en C₁₋₄, phényle, cycloalkyle en C₃₋₁₀, hétéroaryle comportant 5 à 6 chaînons et hétérocycloalkyle comportant 4 à 6 chaînons ;
l'indice m représente un entier choisi parmi 0, 1, 2 ou 3 ;
l'indice n représente un entier choisi parmi 0, 1, 2 ou 3 ;
chaque indice q représente indépendamment un entier choisi parmi 1, 2, 3 ou 4 ; et
l'indice p représente un entier choisi parmi 1, 2, 3 ou 4.

3. Composé selon la revendication **2,** répondant à :
(a) la Formule (II) : 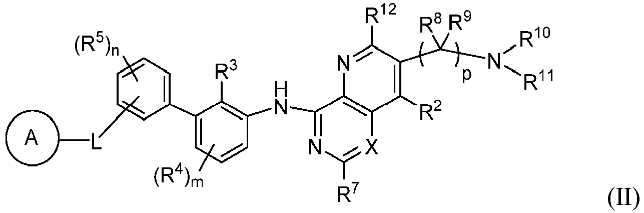 ou un sel pharmaceutiquement acceptable de celui-ci ; ou
(b) la Formule (IIa) : 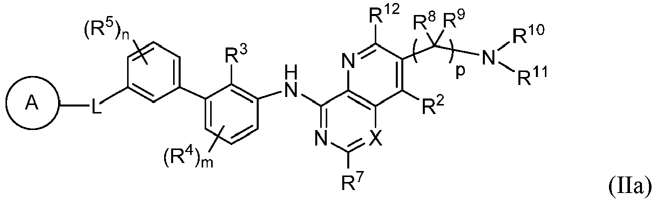 ou un sel pharmaceutiquement acceptable de celui-ci ; ou
(c) la Formule (IIb) : 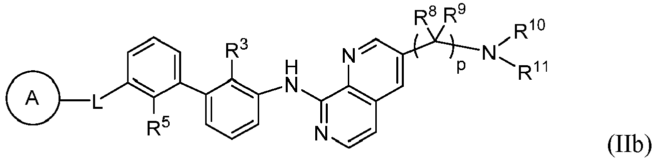 ou un sel pharmaceutiquement acceptable de celui-ci.

4. Composé selon la revendication 2, répondant à :
(a) la Formule (III) : 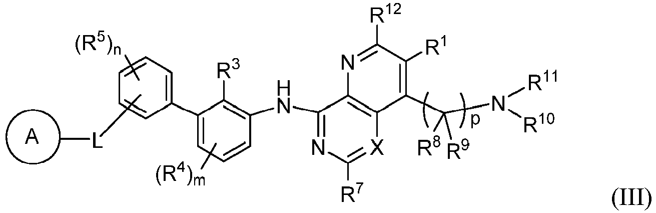 ou un sel pharmaceutiquement acceptable de celui-ci ; ou
(b) la Formule (IIIa) : 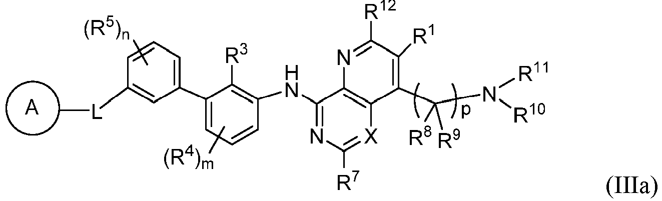 ou un sel pharmaceutiquement acceptable de celui-ci ; ou
(c) la Formule (IIIb) : 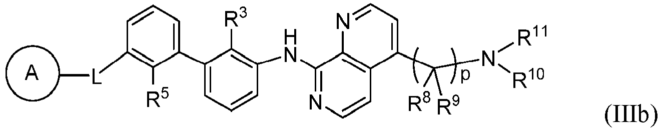 ou un sel pharmaceutiquement acceptable de celui-ci.

5. Composé selon l'une quelconque des revendications 1 à 4, ou un sel pharmaceutiquement acceptable de celui-ci, dans lequel le cycle A est choisi parmi les suivants : 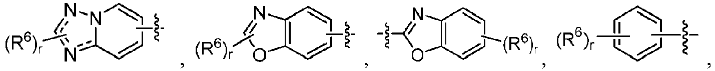 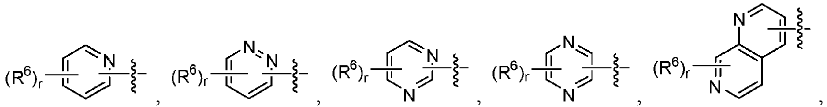 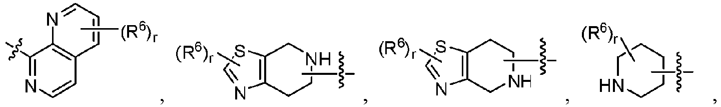 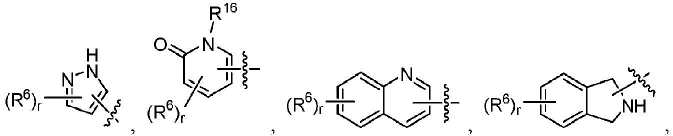 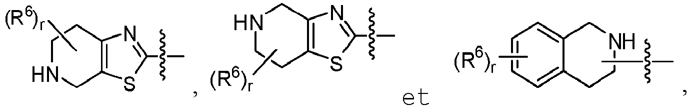 dans lequel chaque indice r représente un entier choisi parmi 1, 2, 3, 4 ou 5 ; R¹⁶ représente un groupement alkyle en C₁₋₆ ; et la ligne ondulée indique le point de liaison à L.

6. Composé selon l'une quelconque des revendications 1 à 5, ou un sel pharmaceutiquement acceptable de celui-ci, dans lequel L représente une liaison, -NH-, - CH=CH- ou -C(O)NH-, dans lequel le groupement carbonyle dans la liaison -C(O)NH- est lié au cycle A.

7. Composé selon l'une quelconque des revendications 1 à 6, ou un sel pharmaceutiquement acceptable de celui-ci, dans lequel l'indice m représente 0.

8. Composé selon l'une quelconque des revendications 1 à 7, ou un sel pharmaceutiquement acceptable de celui-ci, dans lequel l'indice n représente 1 et R⁵ représente un groupement halogéno ou alkyle en C₁₋₄.

9. Composé selon l'une quelconque des revendications **1 à 8,** ou un sel pharmaceutiquement acceptable de celui-ci, dans lequel R³ représente un groupement méthyle, CN ou Cl.

10. Composé selon l'une quelconque des revendications **2 à 9,** ou un sel pharmaceutiquement acceptable de celui-ci, dans lequel R¹² représente H ou un groupement halogéno, CN, alkyle en C₁₋₄ ou alkoxy en C₁₋₄.

11. Composé selon l'une quelconque des revendications **2 à 10,** ou un sel pharmaceutiquement acceptable de celui-ci, dans lequel R⁷ représente H ou un groupement halogéno, CN, alkyle en C₁₋₄, alkoxy en C₁₋₄ ou halogénoalkoxy en C₁₋₄, dans lequel chacun des groupements alkyle en C₁₋₄ et alkoxy en C₁₋₄ de R⁷ est éventuellement substitué par CN.

12. Composé selon l'une quelconque des revendications **2 à 11,** ou un sel pharmaceutiquement acceptable de celui-ci, dans lequel R² représente H.

13. Composé selon l'une quelconque des revendications **2 à 12,** ou un sel pharmaceutiquement acceptable de celui-ci, dans lequel R¹ représente H.

14. Composé selon l'une quelconque des revendications **2 à 13,** ou un sel pharmaceutiquement acceptable de celui-ci, dans lequel l'indice p représente 1.

15. Composé selon l'une quelconque des revendications **2 à 14,** ou un sel pharmaceutiquement acceptable de celui-ci, dans lequel chacun des radicaux R⁸ et R⁹ représente H.

16. Composé selon l'une quelconque des revendications **2 à 15,** ou un sel pharmaceutiquement acceptable de celui-ci, dans lequel R¹⁰ représente H.

17. Composé selon l'une quelconque des revendications **2 à 16,** ou un sel pharmaceutiquement acceptable de celui-ci, dans lequel R¹¹ représentent un groupement 2-hydroxyéthyle, [1-(hydroxyméthyl)cyclopropyl]méthyle, [1-(hydroxyméthyl)cyclobutyl]méthyle ou 2-(diméthylamino)-2-oxo-éthyle.

18. Composé selon l'une quelconque des revendications **2 à 15,** ou un sel pharmaceutiquement acceptable de celui-ci, dans lequel -NR¹⁰R¹¹ représente un groupement (2-hydroxyéthyl)amino, 3-hydroxypyrrolidin-1-yle, 3-carboxypyrrolidin-1-yle, 3-carboxyazétidin-1-yle, 2-carboxy-1-pipéridinyle, 2-oxooxazolidin-3-yle, [1-(hydroxyméthyl)cyclopropyl]méthylamino, [1-(hydroxyméthyl)cyclobutyl]méthylamino ou [2-(diméthylamino)-2-oxo-éthyl]amino.

19. Composé selon l'une quelconque des revendications **1 à 18,** ou un sel pharmaceutiquement acceptable de celui-ci, dans lequel le cycle A représente un groupement 2-pyridyle, éventuellement substitué par 1, 2, 3 ou 4 substituants R⁶ indépendamment choisis.

20. Composé selon l'une quelconque des revendications **1 à 19,** ou un sel pharmaceutiquement acceptable de celui-ci, dans lequel X représente N ou CH.

21. Composé selon la revendication 1 choisi parmi les suivants : 2-(((8-((2-chloro-2'-méthyl-3'-(4,5,6,7-tétrahydrothiazolo[5,4-c]pyridin-2-yl)-[1,1'-biphényl]-3-yl)amino)-1,7-naphtyridin-3-yl)méthyl)amino)éthan-1-ol ;
acide 1-(((6-(2-fluoro-3'-(3-((2-hydroxyéthylamino)méthyl)-1,7-naphtyridin-8-ylamino)-2'-méthylbiphényl-3-ylcarbamoyl)pyridin-3-yl)méthyl)amino)cyclobutanecarboxylique ;
acide (*S*)-1-((6-((2-fluoro-3'-((3-(((2-hydroxyéthyl)amino)méthyl)-1,7-naphtyridin-8-yl)amino)-2'-méthyl-[1,1'-biphényl]-3-yl)carbamoyl)pyridin-3-yl)méthyl)pipéridine-2-carboxylique ;
N-(2-fluoro-3'-((3-(((2-hydroxyéthyl)amino)méthyl)-1,7-naphtyridin-8-yl)amino)-2'-méthyl-[1,1'-biphényl]-3-yl)-5-(((2-hydroxyéthyl)amino)méthyl)picolinamide ;
N-(2-chloro-3'-((3-(((2-hydroxyéthyl)amino)méthyl)-1,7-naphtyridin-8-yl)amino)-2'-méthyl-[1,1'-biphényl]-3-yl)-5-(((2-hydroxyéthyl)amino)méthyl)picolinamide ;
N-(2-chloro-3'-((3-(((*R*)-3-hydroxypyrrolidin-1-yl)méthyl)-1,7-naphtyridin-8-yl)amino)-2'-méthyl-[1,1'-biphényl]-3-yl)-5-(((*R*)-3-hydroxypyrrolidin-1-yl)méthyl)picolinamide ;
acide (*R*)-1-((8-((2'-chloro-3'-(5-(((R)-3-hydroxypyrrolidin-1-yl)méthyl)picolinamido)-2-méthyl-[1,1'-biphényl]-3-yl)amino)-1,7-naphtyridin-3-yl)méthyl)pyrrolidine-3-carboxylique ; et
acide (*R*)-1-((8-((2'-chloro-3'-(5-((3-hydroxypyrrolidin-1-yl)méthyl)picolinamido)-2-méthyl-[1,1'-biphényl]-3-yl)amino)-1,7-naphtyridin-3-yl)méthyl)azétidine-3-carboxylique ;
ou un sel pharmaceutiquement acceptable de ceux-ci.

22. Composé selon la revendication 1 choisi parmi les suivants :
acide (*R*)-1-((8-((3'-((3-(((R)-3-hydroxypyrrolidin-1-yl)méthyl)-1,7-naphtyridin-8-yl)amino)-2,2'-diméthyl-[1,1'-biphényl]-3-yl)amino)-1,7-naphtyridin-3-yl)méthyl)pyrrolidine-3-carboxylique ;
acide (*S*)-1-((8-((3'-((3-(((R)-3-hydroxypyrrolidin-1-yl)méthyl)-1,7-naphtyridin-8-yl)amino)-2,2'-diméthyl-[1,1'-biphényl]-3-yl)amino)-1,7-naphtyridin-3-yl)méthyl)pyrrolidine-3-carboxylique ;
acide (*R*)-1-((8-((3'-((3-(((2-hydroxyéthyl)amino)méthyl)-1,7-naphtyridin-8-yl)amino)-2,2'-diméthyl-[1,1'-biphényl]-3-yl)amino)-1,7-naphtyridin-3-yl)méthyl)pyrrolidine-3-carboxylique ;
acide (*R*)-1-((8-((3'-((3-(((S)-3-hydroxypyrrolidin-1-yl)méthyl)-1,7-naphtyridin-8-yl)amino)-2,2'-diméthyl-[1,1'-biphényl]-3-yl)amino)-1,7-naphtyridin-3-yl)méthyl)pyrrolidine-3-carboxylique ; et
acide (*S*)-1-((8-((3'-((3-(((*S*)-3-hydroxypyrrolidin-1-yl)méthyl)-1,7-naphtyridin-8-yl)amino)-2,2'-diméthyl-[1,1'-biphényl]-3-yl)amino)-1,7-naphtyridin-3-yl)méthyl)pyrrolidine-3-carboxylique ;
ou un sel pharmaceutiquement acceptable de ceux-ci.

23. Composé selon la revendication 1 choisi parmi les suivants :
acide 1-((8-(2-chloro-3'-(1,5-diméthyl-4,5,6,7-tétrahydro-1H-imidazo[4,5-c]pyridine-2-carboxamido)-2'-méthylbiphényl-3-ylamino)-1,7-naphtyridin-3-yl)méthyl)azétidine-3-carboxylique ;
acide (*R*)-1-((5-(2-chloro-3'-(3-(((R)-3-hydroxypyrrolidin-1-yl)méthyl)-1,7-naphtyridin-8-ylamino)-2'-méthylbiphényl-3-ylcarbamoyl)-1-méthyl-6-oxo-1,6-dihydropyridin-3-yl)méthyl)pyrrolidine-3-carboxylique ;
acide (*R*)-1-((8-(2,2'-dichloro-3'-(5-((3-hydroxypyrrolidin-1-yl)méthyl)-1-méthyl-2-oxo-1,2-dihydropyridine-3-carboxamido)biphényl-3-ylamino)-1,7-naphtyridin-3-yl)méthyl)azétidine-3-carboxylique ;
acide 1-((8-(2,2'-dichloro-3'-(5-((2-hydroxyéthylamino)méthyl)-1-méthyl-2-oxo-1,2-dihydropyridine-3-carboxamido)biphényl-3-ylamino)-1,7-naphtyridin-3-yl)méthyl)azétidine-3-carboxylique ;
acide 1-((8-(2,2'-dichloro-3'-(5-((2-hydroxy-2-méthylpropylamino)méthyl)-1-méthyl-2-oxo-1,2-dihydropyridine-3-carboxamido)biphényl-3-ylamino)-1,7-naphtyridin-3-yl)méthyl)azétidine-3-carboxylique ;
2,2'—(((((2,2'-diméthyl-[1,1'-biphényl]-3, 3'-diyl)bis(azanediyl))bis(1,7-naphtyridine-8,3-diyl))bis(méthylène))bis(azanediyl))bis(éthan-1-ol) ;
(3*R*,3'*R*)-1,1'-((((2,2'-diméthyl-[1,1'-biphényl]-3,3'-diyl)bis(azanediyl))bis(1,7-naphtyridine-8,3-diyl))bis(méthylène))bis(pyrrolidin-3-ol) ;
(*R*)-1-((8-(3'-(3-((2-hydroxyéthylamino)méthyl)-1,7-naphtyridin-8-ylamino)-2,2'-diméthylbiphényl-3-ylamino)-1,7-naphtyridin-3-yl)méthyl)pyrrolidin-3-ol ;
(3*R*,3'*R*)-1,1'-((((2,2'-dichloro-[1,1'-biphényl]-3,3'-diyl)bis(azanediyl))bis(1,7-naphtyridine-8,3-diyl))bis(méthylène))bis(pyrrolidin-3-ol) ;
(*R*)-1-((4-(3'-(3-(((R)-3-hydroxypyrrolidin-1-yl)méthyl)-1,7-naphtyridin-8-ylamino)-2,2'-diméthylbiphényl-3-ylamino)pyrido[3,2-d]pyrimidin-7-yl)méthyl)pyrrolidin-3-ol ;
(*R*)-1-((8-(3'-(7-((2-hydroxyéthylamino)méthyl)pyrido[3,2-d]pyrimidin-4-ylamino)-2,2'-diméthylbiphényl-3-ylamino)-1,7-naphtyridin-3-yl)méthyl)pyrrolidin-3-ol ;
(*R*)-1-((8-(3'-(7-(((2-hydroxyéthyl)(méthyl)amino)méthyl)pyrido[3,2-d]pyrimidin-4-ylamino)-2,2'-diméthylbiphényl-3-ylamino)-1,7-naphtyridin-3-yl)méthyl)pyrrolidin-3-ol ;
(*R*)-1-((8-(3'-(3-(((R)-3-hydroxypyrrolidin-1-yl)méthyl)-1,7-naphtyridin-8-ylamino)-2,2'-diméthylbiphényl-3-ylamino)-1,7-naphtyridin-3-yl)méthyl)-N,N-diméthylpyrrolidine-3-carboxamide (*R*)-1-((8-(3'-(7-(((*S*)-2-hydroxypropylamino)méthyl)pyrido[3,2-d]pyrimidin-4-ylamino)-2,2'-diméthylbiphényl-3-ylamino)-1,7-naphtyridin-3-yl)méthyl)pyrrolidin-3-ol ;
acide (*R*)-1-((8-(3'-(5-(((*R*)-3-hydroxypyrrolidin-1-yl)méthyl)-1-méthyl-2-oxo-1,2-dihydropyridine-3-carboxamido)-2,2'-diméthylbiphényl-3-ylamino)-1,7-naphtyridin-3-yl)méthyl)pyrrolidine-3-carboxylique ;
(*R*)-1-((8-(3'-(7-(((*R*)-2-hydroxypropylamino)méthyl)pyrido[3,2-d]pyrimidin-4-ylamino)-2,2'-diméthylbiphényl-3-ylamino)-1,7-naphtyridin-3-yl)méthyl)pyrrolidin- 3-ol ;
(*R*)-1-((8-(3'-(7-((2-hydroxy-2-méthylpropylamino)méthyl)pyrido[3,2-d]pyrimidin-4-ylamino)-2,2'-diméthylbiphényl-3-ylamino)-1,7-naphtyridin-3-yl)méthyl)pyrrolidin-3-ol ;
acide (*R*)-1-((8-(2'-chloro-3'-(1,5-diméthyl-4,5,6,7-tétrahydro-1*H*-imidazo[4,5-c]pyridine-2-carboxamido)-2-méthylbiphényl-3-ylamino)-1,7-naphtyridin-3-yl)méthyl)pyrrolidine-3-carboxylique ;
(*S*)-N-(2-chloro-3'-(3-((3-hydroxypyrrolidin-1-yl)méthyl)-1,7-naphtyridin-8-ylamino)-2'-méthylbiphényl-3-yl)-1,5-diméthyl-4,5,6,7-tétrahydro-1*H*-imidazo[4,5-*c*]pyridine-2-carboxamide ;
acide (*R*)-1-((8-(2'-chloro-3'-(1,5-diméthyl-4,5,6,7-tétrahydro-1*H*-imidazo[4,5-c]pyridine-2-carboxamido)-2-méthylbiphényl-3-ylamino)-1,7-naphtyridin-3-yl)méthyl)-3-méthylpyrrolidine-3-carboxylique ;
acide (*R*)-1-((8-((2,2'-diméthyl-3'-(4,5,6,7-tétrahydrothiazolo[5,4-c]pyridin-2-yl)-[1,1'-biphényl]-3-yl)amino)-1,7-naphtyridin-3-yl)méthyl)pyrrolidine-3-carboxylique ;
(*R*)-1-((8-((2,2'-diméthyl-3'-(4,5,6,7-tétrahydrothiazolo[5,4-c]pyridin-2-yl)-[1,1'-biphényl]-3-yl)amino)-1,7-naphtyridin-3-yl)méthyl)pyrrolidin-3-ol ;
(*S*)-1-((8-((2,2'-diméthyl-3'-(4,5,6,7-tétrahydrothiazolo[5,4-c]pyridin-2-yl)-[1,1'-biphényl]-3-yl)amino)-1,7-naphtyridin-3-yl)méthyl)pyrrolidin-3-ol ;
(*R*)-2-(diméthylamino)-1-(2-(3'-((3-((3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-d]oxazol-5-yl)éthan-1-one ;
(*S*)-2-(diméthylamino)-1-(2-(3'-((3-((3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-d]oxazol-5-yl)éthan-1-one ;
acide (*R*)-1-(2-(2-(3'-((3-((3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-d]oxazol-5-yl)-2-oxoéthyl)azétidine-3-carboxylique ;
acide (*S*)-1-(2-(2-(3'-((3-(((*R*)-3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-d]oxazol-5-yl)-2-oxoéthyl)pyrrolidine-3-carboxylique ;
acide (*R*)-1-(2-(2-(3'-((3-(((R)-3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-*d*]oxazol-5-yl)-2-oxoéthyl)pyrrolidine-3-carboxylique ;
acide (*S*)-1-(2-(2-(3'-((3-(((*R*)-3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-*d*]oxazol-5-yl)-2-oxoéthyl)pipéridine-2-carboxylique ;
acide (*S*)-1-(5-chloro-2-((5-cyanopyridin-3-yl)méthoxy)-4-((3'-((3-(((*R*)-3-hydroxypyrrolidin-1-yl)méthyl)-1,7-naphtyridin-8-yl)amino)-2,2'-diméthyl-[1,1'-biphényl]-3-yl)méthoxy)benzyl)pipéridine-2-carboxylique ; et
acide (*R*)-1-(5-chloro-2-((5-cyanopyridin-3-yl)méthoxy)-4-((3'-((3-(((*R*)-3-hydroxypyrrolidin-1-yl)méthyl)-1,7-naphtyridin-8-yl)amino)-2,2'-diméthyl-[1,1'-biphényl]-3-yl)méthoxy)benzyl)pyrrolidine-3-carboxylique ;
ou un sel pharmaceutiquement acceptable de ceux-ci.

24. Composé selon la revendication 1 choisi parmi les suivants :
acide (*R*)-1-((8-(2'-chloro-2-méthyl-3'-(1-méthyl-4,5,6,7-tétrahydro-1*H*-imidazo[4,5-*c*]pyridine-2-carboxamido)biphényl-3-ylamino)-1,7-naphtyridin-3-yl)méthyl)-3-méthylpyrrolidine-3-carboxylique ;
acide (*R*)-1-((8-(3'-(1,5-diméthyl-4,5,6,7-tétrahydro-1*H*-imidazo[4,5-*c*]pyridine-2-carboxamido)-2,2'-diméthylbiphényl-3-ylamino)-1,7-naphtyridin-3-yl)méthyl)pyrrolidine-3-carboxylique ;
acide *trans*-4-((2-(2-chloro-3'-(3-(((*R*)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-c]pyridin-5(4*H*)-yl)méthyl)cyclohexanecarboxylique ;
acide *cis*-4-((2-(2-chloro-3'-(3-(((*R*)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)méthyl)cyclohexanecarboxylique ;
acide *cis*-4-((2-(2-chloro-2'-méthyl-3'-(3-(pyrrolidin-1-ylméthyl)-1,7-naphtyridin-8-ylamino)biphényl-3-ylcarbamoyl)-1-méthyl-6,7-dihydro-1*H*-imidazo[4,5-c]pyridin-5(4*H*)-yl)méthyl)cyclohexanecarboxylique ;
acide *trans*-4-((2-(2-chloro-3'-(3-(((*S*)-1-hydroxypropan-2-ylamino)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)méthyl)cyclohexanecarboxylique ;
acide *trans*-4-((2-(2-chloro-3'-(3-(((1S,2S)-2-hydroxycyclopentylamino)méthyl)-1,7-naphtyridin-8-ylamino)-2'-méthylbiphényl-3-ylcarbamoyl)-1-méthyl-6,7-dihydro-1*H*-imidazo[4,5-c]pyridin-5(4*H*)-yl)méthyl)cyclohexanecarboxylique ;
acide *trans* 4-(2-(2-(2-chloro-3'-(3-(((R)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-c]pyridin-5(4*H*)-yl)éthyl)cyclohexanecarboxylique ;
acide *cis* 4-(2-(2-(2-chloro-3'-(3-(((*R*)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide 3-(2-(2-chloro-3'-(3-(((*R*)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H-*imidazo[4,5-*c*]pyridin-5(4*H*)-yl)butanoïque ;
acide *cis* 4-((2-(2-chloro-3'-(3-(((*S*)-3-hydroxypyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)méthyl)cyclohexanecarboxylique ;
acide *cis* 4-((2-(2-chloro-3'-(3-(((*R*)-3-hydroxy-3-méthylpyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)méthyl)cyclohexanecarboxylique ;
acide (*R*)-4-(2-(2-chloro-3'-(3-((3-hydroxy-3-méthylpyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)cyclohexanecarboxylique ;
acide (*S*)-4-(2-(2-chloro-3'-(3-((3-hydroxy-3-méthylpyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)cyclohexanecarboxylique;
acide *trans* 4-(2-(2-(2-chloro-3'-(3-(((*R*)-1-hydroxypropan-2-ylamino)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide *trans* 4-(2-(2-(2-chloro-3'-(3-(((*S*)-1-hydroxypropan-2-ylamino)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide trans-4-(2-(2-(2-chloro-3'-(3-(((*R*)-3-hydroxy-3-méthylpyrrolidin-1-yl)méthyl)-1,7-naphtyridin-8-ylamino)-2'-méthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide (*R*)-4-(2-(3'-(3-((3-hydroxypyrrolidin-1-yl)méthyl)-1,7-naphtyridin-8-ylamino)-2,2'-diméthylbiphényl-3-ylcarbamoyl)-1-méthyl-6,7-dihydro-1*H*-imidazo[4,5-*c*]pyridin-5(4*H*)-yl)-1-méthylcyclohexanecarboxylique ;
acide *trans*-4-(2-(2-(3'-(3-(((*R*)-3-hydroxypyrrolidin-1-yl)méthyl)-1,7-naphtyridin-8-ylamino)-2,2'-diméthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide (*R*)-4-(2-(3'-(3-((3-hydroxypyrrolidin-1-yl)méthyl)-1,7-naphtyridin-8-ylamino)-2,2'-diméthylbiphényl-3-ylcarbamoyl)-1-méthyl-6,7-dihydro-1*H*-imidazo[4,5-*c*]pyridin-5(4*H*)-yl)cyclohexanecarboxylique ;
acide *trans-*4-(2-(2-(2,2'-dichloro-3'-(3-(((*R*)-3-hydroxypyrrolidin-1-yl)méthyl)-1,7-naphtyridin-8-ylamino)biphényl-3-ylcarbamoyl)-1-méthyl-6,7-dihydro-1*H*-imidazo[4,5-*c*]pyridin-5(4*H*)-yl)éthyl)cyclohexanecarboxylique ;
acide *trans* 4-(2-(2-(2'-chloro-3'-(3-(((*R*)-3-hydroxypyrrolidin-1-yl)méthyl)-1,7-naphtyridin-8-ylamino)-2-méthylbiphényl-3-ylcarbamoyl)-1-méthyl-6,7-dihydro-1*H*-imidazo[4,5-*c*]pyridin-5(4*H*)-yl)éthyl)cyclohexanecarboxylique ;
acide (*R*)-1-((4-(2'-chloro-3'-(1,5-diméthyl-4,5,6,7-tétrahydro-1*H*-imidazo[4,5-c]pyridine-2-carboxamido)-2-méthylbiphényl-3-ylamino)pyrido[3,2-*d*]pyrimidin-7-yl)méthyl)-3-méthylpyrrolidine-3-carboxylique acide (*R*)-4-(2-(2-chloro-3'-(7-((3-hydroxypyrrolidin-1-yl)méthyl)pyrido[3,2-*d*]pyrimidin-4-ylamino)-2'-méthylbiphényl-3-ylcarbamoyl)-1-méthyl-6,7-dihydro-1*H-*imidazo[4,5-*c*]pyridin-5(4*H*)-yl)-1-méthylcyclohexanecarboxylique ;
acide *trans* 4-((2-(2-chloro-3'-(7-(((*R*)-3-hydroxypyrrolidin-1-yl)méthyl)pyrido[3,2-d]pyrimidin-4-ylamino)-2'-méthylbiphényl-3-ylcarbamoyl)-1-méthyl-6,7-dihydro-1*H*-imidazo[4,5-*c*]pyridin-5(4*H*)-yl)méthyl)cyclohexanecarboxylique ;
acide (*R*)-1-((5-(3'-(3-(((*R*)-3-hydroxypyrrolidin-1-yl)méthyl)-1,7-naphtyridin-8-ylamino)-2,2'-diméthylbiphényl-3-ylamino)pyrido[4,3-b]pyrazin-2-yl)méthyl)pyrrolidine-3-carboxylique ;
acide (3R)-1-((8-(2,2'-diméthyl-3'-(3-(pyrrolidin-2-yl)-1,7-naphtyridin-8-ylamino)biphényl-3-ylamino)-1,7-naphtyridin-3-yl)méthyl)pyrrolidine-3-carboxylique ;
(*R*)-1-((8-(2,2'-dichloro-3'-(3-((2-hydroxyéthylamino)méthyl)imidazo[1,2-*a*]pyrazin-8-ylamino)biphényl-3-ylamino)-1,7-naphtyridin-3-yl)méthyl)pyrrolidin-3-ol ;
(*R*)-1-((8-((2,2'-diméthyl-3'-((3-(pyrrolidin-1-ylméthyl)-1,7-naphtyridin-8-yl)amino)-[1,1'-biphényl]-3-yl)amino)-1,7-naphtyridin-3-yl)méthyl)pyrrolidin-3-ol ;
(*S*)-1-((8-((2'-chloro-3'-((3-(((*R*)-3-hydroxypyrrolidin-1-yl)méthyl)-1,7-naphtyridin-8-yl)amino)-2-méthyl-[1,1'-biphényl]-3-yl)amino)-1,7-naphtyridin-3-yl)méthyl)pyrrolidin-3-ol ;
acide (*R*)-1-((8-((2'-chloro-3'-((3-((3-hydroxypyrrolidin-1-yl)méthyl)-1,7-naphtyridin-8-yl)amino)-2-méthyl-[1,1'-biphényl]-3-yl)amino)-1,7-naphtyridin-3-yl)méthyl)azétidine-3-carboxylique ;
(*R*)-1-((8-((2,2'-dichloro-3'-((3-(((R)-3-hydroxypyrrolidin-1-yl)méthyl)-1,7-naphtyridin-8-yl)amino)-[1,1'-biphényl]-3-yl)amino)-1,7-naphtyridin-3-yl)méthyl)-3-méthylpyrrolidin-3-ol ;
(*R*)-1-((8-((2,2'-dichloro-3'-((3-(((2-hydroxyéthyl)amino)méthyl)-1,7-naphtyridin-8-yl)amino)-[1,1'-biphényl]-3-yl)amino)-1,7-naphtyridin-3-yl)méthyl)pyrrolidin-3-ol ;
acide (*R*)-1-((8-((2,2'-dichloro-3'-((3-(((R)-3-hydroxypyrrolidin-1-yl)méthyl)-1,7-naphtyridin-8-yl)amino)-[1,1'-biphényl]-3-yl)amino)-1,7-naphtyridin-3-yl)méthyl)pyrrolidine-3-carboxylique ;
acide (*R*)-1-((8-((2-chloro-3'-((3-(((*R*)-3-hydroxypyrrolidin-1-yl)méthyl)-1,7-naphtyridin-8-yl)amino)-2'-méthyl-[1,1'-biphényl]-3-yl)amino)-1,7-naphtyridin-3-yl)méthyl)pyrrolidine-3-carboxylique ;
acide (*R*)-1-((8-((2-chloro-3'-((3-((3-hydroxypyrrolidin-1-yl)méthyl)-1,7-naphtyridin-8-yl)amino)-2'-méthyl-[1,1'-biphényl]-3-yl)amino)-1,7-naphtyridin-3-yl)méthyl)azétidine-3-carboxylique ;
acide (*R*)-3-(((8-((2-chloro-3'-((3-((3-hydroxypyrrolidin-1-yl)méthyl)-1,7-naphtyridin-8-yl)amino)-2'-méthyl-[1,1'-biphényl]-3-yl)amino)-1,7-naphtyridin-3-yl)méthyl)amino)propanoïque ;
acide (*R*)-1-((8-((2,2'-dichloro-3'-((3-(((*S*)-3-hydroxypyrrolidin-1-yl)méthyl)-1,7-naphtyridin-8-yl)amino)-[1,1'-biphényl]-3-yl)amino)-1,7-naphtyridin-3-yl)méthyl)pyrrolidine-3-carboxylique ;
acide (*S*)-1-((8-((2,2'-dichloro-3'-((3-(((*S*)-3-hydroxypyrrolidin-1-yl)méthyl)-1,7-naphtyridin-8-yl)amino)-[1,1'-biphényl]-3-yl)amino)-1,7-naphtyridin-3-yl)méthyl)pyrrolidine-3-carboxylique ;
acide (*R*)-1-((8-((3'-(5-(diméthylglycyl)-5,6-dihydro-4*H*-pyrrolo[3,4-*d*]oxazol-2-yl)-2,2'-diméthyl-[1,1'-biphényl]-3-yl)amino)-1,7-naphtyridin-3-yl)méthyl)pyrrolidine-3-carboxylique ;
acide (*R*)-1-((8-((3'-(5-(diméthylglycyl)-5,6-dihydro-4H-pyrrolo[3,4-d]thiazol-2-yl)-2,2'-diméthyl-[1,1'-biphényl]-3-yl)amino)-1,7-naphtyridin-3-yl)méthyl)pyrrolidine-3-carboxylique ;
2-((*R*)-3-hydroxypyrrolidin-1-yl)-1-(2-(3'-((3-(((*R*)-3-hydroxypyrrolidin-1-yl)méthyl)-1,7-naphtyridin-8-yl)amino)-2,2'-diméthyl-[1,1'-biphényl]-3-yl)-4,6-dihydro-5H-pyrrolo[3,4-d]thiazol-5-yl)éthan-1-one ;
acide (*R*)-1-((8-((3'-(5-(diméthylglycyl)-5,6-dihydro-4*H*-pyrrolo[3,4-*d*]thiazol-2-yl)-2,2'-diméthyl-[1,1'-biphényl]-3-yl)amino)-1,7-naphtyridin-3-yl)méthyl)-3-méthylpyrrolidine-3-carboxylique ;
acide 1-((8-((3'-(5-(diméthylglycyl)-5,6-dihydro-4*H-*pyrrolo[3,4-*d*]thiazol-2-yl)-2,2'-diméthyl-[1,1'-biphényl]-3-yl)amino)-1,7-naphtyridin-3-yl)méthyl)azétidine-3-carboxylique ;
acide (*R*)-1-((8-((2-chloro-3'-(5-(diméthylglycyl)-5,6-dihydro-4*H*-pyrrolo[3,4-*d*]thiazol-2-yl)-2'-méthyl-[1,1'-biphényl]-3-yl)amino)-1,7-naphtyridin-3-yl)méthyl)pyrrolidine-3-carboxylique ;
acide (*R*)-1-((8-((2-chloro-3'-(5-(N-éthyl-N-méthylglycyl)-5,6-dihydro-4*H*-pyrrolo[3,4-d]thiazol-2-yl)-2'-méthyl-[1,1'-biphényl]-3-yl)amino)-1,7-naphtyridin-3-yl)méthyl)pyrrolidine-3-carboxylique ;
acide (*R*)-2-(1-((8-((2-chloro-3'-(5-(diméthylglycyl)-5,6-dihydro-4*H*-pyrrolo[3,4-*d*]thiazol-2-yl)-2'-méthyl-[1,1'-biphényl]-3-yl)amino)-1,7-naphtyridin-3-yl)méthyl)pyrrolidin-3-yl)acétique ;
acide 2-((8-((2-chloro-3'-(5-(diméthylglycyl)-5,6-dihydro-4*H*-pyrrolo[3,4-d]thiazol-2-yl)-2'-méthyl-[1,1'-biphényl]-3-yl)amino)-1,7-naphtyridin-3-yl)méthyl)-2-azabicyclo[2.2.1]heptane-5-carboxylique ;
acide (*R*)-2-(1-((8-(2-chloro-3'-(5-(2-(éthyl(méthyl)amino)acétyl)-5,6-dihydro-4*H*-pyrrolo[3,4-*d*]thiazol-2-yl)-2'-méthylbiphényl-3-ylamino)-1,7-naphtyridin-3-yl)méthyl)pyrrolidin-3-yl)acétique ;
acide 2-((8-(2-chloro-3'-(5-(2-(éthyl(méthyl)amino)acétyl)-5,6-dihydro-4*H*-pyrrolo[3,4-*d*]thiazol-2-yl)-2'-méthylbiphényl-3-ylamino)-1,7-naphtyridin-3-yl)méthyl)-2-azabicyclo[2.2.1]heptane-5-carboxylique ;
acide (*R*)-1-((8-((2-chloro-3'-(5-(2-((*R*)-3-hydroxypyrrolidin-1-yl)acétyl)-5,6-dihydro-4*H-*pyrrolo[3,4-*d*]thiazol-2-yl)-2'-méthyl-[1,1'-biphényl]-3-yl)amino)-1,7-naphtyridin-3-yl)méthyl)pyrrolidine-3-carboxylique ;
acide (*R*)-1-((8-((2-chloro-3'-(5-(N-(2-hydroxyéthyl)-N-méthylglycyl)-5,6-dihydro-4*H*-pyrrolo[3,4-d]thiazol-2-yl)-2'-méthyl-[1,1'-biphényl]-3-yl)amino)-1,7-naphtyridin-3-yl)méthyl)pyrrolidine-3-carboxylique ;
acide (*R*)-1-((8-((2-chloro-3'-(5-(2-((*S*)-3-hydroxypyrrolidin-1-yl)acétyl)-5,6-dihydro-4*H-*pyrrolo[3,4-*d*]thiazol-2-yl)-2'-méthyl-[1,1'-biphényl]-3-yl)amino)-1,7-naphtyridin-3-yl)méthyl)pyrrolidine-3-carboxylique ;
acide (*R*)-1-((8-((2-chloro-3'-(5-(2-(3-hydroxyazétidin-1-yl)acétyl)-5,6-dihydro-4*H*-pyrrolo[3,4-d]thiazol-2-yl)-2'-méthyl-[1,1'-biphényl]-3-yl)amino)-1,7-naphtyridin-3-yl)méthyl)pyrrolidine-3-carboxylique ; et
acide *cis*-4-((2-(3'-(3-(((*R*)-3-hydroxypyrrolidin-1-yl)méthyl)-1,7-naphtyridin-8-ylamino)-2,2'-diméthylbiphényl-3-ylcarbamoyl)-1-méthyl-6,7-dihydro-1*H*-imidazo[4,5-c]pyridin-5(4*H*)-yl)méthyl)cyclohexanecarboxylique ;
ou un sel pharmaceutiquement acceptable de ceux-ci.

25. Composé selon la revendication 2, ou un sel pharmaceutiquement acceptable de celui-ci, dans lequel :
le cycle A représente un groupement hétéroaryle comportant 5 à 10 chaînons ou hétérocycloalkyle comportant 4 à 11 chaînons, dans lequel les groupements hétéroaryle comportant 5 à 10 chaînons et hétérocycloalkyle comportant 4 à 11 chaînons comportent chacun 1 à 4 hétéroatomes en tant que chaînons choisis parmi N, O et S, dans lequel l'atome N ou S en tant que chaînon est éventuellement oxydé et un ou plusieurs atomes de carbone en tant que chaînons sont chacun éventuellement remplacés par un groupement carbonyle ;
et dans lequel le cycle A est éventuellement substitué par 1, 2 ou 3 substituants R⁶ ;
L représente une liaison, -C(O)NR¹³-, -NR¹³C(O)-, -NR¹³-, ou CH=CH- ;
X représente N ou CR¹⁷, dans lequel R¹⁷ représente H ou un groupement alkyle en C₁₋₄, alkoxy en C₁₋₄, halogénoalkyle en C₁₋₄, halogénoalkoxy en C₁₋₄, CN, halogéno ou OH, dans lequel chacun des groupements alkyle en C₁₋₄ et alkoxy en C₁₋₄ est éventuellement substitué par 1 ou 2 substituants indépendamment choisis parmi les groupements CN, halogéno et -C(O)NH₂ ;
l'un des radicaux R¹ et R² représente - (CR⁸R⁹)ₚ-NR¹⁰R¹¹ et l'autre représente H ou un groupement alkyle en C₁₋₄, alkoxy en C₁₋₄, halogénoalkyle en C₁₋₄, halogénoalkoxy en C₁₋₄, CN, halogéno ou OH, dans lesquels le groupement alkyle en C₁₋₄ et alkoxy en C₁₋₄ de R¹ ou R² est éventuellement substitué par 1 ou 2 substituants indépendamment choisis parmi les groupements alkoxy en C₁₋₄, halogénoalkyle en C₁₋₄, halogénoalkoxy en C₁₋₄, CN, halogéno et OH ; R³ représente un groupement méthyle, halogéno, CN ou halogénoalkyle en C₁₋₄ ; R⁴ représente un groupement alkyle en C₁₋₄, alkoxy en C₁₋₄ ou halogénoalkyle en C₁₋₄ ;
R⁵ représente un groupement alkyle en C₁₋₄, alkoxy en C₁₋₄, halogénoalkyle en C₁₋₄, halogénoalkoxy en C₁₋₄, CN, halogéno ou OH ;
chacun des radicaux R⁶ est indépendamment choisi parmi H et les groupements halogéno, alkyle en C₁₋₆, alcényle en C₂₋₆, alcynyle en C₂₋₆, halogénoalkyle en C₁₋₆, halogénoalkoxy en C₁₋₆, hétéroaryle comportant 5 à 14 chaînons, hétérocycloalkyle comportant 4 à 10 chaînons, (hétéroaryle comportant 5 à 14 chaînons)-alkyle en C₁₋₄-, (hétérocycloalkyle comportant 4 à 10 chaînons)-(alkyle en C₁₋₄)-, CN, NO₂, OR^{a}, C(O)R^{a}, C(O)NR^{a}R^{a}, C(O)OR^{a}, OC(O)R^{a}, OC(O)NR^{a}R^{a}, NHR^{a}, NR^{a}R^{a}, NR^{a}C(O)R^{a} ou NR^{a}C(O)OR^{a}, dans lesquels chacun des groupements alkyle en C₁₋₆, alcényle en C₂₋₆, alcynyle en C₂₋₆, hétéroaryle comportant 5 à 14 chaînons, hétérocycloalkyle comportant 4 à 10 chaînons, (hétéroaryle comportant 5 à 14 chaînons)-(alkyle en C₁₋₄)- et (hétérocycloalkyle comportant 4 à 10 chaînons)- (alkyle en C₁₋₄)- de R⁶ est éventuellement substitué par 1, 2 ou 3 substituants R^{b} ;
R⁷ représente H ou un groupement alkyle en C₁₋₄, alkoxy en C₁₋₄, halogénoalkyle en C₁₋₄, halogénoalkoxy en C₁₋₄, CN, halogéno ou OH ;
chacun des radicaux R⁸ et R⁹ est indépendamment choisi parmi H et les groupements halogéno, CN, OH, -COOH, alkyle en C₁₋₄, alkoxy en C₁₋₄, -NH-alkyle en C₁₋₄, - N(alkyle en C₁₋₄)₂ et halogénoalkyle en C₁₋₄ ;
chacun des radicaux R¹⁰ et R¹¹ est indépendamment choisi parmi H et les groupements alkyle en C₁₋₆, halogénoalkyle en C₁₋₆, -C(O)R^{g}, -C(O)OR^{g} et -C(O)NR^{g}R^{g}, dans lequel chacun des groupements alkyle en C₁₋₆ et halogénoalkyle en C₁₋₆ de R¹⁰ ou R¹¹ est éventuellement substitué par 1 ou 2 substituants R^{f} indépendamment choisis ;
ou R¹⁰ et R¹¹ forment conjointement avec l'atome d'azote auquel ils sont liés un groupement hétérocycloalkyle comportant 4, 5, 6 ou 7 chaînons, dans lequel le groupement hétérocycloalkyle comportant 4, 5, 6 ou 7 chaînons est éventuellement substitué par 1, 2 ou 3 substituants R^{h} ;
R¹² représente H ou un groupement alkyle en C₁₋₄, alkoxy en C₁₋₄, halogénoalkyle en C₁₋₄, halogénoalkoxy en C₁₋₄, CN, halogéno ou OH ;
chacun des radicaux R¹³ représente indépendamment H ou un groupement halogénoalkyle en C₁₋₆ ou alkyle en C₁₋₆ ;
chacun des radicaux R^{a} est indépendamment choisi parmi H et les groupements CN, alkyle en C₁₋₆, halogénoalkyle en C₁₋₄, alcényle en C₂₋₆ et alcynyle en C₂₋₆ ;
chacun des radicaux R^{d} est indépendamment choisi parmi les groupements alkyle en C₁₋₆, halogénoalkyle en C₁₋₆, halogéno, CN, NH₂, OR^{e}, C(O)R^{e}, C(O)NR^{e}R^{e}, C(O)OR^{e}, OC(O)R^{e}, OC(O)NR^{e}R^{e}, NHR^{e}, NR^{e}R^{e} et NR^{e}C(O)R^{e} ;
chacun des radicaux R^{e} est indépendamment choisi parmi H et les groupements alkyle en C₁₋₆, halogénoalkyle en C₁₋₆, alcényle en C₂₋₆ et alcynyle en C₂₋₆ ;
chacun des substituants R^{b} est indépendamment choisi parmi les groupements halogéno, alkyle en C₁₋₆, halogénoalkyle en C₁₋₆, halogénoalkoxy en C₁₋₆, CN, OH, NH₂, NO², OR^{c}, C(O)R^{c}, C(O)NR^{c}R^{c}, C(O)OR^{c}, OC(O)R^{c}, OC(O)NR^{c}R^{c}, C(=NR^{c})NR^{c}R^{c}, NR^{c}C(=NR^{c})NR^{c}R^{c}, NHR^{c}, NR^{c}R^{c}, NR^{c}C(O)R^{c} et NR^{c}C(O)OR^{c} ; dans lesquels chacun des groupements alkyle en C₁₋₄, halogénoalkyle en C₁₋₄ et halogénoalkoxy en C₁₋₄ de R^{b} est en outre éventuellement substitué par 1 ou 2 substituants R^{d} indépendamment choisis ;
chacun des radicaux R^{c} est indépendamment choisi parmi H et les groupements alkyle en C₁₋₆, halogénoalkyle en C₁₋₄, alcényle en C₂₋₆, alcynyle en C₂₋₆, aryle en C₆₋₁₀, cycloalkyle en C₃₋₁₀, (aryle en C₆₋₁₀)-(alkyle en C₁₋₄)- et (cycloalkyle en C₃₋₁₀)-(alkyle en C₁₋₄)-, dans lesquels chacun des groupements alkyle en C₁₋₆, alcényle en C₂₋₆, alcynyle en C₂₋₆, aryle en C₆₋₁₀, cycloalkyle en C₃₋₁₀, (aryle en C₆₋₁₀)-(alkyle en C₁₋₄)- et (cycloalkyle en C₃₋₁₀)-(alkyle en C₁₋₄)- de R^{c} est éventuellement substitué par 1, 2 ou 3 substituants R^{f} ;
chacun des radicaux R^{f} est indépendamment choisi parmi les groupements alkyle en C₁₋₄, halogénoalkyle en C₁₋₄, alcényle en C₂₋₆, alcynyle en C₂₋₆, halogéno, CN, OR^{g}, C(O)R^{g}, C(O)NR^{g}R^{g}, C(O)OR^{g}, OC(O)R^{g}, OC(O)NRSR^{g}, NHR^{g}, NR^{g}R^{g}, NR^{g}C(O)R^{g} et NR^{g}C(O)OR^{g} ;
chacun des radicaux R^{g} est indépendamment choisi parmi H et les groupements alkyle en C₁₋₆, halogénoalkyle en C₁₋₄, alcényle en C₂₋₆ et alcynyle en C₂₋₆ ;
chacun des radicaux R^{h} est indépendamment choisi parmi les groupements alkyle en C₁₋₆, halogénoalkyle en C₁₋₆, halogénoalkoxy en C₁₋₆, alcényle en C₂₋₆, alcynyle en C₂₋₆, halogéno, CN, ORⁱ, C(O)Rⁱ, C(O)NRⁱRⁱ, C(O)ORⁱ, OC(O)Rⁱ, OC(O)NRⁱRⁱ, NHRⁱ, NRⁱRⁱ, NRⁱC(O)Rⁱ et NRⁱC(O)ORⁱ, dans lesquels chacun des groupements alkyle en C₁₋₆, alcényle en C₂₋₆ et alcynyle en C₂₋₆ de R^{h} est éventuellement en outre substitué par 1, 2 ou 3 substituants R^{j} ;
chacun des radicaux R^{j} est indépendamment choisi parmi les groupements alcényle en C₂₋₄, alcynyle en C₂₋₄, halogéno, alkyle en C₁₋₄, halogénoalkyle en C₁₋₄ et CN ;
ou deux substituants R^{c} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
chacun des radicaux R¹ est indépendamment choisi parmi H et les groupements alkyle en C₁₋₄, halogénoalkyle en C₁₋₆, halogénoalkoxy en en C₁₋₆, alcényle en C₂₋₄ et alcynyle en C₂₋₄ ;
l'indice m représente un entier choisi parmi 0, 1 ou 2 ;
l'indice n représente un entier choisi parmi 0, 1 ou 2 ; et
l'indice p représente un entier choisi parmi 1, 2 ou 3.

26. Composé selon la revendication 2, ou un sel pharmaceutiquement acceptable de celui-ci, dans lequel :
le cycle A représente un groupement hétéroaryle comportant 5 à 10 chaînons ou hétérocycloalkyle comportant 4 à 11 chaînons, dans lequel les groupements hétéroaryle comportant 5 à 10 chaînons et hétérocycloalkyle comportant 4 à 11 chaînons comportent chacun 1 à 4 hétéroatomes en tant que chaînons choisis parmi N, O et S, dans lequel l'atome N ou S en tant que chaînon est éventuellement oxydé et un ou plusieurs atomes de carbone en tant que chaînons sont chacun éventuellement remplacés par un groupement carbonyle ;
et dans lequel le cycle A est éventuellement substitué par 1, 2 ou 3 substituants R⁶ ;
L représente une liaison, -C(O)NR¹³- ou -NR¹³C(O)- ;
X représente CR¹⁷, dans lequel R¹⁷ représente H ou un groupement alkyle en C₁₋₄ ;
l'un des radicaux R¹ et R² représente un groupement - (CR⁸R⁹)ₚ-NR¹⁰R¹¹ et l'autre représente H ou un groupement alkyle en C₁₋₄ ou alkoxy en C₁₋₄ ;
R³ représente un groupement méthyle ou halogéno ;
R⁴ représente un groupement alkyle en C₁₋₄ ou alkoxy en C₁₋₄ ;
R⁵ représente un groupement alkyle en C₁₋₄, alkoxy en C₁₋₄ ou halogéno ;
chacun des radicaux R⁶ est indépendamment choisi parmi H et les groupements halogéno, alkyle en C₁₋₆, alcényle en C₂₋₆, alcynyle en C₂₋₆, hétéroaryle comportant 5 à 14 chaînons, hétérocycloalkyle comportant 4 à 10 chaînons, (hétéroaryle comportant 5 à 14 chaînons)- (alkyle en C₁₋₄)- et (hétérocycloalkyle comportant 4 à 10 chaînons)-(alkyle en C₁₋₄)-, dans lequel chacun des groupements alkyle en C₁₋₆, alcényle en C₂₋₆, alcynyle en C₂₋₆, hétéroaryle comportant 5 à 14 chaînons, hétérocycloalkyle comportant 4 à 10 chaînons, (hétéroaryle comportant 5 à 14 chaînons)- (alkyle en C₁₋₄)- et (hétérocycloalkyle comportant 4 à 10 chaînons)-(alkyle en C₁₋₄)- de R⁶ est éventuellement substitué par 1, 2 ou 3 substituants R^{b} ;
R⁷ représente H ou un groupement alkyle en C₁₋₄ ;
chacun des radicaux R⁸ et R⁹ est indépendamment choisi parmi H et les groupements alkyle en C₁₋₄ ;
chacun des radicaux R¹⁰ et R¹¹ est indépendamment choisi parmi H et les groupements alkyle en C₁₋₆ éventuellement substitués par 1 ou 2 substituants R^{f} indépendamment choisis ;
ou R¹⁰ et R¹¹ forment conjointement avec l'atome d'azote auquel ils sont liés un groupement hétérocycloalkyle comportant 4, 5, 6 ou 7 chaînons, dans lequel le groupement hétérocycloalkyle comportant 4, 5, 6 ou 7 chaînons est éventuellement substitué par 1, 2 ou 3 substituants R^{h} ;
R¹² représente H ou un groupement alkyle en C₁₋₄ ;
chacun des radicaux R¹³ représente indépendamment H ou un groupement alkyle en C₁₋₆ ;
chacun des substituants R^{b} est indépendamment choisi parmi les groupements halogéno, alkyle en C₁₋₆, OH, NH₂, C(O)OR^{c}, NHR^{c} et NR^{c}R^{c} ;
chacun des radicaux R^{c} est indépendamment choisi parmi H et les groupements alkyle en C₁₋₆, alcényle en C₂₋₆, alcynyle en C₂₋₆, cycloalkyle en C₃₋₁₀ et (cycloalkyle en C₃₋₁₀) - (alkyle en C₁₋₄)-, dans lesquels chacun des groupements alkyle en C₁₋₆, alcényle en C₂₋₆, alcynyle en C₂₋₆, cycloalkyle en C₃₋₁₀ et (cycloalkyle en C₃₋₁₀)-(alkyle en C₁₋₄)- de R^{c} est éventuellement substitué par 1 ou 2 substituants R^{f} ;
chacun des radicaux R^{f} est indépendamment choisi parmi les groupements alkyle en C₁₋₄, alcényle en C₂₋₆, alcynyle en C₂₋₆, halogéno, OR^{g} et C(O)OR^{g} ;
chacun des radicaux R^{g} est indépendamment choisi parmi H et les groupements alkyle en C₁₋₆ ;
chacun des radicaux R^{h} est indépendamment choisi parmi les groupements alkyle en C₁₋₆, alcényle en C₂₋₆, alcynyle en C₂₋₆, halogéno, CN, ORⁱ et C(O)ORⁱ ;
ou deux substituants R^{c} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
chacun des radicaux R¹ est indépendamment choisi parmi H et les groupements alkyle en C₁₋₄ ;
l'indice m représente un entier choisi parmi 0 ou 1 ;
l'indice n représente un entier choisi parmi 0 ou 1 ; et
l'indice p représente un entier choisi parmi 1 ou 2.

27. Composé selon la revendication **2**, ou un sel pharmaceutiquement acceptable de celui-ci, dans lequel :
le cycle A représente un groupement hétéroaryle comportant 5 à 10 chaînons, dans lequel le groupement hétéroaryle comportant 5 à 10 chaînons comporte 1 à 4 hétéroatomes en tant que chaînons choisis parmi N, O et S, dans lequel l'atome N ou S en tant que chaînon est éventuellement oxydé et un ou plusieurs atomes de carbone en tant que chaînons sont chacun éventuellement remplacés par un groupement carbonyle ; et dans lequel le cycle A est éventuellement substitué par 1 ou 2 substituants R⁶ ;
L représente une liaison, -C(O)NR¹³- ou -NR¹³C(O)- ;
X représente CR¹⁷, dans lequel R¹⁷ représente H ;
l'un des radicaux R¹ et R² représente -(CR⁸R⁹)_{P}-NR¹⁰R¹¹ et l'autre représente H ;
R³ représente un groupement méthyle ou halogéno ;
R⁴ représente un groupement alkyle en C₁₋₄ ou alkoxy en C₁₋₄ ;
R⁵ représente un groupement alkyle en C₁₋₄ ou halogéno ;
chacun des radicaux R⁶ est indépendamment choisi parmi H et les groupements alkyle en C₁₋₆ et (hétérocycloalkyle comportant 4 à 10 chaînons) - (alkyle en C₁₋₄)-, dans lesquels chacun des groupements alkyle en C₁₋₆ et (hétérocycloalkyle comportant 4 à 10 chaînons) - (alkyle en C₁₋₄)- de R⁶ est éventuellement substitué par 1 ou 2 substituants R^{b} ;
R⁷ représente H ;
chacun des radicaux R⁸ et R⁹ est indépendamment choisi parmi H et les groupements alkyle en C₁₋₄ ;
chacun des radicaux R¹⁰ et R¹¹ est indépendamment choisi parmi H et les groupements alkyle en C₁₋₆ éventuellement substitués par 1 ou 2 substituants R^{f} indépendamment choisis ;
ou R¹⁰ et R¹¹ forment conjointement avec l'atome d'azote auquel ils sont liés un groupement hétérocycloalkyle comportant 4, 5, 6 ou 7 chaînons, dans lequel le groupement hétérocycloalkyle comportant 4, 5, 6 ou 7 chaînons est éventuellement substitué par 1, 2 ou 3 substituants R^{h} ;
R¹² représente H ;
R¹³ représente H ;
chacun des substituants R^{b} est indépendamment choisi parmi les groupements OH, C(O)OR^{c}, NHR^{c} et NR^{c}R^{c} ;
chacun des radicaux R^{c} est indépendamment choisi parmi H et les groupements alkyle en C₁₋₆ et cycloalkyle en C₃₋₁₀, dans lequel chacun des groupements alkyle en C₁₋₆ et cycloalkyle en C₃₋₁₀ de R^{c} est éventuellement substitué par 1 ou 2 substituants R^{f} ;
chacun des radicaux R^{f} est indépendamment choisi parmi OR^{g} et C(O)OR^{g} ;
R^{g} représente H ;
chacun des radicaux R^{h} est indépendamment choisi parmi ORⁱ et C(O)ORⁱ ;
ou deux substituants R^{c} quelconques forment conjointement avec l'atome d'azote auquel ils sont liés un groupement hétérocycloalkyle comportant 4, 5, 6 ou 7 chaînons éventuellement substitué par 1, 2 ou 3 substituants R^{h} indépendamment choisis ;
Rⁱ représente H ;
l'indice m représente un entier choisi parmi 0 ou 1 ;
l'indice n représente un entier choisi parmi 0 ou 1 ; et
l'indice p représente un entier choisi parmi 1 ou 2.

28. Composition pharmaceutique comprenant un composé selon l'une quelconque des revendications **1 à 21** et **25 à 27**, ou un sel pharmaceutiquement acceptable de celui-ci, et un ou plusieurs excipients ou véhicules pharmaceutiquement acceptables.

29. Composition pharmaceutique comprenant un composé selon l'une quelconque des revendications **22 à 24,** ou un sel pharmaceutiquement acceptable de celui-ci, et un ou plusieurs excipients ou véhicules pharmaceutiquement acceptables.

30. Composé selon l'une quelconque des revendications **1 à 21** et **25 à 27,** ou un sel pharmaceutiquement acceptable de celui-ci, ou une composition selon la revendication **28**, pour une utilisation dans une méthode de traitement d'une maladie ou d'un trouble associés à l'inhibition de l'interaction PD-1/PD-L1, dans lequel la maladie ou le trouble sont le cancer ou une infection.

31. Composé, sel ou composition pour une utilisation selon la revendication **30**, dans lesquels la maladie ou le trouble sont une infection, et l'infection est une infection virale.

32. Composé, sel ou composition pour une utilisation selon la revendication **30**, dans lesquels la maladie ou le trouble sont un cancer.

33. Composé, sel ou composition pour une utilisation selon la revendication 32, dans lesquels le cancer est choisi parmi les suivants :
(a) cancer des os, cancer du pancréas, cancer de la peau, cancer de la tête ou du cou, mélanome malin cutané, mélanome malin intra-oculaire, cancer de l'utérus, cancer de l'ovaire, cancer du rectum, cancer de la région anale, cancer de l'estomac, cancer du testicule, cancer de la trompe de Fallope, carcinome de l'endomètre, cancer de l'endomètre, carcinome du col de l'utérus, carcinome du vagin, carcinome de la vulve, maladie de Hodgkin, lymphome non hodgkinien, cancer de l'œsophage, cancer de l'intestin grêle, cancer du système endocrinien, cancer de la thyroïde, cancer de la parathyroïde, cancer de la glande surrénale, sarcome des tissus mous, cancer de l'urètre, cancer du pénis, leucémies chroniques, leucémies aiguës, tumeurs solides de l'enfant, lymphome lymphocytique, cancer de la vessie, cancer du rein, carcinome du bassinet du rein, néoplasie du système nerveux central (SNC), lymphome primaire du SNC, angiogenèse tumorale, tumeur de l'axe rachidien, gliome du tronc cérébral, adénome hypophysaire, sarcome de Kaposi, cancer épidermoïde, cancer à cellules squameuses, lymphome à cellules T, cancers induits par l'environnement y compris ceux induits par l'amiante, mélanome, mélanome malin métastatique, cancer du rein, carcinome à cellules claires, cancer de la prostate, adénocarcinome prostatique réfractaire aux hormones, cancer du sein, cancer du côlon, cancer du poumon, carcinome pulmonaire non à petites cellules, carcinome pulmonaire à petites cellules, tumeurs solides, cancer du foie, cancer de l'estomac, glioblastome, sarcome, cancers hématologiques, lymphome, leucémie, leucémie aiguë lymphoblastique (LAL), leucémie myéloïde aiguë (LMA), leucémie lymphoïde chronique (LLC), leucémie myéloïde chronique (LMC), LDGCB, lymphome à cellules du manteau, LNH en rechute, LNH réfractaire, LNH folliculaire récurrent, myélome multiple, cholangiocarcinome, cancer des voies biliaires, cancer du sein triple négatif, rhabdomyosarcome, léiomyosarcome, carcinome hépatocellulaire, sarcome d'Ewing, cancer du cerveau, tumeur cérébrale, astrocytome, neuroblastome, neurofibrome, carcinome basocellulaire, chondrosarcome, sarcome épithélioïde, cancer de l'œil, cancer de la trompe de Fallope, cancer gastro-intestinal, tumeurs stromales gastro-intestinales, leucémie à tricholeucocytes, cancer de l'intestin, cancer des îlots de Langerhans, cancer oral, cancer de la bouche, cancer de la gorge, cancer du larynx, cancer de la lèvre, mésothéliome, cancer des cavités nasales, cancer oculaire, mélanome oculaire, cancer du bassin, carcinome à cellules rénales, cancer de la glande salivaire, cancer des sinus, cancer du rachis, cancer de la langue, carcinome tubuleux et cancer de l'uretère ; ou
(b) cancer métastatique qui exprime PD-L1 ; ou
(c) cancer du poumon ; ou
(d) carcinome pulmonaire à petites cellules ; ou
(e) carcinome pulmonaire non à petites cellules ; ou
(f) cancer du rein ; ou
(g) cancer du foie ; ou
(h) carcinome hépatocellulaire ; ou
(i) mélanome ; ou
(j) cancer de la vessie ; ou
(k) cancer de l'urètre ; ou
(l) cancer du rein ; ou
(m) carcinome à cellules rénales.

34. Composé selon l'une quelconque des revendications **1-21** et **25-27,** ou un sel pharmaceutiquement acceptable de celui-ci, ou composition selon la revendication **28**, pour une utilisation dans une méthode d'amplification, de stimulation et/ou d'augmentation de la réponse immunitaire chez un patient, dans lesquels le patient souffre d'un cancer ou d'une infection.

35. Composé selon l'une quelconque des revendications **22** à **24**, ou un sel pharmaceutiquement acceptable de celui-ci, ou composition selon la revendication **29**, pour une utilisation dans une méthode de traitement d'une maladie ou d'un trouble associés à l'inhibition de l'interaction PD-1/PD-L1, dans lesquels la maladie ou le trouble sont un cancer ou une infection.

36. Composé, sel ou composition pour une utilisation selon la revendication **35**, dans lesquels la maladie ou le trouble sont un cancer.

37. Composé, sel ou composition pour utilisation selon la revendication **36**, dans lequel le cancer est choisi parmi les suivants : cancer urothélial, cancers avec forte instabilité microsatellitaire (MSI^{high}) , cancers des voies urogénitales, cancers du foie, cancers du système nerveux, cancers gynécologiques, leucémie promyélocytaire aiguë (LPA), maladies myéloprolifératives, myélofibrose primaire (MFP), polyglobulie essentielle (PE), thrombocytose essentielle (TE), syndrome myélodysplasique (SMD), lymphome lymphoblastique aigu à cellules T (LLA-T), ostéosarcome, angiosarcome, fibrosarcome, liposarcome, myxome, rhabdomyome, rhabdosarcome, fibrome, lipome, harmatome, tératome, carcinome bronchogénique, carcinome à cellules squameuses, carcinome à petites cellules indifférenciées, carcinome à grandes cellules indifférenciées, adénocarcinome, carcinome alvéolaire (bronchiolaire), adénome bronchique, harmatome chondromateux, carcinome, adénocarcinome ductal, insulinome, glucagonome, gastrinome, tumeurs carcinoïdes, vipome, cancers de l'intestin grêle, léiomyome, hémangiome, cancers du côlon, adénome tubulaire, adénome villeux, hamartome, cancer colorectal, tumeur de Wilm [néphroblastome], carcinome des cellules transitoires, séminome, carcinome embryonnaire, tératocarcinome, choriocarcinome, sarcome, carcinome à cellules interstitielles, fibroadénome, tumeurs adénomatoïdes, hépatome, hépatoblastome, adénome hépatocellulaire, sarcome ostéogénique, histiocytome fibreux malin, lymphome malin, sarcome à cellules du réticulum, tumeur maligne à cellules géantes, chordome, ostéochronfrome, exostoses ostéocartilagineuses, chondrome bénin, chondroblastome, chondromyxofibrome, ostéome ostéoïde, tumeurs à cellules géantes, cancers du crâne, ostéome, granulome, xanthome, maladie de Paget, cancers des méninges, méningiome, méningiosarcome, gliomatose, méduloblastome, gliome, épendymome, germinome, pinéalome, glioblastome multiforme, oligodendrogliome, schwannome, rétinoblastome, tumeurs congénitales, cancers de la moelle épinière, maladie de Lhermitte-Duclos, carcinome de l'endomètre, cancers du col de l'utérus, dysplasie cervicale prétumorale, carcinome ovarien, cystadénocarcinome séreux, cystadénocarcinome mucineux, carcinome non classifié, tumeurs des cellules thécales de la granulosa, tumeurs des cellules de Sertoli-Leydig, dysgerminome, tératome malin, cancers de la vulve, carcinome intra-épithélial, cancers du vagin, sarcome botryoïde, rhabdomyosarcome embryonnaire, naevi dysplasiques, angiome, dermatofibrome, chéloïdes, syndromes myélodysplasiques et carcinome urothélial.
